# EUROPÄISCHE PATENTSCHRIFT

(11) **EP 1 181 294 B1**
(45) Veröffentlichungstag und Bekanntmachung des Hinweises auf die Patenterteilung: **31.03.2004**
(21) Anmeldenummer: 01914813.9
(22) Anmeldetag: 22.03.2001
(51) Int. Cl.: C07D 491/10, C07D 307/94, A61K 31/55, A61K 31/343, A61P 25/28

(54) **NEUE DERIVATE UND ANALOGA VON GALANTHAMIN**
NOVEL DERIVATIVES AND ANALOGUES OF GALANTHAMIN
NOUVEAUX DERIVES ET ANALOGUES DE LA GALANTHAMINE

(30) Priorität: 31.03.2000 AT 5462000; 15.02.2001 AT 2382001
(43) Veröffentlichungstag der Anmeldung: 27.02.2002
(73) Patentinhaber: Sanochemia Pharmazeutika Aktiengesellschaft, 1090 Wien (AT)
(72) Erfinder: JORDIS, Ulrich, A-1190 Wien (AT); FRÖHLICH, Johannes, A-2392 Dornach im Wienerwald (AT); TREU, Matthias, A-1220 Wien (AT); HIRNSCHALL, Manfred, A-1160 Wien (AT); CZOLLNER, Laszlo, A-2490 Ebenfurth (AT); KÄLZ, Beate, A-7035 Steinbrunn (AT); WELZIG, Stefan, A-1210 Wien (AT)
(74) Vertreter: Beer, Manfred, Dipl.-Ing.
(86) Internationale Anmeldenummer: PCT/AT2001/000082
(87) Internationale Veröffentlichungsnummer: WO 2001/074820

(56) Entgegenhaltungen:
- WO-A-00/32199
- WO-A-96/12692
- WO-A-97/40049
- ROQUES, R. ET AL: "Structure of norgalanthamine hydrochloride" ACTA CRYSTALLOGR., SECT. B (1980), B36(7), 1589-93 , XP001009771
- BASTIDA, JAUME ET AL: "Narcissus alkaloids. Part 19. Alkaloids from Narcissus leonensis" PHYTOCHEMISTRY (1993), 34(6), 1656-8 , XP001009775

## Beschreibung

Die Erfindung betrifft neue, substituierte Benzofuranderivate, Verfahren zu ihrer Herstellung, deren Salze sowie die Verwendung zur
a) Behandlung der Alzheimer'schen Krankheit,
b) Behandlung der Parkinson'schen Krankheit,
c) Behandlung der Huntington'schen Krankheit (Chorea),
d) Behandlung der Multiplen Sklerose,
e) Behandlung der Amyotrophen Lateralsklerose,
f) Behandlung der Epilepsie,
g) Behandlung der Folgen des Schlaganfalles,
h) Behandlung der Folgen des Schädel-Hirn-Traumas,
i) Behandlung und Prophylaxe der Folgen diffusen Sauerstoff- und Nährstoffmangels im Gehirn, wie sie nach Hypoxie, Anoxie, Asphyxie, Herzstillstand, Vergiftungen, sowie bei Komplikationen bei schweren Geburten am Säugling oder bei Narkosen beobachtet werden,
j) insbesondere auch prophylaktischen Behandlung apoptotischer Degeneration in Neuronen, die durch lokale Radio- oder Chemotherapie von Gehirntumoren geschädigt wurden bzw. werden,
k) Behandlung der bakteriellen Meningitis,
l) Behandlung von Erkrankungen mit apoptotischer Komponente, besonders im Gefolge von amyloid-assoziierter Zelldegeneration,
m) Behandlung des Diabetes mellitus, insbesondere, wenn die Krankheit mit Amyloiddegeneration der Inselzellen einhergeht,
n) Erhöhung der Muskelkraft und
o) Erhöhung der Ausdauer von Alzheimer-Patienten.

Aus der WO 97/40049 A sind Analoga und Derivate von Galanthamin der nachstehenden allgemeinen Formel 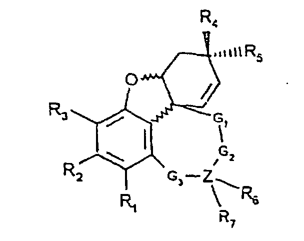 und diese Verbindungen (Benzofuranderivate) enthaltende Arzneimittel bekannt. Weitere Analoga und Derivate des Galanthamins der nachstehenden Formel 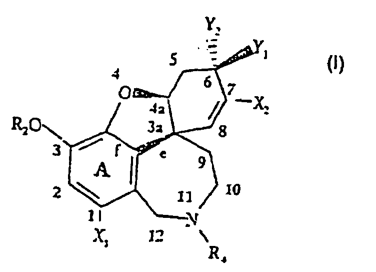 sind aus der WO 96/12692 A bekannt. In der WO 96/12692 A ist auch ein Verfahren zum Herstellen der gattungsgemäßen Benzofuranderivate bekannt.

Die erfindungsgemäßen, neuen Verbindungen sind solche der allgemeinen Formel I 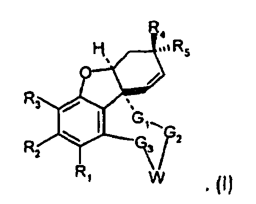 worin die Substituenten die nachstehend erläuterten Bedeutungen haben:
A) R₁ und R₂ sind gleich oder verschieden und bedeuten:
   Aa) Wasserstoff, F, Cl, Br, J, CN. NC, OH, SH, NO₂, SO₃H, PO₃H, NH₂, CF₃, OSO₂(CH₂)ₙCF₃, worin n gleich 0, 1 oder 2 ist, -OSO₂-Aryl, -Vinyl- oder -Ethinyl;
   Ab) eine niedrige (C₁-C₆), gegebenenfalls verzweigte Alkyl-, Aralkyl-, Alkoxyl-, Aralkoxy-, Cycloalkyl- oder Cycloalkoxygruppe,
   Ac) eine Aminogruppe, die gegebenenfalls durch eine oder zwei gleiche oder verschiedene niedrige (C₁-C₆), gegebenenfalls verzweigte Alkyl-, Aralkyl-, oder Alkylcarbonyl-, Aralkylcarbonyl-, oder Alkoxycarbonyl-, Aralkoxycarbonylgruppen oder durch eine Gruppe ausgewählt aus einem Pyrrolidin-, Piperidin-, Morpholin-, Thiomorpholin-, Piperazin- oder Homopiperazinrest substituiert ist:
   Ad) eine -COOH, -COOAlkyl-, -COOAralkyl-, CO-Amino-Gruppe, die gegebenenfalls wie oben unter Ac) angegeben, substituiert ist, oder eine COH-Alkylgruppe oder COH-Aralkylgruppe;
   Ae) eine -(CH₂)ₙX (worin X = Br, Cl, F oder J ist), -(CH₂)ₙOH-, - (CH₂)ₙCHO-, - (CH₂)ₙCOOH-, - (CH₂)ₙCN-, -(CH₂)ₙNC-, -(CH₂)ₙCOAlkyl- oder -(CH₂)ₙCOAryl-Gruppe, worin n 1-4 ist;
   Af) eine -(CH₂)ₙVinyl-, - (CH₂)ₙEthinyl-, -(CH₂)ₙ Cycloalkyl-Gruppe, worin n 0, 1 oder 2 ist, wobei Cycloalkyl ein aliphatischer Ring mit 3 bis 7 C-Atomen ist;
   Ag) eine C₃-C₆ substituierte Alkenylgruppe (gegebenenfalls substituiert mit H, F, Br, Cl, CN, CO₂Alkyl, COAlkyl, COAryl);
   Ah) eine C₃-C₆ substituierte Alkinylgruppe (gegebenenfalls substituiert mit H, F, Br, Cl, CN, CO₂Alkyl, COAlkyl, COAryl); oder
   Ai) R₁ und R₂ bedeuten gemeinsam -CH=CH-CH=CH-, -O(CH₂)ₙO- (n =1 bis 3), -CH=CHA₁-(A₁ ist NH, O oder S), oder -CH₂CH₂A₁- (A₁ ist NH, O oder S);
B) R₃ dieselbe Bedeutung hat wie R₁, insbesondere OH und OCH₃ ist, oder
C) R₂ und R₃ gemeinsam -A₂(CH₂)ₙA₂- bedeuten, worin n 1 bis 3 ist und die Substituenten A₂ gleich oder verschieden sind und NH, O oder S bedeuten;
D) R₄ und R₅ sind entweder
   Da) beide Wasserstoff,
   oder
   Db) einer von R₄ und R₅ ist Wasserstoff, eine Alkyl-, Aralkyl-, Alkenyl-, Aralkenyl-, Alkinyl-, oder Aralkinylgruppe und der andere von R₄ und R₅ ist
      Dbi) OR₆, worin R₆ Wasserstoff, eine niedrige (C₁-C₁₀), gegebenenfalls verzweigte Alkylgruppe, oder Cycloalkylgruppe, eine C₃-C₁₀ substituierte Silylgruppe (beispielsweise Triethylsilyl, Trimethylsilyl, t-Butyldimethylsilyl oder Dimethylphenylsilyl), eine C₂-C₁₀-alpha-Alkoxyalkyl-Gruppe, beispielsweise Tetrahydropyranyl, Tetrahydrofuranyl, Methoxymethyl, Ethoxymethyl, 2-Methoxypropyl, Ethoxyethyl, Phenoxymethyl oder 1-Phenoxyethyl;
      Dbii) O-CS-NHR₆ (Thiourethane), worin R₆ die oben unter Dbi) angegebene Bedeutungen hat;
      Dbiii) O-CO-NHR'₇ mit der nachstehenden Bedeutung: 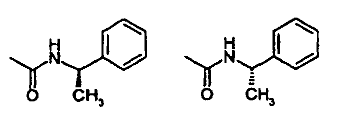
      Dbiv) O-CO-HR₆, worin R₆ die oben unter Dbi) genannte Bedeutungen hat, insbesondere Ester mit den Substitutionsmuster von Aminosäuren (beide Enantiomeren), wie 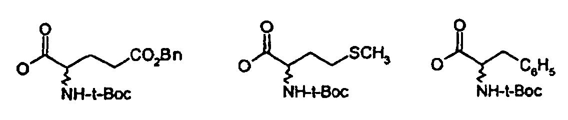
      Dbv) NR₇R₇, worin die beiden Substituenten R₇ gleich oder verschieden sind und Wasserstoff, eine niedrige (C₁-C₄), gegebenenfalls verzweigte, Alkylgruppe oder Cycloalkylgruppe bedeuten, oder die Substituenten R₇ sind gemeinsam -(CH₂)ₙ-, worin n 3 bis 5 ist;
      Dbvi) NH-COR₆ (Amid), worin R₆ die oben unter Dbi) genannte Bedeutungen hat;
      Dbvii) S-R₆, worin R₆ die oben unter Dbi) angegebene Bedeutung hat;
      Dbviii) SOₙR₈, worin n 0, 1 oder 2 ist und worin R₈ eine (C₁-C₁₀), gegebenenfalls verzweigte oder cyclische, gegebenenfalls substituierte Alkyl- oder Aralkylgruppe ist;
      Dbix) R₄ ist Wasserstoff und R₅ OH, CN, CO₂-Alkyl, CONRₐR_{b}, worin Rₐ Wasserstoff, eine niedrige (C₁ - C₆), gegebenenfalls verzweigte oder cyclische substituierte Alkylgruppe und R_{b} Wasserstoff, eine niedrige (C₁ - C₆), gegebenenfalls verzweigte, oder substituierte Alkylgruppe ist, oder Ra+Rb sind gemeinsam -(CH₂)ₙ-, worin n 2 bis 6 bedeutet, oder -(CH₂)ₙE(CH₂)ₙ-, worin E gleich NH, N-Alkyl, O, oder S und n 0 bis 5 ist, Aryl (Phenyl oder Naphthyl), oder ein 6-π Heterozyklus ist;
      Dbx) R₄ und R₅ sind, gemeinsam Carbonyl (=O), Hydrazon (=N-NH-R₉, =N-NR₉R₁₀₎ oder Oxim (=N-OR₁₀) worin R₉ Wasserstoff, eine niedrige (C₁-C₆), gegebenenfalls verzweigte oder cyclische, gegebenenfalls substituierte Alkyl-, Aralkyl-, Alkylcarbonyl-, Aralkylcarbonyl-, Alkylcarbonyloxy- oder Aralkylcarbonyloxygruppe oder eine Sulfonsäuregruppe, wie Tosyl oder Mesyl ist, und R₁₀ Wasserstoff, eine niedrige (C₁-C₆), gegebenenfalls verzweigte oder cyclische, gegebenenfalls substituierte Alkyl-, Aralkyl-, Alkylcarbonyl-, oder Aralkylcarbonylgruppe, eine Sulfonsäuregruppe, wie eine Tosyl- oder Mesylgruppe ist;
      Dbxi) R₄ und R₅ sind, gemeinsam Substituenten der Art 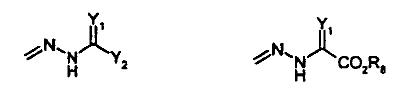
   worin Y₁, Y₂ gleich oder verschieden sind und O, S, NH oder N-R₉ (freie Valenzen sind in jedem Fall Wasserstoff), worin R₉ die oben in Dbx) genannten Bedeutungen hat;
E) G₁: -(CH₂)ₓ-, worin x 1 oder 2 ist;
F) G₂: -(CH₂)_{y}-, worin y 0 bis 2 ist;
   E/Fa: G₁ und G₂ bedeuten gemeinsam oder getrennt:
      - C(R₁₁ R₁₂)-, worin R₁₁ und R₁₂ Wasserstoff, OH, eine niedrige, gegebenenfalls verzweigte oder cyclische, gegebenenfalls substituierte Alkyl-, Aralkyl-, Aryl-, Alkyloxy-, Arakyloxy-, oder Aryloxygruppe oder gemeinsam eine Alkylspirogruppe (C₃-C₇-Spiroring);
   E/Fb: G₁ und G₂ bedeuten gemeinsam 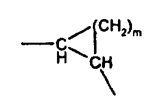 worin m 1 bis 7 ist;
   E/Fc: G₁ und G₂ bedeuten gemeinsam oder getrennt -CR₁₁R₁₂-, worin R₁₁ und R₁₂ Wasserstoff, Hydroxy, eine niedrige, gegebenenfalls verzweigte oder cyclische, gegebenenfalls substituierte Alkyl-, Aralkyl-, Aryl-, Alkoxy-, Aralkoxy-, oder Aryloxygruppe bedeuten;
   E/Fd: G₁ und G₂ sind gemeinsam Alkylspirogruppe (C₃-C₇-Spiroring) ;
G) G₃: -(CH₂)_{z}-, worin z 0 bis 3 ist, mit der Maßgabe, daß die Summe aus x+y+z wenigstens 2 und höchstens 4 ist, oder worin G₃ die Bedeutung Carbonyl oder Thiocarbonyl, - CH(OH)- oder -C(OH)= ist;
H) W ist:
   Ha) CR₁₃R₁₄, worin R₁₃ Wasserstoff und R₁₄ -(CH₂)ₙNR₇R₇, -CO-NR₇R₇ oder -COOR₇ bedeuten, worin n 0 bis 2 ist und R₇ die oben genannten Bedeutungen hat, oder R₇ und R₇ bilden über -(CH₂)ₙ-, worin n 3 bis 5 ist, einen Ring, wobei die Substituenten R₁₃ und R₁₄ vertauscht sein können.
   Hb) N-Phenyl (gegebenenfalls substituiert mit Fluor, Brom, Chlor, (C₁ -C₄)Alkyl, CO₂Alkyl, CN, CONH₂, oder Alkoxy), N-Thien- 2- oder 3-yl, oder N-Fur- 2- oder 3-yl, oder N-1,3,5-Triazinyl bedeutet, wobei der Triazinrest weiter mit Cl, OR₆ oder NR₇R₇ substituiert sein kann, und R₆ und R₇ die oben angeführte Bedeutung haben;
   Hc) einer der nachstehend wiedergegebenen Substituenten 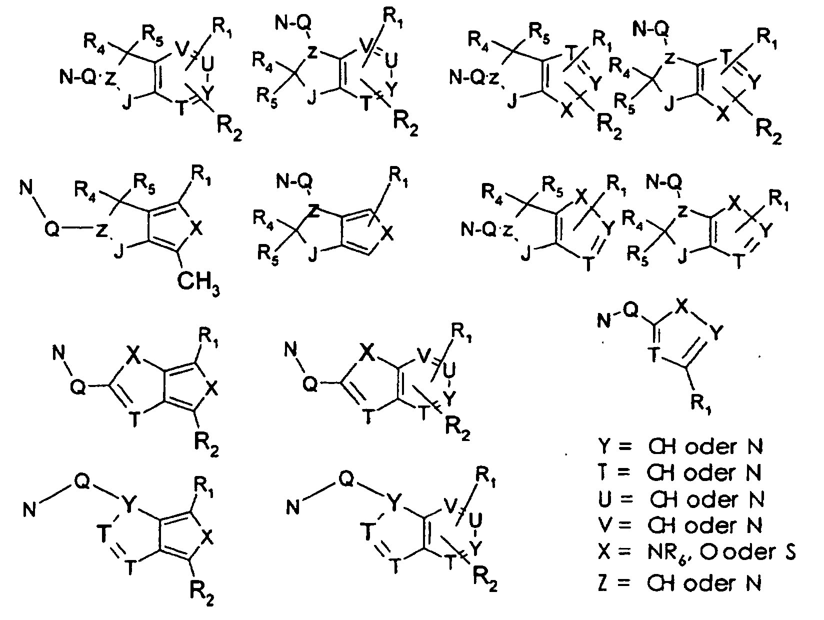 worin J keine Bindung oder -(CH₂)ₙ-, wobei n = 0 bis 3 ist, Carbonyl, Thiocarbonyl, O, S -SO oder SO₂ bedeutet, R₆ die oben angegebenen Bedeutungen hat, und worin Q -(CH₂)ₙ- M*-(CH₂)ₘ- ist, wobei n = 0 bis 4 und m = 0 bis 4 und M* Alkinyl, Alkenyl, disubstituiertes Phenyl, disubstituiertes Thiophen, disubstituiertes Furan, disubstituiertes Pyrazin, disubstituiertes Pydidazin, einen Spacer einer der nachstehend wiedergegebenen Formeln, einen Peptidspacer L oder einen heterocyclischen Spocer HS der nachstehenden Formeln bedeutet, 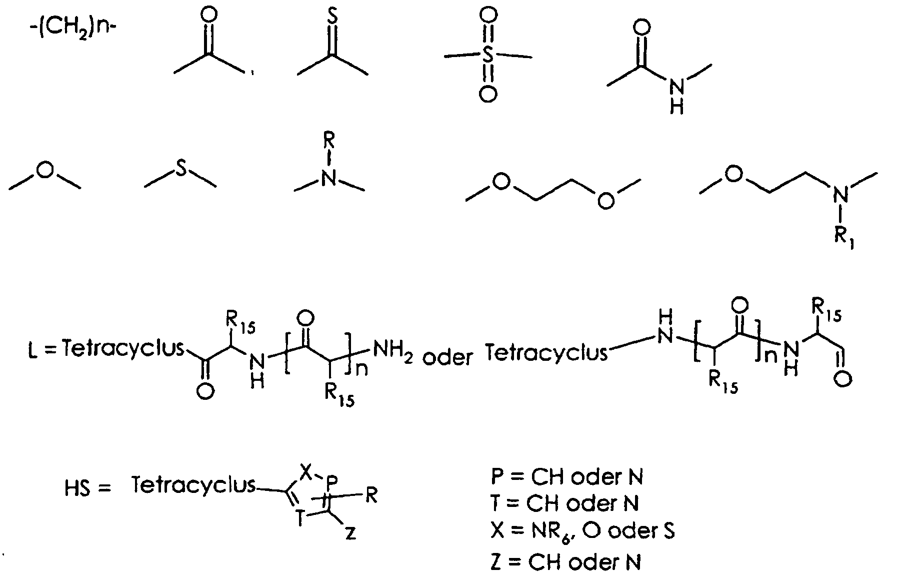 worin R₁₅ die Seitenkette von D-, L-, D,L-Aminosäuren oder unnatürlichen Aminosäuren bedeutet, und für den Fall von n>1 R₁₅ in den einzelnen Resten jeweils eine gleiche oder verschiedene Seitenkette von D-, L-, D,L-Aminosäuren oder unnatürlichen Aminosäuren bedeutet, mit der Maßgabe, daß das Atom N neben Q jeweils mit der Gruppe G2 und G3 der Formel I verbunden ist;
      Hdi) ein, gegebenenfalls wenigstens einfach substituierter, tricyclischer Substituent (Tr) mit wenigstens einem heterocyclischen Ring als Ringbestandteil und einer Bindungsstelle an einem Kohlenstoffatom eines anellierten Benzolringes desselben, der über einen Spacer Q und das Q benachbarte Stickstoffatom jeweils mit G₂ und G₃ der Verbindung der Formel I verbunden ist, wobei Q die oben unter Hc) angegebene Bedeutung hat;
      Hdii) ein cyclischer Kohlenwasserstoff der nachstehenden Formeln ist: 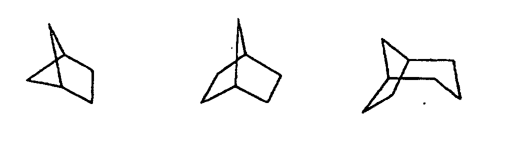 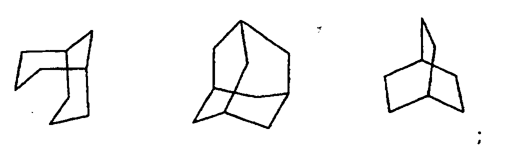
      oder
   He) -NH-, -O-, -S-, -SO- oder -SO₂-;
   Hf) wobei, falls W -NH- ist, z in der Gruppe G₃ 0, 2 oder 3 ist, mit der Maßgabe, dass z in der Gruppe G₃ 1 ist, wenn der Substituent R₁ eine niedrige Alkylgruppe, wie Methyl oder Ethyl, bedeutet.

Beispiele für den tricyclischen Substituent Tr der allgemeinen Formel 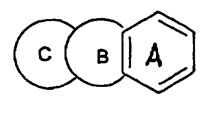 sind:
Carbazol,
1,2,3,4-4a,9a-Hexahydrocarbazol,
9,10-Dihydroacridin,
1,2,3,4-Tetrohydroacridin,
10,11-Dihydro-5H-dibenz[b,f]azepin,
5,6,11,12-Tetrahydrodibenz[b,g]azocin,
6,11-Dihydro-5H-dibenz[b,e,]azepin,
6,7-Dihydro-5H-dibenz[c,e]azepin,
5,6,11,12-Tetrahydrodibenz[b,f]azocin,
Dibenzofuran,
9H-Xanthen,
1-O-11-Dihydrobenz[b,f]oxepin,
6,11-Dihydrobenz[b,e]oxepin,
6,7-Dihydro-5H-dibenz[b,g]oxacin,
Dibenzothiophen,
9H-Thioxanthen,
10,11-Dihydrodibenzo[b,f]thiepin,
6,11-Dihydrodibenzo[b,e]thiepin,
6,7-Dihydro-5H-dibenzo[b,g]thiocin,
10H-Phenothiazin,
10H-Phenoxazin,
5,10-Dihydrophenazin,
10,11-Dibenzo[b,f]-[1,4]thiazepin,
2,3,5,6,11,11a-Hexahydro-1H-pyrrolo[2,1-b][3]benzazepin,
1-O, 11-Dihydro-5H-dibenzo[b,e][1,4]diazepin,
5,11-Dihydrodibenz[b,e][1,4]oxazepin,
5,11-Dihydrodibenzo[b,f][1,4]thiazepin,
10,11-Dihydro-5H-dibenzo[b,e][1,4]diazepin,
1,2,3,3a,8,8a-Hexahydropyrrolo[2,3b]indol.

Der tricyclische Substituent Tr kann ein kondensierter Benzolring der allgemeinen Formel 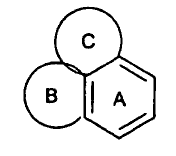 sein und beispielsweise bedeuten:
1H,3H-Naphth[1,8-cd][1,2]oxazin,
Naphth[1,8-de]-1,3-oxazin,
Naphth[1,8-de]-1,2,-oxazin,
1,2,2a,3,4,5-Hexahydrobenz[cd]indol,
2,3,3a,4,5,6-Hexahydro-1H-benzo[de]chinolin,
4H-Pyrrolo[3,2,1-ij]chinolin,
1,2,5,6-Tetrahydro-4H-pyrrolo[3,2,1-ij]chinolin,
5,6-Dihydro-4H-pyrrolo[3,2,1-ij]chinolin,
1H,5H-Benzo[ij]chinolizin,
2,3,6,7-Tetrahydro-1H,5H-benzo[ij]chinolizin,
Azepino[3,2,1-hi]indol,
1,2,4,5,6,7-Hexahydroazepino[3,2,1-hi]indol,
1H-Pyrido[3,2,1-jk][1]benzazepin,
5,6,7,8-Tetrahydro-1H-pyrido[3,2,1-jk][1]benzazepin,
1,2,5,6,7,8-Hexahydro-5H-pyrido[3,2,1-jk][1]benzazepin,
2,3-Dihydro-1H-benz[de]isochinolin,
1,2,3,4,4a,5,6,7-Octahydronaphth[1,8-bc]azepin,
2,3,5,6,7,8-hexahydro-1H-pyrido[3,2,1-jk][1]benzazepin.

Der tricyclische Substituent Tr kann ein kondensierter Benzolring der allgemeinen Formel 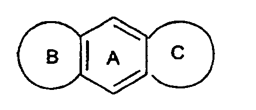 sein. Beispiele für diese Verbindungen sind :
1,2,3,5,6,7-Hexahydrobenzo[1,2-b:4,5b']dipyrrol,
1,2,3,5,6,7-Hexahydrocyclopent[f]indol,
1,2,3,6,7,8-Hexahydrocyclopentan[e]indol oder
2,3,4,7,8-Hexahydro-1H-cyclopenta[f]chinolin.

Der tricyclische Substituent Tr kann ein kondensierter Benzolring der allgemeinen Formel 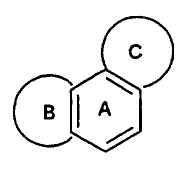 sein. Beispiele hiefür sind:
1,2,3,6,7,8-Hexahydrocyclopent[e]indol oder
2,3,4,7,8,9-Hexahydro-1H-cyclopenta[f]chinolin.

Weitere Beispiele für den tricyclischen Substituenten Tr sind kondensierte Benzolringe der folgenden Formeln wobei die Bindungsstelle zu Q den Platz eines beliebigen Wasserstoffatoms einnehmen kann:

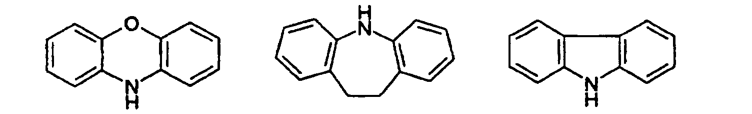

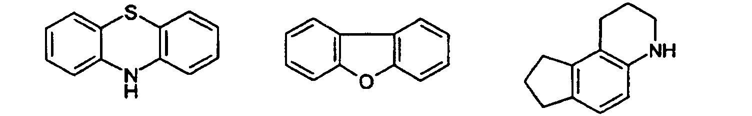

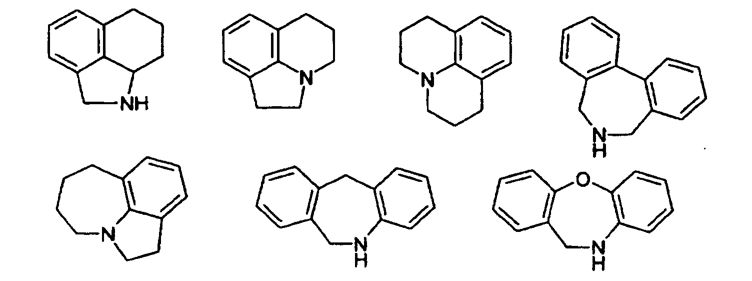

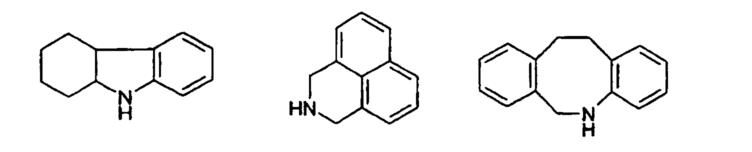

Weiters kann Tr ein cyclischer oder bicyclischer Kohlenwasserstoff sein der durch folgende Formel bezeichnet wird:

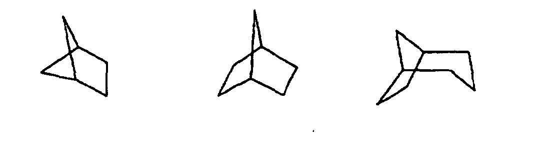

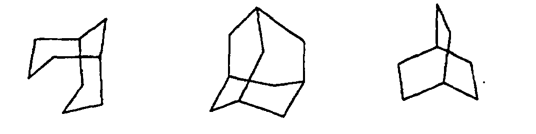

Jeder Substituent Tr kann weiters durch einen oder mehrere Substituenten R₁ substituiert sein, wobei die Definition des Substituenten R₁ dieselbe wie bei Formel 1 ist.

Die Erfindung betrifft weiters Verbindungen der allgemeinen Formel II 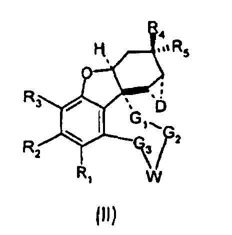 worin D N-H, N-Alkyl, N-Acyl, Sauerstoff oder Schwefel bedeutet und worin die Substituenten R₁ bis R₅, G₁ bis G₃ sowie W die in Patentanspruch 1 bei der allgemeinen Formel I angegebenen Bedeutungen haben.

Die Erfindung betrifft überdies Verbindungen der allgemeinen Formel III 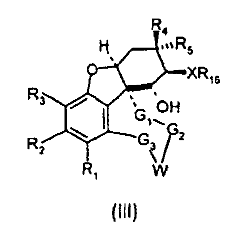 worin X-R₁₆ ein Substituent ist, in dem X Sauerstoff oder Schwefel und R₁₆ Wasserstoff oder eine niedrige (C₁-C₁₀), gegebenenfalls verzweigte, gegebenenfalls substituierte Alkyl- oder Aralkylgruppe ist, und worin die Substituenten R₁ bis R₅, G₁ bis G₃ sowie W die in Patentanspruch 1 bei der allgemeinen Formel 1 angegebenen Bedeutungen haben.

Die Erfindung betrifft schließlich Verbindungen der allgemeinen Formel IV 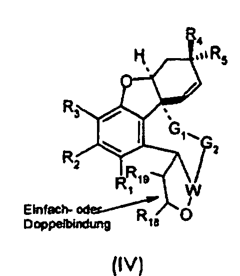 worin R₁₈ und R₁₉ Wasserstoff, Alkyl, Aryl oder Aralkyl bedeuten und in der die die Substituenten R₁₈ und R₁₉ tragenden C-Atome miteinander über eine Einfach- oder eine Doppelbindung verknüpft sind, und worin die Substituenten R₁ bis R₅ und G₁ bis G₃ die in Patentanspruch 1 bei der allgemeinen Formel I angegebenen Bedeutungen haben, wobei W -CH- oder -NHbedeutet.

Die Erfindung bezieht sich auch auf Arzneimittel, die wenigstens eine der Verbindungen der allgemeinen Formeln I, II, III oder IV oder ein pharmazeutisch annehmbares Salz derselben als Wirkstoff enthalten.

Weiters bezieht sich die Erfindung auf die Verbindungen der allgemeinen Formeln I, II, III oder IV, oder ein pharmazeutisch annehmbares Salz derselben zum Herstellen von Arzneimitteln.

Die Erfindung betrifft auch ein Verfahren Verfahren zum Trennen der (+) und (-)-Isomeren von racemischen Verbindungen der allgemeinen Formeln I, II, III oder IV, insbesondere von racemischen (6R)-3-Methoxy-5,6,9,10,11,12-hexahydro-4aH[1]benzofuro[3a,3,2-ef][2-benzyzepin-6-ol (Norgalanthamin) aus einem Isomerengemisch, welches sich dadurch auszeichnet, dass das Verfahren der fraktionierten Kristallisation mit einer chiralen Säure angewendet wird.

Bevorzugte und vorteilhafte Ausgestaltungen der Erfindung sind Gegenstand der abhängigen Ansprüche.

### Trennung der optischen Isomeren von rac. Norgolonthamin:

Die Erfindung beinhaltet außerdem ein Verfahren zur chiralen Trennung von (6R)-3-Methoxy-5,6,9,10,11,12-hexahydro-4aH[1]benzofuro[3a,3,2-ef][2benzazepin-6-ol (Norgalanthamin) (4)

Die Trennung der (+) und (-) Isomeren erfolgt durch fraktionierte Kristallisation in der Weise, daß
- eine Lösung oder Suspension des optischen Isomerengemisches in der 3 bis 50 fachen Menge
- eines Lösungsmittels, wie Wasser, Methanol, Ethanol, Propanol, Isopropanol, Aceton oder Mischungen dieser Lösungsmittel, vorwiegend Methanol
- mit der äquimolaren Menge oder einem Überschuß einer chiralen Säure (unsubstituierte. einfach oder mehrfach substituierte (+) oder (-) Weinsäure, Zitronensäure, Milchsäure. vorzugsweise (+)-O,O-Di-p-Toluoylweinsäure, die in einem der oben genannten Lösungsmittel gelöst ist, zugegeben oder vorgelegt - und die Lösung oder Suspension des optischen Isomerengemisches zugegeben wird,
- daß die Lösung mit aus dem natürlichen (-) Gaianthaminderivaten und chiralen org. Säuren, wie (+)-O,O-Di-p-Toluoylweinsäure, hergestellten Kristallen eingeimpft wird
- und bei - 40 bis +20 Grad, vorzugsweise 0 Grad 2-24 Stunden oder länger stehen gelassen wird.
- daß die gebildeten Kristalle filtriert und getrocknet werden,
- anschließend mit Überschuss NH₄OH versetzt und mit organischem Lösungsmittel, wie Chloroform, Methylenchlorid, Ethylacetat, Butylacetat, Diethylether, t-Butylmethylether, Dibutylether, Petrolether, Xylol, Benzol, Toluol oder ähnlichen Lösungsmitteln extrahiert und durch Destillation des Lösungsmittels das entsprechenden (-) Norgcdanthamin isoliert wird.

In diesem Verfahren ergibt Einengen der Mutterlauge, Aufnehmen im Überschuss NH₄OH, Extraktion mit einem organischen Lösungsmittel (wie oben angegeben) und Eindampfen weitere Fraktionen von Norgalanthamin, aus dem in gleicher Weise wie oben kann mit chiralen organischen Säuren, wie (-)-O,O-Di-p-Toluoylweinsäure das (+) Norgalanthamin hergestellt werden.

Die, nach der Erfindung erhaltenen Produkte können durch ein geeignetes Verfahren gereinigt werden, beispielsweise Sublimation, fraktionierte Kristallisation oder Chromatographie.

Unter den erfindungsgemäßen Verbindungen sind insbesondere die nachstehend genannten Verbindungen in Betracht gezogen:

In der nachstehenden Übersicht bedeutet "AchE":Acetylcholinesterase, "BchE":Butyrylcholinesterase, "hr": human rekombinant, "mE": Vorinkubation des Enzyms mit Hemmstoff und "IC₅₀": Konzentration, bei der eine 50%ige Hemmung eintritt.

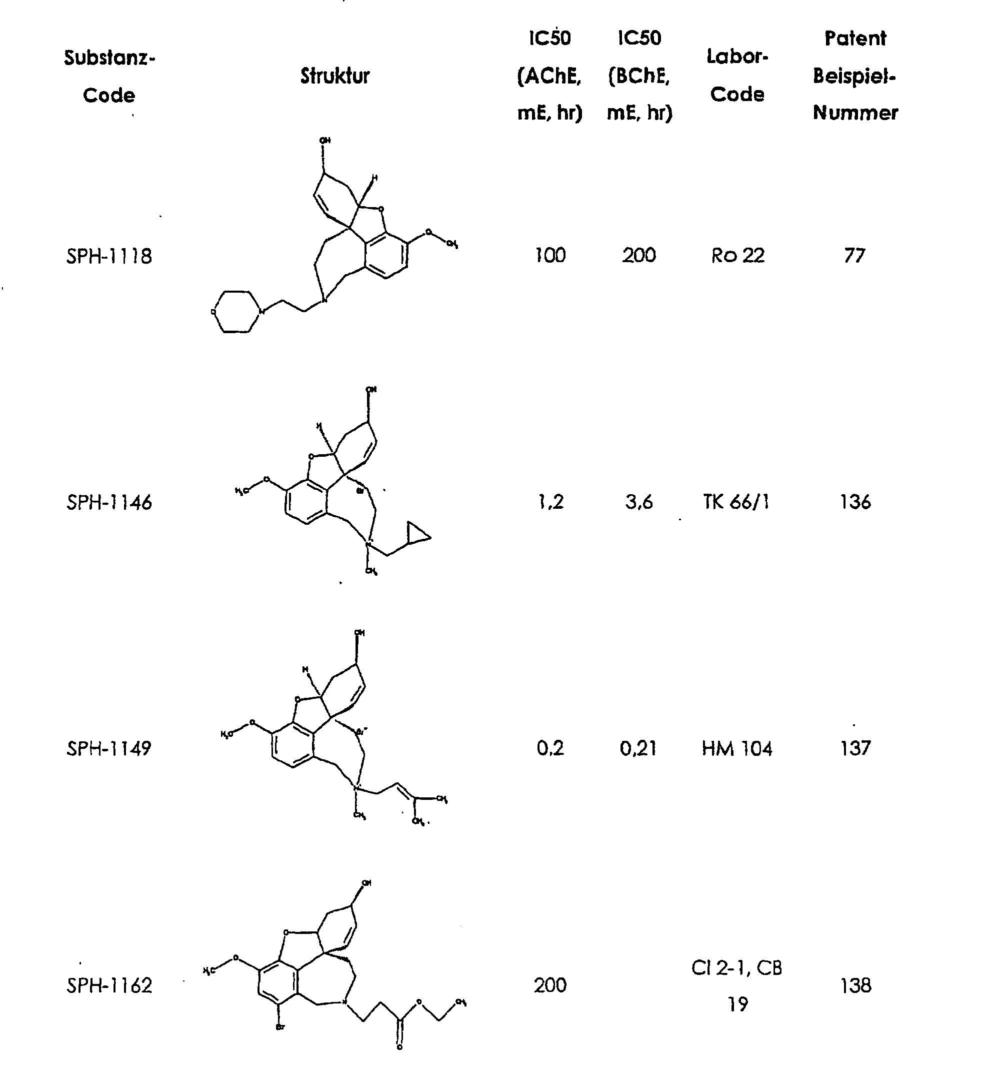

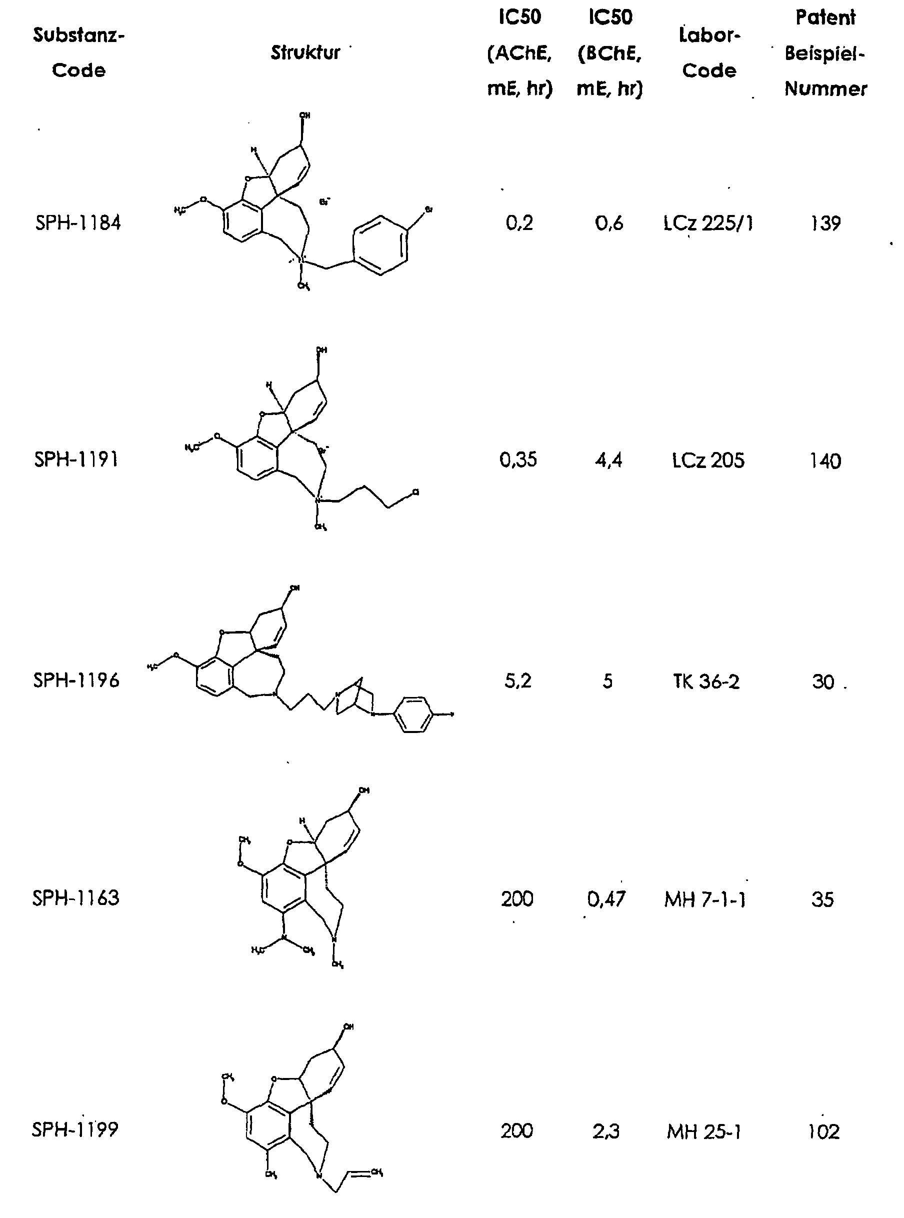

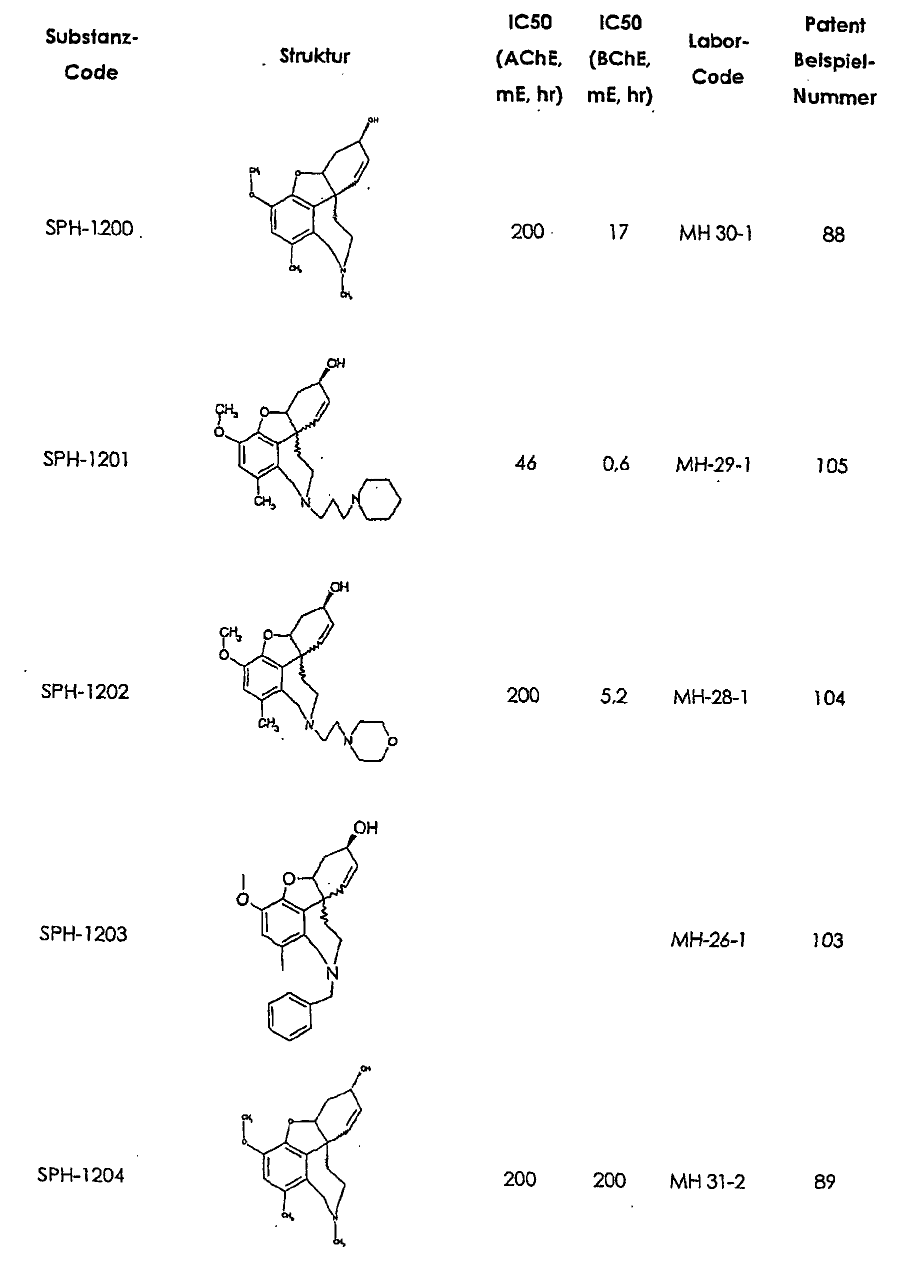

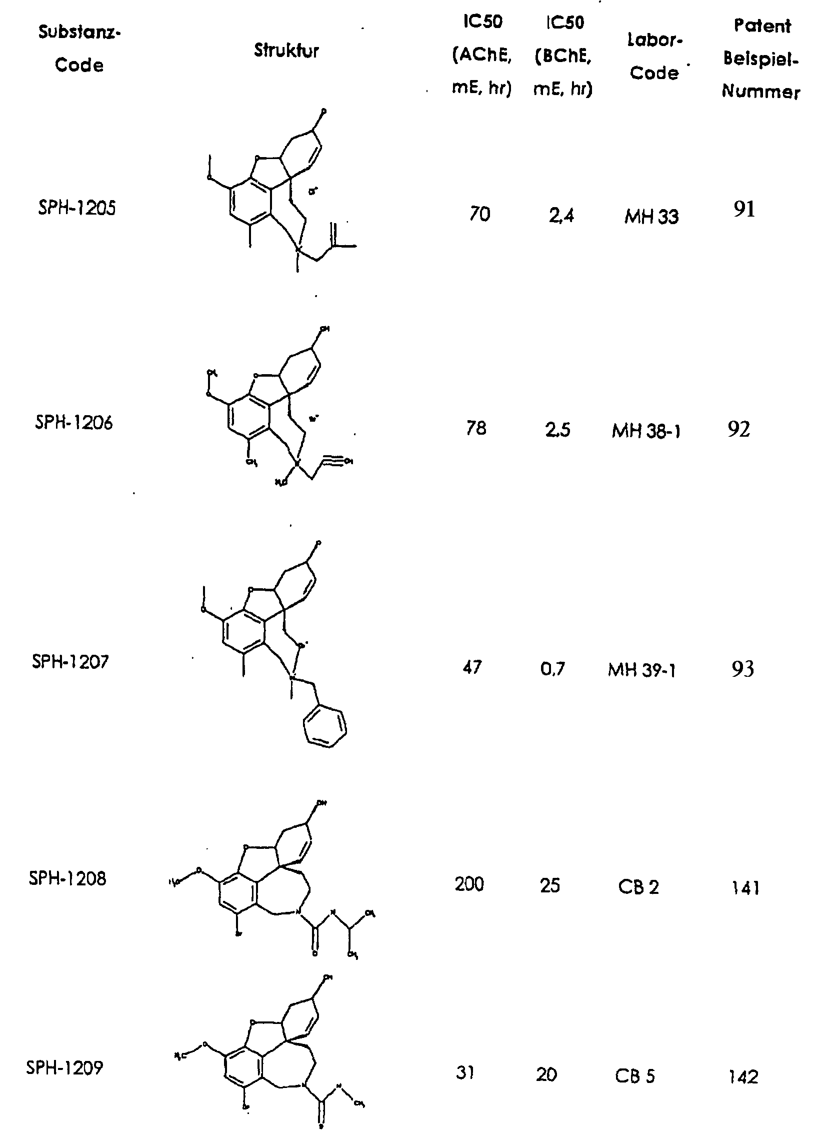

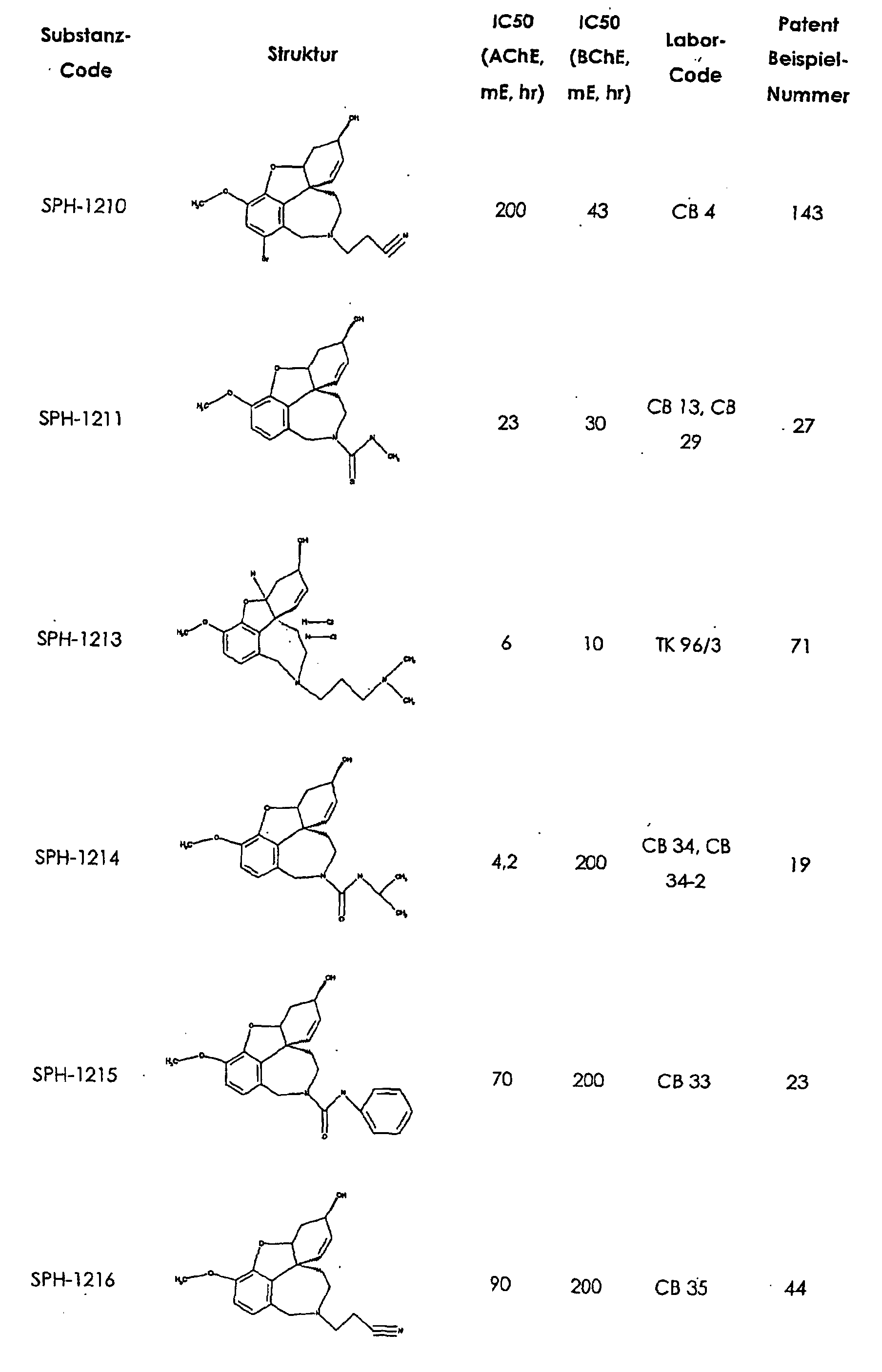

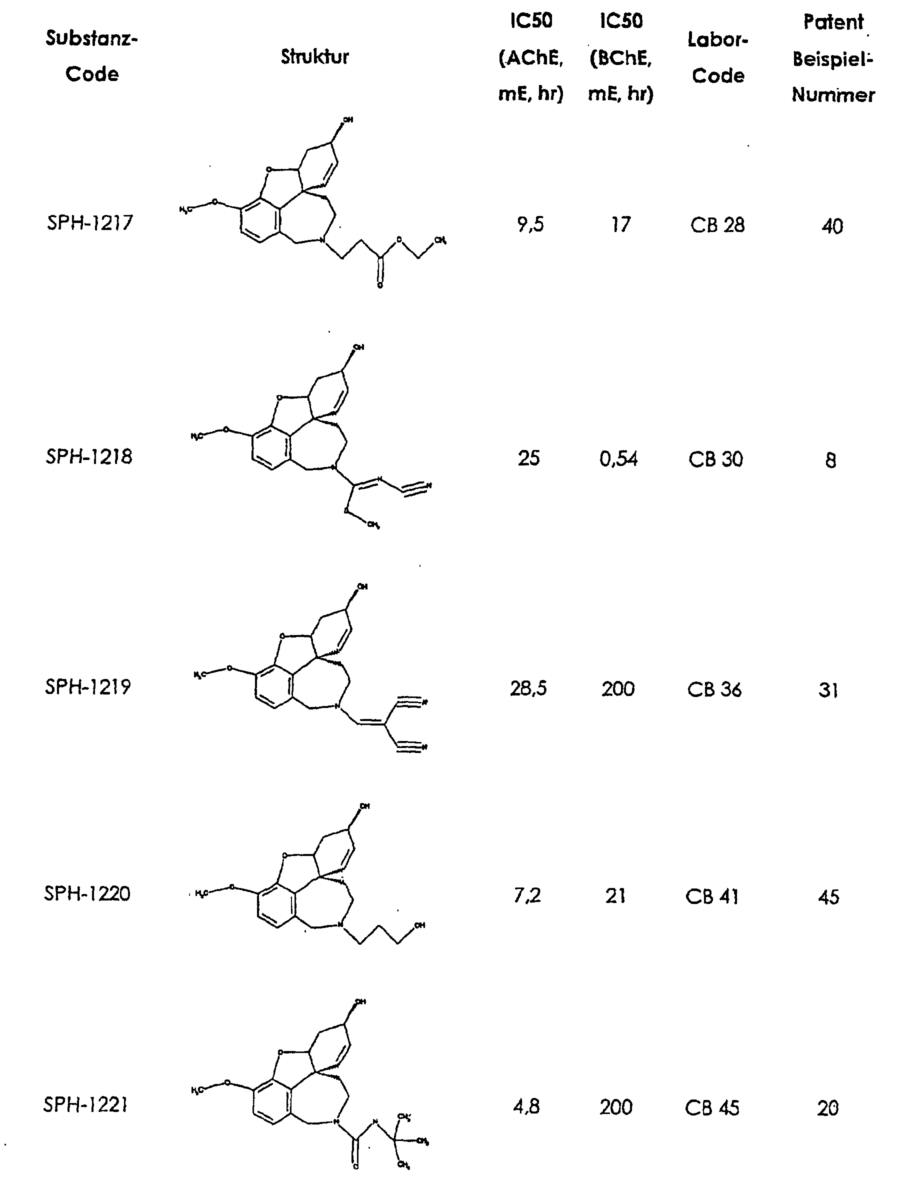

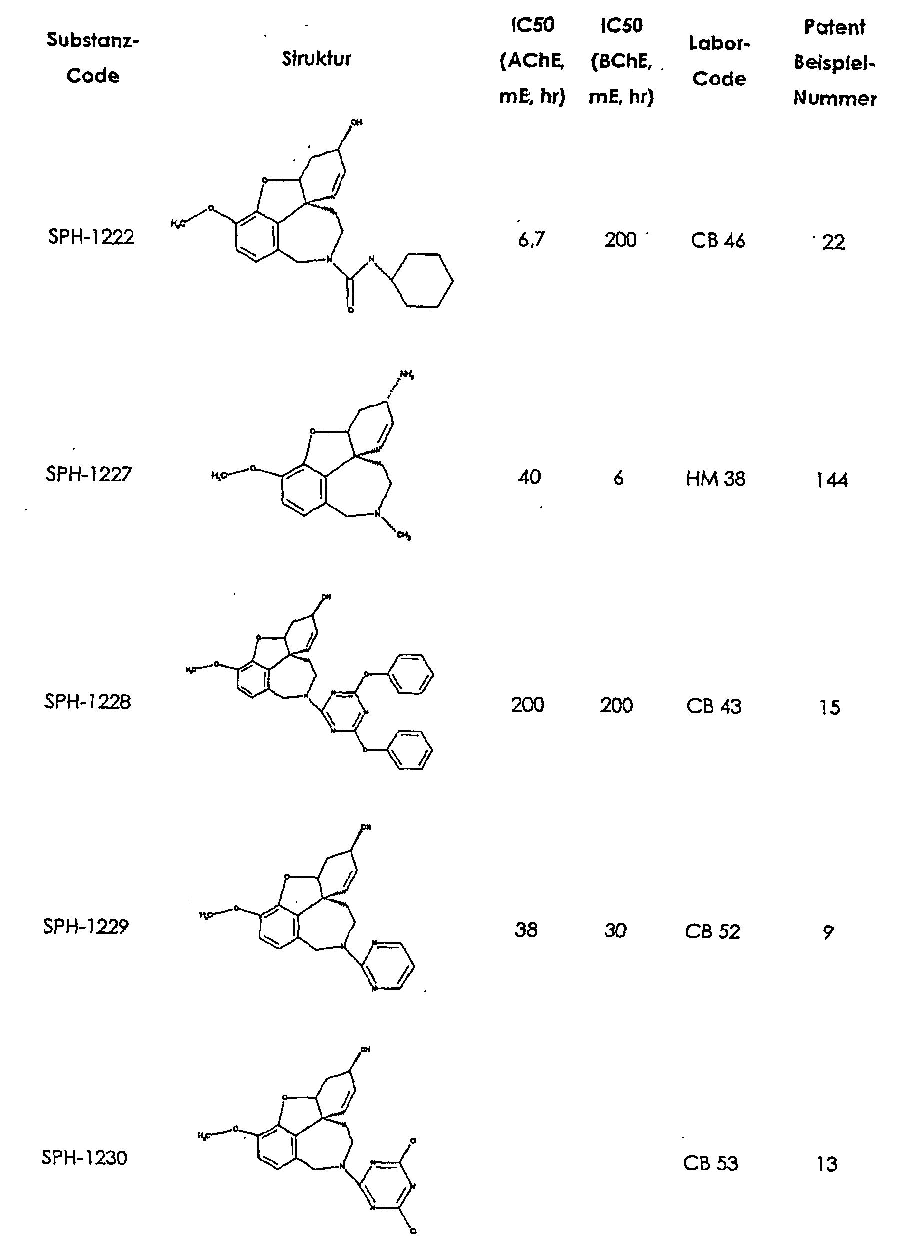

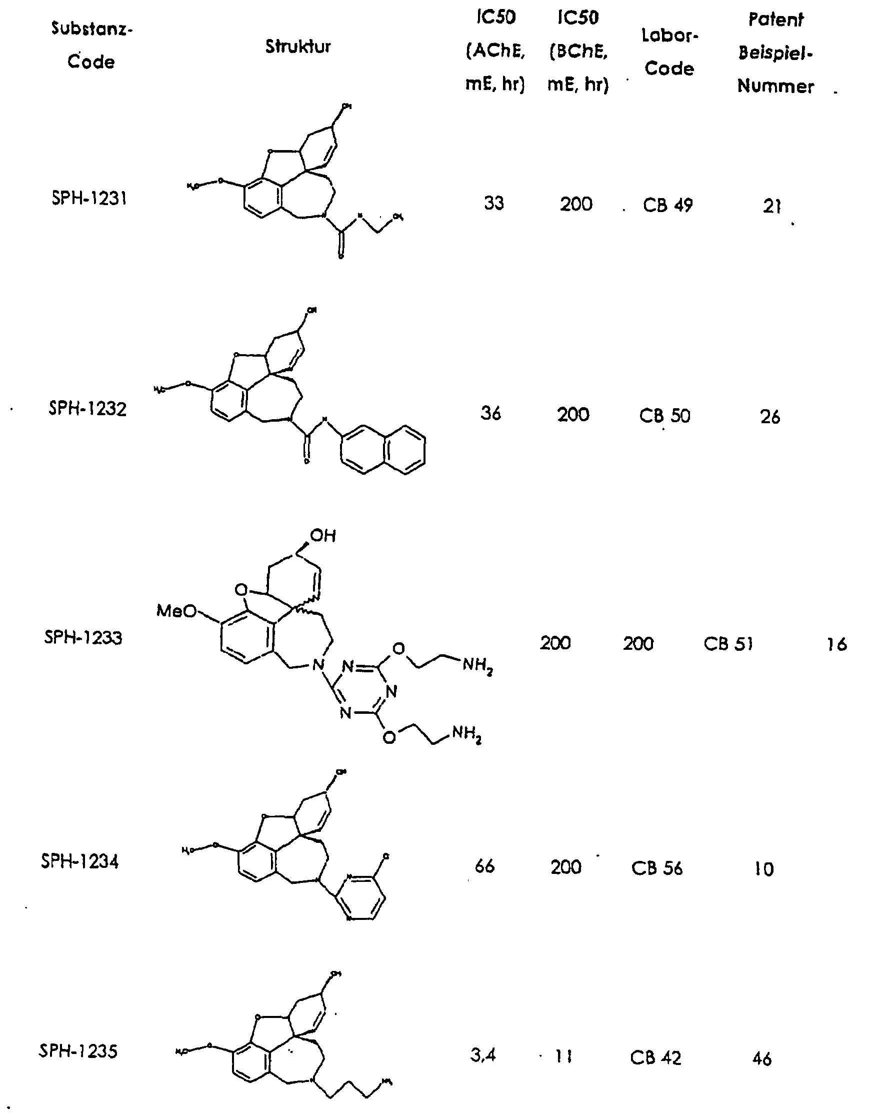

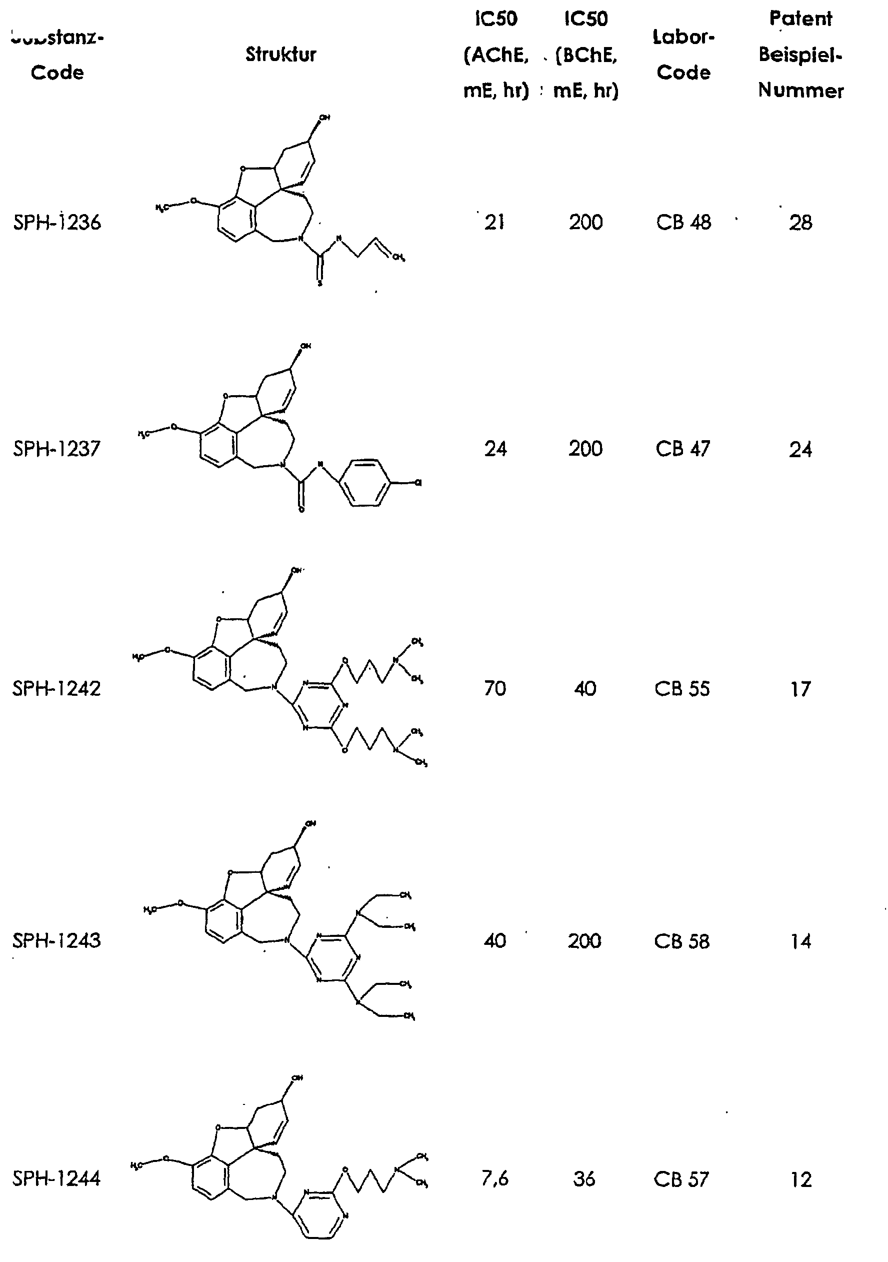

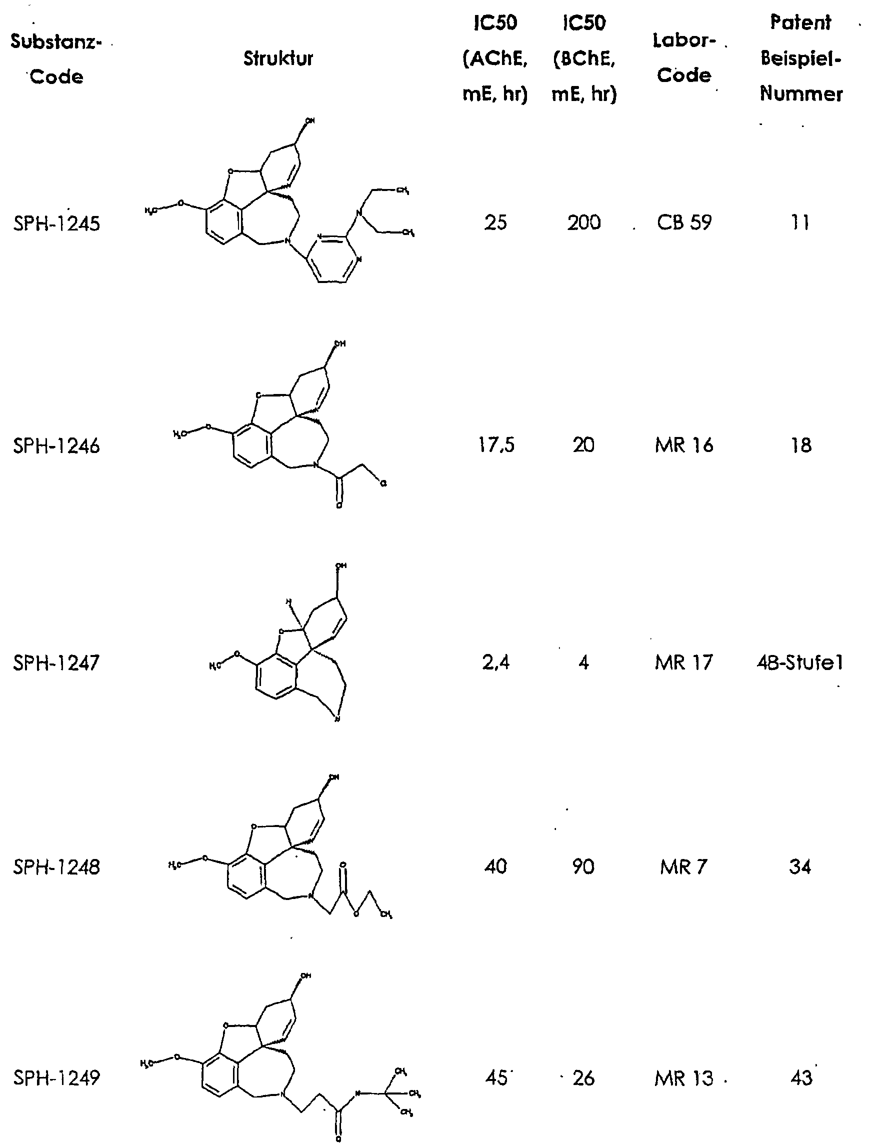

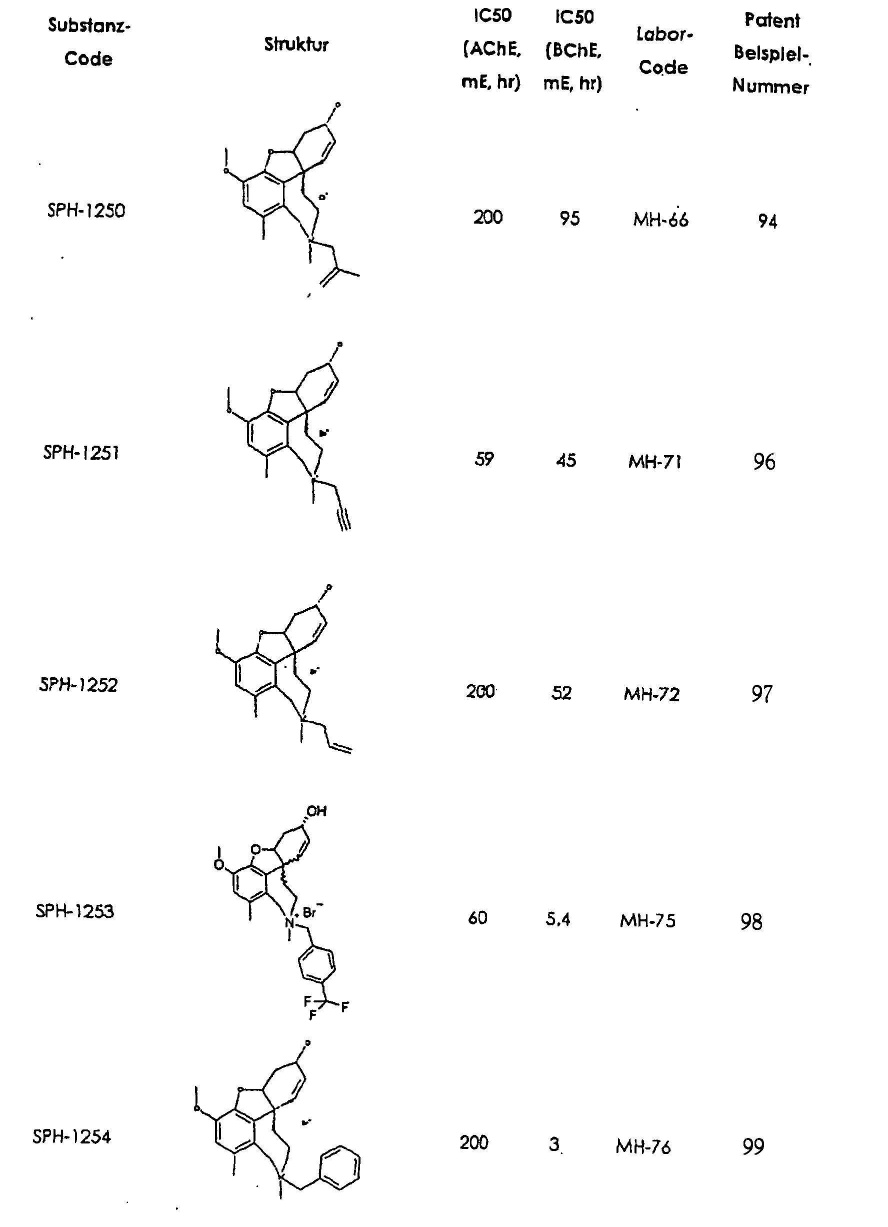

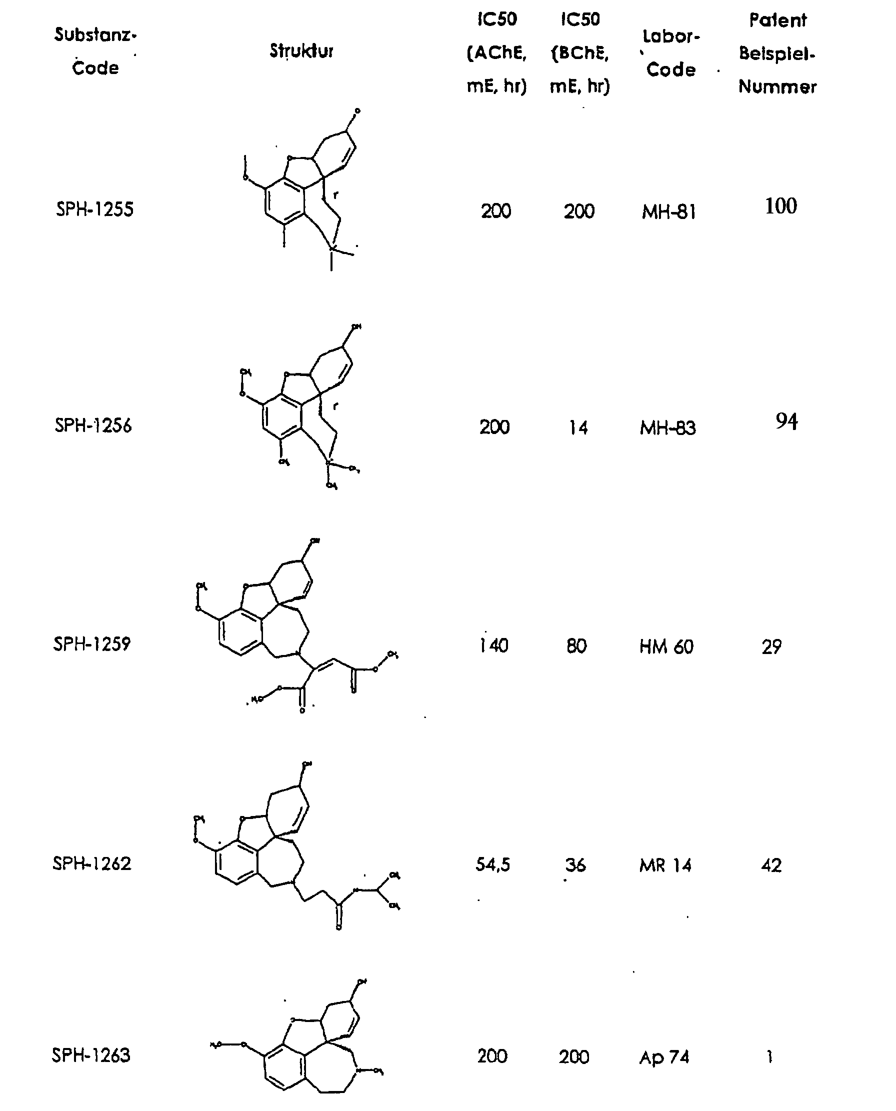

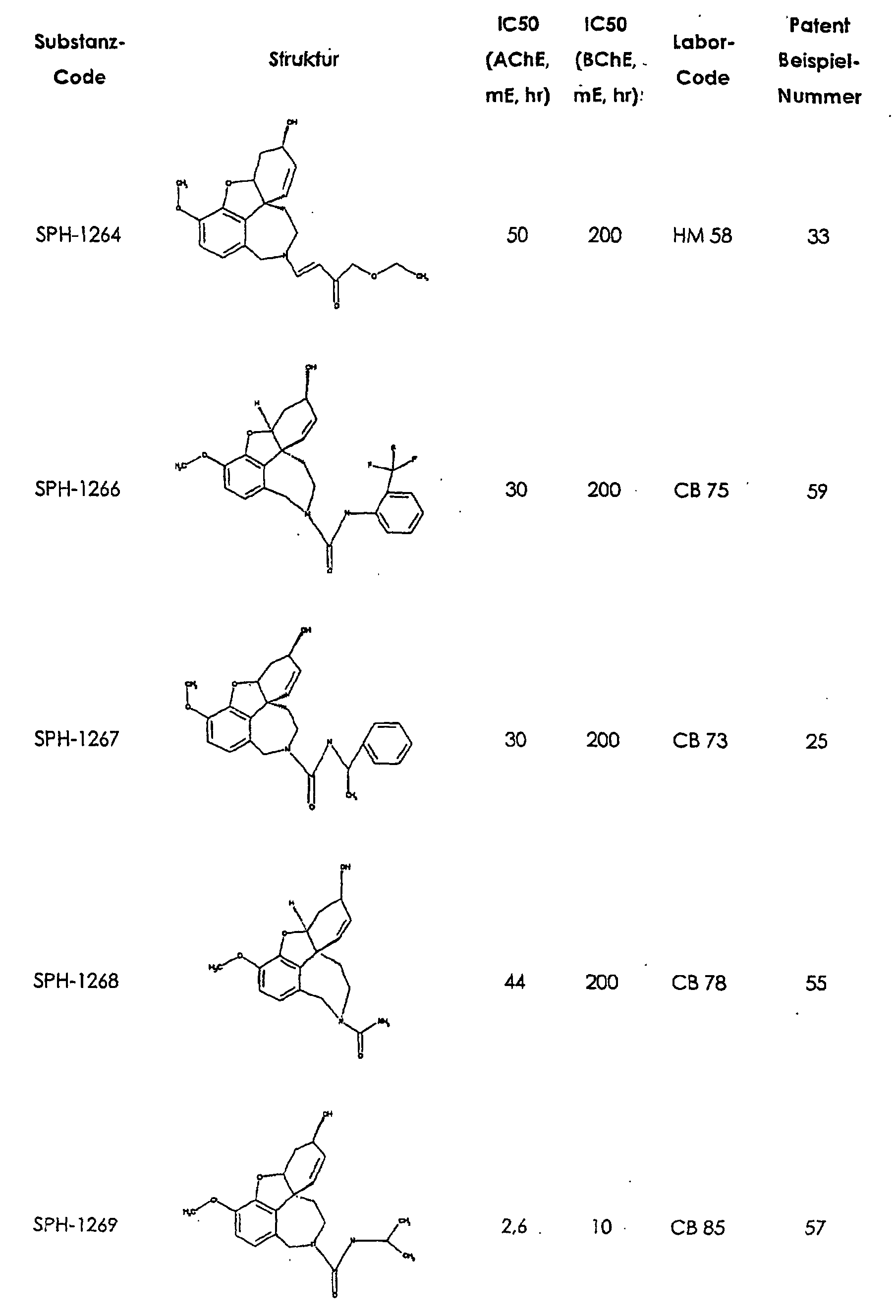

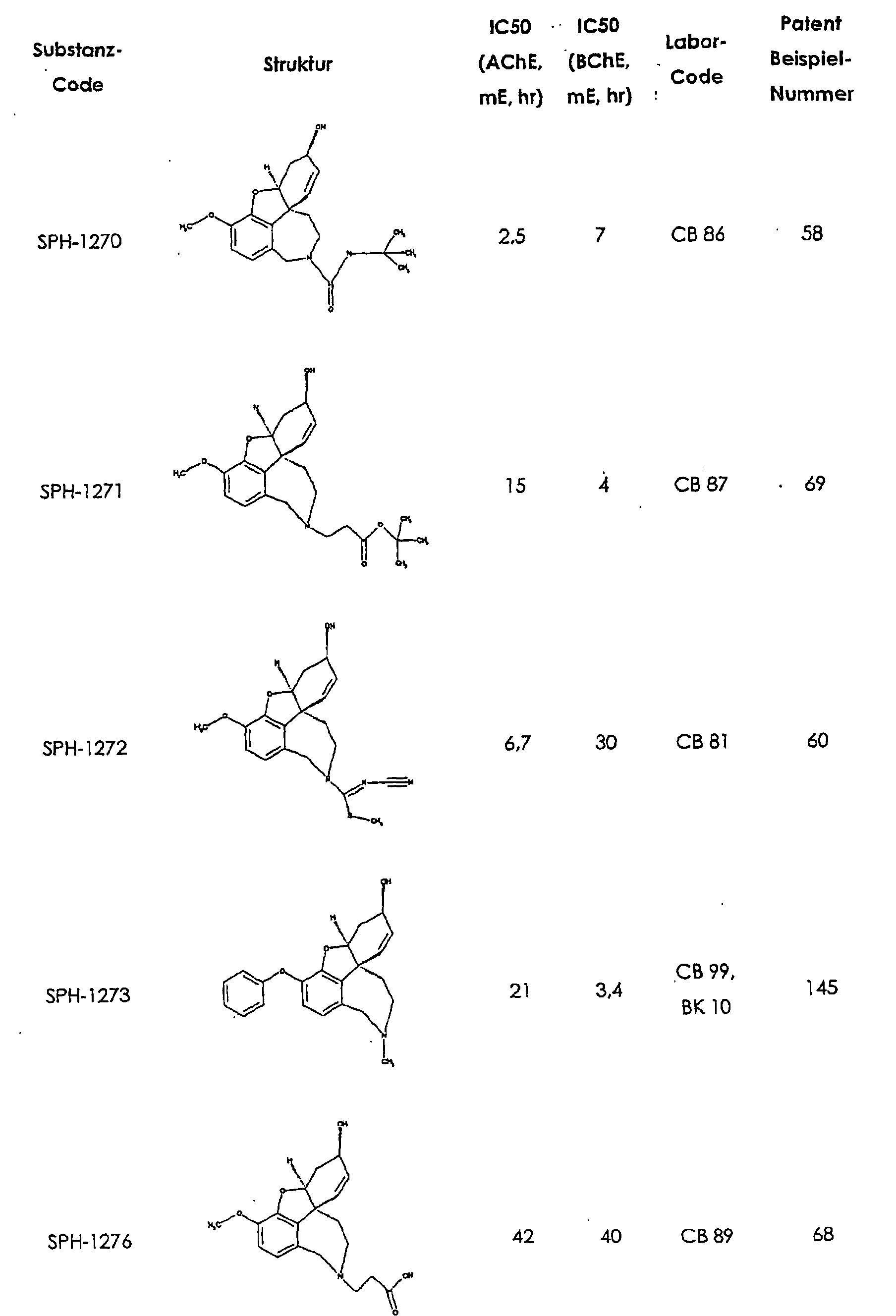

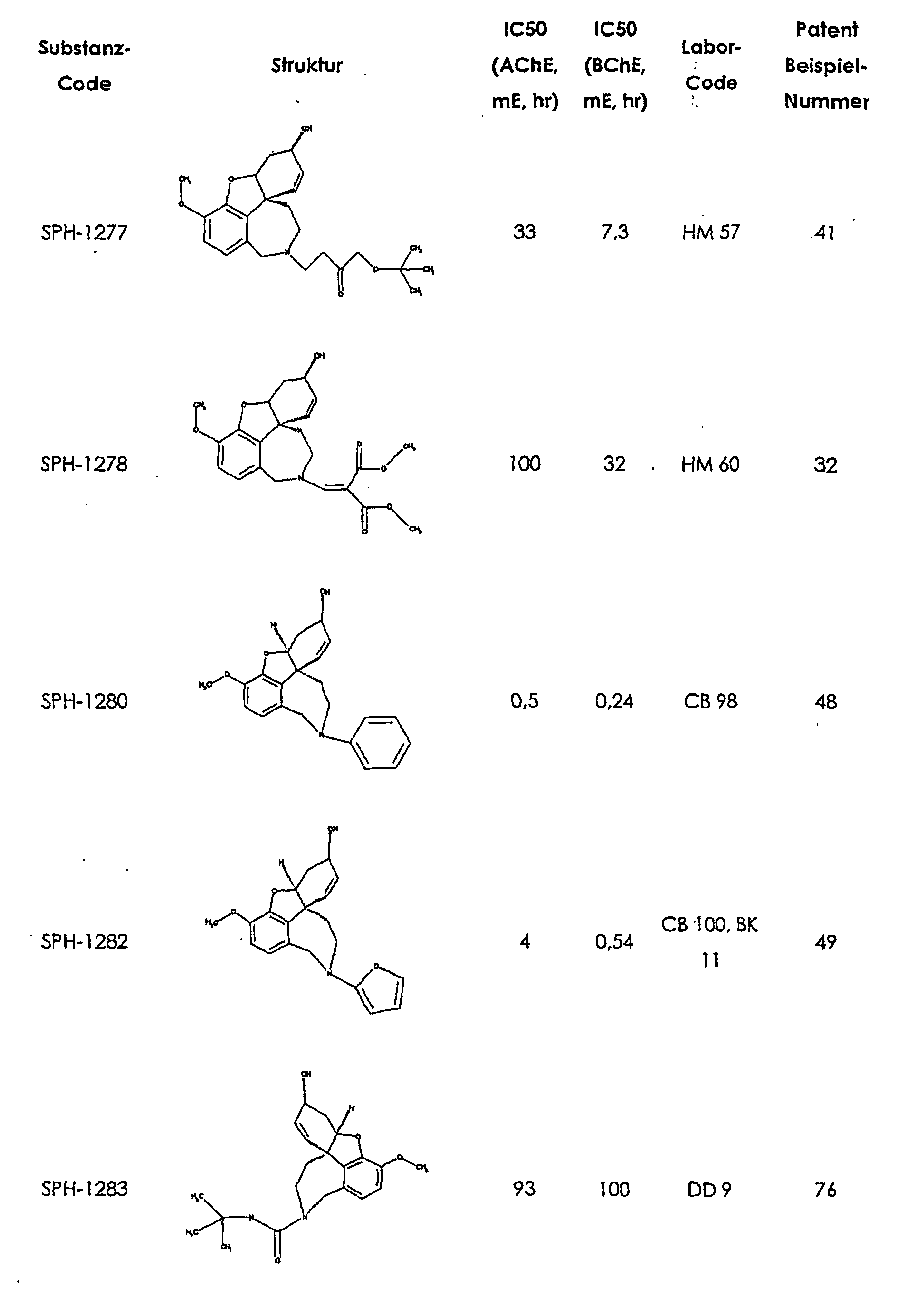

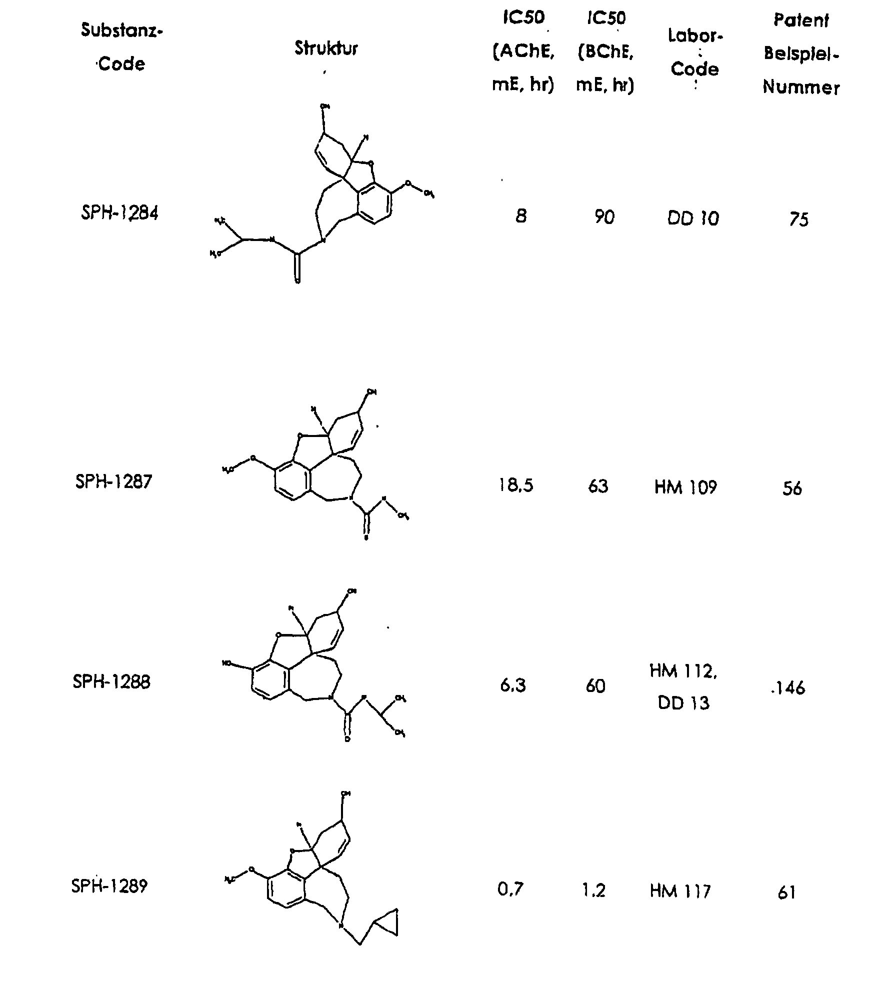

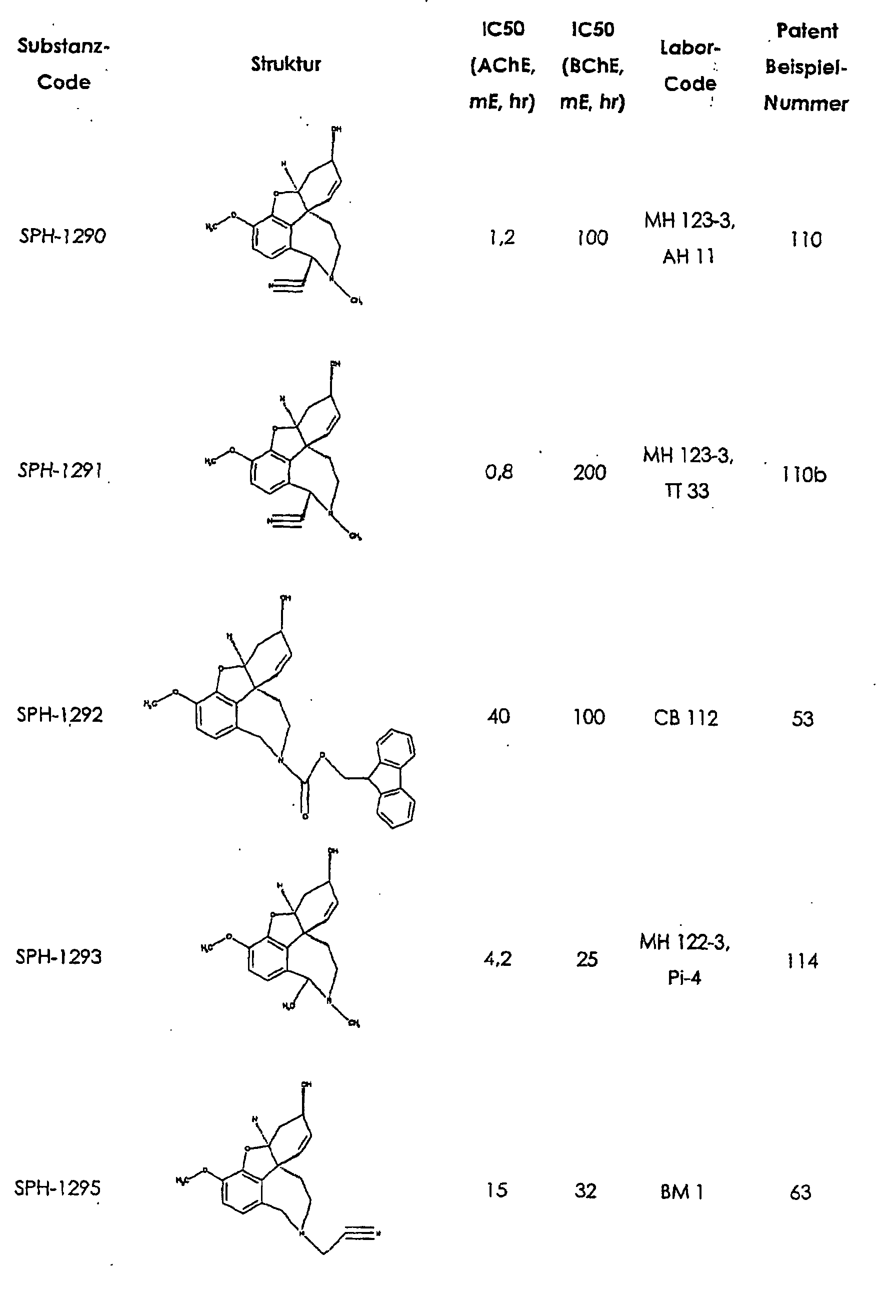

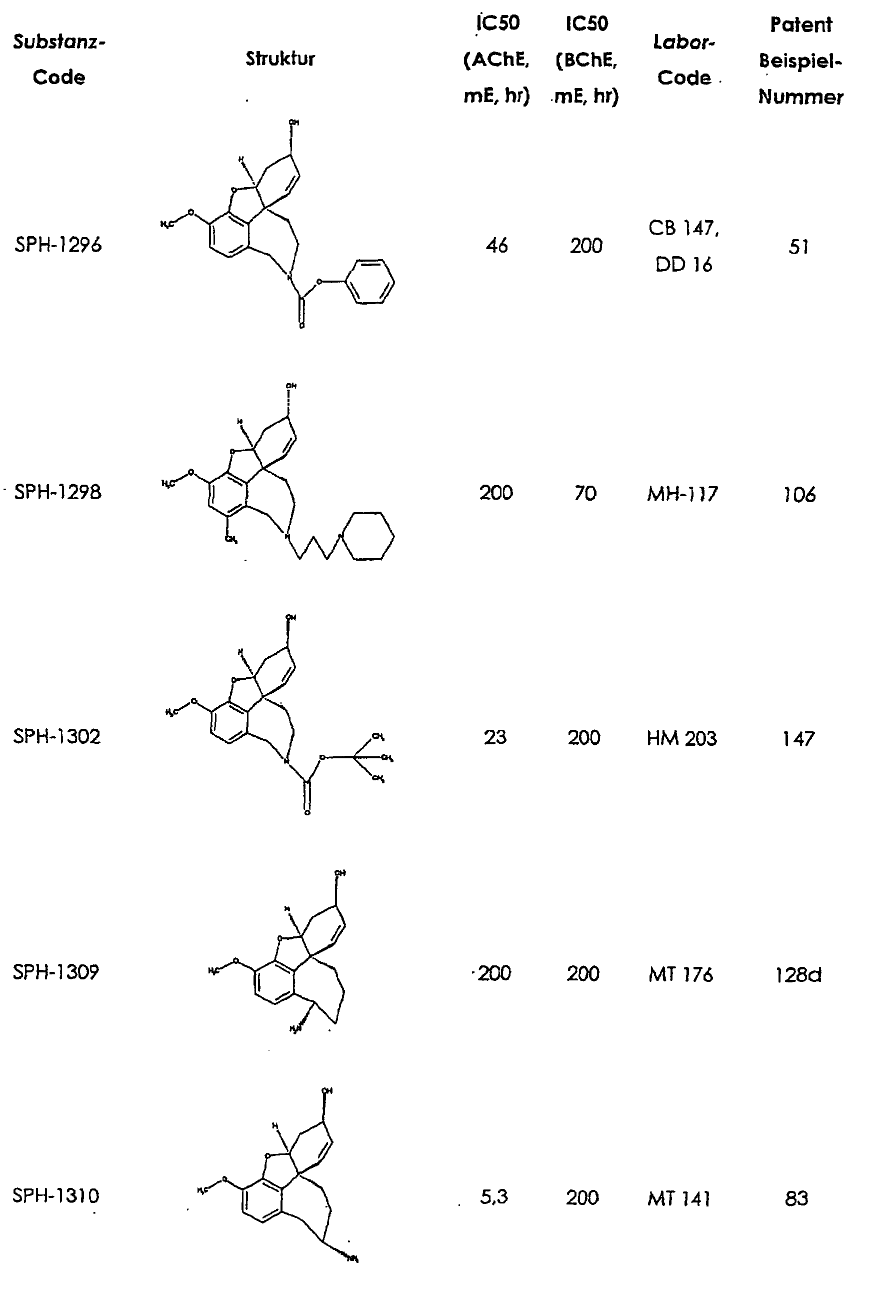

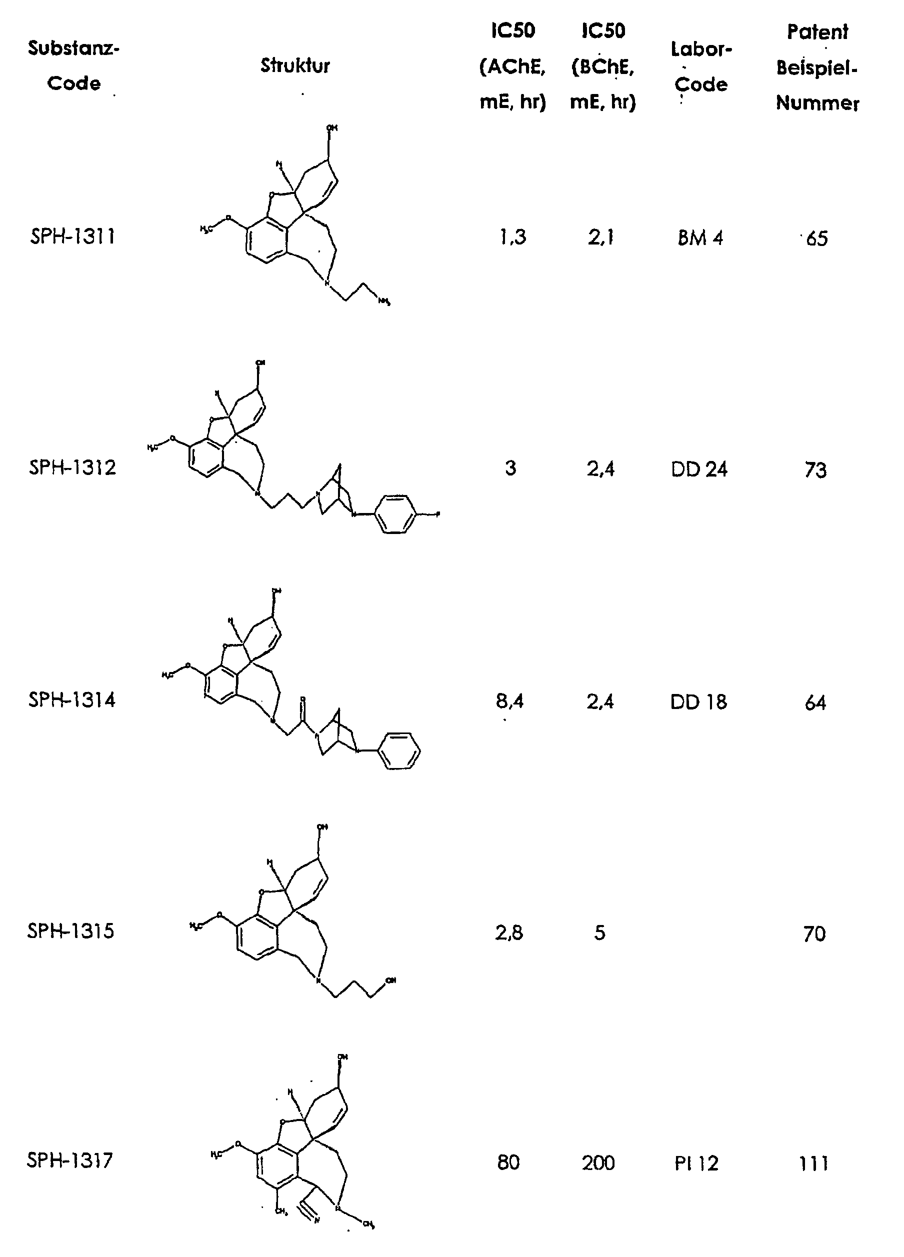

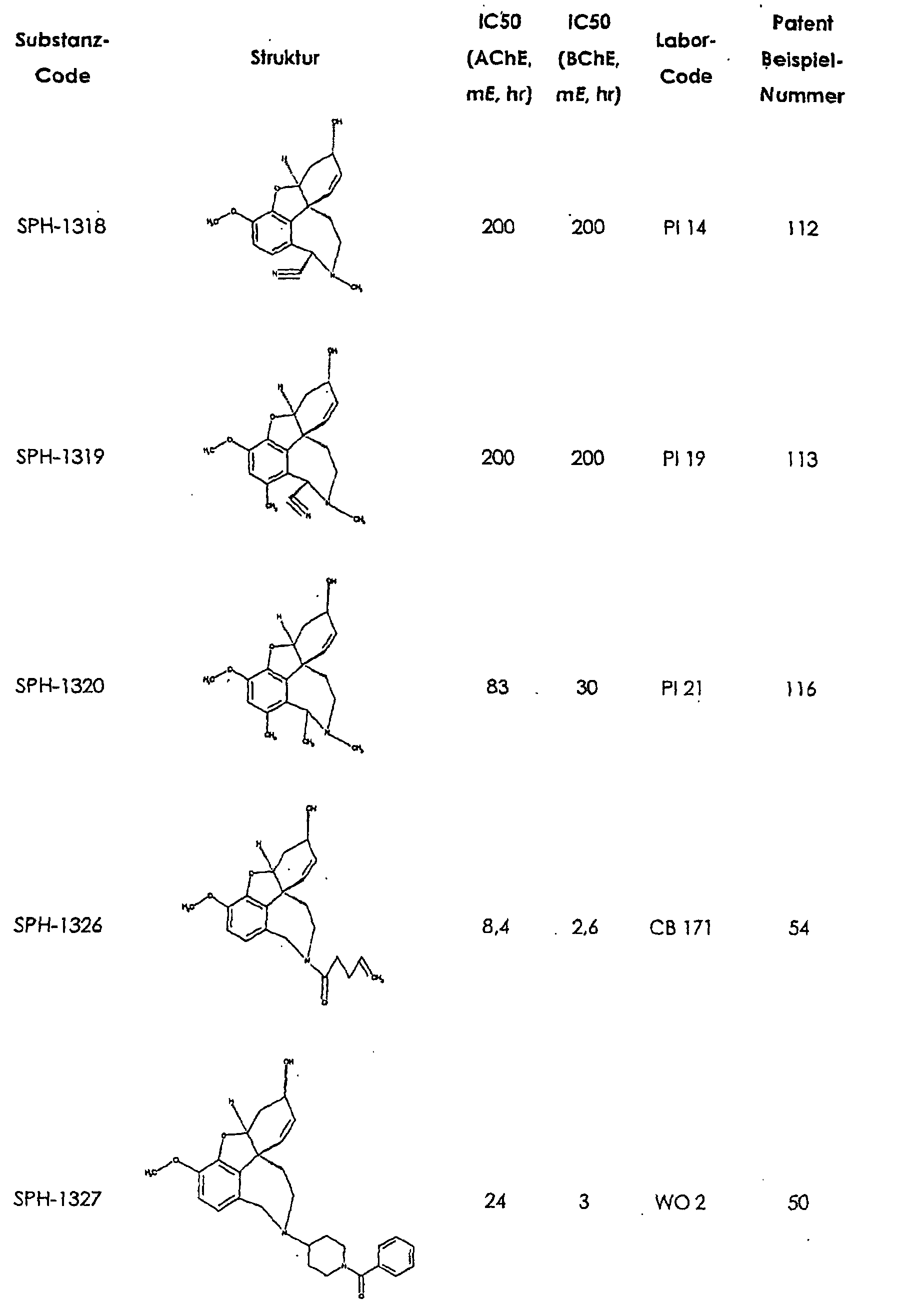

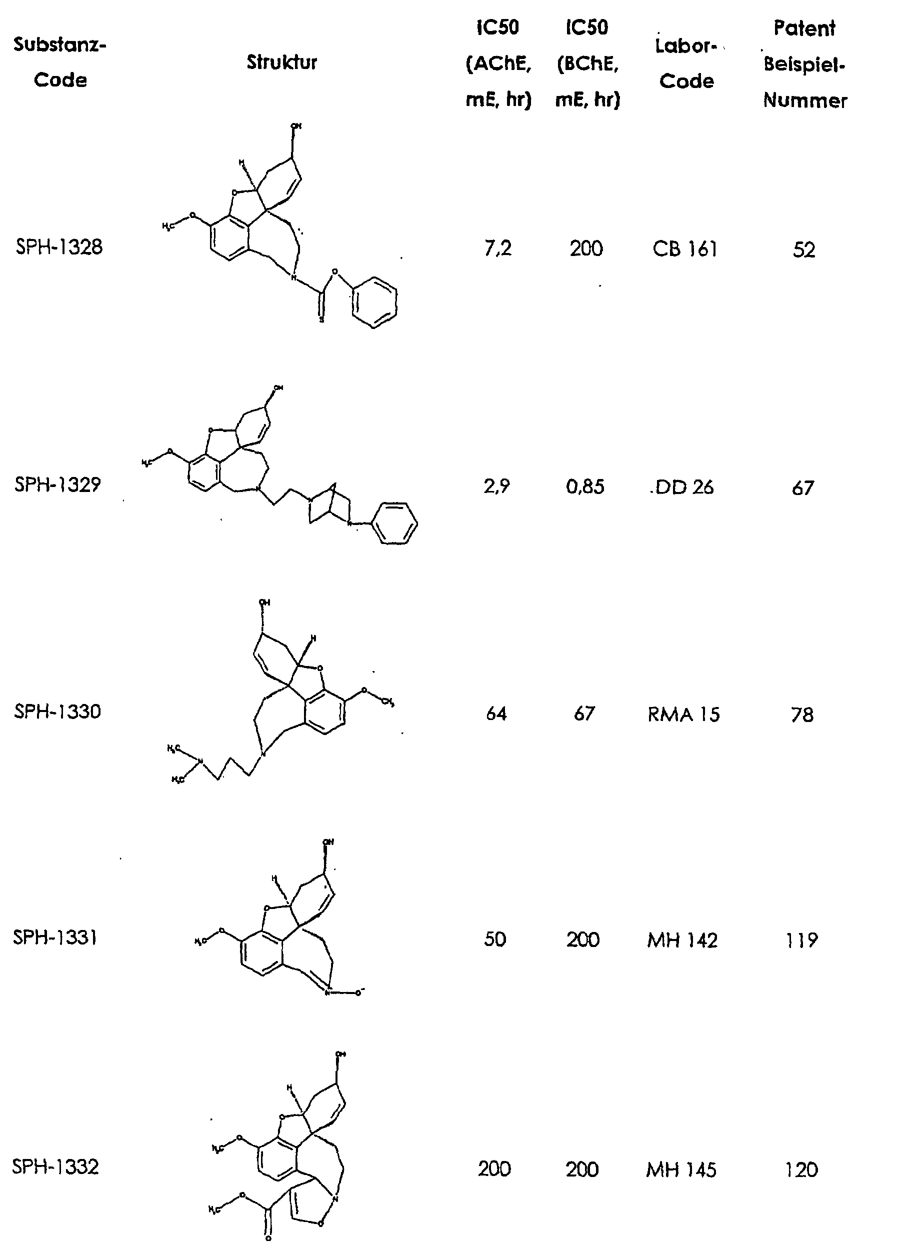

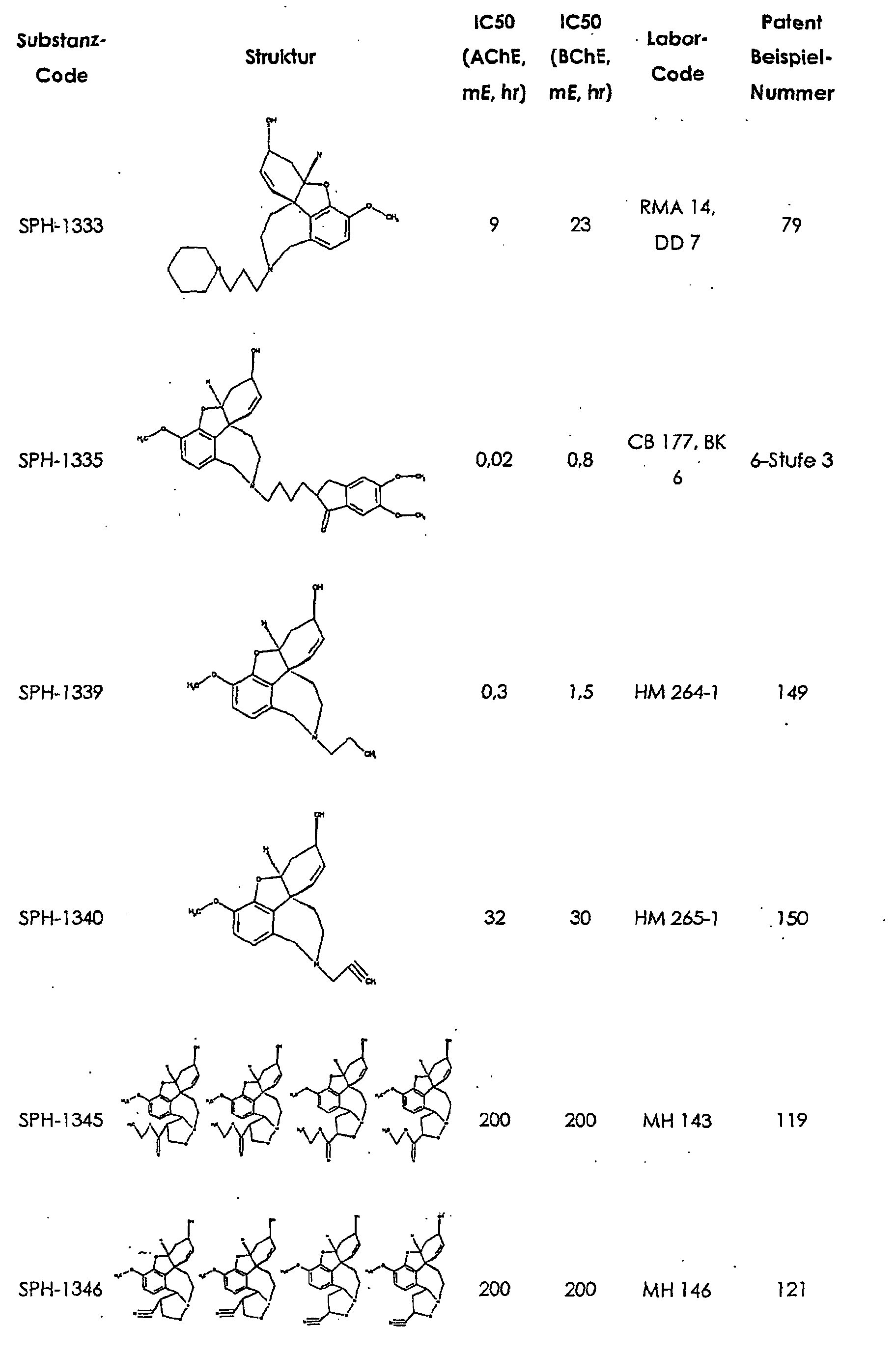

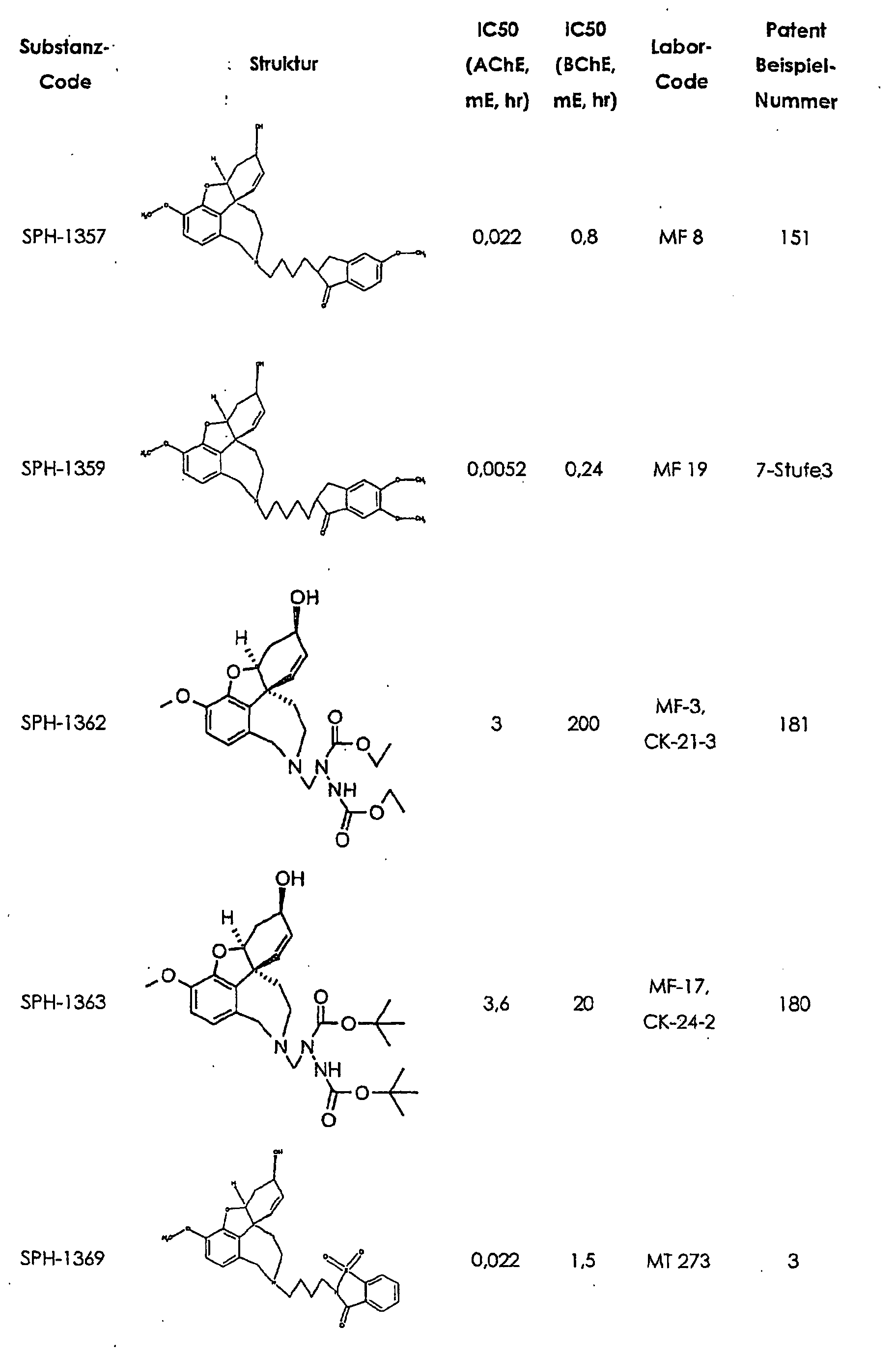

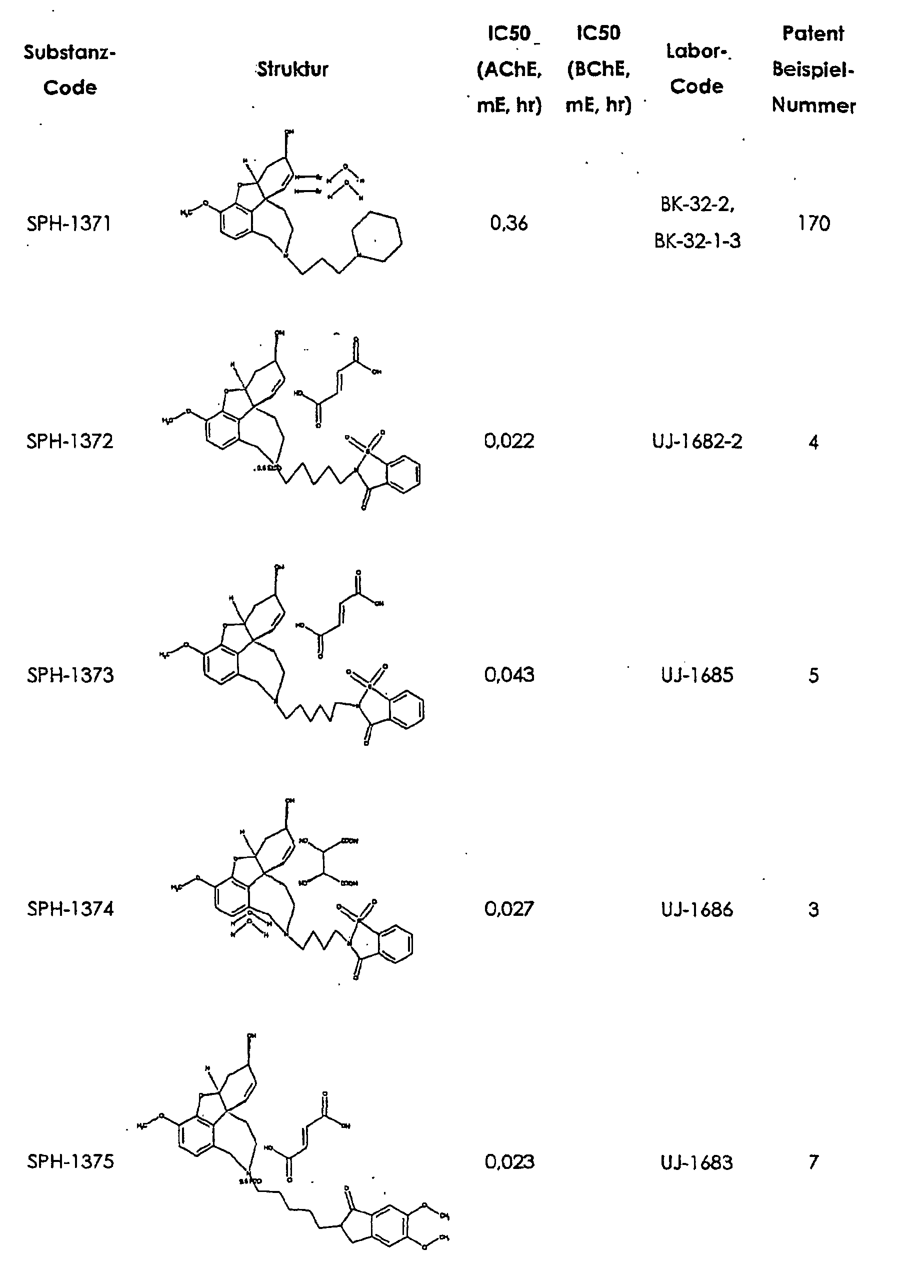

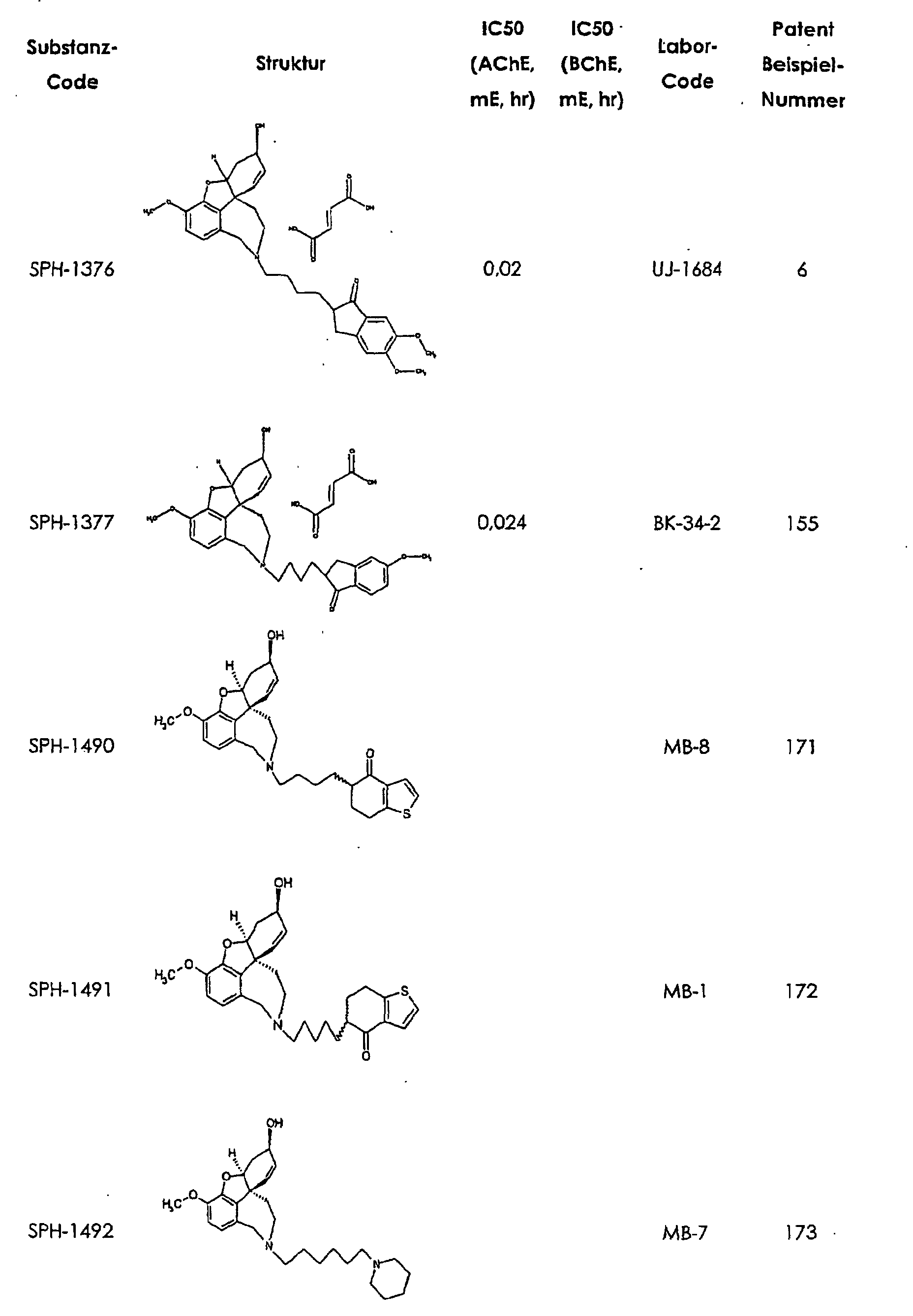

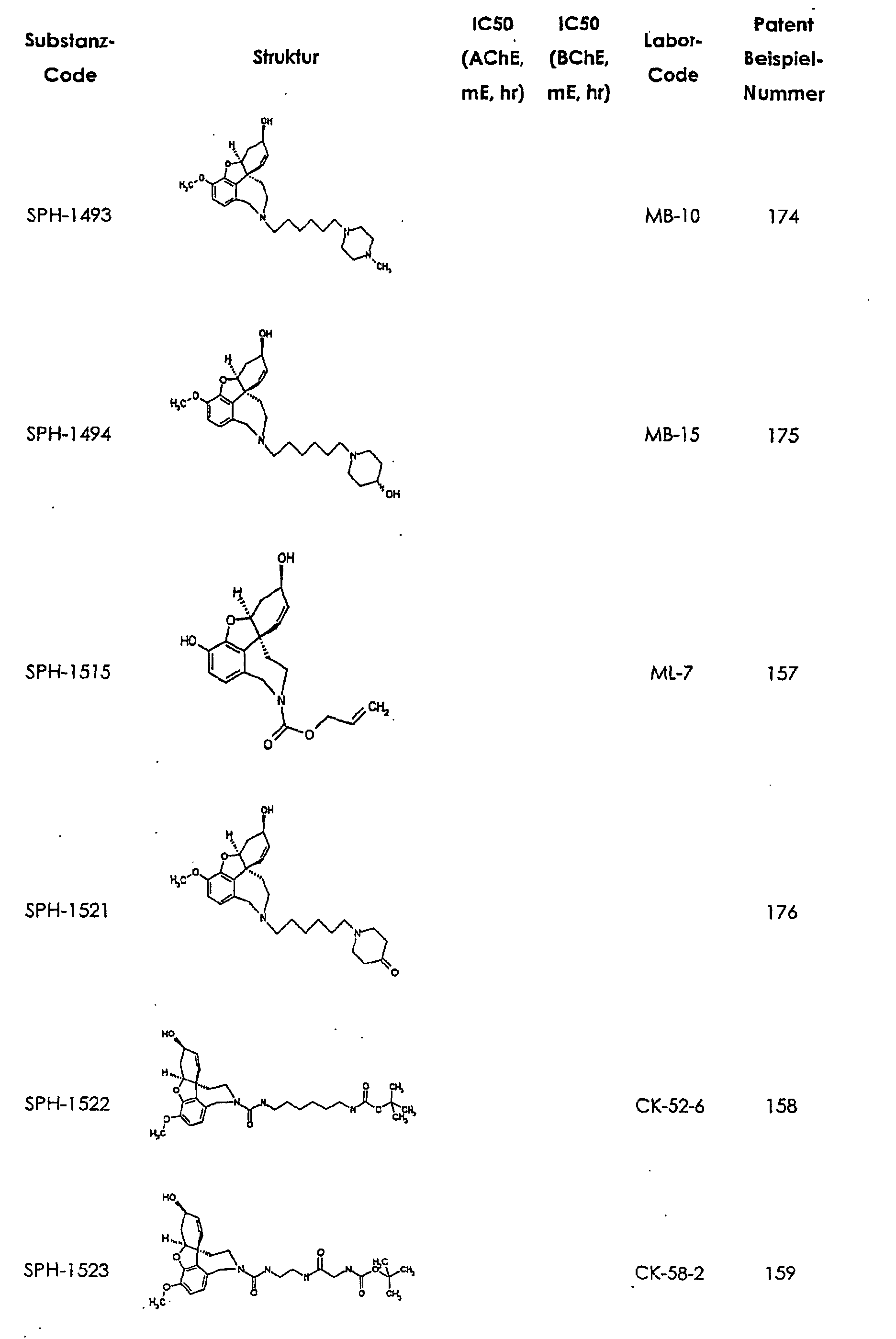

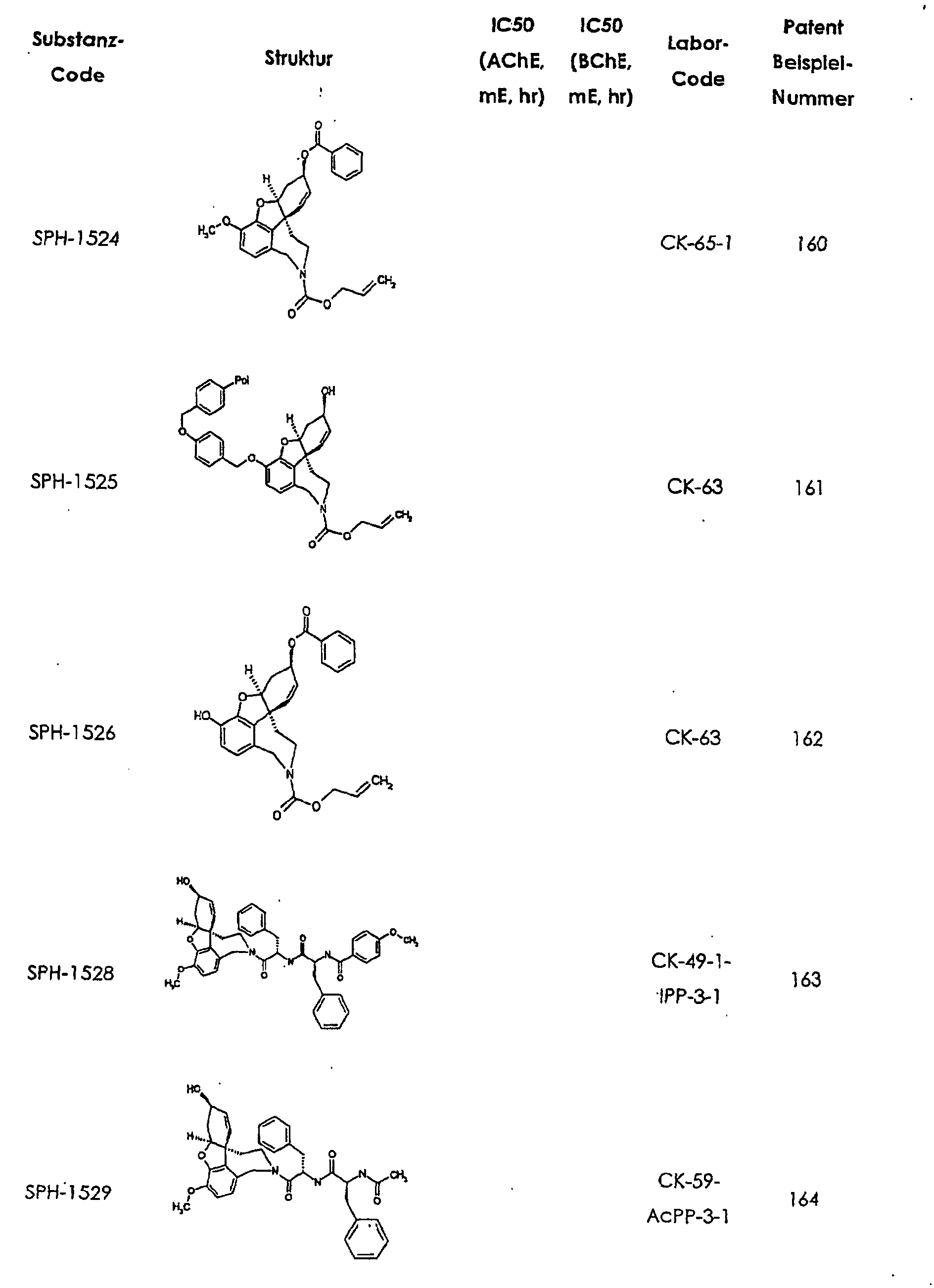

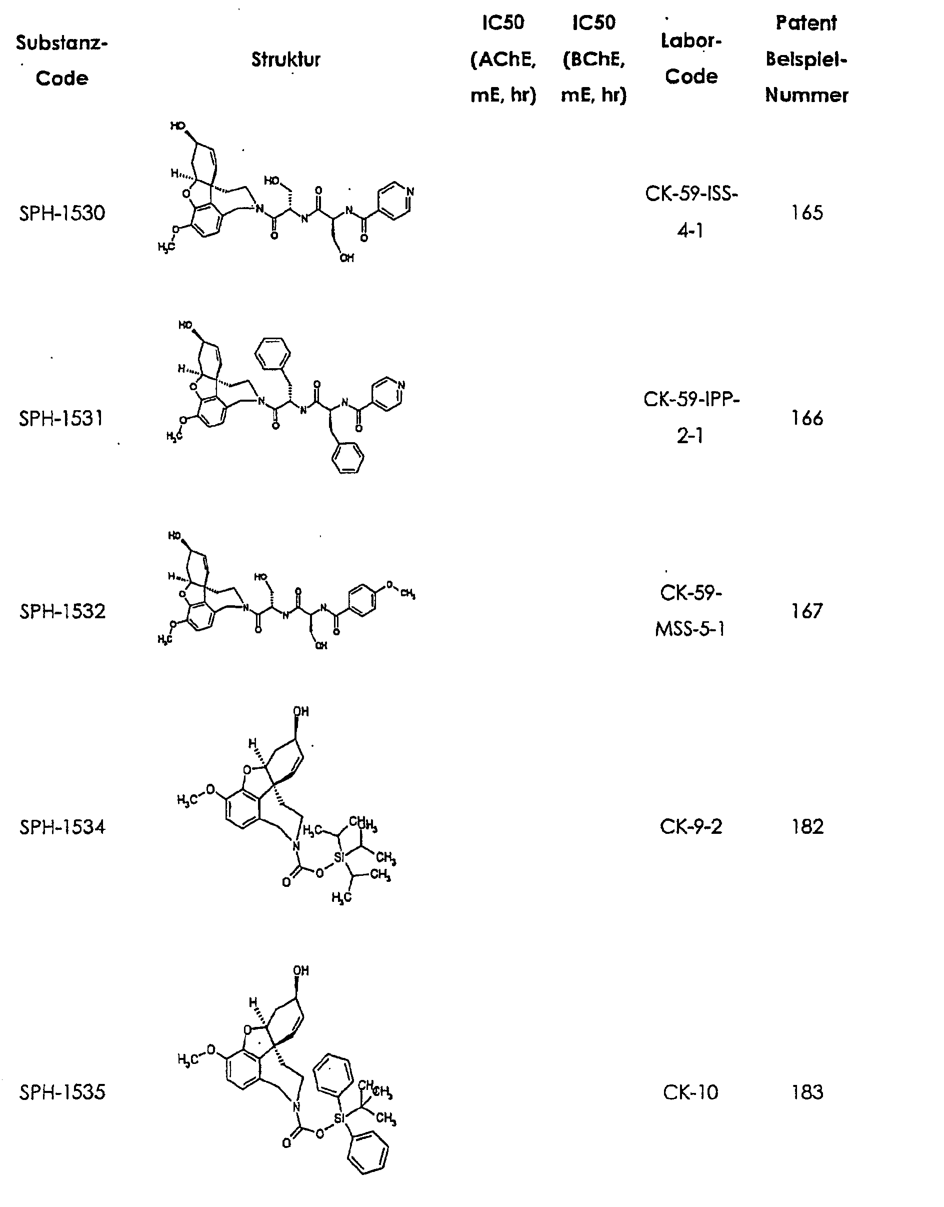

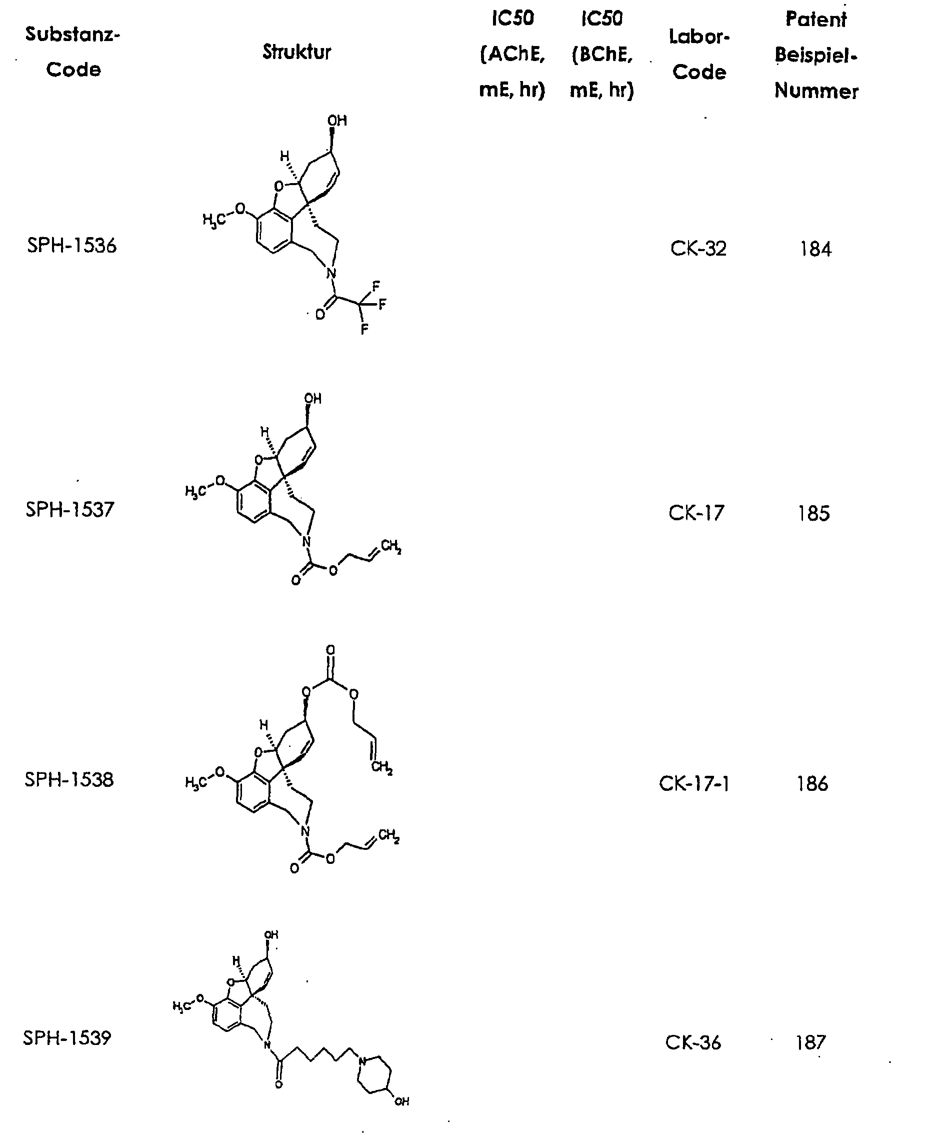

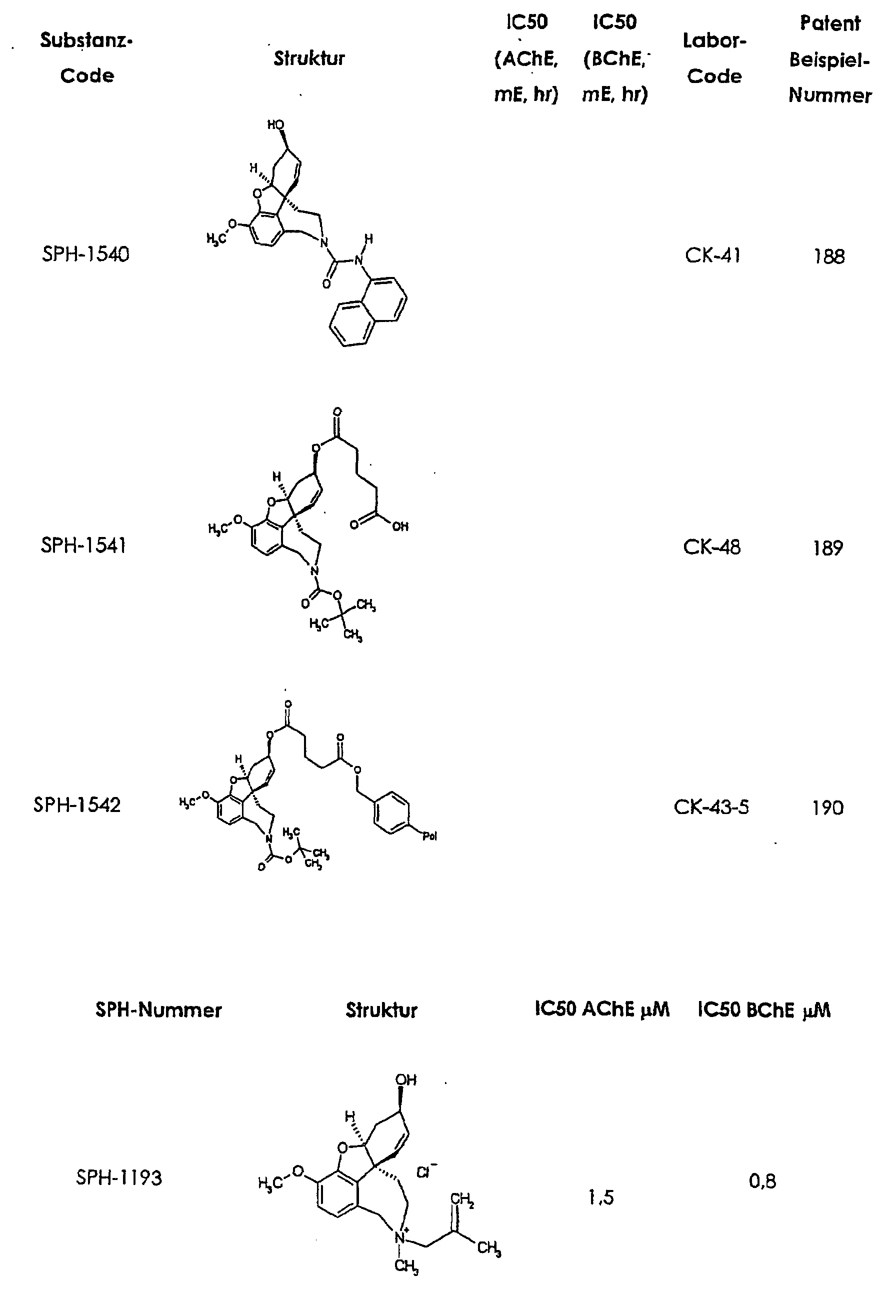

Im Rahmen der Erfindung ist unter anderem besonders in Betracht gezogen die Verbindung (6R)-3-Methoxy-5,6,9,10,11,12-hexahydro-4a[H1] benzofuro[3a,3,2-ef][2]benzazepin-6-ol (Norgalanthamin) und zwar racemisches Norgalanthamin, (-)Norgalanthamin und (+)Norgalanthamin. Sowohl racemisches Norgalanthamin als auch seine (+)- und (-)-Isomeren können in Arzneimitteln zur Behandlung der eingangs unter a) bis m) genannten Krankheiten als Wirkstoff für sich oder in Kombination mit anderen Wirkstoffen verwendet werden .

Die erfindungsgemäßen Verbindungen können unter sinngemäßer Anwendung der in der WO 96/12692 und in der WO 97/40049 beschriebenen Verfahren und Arbeitsweisen zum Herstellen von Galanthamin und Galanthamin-Derivaten synthetisiert werden.

Zusätzlich zu den vorgenannten Synthesewegen können einige der erfindungsgemäßen Verbindungen unter Anwendung der kombinatorischen (oder Parallel-)Synthesetechnologie hergestellt werden. Bei dieser Synthesemethode wird das interessierende Grundgerüst (oder Kernmolekül) auf einer festen Phase (z.B. Glaskügelchen, Polymerkügelchen oder einem anderen inerten Träger) immobilisiert, der das Abtrennen von überschüssigen Reaktionspartnem vom modifizierten Grundgerüst erleichtert. Die jeweils eingesetzte Festphase hängt von der Beladungskapazität, den eingesetzten Reaktanten und den Reaktionslösungsmitteln ab. Insbesondere in Betracht gezogen sind Polymerkügelchen, wie beispielsweise Merriefield-Harz, Wang-Harz oder TentaGel (Rapp)-Harz.

Das Immobilisieren des Grundgerüstes erfolgt durch eine funktionelle Gruppe, die unter geeigneten Reaktionsbedingungen in den letzten Schritten der Synthese wiedergewonnen werden kann. Der letzte Schritt besteht in der Spaltung des gewünschten Produktes von der Festphase. Die Wahl der Linkereinheit, welche das Grundmolekül an der Festphase kuppelt, hängt von der Kombination und/oder der Folge von Reaktanten und den Reaktionsbedingungen ab, die erforderlich sind, um maximale Ausbeuten und/oder Reinheit zu erzielen. Überdies können mit unterschiedlichen Linkem die Produkte von der gleichen Festphase unter unterschiedlichen Bedingungen abgespaltet werden. Diese Technologie erlaubt eine rasche Synthese einschließlich automatisierter Synthesen von Verbindungen gemäß der Erfindung.

Bezüglich der kombinatorischen und/oder Parallelsynthese wird auf die nachstehenden Literaturstellen verwiesen, in denen allgemeine Verfahrensbeschreibungen enthalten sind:
1) Abelson, J.N., Combinatorial Chemistry. Academic Press, San Diego (1996).
2) Epton, R., Innovation and Perspectives in Solid Phase Synthesis and Combinatorial Libraries, Mayflower Scientific Limited, Birmingham (1996).
3) Wilson, S.R. and Czamik, A.W., Combinatorial Chemistry. Synthesis and Applications. John Wiley & Sons, Inc., New York (1997).
4) Gordon, E.M. and Kerwin, J.F.J., Combinatorial Chemistry and Molecular Diversity in Drug Discovery. John Wiley and Sons, Inc., New York (1998).
5) Thompson, L.A., Ellman, J.A. Chem. Rev. 96, 555 (1996).
6) Special issue on combinatorial chemistry, cf., Acc. Chem. Res., 29, 111 (1996).
7) Fruchtel, J.S.; Jung, G.Angew. Chem. Int.Ed.Engl. 35, 17 (1996).
8) Cheng, S.; Comer, D.D.; Williams, J.P.; Myers, P.L.; Boger, D.L.J.Am.Chem.Soc., 118, 2567 (1996).
9) Für weitere Information zu diesem sich rasch entwickelnden Gebiet siehe: A dynamic database of references in molecular diversity at http://www:5z.com.
10) Bayer E.; Angew Chem. Int. Ed., 30, 113-129 (1991).
11) Mayer, J.P.; Zhang, J.; Bjergarde, K.; Lentz, D.M.; Gaudino, J.J.; Tetrahedron Letters, 37,8081 (1996).
12) Bayer, E.; Angew.Chem.Int.Ed., 30,113-129 (1991).
13) DE 19745628 A1.

Am Beispiel eines Norgalanthamingerüstes (G₁ = G₂ = G₃ = Methylen; W = NH) oder "Homogalanthamin (G₁ = G₂ = G₃ = Methylen; W = CH-NH₂) kann eine Bindung zwischen dem Molekül und der Festphase entweder über ein Kohlenstoffzentrum (C-linked), ein Stickstoffzentrum (N-linked), oder ein Sauerstoffzentrum (O-linked) erzielt werden. Die Anknüpfungspunkte hängen von der Art der erwünschten Strukturmodifikation ab. In den nachstehend genannten, beispielhaften Reaktionsschemata sind verschiedene Transformationen von an verschiedenen an Festphasen, durch Linker geknüpften Grundgerüsten wiedergegeben.

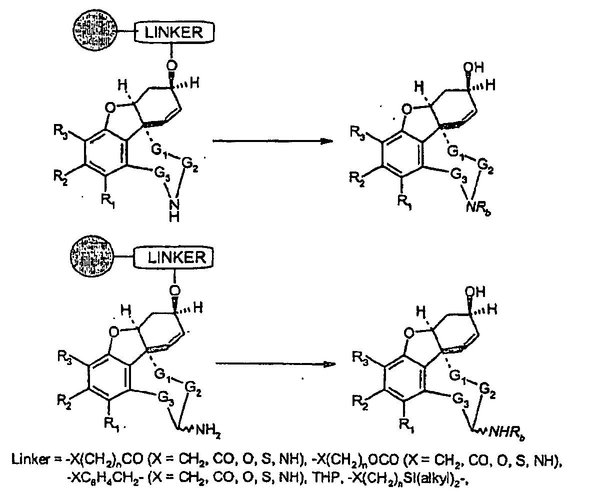

O-Linker Transformationen von Gerüsten des Norgalanthamin-Typs und des "Homogalanthamin"-Typs

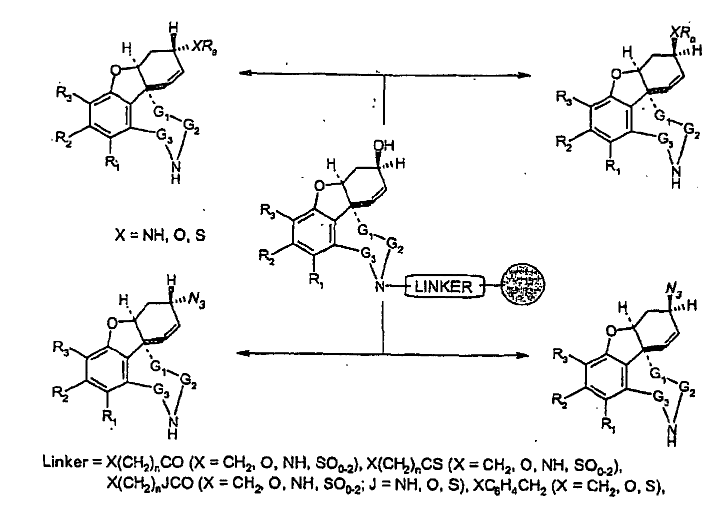

N-Linker Transformationen eines Molekülgerüsts des Norgalanthamin-Typs.

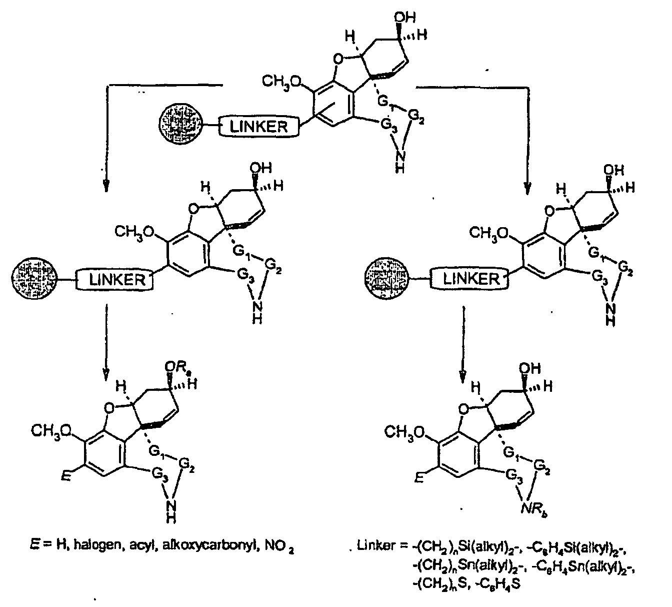

C-Linker Transformationen eines Gerüstes des Norgalanthamin-Typs

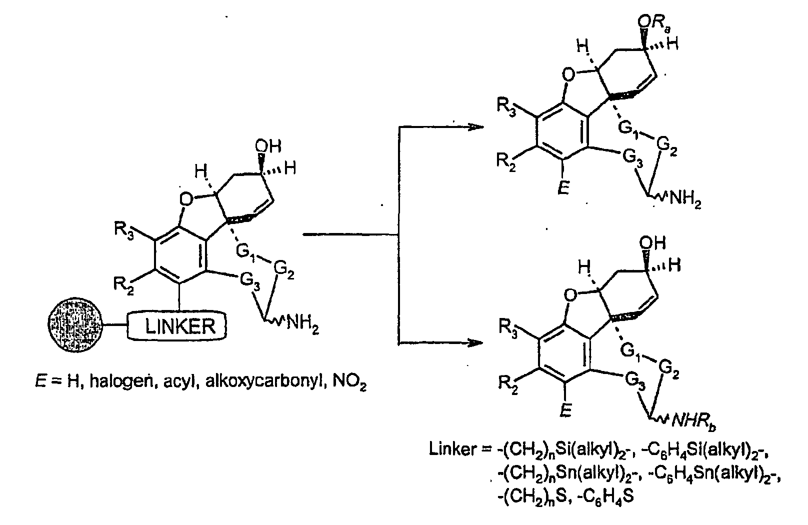

C-Linker Transformationen des "Homogalanthamingerüsts"

Die Verbindungen gemäß der Erfindung, sowie von pharmazeutisch annehmbaren Säureadditionssalzen derselben können als Wirkstoffe in Arzneimitteln beispielsweise zum Behandeln von Erkrankungen mit apoptotischer Komponente verwendet werden.

Neurodegenerative Erkrankungen des menschlichen Nervensystems gehören zu denjenigen Syndromen, für die derzeit keine oder nur ungenügende ursächliche Behandlungsmethoden zur Verfügung stehen. Unter den neurologischen Krankheiten dieser Art mit chronischem Verlauf werden in erster Linie die folgenden verstanden:
- Primär degenerative Demenzen (vor allem die Alzheimer'sche Krankheit),
- Zerebrale und spinale Lähmungen (amyotrophe laterale Sklerose, multiple Sklerose),
- Zentral bedingte Bewegungsstörungen (Parkinson'sche und Huntington'sche Krankheit) und
- Erkrankungen des epileptischen Formenkreises.

Neurodegeneration spielt jedoch auch im unmittelbaren Gefolge neurologischer Akutfälle eine Rolle, unter denen in erster Linie die folgenden zu nennen sind:
- Ischämischer Schlaganfall (Verschluß einer das Gehirn versorgenden Arterie),
- Haemorrhagischer Schlaganfall (innere Hirnblutung),
- Schädel-Hirn-Trauma und
- Hirnschäden nach Herzversagen bzw. Atemstillstand (Hypoxie/Anoxie).

Die Verbindungen der Erfindung sowie pharmazeutisch annehmbare Säureadditionssalze derselben können Wirkstoffe von Arzneimitteln zur Behandlung von neurodegenerativen Prozessen dienen, wobei insbesondere nicht vorrangig auf eine Verbesserung der akuten Symptomatik, sondern auf eine Verlangsamung und Modifizierung der damit verbundenen Prozesse abgezielt wird.

Im Rahmen des Diabetes mellitus Typ II findet sich zunehmend Evidenz für eine Rolle von Amyloid. Fragmenten bei der Zelldegeneration der Insulin-produzierenden Langerhans'schen Inselzellen. Über einen nicht-kontrollierten Calciumeinstrom kann die Zelldegeneration verstärkt werden.^{1,2,3}

Die Verbindungen gemäß der Erfindung, sowie von pharmazeutisch annehmbaren Säureadditionssatzen derselben können als Wirkstoffe in Arzneimitteln beispielsweise zum Behandeln von degenerativen Erkrankungen der Inselzellen (wie z.B. Diabetes mellitus Typ II) eingesetzt werden.

Die Verbindungen der Erfindung können als Wirkstoffe in Arzneimitteln verwendet werden, die wie folgt eingesetzt werden können:
a) zur Behandlung der Alzhelmer'schen Krankheit.
b) zur Behandlung der Parkinson'schen Krankheit,
c) zur Behandlung der Huntington'schen Krankheit (Chorea),
d) zur Behandlung der Multiplen Sklerose,
e) zur Behandlung der Amyotrophen Loteralsklerose,
f) zur Behandlung der Epilepsie,
g) zur Behandlung der Folgen des Schlaganfalles,
h) zur Behandlung der Folgen des Schädel-Him-Traumas,
i) zur Behandlung und Prophylaxe der Folgen diffusen Sauerstoff- und Nährstoffmangels im Gehirn, wie sie nach Hypoxie, Anoxie, Asphyxie, Hertstillstand, Vergiftungen, sowie bei Komplikationen bei schweren Geburten am Säugllng oder bei Narkosen beobachtet werden,
j) zur insbesondere auch prophylaktischen Behandlung apoptotischer Degeneration in Neuronen, die durch lokale Radio- oder Chemotherapie von Gehirntumoren geschädigt wurden bzw. werden,
k) zur Behandlung der bakteriellen Meningitis,
l) zur Behandlung von Erkrankungen mit apoptotischer Komponente, besonders im Gefolge von amyloid-assoziierter Zelldegeneration,
m) zur Behandlung des Diabetes mellitus, insbesondere, wenn er mit Amyloiddegeneration der Inselzellen einhergeht,
n) zum Erhöhen der Muskelkraft und
o) zum Erhöhen der Ausdauer von Alzheimer-Potienten.

Die erfindungsgemäßen Verbindungen oder deren pharmazeutisch annehmbare Säureadditionssalze, z.B. Hydrobromid, Hydrochlorid, Methylsulfat, Methiodid, Tartrat, Fumarat, Oxalat etc. (siehe nachstehende Tabelle), können Patienten oral, rektal oder durch subkutane, intramuskuläre, intravenöse oder intrathekale Injektion oder Infusion, oder intracerebroventrikulär, z.B. mittels eines implantierten Behälters verabreicht werden.

| englisch | Säure | Salz |
|---|---|---|
| | | |
| Sulfamic | Sulfaminsäure | - |
| | Amidosulfonsäure | Amidosulfonat |
| 1,2-ethanedisulfonic | 1,2-Ethandisulfonsäure | 1,2-Ethandisulfonat |
| 2-Ethylsuccinic | 2-Ethylbemsteinsäure | 2-Ethylsuccinat |
| 2-hydroxyethanesulfonic = isethionic | 2-Hydroxyethansulfonsäure | 2-Hydroxyethansulfonat |
| 3-Hydroxynaphthoic | 3-Hydroxynaphthoesäure | 3-Hydroxynaphthoat |
| acetic | Essigsäure | Acetat |
| benzoic | Benzoesäure | Benzoat |
| benzenesulfonic | Benzolsulfonsäure | Benzolsulfonat |
| calcium dihydrogenedetic | Calciumdihydrogenethylendi amintetraessigsäure | Calciumethylendiamintetra acetat |
| camphorsulfonic | Camphersulfonsäure | Camphersulfonat |
| carbonic | Kohlensäure | Carbonat |
| citric | Zitronensäure | Citrat |
| Dodecylsulfonic | Dodecylsulfonsäure | Dodecylsulfonat |
| ethanesulfonic | Ethansulfonsäure | Ethansulfonat |
| edetic | Ethylendiamintetraessigsäure | Ethylendiamintetraacetat |
| fumaric | Fumarsäure | Fumarat |
| Glubionic | Glubionsäure | Glubionat |
| glucoheptonic . | Glucoheptonsäure | Glucoheptonat |
| gluconic | Gluconsäure | Gluconat |
| glutamic | Glutaminsäure | Glutamat |
| hexylresorcinic | Hexylresorcylsäure | Hexylresorcylat |
| HBr | Bromwasserstoffsäure | Hydrobromid |
| HCl | Salzsäure | Hydrochlorid |
| bicarbonic | Kohlensäure | Hydrogencarbonat |
| bitartaric | Weinsäure | Hydrogentartrat |
| hydriodic | lodwasserstoffsäure | Hydroiodid |
| lactic | Milchsäure | Lactat |
| lactobionic | Lactobionsäure | Lactobionat |
| Levulinic | Laevulinsäure | Laevulinat |
| estolic (laurylsulfuric) | Laurylschwefelsäure | Laurylsulfat |
| LIPOIC-(ALPHA) ACID | Liponsäure | Liponat |
| malic | Äpfelsäure | Malat |
| maleic | Maleinsäure | Maleinat |
| Malonic | Malonsäure | Malonat |
| methanesulfonic | Methansulfonsäure. | Methansulfonat |
| naphthalenesulfonic | Napthalinsulfonsäure | Napthalinsulfonat |
| nitric | Salpetersäure | Nitrat |
| Pantothenic | Pantothensäure | Pantothenat |
| phosphoric | Phosphosäure | Phosphat |
| polygalacturonic | Polygalacturonsäure Pectinsäure | Polygalacturonat |
| Propionic | Propionsäure | Propionat |
| salicylic | Salicylsäure | Salicylat |
| succinic | Bernsteinsäure | Succinat |
| sulfuric | Schwefelsäure | Sulfat |
| Tartaric | Weinsäure | Tartrat |

Typische Dosierungsraten bei Verabreichung dieser Wirkstoffe hängen von der Natur der verwendeten Verbindung ab und liegen bei intravenöser Applikation im Bereich von 0,01 bis 2,0 mg pro Tag und Kilogramm Körpergewicht in Abhängigkeit vom physischen Zustand und sonstiger Medikation des Patienten.

Die folgenden spezifischen Formulierungen können Anwendung finden:
Tabletten und Kapseln enthaltend 0,5 bis 50 mg
Lösung zur parenteralen Verabreichung enthaltend 0,1 bis 30 mg Wirkstoff/ml
flüssige Formulierungen zur oralen Verabreichung in einer Konzentration von 0,1 bis 15 mg/ml
flüssige Formulierungen zur intracerebroventrikulären Verabreichung, in einer
Konzentration von 1 oder 5 mg Wirkstoff/ml.
Die erfindungsgemäßen Verbindungen können auch ein transdermales System sein, in welchem 0,1 bis 10 mg/Tag freigesetzt werden.

Ein transdermales Dosiersystem besteht aus einer Vorratsschicht, welche 0,1 bis 30 mg der Wirksubstanz als freie Base oder Salz allenfalls zusammen mit einem Penetrationsbeschleuniger, z.B. Dimethylsulfoxid oder einer Carbonsäure, z.B. Octansäure, und einem hautnahen Polyacrylat, z.B. Hexylacrylat/Vinylacetat/Acrylsäure Copolymer samt Weichmacher, z.B. Isopropylmyristat enthält. Als Abdeckung dient eine wirkstoffundurchlässige Außenschicht, z.B. ein metallbeschichtetes, siliconisiertes Polyethylenpflaster mit einer Dicke von beispielsweise 0,35 mm. Zur Erzeugung einer klebenden Schicht dient z.B. ein Dimethylaminomethycrylat/Methacrylat Copolymer in einem organischen Lösungsmittel.

Die Erfindung bezieht sich auch auf pharmazeutische Zusammensetzungen, die in einem pharmazeutisch annehmbaren Hilfsstoff eine therapeutisch wirksame Menge wenigstens einer der erfindungsgemäß vorgeschlagenen Verbindungen enthält.

Die Erfindung erstreckt sich auch auf die Verwendung dieser Verbindungen zum Herstellen von Arzneimitteln und Verfahren zum Herstellen solcher Verbindungen.

Insbesondere sind die erfindungsgemäßen Verbindungen, die vielfach eine die Cholinesterasen hemmende Wirkung zeigen, als therapeutische und/oder prophylaktische Wirkstoffe für die senile Demenz, Alzheimer-Krankheit, usw. geeignet. Die erfindungsgemäß vorgeschlagenen Verbindungen sind neue tetraryklische, kondensierte, heterocyclische Verbindungen.

Zusätzlich zu den therapeutischen und/oder prophylaktischen Eigenschaften können die erfindungsgemäßen Verbindungen und Zusammensetzungen auch bei der Diagnose von Krankheitszuständen der eingangs genannten Art verwendet werden.

Literatur:
1) Kawahara, M.; Kuroda, Y.; Arispe, N.; Rojas, E.; "Alzheimer's beta-amyloid, human islet amylin, and prion protein fragment evoke intracellular free calcium elevations by a common mechanism in a hypothalamic BnRH neuronal cell line." *J Biol Chem* 2000 May 12; 275 (19): 14077-83
2) Ma, Z.; Westermark, P.; Westermark, GT; "Amyloid in human islets of Langerhans: immunologic evidence that islet amyloid polypeptide is modified in amyloidogenesis." Pancreas 2000 Aug; 21 (2): 212-8
3) Rhoades, E.; Agarwal, J.; Gafni, A.; "Aggregation of an amyloidogenic fragment of human islet amyloid polypeptide." *Biochim Biophys Acta* 2000 Feb 9; 1476(2): 230-8

Nachstehend werden Arbeitsvorschriften und Beispiele zum Herstellen erfindungsgemäßer Verbindungen angegeben.

### Allgemeine Bemerkungen

"Konzentrieren" oder "Konzentration" bezeichnet das Entfernen von Lösungsmitteln unter vermindertem Druck mittels eines Rotationsverdampfers.

"MPLC" bezeichnet eine chromatographische Reinigung an Kieselgel 20-60 µm unter Verwendung von Büchi-Chromatographiesäulen, einer Shimadzu LC-8A Pumpe und einem Shimadzu 6AV UV-Detektor.

### Beispiel 1 :

### Stufe 1: 4-Brom-2-methoxy-5-(2-nitroethenyl)-phenol

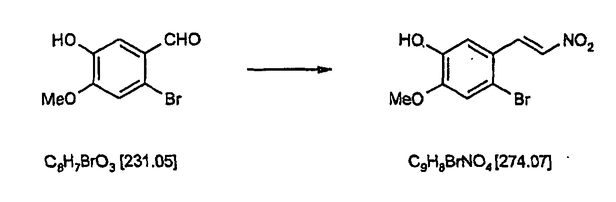

40.0 g (173 mmol) 2-Brom-5-hydroxy-4-methoxybenzaldehyd und 13.3 g (173 mmol) Ammoniumacetat werden in 400 mL Nitromethan 15 Min. auf Rückfluß erhitzt. Das Reaktionsgemisch wird zur Trockene eingedampft, der Rückstand in etwa 70 mL Methanol digeriert und anschließend abgesaugt. Um eine zweite Fraktion des Produkts zu gewinnen wird die Methanol-Lösung auf ca. 30 mL eingeengt und sodann auf 500 mL Wasser gegossen. Der ausgefallene Feststoff wird abgenutscht, mit ca. 100 mL Wasser gewaschen und gemeinsam mit der ersten Fraktion bei 50°C/50 mbar getrocknet, wodurch insgesamt 43.6 g (92 % d. Th.) gelbe Kristalle vom Schmp. 152 - 154°C an 4-Brom-2-methoxy-5-(2-nitroethenyl)-phenol erhalten werden.
- DC:: CH₂Cl₂ : MeOH = 9:1
- ¹H-NMR (CDCl₃; δ (ppm):: 3.85 (s, 3H, OCH₃); 7.30 (s, 1 H, H-6); 7.38 (s, 1H, H-3); 8.03 (d, ³J_{HH} = 13.41 Hz, 1H, ArCH=); 8.16 (d, ³J_{HH} = 13.41 Hz, 1H, =CHNO₂)
- ¹³C-NMR (CDCl₃; δ (ppm):: 56.3 (q, OCH₃); 114.7 (d, C-6); 116.1 (d, C-3); 116.6 (s, C-2); 121.4 (s, C-1); 136.8 (d, ArCH=); 137.6 (d, =CHNO₂); 146.5 (s, C-5); 152.2 (s, C-4)

### Stufe 2: 4-Brom-2-methoxy-5-(2-aminoethyl)-phenol

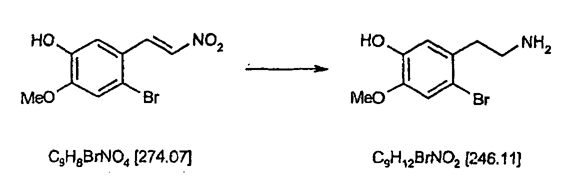

### Methode A:

Zu 168 mL (148 mmol) einer 0.88 N Lithiumaluminiumhydridlösung in Diethylether werden bei 0°C unter Stickstoff-Atmosphäre 7.2 g (74 mmol) konzentrierte Schwefelsäure zugetropft. 10.0 g (36.5 mmol) 4-Brom-2-methoxy-5-(2-nitroethenyl)-phenol werden in einem Liter absolutem Diethylether in der Siedehitze teilweise gelöst und anschließend die überstehende Lösung mit einer Transfernadel und trockenem Stickstoff der Aluminiumhydridlösung bei Raumtemperatur zugegeben. Nach der vollständigen Zugabe werden 700 mL Diethylether aus dem Reaktionsgemisch zum ungelösten 4-Brom-2-methoxy-5-(2-nitroethenyl)-phenol in den Vorlagekolben destilliert. Durch Erhitzen auf Rückfluß wird eine gesättigte Lösung hergestellt, die wie oben dem Reaktionsgemisch zugeführt wird. Dieser Vorgang wird bis zur vollständigen Zugabe von 4-Brom-2-methoxy-5-(2-nitroethenyl)-phenol wiederholt (drei- bis viermal). Anschließend wird mit Wasser bei 0°C hydrolysiert und die etherische Phase zweimal mit je 300 mL 4N Salzsäure extrahiert. Die saure Lösung wird mit 22.2 g (148 mmol) L-(+)-Weinsäure versetzt, mit konzentriertem wäßrigen Ammoniak basisch gemacht und erschöpfend mit Chloroform extrahiert. Die vereinigten organischen Phasen werden mit gesättigter wäßriger Natriumchloridlösung gewaschen, getrocknet (Na₂SO₄), filtriert und eingedampft, wodurch 2.20 g (24 % d. Th.) farblose Kristalle vom Schmp. 170 - 172°C an 4-Brom-2-methoxy-5-(2-aminoethyl)-phenol erhalten werden.

### Methode B:

Zu einer auf Rückfluß erhitzten Lösung von 15.0 g (394.2 mmol) Lithiumaluminiumhydrid in 1l absolutem Tetrahydrofuran wird im Laufe von 2 Stdn. unter Stickstoff eine Lösung von 18.0 g (65.7 mmol) 4-Brom-2-methoxy-5-(2-nifroethenyl)-phenol in 200 mL absolutem Tetrahydrofuran zugetropft. Anschließend wird das Reaktionsgemisch unter Eiskühlung mit ca. 20 mL Wasser hydrolysiert und zur Trockene eingedampft. Der Rückstand wird in 500 mL 2 N Salzsäure aufgenommen und mit 500 mL Essigsäureethylester gewaschen. Die Waschphase wird mit 200 mL 2 N Salzsäure rückgeschüttelt, die vereinigten wäßrigen Phasen mit 70 g (467 mmol) L-(+)-Weinsäure versetzt, mit konzentriertem wäßrigen Ammoniak basisch gemacht und dreimal mit je 800 mL Chloroform extrahiert. Die vereinigten organischen Phasen werden über Natriumsulfat getrocknet, filtriert und eingedampft, wodurch 9.92 g (61 % d. Th.) farblose Kristalle an 4-Brom-2-methoxy-5-(2-aminoethyl)-phenol vom Schmp. 170 - 172°C erhalten werden.

### Stufe 3: 4-Brom-5-{N-[(4-hydroxyphenyl)methyl]-2-aminoethyl]-2-methoxyphenol

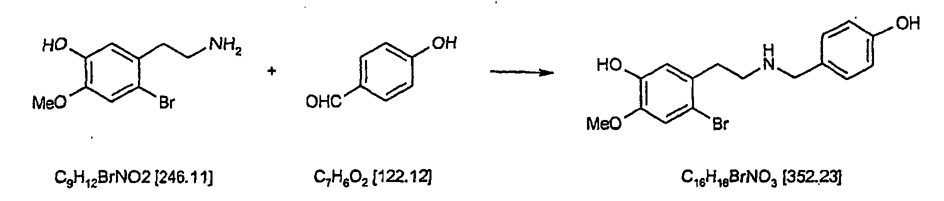

6.4 g (26.0 mmol) 4-Brom-2-methoxy-5-(2-aminoethyl)-phenol und 3.2 g (26.0 mmol) p-Hydroxybenzaldehyd werden in 150 mL absolutem Ethanol 2 Stdn. auf Rückfluß erhitzt. Anschließend werden unter Eiskühlung 5.0 g (132.0 mmol) Natriumborhydrid zugegeben und eine weitere halbe Stunde auf Rückfluß erhitzt, das überschüssige Natriumborhydrid durch Zugabe von etwa 1 mL Eisessig sowie 50 mL Wasser unter Eiskühlung zerstört und die Lösung eingedampft. Der Rückstand wird mit 2 N Salzsäure angesäuert, und mit 50 mL Chloroform gewaschen. Bei der Hydrolyse können sich eventuell größere Feststoffbrocken bilden, welche vor der Extraktion zermahlen werden müssen, da sie große Mengen Produkt einschließen können. Die Waschphase wird mit 30 mL 2 N Salzsäure rückgeschüttelt, die vereinigten wäßrigen Phasen mit konzentriertem wäßrigen Ammoniak basisch gemacht und dreimal mit je 80 mL Essigsäureethylester extrahiert. Die organischen Phasen werden vereinigt, über Natriumsulfat getrocknet, filtriert und eingedampft, wodurch 8.9 g (97 % d. Th.) farblose Kristalle vom Schmp. 69-72°C an 4-Brom-5-{N-[(4-hydroxyphenyl)methyl]-2-aminoethyl}-2-methoxyphenol erhalten werden.
- DC:: CHCl₃ : MeOH = 9:1 + 2% NH₃
- ¹H-NMR (DMSO; δ (ppm)):: 2.55 - 2.78 (m, 4H, ArCH₂CH₂NH); 3.58 (s, 2H, NHCH₂Ph); 3.73 (s, 3H, OCH₃); 6.60 - 6.76, 7.02 - 7.14 (2* m, 6H, 2* Ph)
- ¹³C-NMR (DMSO; δ (ppm)):: 35.2 (t, ArCH₂); 48.7 (t, CH₂CH₂NH); 52.2 (t, NHCH₂Ph); 55.9 (q, OCH₃); 111.3 (s, C-4); 114.8 (d, C-3'); 115.9 (d, C-6); 117.3 (d, C-3); 129.1 (d, C-2'); 130.7 (s, C-5); 131.4 (s, C-1'); 146.0 (s, C-2); 146.8 (s, C-1); 156.0 (s, C-4')

### Stufe 4: N-[2-(2-Brom-5-hydroxy-4-methoxyphenyl)ethyl]-N-[(4-hydroxyphenyl]methyl]formamid:

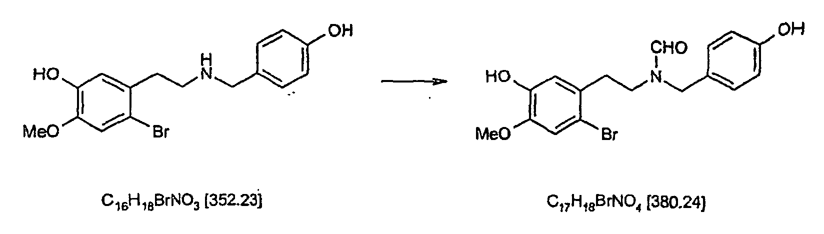

8.5 g (24.l mmol) 4-Brom-5-{N-[(4-hydroxyphenyl)methyl]-2-aminoethyl}-2-methoxyphenol und 10 mL (123.8 mmol) Ameisensäureethylester werden mit 2.5 mL Ameisensäure, 10 mL N,N-Dimethylformamid sowie einer Spatelspitze Dimethylaminopyridin in 150 mL absolutem Dioxan 24 Stdn. auf Rückfluß erhitzt. Gegen Ende der Reaktion klärt sich die anfangs weiße Suspension und die Mischung wird mit 50 mL Wasser versetzt. Das Dioxan wird abdestilliert, der entstandene weiße Niederschlag abgenutscht und mit Wasser gewaschen, wodurch die erste Fraktion Produkt erhalten wird. Das Filtrat wird dreimal mit je 50 mL Essigsäureethylester extrahiert, die vereinigten organischen Phasen über Natriumsulfat getrocknet, filtriert und eingedampft. Durch anschließende Säulenchromatographie (50 g Kieselgel, Laufmittel: CHCl₃ : MeOH = 97:3) wird eine weitere Fraktion erhalten. Beide Fraktionen werden bei 50°C/50 mbar bis zur Gewichtskonstanz getrocknet, wodurch insgesamt 6.6 g (72% d. Th.) farblose Kristalle vom Schmp. 104-106°C an N-[2-(2-Brom-5-hydroxy-4-methoxyphenyl)ethyl]-N-[(4-hydroxyphenyl)methyl]-formamid erhalten werden.
- DC:: CHCl₃ : MeOH = 9:1
- ¹H-NMR (DMSO; δ (ppm)):: 2.56 - 2.78 (m, 2H, ArCH₂); 3.43 - 3.53 (m, 2H, CH₂N); 3.72 (s, 3H, OCH₃): 4.14 (dd, 2H, NCH₂Ph); 6.67 - 6.80, 7.00-7.11 (2* m, 6H, Ar, Ph); 9.30, 9.48 (2* s, 1H, CHO)
- ¹³C-NMR (DMSO; δ (ppm)):: 32.6,34.2 (2* t, ArCH₂); 41.5, 44.3 (2* t, CH₂N); 46.1, 50.4 (2* t, NCH₂Ph); 56.1 (q, OCH₃); 111.4, 111.6 (2* s, C-4); 115.1, 115.2 (2* d, C-6); 115.6, 115.7 (2* d, C-3'); 117.7, 118.0 (2* d, C-3); 126.8, 127.0 (2* s, C-5); 129.4 (d, C-2'); 130.0 (s, C-1'); 146.5, 146.6 (2* s, C-2); 147.5, 147,6 (2* s, C-1); 157.1, 157.5 (2* s, C-4'); 162.7, 163.0 (2* d, CHO)

### Stufe 5: (4aα,8aα)-4a,5,9,10,11-Hexahydro-1-brom-3-methoxy-6-oxo-6H-benzofuro[3a,3,2-ef]-[3]benzazepin-10-carboxaldehyd:

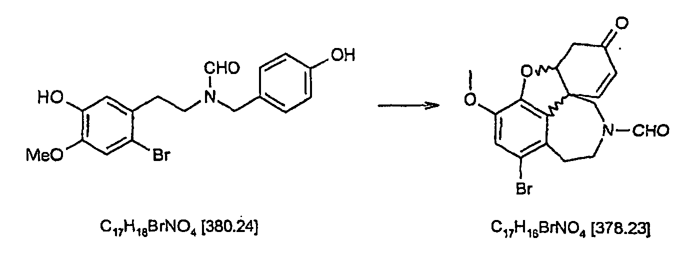

Eine Mischung von 13 g (39.5 mmol) Kaliumhexacyanoferrat(III), 300 mL Chloroform und 50 mL wäßrige 10 %ige Kaliumcarbonatlösung wird auf 60°C erwärmt, unter heftigem Rühren mit 3 g (7.9 mmol) N-[2-(2-Brom-5-hydroxy-4-methoxyphenyl)ethyl]-N-[(4-hydroyphenylyl)methyl]-formamid versetzt und anschließend weitere 10 Minuten heftig mechanisch gerührt. Danach wird der entstandene braune Feststoff über Hyflo abfiltriert, dreimal mit je 30 mL Chloroform nachgewaschen und fest abgepreßt. Das Filtrat wird sodann mit etwa 150 mL Wasser gewaschen, die Waschphase mit 150 mL Chloroform rückgeschüttelt, die vereinigten organischen Phasen über Magnesiumsulfat getrocknet, filtriert und eingedampft. Durch Reinigung über Säulenchromatographie (15 g Kieselgel, Laufmittel: CHCl₃ : MeOH = 97:3) werden 580 mg (19 % d. Th.) farblose Kristalle vom Schmelzpunkt 218-220°C erhalten.
- DC:: CHCl₃ : MeOH = 9:1
- ¹H-NMR (CDCl₃; δ (ppm)):: 2.58-4.27 (m, 8H, H-5/5'/9/9'/11/11'/12/12'); 3.80 (s, 3H, OCH₃); 4.85 (dd, 1 H, H-4a); 6.09 (dd, 1 H, H-8); 6.53 (dd, 1 H, H-7); 7.01 (s, 1 H, H-2); 8.10, 8.30 (2* s, 1 H, CHO _{Konf.A/B})
- ¹³C-NMR (CDCl₃; δ (ppm)):: 33.4, 35.3 (2*t, C-9 _{Konf.A/B}); 37.2, 37.4 (2*t, C-5 _{Konf.A/B}); 43.7 (t, C-11); 48.7, 49.0 (2*t, C-12 _{Kont.A/B}); 50.9, 51.4 (2*s, C-8a _{Kont.A/B}); 56.2 (q, OCH₃); 83.8, 84.3 (2*s, C-4a _{Konf.A/B}); 115.3, 115.7 (2*s, C-1 _{Konf.A/B}); 116.8, 117.0 (2*d, C-8 _{Konf.A/B}); 127.6, 128.9 (2*s, C-12a _{Konf.A/B}); 128.0, 128.8 (2*d, C-7 _{Konf.A/B}); 129.8, 130.8 (2*s, C-12b _{Konf.A/B}); 141.5, 141.7 (2*d, C-2 _{Konf.A/B}); 143.8, 144.0 (2*s, C-3a _{Konf.A/B}); 146.8 (s, C-3); 161.7, 162.3 (2*d, CHO); 193.0, 193.4 (2*s, C-6)
- C₁₇H₁₆BrNO₄ (JOS 1526): 378.23 g/mol
- ber.:: C 53.99 H 4.26 N 3.70
- gef.:: C 53.70H 4.47 N 3.41

### Stufe 6: (4aα,8aα)-4a,5,9,10,11-Hexahydro-1-brom-3-methoxy-6H-benzofuro[3a,3,2-ef]-[3]benzazepin-6-ol:

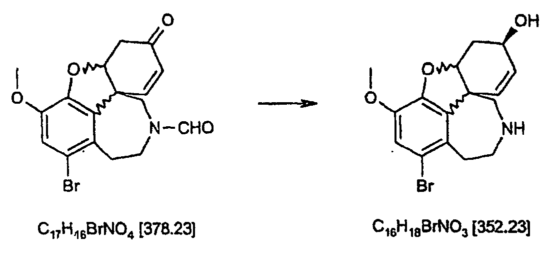

Zu einer Lösung von 500 mg (1.32 mmol) (4aá,8aá)-4a,5,9,10,11-Hexahydro-1-brom-3-methoxy-6-oxo-6H-benzofuro[3a,3,2-ef][3]benzazepin-10-carboxaldehyd in 12 mL absolutem Tetrahydrofuran werden bei -12°C unter Stickstoff 4 mL (4.00 mmol) 1 N L-Selectrid-Lösung zugetropft und das Reaktionsgemisch anschließend eine Stunde bei -10°C gerührt. Danach wird mit 3 mL Methanol hydrolysiert, die Lösung zur Trockene eingedampft, in 50 mL 2 N Salzsäure aufgenommen und eine weitere Stunde heftig gerührt. Die wäßrige Lösung wird mit 50 mL Essigsäureethylester gewaschen, die Waschphase mit 20 mL 2 N Salzsäure rückgeschüttelt, die vereinigten wäßrigen Phasen mit konzentriertem wäßrigen Ammoniak basisch gemacht und dreimal mit je 50 mL Essigsäureethylester extrahiert. Die vereinigten organischen Phasen werden über Magnesiumsulfat getrocknet, filtriert und eingedampft, wodurch 380 mg (82 % d. Th.) hellgelbe Kristalle vom Schmp. 132 - 136°C an (4aα,8aα)-4a,5,9,10,11-Hexahydro-1-brom-3-methoxy-6H-benzofuro[3a,3,2-ef][3]benzazepin-6-ol erhalten werden.
- DC:: CHCl₃ : MeOH = 9:1
- ¹H-NMR (CDCl₃; δ (ppm)):: 1.87 (ddd, 1 H, H-5); 2.62 (ddd, 1H, H-5'); 2.68 (ddd, 1 H, H-11); 2.78 (d, 1H, H-9, ²J_{9/9'}=12.6 Hz); 2.85 (ddd, 1H, H-11'); 2.98 (d, 1 H, H-9', ²J_{9/9'}=12.6 Hz); 3.30 (ddd, 1 H, H-12); 3.37 (ddd, 1 H, H-12'); 3.80 (s, 3H, OCH₃); 4.08 (ddd, 1H, H-6); 4.50 (dd, 1 H, H-4a); 6.08 (dd, 1 H, H-8, ³J_{7/8}=10.2 Hz); 6.15 (d, 1 H, H-7, ³J_{7/8}=10.2 Hz); 6.96 (s, 1 H, H-2)
- ¹³C-NMR (CDCl₃; δ (ppm)):: 30.2 (t, C-5); 36.7 (t C-9); 49.7 (t, C-11); 51.6 (s, C-8a); 56.0 (q, OCH₃); 57.3 (t, C-12); 62.0 (d, C-6); 85.5 (d, C-4a); 114.9 (s, C-1); 115.7 (d, C-8); 127.3 (d, C-2); 127.7 (d, C-7); 130.5 (s, C-12a); 134.2 (s, C-12b); 143.5 (s, C-3a); 145.4 (s, C-3)

### Stufe 7: (4aα,8aα)-4a,5,9,10,11-Hexahydro-1-brom-3-methoxy-10-methyl-6H-benzofuro[3a,3,2-ef]-[3]benzazepin-6-ol

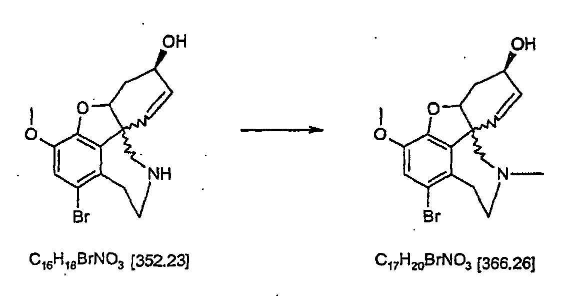

Zu einer Lösung von 370 mg (1.05 mmol) von (4aα,8aα)-4a,5,9,10,11-Hexahydro-1-brom-3-methoxy-6H-benzofuro[3a,3,2-ef][3]benzazepin-6-ol in 12 mL Acetonitril werden unter heftigem Rühren nacheinander 1 mL 35 %ige wäßrige Formaldehydlösung und portionsweise 165 mg (2.63 mmol) Natriumcyanoborhydrid zugegeben und das Reaktionsgemisch bei Raumtemp. eine Stunde heftig gerührt. Danach wird die Lösung mit 2 N Salzsäure angesäuert, mit 15 mL Dichlormethan gewaschen und die Waschphase mit 15 mL 2 N Salzsäure rückgeschüttelt. Die vereinigten wäßrigen Phasen werden mit konzentriertem wäßrigen Ammoniak basisch gemacht und dreimal mit je 30 mL Dichlormethan extrahiert. Die vereinigten organischen Phasen werden über Magnesiumsulfat getrocknet, filtriert und eingedampft, wodurch 355 mg (92 % d. Th.) gelbe Kristalle vom Schmp. 158 - 161°C an (4aα,8aα)-4a,5,9,10,11-Hexahydro-1-brom-3-methoxy-10-methyl-6H-benzofuro[3a,3,2-ef][3]benzazepin-6-ol erhalten werden.
- DC:: CHCl₃ : MeOH = 9:1
- ¹H-NMR (CDCl₃; δ (ppm)):: 1.91 - 2.04 (m, 1 H, H-5); 2.27 - 2.48 (m, 2H, H-5'/11); 2.41 (s, 3H, NCH₃); 2.60 - 2.81 (m, 2H, H-9/11'); 2.92 - 3.16 (m, 2H, 9'/12); 3.34 (dd, ³J_{11/12'} = 6.37 Hz, ²J_{12/12'} = 16.48 Hz, 1 H, H-12'); 4.13 - 4.25 (m, 1 H, H-6); 4.58 (b, 1 H, H-4a); 6.02 (dd, ³J_{7/8} = 10.17 Hz, ⁴J_{6/8} = 5.08 Hz, 1 H, H-8); 6.18 (d, ³J_{7/8} = 10.17 Hz, 1 H, H-7); 6.92 (s, 1 H, H-2)

### Stufe 8: (4aα,8aα)-4a,5,9,10,11-Hexahydro-3-methoxy-10-methyl-6H-benzofuro[3a,3,2-ef]-[3]benzazepin-6-ol

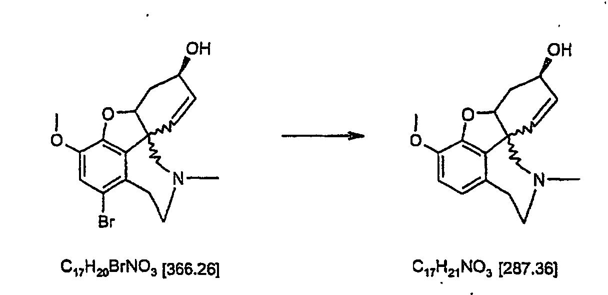

Eine Mischung aus 340 mg (0.93 mmol) (4aα,8aα)-4a,5,9,10,11-Hexahydro-1-brom-3-methoxy-10-methyl-6H-benzofuro[3a,3,2-ef][3]benzazepin-6-ol und 722 mg (6.51 mmol) Calziumchlorid in 40 mL 50 %igem Ethanol wird mit 1.4 g (22.32 mmol) frisch aktiviertem Zinkpulver¹ versetzt und 5 Stdn. auf Rückfluß erhitzt. Anschließend wird das Zink abfiltriert, mit Methanol nachgewaschen und die Restlösung eingedampft. Der Rückstand wird in 50 mL 1 N Salzsäure aufgenommen, mit 30 mL Essigsäureethylester gewaschen und die Waschphase mit 20 mL Salzsäure rückgeschüttelt. Die vereinigten wäßrigen Phasen werden mit konzentriertem wäßrigen Ammoniak basisch gemacht und dreimal mit je 50 ml Essigsäureethylester extrahiert. Die vereinigten organischen Phasen werden über Magnesiumsulfat getrocknet, filtriert und eingedampft, wodurch 230 mg (86 % d. Th.) gelbe Kristalle vom Schmp. 152 - 155°C an (4aα,8aα)-4a,5,9,10,11-Hexahydro-3-methoxy-10-methyl-6H-benzofuro[3a,3,2-ef][3]benzazepin-6-ol erhalten werden.
- DC:: EE: EtOH = 9:1 (sichtbar durch Oxidation in der Jod-Kammer)
- ¹H-NMR (CDCl₃; δ (ppm)):: 1.90-2.04 (m, 1H, H-5); 2.26 - 246 (m, 2H, H-11/11'); 2.42 (s, 3H, NCH₃); 2.62 - 2.80 (m, 3H, H-5'/9/9'); 3.01 - 3.12 (m, 1H, H-12); 3.12-3.29 (m, 1 H, H-12'); 3.83 (s, 3H, OCHs); 4.12 - 4.22 (m, 1H, H-6); 4.57 (b, 1 H, H-4a); 6.01 (ddd, ³J_{7/8} = 10.16 Hz, ⁴J_{6/8} = 5.18 Hz, ⁵J_{5/8} = 0.95 Hz; 1H, H-8); 6.22 (dd, ³J_{7/8} = 10.16 Hz, ⁴J_{5/7} = 1.09 Hz, 1 H, H-7); 6.61 (d, ³J_{1/2} = 8.21 Hz. 1 H, H-2); 6.66 (d, ³J_{1/2} = 8.21 Hz, 1H, H-1)
- ¹³C-NMR (CDCl₃; δ (ppm)):: 30.0 (t, C-5); 34.5 (t, C-9); 48.9 (s, C-8a); 49.3 (q, NCH₃); 55.6 (q, OCH₃); 59.1 (t, C-11); 62.0 (d, C-6); 66.3 (t, C-12); 85.6 (d, C-4a); 111.1 (d, C-1); 121.5 (d, C-8); 126.5 (d, C-2); 128.3 (d, C-7); 130.9 (s, C-12a); 132.7 (s, C-12b); 142.9 (s, C-3a); 145.3 (s, C-3b)

¹ Zinkpulver (Fa. Aldrich) mit 2 N Salzsäure versetzten, gut durchmischen, abfiltrieren und zunächst mit dest Wasser neutral waschen, dann mit Methanol gut nachwaschen.

### Beispiel 2:

### (4aα,8aα)-4a,5,9,10,11-Hexahydro-1-brom-6-[(4-bromphenyl)methyl]-3-methoxy-6Hbenzofuro[3a,3,2-ef][3]benzazepin-6-ol

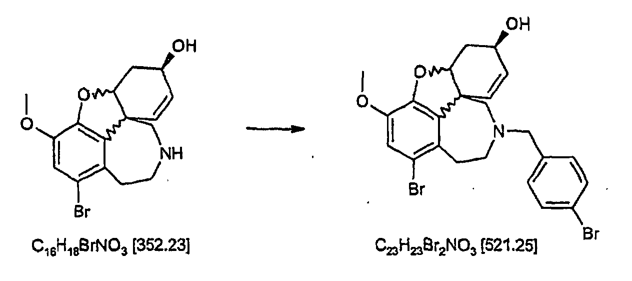

Eine Mischung von 23 mg (0.068 mmol) (4aα,8aα)-4a,5,9,10,11-Hexahydro-1-brom-3-methoxy-6Hbenzofuro[3a,3,2-ef][3]benzazepin-6-ol, 19 mg (0.136 mmol) Kaliumcarbonat und 12 mg (0.082 mmol) Natriumjodid wird in 20 ml absolutem Aceton mit 21 mg (0.082 mmol) 4-Brombenzylbromid versetzt und auf Rückfluß erhitzt. Nach einer Stunde wird das Reaktionsgemisch eingedampft, der Rückstand in 10 ml 2 N Salzsäure aufgenommen, mit Essigsäureethylester gewaschen, mit konzentriertem wäßrigen Ammoniak basisch gemacht und dreimal mit je 5 ml Essigsäureethylester extrahiert. Die vereinigten organischen Phasen werden einmal mit gesättigter wäßriger Natriumchloridlösung gewaschen, getrocknet (Na₂SO₄, Aktivkohle), filtriert und eingedampft. Die weitere Reinigung erfolgt über flash chromatographie (15 g Kieselgel; Laufmittel: CHCl₃ ⇒ CHCl₃ : MeOH = 95:5), wodurch 10 mg (29 % d. Th.) ölige Substanz an (4aα,8aα)-4a,5,9,10,11-Nexahydro-1-brom-6-[(4-bromphenyl]methyl]-3-methoxy-6Hbenzofuro[3a,3,2-ef][3]benzazepin-6-ol erhalten werden.
- DC:: CHCl₃ : MeOH = 9:1
- ¹H-NMR (CDCl₃; δ (ppm)):: 1.78 (ddd, 1 H, H-5); 1.98-2.31 (m, 4H, H-5'/9/11/11'); 2.70 (ddd, 1H, H-9'); 3.57 (ddd, 1H, H-12); 3.82 (s, 3H, OCH₃); 3.86 (ddd, 1 H, H-12'); 4.15 (b, 1H, H-6); 4.42 (d, 1H, NCH₂); 4.65 (b, 1H, H-4a); 5.00 (d, 1H, NCH₂'); 5.91 (d, 1H, H-7); 6.06 (dd, 1H, H-8); 6.92 (s, 1H, H-2); 7.28 (d, 2H, Ph-2/6); 7.43 (d, 2H, Ph-3/5)

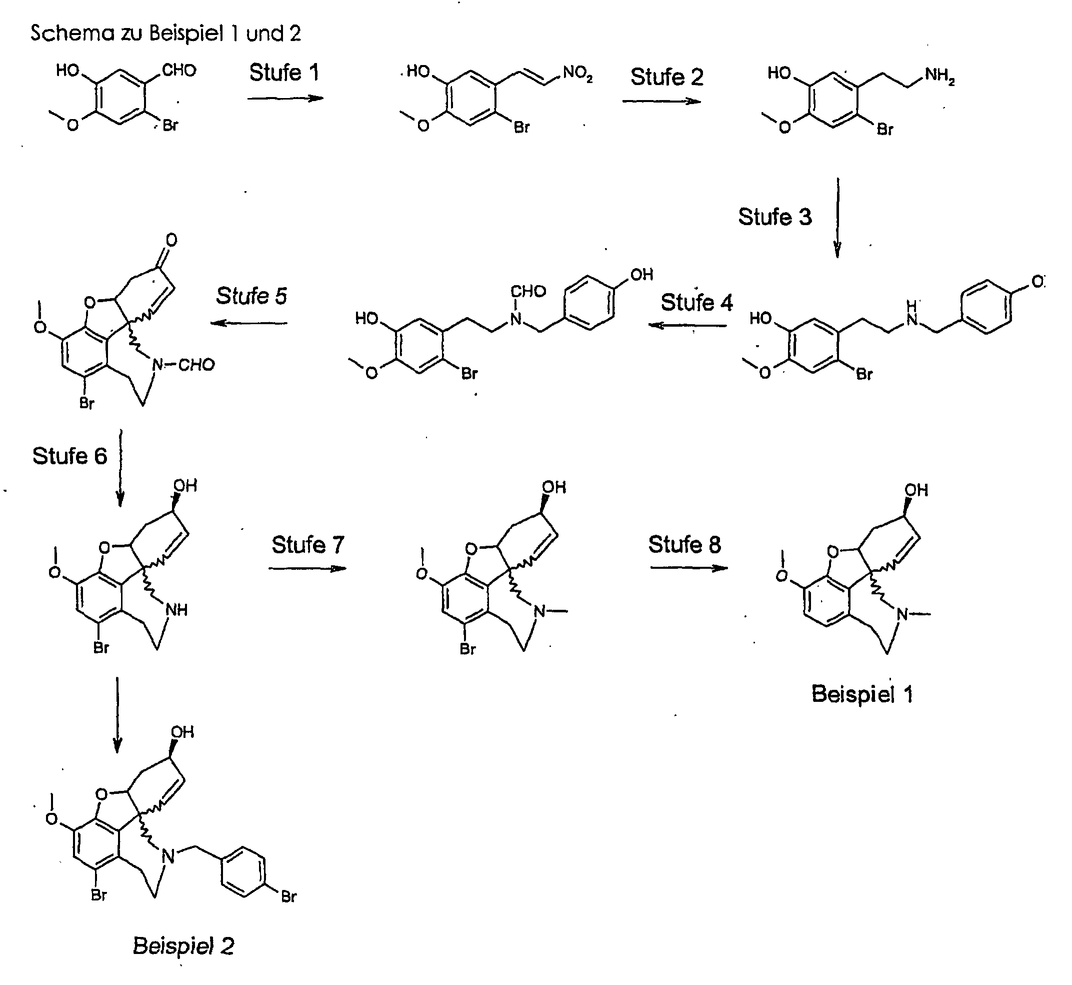

### Beispiel 3:

### 2-[4-[(4aS,6R,8aS)-6-Hydroxy-3-methoxy-5,6,9,10,11,12-hexahydro-4aH-benzofuro[3a,3,2-e,f][2]benzazepin-11-yl]butyl]-1,2-benzoisothiazol-3(2H)-on, 1,1-dioxid tartrat, dihydrat (SPH-1374)

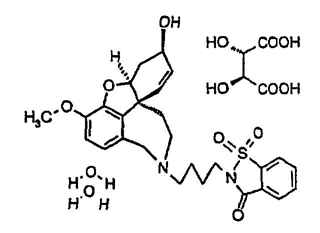

2-(6-Bromhexyl)-1,2-benzisothiazol-3(2H)-on-1,1-dioxid (2.33 g, 7.32 mmol), hergestellt gemäß Hamor, G. H.; Rubessa, F.; *Farmaco Ed. Sci*. **1970**, 25, 36-39, Norgalanthamin (2.00 g, 7.32 mmol) und N-Ethyldiisopropylamin (2.84 g, 22.0 mmol) in absolutem Chloroform (20 ml) werden 24 Stunden bei Siedetemperatur gerührt.

Das Lösungsmittel wird abgezogen und der Rückstand säulenchromatographisch gereinigt (150 g Kieselgel, Chloroform : Methanol : Ammoniak: 96.5 : 3 : 0.5), wodurch das Produkt als farbloser Schaum (2.67 g, 5.23 mmol, 71.4 %) erhalten wird.
- DC:: Chloroform : Methanol: Ammoniak = 89 : 10 : 1; Rf = 0.5
¹H NMR (CDCl₃): δ 8.05 - 7.72 (m, 4H), 6.63 - 6.55 (m, 2H), 6.10 - 5.90 (m, 2H), 4.56 (b, 1H), 4.15 - 4.01 (m, 2H), 3.84 - 3.70 (m, 6H), 3.42 - 3.04 (m, 2H), 2.71 - 2.35 (m, 4H), 2.10 - 1.72 (m, 4H), 1.65 - 1.40 (m, 2H);
¹³C NMR (CDCl₃): δ 158.8 (s), 145.7 (s), 143.9 s), 137.5 (s), 134.6 (d), 134.1 (d), 133.0 (s), 129.4 (s), 127.4 (d), 127.2 (s), 126.8 (d), 124.9 (d), 121.8 (d), 120.7 (d), 111.0 (d), 88.5 (d), 61.9 (d), 57.5 (t), 55.7 (q), 51.4 (t), 50.5 (t), 48.3 (s), 39.1 (t), 32.9 (t), 29.8 (t), 26.0 (t), 24.5 (t)

Die Base (SPH-1369, 2.50g, 4.90 mmol) und (+)-Weinsäure (0.80g, 5.33 mmol, 1.09 Aquivalent) werden in EtOH (95%, ca. 10 mL) bis zur klaren Lösung erwärmt (ca. 50°C) und diese Lösung noch warm tropfenweise innerhalb von 5 min zu magnetisch gerührtem absolutem Ether (ca. 200 mL) zugefügt wobei ein weißer Niederschlag entsteht. Nach Stehen über Nacht bei Raumtemperatur werden die erhaltenen Kristalle abgenutscht und mit absol. Ether (3 × 50 mL) gewaschen und das Produkt im Vakuumexsiccator bei Raumtemperatur/50 mbar über Calciumchlorid getrocknet wobei das Tartrat Dihydrat in Form eines farblosen Pulvers (3.184g, 93.3% d.Th.) erhalten wird. Eine Analysenmenge wird bei 2 mbar und 40°C 8 Stunden über Phosphorpentoxid getrocknet.
C₂₇H₃₀N₂O₆S . C₄H₄O₄ . 2 H₂O (JOS 1659) (697.7)

| | | | |
|---|---|---|---|
| Ber. | C 56.18 | H 5.78 | N 4.23 |
| Gef.a) | C 55.74 | H 5.81 | N 4.15 |
| b) | C 55.76 | H 5.79 | N 4.26 . |

### Beispiel 4:

### 2-[5-[(4aS,6R,8aS)-6-Hydroxy-3-methoxy-5,6,9,10,11,12-hexahydro-4aH-benzofuro[3a,3,2-e,f][2]benzazepin-11-yl]pentyl]-1,2-benzoisothiazol-3(2H)-on, 1,1-dioxid (SPH-1372)

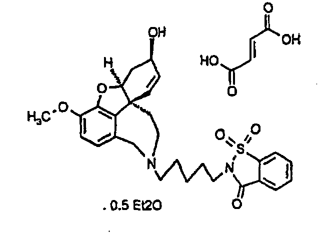

2-(5-Brompentyl)-1,2-benzisothiazol-3(2H)-on-1,1-dioxid (1.66 g, 5.00 mmol), Norgalanthamin (1.37 g, 5.00 mmol) und N-Ethyldiisopro- pylamin (1.94 g, 15.0 mmol) in absolutem Chloroform (15 ml) werden 24 Stunden bei Siedetemperatur gerührt.

Das Lösungsmittel wird abgezogen und der Rückstand säulenchro- matographisch gereinigt (150 g Kieselgel, Chloroform : Methanol : Ammoniak: 96.5 : 3 : 0.5), wodurch das Produkt als farbloser Schaum (2.09 g, 3.99 mmol, 79.7 %) erhalten wird.
- DC:: Chloroform : Methanol : Ammoniak = 89 : 10 : 1; Rf = 0.5
¹H NMR (CDCl₃): δ 8.05 - 7.70 (m, 4H), 6.63 - 6.50 (m, 2H), 6.09 - 5.85 (m, 2H), 4.55 (b, 1H), 4.15 - 3.99 (m, 2H), 3.82 - 3.60 (m, 5H), 3.41 - 2.92 (m, 2H), 2.70 - 2.32 (m, 3H), 2.09 - 1.70 (m, 4H), 1.58 - 1.23 (m, 6H);
¹³C NMR (CDCl₃): δ 158.7 (s), 145.6 (s), 143.8 (s), 137.5 (s), 134.5 (d), 134.1 (d), 133.0 (q), 129.4 (s), 127.3 (d), 127.2 (s), 126.8 (d), 124.8 (d), 121.8 (d), 120.7 (d), 111.0 (d), 88.5 (d), 61.8 (d), 57.5 (t), 55.7 (q), 51.4 (t), 51.0 (t), 48.2 (s), 39.1 (t), 32.8 (t), 29.8 (t), 28.1 (t), 26.6 (t), 24.3 (t), 20.3 (d)

### Herstellung des Fumarats (UJ-1682)

Eine warme (ca, 50°C) Lösung der Base (1.686g, 3.21 mmol) in EtOH (95%, 10 mL) wird mit gesättigter Fumarsäurelösung (10 mL, ca. 0.5 M in 95% Ethanol) vereinigt, bei ca. 60°C bis zum Erreichen einer klaren Lösung erwärmt und diese Lösung noch warm tropfenweise innerhalb von 5 min zu magnetisch gerührtem absolutem Ether (ca. 200 mL) zugefügt wobei ein weißer Niederschlag entsteht. Nach Stehen über Nacht bei Raumtemperatur werden die erhaltenen Kristalle abgenutscht und mit absol. Ether (3 × 50 mL) gewaschen und das Produkt im Vakuumexsiccator bei Raumtemperatur/50 mbar über Calciumchlorid getrocknet wobei das Fumarat in Form eines farblosen Pulvers (1.394g, 67.7% d.Th.) erhalten wird. Eine Analysenmenge wird bei 2 mbar und 40°C 8 Stunden über Phosphorpentoxid getrocknet. Aus der Mutterlauge wird eine zweite Fraktion gewonnen (= UJ-1682-1-2)
C₂₈H₃₂N₂O₆S . C₄H₄O₄ . ½ C₄H₁₀O (JOS 1657) (677.8)

| | | | |
|---|---|---|---|
| Ber. | C 59.54H | 6.21 | N 4.21 |
| Gef. | C 59.49H | 6.18 | N 4.20 |

### Beispiel 5:

### 2-[6-[(4aS,6R,8aS)-6-Hydroxy-3-methoxy-5,6,9,10,11,12-hexahydro-4aH-benzofuro[3a,3,2-e,f][2]benzazepin-11-yl]hexyl]-1,2-benzoisothiazol-3(2H)-on, 1,1-dioxid fumarat (SPH-1373)

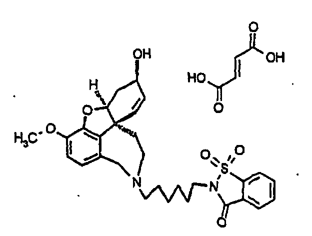

2-(6-Bromhexyl)-1,2-benzisothiazol-3(2*H*)-on-1,1-dioxid (1.50 g, 4.33 mmol), hergestellt gemäß Kim, Sung-Kyu; Cho, Su-Dong; Moon, Jung-Kyen; Yoon, Yong-Jin. J. Heterocycl. Chem. (1996), 33(3), 615-618, Norgalanthamin (1.18 g, 4.33 mmol), und N-Ethyldiisopropylamin (1.68 g, 13.0 mmol) in absolutem Chloroform (15 ml) werden 24 Stunden bei Siedetemperatur gerührt. Das Lösungsmittel wird abgezogen und der Rückstand säulenchromatographisch gereinigt (150 g Kieselgel, Chloroform : Methanol : Ammoniak: 96.5 : 3 : 0.5), wodurch die Base als farbloser Schaum (1.91 g, 3.52 mmol, 81.4 %) erhalten wird.
DC: Chloroform : Methanol : Ammoniak = 89 : 10 : 1; Rf = 0.5
¹H NMR (CDCl₃): δ 8.08 - 7.72 (m, 4H), 6.68 - 6.55 (m, 2H), 6.12 - 5.90 (m, 2H), 4.57 (b, 1H), 4.16 - 4.01 (m, 2H), 3.82 - 3.65 (m, 6H), 3.52 - 3.03 (m, 2H), 2.71 - 2.28 (m, 3H), 2.10 - 1.71 (m, 4H), 1.55 - 1.25 (m, 7H);
158.8 (s), 145.7 (s), 143.9 (s), 137.6 (s), 134.6 (d), 134.2 (d), 133.1 (s), 129.5 (s), 127.4 (d), 127.3 (s), 126.9 (d), 125.0 (d), 121.9 (d), 120.8 (d), 111.1 (d), 88.6 (d), 62.0 (d), 57.6 (t), 55.8 (q), 51.5 (t), 48.3 (t), 39.3 (t), 32.9 (t), 29.9 (t), 28.2 (t), 27.1 (t), 26.7 (t), 26.6 (t)

### Herstellung des Fumarats

Eine durch Erwärmen der Base (1.33g, 2.47 mmol) in Fumarsäurelösung (8 mL, gesättigte Lösung in 95% Ethanol) auf ca. 60°C erhaltene klare Lösung wird tropfenweise innerhalb von 5 min zu magnetisch gerührtem absolutem Ether zugefügt wobei ein weißer Niederschlag entsteht. Nach Stehen über Nacht bei Raumtemperatur werden die erhaltenen Kristalle abgenutscht und mit absol. Ether (3 × 50 mL) gewaschen und das Produkt im Vakuumexsiccator bei Raumtemperatur/50 mbar über Calciumchlorid getrocknet wobei das Fumarat in Form eines farblosen Pulvers (1.170g, 72% d.Th.) erhalten wird. Eine Analysenmenge wird bei 2 mbar und 40°C 8 Stunden über Phosphorpentoxid getrocknet.
C₂₉H₃₄N₂O₆S . C₄H₄O₄ (JOS 1658)

| | | | |
|---|---|---|---|
| Ber. | C 60.54, | H 5.85, | N 4.28. |
| Gef.: | C 60.49, | H 5.97, | N 4.22 |

### Beispiel 6:

### Stufe 1:2-(4-Brombutyl)-5,6-dimethoxy-1-oxoindan-2-carbon-säuremethylester

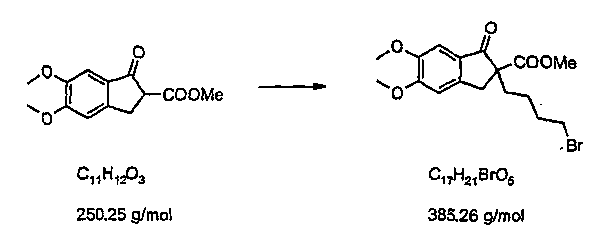

Zu einer Suspension von Natriumhydrid (0.84 g, 17.6 mmol, 50 % in Weißöl, durch Digerieren mit absolutem Petrolether (3 × 50 mL) vom Weißöl befreit) in absolutem DMF wird 5,6-Dimethoxy-1-oxoindan-2-carbonsäuremethylester (4.0 g, 16.0 mmol), hergestellt gemäß Fukushi, Hideto; Mabuchi, Hiroshi; Itoh, Katsumi; Terashita, Zen-ichi; Nishikawa, Kohei; Sugihara, Hirosada; Chem. *Pharm. Bull.* **1994,** 42(3), 541-550, bei Raumtemperatur in Substanz zugegeben und die Lösung 45 Minuten bei Raumtemperatur gerührt. Danach versetzt man mit 1,4-Dibrombutan (24.2 g, 112.0 mmol) und rührt 18 Stunden bei Raumtemperatur. Man verteilt zwischen Wasser und Ether, extrahiert die wäßrige Phase quantitativ mit Ether, wäscht die vereinigten organischen Phasen mit Wasser (5 x), gesättigter Kochsalzlösung (1 x) und trocknet (Natriumsulfat/Aktivkohle). Vom nach dem Eindampfen erhaltenen Rückstand wird überschüssiges Dibromalkan am Hochvakuum durch Kugelrohrdestillation (100 °C/0.05 mbar) abgetrennt und der erhaltene Rückstand aus siedendem *tert*.-Butylmethylether (25 mL) umkristallisiert, wodurch das Produkt in Form farbloser Kristalle (5.02 g, 13.0 mmol, 81.6 %) erhalten wird.
- DC:: Petrolether : Essigsäureethylester = 3 : 1; Rf = 0.15
- Schmp.:: 92 - 93 °C
¹H NMR (CDCl₃): δ 7.13 (s, 1H), 6.88 (s, 1H), 3.94 (s, 3H), 3.87 (s, 3H), 3.65 (s, 3H), 3.58 (d, J=18.3 Hz, 1H), 3.33 (t, J=6.7 Hz, 2H), 2.97 (d, J=17.2 Hz, 1H), 2.20-1.99 (m, 1H), 1.95-1.73 (m, 3H), 1.53-1.26 (m, 2H);
¹³C NMR (CDCl₃): δ 200.7 (s), 171.6 (s), 156.1 (s), 149.7 (s), 148.5 (s), 127.7 (s), 107.1 (d), 104.8 (d), 60.6 (s), 56.2 (q), 56.0 (q), 52.6 (q), 36.3 (t), 33.6 (t), 33.2 (t), 32.6 (t), 23.1 (t)

Anzahl, chemische Verschiebung und Multiplizität der gefundenen Peaks bestätigen die postulierte Struktur

### Stufe 2:

### 2-(4-Brombutyl)-5,6-dimethoxyindan-1-on

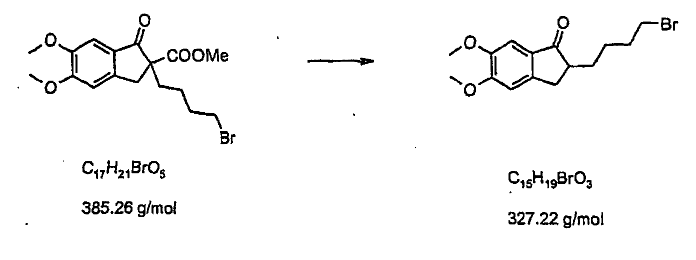

2-(4-Brombutyl)-5,6-dimethoxy-1-oxoindan-2-carbonsäuremethylester (3.0 g, 7.79 mmol) wird in konzentrierter Salzsäure (10 mL) und Essigsäure (30 mL) 12 Stunden bei 60 °C gerührt. Man versetzt mit gesättigter Natriumcarbonatlösung, neutralisiert mit Natriumcarbonat und extrahiert quantitativ mit Ether, wäscht die vereinigten organischen Phasen mit gesättigter Natriumcarbonatlösung (3 x), Wasser (1 x), gesättigter Kochsalzlösung (1 x), trocknet (Natriumsulfat/Aktivkohle) und kristallisiert den nach Eindampfen erhaltenen Rückstand aus tert.-Butylmethylether (10 mL) um. Auf diese Weise erhält man das Produkt in Form farbloser Kristalle (1.85 g, 5.65 mmol, 72.5 %)
- DC:: Petrolether : Essigsäureethylester = 3:1; Rf = 0.2
- Schmp.:: 72-73°C
¹H NMR (CDCl₃): δ 7.15 (s, 1H), 6.85 (s, 1H), 3.95 (s, 3H), 3.88 (s, 3H), 3.40 (t, J=6.8 Hz, 1H), 3.23 (dd, J=18.0 Hz, J=8.0 Hz, 1H), 2.78-2.57 (m, 2H), 2.00-1.72 (m, 3H), 1.65-1.35 (m, 3H); ¹³C NMR (CDCl₃): δ 207.1 (s), 155.5 (s), 149.4 (s), 148.8 (s), 129.3 (s), 107.3 (d), 104.3 (d), 56.1 (t), 56.0 (t), 47.4 (d), 33.5 (t), 32.6 (t), 32.5 (t), 30.6 (t), 25.8 (t)

### Stufe 3:

### 2-[4-[(4aS,6R,8aS)-6-Hydroxy-3-methoxy-5,6,9,10,11,12-hexahydro-4aH-benzofuro[3a,3,2-e,f][2]benzazepin-11-yl]butyl]-5,6-dimethoxyindan-1-on

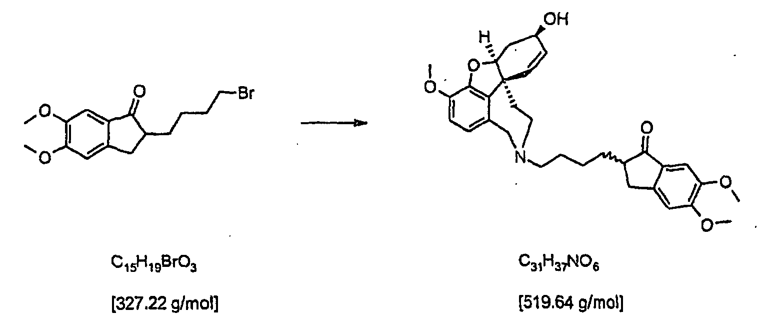

2-(4-Brombutyl)-5,6-dimethoxyindan-1-on (1.0 g, 3.01 mmol), Norgalanthamin (919 mg, 3.36 mmol) und Kaliumcarbonat (1.26 g, 9.09 mmol, wasserfrei, fein vermahlen) werden als in absolutem Acetonitril (10 mL) 24 Stunden bei Siedetemperatur gerührt.
Das Reaktionsgemisch wird filtriert, das Lösungsmittel abgezogen und der Rückstand säulenchromatographisch gereinigt (150 g Kieselgel, Chloroform : Methanol : Ammoniak : 96.5 : 3 : 0.5), wodurch das Produkt als farbloser Schaum erhalten wird (1.21 g, 2.32 mmol, 77.6 %)
- DC:: Chloroform : Methanol : Ammoniak: 89.5 : 10 : 0.5, Rf = 0.65
¹H NMR (CDCl₃): δ 7.14 (s, 1H), 6.83 (s, 1H), 6.67-6.52 (m, 2H), 6.12-5.90 (m, 2H), 4.57 (b, 1H), 4.02-4.18 (m, 2H), 3.93 (s, 3H), 3.87 (s, 3H), 3.80 (s, 3H), 3.75 (d, J=13.7 Hz, 1H), 3.43-3.06 (m, 3H), 2.75-2.35 (m, 5H), 2.11-1.83 (m, 3H), 1.59-1.29 (m, 6H);
¹³C NMR (CDCl₃): δ 207.5 (s), 155.4 (s), 149.3 (s), 148.9 (s), 145.7 (s), 144.0 (s), 133.1 (s), 129.4 (s), 127.5 (d), 126.9 (d), 121.9 (d), 111.1 (d), 107.3 (d), 104.2 (d), 88.6 (d), 62.0 (d), 57.7 (t), 56.1 (q), 56.0 (q), 55.8 (q), 51.5 (t), 51.2 (t), 48.3 (t), 47.5 (d), 32.8 (t), 32.5 (t), 31.5 (t), 29.9 (t), 27.4 (t), 25.1 (t)

### Beispiel 7:

### Stufe 1: 2-(5-Brompentyl)-5,6-dimethoxy-1-oxoindan-2-carbon-säuremethylester

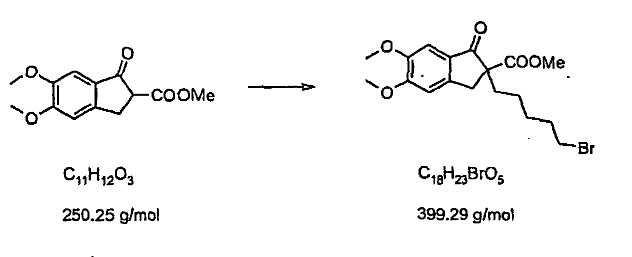

Zu einer Suspension von Natriumhydrid (0.62 g, 13.2 mmol, 50 % in Weißöl , durch Digerieren mit absolutem Petrolether (3 × 50 mL) vom Weißöl befreit) in absolutem DMF wird 5,6-Dimethoxy-1-oxoindan-2-carbonsäuremethylester (3.0 g, 12.0 mmol) bei Raumtemperatur in Substanz zugegeben und die Lösung 45 Minuten bei Raumtemperatur gerührt. Danach versetzt man mit 1,5-Dibrompentan (19.3 g, 84.0 mmol) und rührt 18 Stunden bei Raumtemperatur. Man verteilt zwischen Wasser und Ether, extrahiert die wäßrige Phase quantitativ mit Ether, wäscht die vereinigten organischen Phasen mit Wasser (5 x), gesättigter Kochsalzlösung (1 x) und trocknet (Natriumsulfat/Aktivkohle). Vom nach dem Eindampfen erhaltenen Rückstand *wird* überschüssiges Dibromalkan am Hochvakuum durch Kugelrohrdestillation (100 °C/0.05 mbar) abgetrennt und der erhaltene Rückstand aus siedendem *tert*.-Butylmethylether (20 mL) umkristallisiert, wodurch das Produkt in Form farbloser Kristalle (3.75 g, 9.4 mmol, 78.3 %) erhalten wird.
- DC:: Petrolether : Essigsäureethylester = 3 : 1; Rf = 0.15
- Schmp.:: 108.5 - 110 °C
¹H NMR (CDCl₃): δ 7.15 (s, 1H), 6.89 (s, 1H), 3.96 (s, 3H), 3.89 (s, 3H), 3.67 (s, 3H), 3.60 (d, J=19.1 Hz, 1H), 3.35 (t, J=7.0 Hz, 2H), 2.96 (d, J=19.1 Hz, 1H), 2.20-1.15 (m, 8H);
¹³C NMR (CDCl₃): δ 200.9 (s), 171.8 (s), 156.1 (s), 149.7 (s), 148.4 (s), 127.9 (s), 107.1 (d), 104.9 (d), 60.8 (s), 56.2 (q), 56.1 (q), 52.6 (q), 36.4 (t), 34.5 (t), 33.5 (t), 32.3 (t), 28.3 (t), 26.9 (d), 23.7 (t)

### Stufe 2:

### 2-(5-Brompentyl)-5,6-dimethoxyindan-1-on

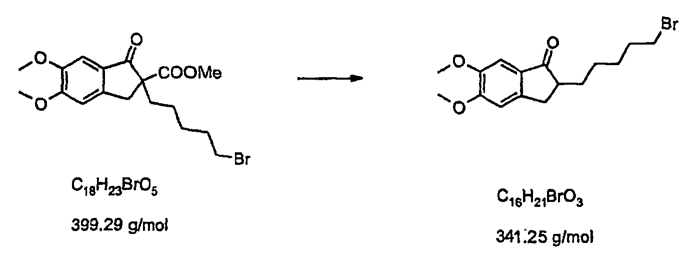

2-(5-Brompentyl)-5,6-dimethoxy-1-oxoindan-2-carbonsäuremethylester (3.0 g, 7.51 mmol) wird in konzentrierter Salzsäure (10 mL) und Essigsäure (30 mL) 12 Stunden bei 60 °C gerührt. Man versetzt mit gesättigter Natriumcarbonatlösung, neutralisiert mit Natriumcarbonat und extrahiert quantitativ mit Ether, wäscht die vereinigten organischen Phasen mit gesättigter Natriumcarbonatlösung (3 x), Wasser (1 x), gesättigter Kochsalzlösung (1 x), trocknet (Natriumsulfat/Aktivkohle) und kristallisiert den nach Eindampfen erhaltenen Rückstand aus *tert*.-Butylmethylether (10 mL) um. Auf diese Weise erhält man das Produkt in Form farbloser Kristalle (1.78 g, 5.22 mmol, 69.5 %)
- DC:: Petrolether : Essigsäureethylester = 3 : 1; Rf = 0.2
- Schmp.:: 67.5 - 68.5 °C
¹H NMR (CDCl₃): δ 7.15 (s, 1H), 6.85 (s, 1H), 3.95 (s, 3H), 3.89 (s, 3H), 3.50 (t, J=7.0 Hz, 2H), 3.20 (dd, J=6.4 Hz, J=9.5 Hz, 1H), 2.72 (d, J=3.2 Hz, 1H), 2.60 (d, J=3.2 Hz, 1H), 2.00-1.65 (m, 3H), 1.55-1.35 (m, 5H);
¹³C NMR (CDCl₃): δ 207.4 (s), 155.5 (s), 149.4 (s), 148.8 (s), 129.4 (s), 107.4 (d), 104.3 (d), 56.2 (q), 56.0 (q), 47.5 (d), 44.9 (t), 32.5 (t), 32.3 (t), 31.4 (t), 26.8 (t), 26.5 (t)

### Stufe 3:

### 2-[5-[(4aS,6R,8aS)-6-Hydroxy-3-methoxy-5,6,9,10,11,12-hexahydro-4aH-benzofuro[3a,3,2-e,f][2]benzazepin-11-yl]pentyl]-5,6-dimethoxyindan-1-on (SPH-1359)

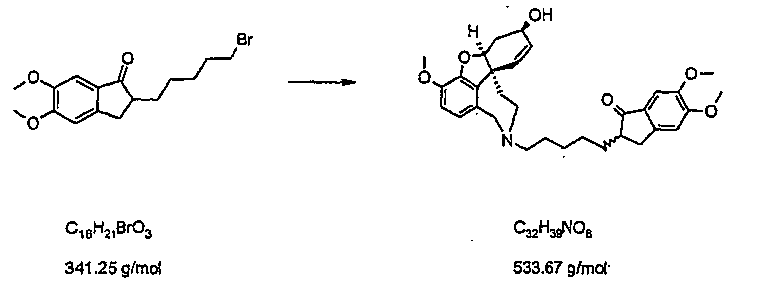

2-(4-Brompentyl)-5,6-dimethoxyindan-1-on (1.66 g, 4.86 mmol), Norgalanthamin (1.46 g, 5.35 mmol) und Kaliumcarbonat (2.01 g, 14.6 mmol, wasserfrei, fein vermahlen) werden als in absolutem Acetonitril (10 mL) 24 Stunden bei Siedetemperatur gerührt.

Das Reaktionsgemisch wird filtriert, das Lösungsmittel abgezogen und der Rückstand säulenchromatographisch gereinigt (150 g Kieselgel, Chloroform : Methanol: Ammoniak: 96.5 : 3 : 0.5), wodurch das Produkt als farbloser Schaum erhalten wird (1.84 g, 2.32 mmol, 70.9 %)
- DC:: Chloroform : Methanol : Ammoniak: 89.5 : 10 : 0.5, Rf = 0.65
¹H NMR (CDCl₃): δ 7.1 (s, 1H), 6.82 (s, 1H), 6.63-6.54 (m, 2H), 6.10-5.88 (m, 2H), 4.55 (b, 1H), 4.17-4.00 (m, 2H), 3.92 (s, 3H), 3.85 (s, 3H), 3.78 (s, 3H), 3.73 (d, J=13.7 Hz, 1H), 3.40-3.01 (m, 3H), 2.72-2.25 (m, 5H), 2.10-1.75 (m, 3H), 1.65-1.19 (m, 8H);
¹³C NMR (CDCl3): δ 207.4 (s), 155.3 (s), 149.2 (s), 148.8 (s), 145.6 (s), 143.8 (s), 133.0 (s), 129.4 (s), 129.3 (s), 127.4 (d), 126.9 (d), 121.8 (d), 111.0 (d), 107.2 (d), 104.2 (d), 88.5 (d), 77.2 (d), 61.9 (d), 57.6 (t), 56.0 (q), 55.9 (q), 55.7 (q), 51.4 (t), 48.2 (s), 47.5 (d), 32.9 (t), 32.4 (t), 31.5 (t), 29.8 (t), 27.2 (t), 27.1 (t)

### Herstellung des Fumarats

Eine Lösung der Base (1.00, 1.874 mmol) in gesättigter Fumarsäurelösung (6 mL, ca. 0.5 M in 95% Ethanol) wird bei ca. 60°C bis zum Erreichen einer klaren Lösung erwärmt und diese Lösung noch warm tropfenweise innerhalb von 5 min zu magnetisch gerührtem absolutem Ether (ca. 150 mL) zugefügt wobei ein weißer Niederschlag entsteht. Nach Stehen über Nacht bei Raumtemperatur werden die erhaltenen Kristalle abgenutscht und mit absol. Ether (3 × 50 mL) gewaschen und das Produkt im Vakuumexsiccator bei Raumtemperatur/50 mbar über Calciumchlorid getrocknet wobei das Fumarat in Form eines farblosen Pulvers (0.694, 57.0% d.Th.) erhalten wird. Eine Analysenmenge wird bei 2 mbar und 40°C 8 Stunden über Phosphorpentoxid getrocknet. Aus der Mutterlauge wird eine zweite Fraktion gewonnen.
C₃₂H₃₉NO₆. C₄H₄O₄.½ H₂O (658.7)

| | | | |
|---|---|---|---|
| Ber. | C 65.64 | H 6.73 | N 2.13 |
| Gef. | C 65.83 | H 6.72 | N 2.10 |

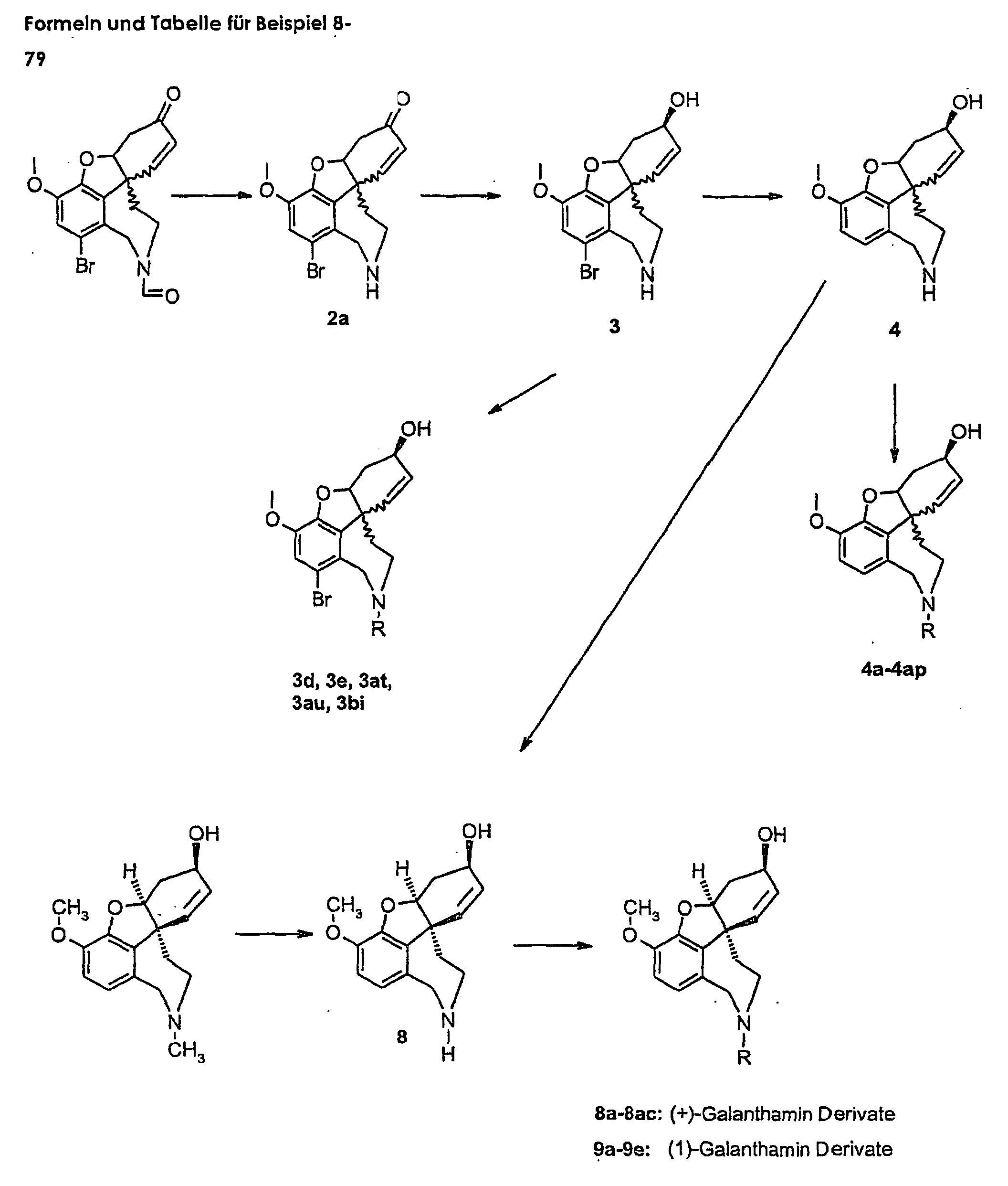

| **Beispiel** | **Nr.** | **SPH** | **BATCH LABCODE** | **R1** |
|---|---|---|---|---|
| 8 | 3bi | SPH-1218 | CB 30 | -C(SMe)=NCN |
| 9 | 4a | SPH-1229 | CB 52 | -pyrimidine-(2-yl) |
| 10 | 4b | SPH-1234 | CB 56 | -2-Cl-pyrimidine-(4-yl) |
| 11 | 4c | SPH-1245 | CB 59 | -2-NEt2-pyrimidine-(4yl) |
| 12 | 4d | SPH-1244 | CB 57 | -2-O(CH2)3NMe2-pyrimidine-(4-yl) |
| 13 | 4e | SPH-1230 | CB 53 | -4,6-Cl-1,3,5-triazine-(2-yl) |
| 14 | 4f | SPH-1243 | CB 58 | 4,6-di(NEt2)2-1,3,5-triazine-(2-yl) |
| 15 | 4g | SPH-1228 | CB 43 | -4,6-OPh-1,3,5-triazine-(2yl) |
| 16 | 4h | SPH-1233 | CB 51 | -4,6-di(O(CH2)2NH2-1,3,5-triazine-(2-yl) |
| 17 | 4i | SPH-1242 | CB 55 | 4,6-di(O(CH2)3NMe2-1,3,5-triazine-(2-yl) |
| 18 | 4j | SPH-1246 | MR 16 | -CO-CH2Cl |
| 19 | 4l | SPH-1214 | CB 34 | -CO-NHCH(Me)2 |
| 20 | 4m | SPH-1221 | CB 45 | -CO-NHC(Me)3 |
| 21 | 4n | SPH-1231 | CB 49 | -CONHEt |
| 22 | 4o | SPH-1222 | CB 46 | -CONH-cyclohexane |
| 23 | 4p | SPH-1215 | CB 33 | -CONHPh |
| 24 | 4q | SPH-1237 | CB 47 | -CONH-Ph(4-Cl) |
| 25 | 4r | SPH-1267 | CB 73 | -CO-NH-CH(Me)Ph, S-(-) |
| 26 | 4s | SPH-1232 | CB 50 | -CONH-2-naphthatine |
| 27 | 4t | SPH-1211 | CB 13 | -CSNHMe |
| 28 | 4u | SPH-1236 | CB 48 | -CSNHCH2CH=CH2 |
| 29 | 4v | SPH-1259 | HM 59 | -C(COOMe)=CHCOOMe |
| 30 | | SPH-1196 | TK 36-2 | -(CH2)3-(2-(4-F-phenyl)-2,5-diazabicyclo[2.2.1]heptane)-5-yl) |
| 31 | 4x | SPH-1219 | CB 36 | -CH=C(CN)2 |
| 32 | 4y | SPH-1278 | HM 60 | CH=C(COOMe)2 |
| 33 | 4z | SPH-1264 | HM 58 | -CH=CHCOCH2OEt |
| 34 | 4ac | SPH-1248 | MR 7 | -CH2-COOEt |
| 37 | 4af | SPH-1116 | Ja 6-2 | -(CH2)2-NH2 |
| 40 | 4al | SPH-1217 | CB 28 | -(CH2)2-COOEt |
| 41 | 4aj | SPH-1277 | HM 57 | -(CH2)2-COOC(Me)3 |
| 42 | 4ak | SPH-1262 | MR 14 | -(CH2)2-CONHCHMe2 |
| 43 | 4ab | SPH-1102 | TK 72/5 | -CH2-CH=CH2 |
| 43 | 4al | SPH-1249 | MR 13 | -(CH2)2-CONHCMe3 |
| 44 | 4am | SPH-1216 | CB 35. | -(CH2)2-CN |
| 45 | 4an | SPH-1220 | CB 41 | -(CH2)3-OH |
| 46 | 4aa | SPH-1103 | TK 74/3 | -Bn |
| 46 | 4ao | SPH-1235 | CB 42 | -(CH2)3-NH2 |
| 47 | 4ap | SPH-1107 | TK 94/3 | -(CH2)3-N-plperidine |
| 48 | 8a | SPH-1260 | CB 98 | -Ph |
| 49 | 8b | SPH-1282 | CB 100 | -thiophene-2yl |
| 50 | 8c | SPH-1327 | WO 2 | -(N-benzoyl)-4-piperidine |
| 51 | 8e | SPH-1296 | CB 147 | -COOPh |
| 52 | 8f | SPH-1328 | CB 161 | -C(=S)OPh |
| 53 | 8g | SPH-1292 | CB 112 | -Fmoc |
| 54 | 8h | SPH-1326 | CB 171 | -CO-(CH2)2-CH=CH2 |
| 55 | 8i | SPH-1268 | CB 78 | -CONH2 |
| 56 | 8j | SPH-1287 | HM 109 | -CSNHMe |
| 57 | 8k | SPH-1269 | CB 85 | -CO-NHCH(Me)2 |
| 58 | 8l | SPH-1270 | CB 86 | -CO-NHC(Me)3 |
| 59 | 8m | SPH-1266 | CB 75 | -CONH-Ph(2-CF3) |
| 60 | 8n | SPH-1272 | CB 81 | -C(SMe)=NCN |
| 61 | 8o | SPH-1289 | HM 117 | -CH2-cyclopropane |
| 63 | 8r | SPH-1295 | BM 1 | -CH2-CN |
| 64 | 8s | SPH-1314 | DD 18 | -CH2-CO-(2-phenyl-2,5-diazabicyclo[2.2.1]heptane)-5-yl) |
| 65 | 8i | SPH-1311 | BM 4 | -(CH2)2-NH2 |
| 66 | 8u | SPH-1117 | Ro21 CB120 | -(CH2)2-N-morpholine |
| 67 | 8v | SPH-1329 | DD 26 | -(CH2)2-(2-phenyl-2,5-diazabicyclo[2.2.1]heptane)-5-yl) |
| 68 | 8w | SPH-1276 | CB 89 | -(CH2)2-COOH |
| 69 | 4ae | SPH-1096 | TK 81/3 | -(CH2)2-OH |
| 69 | 8x | SPH-1271 | CB 87 | -(CH2)2-COOC(Me)3 |
| 70 | 8z | SPH-1315 | | -(CH2)3-OH |
| 71 | 8aa | SPH-1213 | TK 96/3 | -(CH2)3-NMe2 |
| 73 | 4ad | SPH-1099 | TK 80-3 | -CH2-CN |
| 73 | 8ac | SPH-1312 | DD24 | -(CH2)3-(2-(4-F-phenyl)-2,5-diazabicyclo[2.2.1]heptane)-5-yl) |
| 74 | 4ag | SPH-1098 | Ro 11 | -(CH2)2-N-morpholine |
| 75 | 9a | SPH-1284 | DD 10 | -CO-NHCH(Me)2 |
| 76 | 9b | SPH-1283 | DD 9 | -CO-NHC(Me)3 |
| 77 | 9c | SPH-1118 | Ro 22 | -(CH2)2-N-morpholine |
| 78 | 9d | SPH-1330 | RMA-15 | -(CH2)3-NMe2 |
| 79 | 9e | SPH-1333 | RMA 14 | -(CH2)3-N-piperidine |

### Beispiel 8:

### Schritt 1

### (6R)-1-Bromo-3-methoxy-5,6,9,10,11,12-hexahydro-4aH-[1]benzofuro[3a,3,2-ef[2]benzazepin-6-on (2a)

Zu einer gerührten Lösung von (6R)-5,6,9,10,11,12,1-bromo-3-methoxy-6-oxo-4aH-hexahydrobenzofuro[3a,3,2-ef][2]benzazepin-11(12H)-carboxaldehyd (2) (100.0 g, 0.26 mol) in Toluol (2.6 l) Wasser (660 ml) und konzentrierter Salzsäure (400 ml) wurden hinzugefügt. Das Reaktionsgemisch wurde unter Rühren 48 Stunden lang rückflußgekocht. Der Niederschlag wurde abfiltriert mit Wasser (3 × 500 ml). gewaschen. Die Phasen des Hydrates wurden getrennt und die organische Phase mit Wasser (3 × 500 ml) extrahiert. Der Niederschlag wurde mit den vereinigten, wäßrigen Lösungen des Hydrates erhitzt und heißfiltriert. Die Lösung wurde mit 30%igem Natriumhydroxid auf pH = 12 eingestellt. Der Niederschlag wurde filtriert und getrocknet (50°C/50 mbar), um 64.5 g (70%) der Titelverbindung (2a), um einen Schmelzpunkt von 228-231°C zu erhalten. ¹H-NMR(CDCl₃ δ 6.94 (dd, J₁ = 10.3, 1.9 Hz, 1H), 6.62 (s, 1H), 6.00 (d, J = 10.5 Hz, 1H), 4.69 (m, 1H), 4.04 (d, J = 15.9 Hz, 1H), 3.83 (d, J = 15.9 Hz, 1H), 3.80 (s, 3H), 3.29 (m, 2H), 3.07 (d, J = 1.9 Hz, 1H), 2.70 (dd, J₁ = 17.8 Hz, J₂ = 3.7 Hz, 1H), 2.16 (m, 1H), 1.80 (dt, J₁ = 14.0 Hz, J₂ = 2.9 Hz, 1H); ¹³C-NMR (CDCl₃) δ 194.3 (s), 146.9 (s), 143.8 (s), 135.3 (d), 130.6 (s), 129.3 (s), 126.9 (d), 121.9 (d), 111.8 (s), 87.9 (d), 56.3 (t), 55.9 (q), 51.8 (t), 49.0 (s), 37.2 (t), 33.0 (t). Ana). (C₁₆H₁₆BrNO₃.O.4 H₂O) C,H,N.

### Schritt 2:

### (6R)-1-Bromo-3-methoxy-5,6,9,10,11,12-hexahydro-4aH-[1]benzofuro[3a,3,2-ef[2]benzazepin-6-ol (3)

Zu einer Lösung von (6R)-1-Bromo-3-methoxy-5,6,9,10,11,12-hexahydro-4aH[1]benzofuro[3a,3,2-ef][2]benzazepin-6-on (2a) (64.5 g, 0.184 mol) in trockenem THF (1.3 I) wurde eine L-Selektridlösung (1 M, 276 ml, 0.276 mol) bei -10°C zugegeben. Nach 30-minütigem Rühren bei -10 bis -5°C wurde das Reaktionsgemisch mit MeOH (80 ml) hydrolysiert und eingeengt. Der erhaltene Rückstand wurde in 2 N-Salzsäure aufgelöst und 18 h bei Raumtemperatur gerührt. Die Lösung wurde mit konzentriertem Ammoniak auf pH = 9 eingestellt und mit EtOAc (3 × 500 ml) extrahiert, die vereinigten, organischen Schichten wurden mit Brine gewaschen und getrocknet (Na₂SO₄), um 55.9 g (90.6%) des Produktes zu ergeben. ¹H-NMR (CDCl₃) δ 6.85 (s, 1H), 6.05 (m, 2H), 4.56 (b, 1H), 4.48 (d, J = 14.7 Hz, 1H), 4.10 (m, 1H), 3.85 (d, J = 14.7 Hz, 1H), 3.80 (s, 3H), 3.35-3.05 (m, 2H), 2.62 (m, 1H), 2.25 (m, 1H), 1.98 (d, J = 13.2 Hz, 1H), 1.85-1.65 (m, 2H); ¹³C-NMR (CDCl₃) δ 145.8 (s), 144.0 (s), 134.1 (s), 131.6 (s), 127.9 (d), 126.8 (d), 115.5 (d), 113.0 (s), 88.4 (d), 61.7 (d), 56.0 (q), 52.7 (t), 49.3 (s), 46.6 (t), 29.7 (t).

### Schritt 3:

### (6R)-3-Methoxy-5,6,9,10,11,12-hexahydro-4aH-[1]benzofuro[3a,3,2-ef][2]benzazepin-6-ol (= (+/-Norgalanthamin) (4)

Zu einer Lösung von (6R)-1-Bromo-3-methoxy-5,6,9,10,11,12-hexahydro-4aH-[1]benzofuro[3a,3,2-ef][2]benzazepin-6-ol (3) (20.0 g, 56.8 mmol) in 50% EtOH (1000 ml) wurde aktiviertes Zink (89.0 g, 1.36 mol) und Kalziumchlorid (44.0 g, 0.40 mol) hinzugegeben. Das Reaktionsgemisch wurde 18 h lang rückflußgekocht und über Zelite filtriert. Das Filtrat wurde eingeengt, der Rückstand mit 2N-Salzsäure (500 ml) verdünnt und mit EtOAc (3 x 400 ml) extrahiert. Der pH-Wert der wäßrigen Phase wurde mit konzentriertem Ammoniak auf über 8.5 eingestellt und mit CH₂Cl₂ (3 × 100 ml) und mit CH₂Cl₂:MeOH = 9:1 (3 × 100 ml) extrahiert. Die vereinigten, organischen Extrakte wurden mit Brine (200 ml) gewaschen, getrocknet (Na₂SO₄) und eingeengt, um 12.3 g (79.0%) der Verbindung 4 zu ergeben: ¹H-NMR (CDCl₃) δ 6.62 (b, 2H), 6.02 (m, 2H), 4.61 (b, 1H), 4.14 (t, J = 4.3 Hz, 1H), 3.98 (d, J = 5.0 Hz, 2H), 3.83 (s, 3H), 3.30 (m, 1H), 2.69 (t, J = 15.7, 1H), 2.10-1.63 (m, 4H); ¹³C-NMR(CDCL₃) δ 146.2 (s), 144.1 (s), 133.1 (s), 131.7 (s), 127.8 (d), 126.8 (d), 120.8 (d), 111.1 (d), 88.4 (d), 61.9 (d), 55.9 (q), 53.3 (t), 48.5 (s), 46.7 (t), 39.4 (t), 29.9 (t). Anal. (C₂₀H₂₆N₂O₄)C,H,N.

### Schritt 4:

### Methyl (6R)-1-Bromo-N¹¹-cyano-6-hydroxy-3-methoxy-5,6,9,10-tetrahydro-4aH[1]benzofuro[3a,3,2-ef][2]benzazepine-11(12H)carboximidothioat (3bi)

Zu einer Lösung von (+/-)-Norgalanthamin (0.5 g, 1.4 mmol) in EtOH:DMF = 4:1 (20 ml) wurden 0.21 g, 1.4 mmol) N-cyanodithiocarbonimidicsöuredimethylester (0.21 g, 1.4 mmol) hinzugefügt. Das Reaktionsgemisch wurde 4 Tage rückflußgekocht und eingeengt. Der Rückstand wurde in EtOH kristallisiert, um 0.25 g (41.7%) der Verbindung 3bi zu ergeben: ¹H-NMR(CDCl₃): δ 6.90 (s, 1H), 6.05 (dd, J₁ = 10.3 Hz, J₂ = 5.0 Hz, 1H), 5.86 (d, J = 10.3 Hz, 1H), 5.62 (d, J = 16.5 Hz, 1H), 4.62 (b, 1H), 4.36 (d, J = 16.5, 1H), 4.14 (m, 1H), 3.83 (s, 3H), 3.79 (m, 1H), 2.96 (d, J = 15.3 Hz, 1H), 2.77 (s, 3H), 2.68 (m, 1H), 1.92 (m, 3H); ¹³C-NMR (CDCl₃): δ 146.3 (s), 145.0 (s), 132.7 (s), 129.0 (s), 125.4 (d), 125.2 (d), 125.2 (s), 116.0 (d), 114.3 (d), 88.0 (d), 61.3 (d), 56.1 (q), 55.0 (t), 49.6 (t), 48.6 (s), 29.4 (t), 16.1 (q). Anal. (C₁₉H₂₀BrN₃O₃S.0.85 EtOH) C,H,N.

### Beispiel 9:

### (6R)-3-Methoxy-11-(2-pyrimidinyl)-5,6,9,10,11,12-hexahydro-4aH[1]benzofuro[3a,3,2-ef[2]benzazepin-6-ol (4a)

Zu einer Lösung von (+/-)-Norgalanthamin (0.5 g 1.8 mmol) in EtOH (30 ml) wurden 0.21 g, 1.8 mmol) 2-Chlorpyrimidin und Natriumhydrogenkarbonat (0.61 g, 7.2 mmol) hinzugegeben. Das Reaktionsgemisch wurde 2 Tage rückflußgekocht und konzentriert. Der Rückstand wurde mit Wasser (30 ml) verdünnt und EtOAc (3 × 20 ml) extrahiert. Die vereinigten, organischen Extrakte wurden mit Kochsalzlösung gewaschen (20 ml), getrocknet (Na₂SO₄) und eingeengt, um 0.51 g (80.8%) von 4a zu ergeben: ¹H-NMR(DMSO-d) δ 7.82 (d, J = 4.0 Hz, 2H), 6.42 (d, J = 12.0 Hz 1H), 6.23 (d, J = 12.0 Hz, 1H), 6.03 (t, J = 4.0 Hz, 1H), 5.83 (d, J = 8.0 Hz, 1H), 5.54 (dd, J₁ = 8.0 Hz, J₂ = 3.0 Hz, 1H), 4.98 (d, J = 14.0 Hz, 1H), 428 (d, J = 16.0, 1H), 4.09 (b, 1H), 3.94 (d, J = 14.0 Hz, 1H), 3.72 (m, 1H), 3.38 (s, 3H), 3.21 (t, J = 14.0 Hz, 1H), 2.54 (d, J = 12.0 Hz, 1H), 2.15 (m, 1H), 1.55 (m, 3H), ¹³C-NMR(DMSO-d) δ 159.5 (s), 156.7 (2), 145.5 (s), 142.8 (s), 131.6 (s), 128.9 (s), 126.8 (d), 126.1 (d), 120.8 (d), 109.9 (d), 108.8 (d), 86.8 (d), 61.3 (d), 54.8 (q), 50.2 (t), 47.3 (s), 47.4 (t), 34.6 (t), 29.2 (t). Anal. (C₂₀H₂₁N₃O₃.0.15 EtOH) C,H,N.

### Beispiel 10:

(6R)-11-(2-Chloro-4-pyrimidinyl)-3-methoxy-5,6,9,10,11,12-hexahydro-4aH-[1]benzofuro[3a,3,2,ef][2]benzazepin-6-ol (4b) wurde analog Beispiel 9 hergestellt. Reaktionszeit 40 h, Ausbeute 0.62 g (88.6%). ¹H-NMR(CDCl₃) δ 7.84 (d, J = 4.0 Hz, 1H), 6.88 (d, J = 12.0 Hz, 1H), 6.69 (d, J = 12.0 Hz, 1H), 6.05 (b, 2H), 5.90 (d, J = 4.0 Hz, 1H), 5.58 (b, 1H), 4.34 (m, 2H), 4.18 (b, 1H), 3.80 (s, 3H), 3.60 (t, J = 16.0 Hz, 1H), 2.73 (d, J = 16.0 Hz, 1H), 2.39 (m, 1H), 2.04 (d, J = 18 Hz, 1H), 1.87 (m, 2H); ¹³C-NMR(CDCl₃) δ 160.5 (s), 158.4 (s), 157.0 (s), 145.0 (s), 144.1 (s), 132.2 (s), 128.2 (d), 127.8 (s), 126.6 (d), 126.1 (d), 111.0 (d), 107.1 (d), 88.1 (d), 61.6 (d), 55.8 (q), 53.8 (t), 48.3 (s), 46.0 (t), 34.9 (t), 29.6 (t).

### Beispiel 11:

### (6R)-11-(2-Diethylamino)-4-pyrimidinyl)3-methoxy-5,6,9,10,11,12-hexahydro-4aH-[1]benzofuro[3a,3,2-ef][2]benzazepin-6-ol (4c).

Zu einer Lösung von Verbindung 4b (0.5 g, 1.30 mmol) in Diethylamin (6 ml, 57.6 mmol) wurde 0.1 g (1.30 mmol) Kaliumhydroxyd hinzugegeben. Das Reaktionsgemisch wurde 22 h rückflußgekocht und eingeengt. Der Rückstand wurde mit gesättigter Lösung von Kaliumkarbonat (30 ml) verdünnt und mit EtOAc (3 × 20 ml) extrahiert. Die vereinigten, organischen Phasen wurden mit Kochsalzlösung gewaschen (20 ml), getrocknet (Na₂SO₄) und eingeengt. Flashchromatographie ergab 0.21 g (38.5%) von 4c. Nachstehend sind nur die unterschiedlichen NMR-Signale beschrieben: ¹H-NMR(CDCL₃) δ 2.97 (d, J = 16.0 Hz, 4H), 1.34 (m, 6H); ¹³C-NMR(CDCl₃) δ 36.3 (t), 14.0 (q).

### Beispiel 12:

### (6R)-11-(2-(3-(Dimethylamino)propoxy)-4-pyrimidinyl)-3-methoxy-5,6,9,10,11,12-hexahydro-4aH-[1]benzofuro[3a,3,2-ef][2]benzazepin-6-ol (4d): Die Herstellung erfolgte analog Beispiel 11. Reaktionszeit 2 h, Ausbeute 0.16 g (41.0%). Es werden nur die unterschiedlichen NMR-Signale beschrieben: ¹H-NMR (CDCl₃) δ 4.34 (m, 2H), 2.28 (s, 6H), 2.00 (m, 4H); ¹³C-NMR(CDCl₃) 65.0 (t), 56.3 (t), 45.2 (q), 27.0 (t).

### Beispiel 13:

(6R)-11-[4,6-Dichloro-1,3,5-triazin-2-yl)-3-methoxy-5,6,9,10,11,12-hexahydro-4aH-[1]benzofuro[3a,3,2-ef][2]benzazepin-6-ol (4e): Eine Lösung von 2,4,6-trichloro-1,3,5-triazin (0.66 g, 3.7 mmol) in Aceton (16 ml) wurde auf Eiswasser (35 ml) gegossen und bei 0°C (+/-)-Norgalanthamin (1.0 g, 3.7 mmol) in kleinen Teilen zugegeben. Nach Zugabe von 2N-Natriumhydroxyd (2ml) wurde das Reaktionsgemisch 40 h lang rückflußgekocht. Die wäßrige Phase wurde mit EtOAc (3 x 30 ml) extrahiert. Die vereinigten, organischen Phasen wurden mit Kochsalzlösung (30 ml) gewaschen, getrocknet (Na₂SO₄) und eingeengt, um 0.90 g (59.5%) der Verbindung 4e zu ergeben: ¹H-NMR(CDCl₃) δ 6.82 (d, J = 10.0 Hz, 1H), 6.63 (d, J = 10.0 Hz, 1H), 6.02 (b, 2H), 5.30 (d, J = 11.0 Hz, 1H), 4.75 (d, J = 16.0, 1H), 4.50 (b, 1H), 4.22 (d, J = 11.0 Hz, 1H), 4.11 (b, 1H), 3.78 (s, 3H), 3.59 (m, 1H), 3.06 (m, 1H), 2.61 (d, J = 16.0 Hz, 1H), 1.90 (m, 3H); ¹³C-NMR(CDCl₃) δ 207.0 (s) 171.2 (s), 163.7 (s), 146.2 (s), 143.9 (s), 132.3 (d), 129.5 (s), 127.6 (s), 126.7 (d), 121.5 (d), 110.8 (d), 88.0 (d), 61.7 (d), 55.7 (q), 51.8 (t), 48.2 (s), 43.4 (t), 35.9 (t), 29.7 (t).

Die Verbindungen 4f - 4i enthalten das grundlegende Galanthamingerüst wie 4e, unterscheiden sich aber im Stickstoffsubstituenten. Da das Proton und Kohlenstoffsignal des Galanthaminkems sich nicht wesentlich unterscheiden, werden nachstehend die NMR-Signale des Stickstoffsubstituenten wiedergegeben.

### Beispiel 14:

(6R)-11-(4,6-Bis-(diethylamino-1,3,5-triazin-2-yl)-3-methoxy-5,6,9,10,11,12-hexahydro-4aH-[1]benzofuro[3a,3,2-ef][2]benzazepin-6-ol (4f): Eine Lösung von Verbindung 4e (0.30 g, 0.71 mmol) in 40 ml Aceton wurde auf 100 ml Eiswasser gegossen und bei 0°C eine Lösung von Diethylamin (5.7 ml, 54.7 mmol) in Aceton (10 ml) hinzugegeben. Das Reaktionsgemisch wurde 2 h lang rückflußgekocht und dann auf 200 ml Eiswasser gegossen. Die wäßrige Phase wurde mit 3 × 100 ml EtOAc extrahiert. Die vereinigten organischen Phasen wurden mit Kochsalzlösung (100 ml) gewaschen, über Natriumsulfat getrocknet und eingeengt. Flashchromatographie ergab 0.17 g (47.8%) der Verbindung 4f: ¹H-NMR(CDCl₃) δ 3.54 (m, 8H), 1.18 (m, 12H); ¹³C-NMR(CDCl₃) δ 41.7 (t), 13.4 (q).

### Beispiel 15:

(6R)-11-(4,6-Diphenoxy-1,3,5-triazin-2-yl)-3-methoxy-5,6,9,10,11,12-hexahydro-4aH-[1]benzofuro[3a,3,2-ef][2]benzazepin-6-ol (4g): Zu einer Lösung von (+/-)-Norgalanthamin (1.0 g, 3.74 mmol) in Dioxan (60 ml) wurden 6.5 g (18.3 mmol) 2,4,6-triphenoxy-1,3,5-triazin zugegeben. Das Reaktionsgemisch wurde 20 h lang rückflußgekocht. Der Niederschlag wurde abfiltriert und mit Dioxan gewaschen. Das Filtrat wurde eingeengt und Flashchromatographie ergab 0.91 g (45.9%) der Verbindung 4g: ¹H-NMR(CDCl₃) δ 7.42-7.03 (m, 10H); ¹³C-NMR (CDCl₃) δ 172.1 und 162.3 (s), 138.3 und 138.1 (d), 134.6 (d), 131.3 und 130.8 (d).

### Beispiel 16:

(6R)-11-(4,6-Bis-(2aminoethoxy)-1,3,5-triazin-2-yl)-3-methoxy-5,6,9,10,11,12-hexahydro-4aH-[1]benzofuro[3a,3,2-ef][2]benzazepin-6-ol (4H): Nach der in Beispiel 11 angegebenen Arbeitsweise wurde die Verbindung hergestellt, wobei die Reaktionszeit 3 h betrug. Man erhielt 0.15 g (67.9%) der Verbindung 4h. ¹H-NMR (CDCl₃) δ 3.64 (m, 4H), 3.42 (m, 4H); ¹³C-NMR (CDCl₃) δ 61.3 (t), 42.1 (t).

### Beispiel 17:

(6R)-11-(4,6-Bis-(2-(dimethylamino)ethoxy)-1,3,5,-triazin-2-yl)-3-methoxy-5,6,9,10,11,12-hexahydro-4aH-[1]benzofuro[3a,3,2-ef][2]benzazepin-6-ol (4i). Nach der in Beispiel 11 angegebenen Arbeitsweise wurde bei einer Reaktionszeit von 3 h die Verbindung 4i in einer Ausbeute von 0.16 g (59.5%) erhalten: ¹H-NMR (CDCl₃) δ 4.12 (q, J = 6.0 Hz, 4H), 2.29 (d, J = 4.0 Hz, 12H), 1.29 (m, 8H); ¹³C-NMR (CDCl₃) δ 65.6 (t), 56.0 (t), 45.2 (q), 29.2 (t).

### Beispiel 18:

2-Chloro-1-((6R)-6-hydroxy-3-methoxy-5,6,9,10-tetrahydro-4aH-[1]benzofuro[3a,3,2-ef][2]benzazepin-11(12H)-yl)-1-ethanon (4j): Zu einer Lösung von (+/-)-Norgalanthamin (2.0 g, 7.3 mmol) in trockenem TRF (100 ml) wurden 0.82 g Chloracetylchlorid (7.3 mmol) und 0.81 g (8.0 mmol) Triethylamin zugegeben. Das Reaktionsgemisch wurde 3 h lang rückflußgekocht und eingeengt. Der Rückstand wurde mit 2N Salzsäure (100 ml) verdünnt und mit EtOAc (3 × 75 ml) extrahiert. Die wäßrige Phase wurde auf pH > 8.5 mit konzentriertem Ammoniak eingestellt und mit 3 × 75 ml CH₂Cl₂ extrahiert. Die vereinigten, organischen Phasen wurden mit Kochsalzlösung gewaschen, über Natriumsulfat getrocknet und eingeengt. Flashchromatographie ergab 0.20 g (7.7% der Verbindung 4j): ¹H-NMR (CDCl₃) δ 6.78 (b, 1H), 6.12 (m, 2H), 5.30 (d, J = 11.0 Hz, 1H), 4.65 (m, 2H), 4.32-4.01 (m, 3H), 3.78 (s, 3H), 3.59 (m, 1H), 3.06 (m, 1H), 2.61 (d, J = 16.0 Hz, 1H), 1.90 (m, 3H), ¹³C-NMR (CDCl₃) δ 166.0 (s) 146.2 (s), 144.9 (s), 132.3 (d), 128.3 (s), 127.3 (s), 126.0 (d), 120.2 (d), 111.2 (d), 88.2 (d), 61.7 (d), 55.8 (q), 52.8 (t), 48.1 (s), 45.5 (t), 41.4 (t), 35.4 (t), 29.6 (t).

### Beispiel 19:

(6R)-6-Hydroxy-N¹¹-isopropyl-3-methoxy-5,6,9,10-tetrahydro-4aH-[1]benzofuro[3a,3,2-ef[2]benzazepin-11(12H)-carboxamid (41): Nach der in Beispiel 11 angegebenen Arbeitsweise wurde bei einer Reaktionszeit von 4 h 0.50 g der Verbindung 4h mit einem Schmelzpunkt von 106-108°C erhalten: ¹H-NMR (CDCl₃) δ 6.68 (dd, J = 10.3; 8.3 Hz, 2H), 6.00 (m, 2H), 4.59 (b, 1H), 4.47 (d, J = 16.4 Hz, 1H), 4.31 (d, J = 16.4, 1H), 4.16 (m, 1H), 3.86 (m, 1H), 3.83 (s, 3H), 3.36 (dt, J = 12.6; 2.0 Hz 1H), 2.69 (dd, J 15.7; 3.4 Hz, 1H), 2.28 (d, J = 11.3 Hz, 1H), 2.02 (m, 1H), 1.88 (dd, J = 12.3; 3.4 Hz, 1H), 1.77 m, 1H), 1.07 (dd, J = 21.8; 6.4 Hz, 6H); ¹³C-NMR (CDCl₃) δ 156.4 (s), 146.8 (s), 144.5 (s), 132.4 (s), 129.0 (s), 127.9 (d), 126.4 (d), 120.1 (d), 111.0 (d), 88.3 (d), 61.7 (d), 55.8 (q), 51.5 (t), 48.3 (s), 45.4 (t), 42.4 (d), 36.4 (t), 29.7 (t), 23.3 (q), 23.1 (q). Anal. (C₂₀H₂₆N₂O₄), C,H,N.

Die Verbindungen 4m - 4s enthalten das Galanthamingrundgerüst wie 41, unterscheiden sich aber hinsichtlich des Stickstoffsubstituenten. Da die Proton- und Kohlenstoffsignale des Galanthaminkerns sich nicht wesentlich voneinander unterscheiden, sind nur die Signale des Stickstoffsubstituenten nachstehend wiedergegeben.

### Beispiel 20:

(6R)-N¹¹-t-Butyl-6-hydroxy-3-methoxy-5,6,9,10-tetrahydro-4aH-[1]benzofuro[3a,3,2-ef[2]benzazepine-11(12H)-carboxamide (4m): Verfahren gemäß Beispiel 11, Reaktionszeit 3 h, Ausbeute 0.57 g (85%); Schmelzpunkt 204-205°C; ¹H-NMR (CDCl₃) δ 1.24 (s, 9H); ¹³C-NMR (CDCl₃) 156.4 (s), 50.7 (s), 29.3 (q). Anal. (C₂₁H₂₈N₂O₄) C,H,N.

### Beispiel 21:

(6R)-N¹¹-Ethyl-6-hydroxy-3-methoxy-5,6,9,10-tetrahydro-4aH-[1]benzofuro[3a,3,2-ef[2]benzazepine-11(12H)-carboxamide (4n): Verfahren gemäß Beispiel 11, Reaktionszeit 3 h, Ausbeute 0.61 g (98%); Schmelzpunkt 137-139°C; ¹H-NMR (CDCl₃) δ 3.14 (q, J = 4.0 Hz, 2H), 1.04 (t, J = 10 Hz, 3H); ¹³C-NMR (CDCl₃) 157.0 (s), 35.6 (t), 15.3 (q).

### Beispiel 22:

(6R)-N¹¹-Cyclohexyl-6-Hydroxy-3-methoxy-5,6,9,10-tetrahydro-4aH-[1]benzofuro[3a,3,2-ef][2]benzazepine-11-(12H)-carboxamide (4o): Verfahren gemäß Beispiel 11, Reaktionszeit 5 h, Ausbeute 0.56 g (79%); Schmelzpunkt 225-228°C; ¹H-NMR (CDCl₃) δ 1.24 (s, 9H); ¹³C-NMR (CDCl₃) δ 48.8 (d), 33.4 (t), 33.2 (t), 25.5 (t), 24.8 (t), 24.6 (t).

### Beispiel 23:

(6R)-6-Hydroxy-3-methoxy-N¹¹-phenyl-5,6,9,10-tetrahydro-4aH-[1]benzofuro[3a,3,2-ef][2]benzazepine-11-(12H)-carboxamide (4p): Verfahren gemäß Beispiel 11, Reaktionszeit 4 h, Ausbeute 0.34 g (47%); Schmelzpunkt 198-199°C; ¹H-NMR (CDCl₃) δ 7,24 (m, 4H), 6.99 (q, J = 4.2 Hz, 1H); ¹³C-NMR (CDCl₃) δ 154.5 (s), 138.7 (s), 128.7 (d), 122.9 (d), 119.7 (d). Anal. (C₂₃H₂₄N₂O₄.H₂O) C, H. N.

### Beispiel 24:

(6R)-N¹¹-4-Chlorophenyl-6-hydroxy-3-methoxy-5,6,9,10-tetrahydro-4aH-[1]benzofuro[3a,3,2-ef][2]benzazepine-11-(12H)-carboxamide (4q): Verfahren gemäß Beispiel 11, Reaktionszeit 5 h, Ausbeute 0.16 g (21%); ¹H-NMR (CDCl₃) δ 17.49-6.94 (m, 4H); ¹³C-NMR δ (CDCl₃) 154.1 (s), 139.1 (s), 123.4 (s), 122.9 (d), 119.7 (d).

### Beispiel 25:

(6R)-6-Hydroxy-3-methoxy-N¹¹-(S)-(-)α-methylbenzyl-5,6.9,10-tetrahydro-4aH-[1]benzofuro[3a,3,2-ef][2]benzazepine-11-(12H)-carboxamide (4r): Verfahren gemäß Beispiel 11, Reaktionszeit 6 h, Ausbeute 0.66 g (58%); ¹H-NMR (CDCl₃) δ 7.21 (d, J = 6.0 Hz, 4H), 7.17 (m, 1H), 4.91 (m, 1H), 1.41 (dd, J = 20.0; 12 Hz, 3H); ¹³C-NMR (CDCl₃) δ 156.6 und 165.4 (s), 144.5 (s), 128.3 und 128.1 (d), 126.5 und 126.4 (d), 125.9 und 125.5 (d), 46.1 (d), 22,9 und 22.6 (q).

### Beispiel 26:

(6R)-6-Hydroxy-3-methoxy-N¹¹-(S)-(-)α-methylbenzyl-5,6,9,10-tetrahydro-4aH-[1]benzofuro[3a,3,2-ef][2]benzazepine-11-(12H)-carboxamide (4s): Verfahren gemäß Beispiel 11, Reaktionszeit 6 h, Ausbeute 0.66 g (58%); ¹H-NMR (CDCl₃) δ 7.82 (d, J = 8.0 Hz, 1H), 7.61 (d, J = 6.0 Hz, 2H), 7.43 (m, 4H); ¹³C-NMR(CDCl₃) δ 155.4 (s), 134.0 (s), 133.6 (s), 132.5 (s), 128.6 (d), 127.9 (d), 125.9 (d), 125.7 (d), 125.6 (d), 125.6 (d), 121.1 (d).

### Beispiel 27:

(6R)-6-Hydroxy-3-methoxy-N¹¹-methyl-5,6,9,10-tetrahydro-4aH-[1]benzofuro[3a,3,2-ef][2]benzazepine-11-(12H)-carboxamide (4t): Verfahren gemäß Beispiel 11, Reaktionszeit 3 h, Ausbeute 0.57 g (99%); Schmelzpunkt 219-221 °C; ¹H-NMR (CDCl₃) δ 6.81 (d, J = 8.3 Hz, 1H), 6.71 (d, J = 8.1 Hz, 1H), 6.12 (d, J = 10.3 Hz, 1H), 5.81 (dd, J = 10.2; 4.4 Hz), 1H), 5.21 (d. J = 15.8 Hz, 1H), 4.44 (s, 1H), 4.25 (d, J = 5.5, 1H), 4.07 (b, 1H), 3.72 (s, 3H), 3.66 (m, 1H), 2.84 (d, J = 3.4 Hz, 3H); 2.28 (d, J = 11.2 Hz, 1H), 2.04 (d, J = 20.1 Hz, 1H), 1.88 (d, J = 12.1 Hz, 1H), 1.65 (d, J = 13.9 Hz 1H); ¹³C-NMR (CDCl₃) δ 182.2 (s), 147.2 (b), 144.9 (s), 132.4 (s), 128.2 (d), 126.6 (s), 126.2 (d), 120.5 (d), 111.3 (d), 88.3 (d), 61.7 (d), 55.9 (q), 53.7 (t), 50.5 (t), 48.2 (s), 35.6 (t), 32.9 (q) 29.7 (t). Anal. (C₁₈H₂₂N₂O₃S.0.05 CH₃C₆H₅) C, H, N.

### .Beispiel 28:

(6R)-6-Hydroxy-3-methoxy-N¹¹-allyl-5,6,9,10-tetrahydro-4aH-[1]benzofuro[3a,3,2-ef][2]benzazepine-11-(12H)-carbothioamide (4u): Verfahren nach Beispiel 11, Reaktionszeit 5 h, Ausbeute 0.47 g (70%); Schmelzpunkt 192-194°C; gleiches Gerüst wie 47, nur die unterschiedlichen NMR-Signale werden wiedergegeben: ¹H-NMR (CDCl₃) δ 6.85 (m, 1H), 5.13 (m, 2H), 4.14 (m, 2H); ¹³C-NMR (CDCl₃) δ 181.2 (s), 133.7 (d), 116.6 (t), 48.3 (t). Anal. (C₂₀H₂₄N₂O₃S) C, H, N.

### Beispiel 29:

((6R)-6-Hydroxy-3-methoxy-5,6,9,10-tetrahydro-4aH-[1]benzofuro[3a,3,2-ef][2]benzazepin-11-(12H)-yl-fumaraciddimethylester (4v): Verfahren nach Beispiel 11. Zu einer Lösung von (+/-)-Norgalanthamin (0.5 g, 1.74 mmol) in 40 ml CH₂Cl₂ wurden 0.37 g (2.61 mmol) But-2-enedionsäuredimethylester zugegeben und 20 h lang gerührt. Das Lösungsmittel wurde entfernt, um ein öliges Produkt zu erhalten, dessen Flashchromatographie 0.28 g (39.1 %) von 4v ergab. Schmelzpunkt 112-115°C, ¹H-NMR (CDCl₃) δ 6.63 (dd, J₁ = 12.6 Hz, J₂ = 8.1 Hz, 2H), 6.02 (dd, J₁ = 15.9 Hz, J₂ = 11.5 Hz, 2H), 4.77 (b, 1H), 4.59 (b, 1H), 4.22 (d, J = 15.9, 1H), 4.13 (b, 1H), 3.92 (s, 3H), 3.83 (s, 3H), 3.72 (d, J = 15.9 Hz, 1H), 3.59 (s, 3H); 3.46 (m, 1H), 3.19 (dt, J₁ = 15.1 Hz, J₂ = 3.1 Hz, 3H), 2.68 (dd, J₁ = 15.8 Hz, J₂ = 2.2 Hz, 1H), 2.00 (m, 1H), 1.54 (m, 1H); ¹³C-NMR (CDCl₃) δ 167.6 und 165.7 (s), 153.0 (s), 146.0 (s), 144.3 (s), 132.9 (s), 128.5 (s), 127.8 (d), 126.4 (d), 121.8 (d), 111.2 (d), 88.6 (d), 86.6 (d), 61.9 (d), 56.9 (t), 55.8 (q), 55.0 (q), 50.2 (q), 48.3 (s), 33.0 (t), 29.8 (t). Anal. (C₂₂H₂₅NO₇) C, H, N.

### Beispiel 30:

(6R)-11-(3-2-(4-Fluor)phenyl-2,5-diazabicyclo[2.2.1]heptan-5-yl-propyl)-3-methoxy-5,6,9,10,11,12-hexahydro-4aH-[1]benzofuro[3a,3,2-ef][2]benzazepin-6-ol (4w): Verfahren nach Beispiel 11, Reaktionszeit 4 d, Ausbeute 0.14 g (63.0%); ¹H-NMR (CDCl₃) δ 7.21 (m, 2H); 6.68 (m, 3H), 5.0 (s, 1H), 4.47 (d, J = 14.0 Hz, 1H), 3.90 (m, 1H), 3.63 (m, 3H), 3.24 (m, 1H), 2.04 (m, 3H); ¹³C-NMR (CDCl₃) 168.0 und 167.6 (s), 146.4 (d), 144.1 (s), 127.7 und 127.5 (d), 112.5 und 112.4 (d), 67.0 (t), 57.0 und 56.8 (d), 56.8 und 56.6 (t), 51.8 und 51.6 (t), 36.6 (t); 33.7 und 33.6 (t).

### Beispiel 31:

2-((6R)-6-hydroxy-3-methoxy-5,6,9,10-tetrahydro-4aH-[1]benzofuro[3a,3,2-ef][2]benzazepin-11 (12H)-ylmethylene)-malononitrile (4x): Verfahren nach Beispiel 11, Reaktionszeit 6 h, Ausbeute 0.41 g (64.8%); ¹H-NMR (CDCl₃) δ 7.12 (m, 1H), 6.63 (dd, J₁ = 12.6 Hz, J₂ = 8.1 Hz, 2H), 6.02 (dd, J₁ = 15.9 Hz, J₂ = 11.5 Hz, 2H), 4.59 (b, 1H), 4.22 (d, J = 15.9, 1H), 4.13 (b, 1H), 3.83 (s, 3H), 3.72 (d, J = 15.9 Hz, 1H), 3.46 (m, 1H), 3.19 (dt, J₁ = 15.1 Hz, J₂ = 3.1 Hz, 3H), 2.68 (dd, J₁ = 15.8 Hz, J₂ = 2.2 Hz, 1H), 2.00 (m, 1H), 1.54 (m 1H); ¹³C-NMR (CDCl₃) δ 157.2 und 156.8 (d), 146.0 (s), 144.3 (s), 132.9 (s), 128.5 (s), 127.8 (d), 126.4 (d), 124.2 (s), 121.8 (d), 116.8 und 116.5 (s), 115.0 und 114.7 (s), 111.2 (d), 88.6 (d), 61.9 (d), 56.9 (t), 55.8 (q), 48.3 (s), 33.0 (t), 29.8 (t).

### Beispiel 32:

2-((6R)-6-Hydroxy-3-methoxy-5,6,9,10-tetrahydro-4aH-[1]benzofuro[3a,3,2-ef][2] benzazepin-11 (12H)-ylmethylene)-malonoaciddiethylester (4y): Verfahren nach Beispiel 11, Reaktionszeit 21 h, Ausbeute 0.46 g (63.3%), Schmelzpunkt 145-146°C, gleiches Gerüst wie Verbindung 4v, nur die unterschiedlichen NMR-Signale werden beschrieben: ¹H-NMR (CDCl₃) δ 6.83 (s, 1H), 3.43 (m, 6H); ¹³C-NMR (CDCl₃) δ 181.2 (s), 133.7 (d), 116.6 (t), 48.3 (t). Anal. (C₂₂H₂₅NO₇.0.25 C₆H₁₄O) C, H, N.

### Beispiel 33:

3-((6R)-6-Hydroxy-3-methoxy-5,6,9,10-tetrahydro-4aH-[1]benzofuro[3a,3,2-ef][2]benzazepin-11(12H)-yl-acrylacidethylester (4z): Verfahren nach Beispiel 11, Reaktionszeit 20 h, Ausbeute 0.30 g (46.2%), Schmelzpunkt 121-122°C, gleiches Gerüst wie Verbindung 4v, nur die unterschiedlichen NMR-Signale werden beschrieben: ¹H-NMR (CDCl₃) δ 7.40 (dd, J₁ = 16.0 Hz, J₂ = 2.0 Hz, 1H), 4.68 (d, J = 16.0 Hz, 1H), 4.10 (m, 2H), 1,28 8m, 3H); ¹³C-NMR (CDCl₃) δ 169.3 und 167.8 (s), 161.1 (d), 97.5 (d), 59.0 (t), 14.5 und 14.3 (q).
Verfahren E: Eine Lösung von (+/-)-Norgalanthamin (0.5 g, 1.83 mmol), 0.51 g (3.66 mmol) Kaliumkarbonat, (2.20 mmol) Natriumjodid und Alkylhalogenid (2.20 mmol) in Aceton (20 ml) wurden 12 h rückflußgekocht und eingeengt. Der Rückstand wurde in 30 ml 2N Salzsäure aufgelöst und mit 1 × 20 ml AcOEt extrahiert. Die wäßrige Lösung wurde mit konzentriertem Ammoniak auf pH > 8.5 eingestellt und mit AcOEt (3 x 20 ml) extrahiert. Die vereinigten, organischen Extrakte wurden mit Kochsalzlösung gewaschen, getrocknet (Na₂SO₄) eingeengt und durch MPLC gereinigt.

Die Verbindungen 4ab - 4ah und 4an - 4aq enthalten das grundlegende Galanthamingerüst wie 4aa, unterscheiden sich aber hinsichtlich des Stickstoffsubstituenten. Da die Proton- und Kohlenstoffsignale des Galanthaminkerns sich voneinander nicht wesentlich unterscheiden, werden nur die NMR-Signale des Stickstoffsubstituenten nachstehend wiedergegeben.

### Beispiel 34:

Ethyl-2-((6R)-6-Hydroxy-3-methoxy-5,6,9,10-tetrahydro-4aH-[1]benzofuro[3a,3,2-ef][2] benzazepin-11(12H)-yl)acetat (4ac): Nach dem Verfahren E unter Verwendung von Ethylchloracetat und einer Reaktionszeit von 1 h wurden 0.48 g der Verbindung (73%) erhalten; ¹H-NMR (CDCl₃) δ 4.10 (m, 2H), 3.32 (s, 2H), 1.21 (t, J = 7.3 Hz, 3H); ¹³C-NMR (CDCl₃) δ 170.7 (s), 60.4 (t), 58.0 (t), 14.1 (q).

### Beispiel 35:

### Vorschrift:

### SUBSTITUTION IN POSITION 1

### Direkte Einführung neuer Substituenten

### 3.2.1.1 [4aS-(4αa,6β,8aR*)]-4a,5,9,10,11,12-Hexahydro-1-(N,N-dimethylamino)-3-methoxy-11-methyl-6H-benzofuro[3a,3,2-ef][2]benzazepin-6-ol, 1-(N,N-Dimethylamino)-galanthamin (MH-7)

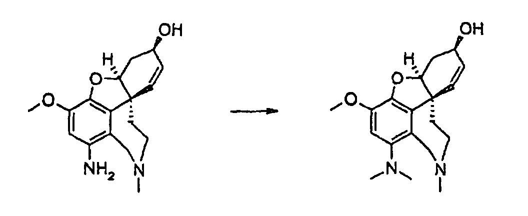

| | |
|---|---|
| 320 mg (1.06 mmol) | 1-Aminogalanthamin (4) |
| 0.50 ml | Ameisensäure in 2 ml Wasser |
| 0.25 ml | Formaldehyd (37%) |

Unter Magnetrührung wurden alle Reaktanden, gelöst in 10 ml Wasser, zusammen auf 70°C erwärmt. Nach 4.5 h wurde mit konz. aq. Ammoniak basisch gemacht, wobei ein weißer Niederschlag in gelber Lösung ausfiel. Das Reaktionsgemisch wurde mit Ethylacetat erschöpfend extrahiert, die vereinigten organischen Phasen über Natriumsulfat getrocknet, filtriert und das Lösungsmittel abgezogen.

Das resultierende Substanzgemisch wurde auf einer Kieselgelsäule (CHCl₃ : MeOH = 1:1 ) aufgetrennt und anschließend im Hochvakuum mittels Kugelrohr destilliert.
Ausbeute: 0.17 g (0.52 mmol = 49% d. Th.) hellgelbes Öl
C₁₉H₂₆N₂O₃ [330.43]
- DC:: R_{f} = 0.49 (CHCl₃ : MeOH = 1:1)
- B.p.:: 180°C / 0.01 Torr
αD²⁰ [c=0.1,CHCl₃]=-156.36°
FID-Nummern:
¹H: MHEMOF.016, ¹³C: MHEM1F.002, DEPT: MHEM2F.002
¹H-NMR (200 MHz, CDCl₃): δ 6.57 (s, 1H), 6.08 (dd, *J* = 10.3, 1.0 Hz, 1H), 5.97 (dd, *J* = 10.3, 4.8 Hz, 1 H), 4.56 (bs, 1H), 4.45 (d, *J* = 15.1 Hz, 1H), 4.12 (bs, 1H), 3.83 (s, 3 H), 3.55 (d, J = 15.1 Hz, 1H), 3.12 (td, *J* = 13.1, 1.7Hz, 1H), 2.97 (dt, *J* = 14.1, 3.5 Hz, 1H), 2.72-2.53 (m, 1H), 2.58 (s, 6 H), 2.44 (s, 3 H), 2.12-1.98 (m, 2H), 1.62 (ddd, *J* = 13.6, 3.8, 2.1 Hz, 1H); ¹³C-NMR (50 MHz, CDCl₃): δ 147.1 (s), 143.2 (s), 142.0 (s), 133.7 (s), 127.3 (d), 127.2 (d), 124.2 (s), 103.9 (d), 88.3 (d), 62.0 (d), 55.9 (q), 54.4 (t), 54.1 (t), 48.4 (s), 45.7 (q), 44.2 (q), 34.8 (t), 29.8 (t)

### Beispiel 37:

(4aS,6R,8aS)-11-(3-Aminoethyl)-3-methoxy-5,6,9,10,11,12-hexahydro-4aH-[1]benzofuro[3a,3,2-ef][2]benzazepin-6-ol (4af), Verfahren F, Reaktionspartnerverbindung 4ad, Reaktionszeit 1 h; Ausbeute 0.31 g (59.2 %); Schmelzpunkt 47-51°C; ¹H-NMR (CDCl₃) δ 2.69 (m, 2H), 1.92 (b, 2H); ¹³C-NMR (CDCl₃) δ 51.9 (s), 38.0 (t).

### Beispiel 40:

Ethyl-3-((*6R*)-6-Hydroxy-3-methoxy-5,6,9,10-tetrahydro-4aH-[1]benzofuro[3a,3,2-ef][2]benzazepin-11(12H)-yl)propanoat (4ai): Verfahren B, Reaktionszeit 4 h; Ausbeute 0.64 g (47.5%); ¹H-NMR (CDCl₃) δ 4.15 (q, J = 6.0 Hz, 2H), 2.81 (t, 7.0 Hz, 2H), 2.47 (t, J = 7.0 Hz, 2H), 1.23 (t, J = 6 Hz, 3H); ¹³C-NMR (CDCl₃) δ 172.4 (s), 60.3 (t), 57.3 (t), 32.9 (t), 14.1 [q).

### Beispiel 41:

t-Butyl-3-((6R)-6-Hydroxy-3-methoxy-5,6,9,10-tetrahydro-4aH-[1]benzofuro[3a,3,2-ef][2]benzazepin-11(12H)-yl)propanoat (4aj): Verfahren B, Reaktionszeit 5 h; Ausbeute 0.83 g (60.0%); ¹H-NMR (CDCl₃) δ 2.82 (t, J = 7.0 Hz, 2H), 2.40 (t, J = 7.0 Hz, 2H), 1.43 (s, 9H); ¹³C-NMR (CDCl₃) δ 172.0 (s), 80.5 (s), 57.7 (t), 34.2 (t), 28.0 (q). Anal. (C₂₄H₃₃NO₅) C,H,N.

### Beispiel 42:

3-((6R)-6-Hydroxy-3-methoxy-5,6,9,10-tetrahydro-4aH-[1]benzofuro[3a,3,2-ef][2]benzazepin-11 (12H)-yl)-N¹¹-isopropylpropanamid (4ak): Verfahren B, Reaktionszeit 18 h; Ausbeute 0.55 g (78.7%); ¹H-NMR (CDCl₃) δ 3.81 (m, 1H), 2.79 (t, J = 6Hz, 2H), 2.32 (t, J = 6.0 Hz, 2H), 1.10 (t, J = 12.0 Hz, 6H); ¹³C-NMR (CDCl₃) δ 171.4 (s), 56.9 (t), 40.7 (d), 33.2 (t), 22.7 (q).

### Beispiel 43:

3-((6R)-6-Hydroxy-3-methoxy-5,6,9,10-tetrahydro-4aH-[1]benzofuro[3a,3,2-ef][2]benzazepin-1 1(12H)-yl)-N1-t-butylpropanamid (4a1): Verfahren B, Reaktionszeit 24 h; Ausbeute 0.37 g (51.2%); ¹H-NMR (CDCl₃) δ 2.76 (t, 6.0 Hz, 2H), 2.29 (m, 2H), 1.28 (s, 9H); ¹³C-NMR (CDCl₃) δ 171.4 (s), 51.4 (t), 50.3 (s), 33.5 (t), 28.7 (q).

### Beispiel 44:

3-((6R)-6-Hydroxy-3-methoxy-5,6,9,10-tetrahydro-4aH-[1]benzofuro[3a,3,2-ef][2]benzazepin-11(12H)-yl)propanitril (4am): Verfahren B, Reaktionszeit 4 h; Ausbeute 0.53 g (90.6%); ¹H-NMR (CDCl₃) δ 2.82 (t, J = 7.1 Hz, 2H), 2.47 (t, J = 6.8 Hz, 2H); ¹³C-NMR (CDCl₃) δ 118.7 (s), 51.6 (t), 46.6 (t), 16.7 (t).

### Beispiel 45:

(6R)-11-(3-Hydroxypropyl)-3-methoxy-5,6,9,10,11,12-hexahydro-4aH-[1]benzofuro[3a,3,2-ef][2]benzazepin-6-ol (4an): Verfahren F, Reaktionspartnerverbindung 4ai, Reaktionszeit 7 h; Ausbeute 0.21 g (47.7%); ¹H-NMR (CDCl₃) δ 3.77 (m, 2H), 2.72 (m, 2H), 2.02 (m, 2H); ¹³C-NMR (CDCl₃) δ 63.9 (t), 57.3 (t), 29.8 (t).

### Beispiel 46:

(6R)-11-(3-Aminopropyl)-3-methoxy-5,6,9,10-tefrahydro-4aH-[1]benzofura[3a,3,2-ef][2]benzazepin-6-ol (4ao): Verfahren F, Reaktionspartnerverbindung 4am, Reaktionszeit 1 h; Ausbeute 78 mg (52.8%); ¹H-NMR (CDCl₃) δ 3.22 (m, 2H), 2.68 (m, 2H), 1.72 (m, 2H); ¹³C-NMR (CDCl₃) δ 51.3 (t), 37.8 (t), 29.3 (t).

### Beispiel 47:

(6R)-11-(3-piperidin-1-yl-propyl)-3-methoxy-5,6,9,10-hexahydro-4aH-[1]benzofuro[3a,3,2-ef][2]benzazepin-6-ol (4ap): Verfahren E, Reaktionszeit 3 d; Ausbeute 0.36 g (53.2%); ¹H-NMR (CDCl₃) δ 2.68 (m, 8H), 1.77 (m, 6H), 1.50 (m, 2H); ¹³C-NMR (CDCl₃) δ 57.4 (t), 44.1 (t), 49.2 (t), 24.7 (t), 23.4 (t), 23.3 (t).

### Beispiel 48:

### Stufe 1:

(4a,*S*,6*R*8a*S*)-3-Methoxy-5,6,9,10,11,12-hexahydro-4aH[1]benzofuro[3a,3,2-ef[2]benzazepin-6-ol, (-)-Norgalanthamin (8).

### Methode 1:

Zu einer Lösung von 10.92 g (40.0 mmol) rac. Norgalanthamin (4) in 40 ml Methanol wird eine Lösung von 7.72 g (20.0 mmol) (+)-O,O-Di-p-Toluoylweinsäure in 15 ml Methanol getropft und anschließend mit 1 ml Methanol nachgewaschen. Die Lösung wird mit einem Impfkristall versetzt (ohne Impfkristall kann die Kristallbildung mehrere Wochen dauern) und zwei Tage bei 4°C stehen gelassen. Dann wird mit einem Glasstab gut durchgerieben und weitere zwei bis fünf Tage bei 4°C stehen gelassen, wobei immer wieder mit einem Glasstab gut durchgerieben wird. Anschließend wird das ausgefallene Salz abgesaugt, dreimal mit eiskaltem Methanol nachgewaschen und in 100 ml Wasser aufgenommen. Die wässrige Phase wird mit konzentriertem wässrigen Ammoniak basisch gemacht und dreimal mit je 60 ml Essigsäureethylester extrahiert. Die vereinigten organischen Phasen werden einmal mit gesättigter wässriger Natriumchloridlösung gewaschen, getrocknet (Na₂SO₄, Aktivkohle), filtriert und eingedampft, wodurch 2.9 g (37,5 % d. Th) farblose Kristalle an (-) Norgalanthamin (8) erhalten werden.

### Methode 2:

Zu einer Lösung von Glanthamin (1) (20.0g, 70 mmol) in CH₂Cl₂ (350 ml) wurden m-CPBA (Peroxidgehalt 76%, 15.6 g 70 mmol) hinzugefügt und die klare Lösung 40 min bei Raumtemperatur gerührt. In diesem Stadium war die Umwandlung in das N-Oxid quantitativ, wie durch HPLC festgestellt wurde. Dann wurde eine Lösung von FeSO₄.7H₂O (9.7 g 35 mmol) in MeOH (100 ml) zugefügt. Die Mischung wurde 20 min gerührt, mit 2N Salzsäure (200 ml) versetzt, die flüchtigen Anteile (CH₂Cl₂ und MeOH) bei vermindertem Druck abgedampft und dann mit Ether (3 × 100 ml) gewaschen. Die wäßrige Lösung wurde mit konzentriertem Ammoniak auf pH> 8.5 eingestellt und mit CH₂Cl₂ (3 × 100 ml) extrahiert und mit CH₂Cl₂:MeOH = 9:1 (3 × 100 ml). Die vereinigten, organischen Extrakte wurden mit Kochsalzlösung (200 ml) gewaschen, über Na₂SO₄ getrocknet und eingeengt, um ein kristallines Produkt zu ergeben (18.2 g, 96%), das aus einer 92:8-Mischung aus Norgalanthamin und Galanthamin bestand. MPLC (CHCl₃:MeOH:Et₃N = 98:1.25:0.5), 16.1 g (84.7%) Norgalanthamin (8):¹H-NMR (CDCl₃) δ 6.62 (b, 2H), 6.02 (m, 2H), 4.61 (b, 1H), 4.14 (t, J = 4.3 Hz, 1H), 3.98 (d, J = 5.0 Hz, 2H), 3.83 (s, 3H), 3.30 (m, 1H), 2.69 (d, J = 15.7, 1H), 2.10-1.63 (m, 4H); ¹³C-NMR (CDCl₃ δ 146.2 (s), 144.1 (s), 133.1 (s), 131.7 (s), 127.8 (d), 126.8 (d), 120.8 (d), 111.1 (d), 88.4 (d), 61.9 (d), 55.9 (q), 53.3 (t), 48.5 (s), 46.7 (t), 39.4 (t), 29.9 (t).

### Stufe 2:

(4a,S,6R8aS)-3-Methoxy-11-Phenyl-5,6,9,10,11,12-hexahydro-4aH[1]benzofuro[3a,3,2-ef[2]benzazepin-6-ol (8a). Zu einer Lösung 1.0 g der Verbindung 8 (-)-Norgalanthamin in 50 ml CH₂Cl₂ wurden 0.28 g Phenylbosäure (7.2 mmol), 0.6 ml Pyridin (7.2 mmol), 0.67 g Kupferacetat (3.6 mmol) und ein Molekularsieb (1.0 g) zugegeben. Das Reaktionsgemisch wurde bei Raumtemperatur 3 Tage lang gerührt. Der Niederschlag wurde abfiltriert und mit 3 × 10 ml CH₂Cl₂ gewaschen. Das Filtrat wurde 3 × mit 50 ml verdünnter Ammoniaklösung extrahiert. Die vereinigten, wäßrigen Phasen wurden mit 3 × 50 ml CH₂Cl₂ extrahiert, die organischen Phasen wurden mit Kochsalzlösung gewaschen, über Natriumsulfat getrocknet und eingeengt. MPLC ergab 0.33 g (26.4%) der Verbindung 8a. Schmelzpunkt 178-180°C;
¹H-NMR (CDCl₃) δ 7.18 (m, 2H), 6.82 (m, 3H), 6.67 (dd, J₁ = 33.8 Hz, J₂ = 7.6 Hz, 2H), 6.05 (b, 2H), 4.66 (b, 1H), 4.53 (m, 1H), 4.19 (d, J = 15.3 Hz, 1H), 3.85 (s, 3H), 3.81 (d, J = 15.3 Hz, 1H), 3.33 (m, 1H), 2.68 (m; 1H), 2.07 (m, 3H), 1.62 (m, 1H); ¹³C-NMR (CDCl₃ δ 158.2 (s), 145.9 (s), 144.5 (s), 133.0 (s), 129.4 (s), 128.9 (d), 128.1 (d), 126.9 (d), 126.1 (d), 121.1 (d), 116.0 (d), 111.4 (d), 88.6 (d), 61.9 (d), 57.0 (t), 55.9 (q), 50.6 (t), 48.2 (s), 32.8 (t), 29.9 (t). Anal. (C₂₂H₂₃NO₃.0.75 H₂O) C, H, N.

### Beispiel 49:

(4a,S,6R,8aS)-3-Methoxy-11-thiophenyl-5,6,9,10,11,12-hexahydro-4aH-[1]benzofuro[3a,3,2-ef][2]benzazepin-6-ol (8b). Die Verbindung wurde wie in Verbindung 8a hergestellt, die Reaktionszeit betrug 3 Tage, Ausbeute 0,14 g (28.0%). Nur die von 8a unterschiedlichen NMR-Signale sind beschrieben: ¹H-NMR (CDCl₃) 7.23 (m, 1H), 6.92 (t, J = 3.0 Hz, 1H), 6.83 (d, J = 2.7 Hz, 1H); ¹³C-NMR (CDCl₃ δ 145.7 (s), 127.6 (d), 126.2 (d), 125.0 (d). Anal. (C₂₀H₂₁NO₃S) C, H, N.

### Beispiel 50:

(4a,S,6R,8aS)-11-Benzoyl-piperidin-4-yl)-3-methoxy-5,6,9,10,11,12-hexahydro-4aH-[1]benzofuro[3a,3,2-ef][2]benzazepin-6-ol (8c). 1-Benzoyl-piperidin-4-on (0.34 g, 1.7 mmol), 0.47 g Titanisopropylat (1.7 mmol) und 3.6 g (1.3 mmol) der Verbindung 8 wurden 30 min bei 110°C geschmolzen. Nach dem Abkühlen bei Raumtemperatur wurde eine Lösung von Natriumcyanoborhydrid (65 mg, 0.9 mmol) in trockenem EtOH (10 ml) zugegeben und das Reaktionsgemisch bei Raumtemperatur 24 Stunden gerührt. Nach Zugabe von 2 ml Wasser wurde der Niederschlag abfiltriert, das Filtrat eingeengt und der Rückstand mit 20 ml EtOAc verdünnt und erneut filtriert. Die klare Lösung wurde eingeengt. MPLC ergab 0.24 g (38.8%) der Verbindung 8c. ¹H-NMR (CDCl₃) δ 7.52-7.31 (m, 5H), 6.65 (b, 2H), 6.08 (m, 2H), 4.64 (b, 1H), 4.22-3.90 (m, 4H), 3.82 (s, 3H), 3.37 (m, 2H), 3.01-2.62 (m, 5H), 2.10-1.82 (m, 5H), 1.67-1.42 (m, 2H); ¹³C-NMR (CDCl₃) 170.1 (s), 145.8 (s), 144.0 (s), 136.0(s), 133.1 (s), 129.4 (d), 128.8 (d), 128.4 (d), 128.3 (d), 127.6 (d), 126.7 (d), 121.6 (d), 111.2 (d), 88.6 (d), 61.9 (d), 55.8 (q), 55.6 (t), 48.3 (t), 30.8 (t), 29.8 (t).

### Beispiel 51:

(4a,S,6R,8aS)-6-Hydroxy-3-methoxy-5,6,9,10-tetrahydro-4aH-[1]benzofuro[3a,3,2-ef][2]benzazepin-1 1(12H)-carboxylicacidphenylester (8e): Zu einer Lösung von 0.50 g der Verbindung 8 (1.74 mmol) in 50 ml CHCl₃ wurden 2.5 g Natriumhydrogenkarbonat (29.8 mmol) und 1.84 ml Phenylchlorformiat (14.6 mmol) zugegeben. Das Gemisch wurde kräftig gerührt und2 Stunden rückflußgekocht und dann mit Wasser (30 ml) verdünnt. Die Phasen wurden getrennt. Die wäßrige Phase wurde mit 2 × 30 ml CH₂Cl₂ extrahiert und die vereinigten, organischen Phasen mit 1N Salzsäure (30 ml) gewaschen, über Natriumsulfat getrocknet und unter vermindertem Druck eingeengt, um das rohr Produkt zu ergeben. MPLC(CH₂Cl₂:MeOH = 99:1) ergab 0.58 g (84.2%) der Verbindung 8e: ¹H-NMR (CDCl₃) δ 7.51-7.04 (m, 5H), 6.82 (dd, J₁ = 24.0 Hz, J₂= 6.0 Hz, 2H), 6.04 (b, 2H), 4.91 (b, 1H), 4.69 (d, J = 6.0 Hz, 2H), 4.20 (b, 1H), 3.83 (s, 3H), 3.42 (m, 1H), 3.19 (m, 1H), 2.43-1.90 (m, 4H); ¹³C-NMR (CDCl₃) δ 151.3 (s), 146.6 (s), 145.7 (s), 130.5 (s), 130.1 (s), 129.4 (d), 129.3 (d), 125.9 (d), 125.3 (d), 125.0 (d), 121.6 (d), 111.9 (d), 88.3 (d), 62.8 (d), 57.7 (t), 55.9 (q), 53.4 (t), 49.3 (s), 43.3 (t), 32.7 (t).

### Beispiel 52:

(4a,S,6R,8aS)-6-Hydroxy-3-methoxy-5,6,9,10-tetrahydro-4aH-[1]benzofuro(3a,3,2-ef][2]benzazepin-11(12H)-carbothionacid-O-phenylester (8f): Zu einer Lösung von 0.50 g der Verbindung 8 (1.74 mmol) in 25 ml CHCl₃ wurden 0,24 ml Chlorothionformiat (1.74 mmol) zugegeben und das Reaktionsgemisch unter Stickstoff 1 Stunde lang gerührt. Das Lösungsmittel wurde abgetrennt, um ein farbloses Öl zu ergeben, das flashchromatographiert wurde, um 0.50 g (71.2%) der Verbindung 8a zu ergeben: ¹H-NMR (CDCl₃) δ 7.48-7.12 (m, 3H), 7.02 (d, J = 6.0 Hz, 2H), 6.83 (m, 2H), 6.04 (b, 2H), 5.08 (m, 1H), 4.71 (d, J = 26.0 Hz, 2H), 4.28 (m, 1H), 3.88 (s, 3H), 3.41 (m, 2H), 2.51-2.09 (m, 4H); ¹³C-NMR (CDCl₃) δ 187.5 und 187.4 (s), 153.8 und 153.7 (s), 146.7 und 146.6 (s), 145.8 und 145.7 (s), 131.3 und 130.6 (s), 130.2 und 129.5 (s), 129.2 und 129.1 (d), 126.0 und 125.9 (d), 125.0 und 124.9 (d), 122.7 und 122.6 (d), 121.6 und 120.4 (d), 115.3 (d), 112.0 (d), 84.4 und 84.2 (d), 63.1 und 62.9 (d), 55.9 (q), 51.5 (t), 49.5 und 49.3 (s), 47.8 (t), 36.9 (t), 33.1 (t).

### Beispiel 53:

(4a,S,6R,8aS)-6-Hydroxy-3-methoxy-5,6,9,10-tetrahydro-4aH-[1]benzofuro[3a,3,2-ef][2]benzazepin-11(12H)-carboxylicacid-9-H-fluor-9-ylmethylester (8g): Zu einer Lösung von 0.5 g der Verbindung 8 (1.8 mmol) und 2.5 ml Triethylamin (1.8 mmol) in 20 ml CH₂Cl₂ wurden 0,5 g 9-fluorenylmethyloxycarbonylchlorid (20 mmol) zugegeben und 30 min bei Raumtemperatur gerührt. Das Reaktionsgemisch wurde eingeengt, der Rückstand mit 80 ml 2 N Salzsäure verdünnt und mit 5 × 50 ml CH₂Cl₂ extrahiert. Die organischen Phasen wurden vereinigt, mit Kochsalzlösung gewaschen, über Natriumsulfat getrocknet und eingeengt, um 0.88 g (99.2%) der Verbindung 8g zu ergeben. Schmelzpunkt 76-79°C; α_{D} = -33.0°; ¹H-NMR (CDCl₃) δ 1.72 (dd, J = 13.5; 5.0 Hz, 1H), 1.80-2.10 (m, 2H), 2.69 (dd, J = 13.5; 5.0 Hz, 1H), 3.20-3.45 (m, 2H), 3.85 (s, 3H), 3.95-4.35 (m, 3H), 4.40-4.52 (m, 2H), 5.78-6.05 (m, 2H), 6.22-6.82 (m, 2H), 7.19-7.82 (m, 8H); ¹³C-NMR (CDCl₃) δ 155.1 (s), 144.4 (s), 144.0 (s), 141.4 (s), 134.0 (s), 129.1 (s), 128.1 (s), 128.0 (d), 127.6 (d), 126.4 (d), 124.9 (d), 124.7 (d), 121.0 (d), 119.9 (d), 111.1 (d), 88.3 (d), 66.9 (t), 61.9 (d), 56.0 (q), 51.5 (t), 48.3 (s), 47.3 (d), 45.9 (t), 36.4 (t), 29.7 (t).

### Beispiel 54:

1-((4a,S,6R,8aS)-6-Hydroxy-3-methoxy-5,6,9,10-tetrahydro-4aH-[1]benzofuro[3a,3,2-ef][2]benzazepin-11(12H)-yl)pent-4-en-1-on (8h): Zu einer Lösung von 1.0 g der Verbindung 8 (3.7 mmol) und 0.51 ml (3.7 mmol) Triethylamin in 40 ml CH₂Cl₂MeOH = 5:2 wurden bei O°C0.97 ml (5.1 mmol) Pent-4-enoikanhydrid zugegeben und 20 min gerührt. Das Reaktionsgemisch wurde mit 20 ml CH₂Cl₂ verdünnt, mit 2 × 20 ml gesättigter, Natriumhydrogenkarbonatlösung extrahiert. Die vereinigten, wäßrigen Phasen wurden mit 2 × 40 ml CH₂ Cl₂ extrahiert, die vereinigten organischen Phasen wurden mit Kochsalzlösung gewaschen, über Natriumsulfat getrocknet und eingeengt, um 1.24 g (95.3%) der. Verbindung 8h zu ergeben: ¹H-NMR (CDCl₃) δ 6.66 (b, 2H), 5.98 (m, 2H), 5.78 (m, 1H), 4.98 (m, 2H), 4.66 (d, J = 12.8 Hz, 1H), 4.55 (s, 1H), 4.41 (d, J = 16.5 Hz, 1H), 4.11 (b, 1H), 3.93 (m, 1H), 3.81 (s, 3H), 3.17 (t, J = 17.7 Hz, 1H), 2.76-2.15 (m, 5H), 1.92 (m, 3H); 13C-NMR (CDCl₃) δ 171.7 (s), 146.8 (s), 144.6 (s), 137.3 (d), 132.4 (s), 128.1 (s), 128.0 (d), 126.3 (d), 120.3 (d), 114.9 (d), 111.0 (d), 88.2 (d), 61.7 (d), 55.8 (q), 52.7 (t), 48.2 (s), 44.6 (t), 35.7 (t), 33.2 (t), 29.7 (t), 28.8 (t).

### Beispiel 55:

1-((4a,S,6R,8aS)-6-Hydroxy-3-methoxy-5,6,9,10-tetrahydro-4aH-[1]benzofuro[3a,3,2-ef][2]benzazepin-11(12H)-carboxamid (8i): Zu einer Suspension von 0.5 g der Verbindung 8 (1.8 mmol) in 25 ml Wasser wurde der pH-Wert mit 2N Salzsäure auf pH = 3 eingestellt und 0.23 g (3.6 mmol) Natriumzyanid zugegeben. Das Reaktionsgemisch wurde 25 Stunden lang bei Raumtemperatur gerührt und dann der pH-Wert mit konzentriertem Ammoniak auf über 8.5 eingestellt und schließlich mit 3 × 20 ml CH₂Cl₂ extrahiert. Die vereinigten, wäßrigen P hasen wurden mit Kochsalzlösung extrahiert, über Natriumsulfat getrocknet und eingeengt. MPLC (CHCl₃ :MeOH(NH₃) = 95 : 5) ergab 0.38 g (66%) der Verbindung 8i: ¹H-NMR (CDCl₃) δ 6.67 (dd, J = 12.8; 8.1 Hz, 2H), 6.00 (dd, J = 15.1; 10.4 Hz, 2H), 4.68 (b, 1H), 4.51 (d, J = 16.8 Hz, 1H), 4.31 (d, J = 16.8 Hz, 1H), 4.11 (m, 1H), 3.81 (s, 3H), 3.35 (t, J = 12.8 Hz, 1H), 2.67 (d, J = 15.3 Hz, 1H), 2.41 (b, 1H), 1.97 (m, 2H), 1.72 (d, J = 13.8 Hz, 1H): ¹³C-NMR (CDCl₃) δ 158.1 (s), 146.8 (s), 144.5 (s), 132.3 (s), 128.6 (s), 128.0 (d), 126.2 (d), 120.3 (d), 111.1 (d), 88.1 (d), 61.6 (d), 55.8 (q), 51.9 (t), 48.3 (s), 45.6 (t), 36.3 (t), 29.7 (t).

### Beispiel 56:

(4a,S,6R,8aS)-6-Hydroxy-3-methoxy-N¹¹-methyl-5,6,9,10-tetrahydro-4aH-[1]benzofuro[3a,3,2-ef][2]benzazepin-11(12H)-carbothioamid (8j): Verfahren nach Beispiel 142, Reaktionszeit 4 Stunden, Ausbeute 1.02 g (88%); Schmelzpunkt 229-230°C; ¹H-NMR und ¹³C-NMR waren identisch mit Verbindung 4t. Anal. C₁₈H₂₂N₂O₃S.0.2 CH₃C₆H₅) C, H, N.

### Beispiel 57:

(4a,S,6R,8aS)-6-Hydroxy-N¹¹-isopropyl-3-methoxy-5,6,9,10-tetrahydro-4aH-[1]benzofuro[3a,3,2-ef][2]benzazepin-11(12H)-carboxamid (8k): Verfahren A, Reaktionszeit 3 Stunden, Ausbeute 1.86 g (71%): ¹H-NMR und ¹³C-NMR waren identisch mit Verbindung 4i.

### Beispiel 58:

(4a,S,6R,8aS)-N¹¹-t-Butyl-6-hydroxy-3-methoxy-5,6,9,10-tetrahydro-4aH-[1]benzofuro[3a,3,2-ef][2]benzazepin-11(12H)-carboxamid (81): Verfahren A, Reaktionszeit 3 Stunden. Ausbeute 1.63 g (60%); Schmelzpunkt 106-108°C; ¹H-NMR und ¹³C-NMR waren identisch mit Verbindung 4m.

### Beispiel 59:

(4a,S,6R,8aS)-6-Hydroxy-3-methoxy-N¹¹-2-trifluoromethyl-5,6,9,10-tetrahydro-4aH-[1]benzofuro[3a,3,2-ef][2]benzozepin-11(12H)-carboxamid (8m): Verfahren A, Reaktionszeit 5 Stunden, Ausbeute 0.60 g (59%);
¹H-NMR (CDCl₃) δ 8.20 (t, J = 8.0 Hz, 1H), 7.24 (m, 2H), 7.02 (m, 1H); ¹³C-NMR (CDCl₃) δ 153.6 (s), 137.6 (s), 127.6 (d); 126.1 (d), 123.0 und 117.8 (s), 122.3 (d), 119.8 (s), 111.3 (d).

### Beispiel 60:

Methyl-(4a,S,6R,8aS)-N¹¹-Cyano-6-hydroxy-3-methoxy-5,6,9,10-tetrohydro-4aH-[1]benzofuro[3a,3,2-ef][2]benzazepin-11(12H)-carboximidothioat (8n). Die Verbindung wurde wie für die Verbindung 3bi beschrieben, hergestellt. Reaktionszeit 3 Tage, Ausbeute 0.90 g (33,2%); ¹H-NMR (CDCl₃) δ 6.72 (m, 2H), 5.98 (d, J = 10.2 Hz, 2H), 4.62 (m, 2H), 4.14 (b, 1H), 3.92 (d, J = 11.8 Hz, 1H), 3.84 (s, 3H), 3.44 (m, 2H), 2.74 (s, 3H), 2.68 (m, 1H), 1.99 (m, 3H); ¹³C-NMR (CDCl₃) δ 146.2 (s), 144.9 (s), 131.7 (s), 128.7 (d), 125.8 (s), 125.5 (d), 121.8 (d), 111.2 (d), 88.1 (d), 61.6 (d), 55.8 (q), 51.2 (t), 49.7 (t), 47.9 (s), 29.6 (t), 16.1 (q).

### Beispiel 61:

(4a,S,6R,8aS)-11-(Cyclopropylmethyl)-3-methoxy-5,6,9,10-tetrahydro-4aH-[1]benzofuro[3a,3,2-ef][2]benzazepin-6-ol (8o): Verfahren nach Beispiel E, Reaktionszeit 36 Stunden, Ausbeute 0.12 g (29.0%); selbes Gerüst wie 4aa, nur die unterschiedlichen NMR-Signale werden beschrieben.
¹H-NMR (CDCl₃) δ 3.48 (t, J = 8.0 Hz, 2H), 0.91 (m, 1H), 0.53 (d, J = 12 Hz, 2H), 0.11 (d, J = 6 Hz, 2H); ¹³C-NMR (CDCl₃) δ 57.2 (t), 9.8 (d), 4.7 (t), 4.1 (t).

### Beispiel 63:

3-((4a,S,6R,8aS)-6-Hydroxy-3-methoxy-5,6,9,10-tetrahydro-4aH-[1]benzofuro[3a,3,2-ef][2]benzazepin-11(12H)-yl)ethannitril (8r): Verfahren wie in Beispiel E, Reaktionszeit 2 Stunden, Ausbeute 1.67 g (61,1%); Schmelzpunkt 169-171°C; ¹H-NMR und ¹³C-NMR waren identisch mit Verbindung 4ad. Anal. (C₁₈H₂₀N₂O₃.0.67 H₂O) C, H, N.

### Beispiel 64:

1-(2-Phenyl-2,5-diazabicyclo[2.2.1]heptan-5-yl)-2-((4a,S,6R,8aS)-6-Hydroxy-3-methoxy-5,6,9,10-tetrahydro-4aH-[1]benzofuro[3a,3,2-ef][2]benzazepin-11(12H)-yl)-1-ethanon (8s): Verfahren wie in Beispiel E, Reaktionszeit 3 Tage, Ausbeute 0.68 g (37.8%); Schmelzpunkt 85-89°C; α_{D} = -169.3°, gleiches Gerüst wie 4aa, nur unterschiedliche NMR-Signale werden beschrieben: ¹H-NMR (CDCl₃) δ 7.21 (m, 2H); 6.68 (m, 3H) 5.0 (s, 1H), 4.47 (d, J = 14.0 Hz, 1H), 3.90 (m, 1H), 3.63 (m, 3H), 3.24 (m, 1H), 2.04 (m, 3H); ¹³C-NMR (CDCl₃) δ 168.0 und 167.6 (s), 146.4 (d), 144.1 (s), 127.7 (d), 127.5 (d), 112.5 (d), 112.4 (d), 67.0 (t), 57.0 und 56.8 (d), 56.8 und 56.6 (t), 51.8 und 51.6 (t), 36.6 (t); 33.7 und 33.6 (t). Anal. (C₂₉H₃₃N₃O₄.0.33 H₂O) C, H, N.

### Beispiel 65:

(4a,S,6R,8aS)-11-(3-Aminoethyl)-3-methoxy-5,6,9,10,11,12-hexahydro-4aH-[1]benzofuro[3a,3,2-ef][2]benzazepin-6-ol (8t): Verfahren nach Beispiel F, Reaktionspartnerverbindung 8r; Reaktionszeit 18 Stunden, Ausbeute 1.01 g (66.1%); Schmelzpunkt 72-75°C; ao = -71.58°, ¹H-NMR und ¹³C-NMR waren identisch mit Verbindung 4af, Anal. (C₁₈H₂₄N₂O₃.0.65 EtOH.0.05 CH₂Cl₂)) C, H, N.

### Beispiel 66:

(4a,S,6R,8aS)-11-(2-Morpholin-4-yl-ethyl)-3-methoxy-5,6,9,10,11,12-hexahydro-4aH-[1]benzofuro[3a,3,2-ef][2]benzazepin-6-ol (8u): Verfahren nach Beispiel E, Reaktionszeit 2 Tage, Ausbeute 1.77 g (63.6%); Schmelzpunkt 101-104°C; α^{D}₂₀ = -110.9°, ¹H-NMR und ¹³C-NMR waren identisch mit Verbindung 4ag. Anal. (C₂₂H₃₀N₂O₄.0.15 CH₂Cl₂) C, H, N.

### Beispiel 67:

(4a,*S*,6*R*,8a*S*)-11-(2-Phenyl-2,5-diazabicyclo[2.2.1]heptan-5-yl-ethyl)-3-methoxy-5,6,9,10,11,12-hexahydro-4aH-[1]benzofuro[3a,3,2-ef][2]benzazepin-6-ol (8v): Verfahren nach Beispiel F, Reaktionspartnerverbindung 8s, Reaktionszeit 30 min, Ausbeute 0.26 g (50.8%); Schmelzpunkt 72-75°C; α^{D}₂₀ = -131.6°, ¹H-NMR und ¹³C-NMR waren identisch mit Verbindung 4ah. Anal. (C₂₉H₃₅N₃O₃.0.35 CH₂Cl₂ .0.5 Et₃N) C, H, N.

### Beispiel 68:

3-((4a,S,6R,8aS)-6-Hydroxy-3-methoxy-5,6,9,10-tetrahydro-4aH-[1]benzofuro[3a,3,2-ef][2]benzazepin-11(12H)-yl)propanacid (8w): Eine Lösung von 8w (0.5 g, 1.25 mmol) wurde in CH₂Cl₂ (20 ml) in Trifluoraceticacid (5 ml) zugegeben. Die Reaktionsmischung wurde bei Raumtemperatur 2 Stunden gerührt und ergab ein Konzentrat von 0.37 g (64.5%) von 8w: gleiches Gerüst wie 4aa, nur unterschiedliche NMR-Signale werden beschrieben: ¹H-NMR (CDCl₃) 8.95 (b, 1H), 2.82 (m, 2H), 2.25 (m, 2H).

### Beispiel 69:

t-Butyl-3-((4a,*S*,6*R*,8a*S*)-6-Hydroxy-3-methoxy-5,6,9,10-tetrahydro-4aH-[1]benzofuro[3a,3,2-ef][2]benzazepin-11(12H)-propanoat (8x): Verfahren B, Reaktionszeit 6 Stunden, Ausbeute 0.74 g (53.2%); ¹H-NMR und ¹³C-NMR waren identisch mit Verbindung 4aj.

### Beispiel 70:

(4a,S,6R,8aS)-11-(3-Hydroxypropyl)-3-methoxy-5,6,9,10,11,12-hexahydro-4aH-[1]benzofuro[3a,3,2-ef][2]benzazepin-6-ol (8z): Verfahren E, Reaktionszeit 4 Tage, Ausbeute 0.32 g (56.7%); α_{D} = 90.0°; ¹H-NMR und ¹³C-NMR waren identisch mit Verbindung 4an.

### Beispiel 71:

(4a,S,6R,8aS)-11-((3-Dimethylamino)propyl-3-methoxy-5,6,9,10,11,12-hexahydro-4aH-[1]benzofuro[3a,3,2-ef][2]benzazepin-6-ol (8aa): Verfahren E, Reaktionszeit 24 Stunden,

Ausbeute 0.45 g (51.6%); selbes Gerüst wie 4aa, nur die unterschiedlichen NMR-Signale werden beschrieben.
¹H-NMR (CDCl₃) δ 2.50 (m, 2H), 2.30 [m, 2H), 2.22 (s, 3H), 2.18 [s, 3H), 1.82 (m, 2H); ¹³C-NMR (CDCl₃) δ 55.6 (t), 53.4 (t), 45.0 (q); 25.3 (t).

### Beispiel 73:

(4a,S,6R,8aS)-11-(3-2-(4-Fluor)phenyl-2,5-diazabicyclo[2.2.1]heptan-5-yl-propyl)-3-methoxy-5,6,9,10,11,12-hexahydro-4aH-[1]benzofuro[3a,3,2-ef][2]benzazepin-6-ol (8ac): Verfahren E, Reaktionszeit 4 Tage, Ausbeute 1.77 g (59.3%); Schmelzpunkt 84-92°C; α^{D}₂₀ = -50.64; ¹H-NMR und ¹³C-NMR waren identisch mit Verbindung 4aq. Anal. (C₃₀H₃₆FN₃O₃.2CH₂Cl₂.2 Et₃N) C, H, N.

### Beispiel 74:

(4a,R,6S,8aR)-3-Methoxy-5,6,9,10,11,12-hexahydro-4aH-[1]benzofuro[3a,3,2-ef][2]benzazepin-6-ol, (+) Norgalanthamin (9) wurde angefertigt und beschrieben für 8: Ausbeute 5.35 g (74.2%); ¹H-NMR und ¹³C-NMR waren identisch mit Verbindung 8.

### Beispiel 75:

(4a,R,6S,8aR)-6-Hydroxy-N¹¹-isopropyl-3-methoxy-5,6,9,10-tetrahydro-4aH-[1]benzofuro[3a,3,2-ef][2]benzazepin-11(12H)-corboxamid (9a): Verfahren A, Reaktionszeit 3 Stunden; Ausbeute 1.03 g (79%); ¹H-NMR und ¹³C-NMR waren identisch mit Verbindung 41.

### Beispiel 76:

(4a,R,6S,8aR)-N¹¹-t-Butyl-6-hydroxy-3-methoxy-5,6,9,10-tetrahydro-4aH-[1]benzofuro[3a,3,2-ef][2]benzazepin-11(12H)-carboxamid (9b): Verfahren A, Reaktionszeit 3 Stunden; Ausbeute 0.85 g (63%); ¹H-NMR und ¹³C-NMR waren identisch mit Verbindung 4m.

### Beispiel 77:

(4a,R,6S,8aR)-3-Methoxy-11-(2-morpholin-4-yl-ethyl)-5,6,9,10,11,12-hexahydro-4aH-[1]benzofuro[3a,3,2-ef][2]benzazepin-6-ol (9c): Verfahren E, Reaktionszeit 2 Tage: Ausbeute 0.12 g (53.2%); ¹H-NMR und ¹³C-NMR waren identisch mit Verbindung 4ag.

### Beispiel 78:

(4a,R,6S,8aR)-11-((3-Dimethylamino)propyl)-3-methoxy-5,6,9,10,11,12-hexahydro-4aH-[1]benzofuro[3a,3,2-ef][2]benzazepin-6-ol (9d): Verfahren E, Reaktionszeit 22 Stunden; Ausbeute 0.19 g (44.6%); ¹H-NMR und ¹³C-NMR waren identisch mit Verbindung 8aa.

### Beispiel 79:

(4a,R,6S,8aR)-11-(3-piperidin-1-yl-propyl)-3-methoxy-5,6,9,10,11,12-hexahydro-4aH-[1]benzofuro[3a,3,2-ef][2]benzazepin-6-ol (9e): Verfahren E, Reaktionszeit 20 Stunden: Ausbeute 0.33 g (75.0%); ¹H-NMR und ¹³C-NMR waren identisch mit Verbindung 4ap.

### Beispiel 80:

### Schritt 1:

### 2-Brom-4-methoxy-5-(1-methylethoxy)benzaldehyd

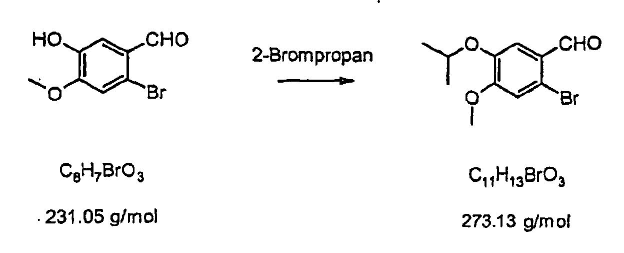

2-Brom-5-hydroxy-4-methoxybenzaldehyd (100.0 g, 433 mmol), 2-Brompropan (160,0 g, 1,30 mol) und Kaliumcarbonat (300 g, 2,16 mol, wasserfrei, frisch gemahlen) werden in Acetonitril (1200 ml) 48 Stunden bei 60°C gerührt.

Das Reaktionsgemisch wird filtriert, das Lösungsmittel am Rotationsverdampfer abdestilliert und der Rückstand zwischen Wasser (800 ml) und Ether (800 ml) verteilt. Die wäßrige Phase wird mit Ether (2 × 300 ml) extrahiert, die vereinigten organischen Phasen werden mit Wasser (2 × 500 ml) und gesättigter Kochsalzlösung (1 × 500 ml) gewaschen, getrocknet (Natriumsulfat/Aktivkohle), filtriert und der noch Abdestillieren des Lösungsmittels erhaltene Rückstand aus Methanol (500 ml) umkristallisiert. Auf diese Weise erhält man das Produkt in Form blaßrosafarbener Kristalle (98,1 g, 83%).
- Schmp.:: 75-76°C
- DC:: Petrolether : Essigsäureethylester = 3:1, Rf = 0,75
¹H NMR (CDCL₃): δ 10.13 (s, 1H), 7.40 (s, 1H), 7.03 (s, 1H), 4.61 (Septett, J = 6.4 Hz, 1H), 3.92 (s, 3 H), 1.38 (d, J = 6.4, Hz 6 H);
¹³ C NMR (CDCL₃): δ 190.8 (d), 155.6 (s), 147.1 (s), 126.4 (s), 120.0 (s), 115.8 (d), 11.3.7 (d), 71.5 (d), 56.4 (q), 21.8 (q)

### Schritt 2:

### 2-Brom-4-methoxy-5-(1-methylethoxy)benzolmethanol

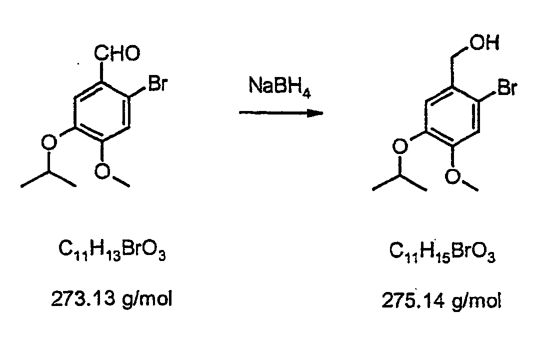

Zu einer Suspension von Natriumborhydrid (1.67 g, 44.1 mmol) in wasserfreiem Ethanol (60 ml) wird bei 15°C 2-Brom-4-methoxy-5-(1-methylethoxy)benzaldehyd (6.0 g, 22.0 mmol) innerhalb von 15 Minuten in Substanz zugegeben und die Mischung eine Stunde bei Raumtemperatur gerührt.

Der nach Abdestillieren des Lösungsmittels verbliebene Rückstand wird zwischen gesättigter Natriumhydrogencarbonatlösung (60 ml) und Ether (100 ml) verteilt. Die wäßrige Phase wird mit Ether (3 × 40 ml) extrahiert, die vereinigten organischen Phasen werden mit gesättigter Natriumhydrogencarbonatlösung (1 × 100 ml), Wasser (1 × 100 ml) und gesättigter Kochsalzlösung (1 × 100 ml) gewaschen, getrocknet Natriumsulfat/Aktivkohle) und filtriert. Nach Abdestillieren des Lösungsmittels am Rotationsverdampfer erhält man das Produkt in Form farbloser Kristalle (5.575 g, 95).
- Schmp.:: 67-69 *°C*
- DC:: Petrolether : Essigsäureethylester = 4:1, Rf = 0,25
¹H NMR (CDCl₃): δ 7.00 (s, 2 H), 4.64 (s, 2 H), 4.50 (Septett, J = 6.4 Hz, 1H), 3.85 (s, 3 H), 2.05 (s, 1 H), 1.34 (d, J = 6.4, Hz 6 H);
¹³ C NMR (CDCl₃): δ 150.1 (s), 146.5 (s), 131.8 (s), 116.1 (s), 115.9 (d), 112.6 (s), 71.7 (t), 64.3 (d), 56.0 (q), 21.8 (q)
MT-44 JOS 1693
C₁₁H₁₅BrO₃

| | | |
|---|---|---|
| Berechnet | C, 48.02; | H, 5.50 |
| Gefunden | C, 48.11; | H, 5.29 |

### Schritt 3:

### 1-Brom-2-(chlormethyl)-5-methoxy-4-(1-methylethoxy)-benzol

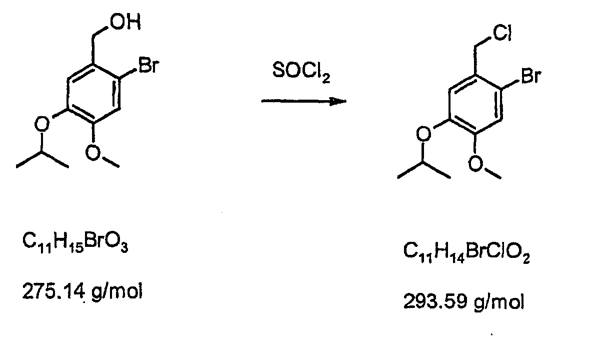

Zu 2-Brom-4-methoxy-5-(1-methylethoxy)benzolmethanol (5.63 g, 20.5 mmol) in absolutem CH₂Cl₂ (60 ml) wird Thionylchlorid (20 ml) in absolutem CH₂Cl₂ (10 ml) innerhalb von 10 Minuten zugetropft und 90 Minuten bei Raumtemperatur gerührt.

Der nach Entfernen des Lösungsmittels am Rotationsverdampfer erhaltene Rückstand wird zwischen Ether (100 ml) und gesättigter Natriumhydrogencarbonatlösung (100 ml) verteilt, die vereinigten organischen Phasen werden mit gesättigter Natriumhydrogencarbonatlösung (2 × 100 ml), Wasser (1 × 100 ml) und gesättigter Kochsalzlösung (1 × 100 ml) gewaschen, getrocknet (Natriumsulfat/Aktivkohle), filtriert und das Lösungsmittel am Rotationsverdampfer abdestilliert. Auf diese Weise erhält man das Produkt in Form farbloser Kristalle (5.72 g, 95%).
- Schmp.:: 68-70°C
- DC: .: Petrolether : Essigsäureethylester = 3:1, Rf = 0,9
¹H NMR (CDCL₃): δ 7.05 (s, 1H), 6.97 (s, 1H), 4.66 (s, 2 H), 4.51 (Septett, J = 6.4 Hz, 1H), 3.85 (s, 3 H), 1.37 (d, J = 6.4, Hz 6 H);
¹³ C NMR (CDCL₃): δ 151.2 (s), 146.8 (s), 128.5 (s), 117.7 (s), 116.1 (d), 114.8 (s), 71.9 (t), 56.2 (d), 64.4 (q), 21.9 (q)
MT-45 JOS 1760
C₁₄H₂₀O₃

| | | |
|---|---|---|
| Berechnet | C, 71.16; | H, 8.53 |
| Gefunden | C, 70.90; | H, 8.28 |

### Schritt 4:

### 1-[4-(1-Methylethoxy)phenyl]ethanon

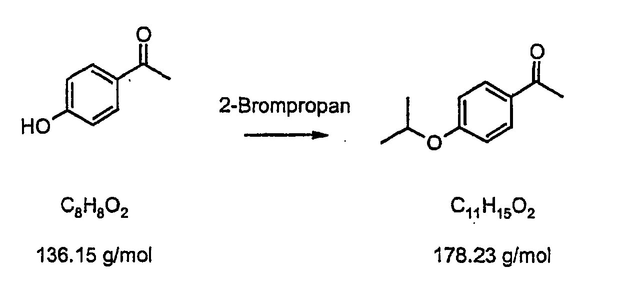

1-(4-Hydroxyphenyl)ethanon (12.7 g, 93.2 mmol), 2-Brompropan (57.3 g, 466 mmol) und Kaliumcarbonat (62.2 g, 466 mmol, wasserfrei, frisch gemahlen) werden in absolutem Acetonitril (150 ml) 24 Stunden bei 60°C gerührt.

Das Reaktionsgemisch wird filtriert, das Lösungsmittel am Rotationsverdampfer abdestilliert und der Rückstand zwischen Wasser (200 ml) und Ether (200 ml) verteilt. Die wäßrige Phase wird mit Ether (2 × 80 ml) extrahiert, die vereinigten organischen Phasen mit Wasser (2 × 100 ml) und gesättigter Kochsalzlösung (1 × 100 ml) gewaschen, getrocknet (Natriumsulfat/Aktivkohle) und filtriert. Auf diese Weise erhält man nach Abdestillieren des Lösungsmittels das Produkt in. Form farbloser Kristalle (16.8 g, 99%).
- Schmp.:: 36-80°C
- DC:: Petrolether : Essigsäureethylester = 4:1, Rf = 0,5
¹H NMR (CDCL₃): δ 7.82 (d, J = 9.5 Hz, 2 H), 6.88 (d, J = 9.5 Hz, 2 H), 4.63 (Septett, J = 6.4 Hz, 1H), 2.52 (s, 3 H), 1.33 (d, J = 6.4 Hz, 6H);
¹³ C NMR (CDCL₃): δ 196.7 (s), 161.9 (s), 130.5 (d), 129.8 (s), 115.0 (d), 70.0 (d), 26.2 (q), 21.8 (q)

### Schritt 5:

### 2-Brom-1-[4-(1-methylethoxy)phenyl]ethanon

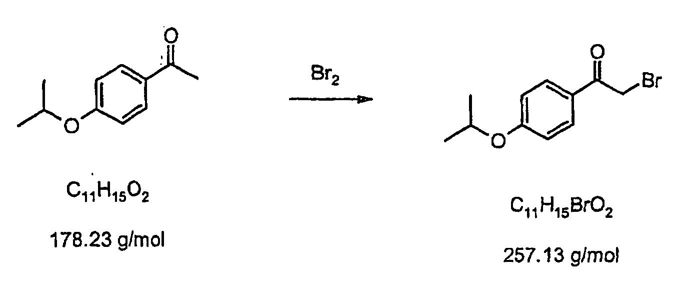

Zu einer Lösung von 1-[4-(1-Methylethoxy)phenyl]ethanon (10.0 g, 56.0 mmol) in absolutem Dioxan (100 ml) wird Brom (11.7 g, 73.5 mmol) in absolutem Dioxan (70 ml)/absolutem Ether (100 ml) innerhalb einer Stunde zugetropft und 2 h bei Raumtemperatur gerührt. Das Reaktionsgemisch wird mit Natriumsulfit (5.0 g) in Wasser (100 ml) versetzt, die Phasen werden getrennt und die wäßrige Ohase mit Ether (3 × 100 ml) extrahiert, die vereinigten organischen Phasen werden mit Wasser (2 × 100 ml), gesättigter Natriumhydrogencarbonatlösung (2 × 150 ml) und gesättigter Kochsalzlösung (1 × 200 ml) gewaschen, getrocknet (Natriumsulfat/Aktivkohle), filtriert und der nach Abdestillieren des Lösungsmittels erhaltene Rückstand unter einem Gemisch aus Petrolether (25 ml) und Cyclohexan (25 ml) bei -20°C kristallisiert. Auf diese Weise erhält man das Produkt in Form farbloser, sich rasch dunkel verfärbender Kristalle (8.80 g, 59%).
- Schmp.:: 36-37°C
- DC:: Petrolether : Essigsäureethylester = 4:1, Rf = 0,7
¹H NMR (CDCL₃): δ 7.93 (d, J = 9.5 Hz, 2 H), 6.92 (d, J = 9.5 Hz, 2 H), 4.63 (Septett, J = 6.4 Hz, 1H), 4.40 (s, 2 H), 1.35 (d, J = 6.4 Hz, 6 H);
¹³ C NMR (CDCL₃): δ 189.8 (s), 162.7 (s), 131.4 (d), 126.4 (s), 115.3 (d), 70.3 (d), 30.7 (t), 21.9 (q)

### Schritt 6:

### 1-(2-Bromethyl)-4-(1-methylethoxy)benzol

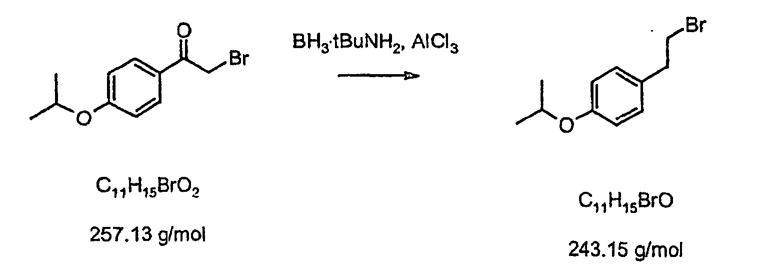

Zu einer Suspension von wasserfreiem Aluminiumchlorid (5.70 g, 43.0 mmol) in absolutem CH₂Cl₂ (100 ml) wird bei 5°C terf.-Butylamin-Borankomplex (7.45 g, 85.0 mmol, Pellets oder Pulver) zugegeben. Nach 15 Minuten wird 2-Brom-1-[4-(1-methylethoxy)phenyl]-ethanon (7.30 g, 28.4 mmol) in wasserfreiem CH₂Cl₂ (50 ml) innerhalb von 30 Minuten zugetropft.

Das Gemisch wird drei Stunden bei Raumtemperatur gerührt, mit 0.1 N Salzsäure (100 ml) versetzt und die wäßrige Phase mit CH₂Cl₂ (2 × 30 ml) extrahiert. Die vereinigten organischen Phasen werden mit 0.1 N Salzsäure (2 × 50 ml), gesättigter Natriumhydrogencarbonatlösung (2 × 50 ml) und gesättigter Kochsalzlösung gewaschen (1 × 100 ml), getrocknet (Natriumsulfat/Aktivkohle) und der nach Eindampfen des Lösungsmittels erhaltene Rückstand durch Kugelrohrdestillation (0.05 mbar/80°C) gereinigt, wobei das Produkt als farbloses Öl (5.81 g, 83%) erhalten wird.
- DC:: Petrolether, Rf = 0,35
¹H NMR (CDCL₃): δ 7.18 (d, J = 9.5 Hz, 2 H), 6.87 (d, J = 9.5 Hz, 2 H), 4.53 [Septett, J = 6.4 Hz, 1H), 3.53 (t, J = 6.9 Hz, 2 H), 3.08 (t, J = 6.9 Hz, 2 H), 1.33 (d, J = 6.4 Hz, 6 H);
¹³ C NMR (CDCL₃): δ 156.7 (s), 130.7 (s), 129.5 (d), 115.8 (d), 69.8 (d), 38.5 (t), 33.2 (t), 21.9 (q)
MT-35 JOS 1760
C₁₁H₁₅BrO

| | | |
|---|---|---|
| Berechnet | C, 54.34; | H, 6.22 |
| Gefunden | C, 54.34; | H, 6.09 |

### Schritt 7:

### 2-[2-[4-(1-Methylethoxy)phenyl]ethyl]propandisäuredimethylester

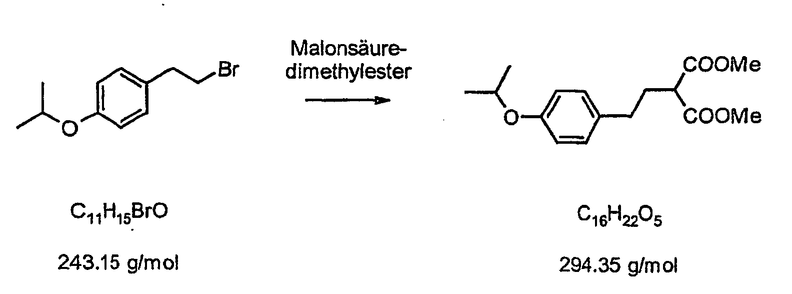

1-(2-Bromethyl)-4-(1-methylethoxy)benzol (19.0 g, 78.1 mmol), Malonsäuredimethylester (40.0 g, 300 mmol) und Kaliumcarbonat (42.0 g, 300 mmol, wasserfrei, frisch vermahlen) in absolutem DMF (400 ml) werden 10 Stunden bei 70°C gerührt.

Das Lösungsmittel wird im Rotationsverdampfer entfernt und der Rückstand zwischen Wasser (250 ml) und Ether (250 ml) verteilt. Die wäßrige Phase wird mit Ether (1 × 100 ml) extrahiert, die vereinigten organischen Phasen mit Wasser (3 × 200 ml) und gesättigter Kochsalzlösung (1 × 150 ml) gewaschen, getrocknet (Natriumsulfat/Aktivkohle) und überschüssiger Malonsäuredimethylester aus dem der nach Entfemen des Lösungsmittels am Rotationsverdampfer erhaltenen Rückstand abdestilliert (160°C/15 mbar).

Das Rohprodukt wird mittels Kugelrohrdestillation (140°C/0,001 mbar) gereinigt, wodurch das Produkt als farbloses Öl erhalten wird (18.9 g, 82%).
- DC:: Petrolether : Essigsäureethylester = 9:1, Rf = 0,4
¹H NMR (CDCL₃): δ 7.08 (d, J = 10 Hz, 2 H), 6.81 (d, J = 10 Hz, 2 H), 4.50 (Septett, J = 6.5 Hz, 1H), 3.71 (s, 6H), 3.32 (t, J = 7.5 Hz, 1H), 2.58 (t, J = 7.5 Hz, 2H), 2.19 (q, J = 7.5 Hz, 2H), 2.65 - 2.47 (m, 2H), 2.26 - 2.06 (m, 2H), 1.31 (d, J = 6 Hz, 6H);
¹³ C NMR (CDCL₃): δ 169.8 (s), 156.3 (s), 132.3 (s), 129.4 (d), 115.9 (d), 69.9 (d), 52.5 (q), 50.8 (d), 32.4 (t), 30.6 (t), 22.1 (q)

### Schritt 8:

### 2-[2-Brom-4-methoxy-5-(1-methylethoxy)phenyl]methyl-2-[2-[4-(1-methylethoxy)phenyl]ethyl]propan- disäuremethylester

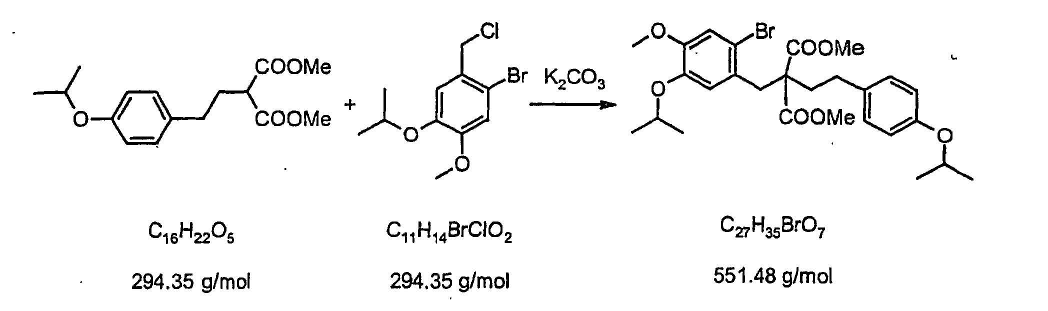

2-[2[4-(1-Methylethoxy)phenyl]ethyl]propandisäuredimethylester (18.9 g, 64.2 mmol), 1-Brom-2-(chlormethyl)-4-(1-methylethoxy)-5-methoxybenzol (18.9 g, 64.2 mmol) und Kaliumcarbonat (45.0 g, 32 I mmol, wasserfrei, frisch vermahlen) in absolutem DMF (300 ml) werden 12 Stunden bei 60°C gerührt.

Der nach Entfernen des Lösungsmittels verbleibende Rückstand wird zwischen Wasser (250 ml) und Ether (250 ml) verteilt. Die wäßrige Phase wird mit Ether (1 × 100 ml) extrahiert, die vereinigten organischen Phasen werden mit Wasser (3 × 200 ml) und gesättigter Kochsalzlösung (150 ml) gewaschen, getrocknet (Natriumsulfat/Aktivkohle), filtriert und das Lösungsmittel am Rotationsverdampfer entfernt.

Nach Trocknen am Hochvakuum erhält man das Produkt als farbloses Öl (33.7 g, 95%).
- DC:: Petrolether : Essigsäureethylester = 9:1, Rf = 0,5
¹H NMR (CDCl₃): δ 7.04 (s, 1H), 7.01 (d, J = 10 Hz, 2H), 6.79 (d. J = 10 Hz, 2H), 6.73 (s, 1H); 4.47 (Septett, J = 6.5 Hz, 1H), 4.36 (Septett, J = 6.5 Hz, 1H), 3.80 (s, 3H), 3.72 (s, 6H), 3.48 (s, 2H), 2.65 - 2.47 (m, 2H), 2.26 - 2.06 (m, 2H), 1.31 (d, J = 6.5 Hz, 12 H);
¹³ C NMR (CDCL₃): δ 171.4 (s), 156.1 (s), 149.8 (s), 146.2 (s), 133.0 (s), 129.3 (s), 129.1 (d), 118.2 (s), 116.2 (d), 116.0 (d), 115.8 (d), 69.7 (d), 58.7 (s), 55.9 (q), 52.3 (q), 37.4 (t), 34.5 (t), 29.9 (t), 21.95 (q), 21.9 (q)
MT-54 JOS 1698
C₂₇H₃₅BrO₇

| | | |
|---|---|---|
| Berechnet | C, 58.81; | H, 6.40 |
| Gefunden | C, 59.00; | H, 6.26 |

### Schritt 9:

### Alpha-[[2-Brom-4-methoxy-5-(1-methylethoxy)phenyl]methyl]-4-(1-methylethoxy)benzolbutansäure

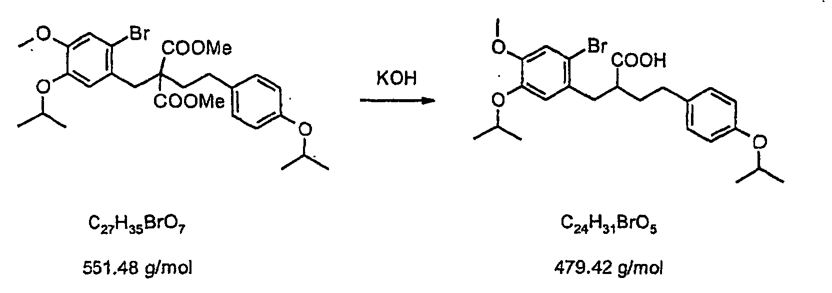

2-[2-Brom-4-methoxy-5-(1-methylethoxy)phenyl]methyl-2-[2-[4-(1-methylethoxy)-phenyl]ethyl]propandisäuredimethylester (33.7 g, 61.1 mmol) und Kaliumhydroxid (17.5 g, 312 mmol) werden in Ethanol (150 ml)/Wasser (30 ml) 12 Stunden bei Siedetemperatur gerührt.

Das Reaktionsgemisch wird mit konzentrierter Salzsäure bis auf einen pH von 1 angesäuert und eine Stunde unter Rückfluß gehalten.

Der nach Entfernen des Lösungsmittels verbliebene Rückstand wird zwischen Wasser (250 ml) und Ether (250 ml) verteilt. Die wäßrige Phase wird mit Ether (2 × 100 ml) extrahiert, die vereinigten organischen Phasen werden mit Wasser neutralgewaschen, mit gesättigter Kochsalzlösung (150 ml) gewaschen, getrocknet (Natriumsulfat/Aktivkohle) und filtriert. Der nach Entfernen des Lösungsmittels verbliebene Rückstand wird im Kugelrohr 30 Minuten bei 140°C decarboxyliert und anschließend bei 150°C/0,005 mbar destilliert. Auf diese Weise wird das Produkt in Form farbloser Kristalle (27.5 g, 94%) erhalten.
- Schmp.:: 114-116°C
- DC:: Chloroform : Methanol = 9:1, Rf = 0.65
¹H NMR (DMSO-d₆): δ 7.09, (s, 1H), 7.01 (d, J = 7.3 Hz, 2H), 6.80 (s, 1H), 6.78 (d, J = 7.3 Hz, 2H), 4.69 - 4.37 (m, 2H), 372 (s, 3H), 3.00 - 2.33 (m, 5H), 1.99 - 1.58 (m, 211),1.18 (d, J = 6.4 Hz, 12H);
¹³ C NMR (DMSO₆): 176.0 (s), 155.6 (s), 149.3 (s), 145.8 (s), 133.1 (s), 130.3 (s), 129.1 (d), 118.1 (d), 116.0 (d), 115.5 (s), 114.1 (d), 70.6 (d), 69.0 (d), 55.8 (q), 44.9 (d), 33.5 (t), 31.9 (t), 21.85 (q), 21.8 (q)
MT-100 JOS 1592
C₂₄H₃₁BrO₅

| | | |
|---|---|---|
| Berechnet: | C, 60.13; | H, 6.52 |
| Gefunden: | C, 60.38; | H, 6.55 |

### Schritt 10:

### Alpha[[2-Brom-4-methoxy-5-(1-methylethoxy)phenyl]methyl]-4-(1-methylethoxy)benzolbutansäureamid

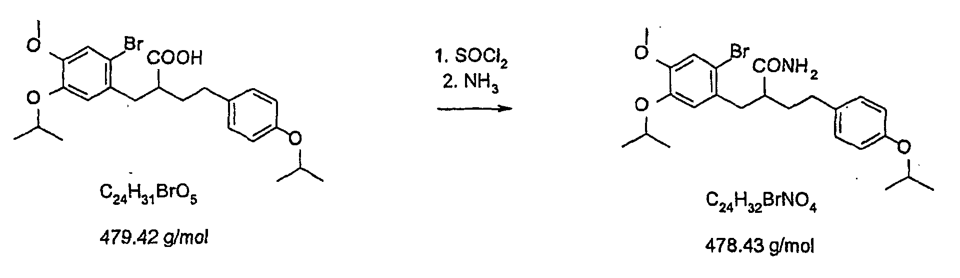

Zu Alpha[[2-Brom-4-methoxy-5-(1-methylethoxy)phenyl]methyl]-4-(1-methylethoxy)-benzoibutansäure (10.0 g, 20.8 mmol) in CH₂Cl₂ (100 ml) wird Thionylchlorid (50 ml) bei 0°C innerhalb von 15 Minuten zugetropft und das Gemisch zwei Stunden bei dieser Temperatur gerührt.

Das Lösungsmittel wird am Rotationsverdampfer entfernt, der Rückstand in absolutem Formamid (15 ml) aufgenommen und bei 0°C mit Ammoniak in Formamid (100 ml einer bei dieser Temperatur gesättigten Lösung) versetzt. Das Gemisch wird eine Stunde bei 0°C gerührt und auf Wasser (1500 ml) gegossen.

Die ausgefallenen Kristalle werden abfiltriert und mit Wasser (4 x 400 ml) digeriert. Auf diese Art wird das Produkt in Form farbloser Kristalle (9.21 g, 92%) erhalten.
- Schmp.:: 154-156°C
- DC:: CH₂Cl₂ : Methanol = 9 : 1, Rf = 0.7
¹H NMR (DMSO-d₆): δ 7.32 (s, 1H), 7.08 (s, 1H), 7.02 (d, J = 7.3 Hz, 2H), 6.83 (s, 1H), 6.80 (s, 1H), 6.78 (d, J = 7.3 Hz, 2H), 4.68 - 4.32 (m; 2H), 3.77 (s, 3H), 3.39 (s, 3H), 3.00 - 2.62 (m, 2H), 2.00 - 1.58 (m, 2H), 1.18 (d, J = 6.4 Hz, 12H);
¹³C NMR (DMSO-d₆): 175.8 (s), 155.5 (s), 149.1 (s), 145.8 (s), 133.5 (s), 130.9 (s), 129.9 (d), 118.1 (d); 115.8 (d), 115.5 (s), 114.1 (d), 70.9 (d), 69.0 (d), 55.8 (q), 45.9 (d), 34.2 (t), 32.1 (t), 21.85 (q), 21.8 (q)
MT-112 JOS 1591
C₂₄H₃₂BrNO₄

| | | | |
|---|---|---|---|
| Berechnet | C, 60.25; | H, 6.74; | N, 2.93 |
| Gefunden | C, 59.99; | H, 6.56; | N, 2.82 |

### Schritt 11:

### Herstellung von Alpha[[2-Brom-5-hydroxy-4-methoxyphenyl]methyl]-4-hydroxybenzolbutansäureamid

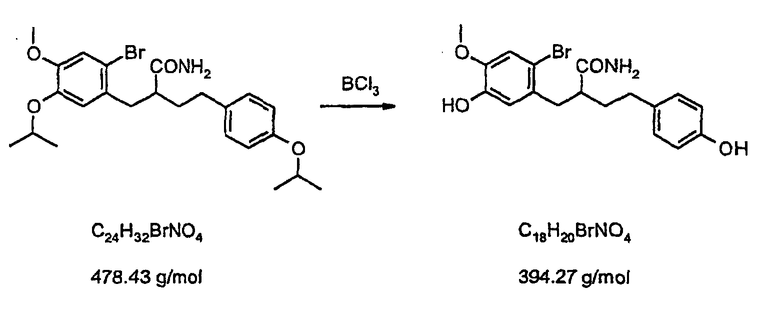

Zu einer Lösung von Alpha[[2-Brom-5-hydroxy-4-methoxyphenyl]methyl]-4-hydroxybenzolbutansäureamid (9.30 g, 19.4 mmol) in absolutem CH₂Cl₂ (150 ml) wird bei-78°C Bortrichlorid (40 ml, 1.6 M in CH₂Cl₂) zugetropft und eine Stunde bei dieser Temperatur gerührt. Danach wird das Gemisch auf Raumtemperatur erwärmt und zwei Stunden gerührt.

Man versetzt mit Wasser (300 ml) und destilliert das organische Lösungsmittel am Rotationsverdampfer ab, wobei das Rohprodukt kristallin ausfällt, das abfiltriert und mit Wasser (6 x 200 ml) und Diisopropylether (2 x 40 ml) digeriert wird. Dabei erhält man das Produkt in Form farbloser Kristalle (6.76 g, 88%).
- Schmp.:: 177 - 179°C
- DC:: CH₂Cl₂ : Methanol = 9 : 1, Rf = 0.4
¹H NMR (DMSO-d₆): δ 9.18 (s, 2H), 7.18 (s, III), 7.04 (s, 1H), 6.97 (d, J = 7.3 Hz, 2H), 6.72 (s, 1H), 6.65 (s, 1H), 6.66 (d, J = 7.3 Hz, 2H), 3.77 (s, 3H), 3.48 (s, 3H), 2.92 - 2.38 (m, 4H);
¹³C NMR (DMSO-d₆): δ 175.6 (s), 155.5 (s), 147.0 (s), 145.8 (s), 131.3 (s), 129.9 (s), 129.8 (d), 117.9 (s), 115.8 (d), 115.0 (d), 11.9 (d), 56.0 (q), 48.1 (d), 37.6 (t), 37.0 (t)
MT-114 JOS 1692
C₁₈H₂₀BrO₄

| | | | |
|---|---|---|---|
| Berechnet | C, 54.84; | H, 5.11; | N, 3.55 |
| Gefunden | C, 54.55; | H, 4.90; | N, 3.28 |

### Schritt 12:

### 1-Brom-4a, 5, 9, 10, 11, 12-hexahydro-3-methoxy-6-oxa-6H-benzo[a]cyclohepta[hi]benzofuran-11-carbonsäureamid

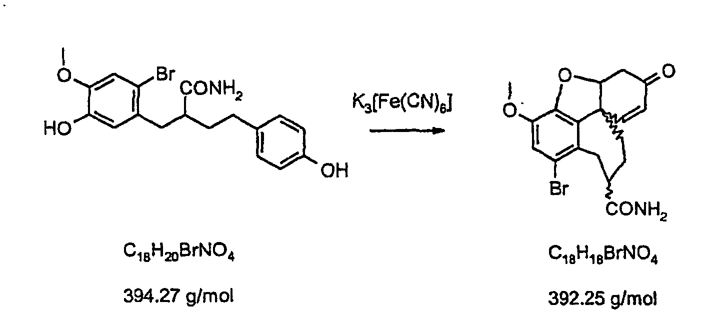

α-[[2-Brom-5-hydroxy-4-methoxyphenyl]methyl]-4-hydroxybenzolbutansäureamid (3.00 g, 7.61 mmol) wird in Chloroform (300 mL) suspendiert und mit einer Lösung von Kaliumhexacyanoferrat(III) (13.2 g, 40.0 mmol) in Kaliumcarbonatlösung (75 mL, zehnprozentig) versetzt.

Das Gemisch wird bei Raumtemperatur 40 Minuten heftig gerührt und über Hyflo filtriert. Die wäßrige Phase wird mit Chloroform (3 × 50 mL) extrahiert, die vereinigten organischen Phasen werden mit Wasser (2 × 200 mL) und gesättigter Kochsalztösung (1 × 150 mL) gewaschen, getrocknet (Natriumsulfat/Kieselgel), filtriert und das nach Eindampfen des Lösungsmittels erhaltene Rohprodukt durch Säulenchromatographie (50 g Kieselgel, Essigsäureethylester) gereinigt. Auf diese Weise erhält man das Produkt in Form farbloser Kristalle (0.36 g, 12 %).
- DC:: Chloroform: Methanol = 9 : 1, Rf =0.4 und 0.5
¹H NMR (CDCl₃): δ 7.00 (s, 1H); 6.86 (dd, *J* = 12 Hz, *J* = 1Hz, 1H), 6.06 (d, *J* = 1 Hz, 1H), 5.02 (bs, 2H), 4.70 (s, 1H), 3.82 (s, 3H), 3.62 (d, *J* = 16 Hz, 1H), 3.23 (dd, *J* = 16 Hz, *J* = 3 Hz, 1H), 3,08 - 2.89 (m, 1H), 2.77 (dd, J = 16 Hz, J = 6 Hz, 1H), 2.62 - 1.70 (m, 5H)
¹³C NMR (DMSO-d₆): δ 202.5 (s), 184.9 und 179.1 (s), 146.5 und 146.1 (d), 145.0 und 145.9 (s), 143.3 und 142.0 (s), 132.0 und 131.8 (s), 128.9 und 128.0 (s), 126.7 und 126.2 (d), 116.3 und 115.0 (s), 114.4 (d), 87.4 und 87.3 (d), 56.0 (q), 49.5 und 49.3 (s), 45.3 (d), 37.3 und 37.0 (t), 35.4 (t), 34.4 (t), 30.4 (t)
Tieferlaufendes Diastereomeres
¹H NMR (CDCl₃): δ 6.70 - 6.85 (m, 2H), 6.07 - 5.91 (m, 2H), 4.54 (s, 1H), 4.12 (s, 1H), 3.82 (s, 3H), 2.99 (s, 1H), 2.86 (t, J = 15 HZ, 1H), 2.72 (d, J = 16 Hz, 1H), 2.63 (dd, J = 16 Hz, J = 3 Hz, 1H), 2.30 - 1.60 (m, 9H);
¹³C NMR (CDCl₃): δ 146.4 (s), 143.9 (s), 133.7 (s), 128.5 (s), 128.1 (d), 127.5 (d), 123.3 (d), 111.7 (d), 88.9 (d), 62.4 (d), 56.3 (q), 52.8 (d), 48.3 (s), 45.1 (t), 35.8 (t), 35.6 (t), 30.4 (t)
MT-115 JOS 1585
C₁₈H₁₈BrNO₄

| | | | |
|---|---|---|---|
| Berechnet | C, 55.12; | H, 4.63; | N, 3.57 |
| Gefunden | C, 54.91; | H, 4.66; | N, 3.41 |

### Beispiel 81:

### 1-Brom-4a, 5, 9, 10, 11, 12-hexahydro-3-methoxy-6-hydroxy-6Hbenzo[a]cyclohepta[hi]benzofuran-11-carbonsäureamid

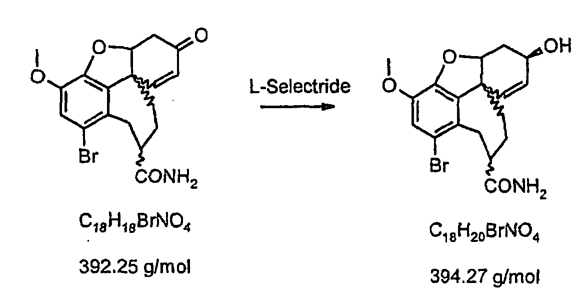

Zu einer Suspension von 1-Brom-4a, 5, 9, 10, 11, 12-hexahydro-3-methoxy-6-oxa-*6H*benzo[a]cyclohepta[hi]benzofuran-11-carbonsäureamid (860 mg, 2.19 mmol) in absolutem THF (5 mL) wird bei 0 °C L-Selectride® (6.6 mL, 6.6 mmol, 1 M in THF) innerhalb von 15 Minuten zugegeben und das Gemisch 12 Stunden bei Raumtemperatur gerührt. Man hydrolysiert mit Wasser (3 mL) und verteilt zwischen Wasser (10 mL) und Essigsäureethylester (10 mL), extrahiert die wäßrige Phase mit Essigsäureethylester (3 × 5 mL), wäscht die vereinigten organischen Phasen mit 1 N Salzsäure (3 × 10 mL), Wasser (2 × 10 mL), gesättigter Natriumhydrogencarbonatlösung (1 × 10 mL) und gesättigter Kochsalzlösung (1 × 10 mL), trocknet (Natriumsulfat/Aktivkohle), filtriert und reinigt das nach Abdestillieren des Lösungsmittels erhaltene Rohprodukt durch Säulenchromatographie (50 g Kieselgel, Essigsäureethylester). Auf diese Weise erhält man das Produkt in Form farbloser Kristalle (741 mg, 86 %).
- DC:: Chloroform : Methanol = 9 : 1, Rf =0.35 und 0.45
¹H NMR (CDCl₃): δ 6.92 (s, 1H), 6.10-5.89 (m, 2H), 5.82 - 5.53 (m, 2H), 4.54 (s, 1H), 4.13 (s, 1H), 3.81 (s, 3H), 3.51 (d, *J* = 15 Hz, 1H), 3.05 (dd, *J* = 17 Hz, *J* = 6 Hz, 1H), 2.96 - 2.84 (m, 1H), 2.65 (d, *J* = 16 Hz, 1H), 2.83 (dd, *J* = 16 Hz, *J* = 6 Hz, 1H), 2.44 - 1.40 (m, 9H);
¹³C NMR (CDCl₃): δ 177.7 und 175.2 (s), 145.3 (s), 145.7 (s), 144.2 (s) und 143.9 (s), 133.8 und 134.2 (s), 128.3 und 128.2 (d), 126.5 (d), 116.1 und 115.9 (s), 115.3 und 115.1 (d), 88.5 (d), 61.8 (d), 56.1 (q), 49.1 und 49.0 (s), 46.0 (d), 41.9 (t), 35.9 und 35.7 (t), 29.8 und 29.6 (t), 28.8 und 26.2 (t)
MT-120
JOS 1710
C₁₈H₂₀BrNO₄

| | | | |
|---|---|---|---|
| Berechnet | C, 54.84; | H, 5.11; | N, 3.55 |
| Gefunden | C, 54.84; | H, 5.18; | N, 3.43 |

### Beispiel 82:

### 11-Amino-1-brom-4a,5,9,10,11,12-hexahydro-3-methoxy-6-hydroxy-6Hbenzo[a]cyclohepta[hi]benzo-furan-6-ol (SPH-1459)

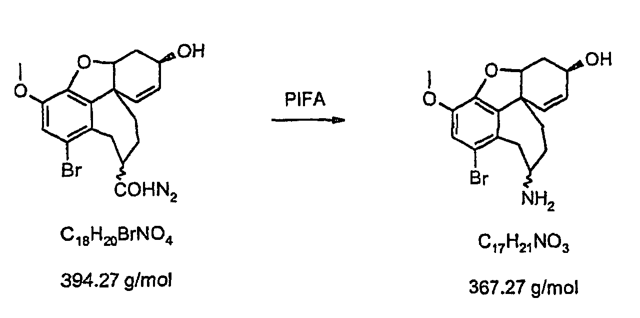

Bis(trifluoracetoxy)iodbenzol (PIFA, 787 mg, 1.78 mmol) wird in Acetonitril (3.5 ml, HPLC-Qualität) gelöst und mit Wasser (3.5 ml, HPLC-Qualität) versetzt. Danach wird 1-Brom-4a,5,9,10,11,12-hexahydro-3-methoxy-6-hydroxy-6H-benzo[a]cyclohepta[hi]benzofuran-11-carbonsäureamid innerhalb von 2 Stunden in Substanz zugegeben und das Gemisch 24 Stunden bei Raumtemperatur gerührt. Das Lösungsmittel wird am Rotationsverdampfer abdestilliert, der Rückstand in Chloroform (5 ml) aufgenommen, filtriert und durch Säulenchromatographie (30 g Kieselgel, Chloroform: Methanol : Ammoniak = 96 : 3 : 1) gereinigt. Auf diese Weise erhält man das Produkt in Form farbloser Kristalle (490 mg, 75%).
- DC:: Chloroform : Methanol = 9 : 1, Rf = 0.2 und 0.25
¹H NMR (MeOH-d₄): δ 7.07 (s, 1H), 6.12 - 5.87 (m, 2H), 5.82 - 5.53 (m, 2H), 4.53 (s, 1H), 4.14 (s, 1H), 3.80 (s, 3H), 3.59 (d, J = 20 Hz, 1H), 3.14 - 2.92 (m, 1H), 2.47 (d, J = 17 Hz, 1H), 2.16 (s, 3H), 2.01 - 2.62 (m, 2H);
¹³ C NMR (MeOH-d₄): δ 148.3 und 148.2 (s), 146.5 und 146.1 (s), 135.8 (s), 129.9 und 129.3 (s), 128.5 und 127.9 (d), 125.9 und 123.9 (d), 118.4 und 118.1 (s), 116.9 und 116.0 (d), 118.4 und 118.0 (s), 116.8 und 116.0 (d), 89.0 und 88.9 (d), 62.4 und 62.3 (d), 57.2 (q), 50.6 und 50.4 (s), 49.8 (d), 38.5 (t), 36.0 und 33.0 (t), 31.8 und 31.0 (t), 31.4 und 28.3 (t)
JOS 1707
C₁₇H₂₀BrNO₃∗1 CHCl₃

| | | | |
|---|---|---|---|
| Berechnet | C, 44.52; | H, 4.36; | N, 2.88 |
| Gefunden | C, 44.90; | H, 4.30; | N, 2.67 |

### Beispiel 83:

### 11-Amino-4a, 5,9,10,11,12-hexahydro-3-methoxy-6-hydroxy-6H benzo[a]cyclohepta[hi]benzofuran-6-ol

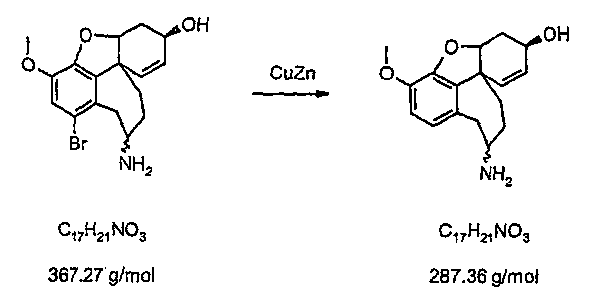

### Herstellung der Kupfer-Zink-Legierung

Zinkpulver (600 mg) und Kupfer(I)iodid werden unter Argon in Wasser (4 mL) und Ethanol (4 mL) 45 Minuten im Ultraschallbad umgesetzt, wobei eine tiefschwarze, feinpulvrige Suspension entsteht.

### Debromierung

11-Amino-1-brom-4a, 5, 9, 10, 11, 12-hexahydro-3-methoxy-6-hydroxy-6H-benzo [a]cyclohepta[hi]benzofuran-6-ol (80 mg, 0.22 mmol) und Calciumchlorid (300 mg, 2.7 mmol) werden in Substanz zu der entstandenen Suspension zugegeben und das Gemisch 12 Stunden bei Siedetemperatur gerührt. Man versetzt mit konzentrierter wäßriger Ammoniaklösung (1 mL), entfernt das Lösungsmittel am Rotationsverdampfer, nimmt den Rückstand in Chloroform (15 mL) auf, filtriert und reinigt den nach Eindampfen des Filtrats am Rotationsverdampfer erhaltenen Rückstand durch Säulenchromatographie (30 g Kieselgel, Chloroform : Methanol : Ammoniak = 96 : 3 : 1). Auf diese Weise lassen sich beide optischen Isomere trennen (10 mg, 0.04 mmol Isomer A; 26 mg, 0.09 mmot Isomer B; gesamt 36 mg, 59 %) und als farbloser Schaum gewinnen. Höherlaufendes Diastereomeres
¹H NMR (CDCl₃): δ 6.73-6.62 (m, 2H), 6.05 (s, 2H), 4.62 (s, 1H), 4.14 (s, 1H), 3.82 (s, 3H), 3.57 (s, 1H), 3.22 (d, J *=* 16 Hz, 1H), 2.83 (dd, J = 16 Hz, J = 6.5 Hz, 1H), 2.24 - 1.60 (m, 9H);
¹³C NMR (CDCl₃): δ 146.2 (s), 144.3 (s), 133.6 (s), 128.4 (s), 128.1 (d), 127.2 (d), 124.8 (d), 111.9 (d), 89.0 (d), 62.6 (d), 56.3 (q) 49.0 (s), 48.3 (d), 41.8 (t), 32.5 (t), 30.4 (t), 30.4 (t)
Tieferlaufendes Diastereomeres
¹H NMR (CDCl₃): δ 6.70-6.58 (m, 2H), 6.07-591 (m, 2H), 4.54 (s, 1H), 4.12 (s, 1H), 3.82 (s, 3H), 2.99 (s, 1H), 2.86 (t, J = 15 Hz, 1H), 2.72 (d, J = 16 Hz, 1H), 2.63 (dd, J = 16 Hz, J = 3 Hz, 1H), 2.30-1.60 (m, 9H);
¹³C NMR (CDCl₃): δ 146.4 (s), 143.9 (s), 133.7 (s), 128.5 (s), 128.1 (d), 127.5 (d), 123.3 (d), 111.7 (d), 88.9 (d), 62.4 (d), 56.3 (q) 52.8 (d), 48.3 (s), 45.1 (t), 35.8 (t), 35.6 (t), 30.4 (t).

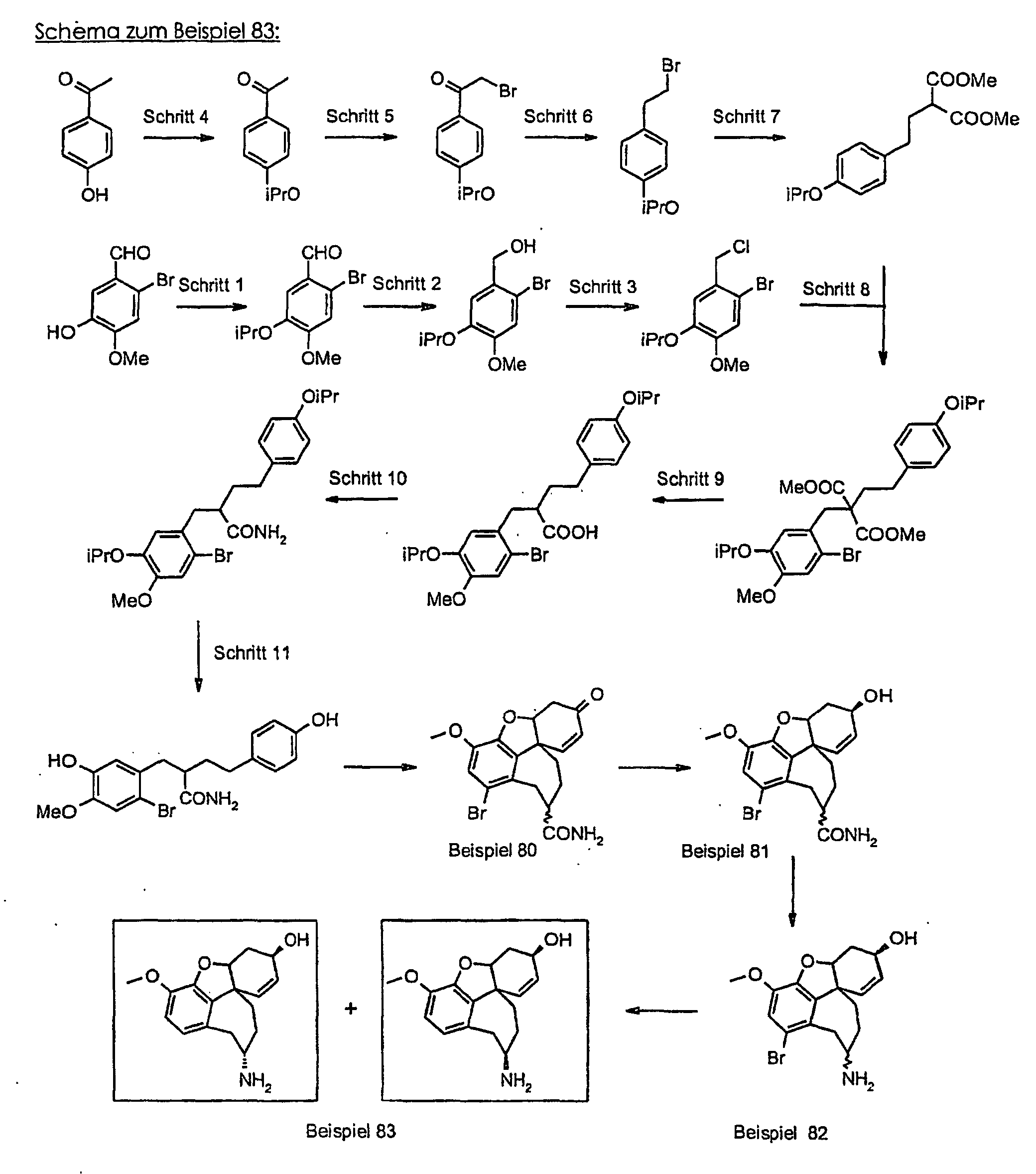

### Beispiel 84:

### Schritt 1:

### Kondensation und Reduktion: Allgemeine Arbeitsvorschrift

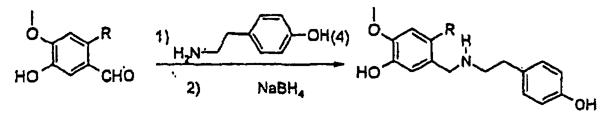

1 eq. 2-Methyl-4-methoxy-5-hydroxybenzaldehyd
bzw. 2,4-Dimethoxy-5-hydroxybenzaldehyd

| | |
|---|---|
| 1 eq. | Tyramin |
| 0.8. eq | Natriumborhydrid |
| 10 ml | Ethanol (96%) / 1g Aldehyd |

Das Edukt wurde in Ethanol suspendiert und unter Rühren das Tyramin zugegeben, dann wurde das Reaktionsgemisch 8.5 h auf Rückfluß erhitzt. Da sich die gebildete Schiff'sche Base (MH-16' bzw. 34') auf der DC-Platte wieder in die Ausgangsmaterialien zerlegt, wurde der Reaktionsfortgang mittels Reduktion einer kleinen Probe mit Natriumborhydrid, üblicher Aufarbeitung und Auftragen des erhaltenen Produkts ermittelt.

Noch 8.5 h wurde das Reaktionsgemisch im Eisbad auf 0°C abgekühlt und das Natriumborhydrid, gelöst in 4 ml Wasser / 1 g, langsam zugetropft, dann im Eisbad 30 min gerührt. Anschließend wurde unter heftigem Rühren auf 150 ml Eis/Wasser / 1 g Aldehyd gegossen, der entstandene weiße Niederschlag abfiltriert und im Vokuumtrockenschrank getrocknet. Aus der Mutterlauge fiel eine zweite Fraktion des Produkts aus, welche gesammelt und getrocknet wurde.

### Schritt 1a:

### 5-(N-[2-[4-Hydroxyphenyl]ethyl]aminomethyl)-2-methoxy-4-methylphenol (MH-16)

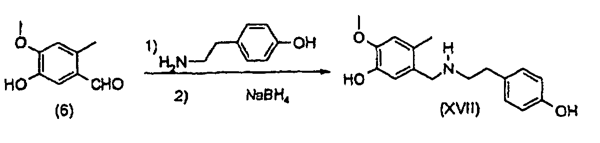

| | |
|---|---|
| 27.8 g (168 mmol) | 2-Methyl-4-methoxy-5-hydroxybenzaldehyd (6) |
| 23.0 g (168 mmol) | Tyramin |
| 5.20 g (134 mmol = 0.8 eq) | Natriumborhydrid |

Ausbeute: 43.4 g ( 161 mmol = 96% d. Th.) beiges Pulver
C₁₇H₂₁NO₃ [287.36]
- DC:: R_{f} = 0.21 (CHCl₃: MeOH = 9:1 + 1% konz. NH₄OH)
- M.p.:: 122-124°C

| | %C | % H | %N |
|---|---|---|---|
| berechnet | 71.06 | 7.37 | 4.87 |
| gefunden | 71.07 | 7.41 | 4.86 |

¹H-NMR (200 MHz, DMSO-d₆): δ 6.90 (m, 2 H), 6.67 (s, 1H), 6.62 (m, 2 H), 6.55 (s, 1H); 3.72 (s, 3 H); 3.51 (s, 2 H); 2.73 (t, J = 6.5 Hz, 2 H); 2.60 (t, J = 6.5 Hz, 2 H); 2.10 (s, 3 H); ¹³C-NMR (50 MHz, DMSOd₆): δ 155.4 (s), 145.8 (s), 143.9 (s), 130.9 (s), 130.4 (s), 129.3 (d), 126.0 (s), 116.2 (d), 115.0 (d), 114.3 (d), 55.7 (q), 51.1 (t), 50.3 (t), 35.0 (t), 17.9 (q)

### Schritt 1b:

### 5-(N-[2-[4-Hydroxyphenyl)ethyl]aminomethyl)-2,4-dimethoxyphenol (MH-34)

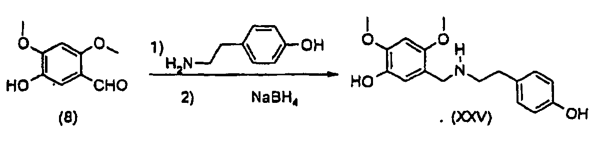

| | |
|---|---|
| 18.85 g (103.47 mmol) | 5-Hydroxy-2,4-dimethoxy-benzaldehyd (8) |
| 14.21 g (103.47 mmol) | Tyramin |
| 3.13 g (82.74 mmol) = 0.8 eq. | NaBH₄ |
| 200 ml | Ethanol 96% |

Ausbeute: 28.1 g = 92.63 mmol = 89.5% d. Th.
C₁₇H₂₁NO₄ [303.36]
- DC:: R₁ = 0.14 (CHCl₃ : MeOH = 9:1 + 1% konz. NH₄OH)
- M.p.:: 170-173°C
C₁₇H₂₁NO₄ [303.36] (verunreinigt mit aliphatischer Substanz ca. C₁₅H₃₂, Schliffett)

| | %C | % H | % N |
|---|---|---|---|
| berechnet | 67.31 | 6.98 | 4.62 |
| gefunden | 68.10 | 7.04 | 4.66 |

¹H-NMR (200 MHz, DMSO-d₆): δ 6.95 (m, 2 H), 6.70 (s; 1H), 6.64 (m, 2 H), 6.57 (s, 1H), 3.75 (s, 3 H), 3.66 (s, 3 H), 3.52 (s, 2 H), 2.59 (bs, 4 H); ¹³C-NMR (50 MHz, DMSO-d₆): δ 155.3 (s), 149.9 (s), 146.3 (s), 139.7 (s), 130.4 (s), 129.3 (d), 120.4 (s), 116.6 (d), 115.0 (d), 98.4 (d), 56.0 (q+q), 50.6 (t), 47.1 (t), 35.0 (t)

### Schritt 2:

### Formylierung: Allgemeine Arbeitsvorschrift

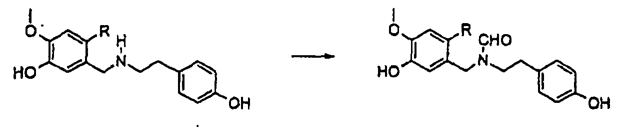

| | |
|---|---|
| 1 eq. | Amin |
| 2 eq. | Ameisensäureester (-ethylester oder -methylester) |
| 10 ml | 1,4-Dioxan, destilliert / 1 g Amin |
| 0.2 ml | Dimethylformamid / 1 g Amin |

katalytische Menge Ameisensäure

Alle Reagentien wurden zusammen auf Rückfluß erhitzt (eventuell mehrmals Ameisensäure zugeben) und das Reaktionsgemisch nach Ende der Reaktion im Vakuum zur Trockene eingeengt. Der feste Rückstand wurde in 10 ml Methanol / 1 g Amin aufgenommen und unter Rühren 50 ml Eis / Wasser / 1 g Amin portionsweise zugegeben, wobei sich die Suspension des Zwischenprodukts unter Hydrolyse zum flockigen Endprodukt umwandelte, welches abgesaugt und getrocknet wurde.

### Schritt 2a:

### N-((5-Hydroxy-4-methoxy-2-phenylmethyl)-N-(2-[4-hydroxyphenyl]ethyl)] formamid (MH-18)

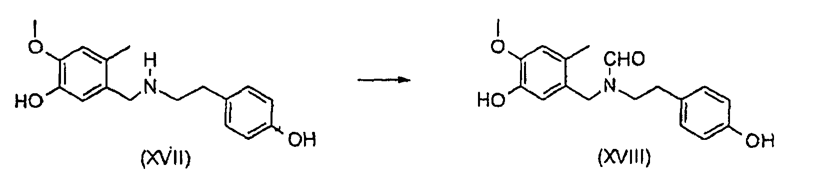

| | |
|---|---|
| 55.0 g (191 mmol) | 5-(N-(2-[4-Hydroxyphenyl]ethyl]aminomethyl)-2-methoxy-4-methylphenol (XVII) |
| 23.5 ml (383 mmol = 2 eq) | Ameisensäuremethylester |
| 11.0 ml | DMF |
| 1.50 ml | Ameisensäure |
| 400 ml | 1,4-Dioxan |

Die Aufarbeitung erfolgt noch 7 h.
Ausbeute: 49.8 g (158 mmol = 82.6% d, Th.) beiges Pulver
C₁₈H₂₁NO₄ [315.37]
- DC:: R_{f} = 0.35 (CHCl₃ : MeOH = 9:1 + 1 % konz. NH₄OH)
- M.p.:: 170-171°C

| | %C | %H | %N |
|---|---|---|---|
| berechnet | 68.55 | 6.71 | 4.44 |
| gefunden | 68.77 | 6.86 | 4.14 |

¹H-NMR (200 MHz, DMSO-d₆): δ 9.20 (s, 1H), 8.74 (d, *J* = 15.3 Hz, 1H), 8.19 (s, 0.5 H), 7.88 (s, 0.5 H), 7.00-6.87 (m, 2 H), 6.74 (s, 1H), 6.72-6.56 (m, 2 H), 6.59 (s, 1H), 4.31 (s, 1H), 4.23 (s, 1H), 3.73 (s, 3 H), 3.21 (dd, J = 15.3, 7.6 Hz, 2 H), 2.60 (t, J = 7.6 Hz, 2 H), 2.12 (s, 3 H); ¹³C-NMR (50 MHz, DMSO-d_{*6*}): 8 162.7 und 162.3 (d), 155.7 (s), 146.7 und 146.5 (s), 144.4 und 144.2 (s), 129.7 und 129.4 (d), 128.9 und 128.4 (s), 126.5 (s), 126.4 und 126.3 (s), 116.3 und 115.9 (d), 115.1 (d), 114.6 und 114.4 (d), 55.6 (q), 48.0 und 47.4 (t), 43.3 und 41.6 (t), 33.2 und 31.9 (t), 18.1 und 18.0 (q)

### Schritt 2b:

### N-(2-(4-Hydroxyphenyl)ethyl)-N-((5-hydroxy-2,4-dimethoxyphenyl)methyl)-formylamid (MH-35)

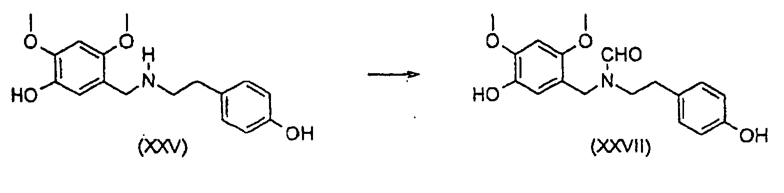

27 g (89 mmol)5-(N-[2-[4-Hydroxyphenyl]ethyl]aminomethyl)-2,4-dimethoxyphenol (XXV)

| | |
|---|---|
| 14.4 ml (178 mmol) | Ameisensäureethylester |
| 200 ml | 1,4-Dioxan |
| 5.5 ml | Dimethylformamid |
| 2 ml | Ameisensäure |

Die Aufarbeitung erfolgte nach 24 h, die Ameisensäure wurde im Abstand von mehreren Stunden in 3 Portionen zugegeben.
Ausbeute: 26.13 g (78.85 mmol = 88.6% d. Th.) beiges Pulver
C₁₈H₂₁NO₅ [331.37]
- DC:: R_{f} = 0.53 (CHCl₃ : MeOH = 9:1 + 1% konz. NH₄OH)
- M.p.:: 130-132°C

| | %C | %H | %N |
|---|---|---|---|
| berechnet | 65.24 | 6.39 | 4.23 |
| gefunden | 64.97 | 6.40 | 4.18 |

¹H-NMR (200 MHz, DMSO-d₆): δ 9.18 (bs, 1H), 8.48 (d, *J* = 7.4 Hz, 1H), 8.16 (s, 0.5 H), 7.86 (s, 0.5 H), 6.98-6.87 (m, 2 H), 6.71-6.58 (m, 4 H), 4.31 (s, 1H), 4.19 (s, 1H), 3.78 (s, 3 H), 3.75 (s, 3 H), 3.21 (dd, J = 16.4, 7.7 Hz, 2 H), 2.69-2.55 (m, 2 H); ¹³C-NMR (50 MHz, DMSO-d₆): δ 162.7 und 162.5 (d), 155.7 und 155.6 (s), 150.6 und 150.3 (s), 146.7 und 147.3 (s), 140.1 und 139.9 (s), 129.6 und 129.3 (d). 129.0 und 128.4 (s), 116.8 und 116.4 (d),116.2 und 115.9 (s), 115.1 (d), 98.5 und 98.4 (d), 56.3 und 56.2 (q), 55.9 (q), 47.9 und 45.2 (t), 43.0 und 38.3 (t), 33.4 und 31.9 (t)

### Schritt 3 :

### Phenolische oxidative Kupplung: 1-Methylgalanthamin (XV)

### [(±)-(4aα,8aR*)]-4a,5,9,10,11,12-Hexahydro-3-methoxy-1-methyl-6-oxo-6H-benzofuro[3a,3,2-ef][2]benzazepin-11-carboxaldehyd, 1-Methyl-N-formylnarwedin (MH-19)

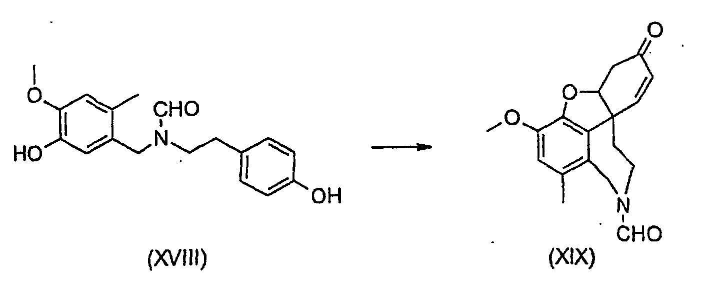

| | |
|---|---|
| 11.4 g (69.8 mmol) | N-((5-Hydroxy-4-methoxy-2-phenylmethyl)-N-(2-[4-hydroxyphenyl]ethyl)]formamid (XVIII) |
| 47.0 g (338 mmol) | Kaliumcarbonat |
| 47.0 g (142 mmol) | Kaliumhexacyanoferrat(III) |
| 1.60 l | Toluol |
| 470 ml | Wasser |
| 40.0 g | Hyflo |

Kaliumcarbonat, Kaliumhexacyanoferrat(III), Wasser und Toluol wurden in einem 4I - Vierhalskolben auf 80°C erwärmt und dann wurde unter starkem mechanischem Rühren das Edukt eingestreut. Die Temperatur wurde 1 h auf 80°C gehalten, dann Hyflo zugegeben und noch 10 Minuten gerührt. Das Reaktionsgemisch wurde abgesaugt und der feste Rückstand 1 x mit Wasser und 3 x mit heißem Toluol nachgewaschen. Die Toluolphase wurde von der wäßrigen Phase getrennt und diese mit Toluol extrahiert. Die organischen Phasen wurden vereinigt, das Lösungsmittel abgezogen und das Produkt im Vakuumtrockenschrank getrocknet.
Ausbeute: 6.17 g (19.7 mmol = 55.0% d. Th.) hellgelbes Pulver
C₁₈H₁₉NO₄ [313.39]
- DC:: R_{f} = 0.48 und 0.42 (2 Rotamere) (CHCl₃ : MeOH = 9:1 + 1 % konz. NH₄OH)
- M.p.:: Zersetzung > 215°C

| | %C | %H | %N |
|---|---|---|---|
| berechnet | 69.00 | 6.11 | 4.47 |
| gefunden | 68.78 | 6.33 | 4.40 |

¹H-NMR (Gemisch aus 2 Rotameren, 200 MHz, DMSO-d₆): δ 8.18 (s, 0.2 H), 8.10 (s, 0.8 H), 7.25 (dd, *J* = 10.4, 1,9 Hz, 0.8 H), 7.15 (dd, *J =* 10.4, 1.9 Hz, 0.2 H), 6.73 (s, 0.2 H), 6.69 (s, 0.8 H), 5.95 (d, *J* = 10.3 Hz, 0.8 H), 5.93 (d, *J* = 10.3 Hz, 0.2 H), 5.14 (d, *J* = 15.4 Hz, 0.8 H), 4.83 (d, *J* = 15.4 Hz, 0.2 H), 4.67 (bs, 1H), 4.51 (d, *J* = 15.4 Hz, 0.2 H), 4.07 (d, *J* = 15.4 Hz, 0.8 H), 3.97 (bs, 1H), 3.78-3.60 (m, 4 H) 3.07 (dd, *J* = 17.4, 3.4 Hz, 1H), 2.78 (dd, *J* = 17.4, 1.9 Hz, 1H), 2.33 (s, 3H), 2.30 (s, 0.8 H), 2.22 (s, 0.2 H), 1.86 (dt, *J* = 13.5, 3.7 Hz, 1H); ¹³C-NMR (Gemisch aus 2 Rotameren, 50 MHz, DMSO-d₆): δ 194.9 (s), 162.8 und 162.1 (d), 145.2 und 144.8 (d), 145.5 und 145.3 (s), 142.9 und 142.8 (s), 130.6 und 130.3 (s), 128.2 (s), 127.5 und 127.0 (s), 126.4 und 126.2 (d), 114.5 und 114.2 (d), 87.0 und 86.8 (d), 55.6 (q), 49.2 und 49.0 (s), 47.4 und 45.6 (t), 41.8 und 40.1 (t), 37.7 (t), 37.5 (t) 37.4 (t), 34.1 (t), 19.2 und 18.9 (q)

### Beispiel 85

### [(±)-(4aα,6β,8aR*)]-4a,5,9,10,11,12-Hexahydro-3-methoxy-1-methyl-6H-benzofuro[3a,3,2-ef][2]benzazepin-6-ol, 1-Methyl-N-demethylgalanthamin (MH-20)

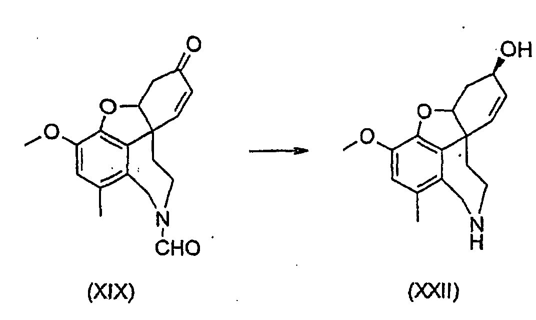

| | |
|---|---|
| 500 mg (1.60 mmol) | 1-Methyl-N-formylnarwedin (XIX) |
| 6.00 ml (6.06 mmol = 3.78 eq) . | L-Selectride 1 M in THF |
| 12.0 ml | abs. THF |

Eine Suspension des fein zerkleinerten Eduktes in abs. THF wurde auf 0°C abgekühlt und dann L-Selectride zugetropft, wobei eine Temperatursteigerung von 5°C zu beobachten war. Bei 0°C wurde 1 h gerührt, wobei sich eine klare Lösung bildete. Nach 70 min wurden 5 Tropfen Wasser und 1 ml konz. aq. Ammoniak zugegeben, 10 min gerührt und dann das Reaktionsgemisch im Vakuum auf die Hälfte eingeengt. Anschließend wurden noch einmal 10 ml Ammoniak zugegeben und die Lösung mit Methylenchlorid extrahiert. Die vereinigten organisch en Phasen wurden einmal mit verdünnter Ammoniaklösung nachgewaschen, über Natriumsulfat getrocknet, filtriert und das Lösungsmittel abgezogen. Das entstandene leuchtend rote Öl wurde über eine Kieselgelsäule gereinigt.
Ausbeute: 440 mg (1.53 mmol = 96.0% d. Th.) farbloses Öl
C₁₇H₂₁NO₃ [287.36]
- DC:: R_{f} = 0.39 (CHCl₃: MeOH = 9:1 + 1% NH₄OH)
C₁₇H₂₁NO₃x 0.8 H₂O [301.76]

| | %C | %H | %N |
|---|---|---|---|
| berechnet | 67.66 | 7.55 | 4.64 |
| gefunden | 67.60 | 7.40 | 4.65 |

¹H-NMR (200 MHz, CDCl₃): δ 6.51 (s, 1H), 6.06 (d, *J* = 10.2 Hz, 1H) , 5.97 (dd, *J* = 10.2, 4.5 Hz, 1H), 4.57 (bs, 1H), 4.27 (d, *J* = 16.0 Hz, 1H), 4.11 (t, *J* = 4.4 Hz, 1H), 3.80 (s, 3 H), 3.77 (d, *J* = 16.0 Hz, 1 H), 3.40-3.10 (m, 2 H), 2.65 [dd, J = 15.6, 3.2 Hz, 1H), 2.23 (s, 3 H); 1.99 (ddd, *J* = 15.6, 4.9, 2.3 Hz, 1 H), 1.89-1.63 (m, 2 H); ¹³C-NMR (50 MHz, CDCl₃): δ 144.4 (s), 142.9 (s), 133.4 (s), 130.5 (s), 127.8 (s), 127.5 (d), 127.1 (d), 113.5 (d), 88.1 (d), 61.4 (d), 55.8 (q), 49.0 (s), 48.9 (t), 46.9 (t), 39.7 (t), 29.8 (t), 19.4 (q)

### Beispiel 86

### [(±)-(4aα,8aR*)]-4a,5,9,10,11,12-Hexahydro-3-methoxy-1,4'-dimethyl-spiro[-6H-benzofuro[3a,3,2-ef][2]benzazepin]-6,2'-[1,3]-dioxolan]-11-carboxaldehyd, 1-Methyl-N-formyl-narwedinketal (MH-21)

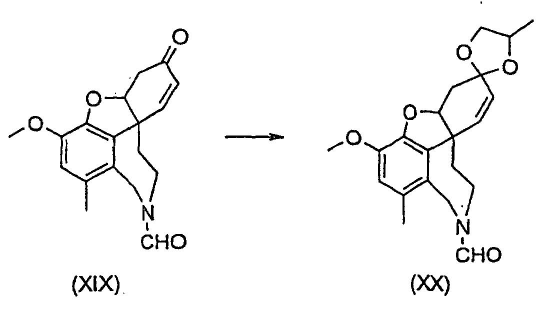

| | |
|---|---|
| 6.17 g (19.7 mmol) | 1-Methyl-N-formylnarwedin (XIX) |
| 40.0 ml | Toluol |
| 375 mg (1.97 mmol) | p-Toluolsulfonsäure |
| 11.1 ml (149 mmol) | Propylenglykol |

In einem Kolben mit Wasserabscheider wurde das Edukt in Toluol vorgelegt und 1/3 der p-Toluolsulfonsäure mit 2/3 des Propylenglykols zugegeben. Das Reaktionsgemisch wurde auf Rückfluß erhitzt und nach 2 h die Lösung der restlichen p-Toluolsulfonsäure in Propylenglykol portionsweise über 5 h zugegeben, dann weitere 6 h auf Rückfluß gekocht. Nach Ende der Reaktion (HPLC-Untersuchung notwendig, da DC wenig aussagekräftig) wurde die Toluolphase abgetrennt und die Propylenglykolphase mit Toluol erschöpfend extrahiert. Die gesammelten Toluolphasen wurden 2 x mit Essigsäure (8% in Wasser), 2 x mit gesättigter Natriumbicarbonatlösung und 2 x mit Wasser extrahiert, dann das Lösungsmittel abgezogen.
Ausbeute: 5.34 g (14.38 mmol = 73% d. Th.) beiger Schaum
C₂₁H₂₅NO₅ [371.44]
- DC:: R_{f} = 0.71 (CHCl₃ : MeOH = 9:1)
C₂₁H₂₅NO₅ x 0.85 H₂O [386.74]

| | %C | %H | %N |
|---|---|---|---|
| berechnet | 65.22 | 6.96 | 3.62 |
| gefunden | 65.39 | 7.19 | 3.52 |

¹H-NMR (Gemisch aus Diastereomeren und Rotameren, 200 MHz, CDCl₃): δ 8.14-8.01 (m, 1H), 7.30-7.09 (m, 2 H), 6.51 (s, 1H), 6.22-5.97 (m, 1H), 5.85-5.61 (m, 1H), 5.38 und 4.77 (d, J = 15.7 Hz, 1 H), 4.49 (bs, 1H), 4.37-4.01 (m, 2 H), 3.93-3.74 (m, 5 H), 3.71-3.10 (m, 1H), 2.79-2.58 (m, 1H), 2.41 (s, 2 H), 2.32 (d, J = 10.2 Hz, 3 H), 2.25-1.74 (m, 3 H); ¹³C-NMR (Gemisch aus Diastereomeren und Rotameren, 50 MHz, CDCl₃): δ 162.5 (d), 161.7 (d), 143.7 (s), 143.6 (s), 143.3 (d), 142.7 (d), 129.9 (s), 129.6 (s), 127.8 (d), 127.6 (d), 126.0 (s), 125.7 (s), 114.6 (d), 114.4 (d), 87.5 (d), 87.4 (d), 68.2 (d), 68.0 (t), 56.1 (q), 56.0 (q), 49.2 (s), 49.0 (s), 48.7 (t), 46.7 (t), 43.2 (t), 41.2 (t), 38.7 (t), 37.2 (t), 37.1 (t), 34.8 (t), 19.7 (q), 19.4 (q), 18.9 (q)

### Beispiel 87

### [(±)-(4aα,8aR*)]-4a,5,9,10,11,12-Hexahydro-3-methoxy-1,11-dimethyl-6H-benzofuro[3a,3,2-ef][2]benzaxepin-6-on, 1-Methylnarwedin (MH-22)

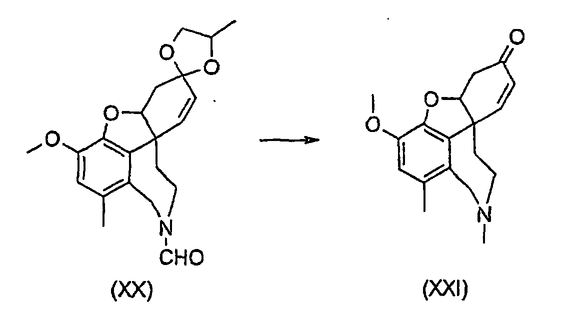

| | |
|---|---|
| 5.34 g (14.4 mmol) | 1-Methyl-N-formylnarwedinketal (XX) |
| 25.2 ml (25.2 mmol) = 1.75 eq. | Lithiumaluminiumhydrid 1M in THF |
| 20 ml | abs. THF |

1-Methyl-N-formylnarwedin (V) wurde in abs. THF gelöst und unter Rühren Lithiumaluminiumhydrid protionsweise zugegeben. Nach 15 min wurde das Reaktionsgemisch mit 10 ml Toluol versetzt, dann mit 1.5 ml Wasser hydrolysiert und nach Zugabe von 1.5 ml Natronlauge (15% in Wasser) 15 min gerührt. Nach Zugabe von 1.5 g Hyflo wurde 1 h auf Rückfluß erhitzt, abfiltriert, der Filterkuchen 3 x mit je 10 ml Toluol : THF = 1:1 aufgekocht und jeweils abgesaugt. Die organischen Phasen wurden zur Trockene eingeengt, mit 25 ml 4N HCl aufgenommen und 25 min bei 60°C gerührt, dann mit Ethylacetat erschöpfend extrahiert. Die gesammelten organischen Phasen wurden mit verdünnter HCl rückgewaschen. Die gesammelten sauren, wäßrigen Phasen wurden durch Destillation von überschüssigem Ethylacetat befreit, dann mit konz. aq. Ammoniak basisch gestellt und mit Chloroform erschöpfend extrahiert. Die gesammelten Chloroformphasen wurden mit gesättigter Kochsalzlösung gewaschen, über Natriumsulfat getrocknet, filtriert und das Lösungsmittel abgezogen. Die Reinigung erfolgte durch Umkristallisieren aus Diisopropylether: Ethylacetat = 9:1.
Ausbeute: 4.01 g (13.36 mmol = 93% d. Th.) hellgelbe, sehr feine Nadeln
C₁₈H₂₁NO₃ [299.37]
- DC:: R_{f} = 0.43 (CHCl₃ : MeOH = 95:5)
- M.p.:: 121-122°C

| | %C | %H | %N |
|---|---|---|---|
| berechnet | 72.22 | 7.07 | 4.68 |
| gefunden | 71.95 | 7.08 | 4.57 |

¹H-NMR (200 MHz, CDCl₃): δ 7.01 (dd, *J* = 10.4, 1.6 Hz, 1H), 6.56 (s, 1H), 5.99 (d, *J* = 10.4 Hz, 1H), 4.68-4.62 (m, 1H), 3.97 (d, *J* = 15.7 Hz, 1H), 3.80 (s, 3 H), 3.79 (d, *J* = 15.7 Hz, 1H), 3.22-2.95 (m, 3 H), 2.71 (dd, *J* = 17.8, 3.7 Hz, 1H), 2.44 (s, 3 H), 2.23 (s, 3 H), 2.20-2.01 (m, 1H), 1.87 (dt, *J* = 13.8, 3.4 Hz, 1H); ¹³C-NMR (50 MHz, CDCl₃): δ 194.4 (s), 145.2 (s), 142.9 (s), 131.0 (s), 128.9 (s), 126.8 (d + d + s), 114.3 (d), 87.7 (d), 55.9 (q), 55.8 (t), 54.1 (t), 48.9 (s), 43.5 (q), 37.1 (t), 33.4 (t), 19.4 (q)

### Beispiel 88

### [(±)-(4aα,6β,8aR*)]-4a,5,9,10,11,12-Hexahydro-3-methoxy-1,11-dimethyl-6H-benzofuro[3a,3,2-ef][2]benzazepin-6-ol, 1-Methylgalanthamin (MH-30)

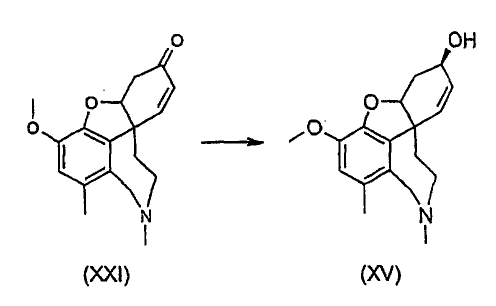

| | |
|---|---|
| 170 mg (0.57 mmol) | 1-Methyl-Narwedin (XXI) |
| 0.70 ml (0.68 mmol) = 1.2 eq. | L-Selectride® 1M in THF |
| 5 ml | absolutes THF |

Das Edukt wurde unter N₂-Atmosphäre in THF vorgelegt und auf -25°C abgekühlt, dann wurde langsam L-Selectride zugetropft. Es wurde 30 min bei -15°C gerührt, wobei die anfängliche Suspension zu einer klaren Lösung wurde. Anschließend wurde das Reaktionsgemisch über 1 h auf Raumtemperatur gebracht, mit 5 Tropfen Wasser hydrolysiert, 30 min gerührt, 0.5 ml konz. aq. NH₄OH zugegeben, weitere 10 min gerührt, wieder 2 ml konz. NH₄OH zugegeben und schließlich mit Methylenchlorid extrahiert. Die vereinigten organischen Phasen wurden mit gesättigter Kochsalzlösung gewaschen, über Natriumsulfat getrocknet, filtriert und das Lösungsmittel abgezogen, wobei 350 mg Rohprodukt entstanden. Dieses Rohprodukt wurde über eine Kieselgelsäule (CHCl₃ : MeOH = 9:1) gereinigt, wobei ein gelbes Öl entstand, welches durch Zugabe von Ether erstarrte.
Ausbeute: 120 mg (0.398 mmol = 70% d. Th.) farbloses Pulver
C₁₈H₂₃NO₃ [301.39]
- DC:: R_{f} = 0.43 (CHCl₃ : MeOH = 95:5), nicht von Edukt zu trennen
¹H-NMR (200 MHz, CDCl₃): δ 6.54 (s, 1H), 6.10 (dd, *J* = 10.2, 1.2 Hz, 1H), 5.98 (dd, *J* = 10.2, 4.7 Hz, 1 H), 4.56 (bs, 1H), 4.12 (bs, 1H), 3.99 (d, *J* = 15.6 Hz, 1H), 3.82 (s, 3 H), 3.81 (d, J = 15.6 Hz, 1H), 3.20 (ddd, *J* = 14.2, 12.1, 2.1 Hz, 1H), 2.96 (dt, *J* = 14.2, 3.4 Hz, 1H), 2.65 (ddd, *J* = 15.7, 3.2, 1.5 Hz, 1H), 2.41 (s, 3 H), 2.24 (s, 3 H), 1.99 (ddd, *J* = 15.5, 5.0, 2.5 Hz, 2 H), 1.60 (ddd, *J* = 13.7, 4.0, 2.4,Hz, 1H); ¹³C-NMR (50 MHz, CDCl₃): δ 144.0 (s), 143.0 (s), 133.4 (s), 128.9 (s), 127.4 (d + d), 127.0 (s), 126.6 (s), 113.6 (d), 88.3 (d), 61.9 (d), 55.7 (q), 55.4 (t), 53.8 (t), 48.2 (s), 42.7 (q), 33.8 (t), 29.8 (t), 19.4 (q) **Herstellung des Hydrobromids:**

Die Reaktionslösung wird mit Ethanol (ca. halbes Reaktionsvolumen) hydrolysiert , 30 min gerührt, dann mit konz. HBr auf pH ≤ 1 gebracht und über Nacht gerührt. Der entstandene Niederschlag wird abgesaugt, mit Ethanol gewaschen und getrocknet.
- M.p.:: 246-250°C (Hydrobromid)
C₁₈H₂₄NO₃Br x 0.5 H₂O [391.30]

| | %C | %H | %N |
|---|---|---|---|
| berechnet | 55.25 | 6.44 | 3.58 |
| gefunden | 55.21 | 6.39 | 3.56 |

### Beispiel 89

### [(±)-(4aα,6α,8aR*)]-4a,5,9,10,11,12-Hexahydro-3-methoxy-1,11-dimethyl-6H-benzofuro[3a,3,2-ef][2]benzazepin-6-ol, 1-Methylepigalanthamin (MH-31)

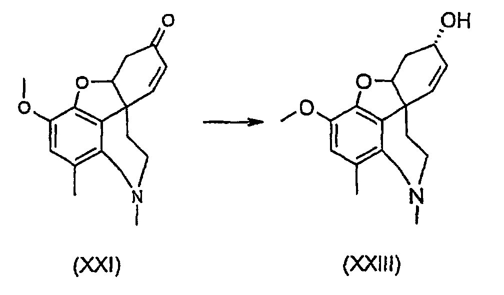

| | |
|---|---|
| 2.00 g (6.68 mmol) | 1-Methylnarwedin (XXI) |
| 150 ml | Methanol |
| 2.50 g (6.68 mmol) | Certrichlorid Heptahydrat |
| 0.50 g (13.4 mmol) = 2 eq. | NaBH₄ |

Das Edukt wurde in der Hitze in Methanol gelöst und dann auf 0°C abgekühlt, CeCl₃ × 7H₂O zugegeben und etwa 30-60 min bei 0°C gerührt. Anschließend wurde portionsweise NaBH₄ zugeben, weitere 2 h bei 0-5°C gerührt, wobei sich ein Niederschlag bildete. Das Reaktionsgemisch wurde mit 5 ml 2N HCl hydrolysiert, Methanol im Vakuum abdestilliert, der Rückstand mit weiteren 150 ml 2N HCl aufgenommen, mit konz. NH₄OH basisch gemacht (violetter Niederschlag), mit Ethylacetat extrahiert, die gesammelten organischen Phasen mit gesättigter Kochsalzlösung gewaschen, über Natriumsulfat getrocknet, filtriert und das Lösungsmittel abgezogen. Als Rohprodukt entstand ein Isomerengemisch von 1-Methylepigalanthamin und 1-Methylgalanthamin im Verhältnis von ca. 5:1, welches chromatographisch getrennt werden konnte [Kieselgel, CHCl₃ : MeOH = 9:1 + 0.5% NH₄OH).
Ausbeute: 1.34 g (4.45 mmol = 66.6% d. Th.) farbloses Öl
C₁₈H₂₃NO₃ [301.39]
- DC:: R_{f} = 0.20 (CHCl₃ : MeOH = 9:1)
¹H-NMR (200 MHz, CDCl₃): δ 6.51 (s, 1H), 6.10 (d, J = 10.2 Hz, 1H), 5.79 (d, *J* = 10.2 Hz, 1H), 4.69-4.56 (m, 1H), 4.55 (bs, 1H), 3.96 (d, *J* = 15.3 Hz, 1H), 3.82 (s, 3 H), 3.79 (d, *J* = 15.3 Hz, 1H), 3.21 (td, *J* = 13.1, 1.7 Hz, 1H), 2.97 (dt, *J* = 14.1, 3.3 Hz, 1H), 2.75 (dddd, *J* = 14.1, 5.3, 4.0, 1.2 Hz, 1H), 2.38 (s, 3 H), 2.23 (s, 3 H), 2.10 (dd, *J* = 13.1, 3.2 Hz, 1H), 2.03 (bs, 1H), 1.69 (ddd, *J* = 13.6, 10.7, 2.6 Hz, 2 H); ¹³C-NMR (50 MHz, CDCl₃): δ 145.0 (s), 142.9 (s); 133.5 (s), 131.7 (d), 128.5 (s), 126.7 (d), 126.6 (s), 113.5 (d), 88.3 (d), 63.1 (d), 55.8 (q), 55,2 (t), 54.1 (t), 48.3 (s), 42.6 (q), 34.6 (t), 32.4 (t), 19.5 (q)

### Herstellung des Hydrobromids:

Das gewonnene 1-Methylepigalanthamin wurde in Ethanol aufgenommen und mit konz. HBr auf pH □ 1 gebracht. Das Hydrobromid wurde in der Kälte zur Kristallisation gebracht und der entstandene Niederschlag abgesaugt, mit kaltem Ethanol gewaschen und getrocknet.
M.p.: 254-255°C (Hydrobromid)
C₁₈H₂₄NO₃Br x 0.5 H₂O [391.30]

| | %C | %H | %N |
|---|---|---|---|
| berechnet | 56.25 | 6.44 | 3.58 |
| gefunden | 56.28 | 6.21 | 3.57 |

### Beispiel 90

### [(±)-(4aα,6α,8aR*)]-4a,5,9,10,11,12-Hexahydro-3-methoxy-1-methyl-6H-benzofuro[3a,3,2-ef][2] benzazepin-6-ol, 1-Methyl-N-demethylepigalanthamin

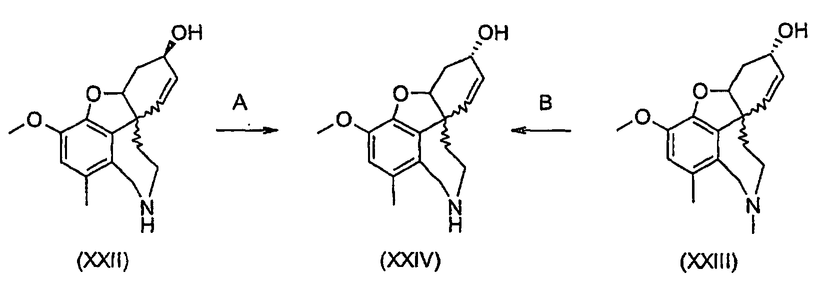

### Methode A (Epimerisierung von 1-Methyl-N-demethylgalanthamin (XXII))

### Schritt 1 (Acetylierung): [(±)-(4aα,6α,8aR*)]-6-O-Acetyloxy-4a,5,9,10,11,12-hexahydro-3-methoxy-1-methyl-6H-benzofuro[3a,3,2-ef][2]benzazepin (MH-67)

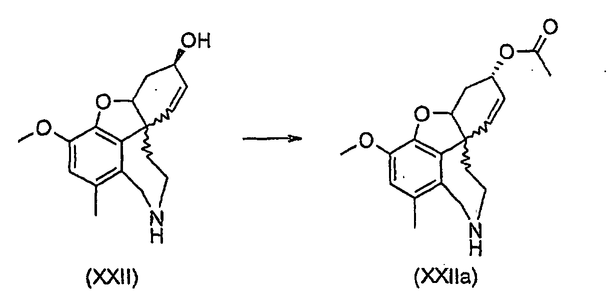

| | |
|---|---|
| 100 mg (0.35 mmol) | 1-Methyl-N-demethylgalanthamin (XXII) |
| 0.50 ml (1.74 mmol) = 5 eq. | N,N-Dimethylformamid-bis(2,2-dimethylpropyl)-acetal |
| 0.10 ml (1.74 mmol) = 5 eq. | Eisessig |
| 12 ml | Toluol |

Das Edukt wurde in 10 ml Toluol unter N₂-Atmosphäre auf 80°C erhitzt und über 1 h ein Gemisch aus N,N-Dimethylformamid-bis-(2,2-dimethylpropyl)acetal und Eisessig in 2 ml Toluol zugetropft. Nach 22 h auf 80°C wurde die erkaltete Toluolphase 1 x mit Wasser dann mit 2 N Salzsäure extrahiert, die sauren, wäßrigen Phasen mit konzentrierter Ammoniaklösung basisch gemacht, mit Ethylacetat extrahiert, die organischen Phasen mit gesättigter Kochsalzlösung gewaschen, über Natriumsulfat getrocknet, filtriert und das Lösungsmittel abgezogen. Der Rückstand wurde säulenchromatographisch gereinigt
(CHCl₃ : MeOH = 95:5).
Ausbeute: 45 mg (0.14 mmol = 39% d. Th.) farbloses Öl
C₁₉H₂₃NO₄ [329.40]
- DC:: R_{f} = 0.20 (CHCl₃: MeOH = 95:5)
¹H-NMR (200 MHz, CDCl₃): δ 6.50 (s, 1H), 6.14 (d, J = 10.2 Hz, 1H), 5.72 (d, J = 10.2 Hz, 1H), 5.67-5.58 (m, 1H), 4.57 (bs, 1H), 4.24 (d, J = 16.0 Hz, 1H), 3.83 (s, 3 H), 3.75 (d, J = 16.0 Hz, 1H), 3.40-3.09 (m, 2 H), 2.90-2.70 (m, 1H), 2.23 (s, 3 H), 2.07 (s, 3 H), 2.01-1.73 (m, 3 H); ¹³C-NMR (50 MHz, CDCl₃): δ 170.2 (s), 145.3 (s), 142.8 (s), 133.0 (s), 130.5 (s), 128.4 (d), 127.2 (d), 127.0 (s), 113.5 (d), 87.3 (d), 66.4 (d), 55.8 (q), 48.8 (s + t), 47.1 (t), 40.4 (t), 28.2 (t), 21.1 (q), 19.4 (q)

### Schritt 2: Esterhydrolyse (MH-78)

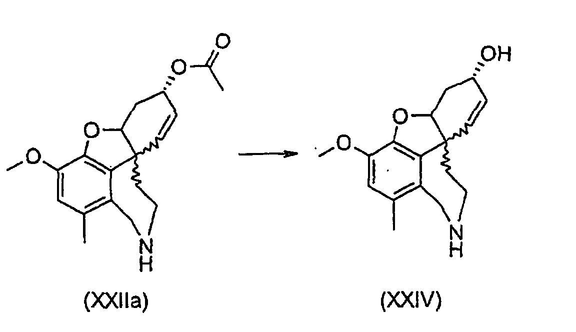

| | |
|---|---|
| 17 mg (0.05 mmol) | 6-O-Acetyl-1-methyl-N-demethylepigalanthamin (XXIIa) |
| 0.5 ml | Methanol |
| 0.1 ml | 2N KOH |
| 17 mg (0.12 mmol) = 2.4 eq. | Kaliumcarbonat |

Die Reagenzien wurden zusammen bei Raumtemperatur gerührt. Nach Ende der.Reaktion wurde mit 1 ml Wasser versetzt, Methanol abgezogen, mit 4 ml 2 N Salzsäure angesäuert, die wäßrige Phase mit Ethylacetat gewaschen, dann mit konzentrierter wässriger Ammoniaklösung basisch gemacht und mit Ethylacetat extrahiert. Die organischen Phasen wurden mit gesättigter Kochsalzlösung gewaschen, über Natriumsulfat getrocknet, filtriert und das Lösungsmittel abgezogen.
C₁₇H₂₁NO₃ [287.36]
- DC:: R_{f} = 0.07 (CHCl₃ : MeOH = 9:1)

### Methode B (Demethylierung von 1-Methylepigalanthamin) (MH-94)

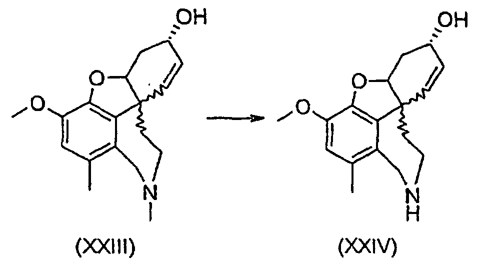

| | |
|---|---|
| 0.80 g (2.65 mmol) | 1-Methylepigalanthamin (XXIII) |
| 1.50 g (6.63 mmol) = 2.5 eq. | Azodicarbonsäure-bis[2-methyl-2-propylester) |
| 80 ml | Tetrahydrofuran |

Die Reagenzien wurden zusammen bei Raumtemperatur 24 h gerührt, dann das Lösungsmittel abgezogen. Der Rückstand wurde in Trifluoressigsäure in Methylenchlorid aufgenommen, 30 min gerührt, im Eisbad mit konzentrierter wässriger Ammoniaklösung basisch gemacht und mit Methylenchlorid extrahiert. Die organischen Phasen wurden mit gesättigter Kochsalzlösung gewaschen, über Natriumsulfat getrocknet, filtriert und das Lösungsmittel abgezogen. Das Rohprodukt wurde säulenchromatographisch gereinigt (CHCl₃: MeOH = 9:1).
Ausbeute: 400 mg (1.39 mmol = 53% d. Th.) farbloses Öl
C₁₇H₂₁NO₃ [287.36]
DC:R_{f} = 0.10 [CHCl₃:MeOH = 9:1]
¹H-NMR (200 MHz, CDCl₃): δ 6.50 (s, 1H), 6.08 (d, *J* = 10.3 Hz, 1H), 5.80 (d, *J* = 10.3 Hz, 1H), 4.70-4.62 (m, 1H), 4.57 (bs, 1H), 4.26 (d, *J* = 15.7 Hz, 1H), 3.83 (s, 3 H), 3.75 (d, *J* = 15.7 Hz, 1H), 3.35-3.20 (m, 1H), 2.85-2.70 (m, 1H), 2.50-2.29 (m, 2 H), 2.23 (s, 3 H), 2.00-1.64 (m, 2 H); ¹³C-NMR (50 MHz, CDCl₃): δ 145.4 (s), 142.9 (s), 133.5 (s), 131.6 (d), 130.7 (s), 127.2 (d), 126.8 (s), 113.3 (d), 88.2 (d), 63.1 (d), 55.9 (q), 48.9 (t), 48.8 (s), 47.2 (t), 32.2 (t), 28.2 (t), 19.5 (q)

### Allgemeine Arbeitsvorschrift für quartäre 1-Methyl- und 1-Methylepi-galanthaminderivate

### (Beisplele 91-100)

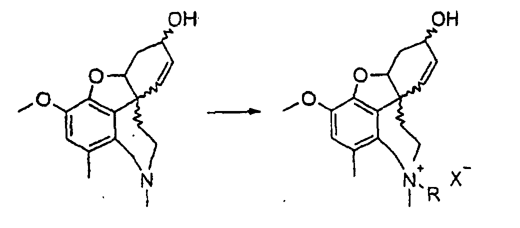

Das Edukt wurde in sehr wenig DMF gelöst*, das Alkylhalogenid zugegeben und das Reaktionsgemisch erhitzt (generell nicht Ober die Siedetemperatur des Alkylhalogenids, maximal aber 70°C). Mittels DC wurde das Ende der Reaktion bestimmt, dann das Reaktionsgemisch langsam unter Rühren auf Ether getropft (ölt in manchen Fällen leicht aus), der Niederschlag abgesaugt und mit Ether gewaschen. Zur Reinigung und zum Entfemen von restlichem DMF wurde der Niederschlag in Ethanol gelöst und abermals in Ethylacetat ausgefällt, dann im Vakuumtrockenschrank bei 50°C getrocknet.
DC: CHCb : MeOH = 9:1, R_{f} generell knapp über dem Start.
* Bei R = CH₂Cl dient Methylenchlorid (p.A. 99.5%) als Lösungsmittel und Reagens.

### Beispiel 91:

### [(±)-(4aα,6β,8aR*)]-4a,5,9,10,11,12-Hexahydro-6-hydroxy-3-methoxy-1,11-dimethyl-11-(2-methyl-2-propenyl)-6H-benzoturo[3a,3,2-ef][2]benzazeplnium. Chlorid (MH-33)

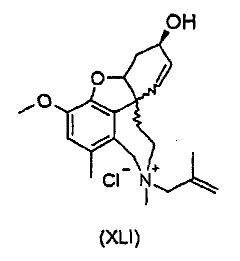

| | |
|---|---|
| 280 mg (0.94 mmol) | 1-Methylgalanthamin (XV) |
| 0.30 ml (3.08 mmol) = 3 eq. | 1-Chlor-2-methylprop-2-en |
| 5.00 ml | Dimethylformamid |

Die Reaktion wurde bei 70°C durchgeführt und noch 2 h aufgearbeitet, indem das Reaktionsgemisch auf 25 ml Diethylether getropft wurde.
Ausbeute: 270 mg (0.69 mmol = 73% d. Th.) farbloses Pulver
C₂₂H₃₀ClNO₃ [391.94]
- DC:: R_{f} = 0.10 (CNCl₃ : MeOH = 9:1)
- M.p.:: 239-241°C
C₂₂H₃₀ClNO₃ x 1.4 H₂O [417.14]

| | %C | %H | %N |
|---|---|---|---|
| berechnet | 63.34 | 7.92 | 3.36 |
| gefunden | 63.22 | 7.85 | 3.59 |

¹³C-NMR (50 MHz, DMSO-d₆): δ 144.6 (s), 134.2 (s), 133.5 (s), 131.1 (s), 130.9 (d), 127.5 (t), 124.9 (d), 115.9 (s), 114.4 (d), 86.3 (d), 73.0 (t), 60.8 (t), 59.4 (d), 55.5 (q), 46.3 (s), 43.0 (q), 31.1 (t), 23.8 (q), 18.9 (q)

### Beispiel 92:

### [(±)-(4aα,6β,8aR*)]-4a,5,9,10,11,12-Hexahydro-6-hydroxy-3-methoxy-1,11-dimethyl-11-(2-propinyl)-6H-benzofuro[3a,3,2-ef][2]benzozepinium, Bromid (MH-38)

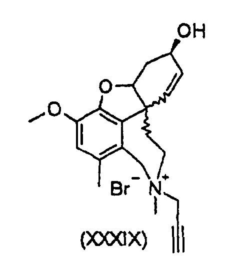

| | |
|---|---|
| 349 mg (1.16 mmol) | 1-Methylgalanthamin (XV) |
| 0.13 ml (1.16 mmol) | 3-Brom-1-propin (Propargylbromid) |
| 4.00 ml | Dimethylformamid |

Die Reaktion wurde bei 60°C durchgeführt und nach 19 h aufgearbeitet, indem das Reaktionsgemisch auf 80 ml Diethylether getropft wurde.
Ausbeute: 300 mg (0.71 mmol = 62% d. Th.) beiges Pulver
C₂₁H₂₆BrNO₃ [420.35]
- DC:: R_{f} = 0.09 (CHCl₃ : MeOH = 9:1)
- M.p.:: 216-218°C
C₂₁H₂₆BrNO₃ x 0.35 H₂O x 0.25 C₃H₇NO [444.93]

| | % C | % H | % N |
|---|---|---|---|
| berechnet | 58.72 | 6.44 | 3.90 |
| gefunden | 58.70 | 6.37 | 3.94 |

¹³C-NMR (50 MHz, DMSO-d₆): δ 144.8 (s), 144.7 (s), 133.4 (s), 131.0 (s), 125.2 (d), 115.3 (s), 114.5 (d), 86.2 (d), 83.7 (d), 72.6 (t), 60.6 (t), 59.7 (d), 55.6 (q), 46.2 (s), 43.0 (q), 31.0 (t), 18.8 (q)

### Beispiel 93:

### [(±)-(4aα,6β,8aR*)]-4a,5,9,10,11,12-Hexahydro-6-hydroxy-3-methoxy-1,11-dimethyl-11-phenylmethyl-6H-benzofuro[3a,3,2-ef][2]benzazepinium, Bromid (MH-39)

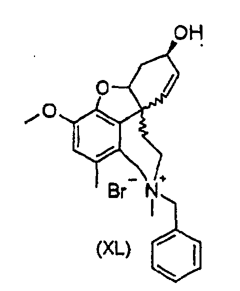

| | |
|---|---|
| 242 mg (0.80 mmol) | 1-Methylgalanthamin (XV) |
| 0.25 ml (1.01 mmol) = 1.4 eq. | Benzylbromid |
| 4.00 ml | Dimethylformamid |

Die Reaktion wurde bei 60°C durchgeführt und nach 10 min aufgearbeitet.
Ausbeute: 262 mg (0.55 mmol = 69% d. Th.) beiges Pulver
C₂₅H₃₀BrNO₃ [472.42]
- DC:: R_{f} = 0.08 (CHCl₃ : MeOH = 9:1)
- M.p.:: 246-248°C

| | % C | % H | %N |
|---|---|---|---|
| berechnet | 63.56 | 6.40 | 2.96 |
| gefunden | 63.35 | 6.34 | 2.93 |

¹³C-NMR (50 MHz, DMSO-d₆): 5 144.7 (s), 133.4 (d), 130.7 (s), 130.4 (d), 129.0 (d), 128.1 (s), 114.5 (d), 86.3 (d), 59.7 (t), 59.5 (d), 55.6 (q), 46.2 (s), 18.6 (q)

### Beispiel 94 :

### [(±)-(4aα,6β,8aR*)]-4a,5,9,10,11,12-Hexahydro-4-hydroxy-3-methoxy-1,11,11-trimethyl-6Hbenzofuro[3a,3,2-ef][2]benzazepinium, Iodid (MH-83)

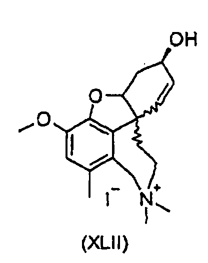

| | |
|---|---|
| 140 mg (0.46 mmol) | 1-Methylgalanthamin (XV) |
| 198 mg (1.39 mmol) = 3 eq. | Methyliodid |
| 4.00 ml | Dimethylformamid |

Die Reaktion wurde bei 40°C durchgeführt und nach 1.5 h aufgearbeitet, Indem das Reaktionsgemisch auf 30 ml Diethylether getropft wurde.
Ausbeute: 146 mg (0.54 mmol = 71% d. Th.) hellbraunes Pulver
C₁₉H₂₆lNO₃[443.32]
- DC:: Rr = 0.05 (CHCl₃ : MeOH = 9:1)
- M.p.:: 278-280°C
C₁₉H₂₆INO₃ x 0.3 H₂O [448.72]

| | %C | %H | %N |
|---|---|---|---|
| berechnet | 50.86 | 5.97 | 3.12 |
| gefunden | 50.57 | 5.85 | 3.43 |

¹³C-NMR (50 MHz, DMSO-d₆): δ 144.6 (s), 144.1 (s), 132.8 (s), 131.6 (s), 114.2 (d), 86.3 (d), 62.6 (t), 59.5 (d), 55.4 (q), 45.9 (s), 31.0 (t), 18.4 (q)

### Beispiel 95:

### [(±)-(4aα,6α,8aR*)]-4a,5,9,10,11,12-Hexahydro-6-hydroxy-3-methoxy-1,11-dimethyl-11-[2-methyl-2-propenyl)-6H-benzofuro[3a,3,2-ef][2]benzazepinlum, Chlorid (MH-66)

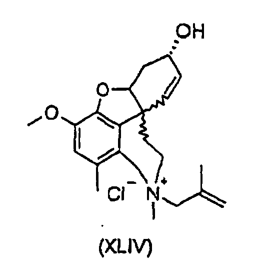

| | |
|---|---|
| 150 mg (0.50 mmol) | 1-Methylepigalanthamin (XXIII) |
| 45.0 mg (1.50 mmol) = 3 eq. | 1-Chlor-2-methylprop-2-en |
| 4.00 ml | Dimethylformamid |

Die Reaktion wurde bei 70°C durchgeführt und nach 100 min aufgearbeitet, indem das Reaktionsgemisch auf 50 ml Diethylether getropft wurde.
Ausbeute: 160 mg (0.41 mmol = 82% d. Th.) farbloses Pulver
C₂₂H₃₀ClNO₃ [391.94]
- DC:: R_{f} = 0.09 (CHCl₃ : MeOH = 9:1)
- M.p.:: 162-164°C
C₂₂H₃₀ClNO₃ x 0.7 H₂O x 0. 15 C₃H₇NO [415.51]

| | % C | %H | % N |
|---|---|---|---|
| berechnet | 64.90 | 7.87 | 3.88 |
| gefunden | 64.77 | 7.68 | 3.95 |

¹³C-NMR (50 MHz, DMSO-d₆): δ 144.7 (s), 134.2 (s), 134.1 (s), 131.1 (d), 127.5 (t), 114.4 (d), 87.3 (d), 73.0 (t), 60.7 (d), 59.4 (t), 55.6 (q), 46.3 (s), 23.8 (q), 18.9 (q)

### Beispiel 96:

### [(±)-(4aα,6α,8aR*)]-4a,5,9,10,11,12-Hexahydro-6-hydroxy-3-methoxy-1,11-dimethyl-11-(2-propinyl)-6H-benzofuro[3a,3,2-ef][2]benzazepinium, Bromid (MH-71)

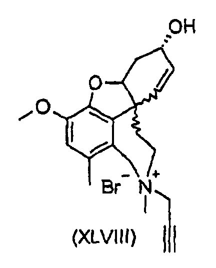

| | |
|---|---|
| 150 mg (0.50 mmol) | 1-Methylepigalanthamin (XXIII) |
| 180 mg (1.50 mmol) = 3 eq. | 3-Brom-1-propin (Propargylbromid) |
| 4.00 ml | Dimethylformamid |

Die Reaktion wurde bei 70°C durchgeführt und nach 2.5 h aufgearbeitet, indem das Reaktionsgemisch auf 30 ml Diethylether getropft wurde.
Ausbeute: 167 mg (0.40 mmol = 82% d. Th.) hellbraunes Pulver
C₂₁H₂₆BrNO₃ [420.35]
- DC:: R_{f} = 0.09 [CHCl₃: MeOH = 9:1)
- M.p.:: 158-162°C
¹³C-NMR (50 MHz, DMSO-d₆): δ 144.8 (s), 133.9 (s), 131.2 (s), 114.5 (d), 87.2 (d), 83.7 (d), 72.6 (d), 55.6 (q), 46.3 (s), 31.9 (t), 18.8 (q)

### Beispiel 97:

### [(±)-(4aα,6α,8aR*)]-4a,5,9,10,11,12-Hexahydro-6-hydroxy-3-methoxy-1,11-dimethyl-11-(2-propenyl)-6H-benzofuro[3a,3,2-ef][2]benzazeplnium, Bromid (MH-72)

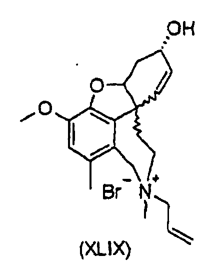

| | |
|---|---|
| 150 mg (0.50 mmol) | 1-Methylepigalanthamin (XXIII) |
| 0.13 ml (1.50 mmol) = 3 eq. | 3-Brom-1-propen (Allylbromid) |
| 4.00 ml | Dimethylformamid |

Die Reaktion wurde bei 60°C durchgeführt und nach 2 h aufgearbeitet.
Ausbeute: 150 mg (0.36 mmol = 64% d. Th.) hellbraunes Pulver
C₂₁H₂₈BrNO₃ [422.36]
- DC:: R_{f} = 0.11 (CHCl₃ : MeOH = 9:1)
- M.p.:: 140-145°C
C₂₁H₂₈BrNO₃ x 1 H₂O x 0.25 C₃H₇NO [458.64)

| | %C | %H | %N |
|---|---|---|---|
| berechnet | 56.96 | 6.98 | 3.82 |
| gefunden | 56.69 | 6.65 | 4.05 |

¹³C-NMR (50 MHz, DMSO-d₆): δ 144.7 (s), 134.5 (d), 134.0 (s), 131.1 (s), 128.3 (s), 126.1 (d), 115. 3 (d), 114.4 (d), 87.2 (d), 60.7 (d), 59.8 (t), 55.6 (q), 46,3 (s), 31.5 (t), 18.8 (q)

### Beispiel 98:

### [(±)-(4aα,6α,8aR*)]-4a,5,9,10,11,12-Hexahydro-6-hydroxy-3-methoxy-1,11-dimethyl-11-(4-(trifluormethyl)phenylmethyl)-6H-benzofuro[3a,3,2-ef][2]benzazepinium, Bromid (MH-75)

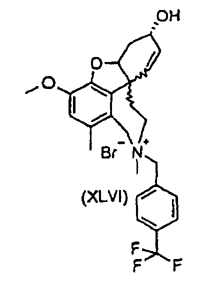

| | |
|---|---|
| 150 mg (0.50 mmol) | 1-Methylepigalanthamin (XXIII) |
| 357 mg (1.50 mmol) = 1.4 eq. | 4-Trifluormethyl-benzylbromid |
| 4.00 ml | Dimethylformamid |

Die Reaktion wurde bei 70°C durchgeführt und nach 1 h aufgearbeitet.
Ausbeute: 142 mg (0.26 mmol = 53% d. Th.) hellgelbes Pulyer
C₂₆H₂₉BrF₃NO₃ [540.42]
- DC:: R_{f} = 0.10 (CHCl₃ : MeOH = 9:1)
- M.p.:: 178-182°C
¹³C-NMR (50 MHz, DMSO-d₆): δ 144.8 (s), 134.4 (d + d), 134.2 (d), 132.6 (s), 131.2 (s), 130.9 (s), 130.3 (s), 126.6 (d), 125.8 (s), 121.2 (d), 114.5 (d), 87.3 (d), 60.8 (d), 55.6 (q), 46.3 (s), 34.3(t), 18.7 (q)

### Beispiel 99:

### [(±)-(4aα,6α,8aR*)]-4a,5,9,10,11,12-Hexahydro-6-hydroxy-3-methoxy-1,11-dimethyl-11-(phenylmethyl)-6H-benzofuro[3a,3,2-ef][2]benzazepinium, Bromid (MH-76)

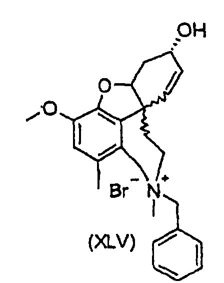

| | |
|---|---|
| 153 mg (0.51 mmol) | 1-Methylepigalanthamin (XXIII) |
| 92 mg (0.51 mmol) | Benzylbromid |
| 4.00 ml | Dimethylformamid |

Die Reaktion wurde bei 70°C durchgeführt und nach 3 h aufgearbeitet.
Ausbeute: 150 mg (0.32 mmol = 63% d. Th.) hellbraunes Pulver
C₂₅H₃₀BrNO₃ [472.42]
- DC:: R_{f} = 0.11 (CHCl₃ : MeOH = 9:1)
- M.p.:: 169-175°C
¹³C-NMR (50 MHz. DMSO-d₆): δ 144.6 (s), 134.1 (s), 133.4 (d), 131.0 (s), 130.4 (d), 128.9 (d), 128.1 (s), 114.4 (d), 87.2 (d), 61.8 (d), 59.4 (t), 55.6 (q), 46.3 (s), 31.5 (t), 18.6 (q)

### Beispiel 100:

### [(±)-(4aα,6α,8aR*)]-4a,5,9,10,11,12-Hexahydro-6-hydroxy-3-methoxy-1,11,11-trimethyl-6Hbenzofuro[3a,3,2-ef][2]benzazepinium, lodld (MH-B1)

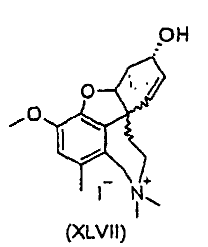

| | |
|---|---|
| 210 mg (0.70 mmol) | 1-Methylepigalanthamin (XXIII) |
| 290 mg (2.10 mmol) = 3 eq. | Methyliodid |
| 4.00 ml | Dimethylformamid |

Die Reaktion wurde bei 70°C durchgeführt und nach 2 h aufgearbeitet, indem das Reaktionsgemisch auf 30 ml Diethylether getropft wurde.
Ausbeute: 240 mg (0.54 mmol = 77% d. Th.) hellbraunes Pulver
C₁₉H₂₆lNO₃ [443.32]
- DC:: R_{f} = 0.05 (CHCl₃ : MeOH = 9:1)
- M.p.:: Zersetzung >280°C

| | %C | %H | %N |
|---|---|---|---|
| berechnet | 51.48 | 5.91 | 3.16 |
| gefunden | 51.25 | 5.75 | 3.32 |

¹³C-NMR (50 MHz, DMSO-d₆): δ 144.7 (s), 133.6 (s), 131.1 (s), 114.4 (d), 87.1 (d), 62.2 (t), 60.7 (q), 55.5 (q), 48.4 (d), 46.2 (s), 31.5 (t), 18.9 (q)

**Allgemeine Arbeitsvorschrift zur Herstellung von Galanthamin-N-Oxiden**

| | |
|---|---|
| 1 eq. | Galanthaminderivat |
| 1-1.5 eq. | 3-Chlorperbenzoesäure |
| 100 ml | Chloroform / 1 g Galanthaminderivat |
| 0.7 ml | H₂O₂ (35%) / 1 g Galanthaminderivat |

Die 3-Chlorperbenzoesöure wird in 1/3 Chloroform gelöst, mit Wasserstoffperoxid versetzt und 2 Minuten gerührt. Anschließend wird diese Lösung zu einer Lösung des Galanthaminderivats in 2/3 Chloroform hinzugefügt, 15 Mlnuten gerührt, eingeengt und mittels Säulenchromatographie gereinigt [Gradient: CHCl₃: MeOH = 9:1 → MeOH)

### Beispiel 101

### [4aS-(4αa,6α,8aR*)]-4a,5,9,10,11,12-Hexahydro-3-methoxy-11-methyl-6H-benzofuro[3a,3,2-ef][2]benzazepin-6-ol, 11-oxid, Epigalanthamin-N-oxid (PI-23)

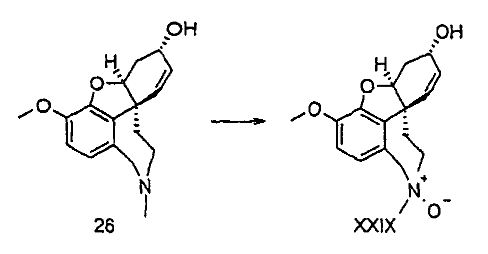

| | |
|---|---|
| 49.7 mg (0.17 mmol) | Epigalanthamin (26) |
| 29.9 mg (0.17 mmol) | 3-Chlorperbenzoesöure |
| 0.03 ml | Wasserstoffperoxid (35%) |
| 5 mi | Chloroform |

Ausbeute: 37 mg (71% d. Th.)
C₁₇H₂₁NO₄ (303.36)
- DC:: R_{f} = 0.05 (CHCl₃ : MeOH = 9:1)
H-NMR (200 MHz, CDCl₃): *δ* 6.67 (s, 2 H), 5.96 (bs, 2 H), 4.78 (d, *J* = 15.0 Hz, 1H), 4.67-4.50 (m, 2 H), 4.26 (d, *J* = 15.0 Hz, 1H), 3.83 (s, 3 H), 3.67-3.41 (m, 2 H), 3.41 (s, 2 H), 2.96 (s, 3 H), 2.77 (dt, *J* = 13.1, 3.7 Hz, 1H), 2.05 (bs, 1H), 1.74 (t, *J* = 11.3 Hz, 1H)
¹³C-Spektren konnten aufgrund der raschen Solvatbildung und Kristallisation in Chloroform nicht aufgenommen werden, der Strukturbeweis konnte jedoch durch Röntgenkristallo-graphie erbracht werden.

### Allgemeine Arbeitsvorschrift zur Herstellung von N-substituierten 1-Methylgalanthaminderivaten (Beispiet 102 - 106)

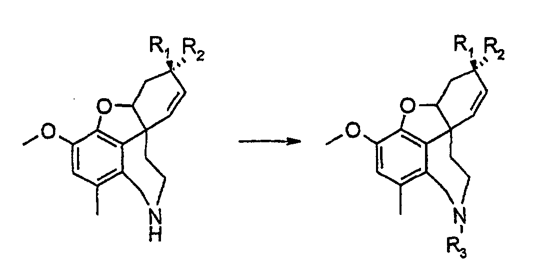

| | |
|---|---|
| 200 mg (0.70 mmol) | 1-Methyl-N-demethylgalanthamin (bzw. -epigalanthamin) |
| 192 mg (1.39 mmol) = 2 eq. | Kaliumcarbonat |
| 117 mg (0.78 mmol) = 1.1 eq. | Natriumiodid |
| (0.84 mmol) = 1.2 eq. | substituiertes Alkylhalogenid |
| 10 ml | Aceton, getrocknet über 4 Å Molsieb |

Natriumiodid, Kaliumcarbonat und Edukt wurden gut in einer Reibschale verrieben, das Gemisch zusammen mit einigen Glaskügelchen im Kolben vorgelegt und in abs. Aceton suspendiert. Das substituierte Alkylhalogenid wurde zudosiert und das Reaktionsgemisch auf Rückfluß erhitzt.
Nach Ende der Reaktion wurde das Reaktionsgemisch im Vakuum zur Trockene eingedampft und der Rückstand mit 2N HCl aufgenommen, die wäßrige Phase mit Ethylacetat gewaschen, dann mit konz. aq. Ammoniak basisch gemacht und wiederum mit Ethylacetat extrahiert. Die gesammelten organischen Phasen wurden mit gesättigter Kochsalzlösung gewaschen, über Natriumsulfat getrocknet, filtriert und das Lösungsmittel abgezogen. Das Rohprodukt wurde über eine Kieselgelsäule gereinigt (Laufmittel: CHCl₃: MeOH = 9:1 + 1% NH₄OH).

### Beispiel 102

### [(±)-(4aα,6β,8aR*)]-4a,5,9,10,11,12-Hexahydro-3-methbxy-1-methyl-11-(2-propenyl)-6Hbenzofuro[3a,3,2-ef][2]benzcizepin-6-ol, 1-Methyl-N-allylgalanthamin (MH-25)

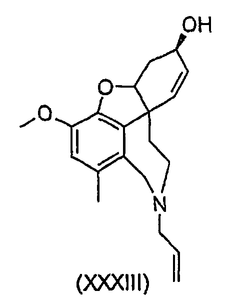

| | |
|---|---|
| 200 mg (0.70 mmol) | 1-Methyl-N-demethylgalanthamin (XXII) |
| 0.07 ml (0.84 mmol) = 1.2 eq. | 1-Brom-2-propen (Allylbromid) |

Nach 10 h wurde die Reaktion aufgearbeitet.
Ausbeute: 50 mg (0.15 mmol = 22% d. Th.) gelbes Öl
C₂₀H₂₅NO₃ [327.43]
- DC:: R_{f} = 0.17 (CHCl₃: MeOH = 9:1 + 1% NH₄OH)
C₂₀H₂₅NO₃ x 0.8 H₂O [341.83]

| | %C | %H | %N |
|---|---|---|---|
| berechnet | 70.27 | 7.84 | 4.10 |
| gefunden | 70.18 | 7.60 | 4.05 |

¹H-NMR (200 MHz, CDCl₃): δ 6.52 (s, 1H), 6.12 (d, *J* = 10.3 Hz, 1H) , 6.03-5.78 (m, 2 H), 5.18 (bs, 1H), 5.11 (d, *J* = 4.5 Hz, 1H), 4.57 (bs, 1H), 4.12 (bs, 1H), 4.09 (d, *J* = 15.0 Hz, 1H), 3.81 (s, 3 H), 3.78 (d, *J* = 15.0 Hz, 1H), 3.32-3.02 (m, 4 H), 2.72-2.58 (m, 1H), 2.21 (s, 3 H), 2.07-1.89 (m, 2 H), 1.57 (ddd, *J* = 13.7, 3.4, 2.7 Hz, 1H); ¹³C-NMR (50 MHz, CDCl₃): δ 144.0 (s), 143.0 (s), 136.0 (d), 133.6 (s), 129.1 (s), 127.4 (d), 127.2 (d), 126.9 (s), 117.5 (t), 113.7 (d), 88.4 (d), 62.0 (d), 57.2 (t), 55.8 (q), 52.9 (t), 52.0 (t), 48.4 (s), 33.9 (t), 29.8 (t), 19.4 (q)

### Beispiel 103

### [(±)-(4aα,6β,8aR*)]-4a,5,9,10,11,12-Hexahydro-3-methoxy-1-methyl-11-(phenylmethyl)-6Hbenzofuro[3a,3,2-ef][2]benzazepin-6-ol, 1-Methyl-N-benzylgalanthamin (MH-26)

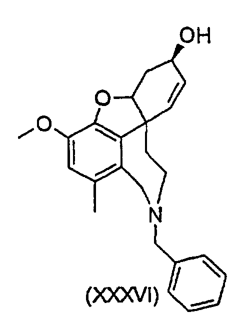

| | |
|---|---|
| 200 mg (0.70 mmol) | 1-Methyl-N-demethylgalanthamin (XXII) |
| 0.1 ml (0.84 mmol) = 1.2 eq. | Brommethylbenzol (Benzylbromid) |

Nach 24 h wurde die Reaktion aufgearbeitet.
Ausbeute: 140 mg (0.37 mmol = 53% d. Th.) gelbes Öl
C₂₄H₂₇NO₃ [377.48]
- DC:: R_{f} = 0.36 (CHCl₃: MeOH = 9:1 + 1% NH₄OH)
¹H-NMR (200 MHz, CDCl₃): δ 7.30 (m, 5 H), 6.50 (s, 1H), 6.16 (d, *J* = 10.2 Hz, 1H), 5.99 (dd, *J* = 10.2, 4.9 Hz, 1H), 4.61 (bs, 1H), 4.13 (bs, 1H), 4.00 (d, *J* = 15.7 Hz, 1H), 3.82 (s, 3 H), 3.81 (d, *J* = 15.7 Hz, 1 H), 3.69 (s, 2H), 3.34 (td, *J* = 14.1, 12.4, 1.8 Hz, 1H), 3.13 (td, *J* = 14.1, 3.5 Hz, 1H), 2.74-2.37 (m, 2 H), 2.19-1.93 (m, 2 H), 1.90 (s, 3 H), 1.57 (dt, *J* = 13.7, 3.0 Hz, 1H); ¹³C-NMR (50 MHz, CDCl₃): δ 143.9 (s), 143.0 (s), 138.9 (s), 133.6 (s), 129.2 (s), 128.7 (d), 128.2 (d), 127.4 (d), 127.3 (d), 127.2 (s), 126.9 (d), 113.7 (d), 88.4 (d), 62.0 (d), 57.4 (t), 55.8 (q), 52.4 (t), 52.2 (t), 48.5 (s), 33.7 (t), 29.8 (t), 39.1 (q)

### Beispiel 104

### [(±)-(4aα,6β,8aR*)]-4a,5,9,10,11,12-Hexahydro-3-methoxy-1-methyl-11-(2-(4-morpholinyl)ethyl)-6H-benzofuro[3a,3,2-ef][2]benzazepin-6-ol, 1-Methyl-N-morpholinoethyl-galanthamin (MH-28)

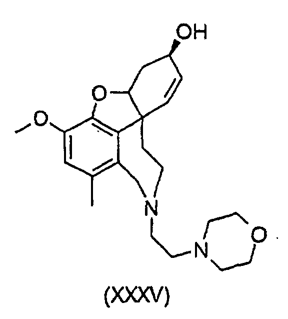

| | |
|---|---|
| 200 mg (0.70 mmol) | 1-Methyl-N-demethylgalanthamin (XXII) |
| 155 mg (0.84 mmol) = 1.2 eq | 4-(2-chlorethyl)morpholin Hydrochlorid |

Nach 24 h wurde die Reaktion aufgearbeitet.
Ausbeute: 210 mg (0.52 mmol = 75% d. Th.) gelbes Öl
C₂₃H₃₂N₂O₄. [400.52]
- DC:: R_{f} = 0.51 (CHCl₃: MeOH = 9:1 + 1% NH₄OH)
C₂₃H₃₂N₂O₄ x 0.9 H₂O [416.72]

| | %C | %H | %N |
|---|---|---|---|
| berechnet | 66.29 | 8.17 | 6.72 |
| gefunden | 66.28 | 8.09 | 6.85 |

¹H-NMR (200 MHz, CDCl₃): δ 6.52 (s, 1H), 6.10 (d, *J* = 10.3 Hz, 1H), 5.97 (dd, *J* = 10.3, 4.8 Hz, 1H), 4.55 (bs, 1H), 4.13 (bs, 1H), 4.12 (d, *J* = 15.9 Hz, 1H), 3.88 (d, *J* = 15.9 Hz, 1H), 3.81 (s, 3 H), 3.75-3.65 (m, 4 H), 3.30 (ddd, *J* = 14.3, 12.4, 2.0 Hz, 1H), 3.10 (dt, *J* = 14.3, 3.3 Hz, 1H), 2.76-2.58 (m, 4 H), 2.55-2.41 (m, 5 H), 2.25 (s, 3 H), 2.08-1.90 (m, 2 H), 1.55 (dd, *J* = 13.7,2.8 Hz, 1H); ¹³C-NMR (50 MHz, CDCl₃): δ 144.1 (s), 143.0 (s), 133.5 (s), 128.9 (s), 127.4 (d), 127.1 (d), 126.9 (s), 113.7 (d), 88.4 (d), 66.7 (t + t), 66.6 (t), 61.9 (d), 57.1 (t), 55.8 (q), 54.0 (t + t), 53.4 (t), 52.0 (t), 48.4 (s), 33.4 (t), 29.8 (t), 19.4 (q)

### Beispiel 105

### [(±)-(4aα,6β,8aR*)]-4a,5,9,10,11,12-Hexahydro-3-methoxy-1-methyl-11-(3-(1-piperidinyl)propyl)-6H-benzofuro[3a,3,2-ef][2]benzazepin-6-ol, 1-Methyl-N-piperidinopropylgalanthamin (MH-29)

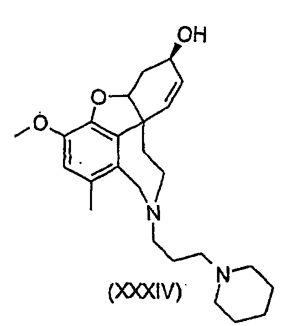

| | |
|---|---|
| 200 mg (0.70 mmol) | 1-Methyl-N-demethylgalanthamin (XXII) |
| 166 mg (0.84 mmol) = 1.2 eq. | 1-(3-Chlorpropyl)piperidin Hydrochlorid |

Nach 24 h wurde die Reaktion aufgearbeitet.
Ausbeute: 180 mg (0.44 mmol = 63% d. Th.) gelbes Öl
C₂₅H₃₆N₂O₃ [412.58]
- DC:: R_{f} = 0.27 (CHCl₃: MeOH = 9:1 + 1% NH₄OH)
C₂₅H₃₆N₂O₃ x 0.50 H₂O [421.58]

| | %C | %H | %N |
|---|---|---|---|
| berechnet | 71.23 | 8.85 | 6.64 |
| gefunden | 71.33 | 8.97 | 6.60 |

¹H-NMR (200 MHz, CDCl₃): δ 6.52 (s, 1H), 6.10 (d, *J* = 10.4 Hz, 1H), 5.96 (dd, J = 10.4, 4.7 Hz, 1H), 4.55 (bs, 1H), 4.12 (bs, 1H), 4.08 (d, *J* = 15.7 Hz, 1H), 3.83 (d, J = 15.7 Hz, 1H), 3.81 (s, 3 H), 3.24 (ddd, *J* = 14.2, 12.2, 2.0 Hz, 1H), 3.07 (dt, *J* = 14.2, 3.5 Hz, 1H), 2.71-2.13 (m, 10 H), 2.24 (s, 3 H), 2.07-1.88 (m, 2 H), 1.77-1.35 (m, 9 H); ¹³C-NMR (50 MHz, CDCl₃): δ 144.0 (s), 142.9 (s), 133.5 (s), 128.9 (s), 127.3 (d + d), 127.2 (s), 113.7 (d), 88.4 (d), 62.0 (d), 57.2 (t), 55.8 (q), 54.5 (t + t + t), 53.3 (t), 51.4 (t), 48.5 (s), 33.4 (t), 29.8 (t), 25.7 (t + t), 25.0 (t), 24.2 (t), 19.5 (q)

### Beispiel 106

### [(±)-(4aα,6α,8aR*)]-4a,5,9,10,11,12-Hexahydro-3-methoxy-1-methyl-11-(3-(1-piperidinyl)propyl)-6H-benzofuro[3a,3,2-ef][2]benzazepin-6-ol, 1-Methyl-N-piperidinopropylepigalanthamin (MH-117)

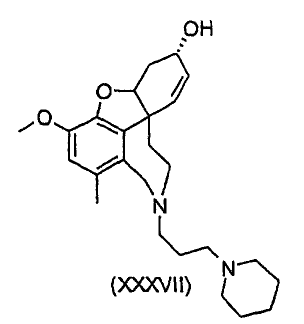

| | |
|---|---|
| 100 mg (0.35 mmol) | 1-Methylepigalanthamin (XXIV) |
| 83 mg (0.42 mmol) = 1.2 eq. | (1-(3-Chlorpropyl)piperidin Hydrochlorid |

Nach 28 h wurde die Reaktion aufgearbeitet.
Ausbeute: 60 mg (0.15 mmol = 42% d. Th.) hellgelbes Öl
C₂₅H₃₆N₂O₃ [412.58]
- DC:: R_{f} = 0.12 (CHCl₃ : MeOH = 9:1)
¹H-NMR (200 MHz, CDCl₃): δ 6.50 (s, 1H), 6.10 (d, J = 10.2 Hz, 1H) , 5.78 (dd, J = 10.2, 1H), 4.70-4.57 (m, 1H), 4.54 (bs, 1H), 4.05 (d, J = 15.2 Hz, 1H), 3.82 (d, J = 15.2 Hz, 1H), 3.82 (s, 3 H), 3.25 (ddd, J = 13.5, 12.8, 1.6 Hz, 1H), 3.09 (dt, J = 13.5, 2.5 Hz, 1H), 2.75 (dt, J = 13.7, 4.1 Hz, 1H), 2.56-2.27 (m, 8 H), 2.23 (s, 3 H), 2.08 (td, J = 13.1, 4.0, 2H), 1.81-1.38 (m, 9 H); ¹³C-NMR (50 MHz, CDCl₃): δ 144.9 (s), 142.7 (s), 133.4 (s), 131.3 (s), 128.3 (d + d), 127.0 (s), 113.3 (d), 88.1 (d), 63.0 (d), 57.2 (t), 55.7 (q), 54.4 (t + t + t), 53.1 (t), 51.6 (t), 48.4 (s), 33.1 (t), 29.5 (t), 25.6 (t + t), 24.8 (t), 24.1 (t), 19.5 (q)

### Allgemeine Arbeitsvorschrift zur Herstellung von Galanthaminiumbromid-Derivaten (Beispiel 107-109)

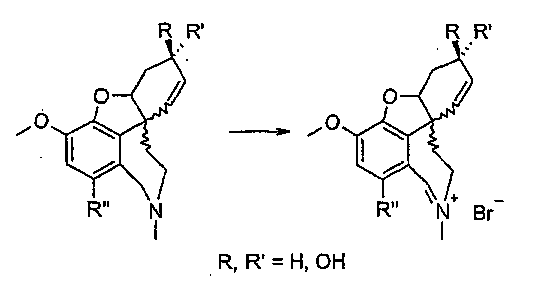

| | |
|---|---|
| 1 eq. | Galanthaminderivat |
| 1 eq. | N-Bromsuccinimid |
| 50 ml | Methylenchlorid oder Chloroform, dest. über P₂O₅ / 1 g Galanthaminderivat |

Das Edukt wird im Lösungsmittel gelöst und unter Rühren N-Bromsuccinimid zugegeben. Es bildet sich sofort ein Niederschlag, der nach einer entsprechenden Zeit abgesaugt, gewaschen und trockengesaugt wird.

Die so gewonnenen Produkte sind in der Regel sehr rein, wichtig ist jedoch die eher große Menge an Lösungsmittel, da sonst Succinimid in die Substanzen eingeschleppt wird und diese nachträglich schwer zu reinigen sind.

### Beispiel 107

### [4aS-(4aα,6β,8aR*)]-4a,5,9,10-Tetrahydro-6-hydroxy-3-methoxy-11-methyl-6H-benzofuro[3a,3,2-ef][2]benzazepinium, Bromid, Galanthaminiumbromid (MH-119)

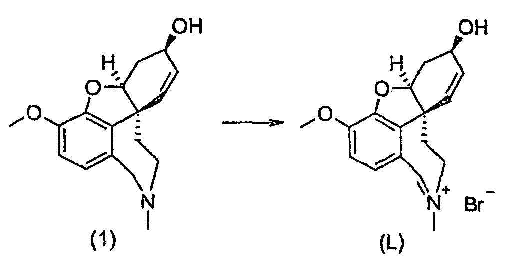

| | |
|---|---|
| 200 mg (0.7 mmol) | Galanthamin (1) |
| 124 mg (0.7 mmol) | N-Bromsuccinimid |
| 10 ml | Methylenchlorid oder Chloroform, dest. über P₂O₅ |

Der entstandene Niederschlag wurde nach 10 min abgesaugt.
Ausbeute: 230 mg (0.63 mmol = 90% d. Th.) hellgelbes Pulver
C₁₇H₂₀BrNO₃ [366.25]
- DC:: R_{f} = 0.58 (CHCl₃ : MeOH = 9:1 + 1 % NH₄OH)
- M.p.:: 216-219°C
C₁₇H₂₀BrNO₃ x 0.1 HBr [374.34]

| | %C | %H | %N |
|---|---|---|---|
| berechnet | 54.55 | 5.41 | 3.74 |
| gefunden | 54.52 | 5.36 | 3.66 |

¹H-NMR (200 MHz, CDCl₃): δ 9.10 (s, 1H), 7.54 (d, *J* = 8.5 Hz, 1H), 7.27 (d, *J* = 8.5 Hz, 1H), 5.92 (dd, *J* = 10.3, 4.5 Hz, 1H), 5.73 (d, *J* = 10.3 Hz, 1H), 4.74 (s, 1H), 4.59 (s, 1H), 4.11 (s, 2 H), 3.94 (s, 3 H), 3.79 (s, 3 H), 2.38 (d, *J* = 15.3 Hz, 1H), 2.15 (m, 3 H); ¹³C-NMR (50 MHz, CDCl₃): δ 167.3 (d), 151.3 (s), 146.2 (s), 136.9 (s), 133.0 (d), 129.8 (d), 126.4 (d), 115.0 (s), 112.9 (d), 87.0 (d), 58.9 (d), 56.4 (q), 54.0 (t), 51.5 (q), 45.9 (s), 31.1 (t), 29.7 (t)

### Beispiel 108

### [(±)-(4aα,6β,8aR*)]-4a,5,9,10-Tetrahydro-6-hydroxy-3-methoxy-1,11-dimethyl-6Hbenzofuro[3a,3,2-ef][2]benzazepinium, Bromid, 1-Methylgalanthaminiumbromid (Pi-8)

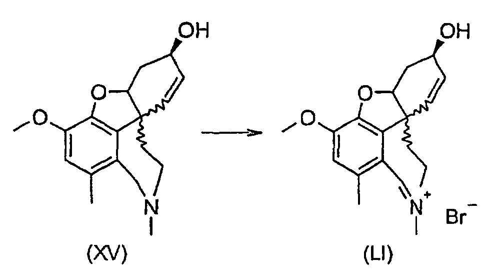

| | |
|---|---|
| 200 mg (0.66 mmol) | 1-Methylgalanthamin (XV) |
| 118 mg (0.66 mmol) | N-Bromsuccinimid |
| 5 ml | Chloroform |

Nach 5 Minuten entstand ein orange-gelber Niederschlag, der nach 15 Minuten abgesaugt wurde. Der Niederschlag (162 mg) wurde zweimal mit Diethylether gewaschen. Das Filtrat wurde eingeengt, in wenig Ethanol aufgenommen und in Diethylether ausgefällt (54 mg).
Ausbeute: 216 mg (0.57 mmol = 86% d. Th.) orange-gelbes Pulver
C₁₈H₂₂BrNO₃ [380.28]
- DC:: R_{f} = 0.02 (CHCl₃ : MeOH = 9:1)
- M.p.:: 223-226°C
C₁₈H₂₂BrNO₃ x 0.35 HBr [408.60]

| | %C | %H | %N |
|---|---|---|---|
| berechnet | 52.91 | 5.51 | 3.43 |
| gefunden | 52.99 | 5.52 | 3.48 |

¹H-NMR (200 MHz, DMSO-d₆): δ 9.06 (s, 1H), 7.04 (s, 1H), 5.81 (dd, *J* = 10.1, 4.5 Hz, 1H), 5.54 (d, *J* = 10.2 Hz, 1H), 4.74 (s, 1H), 4.17-3.95 (m, 4 H), 3.91 (s, 3 H), 3.86 (s, 3 H), 2.53 (s, 3 H), 2.40-1.96 (m, 4 H); ¹³C-NMR (50 MHz, DMSO-d₆): δ 166.4 (d), 150.5 (s), 144.7 (s), 140.4 (s), 136.7 (s), 128.4 (d), 127.9 (d), 114.9 (d), 113.9 (s), 86.5 (d), 58.7 (d), 56.3 (q), 54.4 (t), 50.5 (q), 47.0 (s), 35.1 (t), 29.4 (t), 18.9 (q)

### Beispiel 109

### [4aS-(4aa,6a,8aR*)]-4a,5,9,10-Tetrahydro-6-hydroxy-3-methoxy-11-methyl-6Hbenzofuro[3a,3,2-ef][2]benzazepinium, Bromid, Epigalanthaminiumbromid (Pi-13)

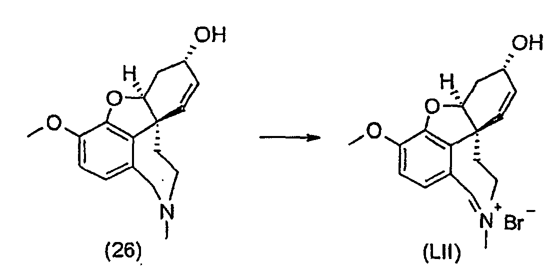

| | |
|---|---|
| 0.78 g (2.71 mmol) | Epigalanthamin (26) |
| 0.48 g (2.71 mmol) | N-Bromsuccinimid |
| 6 ml | absolutes Chloroform |

Nach 3 Minuten entstand ein gelber Niederschlag, der abgesaugt und zweimal mit Diethylether gewaschen wurde. Eine zweite Fraktion konnte durch Einengen des Filtrats und Tropfen auf 60 ml Diethylether gewonnen werden. Die zweite Fraktion wurde zur Reinigung in wenig Ethanol gelöst und auf Diethylether getropft.
Ausbeute: 0.91 g (2.48 mmol = 92% d. Th.)
C₁₇H₂₀BrNO₃ [366.25]
- DC:: R_{f} = 0.05 (CHCl₃ : MeOH = 9:1 + 1% NH₄OH)
- M.p.:: 205-210°C
C₁₇H₂₀BrNO₃ x 0.3 HBr [390.52]

| | %C | %H | %N |
|---|---|---|---|
| berechnet | 52.28 | 5.24 | 3.59 |
| gefunden | 52.12 | 5.18 | 3.88 |

¹H-NMR (200 MHz, DMSO-d₆): δ 9.10 (s, 1H), 7.51 (d, *J* = 11.5 Hz, 1H), 7.20 (d, *J* = 11.5 Hz, 1H), 5.82 (d, J = 12.7 Hz, 1H), 5.68 (d, J = 12.7 Hz, 1H), 4.80 (bs, 1H), 4.40-4.21 (m, 1H), 4.21-4.04 (m, 2 H), 3.94 (s, 3 H), 3.77 (s, 3 H), 2.60 (s, 1H), 2.30-2.10 (m, 2 H), 1.81-1.60 (m, 1H); ¹³C-NMR (50 MHz, DMSO-d₆): δ 167.3 (d), 151.2 (s), 146.5 (s), 137.3 (s), 134.4 (d), 133.0 (d), 126.0 (d), 115.0 (s), 113.0 (d), 88.0 (d), 60.7 (d), 56.4 (q), 54.2 (t), 51.4 (q), 46.2 (s), 31.5 (t), 30.8 (t)

### Allgemeine Arbeitsvorschrift zur Herstellung von Galanthamin-12-carbonitrilderivaten (Beispiel 110-113)

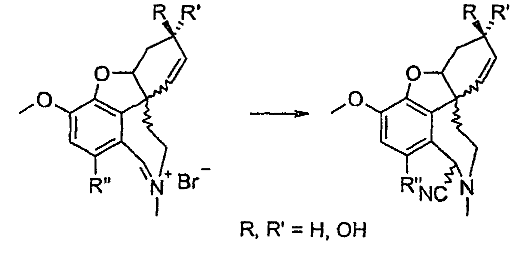

| | |
|---|---|
| 1 eq. | Galanthaminiumderivat |
| 3 eq. | Kaliumcyanid |
| 30 ml | Wasser / 1 g Galanthaminiumderivat |
| 10 ml | Diethylether / 1 g Galanthaminiumderivat |

Das Edukt wurde in einem Scheidetrichter in Wasser gelöst und die Lösung mit Ether überschichtet, dann festes Kaliumcyanid (frisch verrieben) dazugegeben, wobei sich sofort ein weißer Niederschlag in der wäßrigen Phase bildete. Nach etwa 2-3 Minuten wurde das Produkt durch Schütteln in die Etherphase extrahiert. Die wäßrige Phase wurde mit Ether und bei schwerer löslichen Derivaten mit Chloroform erschöpfend extrahiert, die organischen Phasen vereinigt, mit gesättigter wässriger Kochsalzlösung gewaschen, über Na₂SO₄ getrocknet, filtriert und das Lösungsmittel abgezogen. Bei Bedarf wurde das erhaltene Produkt über eine Kieselgelsäule gereinigt (Laufmittel: CHCl₃ : MeOH = 9:1, wenn nicht anders angegeben).

### Beispiel 110

### [4aS-(4aα,6β,8aR*)]-4a,5,9,10,11,12-Hexahydro-6-hydroxy-3-methoxy-11-methyl-6Hbenzofuro[3a,3,2-ef][2]benzazepin-12-carbonitril, Galanthamin-12-carbonitril (MH-123)

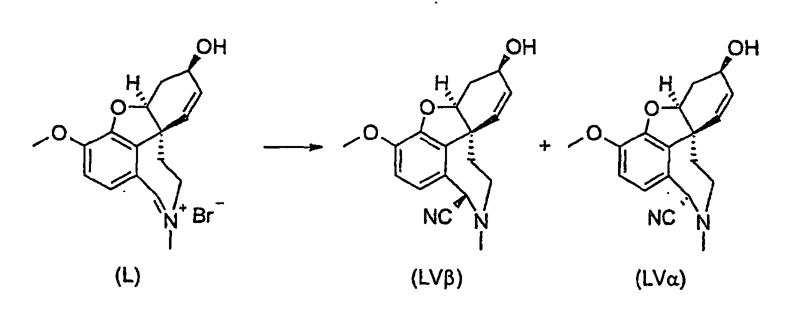

| | |
|---|---|
| 3.50 g (9.56 mmol) | Galanthaminiumbromid (L) |
| 1.90 g (28.7 mmol) = 3eq. | Kaliumcyanid |
| 100 ml | Wasser |
| 40.0 ml | Diethylether |

Die Reaktion wurde nach der allgemeinen Arbeitsvorschrift durchgeführt.
Rohausbeute >2 g

Das ölige Rohgemisch, bestehend aus einem Isomerenverhältnis von ca. 9:1, wurde in möglichst wenig Ethanol gelöst und unter Rühren das Hauptisomer zur Kristallisation gebracht. Der Niederschlag wurde abgesaugt, mit Ethanol gewaschen und das Filtrat mit der Waschlösung vereinigt und eingeengt. Der Vorgang wurde wiederholt, bis die Hauptmenge an reinem Hauptisomer isoliert war.
Ausbeute: 990 mg (3.28 mmol = 34% d. Th.) weißes Pulver
C₁₈H₂₀N₂O₃ [312.37]

Es verblieb ein Gemisch aus beiden Isomeren (Verhältnis 9:1) und Galanthamin, das während der Isolierung des Hauptisomers entsteht, welches durch Säulen gereinigt werden konnte. Aus der Säule eluierte wieder das Isomerengemisch im Verhältnis 9:1, da es sich auf der Säule ineinander umwandelt.
- DC:: R_{f} = 0.77 Hauptisomer
- 0.63 Nebenisomer: (CHCl₃ : MeOH = 9:1)
- M.p.: 151-155°C
C₁₈H₂₀N₂O₃ x 0.1 H₂O [314.17]

| | %C | %H | %N |
|---|---|---|---|
| berechnet | 68.82 | 6.48 | 8.92 |
| gefunden | 68.85 | 6.32 | 8.69 |

¹H-NMR (200 MHz, CDCl₃): δ 6.70 (s, 2 H), 6.35 [d, *J* = 10.2 Hz, 1H), 6.07 (dd, *J* = 10.2, 5.3 Hz. 1H), 4.71 (s, 1H), 4.61 (m, 1H), 4.15 (dt, *J* = 11.1, 5.0 Hz, 1H), 3.85 (s, 3 H), 3.50 (dd, *J* = 15.0, 13.6 Hz, 1 H), 2.91 (dt, *J* = 15.0,3.2 Hz, 1H), 2.74-2.61 (m, 1H), 2.61 (s, 3 H), 2.38 (d, *J* = 11.4 Hz, 1H), 1.98-2.08 (m, 2 H), 1.78 (ddd, *J* = 13.7, 5.0, 1.2 Hz, 1H); ¹³C-NMR (50 MHz, CDCl₃): δ 146.7 (s), 145.6 (s), 132.9 (s), 128.2 (d), 126.9 (d), 124.2 (s), 122.5 (s), 111.6 (d), 88.9 (d), 61.6 (d + d), 55.9 (q), 49.9 (t), 48.1 (s), 46.1 (q), 36.4 (t), 29.7 (t)

### Beispiel 111

### [(±)-(4aα,6β,8aR*)]-4a,5,9,10,11,12-Hexahydro-6-hydroxy-3-methoxy-1,11-dimethyl-6Hbenzofuro[3α,3,2-ef][2]benzazepin-12-carbonitril, 1-Methylgalanthamin-12-carbonitril (Pi-12)

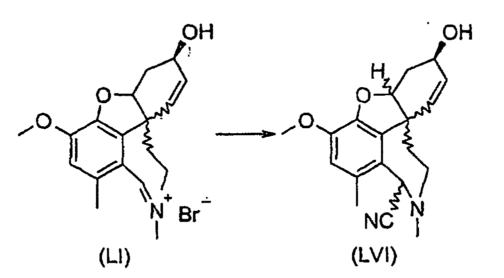

| | |
|---|---|
| 300 mg (0.79 mmol) | 1-Methylgalanthaminiumiumbromid (LI) |
| 154 mg (2.36 mmol) = 3 eq. | Kaliumcyanid |
| 20.0 ml | Wasser |

Es konnten 205 mg eines weißen Rohproduktes isoliert werden.

Es wurde eine säulenchromatographische Trennung der Diastereomeren mit reinem Ethylacetat als Laufmittel versucht. Dabei konnte aber keine Trennung des Diastereomerengemisches erreicht werden.
Ausbeute: 151 mg (0.46 mmol = 59% d. Th.)
C₁₉H₂₂N₂O₃ [326.39]
- DC:: R_{f} = 0.30/0.65 (Diastereomerengemisch; Ethylacetat)
- M.p.:: 72-73°C
C₁₉H₂₂N₂O₃ x 0.5 H₂O [335.39]

| | %C | %H | %N |
|---|---|---|---|
| berechnet | 68.04 | 6.91 | 8.35 |
| gefunden | 67.91 | 6.62 | 8.20 |

¹H-NMR (Gemisch aus 2 Isomeren, 200 MHz, CDCl₃): δ 6.59 (s, 1H), 6.31 (d, J = 8.9 Hz, 0.4 H), 6.27 (d, J = 8.9 Hz, 0.6 H), 6.01 (dd, J = 9.2, 5.0 Hz, 1H), 4.96 (s, 0.6 H), 4.83 (s, 0.4 H), 4.57 (bs, 0.6 H), 4.50 (bs, 0.4 H), 4.12 (dt, J = 15.5, 4.9 Hz, 1H), 3.83 (s, 3 H), 3.47 (ddd, J = 13.9, 9.8, 3.4 Hz, 1H), 2.88 (dt, J = 14.6, 3.8 Hz, 1H), 2.70 (bs, 1H), 2.62 (s, 1.8 H), 2.60 (s, 1.2 H), 2.31 (s, 3 H), 2.10-1.92 (m, 2 H), 1.87-1.68 (m, 1H); ¹³C-NMR (Gemisch aus 2 Isomeren, 100 MHz, CDCl₃): δ 145.5 (s), 145.4 (s), 145.3 (s), 144.8 (s), 135.7 (s), 133.6 (s), 129.7 (d), 129.3 (d), 129.6 (s), 128.7 (s), 128.4 (d), 127.7 (d), 122.9 (s), 122.8 (s), 116.7 (s), 115.3 (s), 114.5 (d), 114.4 (d), 89.4 (d), 88.9 (d), 62.3 (d), 62.2 (d), 57.9 (d), 56.6 (d), 56.5 (q), 56.3 (q), 51.0 (t), 50.3 (t), 49.0 (s), 47.9 (s), 47.2 (q), 47.2 (s), 36.8 (t), 33.7 (t), 30.2 (t), 20.3 (q), 19.3 (q)

### Beispiel 112

### [4aS-(4aa,6a,8aR*)]-4a,5,9,10,11,12-Hexahydro-6-hydroxy-3-methoxy-11-methyl-6Hbenzofuro[3a,3,2-ef][2]benzazepin-12-carbonitril, Epigalanthamin-12-corbonitril (Pi-14)

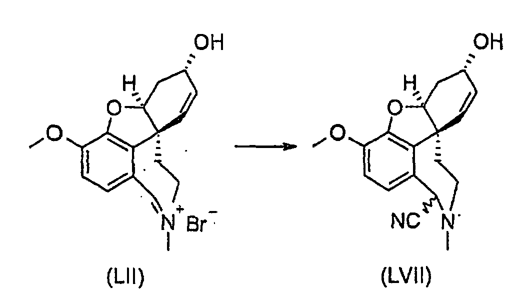

| | |
|---|---|
| 500 mg (1.37 mmol) | Epigalanthaminiumbromid (LII) |
| 270 mg (4.10 mmol) = 3 eq. | Kaliumcyanid |
| 15.0 ml | Wasser |

Ausbeute: 0.33 g (1.06 mmol = 77% d. Th.)
C₁₈H₂₀N₂O₃ [312.37]
- DC:: R_{f} = 0.75 (CHCl₃: MeOH = 9:1)
- M.p.:: 90-96°C
¹H-NMR (Gemisch aus 2 Isomeren, 200 MHz, CDCl₃): δ 6.92 (d, *J* = 8.3 Hz, 0.3 H), 6.72 (d, *J* = 8.0 Hz, 0.3 H), 6.68 (d, *J* = 8.0 Hz, 0.7 H), 6.62 (d, *J* = 8.3 Hz, 0.7 H), 6.31 (dt, *J* = 10.5,1.6 Hz, 0.7 H), 6.03 (d, J = 10.5 Hz, 0.3 H), 5.85 (d, *J* = 10.3 Hz, 1H), 5.22 (s, 0.3 H), 4.64 (s, 0.7 H), 4.58 (bs, 1H), 3.86 (s, 0.9 H), 3.85 (s, 2.1H), 3.12 (dt, *J* = 14.8, 3.2 Hz, 0.3 H), 2.98-2.70 (m, 1.7 H), 2.58 (s, 2.1H), 2.38 (s, 0.9 H), 2.27-2.04 (m, 1.2 H), 1.85 (dd, *J* = 13.5, 4.2 Hz, 1.4 H), 1.71 (ddd, J = 13.5, 10.7, 2.5 Hz, 1.4 H); ¹³C-NMR (Gemisch aus 2 Isomeren, 50 MHz, CDCl₃): δ 147.5 und 147.2 (s), 145.2 und 145.0 (s), 132.6 (s), 132.5 und 131.9 (d), 126.6 (d), 124.0 und 123.3 (s), 121.8 und 120.1 (d), 116.5 (s), 111.3 und 111.2 (d), 88.7 und 88.4 (d), 62.8 (d), 61.4 und 58.4 (d), 55.8 (q), 50.0 (t), 47.9 (s), 45.9 (q), 36.9 (t), 32.0 und 31.7 (t)

### Beispiel 113

### [(±)-(4aα,6α,8aR*)]-4a,5,9,10,11,12-Hexahydro-6-hydroxy-3-methoxy-1,11-dimethyl-6Hbenzofuro[3a,3,2-ef][2]benzazepin-12-carbonitril, 1-Methylepigalanthamin-12-carbonitril (Pi-19)

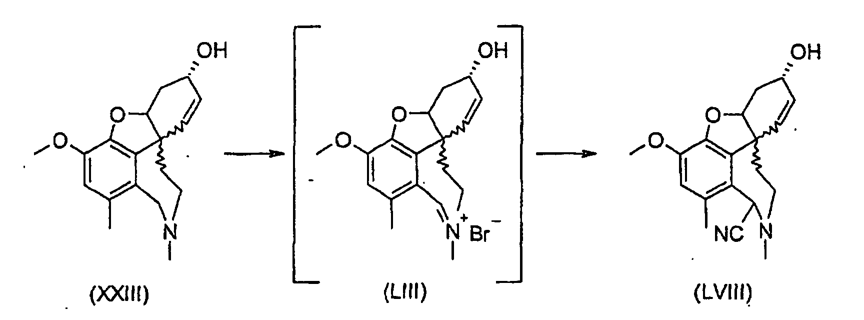

1-Methylepigalanthaminiumbromid wurde nach der allgemeinen Arbeitsvorschrift zur Herstellung von Galanthaminiumderivaten hergestellt, wobei sich jedoch kein Niederschlag bildete. Die Reaktionslösung wurde daher zur Trockene eingeengt, der verbleibende Rückstand in Ether aufgenommen, abgesaugt und gewaschen. Das verbliebene Rohprodukt wurden nach NMR-Analyse direkt in die folgende Reaktion eingesetzt.

| | |
|---|---|
| 500 mg (1.32 mmol) | 1-Methylepigalanthaminiumbromid (LIII) |
| 260 mg (3.96 mmol) = 3 eq. | Kaliumcyanid |
| 50 ml | Wasser |

Die Reinigung erfolgte mittels Säulenchromatographie (CHCl₃ : MeOH = 9:1).
- Ausbeute:: 220 mg (0.67 mmol = 51% d. Th.) weißer Schaum
C₁₉H₂₂N₂O₃ [326.39]
- DC:: R_{f} = 0.70/0.60 (Diastereomerengemisch; CHCl₃ : MeOH = 9:1)
¹H-NMR (Gemisch aus 2 Isomeren, 200 MHz, CDCl₃): δ 6.57 (s, 1H), 6.26 (d, J = 10.4 Hz, 1H), 5.82 (d, J = 10.4 Hz, 1H), 4.94 und 4.82 (s, 1H), 4.74-4.55 (m, 1H), 4.50 und 4.45 (m, 1H), 3.87 und 3.84 (s, 3 H), 3.55-3.32 (m, 1H), 3.05-2.68 (m, 2 H), 2.58 und 2.57 (s, 3 H), 2.33 und 2.30 (s, 3 H), 2.23-2.07 (m, 1H), 1.93-1.63 (m, 2 H); ¹³C-NMR (Gemisch aus 2 Isomeren, 50 MHz, CDCl₃): δ 146.0 (s), 145.8 (s), 144.5 (s), 144.1 (s), 135.0 (s), 133.0 (s), 132.2 (d), 131.7 (d), 129.5 (d), 128.5 (s), 127.6 (s), 127.1 (d), 122.4 (s), 122.2 (s), 116.4 (s), 114.8 (s), 113.9 (d), 113.8 (d), 88.5 (d), 88.4 (d), 63.0 (d), 62.6 (d), 57.2 (d), 56.4 (d), 56.0 (q), 55.8 (q), 50.7 (t), 50.0 (t), 48.4 (s), 47.3 (s), 46.7 (q), 36.9 (t), 34.1 (t), 31.7 (t), 19.8 (q), 18.8 (q)

### Allgemeine Arbeitsvorschrift zur Herstellung von 12-Methylgalanthaminderivaten (Beispiel 114-117)

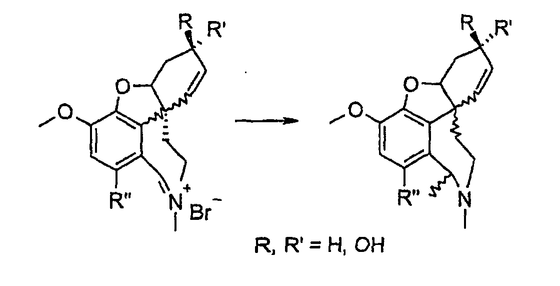

| | |
|---|---|
| 1 eq. | Galanthaminiumderivat |
| 2-4 eq. | Methylmagnesiumiodid (3M in Diethylether) |
| 20 ml | abs. Diethylether / 1 g Galanthaminiumderivat |

Das gesamte Grignardreagens wurde unter N₂-Atmosphäre vorgelegt und anschließend das feste Galanthaminiumderivat ohne Lösungsmittel zugegeben. Nach der jeweils angegebenen Zeit wurde Diethylether zugefügt und eine bestimmte Zeit gerührt, wobei sich die feste Masse auflöste. Dann wurde mit Wasser hydrolysiert, die Reaktionslösung mit konz. Ammoniak basisch gestellt und mit Ethylacetat extrahiert. Die organischen Phasen wurden mit gesättigter Kochsalzlösung gewaschen, über Natriumsulfat getrocknet, filtriert und das Lösungsmittel abgezogen.

### Beispiel 114

### [4aS-(4aα,6β,8aR*)]-4a,5,9,10,11,12-Hexahydro-3-methoxy-11,12-dimethyl-6H-benzofuro[3a,3,2-ef][2]benzazepin-6-ol, 12-Methylgalanthamin (Pi-4)

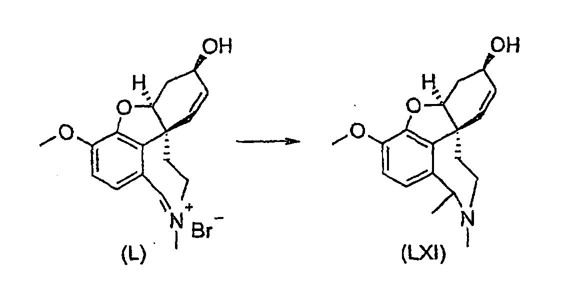

| | |
|---|---|
| 2.00 g (5.46 mmol) | Galanthaminiumbromid (L) |
| 6.70 ml (20.2 mmol) = 3.7 eq. | Methylmagnesiumiodid (3M in Diethylether) |
| 40 ml | abs. Diethylether |

Nach 40 min wurde das Lösungsmittel zugegeben und 5 h gerührt, bevor hydrolysiert wurde.
- Ausbeute:: 760 mg (2.52 mmol = 46% d. Th.) hellgelber Schaum
C₁₈H₂₃NO₃ [301.38]
- DC:: R_{f} = 0.65 (CHCl₃ : MeOH = 9:1 + 1% NH₄OH)
- M.p.:: 46-48°C
¹H-NMR (Gemisch aus 2 Isomeren, 200 MHz, CDCl₃): δ 6.66 (d, *J* = 8.3 Hz, 0.8 H), 6.65 (s, 0.2 H), 6.64 (s, 0.2 H), 6.57 (d, *J* = 8.3 Hz, 0.8 H), 6.13 (d, *J* = 10.1 Hz, 0.2 H), 6.07 (d, *J* = 10.1 Hz, 0.8 H), 5.94 (dd, *J* = 10.1, 4.4 Hz, 1H), 4.54 (bs, 1H), 4.26 (q, *J* = 7.0 Hz, 0.2 H), 4.08 (t, J = 4.4 Hz, 1H), 3.88 (q, *J* = 7.4 Hz, 0.8 H), 3.80 (s, 0.6 H), 3.78 (s, 2.4 H), 3.62 (ddd, *J* = 14.6, 13.2, 1.0 Hz, 0.8 H), 3.45 (d, 14.2 Hz, 0.2 H), 3.12 (dt, *J* = 14.8,3.3 Hz, 0.2 H), 2.85 (td, *J* = 15.5, 3.5 Hz, 0.8 H), 2.76 (bs, 1H), 2.63 (d, *J* = 15.6 Hz, 1H), 2.43 (s, 3 H), 2.16 (d, J = 2.7 Hz, 0.2 H), 1.98 (dt, *J* = 15.5, 2.3 Hz, 0.8 H), 1.95 (dd, *J* = 15.5, 2.3 Hz, 0.2 H), 1.51 (d, *J* = 7.3 Hz, 2.4 H), 1.47 (d, *J* = 7.3 Hz, 0.6 H); ¹³C-NMR (Gemisch aus 2 Isomeren, 50 MHz, CDCl₃): δ 146.1 und 145.6 (s), 143.8 und 143.4 (s), 134.9 und 132.6 (s), 132.4 und 131.3 (s), 129.3 (d), 127.6 und 127.3 (d), 126.9 (d), 122.0 und 119.9 (d), 111.5 und 110.8 (d), 88.6 und 88.5 (d), 64.0 (d), 61.9 und 61.6 (d), 58.3 und 55.7 (q), 48.8 (s), 44.1 (t), 41.3 (q), 31.5 und 31.0 (t), 29.9 und 29.7 (t), 21.8 und 17.5 (q)

### Beispiel 115

### [4aS-(4a?,6?,8aR*,12R*)]-4a,5,9,10,11,12-Hexahydro-3-methoxy-11,12-dimethy]-6Hbenzofuro[3a,3,2-ef][2]benzazepin-6-ol, (=12-Methylgalanthamin); (Hauptisomer)

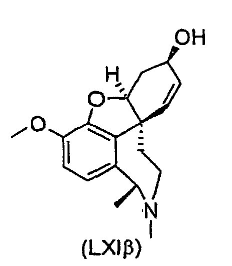

Das Isomerengemisch wurde säulenchromatographisch über Kieselgel gereinigt (CHCl₃: MeOH = 9:1 + 1% NH₄OH), wobei ein reines Isomer erhalten werden konnte.
¹H-NMR (200 MHz, CDCl₃): δ 6.66 (d, J = 8.3 Hz, 1H), 6.57 (d, J = 8.3 Hz, 1H), 6.07 (d, *J* = 10.1 Hz, 1 H), 5.94 (dd, *J* = 10.1, 4.4 Hz, 1H), 4.54 (bs, 1H), 4.08 (t, *J* = 4.4 Hz, 1H), 3.88 (q, *J* = 7.4 Hz, 1H), 3.78 (s, 3 H), 3.62 (dd, *J* = 14.6, 13.2 Hz, 1H), 2.85 (td, J = 15.5, 3.5 Hz, 1H), 2.63 (d, *J* = 15.6 Hz, 1H), 2.43 (s, 3 H), 2.11 (dt, *J* = 13.3, 2.4 Hz, 1H), 1.95 (ddd, *J* = 16.5, 5.0,1.8 Hz, 1H), 1.51 (d, *J* = 7.3 Hz, 3 H), 1.47 (dd, *J* = 13.3 Hz, 1H); ¹³C-NMR (50 MHz, CDCl₃): δ 146.2 (s), 143.5 (s), 135.1 (s), 131.4 (s), 129.4 (d), 127.4 (d), 122.2 (d), 111.6 (d), 88.8 (d), 64.0 (d), 61.8 (d), 55.8 (q), 48.9 (s), 44.2 (t), 41.5 (q), 31.7 (t), 29.8 (t), 21.9 (q)

### Beispiel 116

### [(±)-(4aα,6β,8aR*)]-4a,5,9,10,11,12-Hexahydro-3-methoxy-6H-1,11,12-trimethyl-benzofuro[3a,3,2-ef][2]benzazepin-6-ol, 1,12-Dimethylgalanthamin (Pi-21)

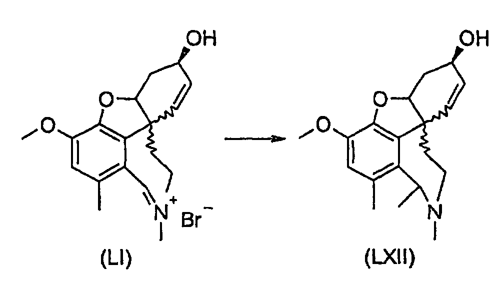

| | |
|---|---|
| 500 mg (1.31 mmol) | 1-Methylgalanthaminiumbromid (LI) |
| 1.00 ml (3.00 mmol) = 2.3 eq. | Methylmagnesiumiodid (3M in Diethylether) |
| 15 ml | absoluter Diethylether |

Während der Eduktzugabe wurden 5 ml Diethylether zugefügt, um das Reaktionsgemisch rührbar zu halten. Nach Ende der Eduktzugabe (30 min) wurden weitere 10 ml Diethylether zugegeben. Nach 2.5 Stunden wurde das Reaktionsgemisch hydrolysiert.
Ausbeute: 73 mg (0.23 mmol = 18% d. Th.)
C₁₉H₂₅NO₃ [315.41]
- DC:: R_{f} = 0.50 (CHCl₃ : MeOH = 9:1 + 1% NH₄OH)
- M.p.:: 45-50°C
¹H-NMR (Gemisch aus 2 Isomeren, 200 MHz, CDCl₃): δ 6.57 (s, 1H), 6.06 (d, *J* = 10.2 Hz, 1H), 5.95 (dd, *J* = 10.2, 4.5 Hz, 1H), 4.59-4.44 (m, 1H), 4.17-4.03 (m, 2 H), 3.81 (s, 3 H), 3.75-3.55 (m, 1H), 2.97-2.77 (m, 1H), 2.73-2.55 (m, 1H), 2.51 (s, 0.5 H), 2.46 (s, 2.5 H), 2.25 (s, 3 H), 2.15-1.87 (m, 2 H), 1.51 (d, *J* = 7.3 Hz, 3 H), 1.30-1.18 (m, 1H); ¹³C-NMR (Gemisch aus 2 Isomeren, 50 MHz, CDCl₃): δ 144.7 (s), 142.9 (s), 132.5 und 131.7 (s), 130.4 und 129.0 (s), 129.3 (d), 127.8 und 126.8 (s), 127.5 (d), 114.3 und 114.0 (d), 88.7 und 88.4 (d), 62.2 und 61.8 (d), 59.3 und 58.8 (d), 56.0 und 55.8 (q), 49.4 und 48.4 (s), 44.3 (t), 41.5 (q), 31.7 (t), 29.9 (t), 19.7 und 19.2 (q), 18.8 (q)

### Beispiel 117

### [4aS-(4aa,6a,8aR*)]-4a,5,9,10,11,12-Hexahydro-3-methoxy-11,12-dimethyl-6H-benzofuro[3a,3,2-ef][2]benzazepin-6-ol, 12-Methylepigalanthamin (Pi-22)

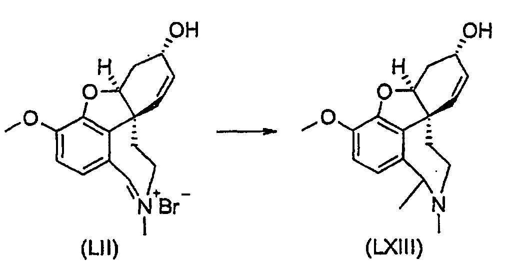

| | |
|---|---|
| 300 mg (0.82 mmol) | Epigalanthaminiumbromid (LII) |
| 1.00 ml (3.00 mmol) = 3.70 eq. | Methylmagnesiumiodid (3M in Diethylether) |

Das Grignardreagens wurde über 30 min zugegeben, anschließend 5 ml Ether zugefügt. Nach 20 min wurden weitere 15 ml Ether zugegeben und nach 3 h hydrolysiert.

Die Reinigung erfolgte säulenchromatographisch (CHCl₃ : MeOH = 9:1).
Ausbeute: 60 mg (0.20 mmol = 24% d. Th.)
C₁₈H₂₃NO₃ [301.39]
- DC:: R_{f} = 0.78 (CHCl₃ : MeOH = 9:1 + 1% NH₄OH)
¹H-NMR (200 MHz, CDCl₃): δ 6.65 (d, J = 8.3 Hz, 1H), 6.53 (d, *J* = 8.3 H, 1H), 6.04 (d, *J* = 10.4 Hz, 1 H), 5.76 (d, *J* = 10.4 Hz, 1H), 4.70-4.57 (m, 1H), 4.54 (bs, 1H), 3.93-3.82 (m, 1H), 3.81 (s, 3 H), 3.61 (t, *J* = 13.6 Hz, 1H), 2.94-2.67 (m, 3 H), 2.41 (s, 3 H), 2.20 (td, *J* = 13.2, 2.4 Hz, 1H), 1.69 (ddd, *J* = 13.6, 10.6, 2.0 Hz, 1H), 1.52 (d, *J* = 7.3 Hz, 3 H), 1.59-1.44 (m, 1H); ¹³C-NMR (50 MHz, CDCl₃): δ 147.0 (s), 143.2 (s), 134.5 (s), 131.3 (d), 131.1 (s), 128.9 (d), 121.5 (d), 111.2 (d), 88.6 (d), 64.1 (d), 62.7 (d), 55.6 (q), 48.6 (s), 44.4 (t), 41.2 (q), 32.0 (t + t), 21.8 (q)

### Beispiel 118

### [4aS-(4αa,6β,8aR*)]-4a,5,9,10-Tetrahydro-6-hydroxy-3-methoxy-11-methyl-6H-benzofuro[3a,3,2-ef][2]benzazepin-12(11H)-on (MH-128)

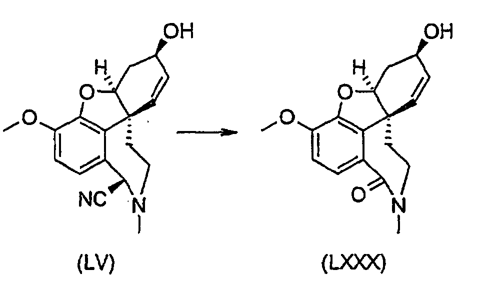

| | |
|---|---|
| 200 mg (0.64 mmol) | Galanthamin-12-carbonitril (LV) |
| 0.64 ml (0,64 mmol) | Natriumtrimethylsilanolat (1 M in CH₂Cl₂) |
| 5 ml | absolutes Tetrahydrofuran |

Die Edukte wurden unter N₂-Atmosphäre bei Raumtemperatur 72 h gerührt, wobei sich ein Niederschlag bildete, der abgesaugt, mit Tetrahydrofuran gewaschen und getrocknet wurde.
Ausbeute: 177 mg (0.59 mmol = 92% d. Th.) hellgelbes Pulver
C₁₇H₁₉NO₄ [301.35]
- DC:: R_{f} = 0.65 (CHCl₃ : MeOH = 9:1)
- M.p.:: 251-255°C
¹H-NMR (200 MHz, CDCl₃): δ 7.49 (d, *J* = 8.5 Hz, 1H), 6.89 (d, *J* = 8.5 Hz, 1H), 5.87 (dd, *J* = 9.8, 5.3 Hz, 1H), 5.53 (d, *J* = 9.8 Hz, 1H), 4.74 (bs, 1H), 4.13 (dt, *J* = 10.1, 4.8 Hz, 1H), 3.91 (s, 3 H), 3.80 (dt, *J* = 14.1, 2.1 Hz, 1H), 3.25-3.16 (m, 1H), 3.19 (s, 3 H), 2.71 (dt, *J* = 15.7, 1.7 Hz, 1H), 2.31 (dt, J = 14.1, 3.9 Hz, 1H), 2.06 (ddd, J = 15.7, 5.0, 2.3 Hz, 1H), 1.83 (dt, *J* = 14.6, 2.5 Hz, 1H); ¹³C-NMR (50 MHz, CDCl₃): 8 168.3 (s), 146.9 (s), 145.1 (s), 131.7 (s), 131.6 (d), 125.2 (d), 124.4 (d), 123.4 (s), 111.9 (d), 89.2 (d), 61.0 (d), 55.8 (q), 49.5 (t), 48.0 (s), 38.3 (t), 34. 9 (q), 29.3 (t)

### Beispiel 119

### [4aS-(4aα,6β,8aR*)]-4a,5,9,10-Tetrahydro-3-methoxy-6H-benzofuro[3α,3,2-ef][2]benzazepin-6-ol, 11-oxid (MH-142)

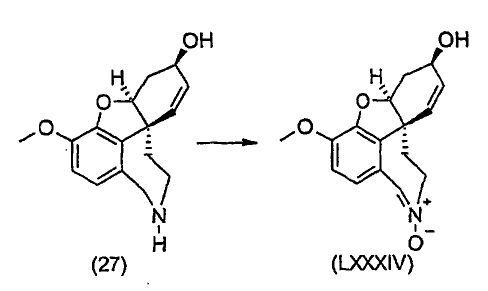

| | |
|---|---|
| 4.25 g (15.55 mmol) | Demethylgalanthamin (27) |
| 85 mg (0.77 mmol) = 5% | Selendioxid |
| 70 ml | 10% wäßrige H₂O₂-Lösung (35%) in Aceton (Oxidationslösung) |

Demethylgalanthamin wurde unter Ausschluß von Luftfeuchtigkeit in der Oxidationslösung gelöst und auf 0°C gekühlt. Dann wurde SeO₂ zugegeben und zuerst 20 min bei 0°C und dann 4 h bei Raumtemperatur gerührt, wobei ein weißer Niederschlag ausfiel, der abgesaugt, mit Aceton gewaschen und getrocknet wurde. Das Filtrat wurde mit Wasser versetzt, das Aceton im Vakuum abdestilliert und die verbleibende wäßrige Phase mit Methylenchlorid extrahiert. Die gesammelten organischen Phasen wurden mit gesättigter Kochsalzlösung gewaschen, über Natriumsulfat getrocknet, filtriert und das Lösungsmittel abgezogen. Der ölige Rückstand wurde in Aceton aufgenommen, wobei ein Niederschlag ausfiel, der als zweite Fraktion gewonnen werden konnte. Durch wiederholtes Einengen des Filtrats und Aufnehmen in Aceton konnten weitere Fraktionen gewonnen werden.
Ausbeute: 3.53 g (12.29 mmol = 79% d. Th.) weißes Pulver
C₁₆H₁₇NO₄ [287.31]
- DC:: R_{f} = 0.42 (CHCl₃ : MeOH = 9:1 + 1% konz. NH₄OH)
- M.p.:: 232-233°C (CHCl₃); ab 215°C Abgabe einer Flüssigkeit
C₁₆H₁₇NO₄ x 0.2 H₂O [290.91]

| | %C | %H | %N |
|---|---|---|---|
| berechnet | 66.06 | 6.03 | 4.81 |
| gefunden | 66.11 | 6.05 | 4.73 |

¹H-NMR (200 MHz, DMSO-d₆): δ 7.82 (s, 1H), 6.90 (s, 2 H), 5.81 (dd, *J* = 10.1, 4.4 Hz, 1H), 5.54 (d, *J* = 10.1 Hz, 1H), 4.64 (bs, 1H), 4.36 (d, J = 5.5 Hz, 1H), 4.14-4.02 (m, 2 H), 3.79 (s, 3 H), 2.39-1.99 (m, 4 H); ¹³C-NMR [50 MHz, DMSO-d₆): δ 146.1 (s), 144.6 (s), 134.6 (d), 131.8 (s), 128.3 (d), 127.6 (d), 122.4 (d), 118.3 (s), 112.6 (d), 86.7 (d), 61.8 (d), 59.1 (t), 55.7 (q), 45.3 (s), 34. 2 (t), 29.7 (t)

### Beispiel 119A :

### [4aS-(4aα,6β,8aR*)]-4a,5,9,10,13,14a-Hexahydro-6-hydroxy-3-methoxy-6H,14Hbenzofuro[3a,3,2-ef]isoxazolo[3,2-a][2]benzazepin-13(bzw. 14)-carbonsäuremethylester (MH-143)

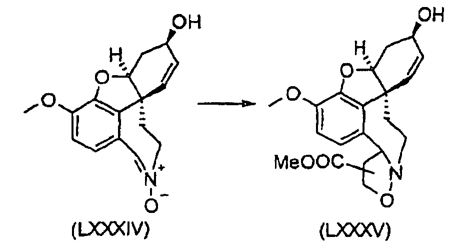

| | |
|---|---|
| 175 mg (0.61 mmol) | Golanthaminnitron (LXXXIV) |
| 0.05 ml (0.61 mmol) | Acrylsäuremethylester |
| 6 ml | absolutes Toluol |

Die Reagenzien wurden 48 h unter Argon-Atmosphäre auf Rückfluß erhitzt, dann das Lösungsmittel abgezogen. Der Rückstand wurde säulenchromatographisch gereinigt (CHCl₃ : MeOH = 9:1 + 1% konz. NH₄OH).
Ausbeute: 225 mg (0.60 mmol = 99% d. Th.) hellbraunes, glasartig erstarrendes Öl
C₂₀H₂₃NO₆ [373.40]
- DC:: R_{f} = 0.74 (CHCl₃ : MeOH = 9:1 + 1% konz. NH₄OH)
C20H23NO6 x 0.5 H₂O [382.40]

| | %C | % H | %N |
|---|---|---|---|
| berechnet | 62.82 | 6.33 | 3.66 |
| gefunden | 62.88 | 6.17 | 3.65 |

Gemisch aus Stereo- und Regioisomeren. Die genauere Behandlung der Spektren findet sich in Kapitel 2.2, Strukturaufklärungen

### Beispiel 120

### [4aS-(4aα,6β,8aR*,14aS*)]-4a,5,9,10-Tetrahydro-6-hydroxy-3-methoxy-6H,14aHbenzofuro[3a,3,2-ef]isoxazolo[3,2-a][2]benzazepin-14-carbonsäure, methylester (MH - 145)

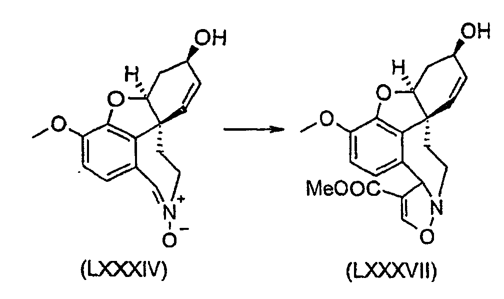

| | |
|---|---|
| 200 mg (0.70 mmol) | Galanthaminnitron (LXXXIV) |
| 0.06 ml (0.70 mmol) | Acetylencarbonsäuremethylester (Propiolsäuremethylester) |
| 5 ml | absolutes Toluol |

Die Reagenzien wurden 10 min unter Argon-Atmosphäre auf Rückfluß erhitzt, wobei sich die Lösung schon beim Aufwärmen orange färbte, dann das Lösungsmittel abgezogen. Der Rückstand wurde säulenchromatographisch gereinigt (CHCl₃ : MeOH = 9:1). Der ölige Rückstand wurde aus Ethanol auskristallisiert, wobei gelbe Nadeln gewonnen werden konnten.
Ausbeute: 261 mg (0.70 mmol = 100% d. Th.) hellgelbe Nadeln
C₂₀H₂₁NO₆ [371.39]
- DC:: R_{f} = 0.73 (CHCl₃ : MeOH = 9:1)
- M.p.:: 151-154°C

| | %C | %H | %N |
|---|---|---|---|
| berechnet | 64.68 | 5.70 | 3.77 |
| gefunden | 64.59 | 5.89 | 3.67 |

¹H-NMR (200 MHz, CDCl₃): δ 7.54 (s, 1H), 6.96 (d, *J* = 8.4 Hz, 1H), 6.74 (d, *J* = 8.4 Hz, 1H), 5.96-5.77 (m, 2 H), 5.67 (s, 1H), 4.55 (bs, 1H), 4.10 (bs, 1H), 3.85 (s, 3 H), 3.68 (s, 3 H), 3.59 (ddd, *J* = 14.3, 6.8, 3.8 Hz, 1H), 3.30 (ddd, *J* = 12.9, 9.3, 3.4 Hz, 1H), 2.64 (dd, J = 15.7, 3.7 Hz, 1H), 2.15 (td, *J* = 7.7, 3.4 Hz, 1H), 2.01 (ddd, *J* = 15.6, 5.3, 1.9 Hz, 1H), 1.54 (ddd, J = 15.6, 6.7,3.5 Hz, 2 H); ¹³C-NMR (50 MHz, CDCl₃): δ 163.9 (s), 154.6 (d), 146.8 (s), 145.1 (s), 133.3 (s), 130.3 (d), 126.9 (d), 125.3 (s), 123.3 (d), 111.3 (d), 109.9 (s), 89.1 (d), 68.7 (d), 61.4 (d), 55.8 (q), 52.4 (t), 51.4 (q), 47.2 (s), 29.2 (t), 28.0 (t)

### Beispiel 121

### [4aS-(4aα,6β,8aR*)]-4α,5,9,10,13,14a-Hexahydro-6-hydroxy-3-methoxy-6H,14Hbenzofuro[3a,3,2-ef]isoxazolo[3,2-a][2]benzazepin-13(bzw. 14)-carbonitril (MH-146)

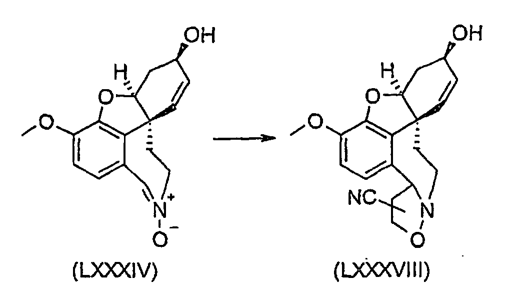

| | |
|---|---|
| 200 mg (0.70 mmol) | Galanthaminnitron (LXXXIV) |
| 0.05 ml (0.70 mmol) | Acrylnitril |
| 5 ml | absolutes Toluol |

Die Reagenzien wurden 2 h unter Argon-Atmosphäre auf Rückfluß erhitzt, dann das Lösungsmittel abgezogen. Der Rückstand wurde säulenchromatographisch gereinigt (CHCl₃ : MeOH = 9:1).
Ausbeute: 230 mg (0.68 mmol = 97% d. Th.) hellgelbes Öl
C₁₉H₂₀N₂O₄ [340.38]
- DC:: R_{f} = 0.74 (CHCl₃ : MeOH = 9:1)
C₁₉H₂₀N₂O₄ x 0.2 H₂O [343.98]

| | %C | %H | %N |
|---|---|---|---|
| berechnet | 66.34 | 5.98 | 8.14 |
| gefunden | 66.22 | 6.03 | 7.86 |

Gemisch aus 4 Stereo- und Regioisomeren. Die genauere Behandlung der Spektren findet sich in Kapitel 2.2, Strukturaufklärungen

### Beispiel 122

### [4aS-(4aα,6β,8aR*,14aS*)]-4a,5,9,10,13,14a-Hexahydro-6-hydroxy-3-methoxy-6H,14aH-benzofuro[3a,3,2-ef]isoxazolo[3,2-a][2]benzazepin-6,13-diol, 13-acetat (MH-153)

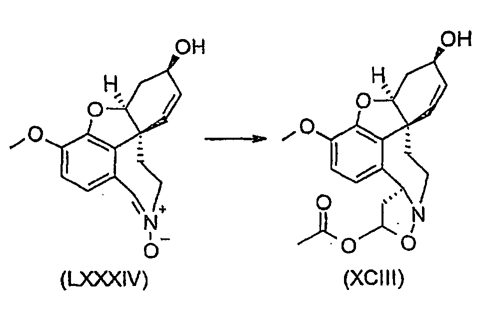

| | |
|---|---|
| 200 mg (0.70 mmol) | Galanthaminnitron (LXXXIV) |
| 0.24 ml (2.10 mmol) = 4 eq. | Essigsäurevinylester |
| 5 ml | absolutes Toluol |

Die Reagenzien wurden 4 Tage unter N₂-Atmosphäre auf Rückfluß erhitzt, wobei jeden Tag 1 eq. Essigsäurevinylester zugegeben wurde. Dann wurde das Lösungsmittel abgezogen und der Rückstand säulenchromatographisch gereinigt (CHCI₃ : MeOH = 9:1). Das gereinigte Öl wurde aus Methanol kristallisiert.
Ausbeute: 256 mg (0.69 mmol = 98% d. Th.) beige Kristalle
C₂₀H₂₃NO₆ [373.41]
- DC:: R_{f} = 0.70 (CHCl₃ : MeOH = 9:1)
- M.p.:: 132-134°C
C₂₀H₂₃NO₆ x 0.6 H₂O [384.21]

| | %C | %H | %N |
|---|---|---|---|
| berechnet | 62.52 | 6.35 | 3.65 |
| gefunden | 62.59 | 6.12 | 3.61 |

¹H-NMR (Gemisch aus 2 Isomeren, 200 MHz, CDCl₃): δ 6.76 und 6.73 (d, J = 8.3 Hz, 1H), 6.68 und 6.60 (d, J = 7.7 Hz, 1H), 6.36 (d, J = 4.1 Hz, 1H), 6.30 (d, J = 10.6 Hz, 1H), 6.08-5.91 (m, 1H), 4.60 und 4.50 (bs, 1H), 4.32 (dd, J = 11.3, 5.6 Hz, 1H), 4.13 (bs, 1H), 3.84 (s, 3 H), 3.80 (dd, J = 19.1, 9.8 Hz, 1H), 3.22 (ddd, J = 10.0, 6.8, 2.8 Hz, 1H), 2.92 (dd, J = 12.3, 5.8 Hz, 1H), 2.78-2.57 (m, 2 H), 2.09 (s, 3 H), 2.07-1.79 (m, 2 H); ¹³C-NMR (Gemisch aus 2 Isomeren, 50 MHz, CDCl₃): δ 169.6 (s), 145.8 (s), 143.8 (s), 134.1 (s), 129.8 (d), 128.0 und 127.7 (d), 127.0 (s), 118.7 und 118.5 (d), 111.4 und 110.9 (d), 95.3 und 94.5 (d), 88.7 und 88.2 (d), 61.4 (d + d), 55.7 und 55.5 (q), 54.5 (t), 47.3 (s), 41.7 (t), 29.7 (t), 29.3 (t), 21.0 und 20.9 (q)

### Beispiel 123

### [4aS-(4aα,6β,8aR*,14aS*)]-4a,5,9,10-Tetrahydro-6-hydroxy-3-methoxy-6H,14aH-benzofuro[3a,3,2-ef]isoxazolo[3,2-a][2]benzazepin-14-carbonitril (MH-159)

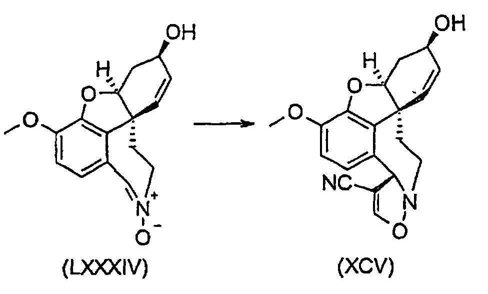

| | |
|---|---|
| 500 mg (1.74 mmol) | Galanthaminnitron (LXXXIV) |
| 90 mg (1.74 mmol) | Acetylencarbonitril |
| 10 ml | absolutes Toluol |

Die Reagenzien wurden 7 Tage unter Argon-Atmosphäre bei Raumtemperatur gerührt, wobei sich die Lösung gelb färbte, dann das Lösungsmittel abgezogen. Der Rückstand wurde aus Methanol kristallisiert, abgesaugt und mit Methanol gewaschen. Das Filtrat wurde eingeengt und aus dem Rückstand säulenchromatographisch (CHCl₃ : MeOH = 9:1) eine 2. Produktfraktion gewonnen.
Ausbeute: 570 mg (1.68 mmol = 97% d. Th.) farblose Kristalle
C₁₉H₁₈N₂O₄ [338.37]
- DC:: R_{f} = 0.60 (CHCl₃ : MeOH = 9:1)
- M.p.:: 137-139°C

| | %C | %H | %N |
|---|---|---|---|
| berechnet | 67.45 | 5.36 | 8.28 |
| gefunden | 67.17 | 5.41 | 8.19 |

¹H-NMR (200 MHz, CDCl₃): δ 7.09 (d, *J* = 8.6Hz, 1H), 6.76 (d, *J* = 8.6 Hz, 1H), 5.98 (S, 2H), 5.54 (s, 1 H), 4.52 (bs, 1H), 4.11 (bs, 1H), 3.83 (s, 3 H), 3.75-3.59 (m, 1H), 3.42-3.25 (m, 1H), 2.64 (dd, J = 15.9, 3.2 Hz, 1H), 2.44 (d, *J* = 11.5 Hz, 1H), 2.11-1.94 (m, 2 H), 1.71-1.52 (m, 1H); ¹³C-NMR (50 MHz, CDCl₃): δ 156.5 (d), 147.1 (s), 145.5 (s), 132.7 (s), 129.6 (d), 127.9 (d), 123.8 (s), 120.8 (d), 114.0 (s), 111.7 (d), 88.9 (d), 88.6 (s), 68.4 (d), 61.3 (d), 55.9 (q), 52.5 (t), 47.2 (s), 29.3 (t), 28.3 (t)

### Beispiel 125:

### Schritt 1:

### 1-Brom-5-methoxy-2-(2-methoxyethen-1-yl)-4-(1-methylethoxy)benzol

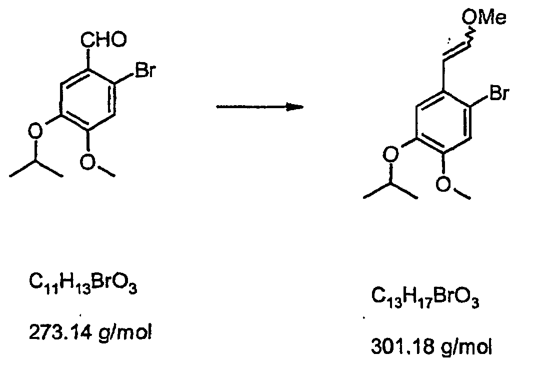

Zu einer Suspension von (Methoxymethyl)triphenylphosphoniumchlorid (50.0 g, 152 mmol) in absolutem THF (330 ml) wird unter Eiskühlung Kaliumtertiärbutylat (20.5 g, 183 mmol) zugegeben. Nach 15 min wird portionweise 2-Brom-4-methoxy-5-(1-methylethoxy)benzaldehyd (33.1 g, 121 mmol) zugegeben.

Nach 15 min wird der nach Entfernen des Lösungsmittels am Rotationsverdampfer erhaltene Rückstand zwischen Wasser (300 ml) und Ether (300 ml) verteilt. Die organische Phase wird getrocknet (Natriumsulfat), filtriert und der nach Eindampfen erhaltene Rückstand (37.3 g) mittels MPLC (Petrolether:Essigsäureethylester = 2 : 1, Fluß 70 ml/min) gereinigt. Auf diese Weise erhält man das Produkt in Form farbloser Kristalle (32.5 g, 85%).
Schmelzpunkt: 43-45°C
- DC:: Petrolether : Essigsäureethylester = 2 : 1 Rf = 0.75
¹H: NMR (CDCl₃) δ 7.00 (s, 1H); 6.90 (s, 1H), 6.83 (d, J = 12.7 Hz, 1 Hₜᵣₐₙₛ), 6.13 (d, J = 7.6 Hz, 1H_{cis}), 5.98 (d, J = 12.7 Hz, 1 Hₜᵣₐₙₛ), 5.50 (d, J = 7.6 Hz, 1 H_{cis}), 4.49 (Septett, J = 6.4 Hz, 1H), 3.81 (s, 3H), 3.74 (s, 3Hₜᵣₐₙₛ), 3.70 (s, 3H_{cis}), 1.35 (d, J = 6.4 Hz, 6H);
¹³C-NMR (CDCl₃) δ 149.2 und 149.6 (s), 147.6 und 148.9 (s), 146.0 und 146.7 (d), 127.5 und 128.4 (s), 115.7 und 117.2 (d), 113.6 und 116.2 (d), 113.3 und 113.7 (s), 103.8 und 104.2 (d), 71.5 und 71.9 (d), 56.1 und 56.4 (q), 56.0 und 60.6 (q), 21.9 und 22.0 (q)

### Schritt 2:

### 2-Brom-4-methoxy-5-(1-methylethoxy)benzolacetaldehyd

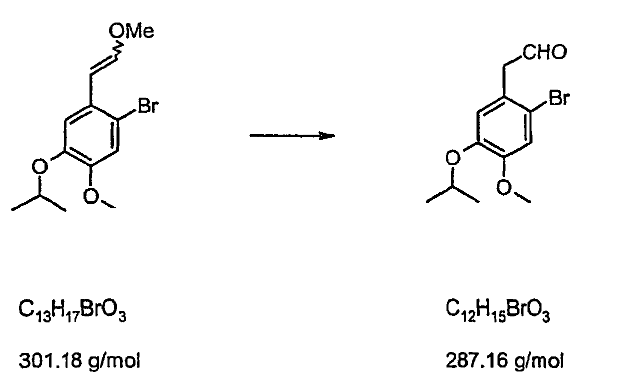

1-Brom-5-methoxy-2-(2-methoxyethen-1-yl)-4-(1-methylethoxy)benzol (20.0 g, 66.4 mmol) werden in Tetrahydrofuran (250 ml) /2N Hcl (10 ml) drei Stunden bei Siedetemperatur gerührt.

Nach Abziehen des Lösungsmittels am Rotationsverdampfer wird der Rückstand zwischen Wasser (200 ml) und Ether (200 ml) verteilt, die wäßrige Phase mit Ether (3 × 100 ml) extrahiert, die vereinigten organischen Phasen mit Wasser (4 × 150 ml), gesättigter Natriumhydrogencarbonatlösung (2 × 200 ml) und gesättigter Kochsalzlösung (1 × 200 ml) gewaschen, getrocknet (Natriumsulfat/Aktivkohle) und filtriert. Nach Eindampfen erhält man das Produkt als gelbes Öl (18.7 g, 98%).
- DC:: Petrolether : Essigsäureethylester = 4 : 1 Rf = 0.77
¹H: NMR (CDCl₃) δ 9.71 (t, J = 1.71 Hz, 1H), 7.06 [s, 1H), 6.80 (s, 1H), 4.49 (Septett, J = 6.4 Hz, 1H), 3.90 (s, 3H), 3.73 (d, J = 1.71 Hz, 2H), 1.35 (d, J = 6.4 Hz, 6H);
¹³C NMR (CDCl₃) δ 191.8 (d), 146.6 (s), 145.1 (s), 125.4 (s), 118.1 (s), 115.8 (d), 113.7 (d), 71.5 (d), 56.4 (q), 49.8 (t), 21.8 (q)

### Schritt 3:

### 2-Brom-4-methoxy-5-(1-methylethoxy)benzolethanol

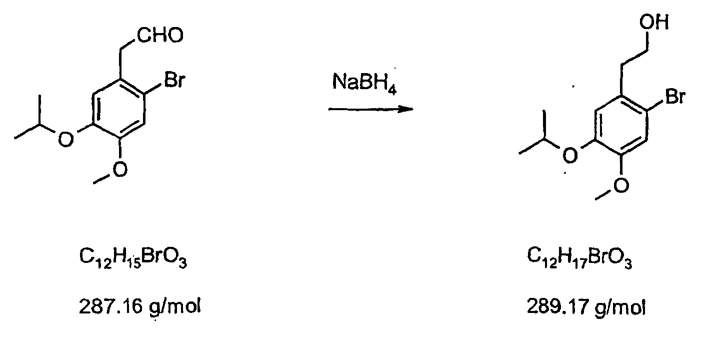

2-Brom-5-(1-methylethoxy)-4-methoxybenzolacetaldehyd (2.60 g, 9.05 mmol) werden bei 15°C innerhalb von 30 min zu einer Suspension von Natriumborhydrid (0.341 g, 9.05 nnol) in absolutem Ethanol (40 ml) zugegeben und zwei Stunden bei dieser Temperatur gerührt.

Der Ethanol wird am Rotationsverdampfer entfernt und der Rückstand zwischen gesättigter Natriumhydrogencarbonatlösung (200 ml) und Ether (200 ml) verteilt. Die wäßrige Phase wird mit Ether (3 × 50 ml) extrahiert. Die vereinigten organischen Phasen werden mit Wasser (3 × 200 ml) und gesättigter Kochsalzlösung (1 × 200 ml) gewaschen, getrocknet (Natriumsulfat/Aktivkohle) getrocknet und filtriert. Nach Eindampfen erhält man das Produkt in Form farbloser Kristalle (2.60 g, 99%).
- DC:: PE : EE = 9 : 1, 0.25
¹H NMR (CDCl₃) δ 6.98 (s, 1H), 6.80 (s, 1H), 4.47 (Septett, J = 6.3 Hz, 1H), 3.82 (t, J = 7.0 Hz, 2H), 3.80 (s, 3H), 2.90 (t, J = 7.0 Hz, 2H), 1.32 (d, J = 7.3 Hz, 6H);
¹³C NMR (CDCl₃) δ 149.7 (s), 146.4 (s), 129.6 (s), 118.5 (d), 116.3 (d), 114.8 (s), 71.8 (d), 62.2 (t), 56.1 (q), 38.8 (t), 21.9 (q)
MT-163 JOS 1682
C₁₂H₁₇BrO₃

| | | |
|---|---|---|
| Berechnet | C, 49.84; | H, 5.93 |
| Gefunden | C, 49.69; | H, 5.79 |

### Schritt 4:

### 1-Brom-2-(2-iodethyl)-5-methoxy-4-(1-methylethoxy)benzol

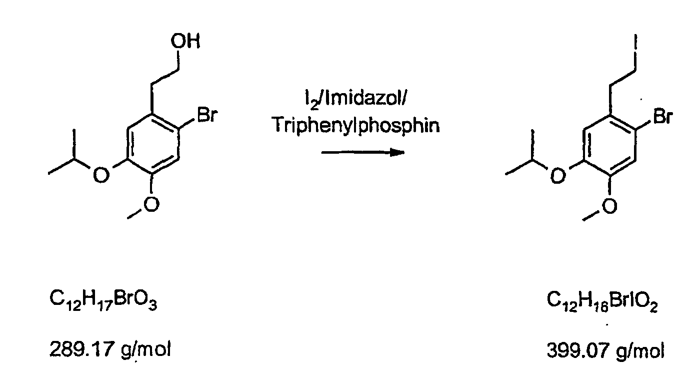

Triphenylphosphin (24.7 g, 94.0 mmol), Imidazol (12.8 g, 188.0 mmol) und Iod (23.06 g, 90.9 mmol) werden in absolutem CH₂Cl₂ (150 ml) eine Stunde bei 15°C gerührt.

2-Brom-5-(1-methylethoxy)-4-methoxybenzolethanol (18.0 g, 62.2 mmol) in absolutem CH₂Cl₂ (100 ml) werden bei dieser Temperatur innerhalb von 10 min zugetropft und das Gemisch zwei Stunden bei Raumtemperatur gerührt.

Das Gemisch wird filtriert und das Filtrat mit Wasser (1 × 200 ml) gewaschen. Die wäßrige Phase wird mit CH₂Cl₂ (2 × 50 ml) extrahiert und die vereinigten organischen Phasen mit Natriumthiosulfatlösung (1 × 200 ml), Wasser (1 × 200 ml), Kupfersulfatlösung (1 × 200 ml), Wasser (1 × 200 ml) und gesättigter Kochsalzlösung (1 × 200 ml) gewaschen, getrocknet (Natriumsulfat/Aktivkohle), filtriert und das Lösungsmittel am Rotationsverdampfer entfernt.

Nach Reinigung durch Säulenchromatographie (1000 g Kieselgel/Petrolether :
Essigsäureethylester = 96 : 4) erhält man das Produkt in Form farbloser Nadeln (19.0 g, 77%).
¹H NMR (CDCl₃) δ 7.00 (s, 1H), 6.77 (s, 1H), 4.49 (Septett, J = 6.3 Hz, 1H), 3.81 (s, 3H), 3.39-3.24 (m, 2H), 3.24-3.09 (m, 2H), 1.36 (d, J = 7.3 Hz, 6H);
¹³C NMR (CDCl₃): δ 150.0 (s), 146.5 (s), 131.7 (s), 118.0 (d), 116.3 (d), 114.3 (s), 71.8 (d), 56.1 (q), 40.0 (t), 22.0 (q), 4.2 (t)
MT-164 JOS 1704
C₁₂H₁₆BrlO₂

| | | |
|---|---|---|
| Berechnet | C, 36.12; | H, 4.04 |
| Gefunden | C, 36.38; | H, 3.91 |

### Schritt 5:

### 2-[2-[2-Brom-4-methoxy-5-(1-methylethoxy)phenyl]ethyl]propandisäuredimethylester

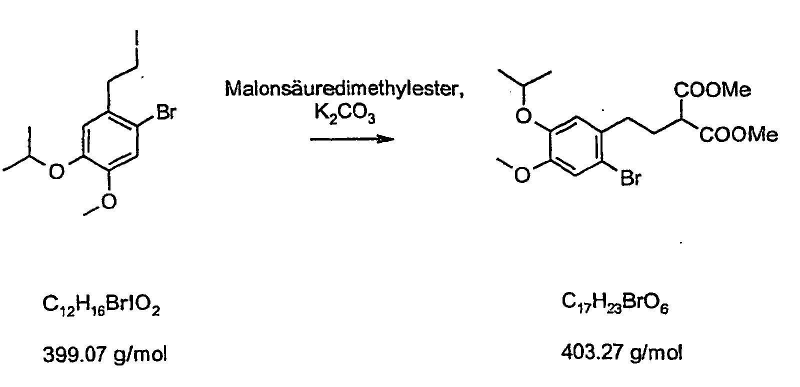

1-Brom-2-(2-iodethyl)-4-(1-methylethoxy)-5-methoxybenzol (18.0 g, 45.1 mmol), Kaliumcarbonat (32.0 g, 321 mmol, wasserfrei, frisch vermahlen) und Malonsäuredimethylester (50.0 g, 378 mmol) werden in absolutem DMF (200 ml) 12 Stunden bei 80°C gerührt.

Das Gemisch wird filtriert, das Lösungsmittel am Rotationsverdampfer entfernt und der Rückstand zwischen Wasser (300 ml) und Ether (300 ml) verteilt. Die wäßrige Phase wird mit Ether (3 × 50 ml) extrahiert, die vereinigten organischen Phasen mit Wasser (4 × 150 ml) und gesättigter Kochsalzlösung (1 × 200 ml) gewaschen, getrocknet (Natriumsulfat/Aktivkohle), filtriert, der nach Abdestillieren des Lösungsmittels erhaltene Rückstand durch Destillation von überschüssigem Malonester befreit (160°C/15 mbar) und mittels Kugelrohrdestillation (170°C/0,06 mbar) gereinigt, wodurch man das Produkt als farbloses Öl erhält (18.9 g, 72%).
¹H: NMR (CDCl₃) δ 6.99 (s, 1H), 6.73 (s, 1H), 4.49 (Septett, J = 6.3 Hz, 1H), 3.81 (s, 3H), 3.76 (s, 6H), 3.39 (t, J = 7.9 Hz, 1H), 2.68 (t, J = 7.9 Hz, 2H), 2.18 (q, J = 7.9 Hz, 2H), 1.34 d, J = 6.3 Hz, 6H);
¹³C NMR (CDCl₃) δ 169.6 (s), 149.7 (s), 146.6 (s), 131.7 (s), 117.8 (d), 116.3 (d), 114.6 (s), 71.8 (d), 56.2 (q), 52.5 (q), 50.9 (d), 33.1 (t), 29.0 (t), 22.0 (q)
MT-165 JOS 1771
C₁₇H₂₃BrO₆

| | | |
|---|---|---|
| Berechnet | C, 50.63; | H, 5.75 |
| Gefunden | C, 50.87; | H, 5.62 |

### Schritt 6:

### 2-[2-[2-Brom-4-methoxy-5-(1-methylethoxy)phenyl]ethyl]-2-[4-(1-methylethoxy)phenylmethyl]propandisäuredimethylester

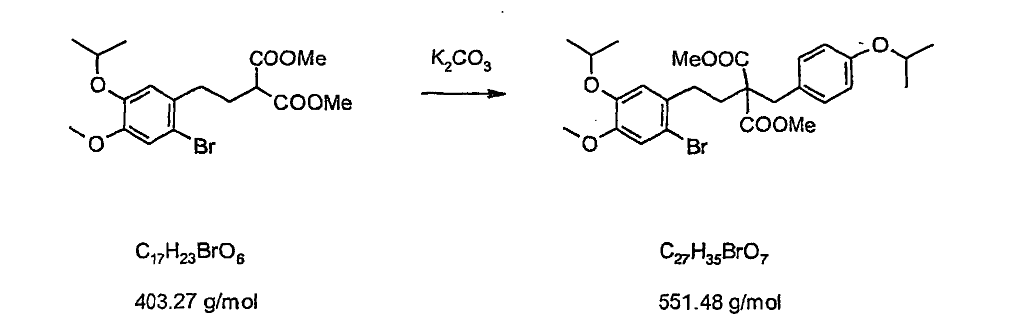

1-Brom-2-(2-iodethyl)-4-(1-methylethoxy)-5-methoxybenzol (18.0 g, 45.1 mmol), Kaliumcarbonat (32.0 g, 321 mmol, wasserfrei, frisch vermahlen) und (50.0 g, 378,4 mmol) Malonsäuredimethylester werden (200 ml) wasserfreiem DMF 12 Stunden bei 80°C gerührt.

Das Gemisch wird filtriert, das Lösungsmittel abgezogen und der Rückstand zwischen 300 ml Wasser und 300 ml Ether verteilt. Die wäßrige Phase wird dreimal mit je 50 ml extrahiert, die vereinigten organischen Phasen viermal mit je 150 ml Wasser und einmal mit 200 ml gesättigter Kochsalzlösung gewaschen, über Natriumsulfat/Aktivkohle getrocknet, filtriert und das Lösungsmittel abgezogen. Überschüssiger Malonsäuredimethylester wird durch Destillation (160°C/15 mbar) abgetrennt und der Rückstand mittels Kugelrohrdestillation (170°C(0,06 mbar) gereinigt, wodurch man das Produkt in Form eines farblosen Öls erhält (18.9 g, 72%).
MT-166 JOS 1694
C₂₇H₃₅BrO₇

| | | |
|---|---|---|
| Berechnet | C, 58.81; | H, 6.40 |
| Gefunden | C, 59.03; | H, 6.24 |

### Schritt 7:

### 4-[2-Brom-4-methoxy-5-(1-methytethoxy)]-α-[4-(1-methylethoxy)-phenylmethyl]benzolbutansäure

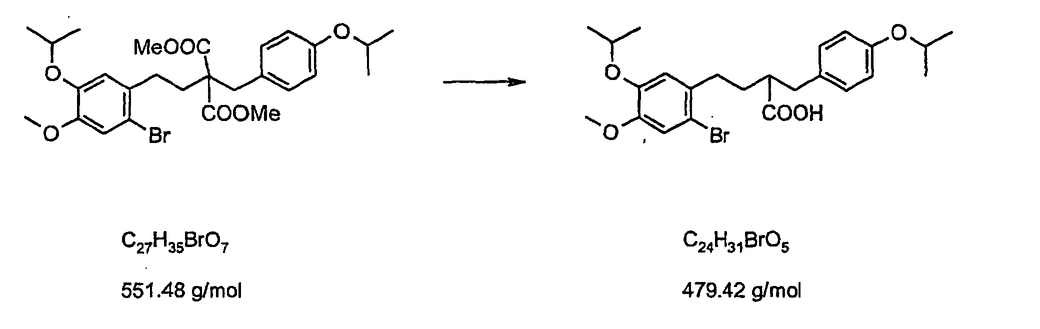

2-[2-[2-Brom-5-(1-methylethoxy)-4-methoxyphenyl]ethyl]-2-[4-(1-methylethoxy)phenylmethyl]propandisäuredimethylester (18.1 g, 32.8 mmol) und Kaliumhydroxid (17.5 g, 312 mmol) werden in einem Gemisch aus Ethanol (100 ml) und Wasser (20 ml) 12 Stunden bei Siedetemperatur gerührt.

Das Reaktionsgemisch wird mit konzentrierter Salzsäure bis auf einen pH von 1 angesäuert und eine Stunde unter Rückfluß gehalten.

Der nach Entfernen des Lösungsmittels verbliebene Rückstand wird zwischen Wasser (250 ml) und Ether (250 ml) verteilt. Die wäßrige Phase wird mit Ether (2 x 100 ml) extrahiert, die vereinigten, organischen Phasen mit Wasser neutralgewaschen, mit gesättigter Kochsalzlösung (150 ml) gewaschen und getrocknet (Natriumsulfat/Aktivkohle). Der nach Entfernen des Lösungsmittels verbliebene Rückstand wird im Kugelrohr 30 min bei 160°C am Hochvakuum decarboxyliert und anschließend bei 210°C/0.008 mbar destilliert. Auf diese Weise wird das Produkt in Form farbloser Kristalle (13.3 g, 84%) erhalten.
¹H: NMR (CDCl₃) δ 7.04 (d, J = 9.5 Hz, 2H), 6.99 (s, 1H), 6.80 (d, J = 9.5 Hz, 2H), 6.77 (s, 1H), 4.60-4.39 (m, 2H), 3.79 (s, 3H), 3.09-2.58 (m, 5H), 2.09-1.72 (m, 2H), 1.43-1.29 (m, 1H);
¹³C NMR (CDCl₃): 181.0 (s), 156.2 (s), 149.3 (s), 146.3 (s), 132.3 (s), 130.7 (s), 129.6 (d), 117.6 (d), 116.1 (d), 115.7 (d), 114.3 (s), 71.6 (d), 69.6 (d), 55.9 (q), 46.7 (d), 37.0 (t), 33.1 (t), 317 (t), 21.8 (q).

### Schritt 8:

### 4-[2-Brom-4-methoxy-5-(1-methy)ethoxy)]-α-[4-(1-methylethoxy)-phenylmethyl]benzolbutansäureamid

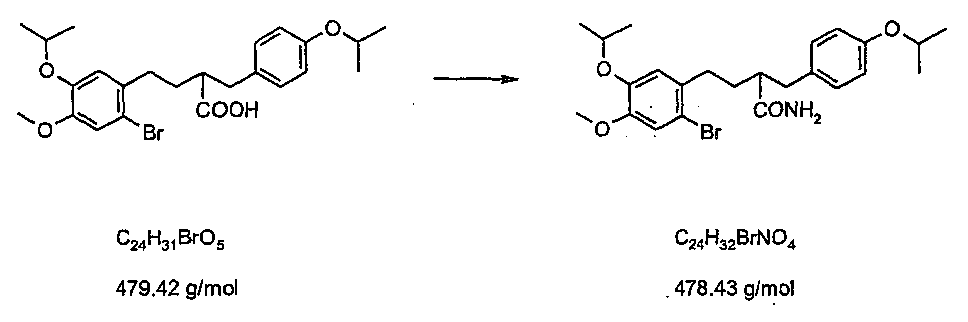

Zu 4-[2-Brom-4-methoxy-5-(1-methylethoxy)]-α-[4-(1-methylethoxy)-phenylmethyl]benzolbutansäure (24.0 g, 50.1 mmol) in absolutem CH₂Cl₂ (200 ml) wird Oxalsäuredichlorid (15 ml) bei 0°C innerhalb von 15 min zugetropft und das Gemisch zwei Stunden bei dieser Temperatur gerührt.

Das Lösungsmittel wird am Rotationsverdampfer entfernt, der Rückstand in absolutem THF (100 ml) aufgenommen und bei 0°C zwei Stunden lang Ammoniak eingeleitet. Das Gemisch wird eine Stunde bei 0°C gerührt und auf Wasser (1000 ml) gegossen.

Die ausgefallenen Kristalle werden abfiltriert und mit Wasser (4 × 500 ml) digeriert. Auf diese Art wird das Produkt in Form farbloser Kristalle (19.9 g, 83%) erhalten.
¹H NMR (CDCl₃) δ 7.04 (d, J = 9.5 Hz, 2H), 6.96 (s, 1H), 6.72 (d, J = 9.5 Hz, 2H), 6.70 (s, 1H), 6.00 (b,
1H), 5.55 (b, 1H), 4.60-4.30 (m, 2H), 3.77 (s, 3H), 2.96-2.52 (m, 4H), 2.51-2.28 (m, 1H), 2.03-1.60 (m, 2H), 1.36-1.20 (m, 12H);
¹³C NMR (CDCl₃): 1774. (s), 156.2 (s), 149.2 (s), 146.4 (s), 132.7 (s), 131.1 (d), 129.7 (d), 117.5 (s), 116.1 (d), 115.7 (d), 114.4 (d), 71.6 (d), 69.6 (d), 56.0 (q), 48.6 (d), 38.0 (t), 33.3 (t), 32.5 (t), 21.9 (q).
MT-168 JOS 1770
C₂₄H₃₂BrNO₄

| | | | |
|---|---|---|---|
| Berechnet | C, 60.25; | H, 6.74; | N, 2.93 |
| Gefunden | C, 60.15; | H, 6.55; | N, 2.77 |

### Schritt 9:

### 4-(2-Brom-5-hydroxy-4-methoxy)-α-(4-hydroxyphenylmethyl)-benzolbutansäureamid

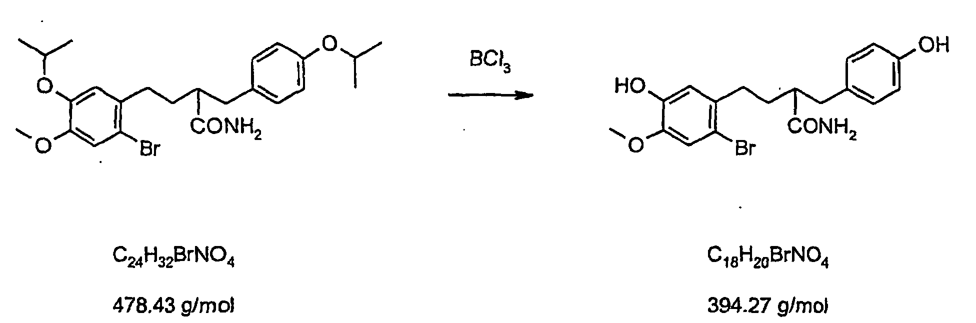

4-[2-Brom-4-methoxy-5-(1-methylethoxy)]-α-[4-(1-methylethoxy)-phenylmethyl]benzolbutansäureamid (10.0 g, 20.9 mmol) in absolutem CH₂Cl₂ (150 ml) wird bei -78°C Bortrichlorid (45 ml, 1.6 M in CH₂Cl₂) zugetropft und eine Stunde bei dieser Temperatur gerührt. Danach wird das Gemisch auf Raumtemperatur erwärmt und zwei Stunden gerührt.

Man versetzt mit Wasser (400 ml) und destilliert das organische Lösungsmittel am Rotationsverdampfer ab, wobei das Rohprodukt kristallin ausfällt, das abfiltriert und mit Wasser (6 × 200 ml) und Diisopropylether (2 × 40 ml) digeriert wird. Dabei erhält man das Produkt in Form farbloser Kristalle (7.11 g, 86%).
MT-171 JOS 1714
C₁₈H₂₀BrNO₄∗0.25 H₂O

| | | | |
|---|---|---|---|
| Berechnet | C, 54.22; | H, 5.18; | N, 3.51 |
| Gefunden | C, 54.05; | H, 4.95; | N, 3.54 |

### Schritt 10:

### 1-Brom-4a,5,9,10,11,12-hexahydro-3-methoxy-6-oxa-6H-benzo[a]cyclohepta[hl]benzofuran-10-carbonsäureamid (SPH-1478)

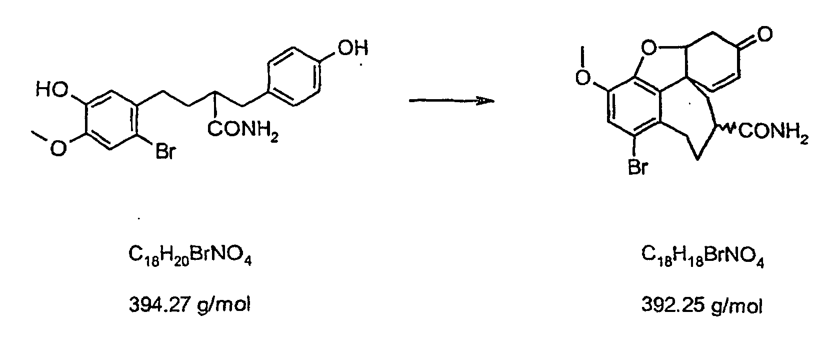

α-[[2-Brom-5-hydroxy-4-methoxyphenyl]methyl]-4-hydroxybenzolbutansäureamid (3.00 g, 7.61 mmol) werden in Chloroform (300 ml) suspendiert und mit einer Lösung von Kaliumhexacyanoferrat (III) (13.2 g, 40.0 mmol) in Kaliumcarbonatlösung (75 ml, zehnprozentig) versetzt.

Das Gemisch wird bei Raumtemperatur 40 min heftüg gerührt und über Hyflo filtriert. Die wäßrige Phase wird mit Chloroform (2 × 50 ml) extrahiert, die vereinigten organischen Phasen werden mit Wasser (2 × 200 ml) und gesättigter Kochsalzlösung (1 × 150 ml) gewaschen, getrocknet (Natriumsulfat/Kieselgel) und das nach Eindampfen des Lösungsmittels erhaltene Rohprodukt über Säulenchromatographie (50 g Kieselgel, Essigsäureethylester) gereinigt. Auf diese Weise erhält man das Produkt in Form farbloser Kristalle (179 mg, 6%).
DC: Essigsäureethylester, R_{f} = 0.6
¹H NMR (CDCl₃): δ 6.95 (s, 1H), 6.71 (dd, J = 12.1 Hz, J = 2.0 Hz, 1H), 6.02 (d, J = 12.1 Hz, 1H), 5.70 (b, 2H), 4.82 (s, 1H), 3.81 (s, 3H), 3.58 (dd, J = 16.5 Hz, J = 6.0 Hz, 1H), 3.13 (dd, J = 6.0 Hz, J = 16.5 Hz, 1H), 2.82 - 2.57 (m, 3H), 2.48 - 2.15 (m, 2H), 2.12 -1.62 (m, 2H);
¹³C NMR (DMSO-d₆): δ 196.7 (s), 178.2 (s), 147.3 (d), 145.6 (s), 143.9 (s), 132.5 (s), 131.4 (s), 127.5 (d), 117.0 (s), 114.8 (d), 88.3 (d), 53.5 (q), 49.7 (s), 43.7 (d), 40.9 (t), 39.7 (t), 38.0 (t), 32.1 (t);
¹³C NMR (CDCl₃): δ 193.8 (s), 176.7 (s), 146.7 (d), 143.5 (s), 143.2 (s), 131.0 (s), 129.9 (s), 127.7 (d), 116.5 (s), 115.1 (d), 87.6 (d), 56.1 (q), 49.1 (s), 44.2 (d), 39.4 (t), 37.0 (t), 32.0 (t), 31.7 (t)

### Beispiel 126:

### 1-Brom-4a,5,9,10,11,12-hexahydro-3-methoxy-6-hydroxy-6Hbenzo[a]cyclohepta[hi]benzofuran-10-carbonsäureamid (SPH-1479)

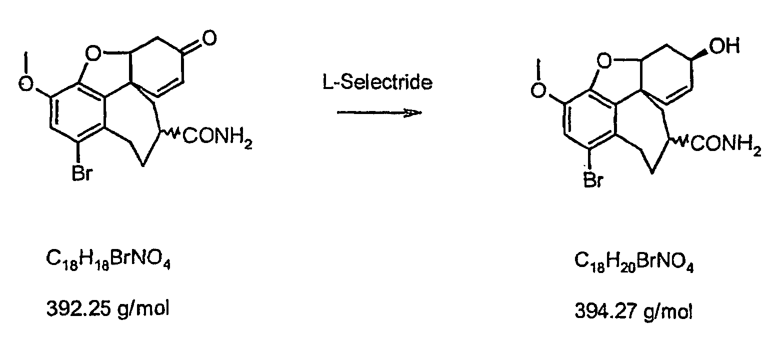

Zu einer Suspension von 1-Brom-4a,5,9,10,11,12-hexahydro-3-methoxy-6-oxa-6Hbenzo[a]cyclohepta[hi]benzofuran-10-carbonsäureamid (160 mg, 0.41 mmol) in absolutem THF (5 ml) wird bei 0°C L-Selektride ^{R} (2.0 ml, 2.0 mmol, 1 M in THF) innerhalb von 15 min zugegeben und das Gemisch 12 Stunden bei Raumtemperatur gerührt. Man hydrolysiert mit Wasser (2 ml) und verteilt zwischen Wasser (10 ml) und Essigsäureethylester (10 ml), extrahiert die wäßrige Phase mit Essigsäureethylester (3 × 5 ml), wäscht die vereinigten organischen Phasen mit 1 N Salzsäure (3 × 10 ml), Wasser (2 × 10 ml), gesättigter Natriumhydrogencarbonatlösung (1 × 10 ml) und gesättigter Kochsalzlösung (1 × 30 ml), trocknet (Natriumsulfat/Aktivkohle), filtriert und reinigt das nach Abdestillieren des Lösungsmittels erhaltene Rohprodukt mittels Säulenchromatographie (10 g Kieselgel, Essigsäureethylester). Auf diese Weise erhält man das Produkt in Form farbloser Kristalle (137 mg, 85%).
MT-194 JOS 1712
C₁₈H₂₀BrNO₄

| | | | |
|---|---|---|---|
| Berechnet | C, 54.84; | H, 5.11; | N, 3.55 |
| Gefunden | C, 54.55; | H, 5.22; | N, 3.34 |

DC: Essigsäureethylester, R_{f} = 0.5
¹H NMR (MeOH-d₄): δ 6.99 (s, 1H), 6.03 (d, J = 16.5 Hz, 1H), 5.94 (dd, J = 16.5 Hz, J = 5.9 Hz, 1H), 4.52 (s, 1H), 4.16 (s, 1H), 3.76 (s, 3H), 3.49 (dd, J = 19.8 Hz, J = 5.9 Hz, 1H), 2.90 (t, J = 17.5 Hz, 1H), 2.77 (t, J = 17.5 Hz, 1H), 2.46 (d, J = 17.6 Hz, 1H), 2.29 - 2.10 (m, 2H), 1.98 - 1.53 (m, 3H);
¹³C NMR (MeOH-d₄): δ 181.6 (s), 148.1 (s), 145.4 (s), 135.4 (s), 132.3 (s), 129.5 (d), 128.7 (d), 117.8 (d), 115.4 (s), 89.0 (d), 70.3 (d), 62.6 (d), 57.2 (q), 45.7 (s), 42.5 (t), 33.5 (t), 33.1 (t), 31.9 (t)

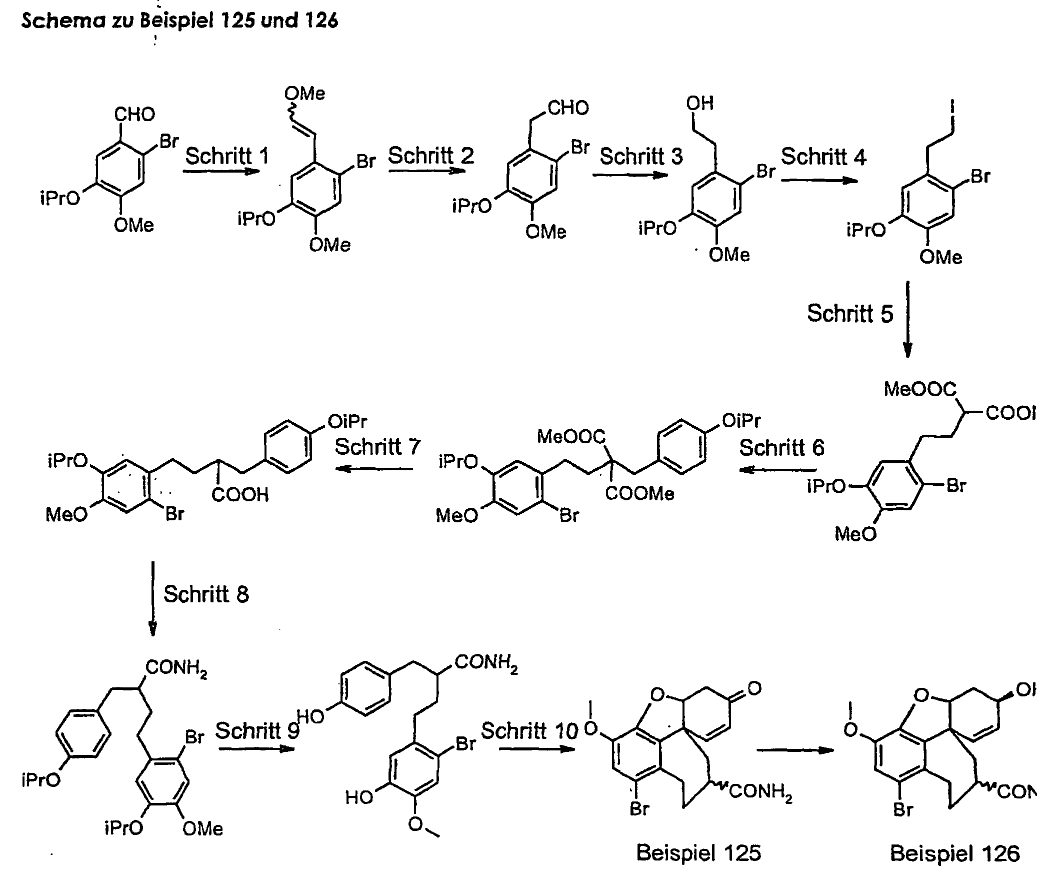

### Beispiel 127

### Stufe 1

### 5-(6-Acetyloxy-1-oxohexyl)-5,6-dihydro-4H-pyrrolo[3,2,1-ij]chinolin-2(1H)-on

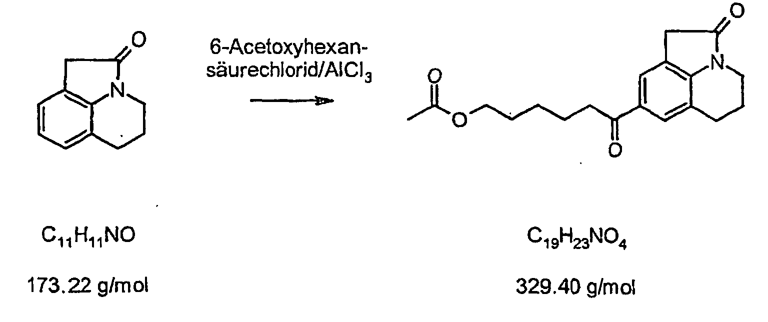

Zu einer Suspension von wasserfreiem Aluminiumchlorid (61.5 g, 461.6 mmol) in absolutem CH₂Cl₂ (500 mL) wird 6-Acetyloxyhexansäurechlorid (16.7 g, 86.6 mmol) in absolutem CH₂Cl₂ (50 mL) bei 0°C innerhalb von 10 Minuten zugetropft und 15 Minuten bei dieser Temperatur gerührt. 5,6-Dihydro-4H-pyrrolo[3,2,1-*ij*]chinolin-2(1*H*)-on (10.0 g, 57.7 mmol) in absolutem CH₂Cl₂ (100 mL) wird innerhalb von 15 Minuten bei 0°C zugetropft, dann wird auf Siedetemperatur erhitzt und 30 Minuten gerührt. Man kühlt auf 0°C ab, hydrolysiert mit Eis und verteilt zwischen Wasser (300 mL) und CH₂Cl₂ (100 mL). Die wäßrige Phase wird mit CH₂Cl₂ (2 x 100 mL) extrahiert, die vereinigten organischen Phasen werden mit 2 N Salzsäure (2 x 250 mL), Wasser (2 x 250 mL),
halbkonzentrierter wäßriger Na₂CO₃-Lösung (2 x 250 mL), konzentrierter wäßriger Na₂CO₃-Lösung (2 x 250 mL) und konzentrierter Kochsalzlösung (1 x 250 mL) gewaschen, getrocknet (Natriumsulfat/Aktivkohle) und das Lösungsmittel am Rotationsverdampfer entfernt.
Nach dem Umkristallisieren aus Methanol (150 mL) erhält man das Produkt in Form hellgelber Kristalle (14.3 g, 75.5 %).
MT-304 JOS 1675
C₁₉H₂₃NO₄

| | | | |
|---|---|---|---|
| Berechnet | C, 69.28; | H, 7.04; | N, 4.25 |
| Gefunden | C, 69.27; | H, 6.99; | N, 4.25 |

¹H NMR (CDCl₃): δ 7.72 (s, 2H), 4.06 (t, J = 6.5 Hz, 2H), 3.72 (t, J = 5.7 Hz, 2H), 3.54 (s, 2H), 2.90 (t, J = 7.0 Hz, 2H), 2.80 (t, J = 6.0 Hz, 2H), 2.09 - 1.93 (m, 5H), 1.85 -1.56 (m, 4H), 1.50- 1.30 (m, 2H);
¹³C NMR (CDCl₃): ä 198.9 (s), 174.2 (s), 171.1 (s), 145.5 (s), 131.3 (s), 127.8 (d), 122.9 (s), 122.4 (d),119.5 (s), 64.3 (t), 38.9 (t), 38.0 (t), 36.1 (t), 28.5 (t), 25.7 (t), 24.4 (t), 24.1 (t), 21.0 (q), 20.9 (t)

### Stufe 2 5-(6-Hydroxy-1-oxohexyl)-5,6-dihydro-4H-pyrrolo[3,2,1-ij]chinolin-2(1H)-on

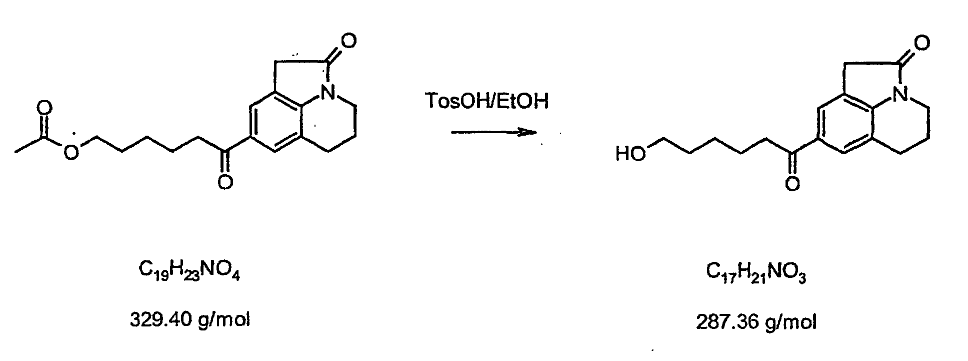

5-(6-Acetyloxy-1-oxohexyl)-5,6-dihydro-4*H*-pyrrolo[3,2,1-*ij*]chinolin-2(1*H*)-on (10.0 g, 30.6 mmol) wird in wasserfreiem Ethanol (150 mL) suspendiert, mit katalytischen Mengen 4-Methylbenzolsulfonsäuremonohydrat versetzt und fünf Stunden bei Siedetemperatur gerührt. Das Lösungsmittelvolumen wird auf ein Drittel eingeengt und das Produkt in Form hellgelber Nadeln (8.22 g, 93.5 %) durch Kristallisation bei - 20°C gewonnen.
MT-305 JOS 1672
C₁₇H₂₁NO₃

| | | | |
|---|---|---|---|
| Berechnet | C, 71.06; | H, 7.37; | N, 4.87 |
| Gefunden | C, 71.30; | H, 7.37; | N, 4.87 |

¹H NMR (CDCl₃): ä 7.67 (s, 2H), 3.80 - 3.52 (m, 4H), 3.48 (s, 2H), 2.97 - 2.66 (m, 4H), 2.08 - 1.86 (m, 2H), 1.82 - 1.26 (m, 6H);
¹³C NMR (CDCl₃): ä199.3 (s), 174.2 (s), 145.4 (s), 131.2 (s), 127.8 (d), 122.8 (s), 122.3 (d), 119.4 (s), 62.3 (t), 38.7 (t), 38.0 (t), 36.0 (t), 32.3 (t), 25.4 (t), 24.3 (t), 24.1 (t), 20.8 (t)

### Stufe 3 5-(6-lod-1-oxohexyl)-5,6-dihydro-4H-pyrrolo[3,2,1-ij]chinolin-2(1H)-on

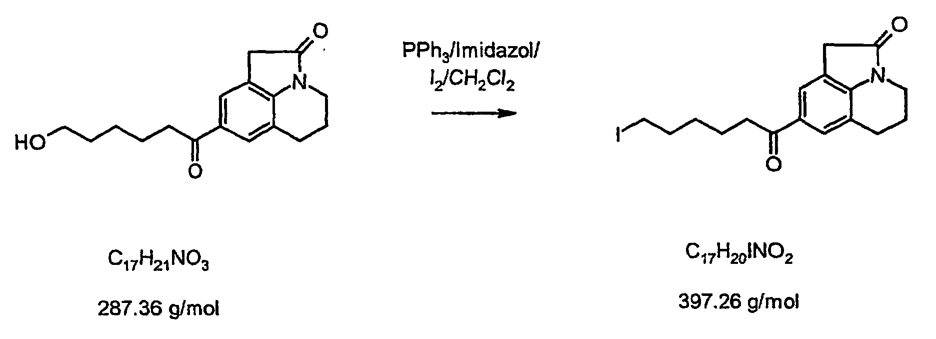

Triphenylphosphin (2.02 g, 7.74 mmol), Iod (3.08 g, 12.12 mmol) und Imidazol (0.618 g, 9.08 mmol) werden in absolutem CH₂Cl₂ (30 mL) 30 Minuten bei 15°C gerührt. 5-(6-Hydroxy-1-oxohexyl)-5,6-dihydro-4H-pyrrolo[3,2,1-*ij*]chinolin-2(1*H*)-on (2.0 g, 6.96 mmol) in wasserfreiem CH₂Cl₂ (10 mL) wird innerhalb von 5 Minuten bei dieser Temperatur zugetropft, dann wird 40 Minuten bei Raumtemperatur gerührt.

Man versetzt mit halbgesättigter Natriumsulfit-Lösung (50 mL), trennt die Phasen, extrahiert die wäßrige Phase mit CH₂Cl₂, wäscht die vereinigten organischen Phasen mit 2 N Salzsäure (3 × 100 mL), Wasser (2 × 100 mL), gesättigter Natriumhydrogencarbonatlösung (2 × 100 mL) und gesättigter Kochsalzlösung (1 × 100 mL), trocknet (Natriumsulfat/Aktivkohle), filtriert und kristallisiert das nach Entfernen des Lösungsmittels am Rotationsverdampfer erhaltene Rohprodukt aus Methanol (10 mL) um.

### Variante A:

Der Rückstand wird durch Säulenchromatographie (100 g Kieselgel, Chloroform) gereinigt, wodurch das Produkt in Form hellgelber Kristalle (2.44 g, 88.3 %) erhalten wird.

### Variante B:

Der Rückstand wird ein weiteres Mal aus Methanol (10 mL)umkristallisiert, wodurch das Produkt in Form hellgelber Kristalle (2.28 g, 82.4 %) erhalten wird.
MT-308
JOS 1670 C₁₇H₂₀INO₂

| | | | |
|---|---|---|---|
| Berechnet | C, 51.40; | H, 5.07; | N, 5.53 |
| Gefunden | C, 51.56; | H, 4.97; | N, 3.46 |

¹H NMR (CDCl₃): ä 7.70 (s, 2H), 3.72 (t, J = 5.7 Hz, 2H), 3.52 (s, 2H), 3.18 (t, *J* = 6.9 Hz, 2H), 2.91 (t, *J* = 7.2 Hz, 2H), 2.80 (t, *J* = 6.1 Hz, 2H), 2.12 - 1.61 (m, 6H), 1.57 - 1.36 (m, 2H);
¹³C NMR (CDCl₃): ä 198.7 (s), 174.1 (s), 145.5 (s), 131.2 (s), 127.8 (d), 122.9 (s), 122.3 (d), 119.4 {s), 38.8 (t), 37.9 (t), 36.0 (t), 33.2 (t), 30.1 (t), 24.0 (t), 23.3 (t), 20.9 (t), 6.6 (t)

### Stufe 4 5-(6-Methylsulfonyloxy-1-oxohexyl)-5,6-dihydro-4H-pyrrolo[3,2,1-ij]chinolin-2(1H)-on

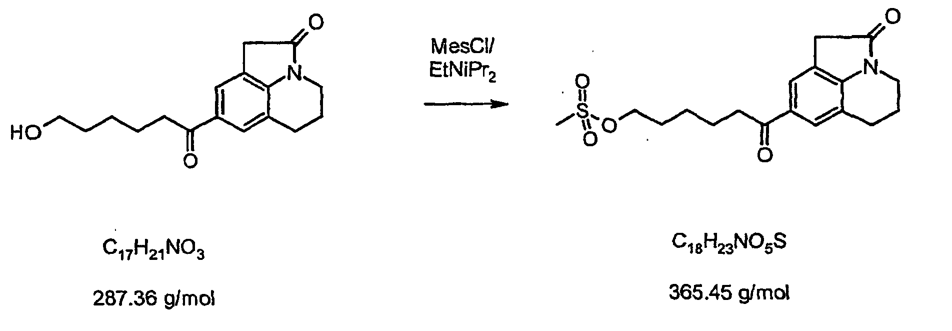

Zu 5-(6-Hydroxy-1-oxohexyl)-5,6-dihydro-4H-pyrrolo[3,2,1-*ij*]chinolin-2(1*H*)-on (1.0 g, 3.48 mmol) und N-Ethyldiisopropylamin (560 mg, 4.35 mmol) in wasserfreiem CH₂Cl₂ (10 mL) wird innerhalb von 5 Minuten Methansulfonsäurechlorid (458 mg, 4.00 mmol) bei 15°C zugetropft, dann wird zwei Stunden bei Raumtemperatur gerührt.
Man versetzt mit Wasser (20 mL), trennt die Phasen, extrahiert die wäßrige Phase mit CH₂Cl₂ (1 × 10 mL), wäscht die vereinigten organischen Phasen mit 2 N Salzsäure (3 × 10 mL), Wasser (2 × 10 mL), gesättigter Natriumhydrogencarbonatlösung (2 × 10 mL) und gesättigter Kochsalzlösung (1 × 10 mL), trocknet (Natriumsulfat/Aktivkohle), filtriert und digeriert das nach Entfernen des Lösungsmittels am Rotationsverdampfer erhaltene Rohprodukt mit Diisopropylether (10 mL), wodurch das Produkt in Form hellgelber Kristalle (1.17g, 92.2 %) erhalten wird.
¹H NMR (CDCl₃): ä 7.70 (s, 2H), 4.22 (t, J = 6.5 Hz, 2H), 3.71 (t, J = 5.8 Hz, 2H), 3.52 (s, 2H), 2.99 (s, 3H), 2.92 (t, J = 7.0 Hz, 2H), 2.80 (t, J = 6.0 Hz, 2H), 2.17 - 1.92 (m, 5H), 1.90 - 1.64 (m, 4H), 1.60 - 1.37 (m, 2H);
¹³C NMR (CDCl₃): ä 198.7 (s), 174.2 (s), 145.6 (s), 131.2 (s), 127.8 (d), 123.0 (s), 122.4 (d), 119.5 (s),69.8 (t), 38.8 (t), 37.8 (t), 37.3 (q), 36.1 (t), 28.9 (t), 25.1 (t), 24.3 (t), 23.7 (t), 20.9 (t)

### Stufe 5

### 5-[6-[(4aS,6R,8aS)-4a,5,9,10,11,12-hexahydro-6-hydroxy-3-methoxy-6H-benzofuro[3a,3,2-ef][2]benzazepin-11-yl]-1-oxohexyl]-5,6-dihydro-4H-pyrrolo[3,2,1-ij]chinolin-2(1H)-on (SPH-1500)

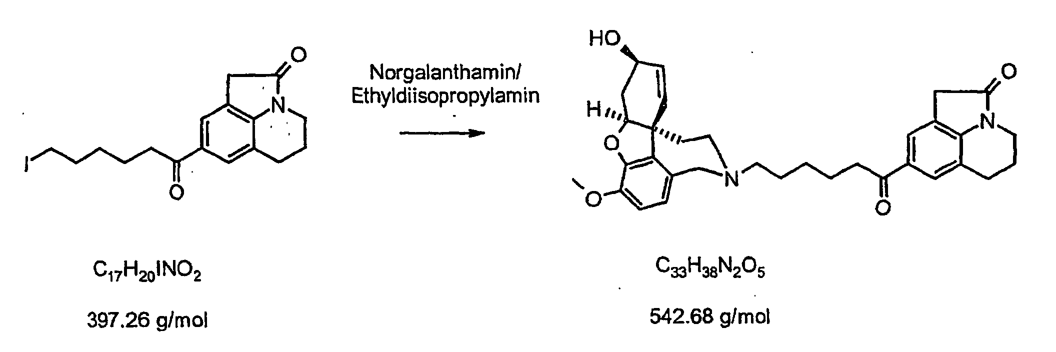

Norgalanthamin (1.13 g, 1.64 mmol), 5-(6-lod-1-oxohexyl)-5,6-dihydro-*4H*-pyrrolo(3,2,1-ij]chinolin-2(1*H*)-on (1.50 g, 3.75 mmol) und N-Ethyldiisopropylamin (1.46 g, 11.3 mmol) werden in absolutem Chloroform (20 mL) 54 Stunden bei Siedetemperatur gerührt.

Der nach Entfernen des Lösungsmittels am Rotationsverdampfer erhaltene Rückstand wird durch Säulenchromatographie (200 g Kieselgel, Chloroform : Methanol : Ammoniak = 96 : 3 : 1) gereinigt, wodurch das Produkt als hellgelber Schaum (1.31 g, 64.3 %) erhalten wird.
¹H NMR (CDCl₃): ä 7.63 (s, 2H), 6.68 - 6.46 (m, 2H), 6.00 (d, *J* = 10.3 Hz, 1H), 5.90 (dd, *J* = 10.3 Hz, *J* = 4.6 Hz, 1H), 4.51 (s, 1H), 4.19 - 3.96 (m, 2H), 3.75 (s, 1H), 3.73 (s, 3H), 3.70 - 3.58 (m, 2H), 3.44 (s, 2H), 3.35 - 2.98 (m, 2H), 2.96 - 6.67 (m, 4H), 2.66 - 2.29 (m, 4H), 2.15 -1.84 (m, 4H), 1.82 - 1.11 (m, 6H);
¹³C NMR (CDCl₃): ä 199.1 (s), 174.1 (s), 145.6 (s), 145.3 (s), 143.8 (s), 133.0 (s), 131.2 (s), 129.3 (s), 127.7 (d), 127.4 (d), 126.8 (d), 122.8 (s), 122.3 (d), 121.7 (d), 119.4 (s), 111.0 (d), 88.5 (d), 61.8 (d), 57.6 (t), 55.7 (q), 51.4 (t), 51.2 (t), 48.2 (s), 38.7 (t), 38.0 (t), 36.0 (t), 32.8 (t), 29.8 (t), 27.1 (t), 26.9 (t), 24.3 (t), 24.2 (t). 20.8 (t)

### Stufe 6

### 5-[6-[(4aS,6R,8aS)-4a,5,9,10,11,12-hexahydro-6-hydroxy-3-methoxy-6H-benzofuro[3a,3,2-ef][2]benzazepin-11-yl]-1-oxohexyl]-5,6-dihydro-4H-pyrrolo[3,2,1-ij]chinolin-2(1H)-on Fumarat (SPH-1499)

Die Fällung des Fumarats erfolgte analog Beispiel 4.
MT-311 JOS 1762
C₃₇H₄₂N₂O₉*H₂O

| | | | |
|---|---|---|---|
| Berechnet | C, 65.67; | H, 6.55; | N, 4.14 |
| Gefunden | C, 65.93; | H, 6.54; | N, 4.03 |

### Beispiel 128a

### Stufe 1

### 2-[[4-(1-Methylethoxy)phenyl]methyl]propandisäuredimethylester

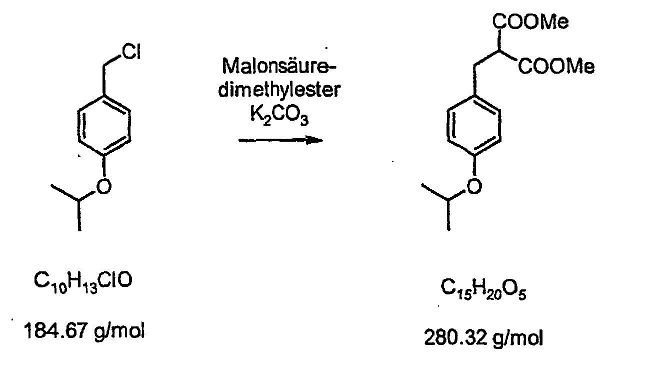

1-(Chlormethyl)-4-(1-methylethoxy)benzol (20.5 g, 111 mmol), Malonsäuredimethylester (102.5 g, 776 mmol) und Kaliumcarbonat (46.5 g, 332 mmol, wasserfrei, frisch vermahlen) werden in absolutem DMF (250 mL) 24 Stunden bei 70°C gerührt.

Das Gemisch wird filtriert und der nach Eindampfen des Filtrats am Rotationsverdampfer erhaltene Rückstand zwischen Ether (250 mL) und Wasser (250 mL) verteilt. Die organische Phase wird mit Wasser (3 × 200 mL) und gesättigter Kochsalzlösung (1 × 200 mL) gewaschen, getrocknet (Natriumsulfat/Aktivkohle), filtriert und das Lösungsmittel abgezogen.

Der überschüssige Malonsäuredimethylester wird durch Vakuumdestillation (85°C/15 mbar) abgetrennt und das im Rückstand verbliebene Rohprodukt durch Kugelrohrdestillation (130°C/0.001 mbar) gereinigt. Auf diese Weise erhält man das Produkt als farbloses Öl (23.6 g, 78 %).
MT-67 JOS 1774
C₁₅H₂₀O₅

| | | |
|---|---|---|
| Berechnet | C, 64.27; | H, 7.19 |
| Gefunden | C, 64.28; | H, 7.07 |

### Stufe 2:

### 4-(1-Methylethoxy)benzolpropansäure

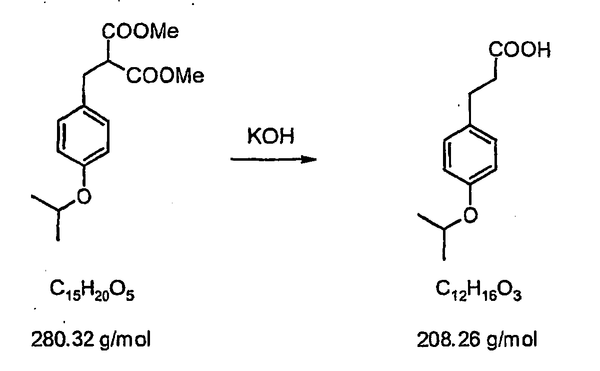

2-[[4-(1-Methylethoxy)phenyl]methyl]-propandisäuredimethylester (23.6 g, 84.2 mmol) wird in 2 N Kaliumhydroxidlösung (15 mL)/Ethanol (25 mL) 18 Stunden bei Siedetemperatur gerührt.

Der Ethanol wird am Rotationsverdampfer abdestilliert, der Rückstand mit konzentrierter Salzsäure auf einen pH von 1 gebracht und mit Ether (3 x 150 mL) extrahiert. Die vereinigten organischen Phasen werden mit Wasser (6 x 200 mL) und gesättigter Kochsalzlösung (200 mL) gewaschen, getrocknet (Natriumsulfat/Aktivkohle) und filtriert. Der nach Eindampfen am Rotationsverdampfer erhaltene Rückstand wird im Kugelrohr decarboxyliert (140°C/0.08 mbar) und anschließend destilliert (155°C/0.08 mbar). Auf diese Weise erhält man das Produkt in Form farbloser Kristalle (14.4 g, 82 %).
¹H NMR (CDCl₃): ä 7.12 (d, *J* = 9.5 Hz, 2H), 6.82 (d, *J* = 9.5 Hz, 2H), 4.50 (Septett, *J* = 6.3 Hz, 1H), 2.89 (t, *J* = 7.9 Hz, 2H), 2.63 (t, *J* = 7.9 Hz, 2H), 1.32 (d, *J* = 6.3 Hz, 6H);
¹³C NMR (CDCl₃): ä 178.8 (s), 156.4 (s), 132.1 (s), 129.2 (d), 116.0 (d), 69.9 (d), 35.8 (t), 29.7 (t), 22.1 (q)

### Stufe 3

### 2-Brom-4-methoxy-5-(1-methylethoxy)benzotacetonifril

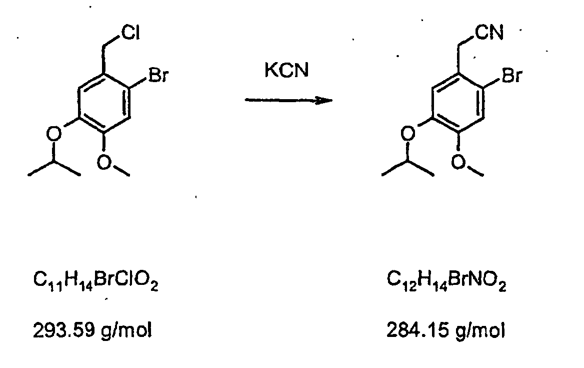

1-Brom-2-(chlormethyl)-5-methoxy-4-(1-methylethoxy)benzol (7.00 g, 23.8 mmol) und Kaliumcyanid (1:70 g, 26.1 mmol, frisch vermahlen) werden in absolutem DMSO (70 mL) 12 Stunden bei Raumtemperatur gerührt.

Das Gemisch wird auf Wasser (700 mL) gegossen, die wäßrige Phase mit Ether (3 × 150 mL) extrahiert, die vereinigten organischen Phasen werden mit Wasser (5 x 150 mL) und gesättigter Kochsalzlösung (1 × 200 mL) gewaschen, getrocknet (Natriumsulfat/Aktivkohle), filtriert und der nach Eindampfen erhaltene Rückstand mit Diisopropylether (15 mL) digeriert. Auf diese Weise erhält man das Produkt in Form farbloser Kristalle (6.46 g, 95 %).
MT-72 JOS 1695
C₁₂H₁₄BrNO₂

| | | | |
|---|---|---|---|
| Berechnet | C, 50.72; | H, 4.97; | N, 4.93 |
| Gefunden | C, 50.73; | H, 4.84; | N, 4.89 |

¹H NMR (CDCl₃): ä 7.02 (s, 1H), 6.97 (s, 1H), 4.50 (Septett, *J* = 6.3 Hz, 1H), 3.81 (s, 3H), 3.72 (s, 2H), 1.36 (d, *J* = 6.3 Hz, 6H);
¹³C NMR (CDCl₃): ä 150.8 (s), 147.1 (s), 121.5 (s), 117.3 (s), 116.7 (d), 116.3 (d), 113.9 (s), 72.1 (d), 56.2 (q), 24.2 (t), 21.9 (q)

### Stufe 4

### 4-(1-Methylethoxy)benzolpropansäure-(1-methyl)ethylester

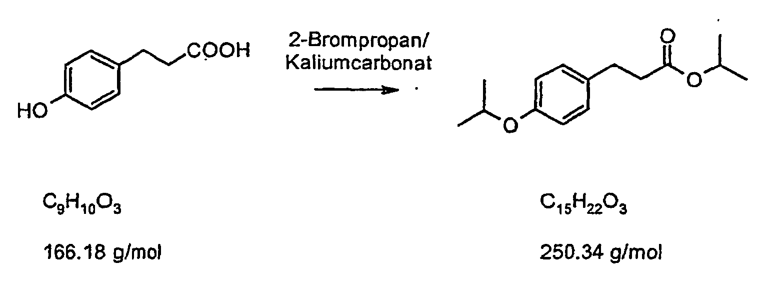

4-Hydroxybenzoipropansäure (50.0 g, 300 mmol), Kaliumcarbonat (210 g, 1.5 mol, wasserfrei, frisch vermahlen) und 2-Brompropan (221 g, 1.8 mol) werden in absolutem DMF (500 mL) 24 Stunden bei 60°C gerührt.

Die Lösung wird filtriert und der nach Eindampfen am Rotationsverdampfer erhaltene Rückstand zwischen Ether (500 mL) und 2 N Natronlauge (500 mL) verteilt. Die organische Phase wird mit 2 N Natronlauge (2 x 200 mL), Wasser (3 x 500 mL) und gesättigter Kochsalzlösung (200 mL) gewaschen, getrocknet (Natriumsulfat/Aktivkohle) und filtriert. Der nach Abdestillieren des Lösungsmittels am Rotationsverdampfer erhaltene Rückstand wird durch Kugelrohrdestillation (139 - 142°C/ 0.025 mbar) gereinigt, wodurch das Produkt als farbloses Öl (70.8 g, 94 %) erhalten wird.
MT-159 JOS 1768
C₁₅H₂₂O₃

| | | |
|---|---|---|
| Berechnet | C, 71.97; | H, 8.86 |
| Gefunden | C, 71.84; | H, 8.75 |

¹H NMR (CDCl₃): ä 7.10 (d, *J* = 9.5 Hz, 2H), 6.81 (d, *J* = 9.5 Hz, 2H), 4.99 (Septett, *J*= 6.3 Hz, 1H), 4.48 (Septett. *J* = 6.3 Hz, 1H), 2.87 (t, *J* = 7.9 Hz, 2H), 2.54 (t, J = 7.9 Hz, 2H), 1.20 (d, J = 6.3 Hz, 6H), 1.31 (d, *J* = 6.3 Hz, 6H);
¹³C NMR (CDCl₃): ä 172.4 (s), 156.2 (s), 132.4 (s), 129.1 (d), 115.8 (d), 69.7 (d), 67.4 (d), 36.4 (t), 30.1 (t), 22.0 (q), 21.7 (q)

### Stufe 5

### 4-(1-Methylethoxy)benzolpropanol

### 1. Aus 4-(1-Methylethoxy)benzolpropansäure

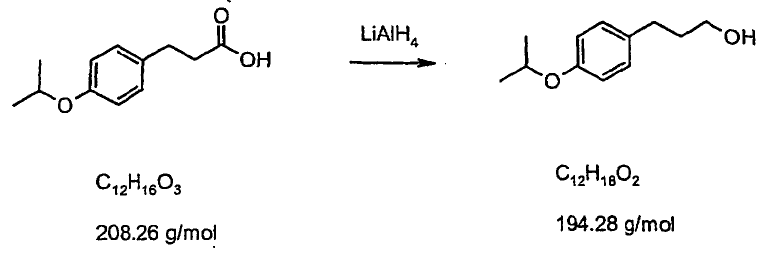

4-(1-Methylethoxy)benzolpropansäure (7.57 g, 36.3 mmol) in absolutem THF (80 mL) wird bei 0°C zu einer Suspension von Lithiumaluminiumhydrid (4.17 g, 110 mmol) in absolutem THF (80 mL) innerhalb von 30 Minuten zugetropft und 12 Stunden bei Raumtemperatur gerührt.
Man hydrolysiert mit Wasser (30 mL) und versetzt mit konzentrierter Salzsäure, bis die Lösung klar wird, verteilt zwischen Wasser (30 mL) und Ether (60 mL), extrahiert die wäßrige Phase mit Ether (2 x 20 mL), wäscht die vereinigten organischen Phasen mit 2 N Salzsäure (3 x 100 mL), Wasser (1 x 100 mL), gesättigter Natriumhydrogencarbonatlösung (2 x 100 mL) und gesättigter Kochsalzlösung (1 x 100 mL), trocknet (Natriumsulfat/Aktivkohle) und filtriert.

Nach Abdestillieren des Lösungsmittels am Rotationsverdampfer erhält man das Produkt in Form farbloser Kristalle (6.84 g, 97 %).

### 2. Aus 4-(1-Methylethoxy)benzolpropansäure-(1-methyl)ethylester

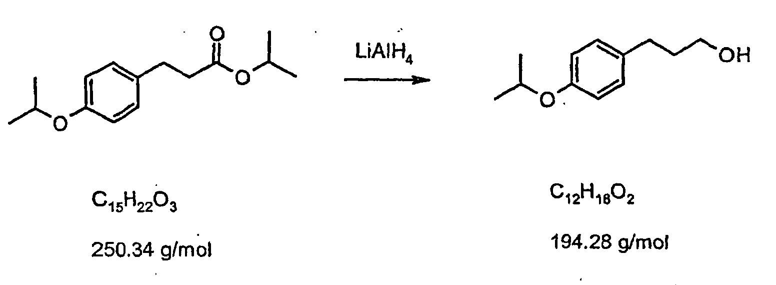

4-(1-Methylethoxy)benzolpropansäure-(1-methyl)ethylester (10.0 g, 39.9 mmol) in absolutem THF (100 mL) wird bei 0°C zu einer Suspension von Lithiumaluminiumhydrid (3.04 g, 80 mmol) in absolutem THF (100 mL) innerhalb von 30 Minuten zugetropft und 12 Stunden bei Raumtemperatur gerührt.

Man hydrolysiert mit Wasser (30 mL) und versetzt mit konzentrierter Salzsäure, bis die Lösung klar wird, verteilt zwischen Wasser (30 mL) und Ether (60 mL), extrahiert die wäßrige Phase mit Ether (2 × 20 mL), wäscht die vereinigten organischen Phasen mit 2 N Salzsäure (3 × 100 mL), Wasser (1 × 100 mL), gesättigter Natriumhydrogencarbonatlösung (2 x 100 mL) und gesättigter Kochsalzlösung (1 × 100 mL), trocknet (Natriumsulfat/Aktivkohle) und filtriert.

Nach Abdestillieren des Lösungsmittels am Rotationsverdampfer erhält man das Produkt in Form farbloser Kristalle (7.04 g, 99 %).
MT-89 JOS 1700
C₁₂H₁₈O₂

| | | |
|---|---|---|
| Berechnet | C, 74.19; | H, 9.34 |
| Gefunden | C, 73.93; | H, 9.07 |

¹H NMR (CDCl₃): ä 7.10 (d, *J* = 9.5 Hz, 2H), 6.82 (d, *J* = 9.5 Hz, 2H), 4.50 (Septett, *J* = 6.3 Hz, 1H), 3.68 (t, *J* = 7.9 Hz, 2H), 2.66 (t, *J* = 7.9 Hz, 2H), 2.0 (b, 1H), 1.93 - 1.78 (m, 2H), 1.32 (d, *J* = 6.3 Hz, 6H);
¹³C NMR (CDCl₃): ä 155.9 (s), 133.7 (s), 129.2 (d), 115.9 (d), 69.9 (d), 62.0 (f), 34.3 (f), 31.1 (f), 22.0 (q)

### Stufe 6

### 1-(3-Iodpropyl)-4-(1-methylethoxy)benzol

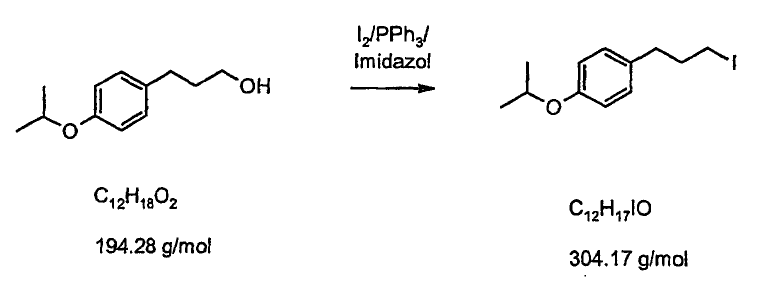

Triphenylphosphin (13.1 g, 49.9 mmol), lod (19.9 g, 78.4 mmol) und Imidazol (4.0 g, 58.8 mmol) werden in absolutem CH₂Cl₂ (250 mL) 20 Minuten bei Raumtemperatur gerührt. Man tropft bei 15°C 4-(1-Methylethoxy)benzolpropanol (8.74 g, 45.0 mmol) in CH₂Cl₂ (100 mL) zu und rührt 12 Stunden bei Raumtemperatur.

Man verteilt zwischen Wasser (300 mL) und CH₂Cl₂ (150 mL), extrahiert die wäßrige Phase mit CH₂Cl₂ (2 × 50 mL), wäscht die vereinigten organischen Phasen mit Wasser (1 × 200 mL), halbgesättigter Kupfer(II)sulfat-Lösung (2 × 200 mL), Wasser (1 × 200 mL), zehnprozentiger Natriumsulfitlösung (1 × 200 mL), gesättigter Kochsalzlösung (1 × 200 mL), trocknet (Natriumsulfat/Aktivkohle), filtriert und nimmt den nach Eindampfen am Rotationsverdampfer erhaltenen Rückstand in Diisopropylether (200 mL) auf. Man filtriert und reinigt den aus dem Filtrat nach Abziehen des Lösungsmittels am Rotationsverdampfer erhaltenen Rückstand durch Säulenchromatographie (900 g Kieselgel; Petrolether : Essigsäureethylester = 95 : 5). Auf diese Weise erhält man das Produkt als farbloses Öl (10.9 g, 79 %).
MT-151 JOS 1755
C₁₂H₁₇IO

| | | |
|---|---|---|
| Berechnet | C, 47.39; | H, 5.63 |
| Gefunden | C, 47.37; | H, 5.41 |

¹H NMR (CDCl₃): ä 7.11 (d, *J* = 9.5 Hz, 2H), 6.82 (d, J = 9.5 Hz, 2H), 4.53 (Septett, *J* = 6.3 Hz, 1H), 3.18 (t, *J* = 7.9 Hz, 2H), 2.67 (t, *J* = 7.9 Hz, 2H), 2.10 (Quinteft, *J* = 7.9 Hz, 2H), 1.35 (d, *J* = 6.3 Hz, 6H);
¹³C NMR (CDCl₃): ä 156.2 (s), 132.2 (s), 129.4 (d), 115.9 (d), 69.8 (d), 35.2 (t), 35.0 (t), 22.1 (q), 6.5 (t)

### Stufe 7

### α-[2-Brom-4-methoxy-5-(1-methylethoxy)phenyl]4-(1-methylethoxy)-benzolpentannitril

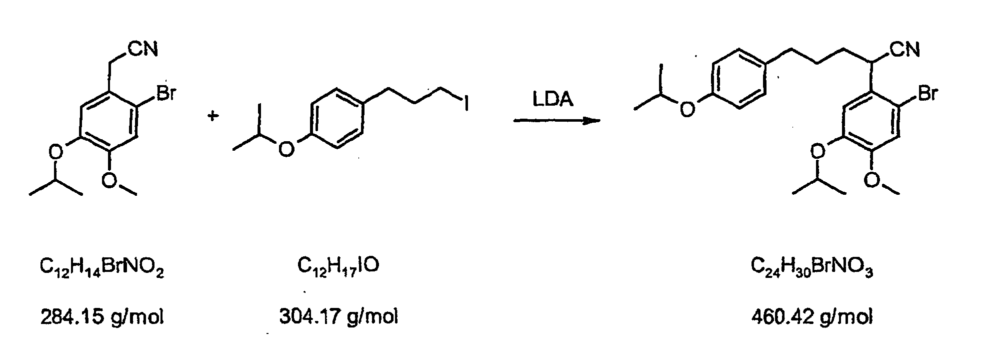

Zu einer Lösung von Diisopropylamin (3.55 g, 35.08 mmol) in absolutem THF (50 mL) wird n-Butlylithium (12.7 mL, 27.5 mmol, 2.2 M in Hexan) innerhalb von 15 Minuten bei einer Temperatur von - 78 C°zugegeben, dann wird das Gemisch auf - 30°C erwärmt und 30 Minuten bei dieser Temperatur gerührt.

Die Lösung wird auf- 78°C abgekühlt, mit 2-Brom-4-methoxy-5-(1-methylethoxy)-benzolacetonitril (7.94 g, 27.9 mmol) in absolutem THF (100 mL) versetzt, 20 Minuten bei dieser Temperatur gerührt, auf Raumtemperatur erwärmt und eine weitere Stunde gerührt. Das Gemisch wird auf - 78°C gekühlt, danach wird 1-(3-Iodpropyl)-4-(1-methylethoxy)benzol (8.50 g, 27.9 mmol) in absolutem THF (50 mL) innerhalb von 15 Minuten zugetropft und das Gemisch 45 Minuten gerührt.

Man versetzt mit gesättigter Ammoniumchloridlösung (50 mL) und erwärmt auf Raumtemperatur. Der nach Eindampfen erhaltene Rückstand wird zwischen 2 N Salzsäure (200 mL) und Ether (200 mL) verteilt. Die wäßrige Phase wird mit Ether (3 × 50 mL) extrahiert, die vereinigten organischen Phasen werden mit Wasser (3 × 200 mL), gesättigter Natriumhydrogencarbonatlösung (1 × 200 mL) und gesättigter Kochsalzlösung (1 × 200 mL) gewaschen, getrocknet (Natriumsulfat/Aktivkohle), filtriert und der nach Entfernen des Lösungsmittels am Rotationsverdampfer verbleibende Rückstand säulenchromatographisch (1000 g Kieselgel, Petrolether : Essigsäureethylester = 98 : 2) gereinigt. So erhält man das Produkt als farbloses Öl (11.46 g, 71 %).
MT-158 JOS 1699
C₂₄H₃₀BrNO₃

| | | | |
|---|---|---|---|
| Berechnet | C, 62.61; | H, 6.57; | N, 3.04 |
| Gefunden | C, 62.32; | H, 6.31; | N, 2.97 |

### Stufe 8

### α-[2-Brom-4-methoxy-5-(1-methylethoxy)phenyl]-4-(1-methylethoxy)-benzolpentansäureamid

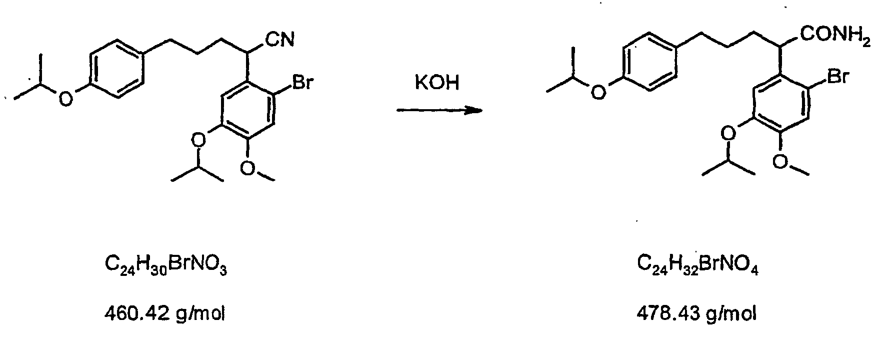

α-[2-Brom-4-methoxy-5-(1-methylethoxy)phenyl]-4-(1-methylethoxy)benzolpentannitril (30.0 g, 65.2 mmol) in Ethanol (600 mL) wird mit Kaliumhydroxid (60.0 g, 1.07 mol) in Wasser (100 mL) versetzt und 6 Stunden bei Siedetemperatur gerüht.

Der nach Eindampfen erhaltene Rückstand wird zwischen 2 N Salzsäure (200 mL) und Ether (300 mL) verteilt. Die wäßrige Phase wird mit Ether (3 × 75 mL) extrahiert. Die vereinigten organischen Phasen werden mit Wasser (3 × 200 mL), gesättigter Natriumhydrogencarbonatlösung (1 × 200 mL) und gesättigter Kochsalzlösung (1 × 200 mL) gewaschen, getrocknet (Natriumsulfat/Aktivkohle), filtriert und der nach Entfemen des Lösungsmittels verbleibende Rückstand säulenchromatographisch (1000 g Kieselgel, Petrolether : Ether = 1 : 2) gereinigt. Die höherlaufende Fraktion wird in absolutem CH₂Cl₂ (100 mL) aufgenommen, bei 0°C mit Oxalsäuredichlorid (3 mL) und einem Tropfen DMF versetzt und zwei Stunden gerührt. Der nach Abziehen des Lösungsmittels am Rotationsverdampfer erhaltene Rückstand wird in absolutem THF (100 mL) suspendiert, worauf 2 Stunden Ammoniak unter die Oberfläche eingeleitet werden. Das Gemisch wird filtriert und der nach Eindampfen erhalteneRückstand zwischen Wasser (100 mL) und Ether (100 mL) verteilt. Die wäßrige Phase wird mit Ether (3 × 50 mL) extrahiert, die vereinigten organischen Phasen werden mit Wasser (3 × 200 mL) und gesättigter Kochsalzlösung (1 × 200 mL) gewaschen, getrocknet (Natriumsulfat/Aktivkohle) getrocknet,filtriert, und der nach Eindampfen erhaltene Rückstand mit der durch Säulenchromatographie erhaltenen tieferlaufenden Fraktion vereinigt, unter Diisopropylether kristallisiert und mit Diisopropylether (100 mL) digeriert. Auf diese Weise erhält man das Produkt in Form farbloser Kristalle (26.0 g, 83.5 %).
¹H NMR (CDCl₃): ä 7.01 (d, *J* = 8.9 Hz, 2H), 6.98 (s, 1H), 6.92 (s, 1H), 6.75 (d, *J* = 8.9 Hz, 2H), 5.98 (b, 1H), 5.52 (b, 1H), 4.47 (Septett, *J* = 6.3 Hz, 2H), 3.91 (t, *J* = 7.0 Hz, 1H), 3.82 (s, 3H), 2.74 - 2.40 (m, 2H), 2.22 - 2.00 (m, 1H), 1.91 - 1.36 (m, 3H), 1.35- 1.22 (m, 6H);
¹³C NMR (CDCl₃): ä 175.2 (s), 155.8 (s), 149.9 (s), 147.0 (s), 133.9 (s), 130.8 (s), 129.1 (d), 115.7 (d), 114.8 (d), 114.7 (d), 71.4 (d), 69.7 (d), 56.0 (q), 49.7 (d), 34.6 (t), 31.9 (t), 29.1 (t), 22.0 (q), 21.8 (q), 21.7 (q)

### Stufe 9

### α-[2-Brom-5-hydroxy-4-methoxyphenyl]-4-hydroxybenzolpentansäureamid

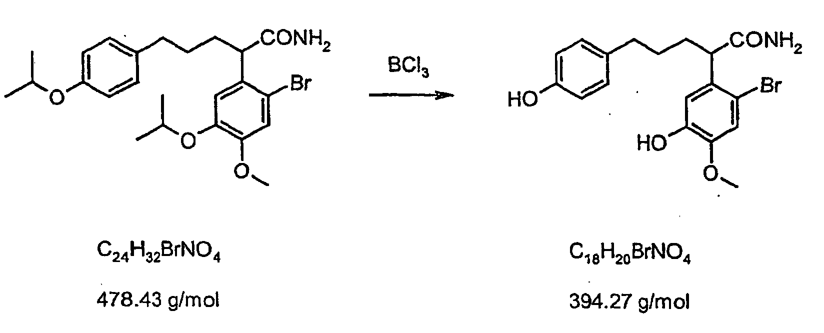

α-[2-Brom-4-methoxy-5-(1-methylethoxy)phenyl]-4-(1-methylethoxy)benzolpentansäureamid (24.0 g, 50.2 mmol) in absolutem CH₂Cl₂ (300 mL) werden bei - 78°C mit Bortrichlorid (150 mL, 150 mmol, 1 M in CH₂Cl₂) versetzt und vier Stunden bei Raumtemperatur gerührt.
Wasser (200 mL) wird zugetropft und die organische Phase am Rotationsverdampfer entfernt. Die ausgefallenen Kristalle werden mit Wasser (6 × 200 mL) digeriert, wodurch das Produkt in Form farbloser Kristalle erhalten wird (19.8 g, quant.).
MT-161 JOS 1713
C₁₈H₂₀BrNO₄

| | | | |
|---|---|---|---|
| Berechnet | C, 54.84; | H, 5.11; | N, 3.55 |
| Gefunden | C, 54.56; | H, 5.40; | N, 3.25 |

### Stufe 10

### 1-Brom-4a,5,9,10,11,12-hexahydro-3-methoxy-6-oxa-6H-benzo[a]cyclohepta[hi]benzoturan-12-carbonsäureamid (SPH-1484)

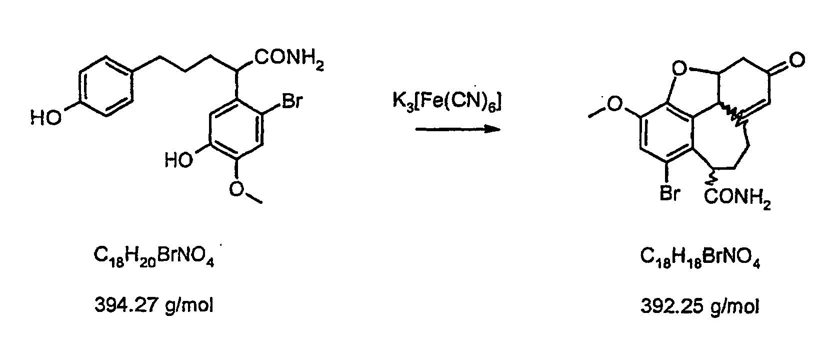

α-(2-Brom-5-hydroxy-4-methoxyphenyl)-5-hydroxybenzolpentansäureamid (3.00 g, 7.61 mmol) wird in Chloroform (300 mL) suspendiert und mit einer Lösung von Kaliumhexacyanoferrat(III) (13.2 g, 40.0 mmol) in Kaliumcarbonatlösung (75 mL, zehnprozentig) versetzt.

Das Gemisch wird bei Raumtemperatur 40 Minuten heftig gerührt und über Hyflo filtriert. Die wäßrige Phase wird mit Chloroform (2 × 50 mL) extrahiert, die vereinigten organischen Phasen werden mit 2N Salzsäure (2 × 100 mL), Wasser (2 × 200 mL) und gesättigter Kochsalzlösung (1 × 150 mL) gewaschen, getrocknet (Natriumsulfat/Aktivkohle) und das nach Eindampfen des Lösungsmittels erhaltene Rohprodukt über Säulenchromatographie (50 g Kieselgel, Essigsäureethylester) gereinigt. Auf diese Weise erhält man das Produkt als Gemisch aus zwei diastereomeren Enantiomerenpaaren, wobei das tieferlaufende zum höherlaufenden isomerisiert.

Durch Säulenchromatographie (Chloroform: Methanol = 96 : 4) erhält man das Enantiomerenpaar mit dem höheren Rf-Wert in Form farbloser Kristalle (0.24 g, 8 % d. Th.).
MT-162/OF JOS 1679
C₁₈H₁₈BrNO₄

| | | | |
|---|---|---|---|
| Berechnet | C, 55.12; | H, 4.63; | N, 3.57 |
| Gefunden | C, 55.15; | H, 4.71; | N, 3.38 |

¹H NMR (DMSO-d₆): δ 7.57 (s, 1H), 7.48 (d, J = 14.5 Hz, 1H), 7.14 (s, 2H), 5.89 (d, J = 14.5 Hz, 1H), 4.66 (s, 1H), 4.32 (s, 1H), 4.01 (q, J = 7.7 Hz, 1H), 3.78 (s, 3H), 3.02 (d, J = 19.6 Hz, 1H), 2.79 (d, J = 19.6 Hz, 1H), 2.52 (d, J = 16.5 Hz, 1H), 2.16 (d, J = 16.5 Hz, 1H), 1.96 - 1.67 (m, 2H), 1.14 (t, J = 7.7 Hz, 1H) ¹³C NMR (DMSO-d₆): δ 195.6 (s), 174.6 (s), 149.5 (d), 147.9 (s), 144.4 (s), 133.6 (s), 130.6 (s), 126.5 (d), 117.5 (s), 117.1 (d), 88.4 (d), 56.8 (q), 52.1 (s), 51.6 (d), 37.9 (t), 36.6 (t), 33.3 (t), 21.5 (t)

### Beispiel 128b

### (6R)-1-Brom-4a,5,9,10,11,12-hexahydro-3-methoxy-6-hydroxy-6Hbenzo[a]cyclohepta[hi]benzofuran-12-carbonsäureamid (SPH-1483)

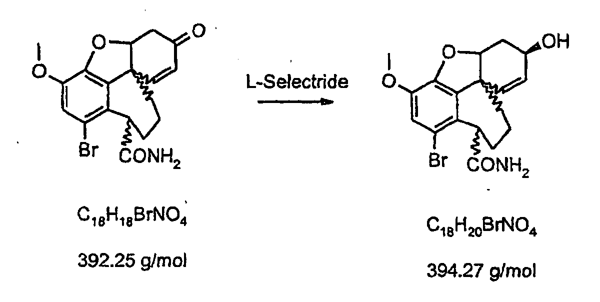

Zu einer Suspension von 1-Brom-4a,5,9,10,11,12-hexahydro-3-methoxy-6-oxa-6*H*benzo[a]cyclohepta[hi]benzofuran-12-carbonsäureamid (600 mg, 1.52 mmol) in absolutem THF (5 mL) wird bei 0°C L-Selectride® (4.6 mL, 4.6 mmol, 1 M in THF) innerhalb von 15 Minuten zugegeben und das Gemisch 12 Stunden bei Raumtemperatur gerührt. Man hydrolysiert mit Wasser (3 mL) und verteilt zwischen Wasser (10 mL) und Essigsäureethylester (10 mL), extrahiert die wäßrige Phase mit Essigsäureethylester (3 × 5 mL), wäscht die vereinigten organischen Phasen mit 1 N Salzsäure (3 × 10 mL), Wasser (2 × 10 mL), gesättigter Natriumhydrogencarbonatlösung (1 × 10 mL) und gesättigter Kochsalzlösung (1 × 10 mL), trocknet (Natriumsulfat/Aktivkohle), filtriert und reinigt das nach Abdestillieren des Lösungsmittels erhaltene Rohprodukt mittels Säulenchromatographie (50 g Kieselgel, Essigsäureethylester). Auf diese Weise erhält man das Produkt in Form farbloser Kristalle (798 mg, 83 %).
MT-169/OF JOS 1677
C₁₈H₂₀BrNO₄

| | | | |
|---|---|---|---|
| Berechnet | C, 54.84; | H, 5.11; | N, 3.55 |
| Gefunden | C, 54.67; | H, 5.10; | N, 3.46 |

¹H NMR (CDCl₃/DMSO-d₆): *δ* 6.97 (s, 1H), 6.79 (b, 1H), 6.49 (b, 1H), 6.12 (d, *J* = 11.4 Hz, 1H), 5.83 (dd, *J* = 11.4 Hz, *J* = 5.1 Hz, 1H), 4.42 (s, 1H), 4.31-4.21 (m, 1H), 3.78 (s, 3H), 3.42 - 3.18 (m, 2H), 2.68 - 2.29 (m, 2H), 2.14-1.38 (m, 5H);
¹³C NMR (CDCl₃/DMSO-d₆): δ 173.4 (s), 146.3 (s), 143.6 (s), 134.2 (s), 128.8 (d), 128.6 (d), 126.8 (s), 116.1 (s), 115.6 (d), 87.1 (d), 60.1 (q), 55.6 (d), 50.1 (s), 49.5 (d), 37.5 (t), 31.0 (t), 29.8 (t), 20.3 (t)

### Beispiel 128c

### (6R)-10-Amino-1-brom-4a,5,9,10,11,12-hexahydro-3-methoxy-6-hydroxy-6Hbenzo[a]cyclohepta[hi]benzofuran-6-ol (SPH-1482)

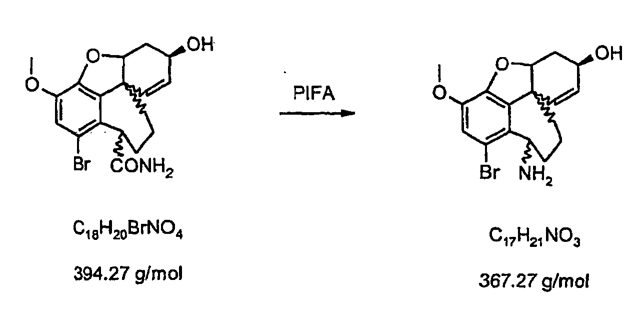

Bis(trifluoracetoxy)iodbenzol (300 mg, 0.76 mmol) wird Acetonitril (1.5 mL, HPLC-Qualität) gelöst und mit Wasser (1.5 mL, HPLC-Qualität) versetzt. Danach wird (6R)-1-Brom-4a,5,9,10,11,12-hexahydro-3-methoxy-6-hydroxy-6H-benzo[a]cyclohepta[hi]benzofuran-12-carbonsäureamid (338 mg, 0.76 mmol) innerhalb von 2 Stunden in Substanz zugegeben und das Gemisch 24 Stunden bei Raumtemperatur gerührt. Das Lösungsmittel wird am Rotationsverdampfer abdestilliert, der Rückstand in Chloroform (5 mL) aufgenommen, filtriert und durch Säulenchromatographie (30 g Kieselgel, Chloroform : Methanol : Ammoniak = 96 : 3 : 1) gereinigt. Auf diese Weise erhält man das Produkt in Form farbloser Kristalle (161 mg, 58 %).
MT-170 JOS 1705
C₁₇H₂₀BrNO₃·0.66 H₂O

| | | | |
|---|---|---|---|
| Berechnet | C, 54.02; | H, 5.68; | N, 3.71 |
| Gefunden | C, 53.96; | H, 5.52; | N, 3.60 |

¹H NMR (MeOH-d₄): δ 7.08 (s, 1H), 6.41 (d, J = 14.5 Hz, 1H), 5.8883 (dd, J = 14.5 Hz, *J* = 5.1 Hz, 1H), 4.72 (s, 1H), 4.58 (s, 1H), 4.13 (t, J = 3.6 Hz, 1H), 3.82 (s, 3H), 2.49 (d, J = 17.2 Hz, 1H), 2.45 - 2.07 (m, 4H), 1.92 - 1.58 (m, 4H);
¹³C NMR (MeOH-d₄): δ 147.2 (s), 144.7 (s), 134.5 (s), 133.3 (s), 130.9 (d), 126.4 (d), 116.6 (d), 115.5 (s), 87.8 (d), 61.2 (d), 57.3 (q), 54.0 (d), 48.6 (s), 38.3 (t), 35.2 (t), 30.1 (t), 17.9 (t)

### Beispiel 128d

### (6R)-10-Amino-4a,59,10,11,12-hexahydro-3-methoxy-6-hydroxy-6H benzo[a]cyclohepta[hi]benzofuran-6-ol

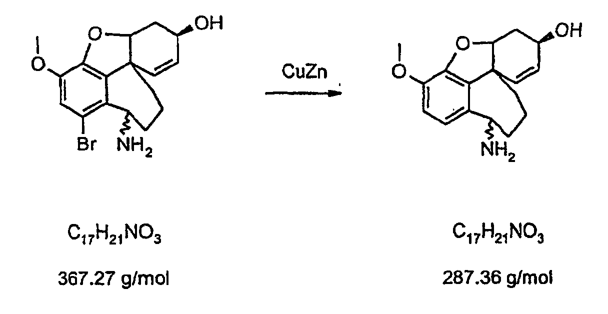

(6R)-10-Amino-1-brom-4a,5,9,10,11,12-hexahydro-3-methoxy-6-hydroxy-6Hbenzo[*a*]cyclohepta[*hi*]benzofuran-6-ol (70 mg, 0.19 mmol) und Calciumchlorid (300 mg, 2.7 mmol) werden in Substanz zu einer schwarzen Suspension von Zink (Herstellung: Zinkpulver (500 mg) und Kupfer(I)iodid (500 mg) werden unter Argon in Wasser (4 mL) und Ethanol (4 mL) 45 Minuten im Ultraschallbad behandelt) zugegeben und das Gemisch 5 Stunden bei Siedetemperatur gerührt. Man versetzt mit konzentrierter wäßriger Ammoniaklösung (1 mL), entfernt das Lösungsmittel am Rotationsverdampfer, nimmt den Rückstand in Chloroform (15 mL) auf, filtriert und reinigt den nach Eindampfen des Filtrats am Rotationsverdampfer erhaltenen Rückstand durch Säulenchromatographie (30 g Kieselgel, Chloroform : Methanol : Ammoniak = 96 : 3 : 1). Auf diese Weise erhält man das Produkt in Form farbloser Kristalle (42 mg, 78 %).
¹H NMR (CDCl₃): δ 6.81 - 6.61 (m, 3H), 6.97 (dd, J = 14 Hz, J = 4 Hz, 1H), 4.44 (s, 1H), 4.30 (s, 1H), 4.24 (t, J = 3Hz, 1H), 3.85 (s, 3H), 2.63 (dd, J = 17 Hz, J = 6 Hz, 1H), 2.40 (q, *J* = 15 Hz, 1H), 2.19 - 2.08 (m, 1H), 2.02 (dd, J = 18 Hz, J = 4 Hz, 1H), 1.97 - 1.52 (m, 9 H)
¹³C NMR (CDCl₃): δ 145.4 (s), 143.2 (s), 134.1 (s), 132.6 (s), 129.9 (d), 125.4 (d), 121.9 (d), 109.9 (d), 87.7 (d), 61.1 (d), 54.8 (q), 48.5 (s), 37.0 (t), 34.4 (t), 29.0 (t), 25.8 (t), 16.9 (t)

### BEISPIEL 129

### 8-[6-[(4aS,6R,8aS)-4a,5,9,10,11,12-hexahydro-6-hydroxy-3-methoxy-6H-benzofuro[3a,3,2-ef][2]benzazepin-11-yl]-1-oxohexyl]-1,2,5,6-tetrahydro-4H-pyrrolo[3,2,1-ij]chinolin-4-on (SPH-1516)

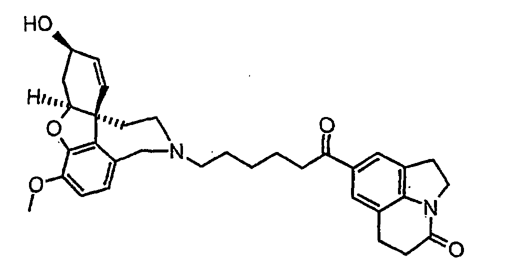

### 1. Synthese in Lösung:

Norgalanthamin (1.13 g, 4.13 mmol), 8-(6-lod-1-oxohexyl)-1,2,5,6-tetrahydro-4H-pyrrolo[3,2,1-ij]chinolin-4-on (1.50 g, 3.75 mmol) und N-Ethyldiisopropylamin (1.46 g, 11.3 mmol) werden in absolutem Chloroform (20 mL) 54 Stunden bei Siedetemperatur gerührt.
Der nach Entfemen des Lösungsmittels am Rotationsverdampfer erhaltene Rückstand wird durch Säulenchromatographie (200 g Kieselgel, Chloroform : Methanol : Ammoniak = 96 : 3 : 1) gereinigt, wodurch das Produkt als hellgelber Schaum (1.87 g, 92 %) erhalten wird.
- DC:: CHCl₃ : MeOH : NH₃ = 89 : 10 : 1, R_{f} = 0.5
¹H NMR (CDCl₃): δ 7.62 (s, 1H), 7.67 (s, 1H), 6.68 - 6.43 (m, 2H), 6.00 (d, J = 9.7 Hz, 1H), 5.93 - 5.81 (m, 1H), 4.51 (s, 1H), 4.22 - 3.91 (m, 4H), 3.92 - 3.64 (m, 4H), 3.48 - 2.28 (m, 13 H), 2.20 - 1.12 (m, 10H);
¹³C NMR (CDCl₃): δ 198.7 (s), 167.5 (s), 145.5 (s), 145.1 (s), 143.7 (s), 132.8 (s), 132.6 (s), 129.1 (s), 128.9 (s), 127.2 (d), 126.7 (d), 126.3 (d), 123.6 (d), 121.6 (d), 119.3 (s), 110.8 (d), 88.3 (d), 61.6 (d), 57.4 (t), 55.6 (q), 51.2 (t), 51.0 (t), 48.1 (s), 45.4 (t), 38.0 (t), 32.6 (t), 31.1 (t), 29.7 (t), 27.0 (t), 26.8 (t), 24.2 (t), 23.9 (t)
Herstellung des Fumarats (SPH-1519) analog Beispiel 4
MT-407 JOS 1761
C₃₇H₄₂N₂O₉*H₂O

| | | | |
|---|---|---|---|
| Berechnet | C, 65.67; | H, 6.55; | N, 4.14 |
| Gefunden | C, 65.69; | H, 6.49; | N, 4.02 |

### 2. Durch Festphasensynthese

0.300 g (0.102 mmol) Norgalanthamin-6-yloxy-1,5-dioxopentyloxymethyl-Merrifieldharz werden in einer beiderseits verschlossenen 5-ml-Polyethylenfritte 30 Minuten in 3 ml Dimethylformamid/Aceton (1/1) gequollen und nach dem Filtrieren in einer Lösung von 0.125 g (0.315 mmol) 8-(6-lodo-1-oxohexyl)-1,2,5,6-tetrahydro-4*H*-pyrrolo[3,2,1-*ij*]chinolin-4-on und 54 µl (0.041 g, 0.315 mmol) Ethyldiisopropylamin in 3 ml Dimethylformamid/Aceton (1/1) suspendiert.

Die Suspension wird bei Raumtemperatur 19 Stunden geschüttelt. Ein negativer Chloranil-Test zeigt den vollständigen Umsatz des sekundären Amins an. Das Harz wird dreimal mit Dimethylformamid (2 min, 3 ml) und sechsmal mit Tetrahydrofuran/Methanol (4/1, 2 min, 3 ml) gewaschen. Das Harz wird nachfolgend in einer Lösung aus 0.113 g (0.63 mmol) 30 %ger Natriummethanolat-Methanol-Lösung und 3.0 ml Tetrahydrofuran/Methanol (4/1) suspendiert. Noch 15 Stunden wird die Lösung abfiltriert, und das Harz sechsmal mit jeweils 3 ml Dichlormethan extrahiert.

Die vereinigten Filtrate werden mit methanolischer Salzsäure neutralisiert, mit 10 ml Dichlormethan verdünnt, zweimal mit 15 ml gesättigter Natriumchlorid-Lösung gewaschen, über Natriumsulfat getrocknet, filtriert und im Rotationsverdampfer unter verminderten Druck eingeengt. Das Rohprodukt wird mittels MPLC (200 g Kieselgel, v = 285 nm, Chloroform/Methanol/conc. Ammoniak = 96/3/1) getrennt. Nach dem Einengen erhält man ein gelbes Öl, welches beim Stehen kristallisiert: 0.043 g (0.041 g, 0.075 mmol, 74 %), gelbliche Kristalle (M_{w} = 542.7), HPLC, DC ident mit einer Referenzprobe:
- DC: R_{f} = 0.55 (Chloroform/Methanol = 8/2 + 2 % conc. Ammoniak)
- HPLC:: t_{Ret} = 13.7 min, 95.7 % (Merck Purospher-Säule, 4.0 mm x 125 mm, RP-18e, 5.0 µm, 1 ml/min, 285 nm, Acetonitril/ 20 mM Cl₃CCO₂H in H₂O (5/95 v/v für 5 min, 5/95 □ 60/40 v/v in 18 min (konvex), 60/40 v/v für 5 min)

### Beispiel 130a

### (4aS,6R,8aS)-4a,5,9,10,11,12-Hexahydro-3-methoxy-11-methyl-6H-benzofuro[3a,3,2-ef][2]benzazepin-6-ol, Galanthamin (HM 424)

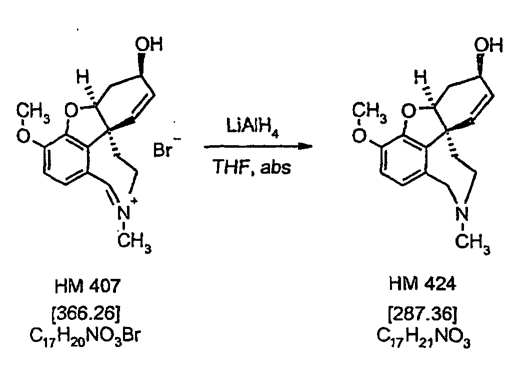

Zu einer Suspension von Galanthaminiumbromid HM 407 ( 1.0 g, 2.73 mmol) in absolutem Tetrahydrofuran (50 mL) wurde Lithiumaluminiumhydrid (104 mg, 2.73 mmol) hinzugefügt und für 3 Stunden bei Raumtemperatur gerührt. Danach wurde überschüssiges Lithiumaluminiumhydrid mit Ethylacetat vernichtet mit Wasser (49 mg, 2.73 mmol) zur Bildung eines filtrierbaren Niederschlags zugegeben. und gefällt. Das enstandene Al₂O₃ wurde abfiltriert, das Filtrat über Natriumsulfat getrocknet und das Lösungsmittel am Rotavapor entfernt. Es wurden 750 mg (96 % d. Th.) an Galanthamin als weißer Schaum erhalten.
DC: CHCl₃ : MeOH/NH₃ (9:1)
¹H NMR (CDCl₃): δ 6.66-6.58 (m, 2H), 6.08-5.94 (m, 2H), 4.58 (b, 1H), 4.15 (b, 1H), 4.06 (d, J = 15.2 Hz, 1H), 3.78 (s, 3H), 3.66 (d, J = 15.2 Hz, 1H), 3.25 (ddd, J = 14.4, 2.2, 1.9 Hz, 1H), 3.05 (ddd, J = 14.9, 3.1, 3.1 Hz, 1H), 2.68 (ddd, J = 15.7, 1.8, 1.8, 1H), 2.40 (s, 3H), 2.15-1.90 (m, 2H), 1.55 (ddd, J = 13.7, 4.1, 2.0 Hz, 1H); ¹³C NMR (CDCl₃): δ 145.8 (s), 144.1 (s), 133.1 (s), 129.2 (s), 127.6 (d), 126.8 (d), 122.1 (d), 111.1 (d), 88.7 (d), 62.0 (d), 60.4 (t), 55.8 (q), 53.7 (t), 48.2 (s), 41.9 (q), 33.4 (t), 29.9 (t)

### Beispiel 130b:

### (4aS,6R,8aS)-4a,5,9,10,11-Pentahydro-12-deutero-3-methoxy-11-methyl-6H-benzofuro[3a,3,2-ef][2]benzazepin-6-ol, (12-Deuterogalanthamin, SPH-1520)

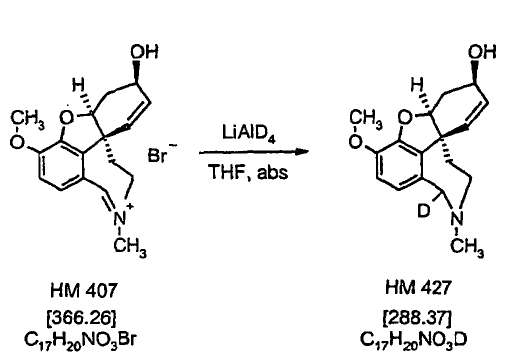

Zu einer Suspension von Galanthaminiumbromid HM 407 (250 mg, 0.683 mmol) in absolutem Tetrahydrofuran (15 mL) wurde Lithiumaluminiumdeuterid (28 mg, 0.68 mmol) hinzugefügt und für 3 Stunden bei Raumtemperatur gerührt. Danach wurde überschüssiges Lithiumaluminiumdeuterid mit Ethylacetat zersetzt und mit Deuteriumoxid (12 mg, 0.68 mmol) Al₂O₃ gefällt. Das ehstandene Al₂O₃ wurde abfiltriert, das Filtrat über Natriumsulfat getrocknet und das Lösungsmittel am Rotavapor entfernt. Es wurden 100 mg (51% d. Th.) an HM 427 als weißer Schaum erhalten.
DC: CHCl₃ : MeOH/NH₃ (9:1)
¹H NMR (CDCl₃): δ 6.66-6.58 (m, 2H), 6.08-5.94 (m, 2H), 4.58 (b, 1H), 4.14 (b, 1H), 4.06 (d, J = 15.2 Hz, 0.5H), 3.78 (s, 3H), 3.66 (d, J = 15.2 Hz, 0.5H), 3.25 (ddd, J = 14.4, 2.2, 1.9 Hz, 1H), 3.05 (ddd, J = 14.9, 3.1, 3.1 Hz, 1H), 2.68 (ddd, J = 15.7, 1.8, 1.8, 1H), 2.40 (s, 3H), 2.15-1.90 (m, 2H), 1.55 (ddd, J = 13.7, 4.1, 2.0 Hz, 1H); ¹³C NMR (CDCl₃): δ 145.8 (s), 144.1 (s), 133.1 (s), 129.2 (s), 127.6 (d), 126.8 (d), 122.1 und 122.0 (d), 111.1 (d), 88.7 (d), 62.0 (d), 60.4 (t), 55.8 (q), 53.8 und 53.7 (t), 48.2 (s), 42.1 und 41.9 (q), 33.8 und 33.7 (t), 29.9 (t)

LC/MS: 30*2.1 mm Zorbax SB C 18 3µm, 40% MeOH für 2 Min. auf 100% @ 10 Min. für 10 Min; Rest H2O bei 0.5 ml/Min, UV 210, 250, 280 und 310 nm, ein einziger Peak (RT ca. 6.0 min). PI-MS m/z 289 ([M+H]⁺), 271 ([M+H-H₂O]⁺). NI-MS m/z 287 ([M-H]⁻). 269 ([M-H-H₂O]⁻).

### Beispiel 131:

### Norsanguinine (SPH-1486)

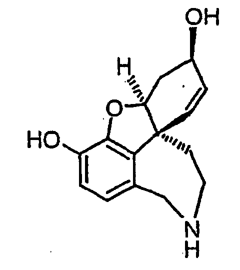

Eine Lösung von Norgalanthamin (1.0 g, 3.66 mmol) in 40 ml absolutem THF wird bei Raumtemperatur mit 17 ml L-Selectrid ^{R} (1 M in THF) versetzt und 24 Stunden bei Siedetemperatur gerührt.

Man kühlt auf Raumtemperatur ab, versetzt mit Essigsäureethylester (20 ml), dann mit Wasser (100 ml) und trennt die Phasen. Die organische Phase wird mit Wasser (4 x 20 ml) extrahiert, die vereinigten wäßrigen Phasen mit Essigsäureethylester (2 x 20 ml) extrahiert und der nach Eindampfen verbliebene Rückstand über Säulenchromatographie (100 g) Kieselgel, Chloroform : Ammoniak = 90 :9 : 1) gereinigt und unter Aceton kristallisiert. Auf diese Weise erhält man das Produkt in Form farbloser Kristalle (0.78 g, 82.3%).
¹H NMR (DMSO-d₆): δ 6.52-6.37 (m, 2H), 6.03 (d, J = 10.3 Hz, 1H), 5.78 (dd, J = 10.3 Hz, J = 4.6 Hz,
1H), 4.43 (s, 1H), 4.09 (s, 1H), 3.90 (d, J = 16 Hz, 1H), 3.71 (d, J = 16 Hz, 1H), 3.25-2.92 (m, 2H), 2.24-2.90 (m, 2H), 2.39 (d, J = 14 Hz, 2H);
¹³C NMR (DMSO-d₆): δ 145.6 (s), 140.4 (s), 133.0 (s), 132.3 (s), 127.7 (d), 127.6 (d), 119.6 (d), 114.8 (d), 86.5 (d), 60.1 (d), 53.1 (t), 48.3 (s), 46.6 (t), 40.2 (t), 30.8 (t)

### Beispiel 132:

### (4a,S,6R,8aS)-4a,5,9,10,11,12-hexahydro-3-methyl-6H-benzofuro[3a,3,2-ef][2]benzazepin-6-ol (SPH-1487)

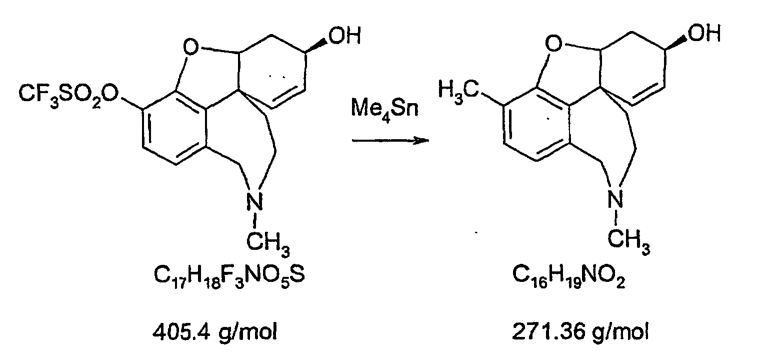

(4aS,6R,8aS)-4a,5,9,10,11,12-hexahydro-3-trifluormethylsulfonyloxy-6H-benzofuro[3a,3,2-ef[2]benzazepin-6-ol (200 mg, 0.49 mmol), Tetramethylstannan (106 mg, 0.59 mmol), wasserfreies Lithiumchlorid (62 mg, 1.47 mmol) und Tetrakistriphenylphosphinpalladium (28 mg, 0.025 mmol, 0.05 Äquivalente) werden in absolutem DMF (3 ml) 24 Stunden bei 100°C gerührt. Man verteilt zwischen Wasser (20 ml) und Essigsäureethylester (30 ml), extrahiert, die wäßrige Phase mit Essigsäureethylester (5 × 30 ml), wäscht die vereinigten, organischen Phasen mit Wasser (3 × 10 ml) und gesättigter Kochsalzlösung (15 ml) und reinigt den nach Eindampfen erhaltenen Rückstand durch Säulenchromatographie (20 g Kieselgel, Chloroform : Methanol : Ammoniak = 96 : 3: 1). Auf diese Weise erhält man das Produkt in Form farbloser Kristalle (102 mg, 77%).
MT-298 JOS 1711
C₁₇H₂₁NO₂*0.25 H₂O

| | | | |
|---|---|---|---|
| Berechnet | C, 74.02; | H, 7.86; | N, 5.08 |
| Gefunden | C, 73.77; | H, 7.67; | N, 5.04 |

¹H NMR (CDCl₃): δ 6.90 (d, J = 7.0 Hz, 1H), 6.46 (d, J = 7.0 Hz, 1H), 6.08 (d, J = 11.5 Hz, 1H), 6.00 (dd, J = 8.5 Hz, J = 5.2 Hz, 1H), 4.54 (s, 1H), 4.13 (s, 1H), 4.11 (d, J = 16.5 Hz, 1H), 3.70 (d, J = 16.5 Hz, 1H), 3.30 (t, J = 12.7 Hz, 1H), 3.08 (d, J = 12.7 Hz), 2.66 (dd, J = 15.2 Hz, J = 5.0 Hz, 1H), 2.42 (s, 3H), 2.18 (s, 3H), 2.17-2.00 (m, 2H), 1.57 (dd, J = 13.3 Hz, J = 5.0 Hz, 1H);
¹³C NMR (CDCl₃): δ 156.4 (s), 135.3 (s), 131.6 (s), 130.1 (d), 127.7 (d), 127.6 (d), 1220 (d), 119.8 (s), 88.1 (d), 62.7 (d), 61.3 (t), 54.2 (t), 48.5 (s), 42.4 (d), 33.9 (t), 30.4 (t), 15.3 (q).

### Beispiel 136:

### SPH-1146 TK 66/1

### (-) Cyclopropylmethylgalanthaminiumbromid

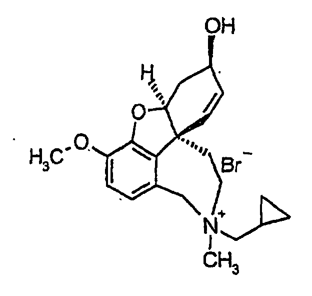

Herstellung analog Beispiel 90-99, Schmp. 230 - 237□
a^{D}₂₀=-110□ (C=1,5 in Wasser)

### Beispiel 137:

### SPH-1149 HM 104

### (-) (3-Methylbut-2-en-1-yl)-galanthaminiumbromid

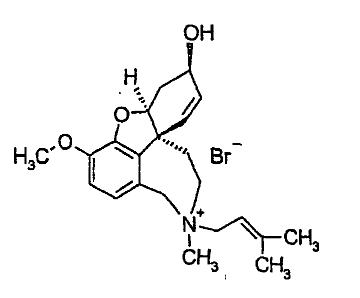

Herstellung analog Beispiel 90-99, Schmp. 198-201□
a^{D}₂₀=-118.2□ (1,5 in Wasser)

### Beispiel 138:

### SPH-1162 Cl 2-1 3au

### 3-((6R)-1-Brom-6-hydroxy-3-methoxy-5,6,9,10-tetrahydro-4aH-[1]benzofuro[3a,3,2-ef][2]benzazepin-11(12H)-yl)propansäureethylester

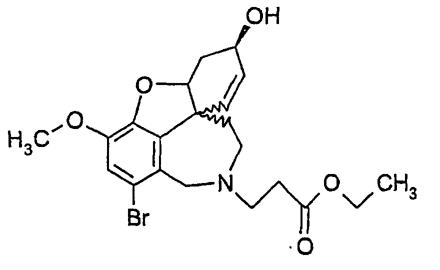

Herstellung analog Beispiel 143
Reaktionszeit: 8 h, Ausbeute: 80% farbloser Schaum

Gleiches Grundgerüst wie Beispiel 143, hier nur Angabe der dazu unterschiedlichen Signale: 1H-NMR (CDCl3) δ 4.13 (q, J = 6.0 Hz, 2H), 2.85 (t, 7.0 Hz, 2H), 2.58 (t, J = 7.0 Hz, 2H), 1.27 (t, J = 6 Hz, 3H);
13C-NMR(CDCl3) δ172.4 (s), 60.3 (t), 57.3 (t), 32.9 (t), 14.1 (q).

### Beispiel 139:

### SPH-1184 LCz 225/1

### (-) (4-Bromphenyl)methylgalanthaminiumbromid hemihydrat

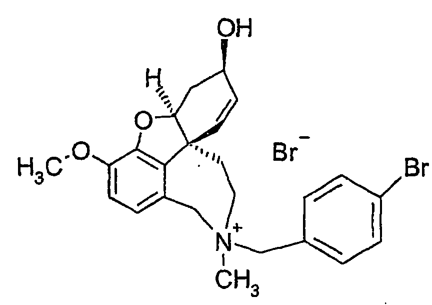

Herstellung analog Beispiel 90-99,

| | | | |
|---|---|---|---|
| Ber. | C 52.77, | H 5.17, | N 2.56 |
| Gef. | C 52.45, | H 5.15, | N 2.52 |

### Beispiel 140:

### SPH-1191 LCz 205

### (-) (3-Chlorpropyl)-galanthaminiumbromid . 1.25 H₂O

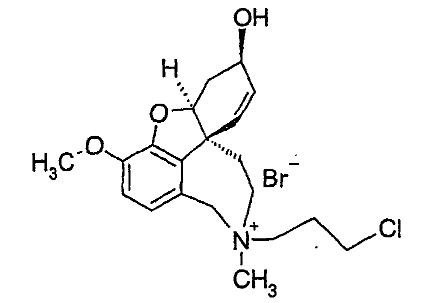

Herstellung analog Beispiel 90-99,

| | | | |
|---|---|---|---|
| Ber. | C 51.40, | H 6.36, | N 3.00 |
| Gef. | C 51.08, | H 6.07, | N 2.92 |

### Beispiel 141:

### SPH-1208 CB 2

### (6R)-1-Brom-6-hydroxy-N¹¹-isopropyl-3-methoxy-5,6,9,10-tetrahydro-4aH-[1]benzofuro[3a,3,2-ef][2]benzazepin-11(12H)-carbonsäureamid

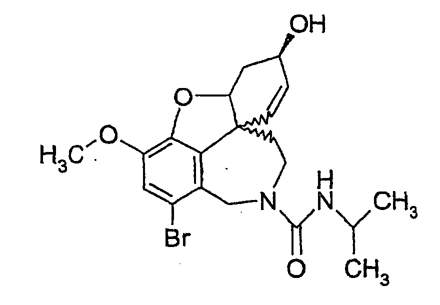

Herstellung analog Beispiel 142, Ausbeute: 96 %;
¹H-NMR (CDCl₃) δ 6.87 (s, 1H), 6.04 (dd, J = 16.0; 10.0 Hz, 2H), 4.88 (d, J = 18.0 Hz, 1H), 4.61 (m, 1H), 4.28 (d, J = 18.0, 1H), 4.13 (b, 1H), 3.90 (m, 1H), 3.85 (s, 3H), 3.26 (t, J = 12.0 Hz, 1H), 2.67 (dd, J = 16.0; 3.0 Hz, 1H), 2.29 (d, J = 10.0 Hz, 1H), 1.99 (m, 2H), 1.72 (d, J = 17 Hz, 1H); 1.12 (dd, J = 20.0; 5.0 Hz, 6H); ¹³C-NMR (CDCl₃) δ 156.0 (s), 146.5 (s), 144.9 (s), 133.8 (s),128.6 (d), 128.2 (s), 127.5 (s), 125.8 (d), 115.1 (d), 112.3 (d), 88.5 (d), 61.6 (d), 56.1 (q), 50.3 (t), 49.1 (s), 45.2 (t), 42.7 (d), 36.6 (t), 29.6 (t) 23.4 (q), 23.0 (q). Anal. (C₂₀H₂₅BrN₂O₄·0.3 H₂O)

| | | | |
|---|---|---|---|
| Ber. | C 54.26 | H 5.83 | N 6.33 |
| Gef. | C 54.28 | H 5.79 | N 6.14 |

### Beispiel 142:

### SPH-1209 CB 5

### (6R)- 1-Brom-6-hydroxy-3-methoxy-N11-methyl 5,6,9,10-tetrahydro-4aH-[1]benzofuro[3a,3,2-ef][2]benzazepin-11(12H)-thiocarbonsäureamid

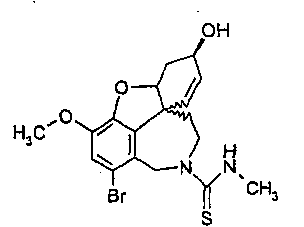

Zu einer gerührten Lösung von Bromnorgalanthamin (0.2 g, , 0.57 mmol) in Toluol (10 mL) wurde Methytisothiocyanate (42.0 mg, 0.57 mmol) zugetropft und 3 Stunden unter Rückfluß erhitzt. Nach Eindampfen wurde der Rückstand in in 2 N HCl (20 mL) aufgenommen und mit AcOEt (1 × 10 mL) gewaschen. Die wäßrige Lösung wurde mit konzentriertem Ammoniak auf einen pH > 8.5 gebracht und mit AcOEt (3 × 10 mL) extrahiert. Die vereinigten organischen Phasen wurden mit gesättigter Kochsalzlösung gewaschen, getrocknet (Na₂SO₄) and eingedampft, wodurch das Produkt in Form farbloser Kristalle vom Schmelzpunkt 183 - 185°C erhalten wurde (0.22 g, 99 %); ¹H-NMR (CDCl₃) δ 7.35 (b, 1H), 6.89 (m, 1H), 6.10 (m, 2H), 5.50 (d, J = 12.0 Hz, 1H), 5.11 (d, J = 12.0 Hz, 1H), 4.69 (b, 1H), 4.52 (d, J = 12.0 Hz, 1H), 4.17 (b, 1H), 3.85 (s, 3H), 3.60 (t, J = 18.0 Hz, 1H), 3.10 (d, J = 3.4 Hz, 3H), 2.72 (dd, J = 18.8; 2.0 Hz, 1H), 2.13 (m, 2H), 1.79 (d, J = 12.0 Hz, 1H); ¹³C-NMR (CDCl₃) δ 181.5 (s), 146.8 (s), 145.4 (s), 133.9 (s), 128.9 (d), 128.2 (s), 125.5 (d), 115.3 (d), 112.5 (d), 88.6 (d), 61.5 (d), 56.2 (q); 51.7 (t), 51.2 (t), 48.9 (s), 35.8 (t), 33.0 (q), 29.6 (t).
Anal. (C₁₈H₂₁BrN₂O₃S·0.5 H₂O)

| | | | |
|---|---|---|---|
| Ber. | C 50.83 | H 4.98 | N 6.59 |
| Get. | C 50.73 | H 5.02 | N 6.63 |

### Beispiel 143:

### SPH-1210 CB 4

### 3-((6R)-1-Brom-6-hydroxy-3-methoxy-5,6,9,10-tetrahydro-4aH-[1]benzofuro[3a,3,2-ef][2]benzazepin-11(12H)-yl)propannitril

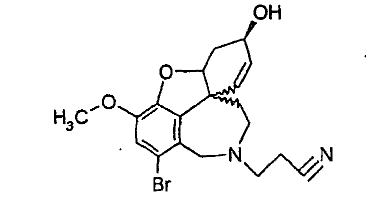

Zu einer Lösung von Norgalanthamin (200 mg, 0.57 mmol) in 50% EtOH (20 mL) wurde Acrylnitril (0.05 mL,'0.85 mmol) und Calciumchlorid (200 mg, 1.80 mmol) zugefügt und die Reaktion 3 h unter Rückfluß erhitzt. Die Reaktion wurde aufkonzentriert, der Rückstand in 2 N HCl (50 mL) aufgenommen und mit EtOAc (3 × 25 mL, organische Phase verwerfen) gewaschen. Die wäßrige Lösung wurde mit konz. NH₃ auf pH >8.5 gebracht und mit Methylenchlorid (3 x 25 mL) extrahiert. Die vereinigten organischen Phasen wurden mit gesättigter Natriumchloridlösung (200 mL) gewaschen, getrocknet (Na₂SO₄) und eingedampft und das Produkt als falbloser Schaum 220mg (95.7%) erhalten.
¹H-NMR (CDCl₃) δ 6.90 (s, 1H), 6.04 (dd, J₁ = 16.0 Hz, J₂ = 10.0 Hz, 2H), 4.60 (b, 1H), 4.38 (d, *J* = 16.0, 1H), 4.12 (b, 1H), 4.08(d, J = 16.0 Hz, 1H), 3.83 (s, 3H), 3.47 (t, J = 10.0 Hz, 1H), 3.18 (d, J = 18.0 Hz, 1H), 2.80 (t, J = 10.0 Hz, 2H), 2.63 (m, 2H), 2.61 (m, 1H), 2.03 (m, 2H), 1.60 (d, J = 10.0 Hz, 1H); ¹³C-NMR (CDCl₃) δ 145.6 (s), 144.5 (s), 134.1 (s), 128.4 (d), 127.1 (s), 126.1 (d), 118.6 (s), 115.8 (d), 114.3 (d), 88.7 (d), 61.7 (d), 56.1 (q), 54.9 (t), 52.0 (t), 48.9 (s), 47.2 (t), 33.3 (t), 29.7 (t), 16.8 (t).
Anal. (C₁₉H₂₁BrN₂O₃)

| | | | |
|---|---|---|---|
| Ber. | C 56.31 | H 5.22 | N 6.91 |
| Gef. | C 56.53 | H 5.44 | N 6.64 |

### Beispiel 144

### SPH-1227

### [4aS-(4aα,6α,8aR*)]-4a,5,9,10,11,12-Hexahydro-3-methoxy-11-methyl-6H-benzofuro[3a,3,2-ef][2]benzazepin-6-amin

### Schritt 1

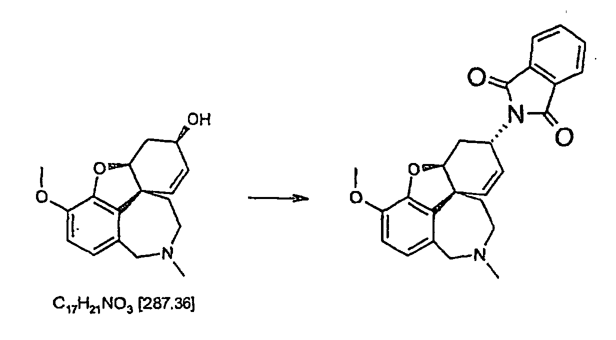

Eine Lösung von 300 mg (1.05 mmol) Galanthamin, 208 mg (1.20 mmol) Azadicarbonsäurediethylester, 314 mg (1.20 mmol) Triphenylphosphin und 1.20 mmol Phthalimid in 30 ml absolutem Tetrahydrofuran wird 24 Stdn. bei Raumtemp. gerührt. Anschließend wird das Tetrahydrofuran abrotiert, der Rückstand in 30 ml 2 N Salzsäure aufgenommen, dreimal mit je 30 ml Essigsäureethylester gewaschen und mit konzentriertem wäßrigen Ammoniak basisch gemacht. Danach wird die Lösung dreimal mit je 30 ml Essigsäureethylester extrahiert, die vereinigten organischen Phasen einmal mit gesättigter wäßriger Natriumchloridlösung gewaschen, getrocknet (Na₂SO₄), filtriert und eingedampft. Das Rohprodukt wird mittels FLC (15 g Kieselgel, Laufmittel: CHCl₃ : MeOH = 97:3) gereinigt.
83 % farblose Kristalle, Schmp.: 60 - 63°C
- DC:: CHCl₃ : MeOH = 9:1

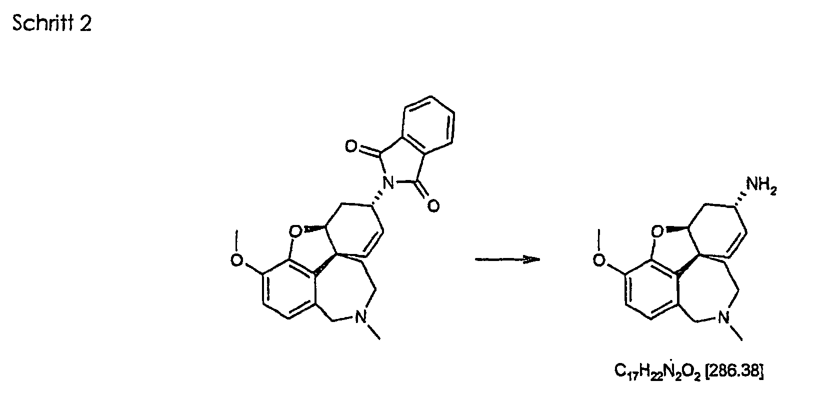

Zu einer auf -5°C gekühlten Lösung von 0.72 mmol Edukt in 5 ml absolutem Methanol werden 146 mg (1.44 mmol) Triethylamin sowie 162 mg (1.58 mmol) 3-(Dimethylamino)propylamin zugetropft. Anschließend läßt man das Reaktionsgemisch bei Raumtemp. 24 Stdn. rühren und rotiert sodann Methanol, Triethylamin und 3-(Dimethylamino)propylamin ab. Das erhaltene Rohprodukt wird über FLC (15 g Kieselgel, Laufmittel: CHCl₃: MeOH = 9:1 mit 0.5 % konzentriertem wäßrigen Ammoniak) gereinigt, wodurch farblose Kristalle vom Schmp. 119 - 121°C mit einem Drehwert von α_{D}²⁰[c = 0.1, CHCl₃] = -264° an Produkt erhalten werden.
- DC:: CHCl₃ : MeOH = 9:1
¹H NMR (CDCl₃): δ 1.56 - 1.89, m, 2H; 2.78, m, 1 H; 3.02, m, 1 H; 3.24, m, 1H; 3.48, m, 1H; 2.32, s, 1 H; 3.83, s, 1H; 3.63, d, 1 H; 4.07, d, 1H; 4.62, b, 1H; 4.98, b, 1H; 5.74, d, 1 H; 6.11, d, 1 H; 6.54, d, 1 H; 6.64, d, 1H.

### Beispiel 145:

### SPH-1273 CB 99

### (4aS,6R,8aS)-11-Methyl-3-phenoxy-5,6,9,10,11,12-hexahydro-4aH-[1]benzofuro[3a,3,2-ef][2]benzazepin-6-ol

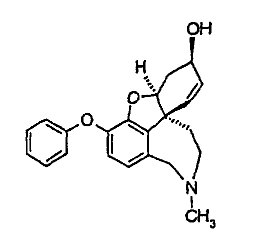

Zu einer Lösung von 1.0g (3.6mmol) O-Demethylgalanthamin in 50 ml Dichlormethan wurden 0.44 g (3.6 mmol) Benzolborsäure, 2.5ml (9mmol) Triethylamin, 0.67 g (3.6 mmol) Kupfer(II)acetat und 1 g Molsieb (4Â, zerkleinert) gegeben. Das Reaktionsgemisch wurde 44h bei Raumtemperatur gerührt. Der Feststoffe wurde abfiltriert. Das Filtrat wurde 2 mal mit je 30 ml gesättigter Natriumhydrogencarbonat - Lösung extrahiert. Die wäßrige Phase wurde mit 3 mal mit je 30 ml Methylenchlorid rückextrahiert. Die vereinigten organischen Phasen wurden über Natriumsulfat getrocknet und das Lösungsmittel wurde abdestilliert. Das Rohprodukt (0.55 g 43.7 % d. Th.) wurde mittels Säulenchromatographie (CHCl₃: MeOH = 95:5) gereinigt.
Ausbeute : 0.3 g (23.8% d. Th.)
- DC:: CHCl₃ : CH₃OH = 9:1
CB 99:
¹H-NMR (CDCl₃): δ 7.29 (m, 2H), 7.04 (t, J = 7.2 Hz, 1H), 6.89 (d, J = 8.7 Hz, 2H), 6.73 (dd, J₁ = 31.6 Hz, J₂ = 8,7 Hz, 2H), 6.03 (m, 2H), 4.59 (s, 1H), 4.51 (b, 1H), 4.17 (d, J = 15.3 Hz, 1H), 3.77 (d, J = 15.3 Hz, 1H), 3.33 (t, J = 13.0 Hz, 1H), 3.10 (d, J = 14.5 Hz, 1H), 2.53 (m, 1H), 2.43 (s, 3H), 2.15 (m, 1H), 1.89 (m, 1H), 1.62 (d, J = 13.8 1H); ¹³C-NMR (CDCl₃): δ 157.3 (s), 148.1 (s), 139.6 (s), 134.2 (s), 129.6 (2*d), 128.1 (d), 126.2 (d), 122.8 (d), 122.7 (d) 120.2 (d), 116.8 (d), 88.7 (d), 61.7 (d), 59.9 (t), 53.2 (t), 48.1 (s), 41.2 (q), 32.8 (t), 29.7 (t).
Anal. (C₂₂H₂₃NO₃* 0.2 CHCl₃)

| | | | |
|---|---|---|---|
| Ber. | C 71.43 | H 6.26 | N 3.75 |
| Gef. | C 71.43 | H 6.61 | N 3.84 |

### Beispiel 146:

### SPH-1288 HM 112, DD 13

### (6R)-3,6-Dihydroxy-N¹¹-isopropyl-5,6,9,10-tetrahydro-4aH-[1]benzofuro[3a,3,2-ef][2]benzazepin-11 (12H)-carbonsäureamid

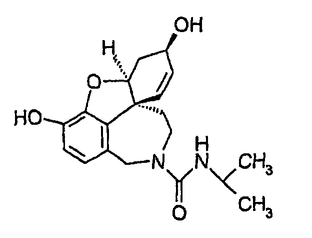

Zu einer Lösung von 1.6 mmol Edukt in 17 ml absolutem Dichlormethan wurde unter Argon bei-5°C eine Lösung von 0,42 ml (4,3 mmol) Bortribromid in 4 ml absolutem Dichlormethan langsam zugetropft. Nach 3 Stunden Rühren bei -5 bis 0°C wurde das Reaktionsgemisch auf 20 ml Wasser gegossen und mit Natriumhydrogencarbonat gesättigt. Die wäßrige Phase wurde 4 mal mit je 15 ml n-Butanol extrahiert und das Lösungsmittel wurde abdestilliert. Der Rückstand wurde mittels Säulenchromatographie (LM: CHCl₃:CH₃OH = 97:3) gereinigt und bei 50°C/50 mbar getrocknet. ¹H-NMR (CDCl₃): δ 6.57 (dd, J₁ = 18.7 Hz, J₂ = 8.0 Hz, 2H), 5.94 (dd, J₁ = 21.4 Hz, J₂ = 10.4 Hz, 2H), 4.90 (dd, J₁ = 10.6 Hz, J₂ = 6.0 Hz, 1H), 4.36 (m, 3H), 3.85 (m, 1H), 3.33 (t, J = 12.1 Hz, 1H), 2.93 (m, 1H), 2.25 (m, 1H), 1.87 (m, 2H), 1.24 (m, 1H), 1.06 (dd, J₂ = 21.3 Hz, J₂ = 6.5 Hz, 6H); ¹³C-NMR (CDCl₃): δ 156.7 (s), 146.5 (s), 141.0 (s), 131.5 (s),130.1 (s), 128.1 (d), 127.1 (d), 120.1 (d), 115.5 (d), 88.2 (d), 51.6 (t), 48.0 (s), 45.9 (t), 42.8 (d), 42.0 (d) 36.8 (t), 34.2 (t), 23.5 (q), 23.1 (q).
Anal. (C₁₉H₂₄N₂O₄* 0.8 CHCl₃) (JOS 1622).

| | | | |
|---|---|---|---|
| Ber. | C 54.0 | H 5.68 | N 6.37 |
| Gef. | C 54.08 | H 5.61 | N 6.33 |

### Beispiel 147:

### SPH-1302 HM 203

### (4aa,6β,8aR*)-4a,5,9,10-Tetrahydro-6-hydroxy-3-methoxy-6H-benzofuro[3a,3,2-ef][2]benzazepin-11(12H)-carbonsäure- 1,1-dimethylethylester (8d)

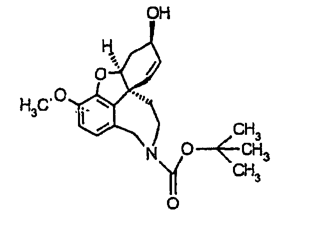

Zu einer Lösung von 12.0 g eines Gemisches von Norgalanthamin und Galanthamin im Verhältnis 94:6 (entsprechend 41.3 mmol Norgalanthamin) und 7.10 g (70.2 mmol) Triethylamin in 400 ml absolutem Tetrahydrofuran wurde innerhalb von 30 Minuten wurde eine Lösung von 9.00 g (41.30 mmol) Pyrokohlensäure-di-tertär-butylester in 150 ml absolutem Tetrahydrofuran unter Eiskühlung zugetropft. Nach 10 Minuten wurde die Eiskühlung entfernt und für 16 Stunden bei Raumtemperatur gerührt. Danach wurde das organische Lösungsmittel abgedampft, der Rückstand in Essigsäureethylester aufgenommen, dreimal mit je 150 ml 1 N wässriger Salzsäure, dreimal mit je 200 ml ges. Natriumhydrogencarbonatlösung und zweimal mit je 200 ml ges. Natriumchloridlösung gewaschen. Das Lösungsmittel wurde über Natriumsulfat getrocknet, eingedampft und das Rohprodukt mittels MPLC gereinigt; Laufmittel: Chloroform : Methanol 99 : 1 → 90:10. Es wurden 11.2 g weißer Schaum an HM 203 erhalten (73 % d. Th.)
- DC:: CHCl₃ : MeOH/NH₃ 9 : 1
¹H-NMR (CDCl₃, 200 MHz): 1.35-1.45 (m, 9H), 1.75 (m, 1H), 1.97 (m, 1H), 2.05 (m, 1H), 2.40 (m, 1H), 2.69 (b, 1H), 3.30 (b, 1H), 3.85 (OCH₃, s, 3H), 4.08-4.17 (m, 3H), 4.60 (b, 1H), 5.97-6.06 (m, 2H), 6.70-6.78 (m, 2H)
Anal. (C₂₁H₂₇NO₅.0.4 MeOH)

| | | | |
|---|---|---|---|
| Ber. | C 66.54 | H 7.46 | N 3.63 |
| Gef. | C 66.59 | H 7.59 | N 3.47 |

### Beispiel 149:

### SPH-1339 HM 264-1

### (4aS,6R,8aS)-11-propyl-3-methoxy-5,6,9,10,11,12-hexahydro-4aH-[1]benzofuro[3a,3,2-ef][2] benzazepin-6-ol

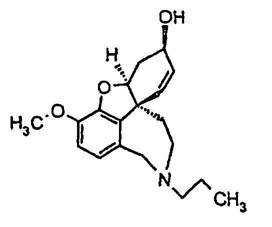

### Methode 1:

Eine Lösung von 250 mg (0.92 mmol) (-)-Norgalanthamin und 160 mg (2.76 mmol) Propanal in 20 ml absolutem Acetonitril wurden portionsweise mit 145 mg (2.3 mmol) Natriumcyanoborhydrid versetzt und für 12 Stunden bei Raumtemperaturgerührt. Anschließend wurden nochmals 145 mg (2.3 mmol) Natriumcyanoborhydrid portionsweise hinzugefügt und das Reaktionsgemisch für weitere 6 Stunden gerührt. Nach dem Abdampfen des Lösungsmittels erfolgte die Aufarbeitung nach Vorschrift A1. Die weitere Reinigung erfolgte mittels MPLC (Laufmittel: Chloroform : Methanol/NH₃ = 95 : 5). Es wurden 200 mg (70 % d. Th.) an HM 264 erhalten.
DC: CHCl₃: MeOH/NH₃ 9 : 1

### Methode 2:

Eine Lösung von 200 mg (0.73 mmol) (-) Norgalanthamin und 120 mg (1.46 mmol) Natriumacetat in 12 ml Wasser, 4 ml absolutem Ethanol und 0.62 ml Eisessig wurde auf 0°C gekühlt, mit 211 mg (3.65 mmol) Propanal versetzt und für 5 Minuten gerührt. Anschließend wurden 138 mg (3.65 mmol) Natriumborhydrid in 10 mg Portionen zugegeben. Nach 20 Minuten wurden weitere 211 mg (3.65 mmol) Propanal und 138 mg (3.65 mmol) Natriumborhydrid zugegeben und für 30 Minuten gerührt. Dann wurde das Reaktionsgemisch wie in Vorschrift A 1 beschrieben aufgearbeitet. Es wurden 210 mg (91 % d. Th.) an HM 264 erhalten.
DC: CHCl₃ : MeOH/NH₃ 9 : 1
¹H-NMR (CDCl₃, 200.13 MHz): δ 0.88 (t, J = 7.2, 3H), 1.96-2.11 (m, 2H), 2.45 (sextett, J = 7.8, J = 4.6, J = 5.0 Hz, 2H), 2.68 (ddd, J = 15.7, J = 1.8, J = 1.8, 1H), 3.18 (ddd, J = 14.9, J = 3.1, J = 3.1 Hz, 1H), 3.35 (ddd, J = 14.4, J = 2.2, J = 1.9, 1H), 3.80 (d, J = 15.3 Hz, 1H), 3.85 (s, 3H), 4.10 (d, J = 15.3, 1H), 4.12 (b, 1H), 4.60 (b, 1H), 5.96-6.13 (m, 2H), 6.61 (d, J = 8.2, 1H), 6.68 (d, J = 8.2, 1H); ¹³C-NMR (CDCl₃, 50.32 MHz): δ 11.8 (q), 20.5 (t), 29.9 (t), 32.9 (t), 48.4 (s), 51.4 (t), 53.5 (t), 55.8 (q), 57.7 (t), 62.0 (d), 88.6 (d), 111.1 (d), 121.9 (d), 127.0 (d), 127.4 (d), 129.6 (s), 133.1 (s), 143.9 (s), 145.7 (s)

### Beispiel 150:

### SPH-1340 HM 265-1

### N-Demethyl-N-propargyl-galanthamin (

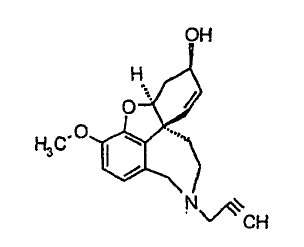

Eine Lösung aus 0.50 g (1.83 mmol) (-)Demethylgalanthamin, 0.51 g (3.66 mmol) Kaliumcarbonat und 0.55 g (3.66 mmol) Natriumiodid in 25 ml Dimethylformamid wurde mit 2.20 mmol Reagens versetzt und für sechs Stunden auf 70-80°C erhitzt. Danach wurde das Lösungsmittel abgedampft. Der Rückstand wurde in 50-100 ml 2 N wässriger Salzsäure aufgenommen und zweimal mit je 40-70 ml Essigsäureethylester gewaschen. Anschließend wurde mit konz. wässrigem Ammoniak basisch gemacht und dreimal mit je 40-70 ml Dichlormethan extrahiert. Die vereinigten organischen Phasen wurden zweimal mit je 40-70 ml ges. Natriumchloridlösung gewaschen, über Natriumsulfat getrocknet und das Lösungsmittel abgedampft.

Die weitere Reinigung erfolgte mittels MPLC (Laufmittel: Chloroform : Methanol/NH₃ = 95 : 5) Ausbeute: 0.26 g (46 % d. Th.) farbloses Öl
DC: CHCl₃ : MeOH/NH₃ 9 : 1
¹H-NMR (CDCl₃, 200.13 MHz): δ 1.53 (ddd, J = 13.8, J = 3.7, J = 2.1, 1H), 1.89-2.09 (m, 4H), 2.27 (t, J = 2.3, 2H), 2.65 (ddd, J = 15.8, J = 1.6, J = 1.6, 1H), 3.15-3.43 (m, 2H), 3.79 (d, J = 15.0 Hz, 1H), 3.85 (s, 3H), 4.11 (d, J = 15.0 Hz, 1H), 4.13 (b, 1H), 4.58 (b, 1H), 5.91-6.09 (m, 2H), 6.63 (b, 2H); ¹³C-NMR (CDCl₃, 50.32 MHz): δ 29.9 (t), 34.5 (t), 44.2 (t), 48.0 (s), 51.5 (t), 55.8 (q), 58.2 (t), 61.9 (d), 72.8 (s), 79.4 (d), 88.6 (d), 111.3 (d), 122.0 (d), 126.8 (d), 127.6 (d), 128.7 (s), 132.9 (s), 144.1 (s), 145.8 (s)

### Beispiel 151:

### SPH-1357 MF 8

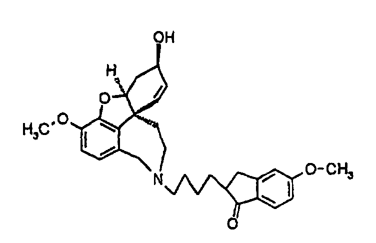

Herstellung analog Beispiel 6/Stufe 3, jedoch unter Verwendung von 2-(4-Brombutyl)-5-methoxyindan-1-on, falbloser Schaum.
¹H-NMR (ppm, CDCl₃): δ 7.65 (d, J = 8.1 Hz, 1H), 6.87 (d, J = 7.7 Hz, 2H), 6.62 (dd, J₁ = 12.9 Hz, J₂ = 8.4 Hz, 2H), 6.04 (m, 2H), 4.60 (b, 1H), 4.14 (m, 2H), 3.85 (s, 3H), 3.83 (s, 3H), 3.81 (m, 1H), 3.61 (d, J = 6.24 Hz, 1H), 3.25 (m, 2H), 2.88 (d, J = 15.1, 5H), 2.52 (b, 1H), 2.07 (m, 3H), 1.93 (m, 1H), 1.64-1.48 (m, 4H); ¹³C-NMR (ppm, CDCl₃): 207.0 (s), 165.3 (s), 162.5 (s), 156.6 (s), 145.8 (s), 144.1 (s), 133.1 (s), 129.5 (s), 127.6 (d), 126.9 (d), 122.0 (d), 115.2 (d), 111.2 (d), 109.6 (d), 88.7 (d), 62.0 (t),57.6 (t), 55.9 (q), 55.8 (q), 51.5 (t), 48.4 (d), 47.6 (d), 32.8 (t), 31.5 (t), 29.9 (t), 29.6 (t), 27.4 (t), 25.1 (t).

### Beispiel 155:

### SPH-1377 BK-34-2

### 2-[4-[(4aS,6R,8aS)-4a,5,9,10,11,12-hexahydro-6-hydroxy-3-methoxy-6H-benzofuro[3a,3,2-ef][2]benzazepin-11-yl]butyl]-5-methoxyindan-1-on, fumarat

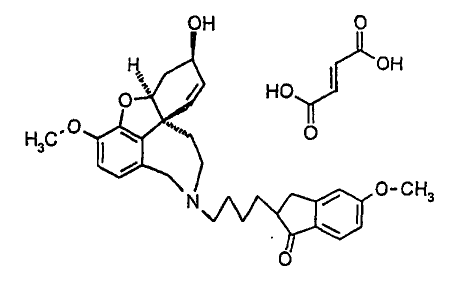

Herstellung aus Beispiel 151 analog Beispiel 7/Herstellung des Fumarats.
Schp.: 107-110°C
C₃₀H₃₅NO₅ 5/4C₄H₄O₄ 1 H₂O

| | | | |
|---|---|---|---|
| Berechnet | C, 64.07 | H, 6.44 | N, 2.11 |
| Gefunden | C, 64.26 | H, 6.41 | N, 2.23 |

¹H-NMR (ppm, CDCl₃): δ 7.56 (d, J = 10 Hz, 1H), 7.10 (s, 1H), 6.98 (d, J =10 Hz, 1H), 6.80 (m, 2H), 6.63 (s, 2H), 6.13 (d, J = 12.0 Hz, 1H), 5.89 (m, 1H), 4.61 (s, 1H), 4.50 (d, J = 8.0 Hz, 1H), 4.07 (b, 2H), 3.88 (s, 3H), 3.72 (s, 3H), 3.52 (t, J = 12 Hz, 1H), 3.31 (m, 2H), 2.69 (m, 5H), 2.30 (d, J = 12 Hz, 1H), 2.07 (m, 2H), 1.74 (m, 4H), 1.38 (m, 3H); ¹³C-NMR (ppm, CDCl₃): 206.3 (s), 166.9 (s), 165.3 (s), 157.2 (s), 146.3 (s), 144.6 (s), 133.1 (d), 129.7 (s), 129.4 (s), 126.4 (d), 125.1 (d), 122.5 (d), 115.8 (d), 112.7 (d), 110.3 (d), 86.8 (d), 65.3 (t), 60.0 (t), 56.1 (q), 55.8 (q), 51.1 (t), 47.5 (d), 46.9 (d), 32.5 (t), 32.5 (t), 31.9 (t), 31.2 (t), 30.8 (t), 24.9 (t), 24.2 (t).

### Beispiel 157:

### SPH-1515

### (4aS,6R,8aS)-3,6-Dihydroxy-5,6,9,10-tetrahydro-4aH-[1]benzofuro[3a,3,2-ef][2]benzazepin-11(12H)-yl)carbonsäureallylester (ML-7)

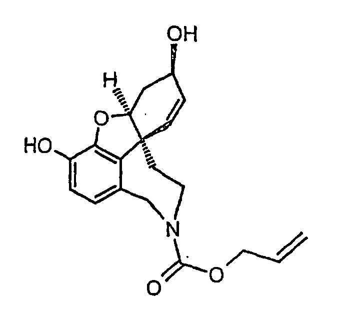

Zu einer Suspension von 0.788 g (0.552 g, 2.13 mmol) Norsanguinin (HPLC-Reinheit 70 %) und 10 ml absolutem Dichlormethan werden unter Inertgasatmosphäre mittels einer Spritze über ein Septum 2.11 ml (1.538 g, 19.3 mmol) Triethylamin und 0.81 ml (0.693 g, 6.384 mmol) Trimethylsilylchlorid zugespritzt, und die Suspension wird drei Stunden bei Raumtemperatur gerührt. Währenddessen fällt ein flockiger Niederschlag aus. Dann werden 0.34 ml (0.385 g, 3.192 mmol) Chlorameisensäureallylester unter Eisbadkühlung zugegeben (exotherm). Das Reaktionsgemisch wird innerhalb von zwei Stunden unter Rühren auf Raumtemperatur erwärmt, indem man das Eisbad auftauen läßt. Die Reaktion wird durch die Zugabe von 13 ml 2 N Salzsäure gestoppt, und die Phasen werden getrennt. Die organische Phase wird viermal mit jeweils 10 ml 2 N Salzsäure und einmal mit Kochsalzlösung gewaschen, die vereinigten wäßrigen Phasen werden einmal mit 20 ml Dichlormethan rückextrahiert. Die vereinigten organischen Phasen werden über Natriumsulfat getrocknet und filtriert. Nach dem Abdestillieren des Lösungsmittels im Rotationsverdampfer wird das Rohprodukt (HPLC-Reinheit 87.5 %) mittels MPLC gereinigt (50 g Kieselgel, ν = 285 nm, Chloroform/Methanol = 95/5). Nach dem Einengen und dem Trocknen im Hochvakuum wird das Produkt als gelbliches, hochviskoses Öl erhalten, welches beim Einengen aus Dichlormethan kristallisiert. Ausbeute: 0.443 g (1.29 mmol, 61 %), farbloser kristalliner Feststoff, (M_{w} = 343.4), DC: R_{f} = 0.55 (Cloroform/Methanol = 9/1), Schmp.: 197-198°C (Dichlormethan).
¹H-NMR: (200.13 MHz, CDCl₃, TMS) δ 7.66 (bs, 0.3 H), 6.52 - 6.76 (m, 2 H), 5.95 (bs, 2 H), 5.72-5.90 (m, 1H), 5.06 - 5.36 (m, 2 H), 4.90 (d, J = 12.7 Hz, 0.5 H) und 4.79 (d, J = 12.7 Hz, 0.5 H), 4.51 (bs, 3 H), 4.00 - 4.41 (m, 3 H), 3.22 - 3.53 (m, 1H), 3.13 (bs, 0.3 H), 2.58 (bd, J = 13.4 Hz, 1H), 1.63 - 2.10 (m, 3 H), ¹³C-NMR: (50.32 MHz, CDCl₃, TMS), δ 155.4 und 155.2 (s), 145.4 (s), 140.6 (s), 132.7 und 132.6 (d), 131.9 und 131.7 (s), 128.3 (s), 127.1 (d), 127.0 (d), 121.6 und 121.1 (d), 117.4 und 116.8 (t), 115.8 und 115.7 (d), 87.7 (d), 66.1 und 65.9 (t), 61.8 (d), 51.9 und 51.5 (t), 48.4 (s), 45.9 und 45.4 (t), 37.0 und 36.0 (t), 29.5 (t).

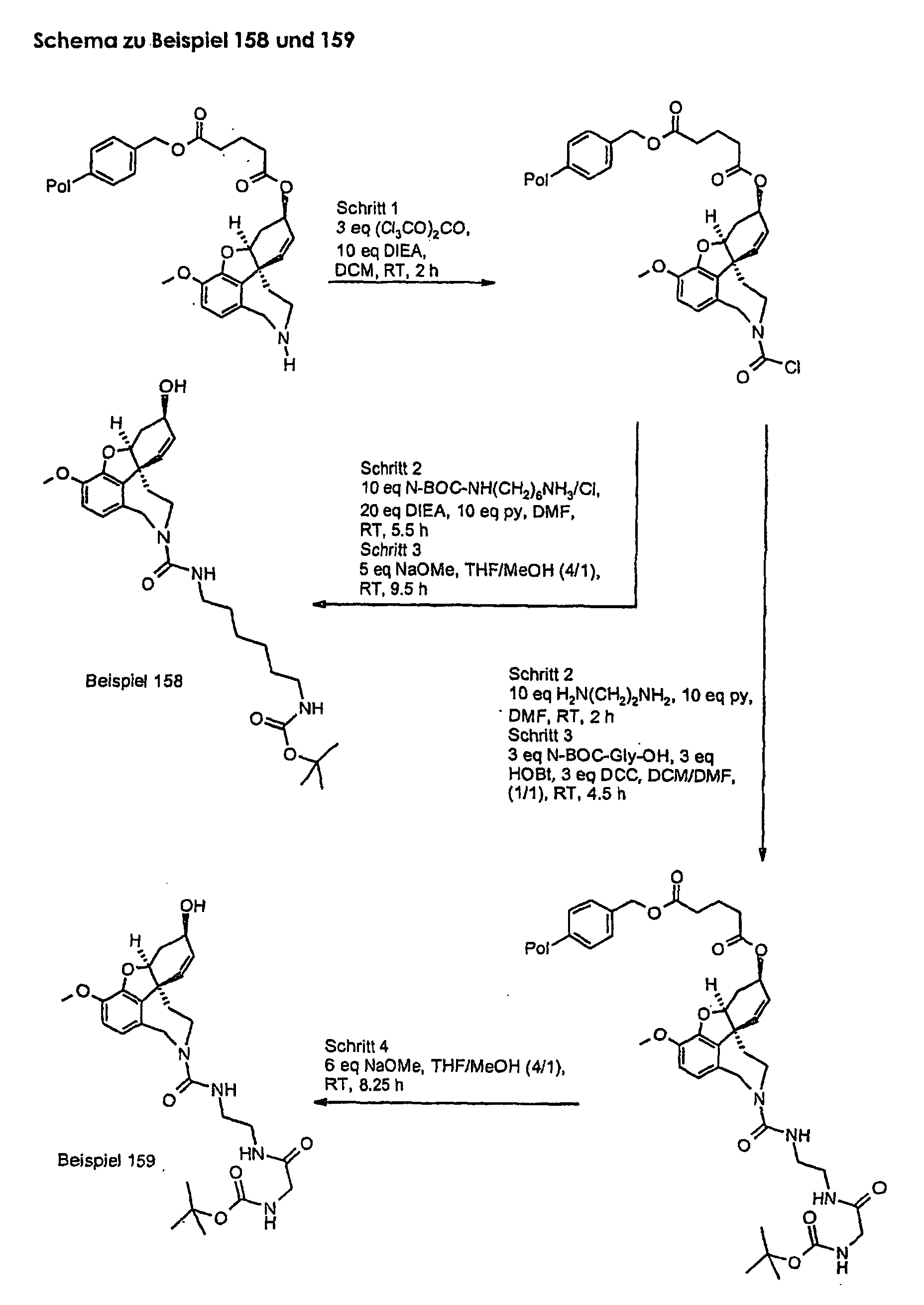

### Beispiel 158

### SPH-1522

### (4aS,6R,8aS)-N¹¹-(N-tert.-Butoxycarbonyl-6-aminohexyl)-6-hydroxy-3-methoxy-5,6,9,10-tetrahydro-4aH-[1]benzofuro-[3a,3,2-ef]-[2]benzazepin-11(12H)carboxamid (CK-52-6)

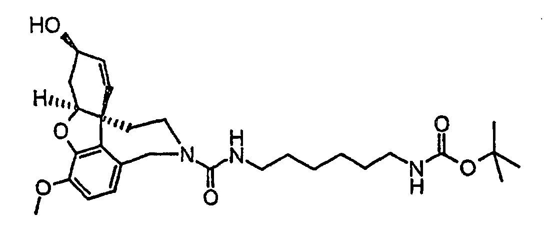

0.600 g (0.426 mmol) N-*tert*.-Butoxycarbonylnorgalanthamin-6-yloxy-1,5-dioxopentyloxymethyl-Merrifieldharz werden in einer beiderseits verschließbaren 10-ml-Polyethylenfritte 30 Minuten in ausreichend Dichlormethan gequollen und analog der Entschützungsvorschrift für die Immobilisierung von N-*tert*.-Butoxycarbonylnorgalanthamin (Beispiel 147) demaskiert und gewaschen (jeweils 6 ml Lösungsmittel). Anschließend wird das Harz analog der obigen Vorschrift mit 730 µl (0.551 g, 4.260 mmol) Ethyldiisopropylamin und 0.379 g (1.278 mmol) Triphosgen in 6 ml absolutem Dichlormethan umgesetzt. Nach dem Waschen und dem Trocknen des Harzes im Vakuum über Nacht erhält man 0.653 g des N-Chlorocarbonylnorgalanthamin-6-yloxy-1,5-dioxopentyloxymethyl-Merrifieldharzes.

Nach dem Quellen des Harzes (0.103 g, 0.065 mmol) und dem Filtrieren wird es in einer Lösung aus 0.164 g (0.650 mmol) N-tert.-Butoxycarbonyl-1,6-diaminohexanhydrochlorid, 222 µl (0.168 g, 1.300 mmol) Ethyldiisopropylamin, 0.053 µl (0.051 g, 0.650 mmol) Pyridin und 2.0 ml Dimethylformamid suspendiert (Um das Hydrochlorid zu lösen, wird die Lösung vorher erwärmt). Anschließend wird die Suspension bei Raumtemperatur 5.5 Stunden geschüttelt. Das Harz wird dreimal mit Dimethylformamid (2 min, 1 ml) und sechsmal mit Dichlormethan (2 min, 1 ml) gewaschen. Nach dem Trocknen im Vakuum wird das Harz in 2 ml Tetrahydrofuran für 30 Minuten geschüttelt und nach dem Filtrieren mit einer Lösung aus 0.059 g (0.018 g, 0.325 mmol) 30 %ger Natriummethanolat-Methanol-Lösung und 1.5 ml Tetrahydrofuran/Methanol (4/1) versetzt. Nach 9.5 Stunden Schütteln bei Raumtemperatur wird die Lösung abfiltriert, und das Harz dreimal mit jeweils 1.5 ml Dichlormethan/Methanol (1/1) und dreimal mit jeweils 1.5 ml Dichlormethan extrahiert. Die vereinigten Filtrate werden mit methanolischer Salzsäure neutralisiert, mit 10 ml Dichlormethan verdünnt, einmal mit 15 ml 2 N Salzsäure und zweimal mit jeweils 15 ml gesättigter Natriumchlorid-Lösung gewaschen, über Natriumsulfat getrocknet, filtriert und im Rotationsverdampfer unter verminderten Druck eingeengt. Das Rohprodukt (0.051 g) wird mittels Säulenchromatographie (5 g Kieselgel, Chloroform/Methanol = 50/1) getrennt.

Nach dem Einengen erhält man ein farbloses Öl.
- Ausbeute:: 0.030 g (0.058 mmol, 89 %), farbloses Öl (M_{w} = 515.7)
- DC: R_{f} = 0.47 (Chloroform/Methanol = 9/1)
- 1H-NMR:: (200.13 MHz, CDCl₃, TMS)
6.73 (d, *J* = 8.1 Hz, 1H), 6.66 (d, *J* = 8.3 Hz, 1H), 5.90 - 6.07 (m, 2 H), 4.23 - 4.70 (m, 5 H), 4.13 (bs, 1H), 3.82 (s, 3 H), 3.35 (t, *J* = 13.5 Hz, 1.0 H), 2.96 - 3.20 (m, 4 H), 2.67 (bd, *J* = 15.7 Hz, 1H), 1.65 - 2.10 (m, 3 H), 1.42 (s, 9 H), 1.06 - 1.40 (m, 8 H)
- ¹³C-NMR:: (50.32 MHz, CDCl₃, TMS)
δ 157.2, 156.0, 147.0, 144.7, 132.5, 129.2, 128.1, 126.5, 120.2, 111.2, 88.4, 61.9, 55.9, 51.7, 48.5, 45.7, 42.1, 40.5, 39.3, 36.6, 30.0, 29.7, 28.4, 26.1, 23.5
- HPLC:: t_{Ret} = 17.8 min, 98.7 % (Merck Purospher-Säule, 4.0 mm x 125 mm, RP-18e, 5.0 µm, 1 ml/min, 285 nm, Acetonitril/ 20 mM Cl₃CCO₂H in H₂O (20/80 für 5 min, 20/80 □ 60/40 in 12 min, 60/40 für 5 min, v/v)

### Beispiel 159

### N-tert.-Butyloxycarbonylglycin-[4-[(4aS,6R,8aS)-6-hydroxy-3-methoxy-5,6,9,10-tetrahydro-4aH-[1]benzofuro[3a,3,2-ef][2]benzazepin-11(12H)-yl]-3-aza-4-oxobutyl]amid (CK-58-2)

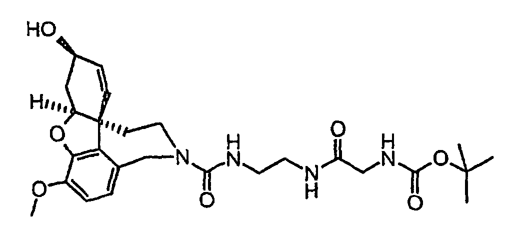

0.199 g (0.102 mmol) Norgalanthamin-6-yloxy-1,5-dioxopentyloxymethyl-Merrifieldharz werden in einer beiderseits verschließbaren 5-ml-Polyethylenfritte 30 Minuten in 2 ml Dichlormethan gequollen und nach dem Filtrieren in einer Lösung von 120 µl (0.091 g, 0.700 mmol) Ethyldiisopropylamin in 1.5 ml Dichlormethan suspendiert. Anschließend wird die Suspension mit einer auf 0°C gekühlten Lösung aus 0.062 g (0.210 mmol) Triphosgen und 0.5 ml Dichlormethan versetzt und bei Raumtemperatur zwei Stunden geschüttelt. Das Harz wird dreimal mit Dichlormethan (2 min, 2 ml) und dreimal mit Dimethylformamid (2 min, 2 ml) gewaschen. Nachfolgend fügt man eine Lösung aus 47 µl (0.042 g, 0.700 mmol) Ethylendiamin und 2.0 ml Dimethylformamid zu und schüttelt die Suspension bei Raumtemperatur. Nach drei Stunden wird die Lösung abfiltriert, und das Harz sechsmal mit jeweils 2 ml Dimethylformamid (2 min) gewaschen (Der Kaiser-Test ist nicht auswertbar. Die Beads zeigen eine braunrote Färbung). Das Harz versetzt man mit einer Lösung aus 0.037 g (0.210 mmol) N-BOC-Glycin, 0.028 g (0.210 mmol) 1-Hydroxybenzotriazol und 1 ml Dimethylformamid und schüttelt die Suspension fünf Minuten. Anschließend werden 0.043 g (0.210 mmol) Dicyclohexylcarbodiimid, gelöst in 1 ml Dichlormethan, zugesetzt. Man schüttelt die Suspension drei Stunden bei Raumtemperatur, filtriert das Harz und wäscht es dreimal mit Dimethylformamid (2 ml, 2 min) und sechsmal mit Dichlormethan (2 ml, 2 min). Nach dem Trocknen im Vakuum erhält man 0.239 g Harz, welches in Tetrahydrofuran für 30 Minuten geschüttelt und nach dem Filtrieren mit einer Lösung aus 0.076 g (0.023 g, 0.63 mmol) 30 %ger Natriummethanolat-Methanol-Lösung und 2.0 ml Tetrahydrofuran/Methanol (4/1) versetzt wird. Nach 8.25 Stunden Schütteln wird die Lösung abfiltriert, und das Harz dreimal mit jeweils 2 ml Dichlormethan/Methanol (1/1) und dreimal mit jeweils 2 ml Dichlormethan extrahiert. Die vereinigten Filtrate werden mit methanolischer Salzsäure neutralisiert, mit 10 ml Dichlormethan verdünnt, zweimal mit 15 ml gesättigter Natriumchlorid-Lösung gewaschen, über Natriumsulfat getrocknet, filtriert und im Rotationsverdampfer unter verminderten Druck eingeengt. Das Rohprodukt (0.064 g) wird mittels Säulenchromatographie (5 g Kieselgel, Chloroform/Methanol = 25/1 □ 15/1) getrennt. Nach dem Einengen erhält man ein farbloses Öl.
- Ausbeute:: 0.030 g (0.025 g, 0.048 mmol, 47 %), farbloses Öl (M_{w} = 516.6)
- DC: R_{f} = 0.38 (Chloroform/Methanol = 9/1)
- 1H-NMR:: (200.13 MHz, CDCl₃, TMS)
ä 6.90 (bs, 1H), 6.83 [d, J = 8.1 Hz, 1H), 6.60 - 6.71 (m, 1H), 5.88 - 6.08 (m, 2 H), 5.39 (bs, 1H), 5.19 (bs, 1H), 4.50 - 4.70 (m, 2 H), 4.04 - 4.34 (m, 3 H), 3.82 (s, 3 H), 3.70 (d, J = 6.1 Hz, 1H), 3.65 (d, *J* = 5.6 Hz , 1H), 3.11 - 3.49 (m, 5 H), 2.68 (d, *J* = 15.9 Hz, 1H), 2.41 (d, J = 10.9 Hz , 1H), 1.65 - 2.09 (m, 3 H), 1.44 (s, 9 H)
- HPLC:: t_{Ret} = 15.2 min, 82.3 % (Merck Purospher-Säule, 4.0 mm x 125 mm, RP-18e, 5.0 µm, 1 ml/min, 285 nm, MeOH/ H₂O (5/95 für 5 min, 5/95 D 100/0 in 15 min (konvex), 100/0 für 10 min, v/v)
- LC/MS:: f_{Ret} = 30.6 min, (Phenomenex Luna-Säule, 3.0 mm x 50 mm, RP-18, 3.0 µm, 0.8 ml/min, Methanol/H₂O (10/90 für 2 min, 10/90 □ 100/0 in 15 min, 100/0 für 5 min, v/v) APCl-PI-MS
517 (17), 499 (5), 461 (55), 443 (39), 417 (100), 399 (18), 274 (43), 256 (16)

### Beispiel 160

### SPH-1524

### (4aS,6R,8aS)-6-(Benzoyloxy)-3-methoxy-5,6,9,10-tetrahydro-4aH-[1]benzofuro[3a,3,2-ef][2]benzazepin-11(12H)-yl)carbonsäureallylester (CK-65-1)

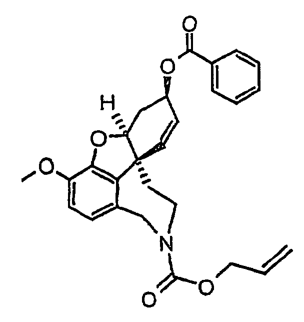

0.075 g (0.210 mmol) N-Allyloxycarbonylnorgalanthamin, 0.475 g (2.098 mmol) Benzoesäureanhydrid und 0.013 g (0.105 mmol) Dimethylaminopyridin werden in 2 ml Dichlormethan vorgelegt und anschließend mit 0.185 ml (0.136 g, 1.049 mmol) Ethyldiisopropylamin versetzt. Nach 13 Stunden bei Raumtemperatur wird die Lösung mit 5 ml gesättigter Natriumhydrogencarbonatlösung aufgenommen, dreimal mit jeweils 5 ml Essigsäureethylester extrahiert. Die vereinigten organischen Extrakte werden mit 10 ml gesättigter Natriumhydrogencarbonatlösung, zweimal mit 10 ml 2 N Salzsäure und zweimal mit 10 ml gesättigter Natriumchlorid-Lösung gewaschen, über Natriumsulfat getrocknet, filtriert und im Rotationsverdampfer unter verminderten Druck eingeengt. Der Rückstand wird zweimal jeweils 10 ml Petrolether digeriert, und die Lösung wird abdekantiert. Der Rückstand (0.100 g) wird durch Säulenchromatographie (10 g Kieselgel, Laufmittel = Petrolether/Essigsäureethylester = 2/1) gereinigt.
- Ausbeute:: 0.066 g (0.080 mmol, 68 %), farbloser Schaum (M_{w} = 461.5)
- DC:: R_{f} = 0.52 (Essigsäureethylester/Petrolether = 2/1)
- mp.:: 45 - 49°C (Essigsäureethylester/Petrolether = 2/1)
- IR: KBr: ν (cm⁻¹) 2946 (m), 1708 (s), 1509 (m), 1483 (m), 1276 (s), 1108 (m), 1056 (m), 714 (m)
- ¹H-NMR:: (200.13 MHz, CDCl₃, TMS)
δ 8.06 (d, *J* = 7.1 Hz, 1H), 7.29 - 7.61 (m, 3 H), 6.59 - 6.84 (m, 2 H), 6.28 (d, *J* = 10.3 Hz, 5 H), 6.07 (dd, *J* = 5.0 Hz, *J* = 10.2 Hz, 1H), 5.76 - 5.99 (m, 1H), 5.59 (t, *J* = 4.4 Hz, 1H), 5.09 - 5.35 (m, 2 H), 4.87 (dd, *J* = 15.7 Hz, J = 22.0 Hz, 1H), 4.05 - 4.70 (m, 5 H), 3.89 (s, 3 H), 3.29 - 3.60 (m, 1H), 2.81 (bd, *J* = 16.1 Hz, 1H), 1.74 - 2.25 (m, 3 H)
- ¹³C-NMR:: (50.32 MHz, CDCl₃, TMS)
δ 166.2 (s), 155.3 und 155.1 (s), 147.4 und 147.3 (s), 144.3 (s), 132.99 und 132.85 (d), 131.7 (d), 131.4 und 131.0 (s), 130.4 (s), 129.9 (d), 128.4 (d) 127.1 (d), 120.7 und 120.2 (d), 117.4 und 116.8 (t), 111.4 und 111.3 (d), 86 (d), 66.1 und 66.0 (t), 63.4 (d), 56.0 (q), 63.8 (d), 51.9 und 51.4 (t), 48.3 (s), 45.8 und 45.4 (t), 37.9 und 37.0 (t), 27.8 (t)

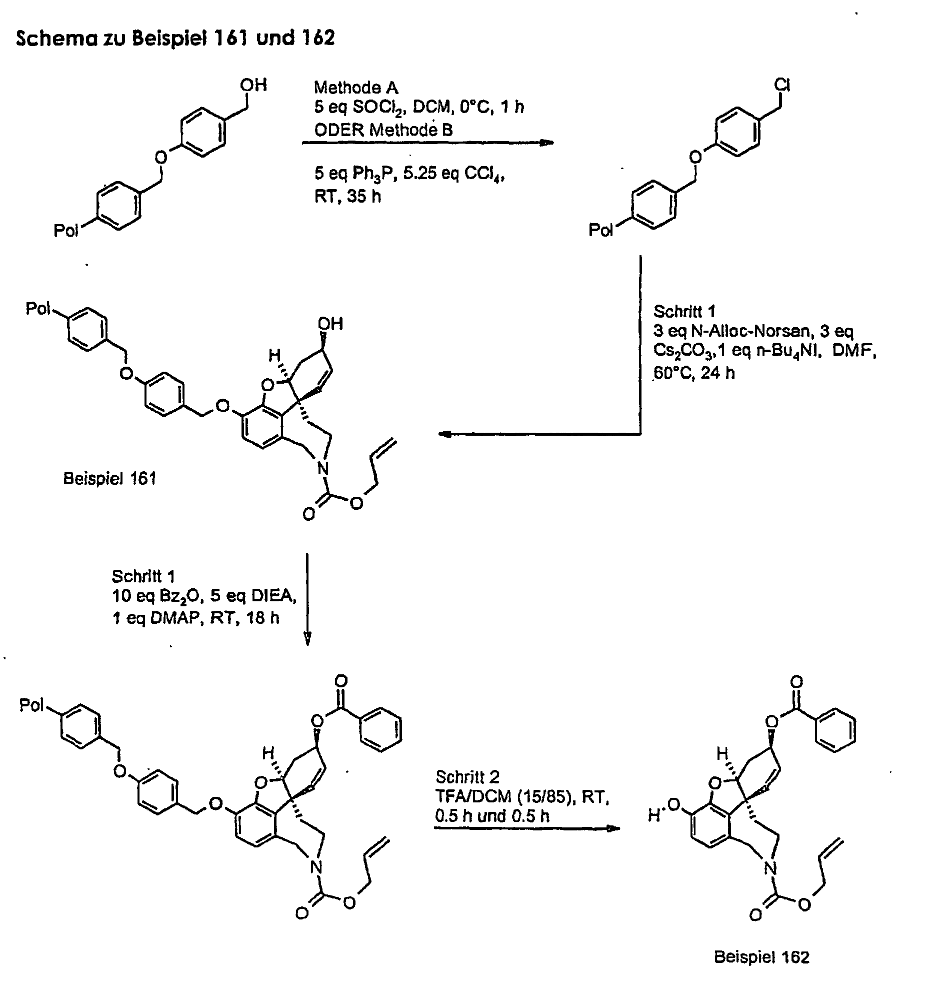

### Beispiel 161

### SPH-1525

### Immobilisierung von (4aS,6R,8aS)-3,6-Dihydroxy-5,6,9,10-tetrahydro-4aH-[1]benzofuro[3a,3,2-ef]-[2]benzazepin-11(12H)-yl)carbonsäureallylester an einem para-Hydroxymethylphenoxy-Polystyroiharz (Wang-Harz)

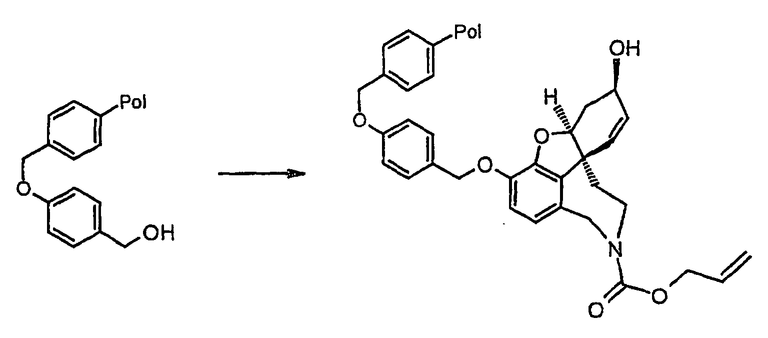

### Methode A (CK-63-2)

0.257 g (0.244 mmol) Wang-Harz¹ werden unter Argonatmosphäre in 3 ml absolutem Dichlormethan für 15 Minuten gerührt. Anschließend tropft man innerhalb von fünf Minuten bei 0°C 86 µl (0.141 g, 1.19 mmol) Thionylchlorid zu. Die Suspension wird bei 0°C eine Stunde lang gerührt. Danach wird das Harz in eine beidseitig verschließbare Polyethylenfritte überführt, fünfmal mit Dichlormethan (2 min, 2.5 ml), zweimal mit Methanol (2 min, 2.5 ml), einmal mit Dichlormethan (2 min, 2.5 ml) und einmal mit Diethylether (2 min, 2.5 ml) gewaschen. Das Harz wird anschließend im Vakuum über Phosphorpentoxid getrocknet.
¹ *P*-Alkoxybenzylalkohol-Resin, D-1250, Bachem Feinchemikalien AG

### Schritt 1

0.2334 g des so hergestellten Chlor-Wang-Harzes, 0.232 g (0.713 mmol) Cäsiumcarbonat, 0.088 g (0.238 mmol) Tetra-n-butylammoniumjodid und 0.245 g (0.713 mmol) N-Alloc-Norsanguinin werden in 3 ml absolutem Dimethylformamid unter Rühren suspendiert. Anschließend rührt man die Suspension für 24 Stunden bei 60°C. Nach fünf Stunden fügt man 1 ml Dimethylformamid hinzu, um den ausgefallenen Niederschlag zu suspendieren. Danach wird das Harz eine beidseitig verschließbare Polyethylenfritte mit Dimethylformamid/Wasser-Lösung (2/1) überführt, zweimal mit Dimethylformamid/Wasser-Lösung (2/1, 2 min, 2.5 ml), zweimal mit Dimethylformamid/Wasser-Lösung (1/2, 2 min, 2.5 ml), zweimal mit Methanol/Wasser-Lösung (1/1, 2 min, 2.5 ml), zweimal mit Methanol (2 min, 2.5 ml) und sechsmal mit Dichlormethan (2 min, 2.5 ml) gewaschen.

### Methode B (CK-63-1)

0.121 ml (0.192 g, 1.247 mmol) Tetrachlorkohlenstoff werden in 2.0 ml absolutem Dichlormethan bei 0°C vorgelegt. Unter Rühren (KPG-Rührer) werden 0.311 g (1.188 mmol) Triphenylphosphin, gelöst in 1.0 ml absolutem Dichlormethan, innerhalb von 15 Minuten bei 0°C zugetropft. Man rührt weitere zehn Minuten bei 0°C und dann weitere zehn Stunden bei Raumtemperatur, bis man dünnschichtchromatographisch kein Triphenylphosphin mehr nachweisen kann. Nachfolgend werden 0.2506 g (0.238 mmol) Wang-Harz¹ und 1.5 ml Dichlormethan zugefügt, und die Suspension wird für 35 Stunden bei Raumtemperatur gerührt (300 s⁻¹). Die Aufarbeitung des chlorierten Wang-Harzes erfolgt analog der Methode A. Nach dem Trocknen erhält man 0.2403 g Chlor-Wang-Harz, welches analog dem obigen Schritt 1 mit den gleichen Stoffmengen an Cäsiumcarbonat, Tetra-n-butylammoniumjodid und N-Alloc-Norsanguinin umgesetzt wird.

### Beispiel 162

### SPH-1526

### Feststellung der Beladung, (4aS,6R,8aS)-6-(Benzoyloxy)-3-hydroxy-5,6,9,10-tetrahydro-4aH-[1]benzofuro[3a,3,2-ef][2]benzazepin-11(12H)-yl)carbonsäureallylester

### 1.1.1 Schritt 1 (CK-63-2)

Zur Feststellung der Beladung und zum Nachweis, daß das N-Alloc-Norsanguinin über die Phenolfunktion an das Harz gebunden wird, wird das Harz, welches durch die Methode A und Schritt 1 erhalten wird, sukzessive mit einer Lösung bestehend aus 0.537 g (2.375 mmol) Benzoesäureanhydrid und 2 ml Dichlormethan und einer Lösung bestehend aus 0.015 g (0.119 mmol) Dimethylaminopyridin, 0.203 ml (0.154 g, 1.188 mmol) Ethyldiisopropylamin und 0.5 ml Dichlormethan versetzt. Anschließend schüttelt man die Suspension bei Raumtemperatur. Nach 18 Stunden wird das Harz abfiltriert, sechsmal mit jeweils 2.5 ml Dichlormethan (2 min) und einmal mit 2.5 ml Diethylether (2 min) gewaschen und im Vakuum über Phosphorpentoxid getrocknet. Man erhält 0.3085 g substituiertes Harz, weiches in absolutem Dichlormethan für 15 Minuten suspendiert wird und anschließend gut filtriert wird.

### Schritt 2

Zur Abspaltung wird das Harz mit 2.5 ml Trifluoressigsäure/Dichlormethan-Lösung (15/85) versetzt, 30 Minuten bei Raumtemperatur geschüttelt, filtirert und nochmals mit 2.5 ml Trifluoressigsäure/Dichlormethan-Lösung (15/85)in geschüttelt, filtriert und abschließend viermal mit jeweils 2.5 ml Dichlormethan extrahiert. Die vereinigten Filtrate bzw. Extrakte werden mit 10 ml aqua dest aufgenommen und mit Natriumhydrogencarbonat auf pH 6 eingestellt. Die Phasen werden separiert, und die wäßrige Phase wird dreimal mit 10 ml Dichlormethan extrahiert. Die vereinigten organischen Extrakte werden zweimal mit jeweils 10 ml gesättigter Natriumchlorid-Lösung gewaschen, über Natriumsulfat getrocknet, filtriert und konzentriert. Die Reinigung des Rohproduktes (0.078 g) erfolgt mittels Säulenchromatographie (10 g Kieselgel; Essigsäureethylester/Petrolether = 2/1). Nach dem Einengen und Trocknen im Hochvakuum erhält man 0.078 g eines farblosen Schaumes, der laut HPLC-Analyse 95 % rein ist. Der farblose Schaum kann durch Umkristallisation aus Diethylether bei -22°C (Eisfach vom Kühlschrank) gereinigt werden.
- Ausbeute:: 0.059 g (0.056 , 0.0125 mmol, Somit errechnet sich eine Beladung von 0.41 mmol/g, 61 % der theoretischen Maximalbeladung¹⁾, farbloser Schaum (M_{w} = 447.5)
- DC:: R_{f} = 0.29 (Essigsäureethylester/Petrolether = 1/1)
- mp.:: 158-159°C (Diethylether)
- ¹H-NMR:: (200.13 MHz, d₆-DMSO)
δ 7.99 (d, *J* = 6.5 Hz, 2 H), 7.38 - 7.71 (m, 3 H), 6.39 - 6.63 (m, 3H), 5.73 - 6.17 (m, 2 H), 5.47 (t, *J* = 4.3 Hz, 1H), 5.04 - 5.33 (m, 2 H), 4.00 - 4.72 (m, 6 H), 2.60 - 2.89 (m, 1H), 2.55
(bd, *J* = 18.6 Hz, 1H) 2.25 (bd, *J* = 15.7 Hz, 1H), 1.70 - 1.93 (m, 2 H)
- ¹³C-NMR:.: (50.32 MHz, d₆-DMSO)
δ 165.4 (s), 154.6 und 154.4 (s), 146.3 (s), 141.3 (s), 133.5 (d), 133.3 (d), 133.2 (d), 131.8 (d), 131.5 (s), 130.2 (s), 129.5 (d), 128.6 (d), 128.0 (s), 122.2 (d), 120.4 und 120.0 (d), 117.0 und 116.1 (t), 115.0 (d), 84.7 (d), 65.2 und 65.0 (t), 63.8 (d), 51.0 und 50.5 (t), 48.0 (s), 45.1 und 44.7 (t), 37.3 und 36.4 (t), 27.4 (t)
- HPLC:: t_{Ret} = 21.41 min, 95.1 % (Merck Purospher-Säule, 4.0 mm x 125 mm, RP-18e, 5 µm, 285 nm, 1 ml/min, Acetonitril/ 20 mM Cl₃CCO₂H in H₂O (15/85 für 5 min, 15/85 → 60/40 in 12 min, 60/40 für 5 min, v/v)

¹ 0.67 mmol/g = 0.95 mmol/g / (1 g + 1 g * 0.95 mol/g * (447.5 g/mol - 18 g/mol)/1000)

### (CK-63-1)

Das Harz, welches durch die Methode B und Schritt 1 erhalten wird, wird analog Schritt 1 mit Benzoesäureanhydrid umgesetzt, und nach dem Trocknen unter Vakuum erhältman 0.3004 g des substituierten Harzes. Die Abspaltung des Produktes und die wäßrige Aufarbeitung erfolgen analog Schritt 2. Nach dem Einengen und dem Trocknen im Vakuum erhält man 0.070 g Rohprodukt, welches mittels Säulenchromatographie (10 g Kieselgel, Essigsäureethylester/Petrolether = 2/1) gereinigt wird. Nach dem Einengen und Trocknen im Hochvakuum erhält man 0.051 g eines farblosen Schaumes, der laut HPLC-Analyse 93 % rein ist.
- Ausbeute:: 0.051 g (0.047 g, 0.0106 mmol, Somit errechnet sich eine Beladung von 0.35 mmol/g, 52 % der theoretischen Maximalbeladung²⁾, farbloser Schaum (M_{w} = 447.5)
- HPLC:: t_{Ret} = 21.41 min, 92.7 % (Merck Purospher-Säule, 4.0 mm × 125 mm, RP-18e, 5 µm, 285 nm, 1 ml/min, Acetonitril/ 20 mM Cl₃CCO₂H in H₂O (15/85 für 5 min, 15/85 → 60/40 in 12 min, 60/40 für 5 min, v/v)

² 0.67 mmol/g = 0.95 mmol/g / (1 g + 1 g * 0.95 mol/g * (447.5 g/mol - 18 g/mol)/1000)

### Beispiel 163: Synthese an der festen Phase / manuell

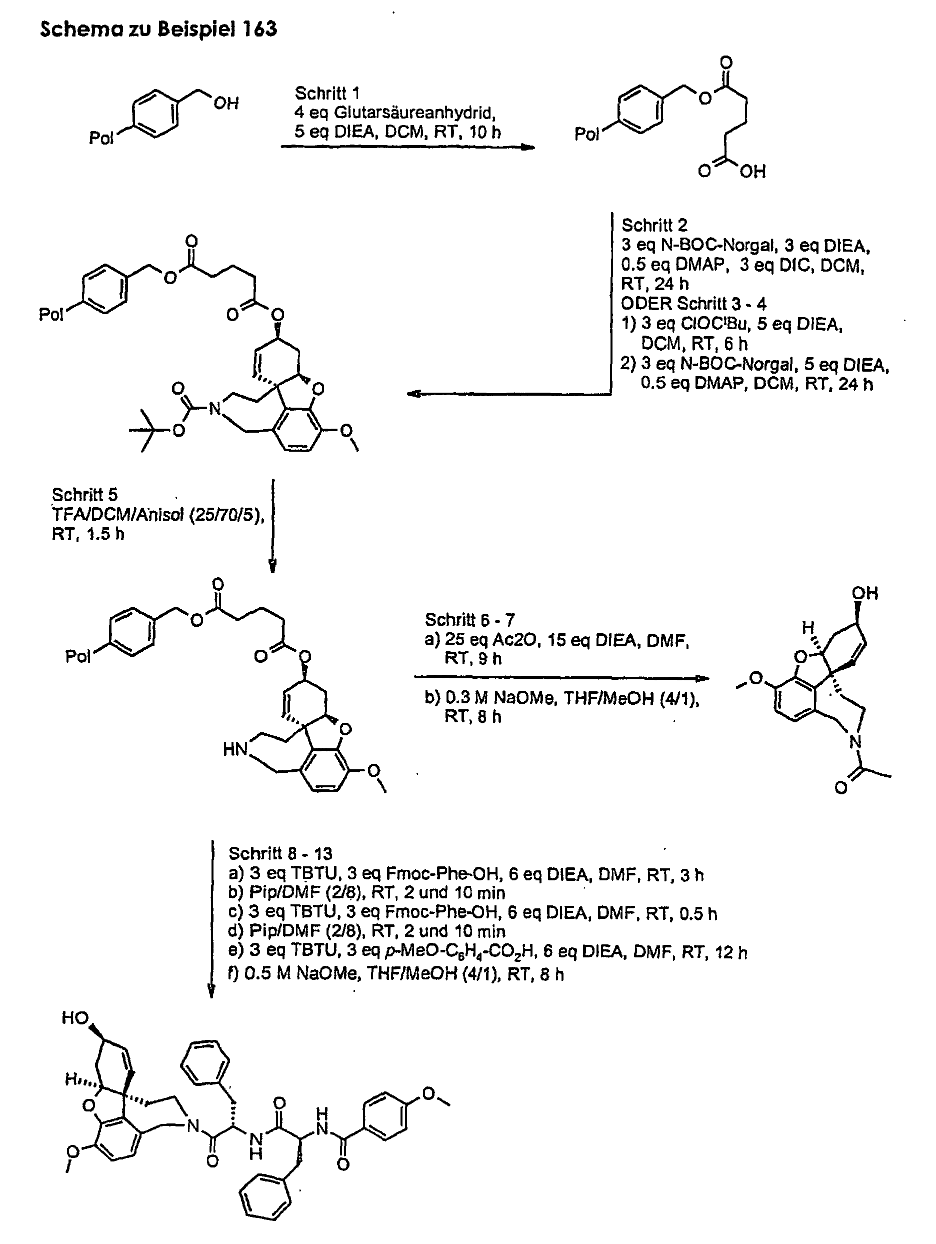

### Beispiel 163/Schritte 1 - 7

### Immobilisierung von (4aR,6S,8aR)-3-Methoxy-5,6,9,10,11,12-hexahydro-4aH-[1]benzofuro[3a,3,2-ef][2]benzazepin-6-ol an einem Hydroxymethyl-Polystyrolharz (Merrifield-Harz)

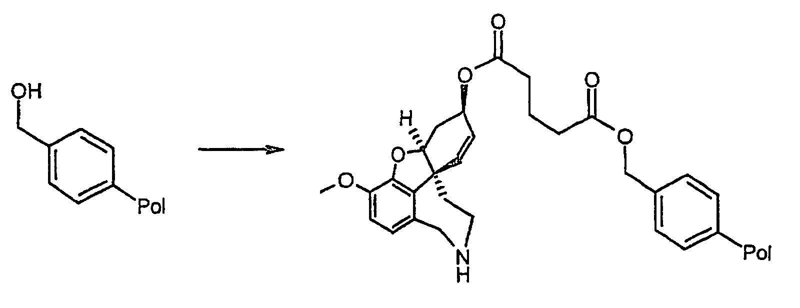

### Methode A (CK-41-1) Schritte 1 - 2 und 5 - 7

5.000 g (5.2 mmol) Hydroxymethyl-Merrifieldharz¹ werden in einem Dreihalsglasreaktor mit einer am Boden eingelassenen Fritte und KPG-Rührer in 60 ml Dichlormethan für eine Stunde gerührt (300 s⁻¹). Nach dem Filtrieren wird die Suspension mit einer Lösung von 2.373 g (20.8 mmol) Glutarsäureanhydrid, 4.45 ml (3.362 g, 26.0 mmol) Ethyldiisopropylamin in 30 ml absolutem Dichlormethan versetzt. Die Suspension wird bei Raumtemperatur zehn Stunden gerührt, gefiltert, einmal mit 50 ml Dichlormethan, einmal mit 50 ml Methanol und dreimal mit jeweils 50 ml Dichlormethan gewaschen. Das Harz wird anschließend im Vakuum getrocknet.
4 Hydroxymethyl-Resin, D-1160, Bachem Feinchemikalien AG

4.385 g des so hergestellten 4-Carboxy-1-oxobut-1-yloxymethyl-Merrifield-Harz werden für 30 Minuten in 50 ml absolutem Dichlormethan unter Rühren suspendiert und filtriert. Anschließend fügt man 4.569 g (12.23 mmol) N-*tert*.-Butoxycarbonylnorgalanthamin und 0.249 g (2.04 mmol) Dimethylaminopyridin, beides gelöst in 15 ml Dichlormethan, und 2.10 ml (1.582 g, 12.23 mmol) Ethyldiisopropylamin, gelöst in 5 ml Dichlormethan, zu. Unter Rühren tropft man eine Lösung aus 1.89 ml (1.544 g, 12.23 mmol) Diisopropylcarbodiimid und 5 ml Dichlormethan innerhalb von fünf Minuten bei Raumtemperatur zu. Nach 24 Stunden wird das Harz abfiltriert, mit 40 ml Methanol gewaschen und filtriert. Dann wird das Harz sechsmal mit jeweils 40 ml Dichlormethan (5 min) gewaschen und im Vakuum getrocknet.

Das Harz wird mit 42 ml absolutem Dichlormethan und 3 ml Anisol versetzt und 30 Minuten gerührt (150 s⁻¹) gerührt. Anschließend werden unter Rühren (300 s⁻¹) 15 ml Trifluoressigsäure innerhalb von 15 Minuten hinzugefügt. Anschließend wird die Suspension 60 Minuten gerührt (150 s⁻¹), filtriert und mit Dichlormethan (2 x 40 ml, 5 min), mit Dichlormethan/Dimethylformamid/Triethylamin (5/4/1) (3 x 40 ml, 5 min) und abschließend mit Dichlormethan (5 x 40 ml, 5 min) gewaschen. Nach dem Trocknen im Vakuum erhält man 4.353 g Harz.
Zur Feststellung der Beladung werden 0.2384 g des Harzes in 3 ml Dimethylformamid i n einer beidseitig verschließbaren Polyethylenfritte suspendiert. Nach dem Filtern wird das Harz in einer Lösung aus 177 µl (0.191 g, 1.875 mmol) Essigsäureanhydrid, 180 µl (0.136 g, 1.050 mmol) Ethyldiisopropylamin und 2 ml Dimethylformamid neun Stunden bei Raumtemperatur geschüttelt. Nach dem Waschen (Dimethylformamid (3 × 2.5 ml, 2 min) und Tetrahydrofuran/Methanol (4/1) (5 × 2.5 ml, 2 min) zeigt das Harz mit dem Chloranil-Test keine Reaktion. Man suspendiert das Polymer in einer 0.3 M Natriummethanolat-Lösung in Tetrahydrofuran/Methanol (4/1). Das Harz wird 8 Stunden bei Raumtemperatur geschüttelt, filtriert und mit Methanol/Dichlormethan (1/1, 3 × 2.5 ml) und mit Dichlormethan (3 × 2.5 ml) extrahiert. Die vereinigten Filtrate werden mit methanolischer Salzsäure neutralisiert. Die Lösung wird mit ca. 10 ml Dichlormethan verdünnt, mit jeweils 25 ml ges. Natriumhydrogencarbonat-Lösung, 1 N Salzsäure und gesättigter Natriumchlorid-Lösung gewaschen, über Natriumsulfat getrocknet, filtriert und konzentriert. Die Reinigung erfolgt mittels Säulenchromatographie (10 g Kieselgel, Diethylether/Ethanol = 100/15). Nach dem Einengen und Trocknen im Hochvakuum erhält man 0.028 g eines gelblichen Schaumes, der laut HPLC-Analyse 10 % andere Verunreinigungen enthält.
- Ausbeute:: 0.025 g (0.080 mmol, Somit errechnet sich eine Beladung von 0.34 mmol/g, 45 % der theoretischen Maximalbeladung¹⁾, gelblicher Schaum (M_{w} = 315.4)
- DC:: R_{f} = 0.29 (Diethylether/Ethanol = 100/15)
- IR:: KBr
v (cm⁻¹) 3551 (v), 3305 (bm), 2926 (m), 2864 (v), 1650 (m), 1615 (s), 1443 (m), 1257 (m), 1070 (m)
- ¹H-NMR:: (200.13 MHz, CDCl₃, TMS)
δ 6.54 - 6.89 (m, 2 H), 5.75 - 6.09 (m, 2 H), 5.14 - 5.33 (m, 0.3 H), 4.32 - 4.74 (m, 3.3 H), 4.12 (bs, 1H), 3.86 - 4.00 (m, 0.3 H), 3.81 und 3.79 (s, 3 H), 3.56 - 3.76 (m, 0.8 H), 3.23 (bt, *J* = 12.6 Hz, 0.8 H), 2.67 (bd, *J* = 15.9 Hz, 1H), 2.38 (bs, 0.7 H), 2.06 (m, 3 H), 1.62 - 2.00 (m, 3 H),
- HPLC:: t_{Ret} = 13.9 min, 89.8 % (Merck Purospher-Säule, 4.0 mm × 125 mm, RP-18e, 5 µm, 285 nm, 1 ml/min, Acetonitril/ 20 mM Cl₃CCO₂H in H₂O (05/95 für 5 min, 05/95 → 60/40 in 10 min, 60/40 für 10 min, v/v, pH 10)

¹ 0.75 mmo)/g = 1.04 mmol/g / (1 g + 1 g * 1.04 mol/g * (387.4 g/mol - 18 g/mol)/1000)

### Methode B (CK-43-2), Schritte 1 und 3 - 7

3.500 g (3.64 mmol) Hydroxymethyl-Merrifield-Harz² werden analog der unter Methode A beschriebenen Methode mit 1.661 g (14.6 mmol) Glutarsäureanhydrid, 3.15 ml (2.378 g, 18.39 mmol) Ethyldiisopropylamin und 0.044 g (0.364 mmol) Dimethylaminopyridin in 20 ml absolutem Dichlormethan umgesetzt. Nach dem Waschen wird den Harz sukzessive mit einer Lösung aus 3.15 ml (2.378 g, 18.39 mmol) Ethyldiisopropylamin und 10 ml absolutem Dichlormethan und mit einer Lösung aus 1.34 ml (1.317 g, 10.92 mmol) Pivaloylchlorid und neun Stunden bei Raumtemperatur gerührt. Nach dem Waschen (Dichlormethan (4 x 30 ml, 5 min), Tetrahydrofuran (30 ml, 5 min) und Dichlormethon (2 x 30 ml, 5 min) wird das Harz sukzessive mit 4.061 g (10.87 mmol) N-*tert*.-Butoxycarbonylnorgalanthamin und 0.222 g (1.82 mmol) Dimethylaminopyridin, beides gelöst in 15 ml Dichlormethan, und 3.15 ml (2.378 g, 18.39 mmol) Ethyldiisopropylamin, gelöst in 15 ml Dichlormethan, zu. Nach 24 Stunden bei Raumtemperatur wird die Lösung abfiltriert, und das Harz mit 40 ml einer trockenen Lösung aus Methanol und Dichlormethan versetzt und zehn Minuten bei Raumtemperatur gerührt. Noch dem Filtrieren, dem sechsmaligen Waschen mit Dichlormethan und dem Trocknen im Vakuum wird analog der Methode A das Harz mit 40 ml Trifluoressigsäure/Dichlormethan/Anisol (25/70/5), gewaschen und getrocknet. Man erhält 4.145 g eines gelben Harzes. Davon werden 0.2214 g des Harzes zur Bestimmung der Beladung analog der Methode umgesetzt und gereinigt. Man erhält 0.024 g eines gelblichen Schaumes, der laut HPLC-Analyse 90 % rein ist.
- Ausbeute:: 0.022 g (0.069 mmol, Das entspricht einer Beladung von 0.31 mmol/g, 41 % der theoretischen Maximalbeladung)
- HPLC:: t_{Ret} = 14.4 min, 89.0 % (Merck Purospher-Säule, 4.0 mm x 125 mm, RP-18e, 5 µm, 285 nm, 1 ml/min, Acetonitril/ 20 mM Cl₃CCO₂H in H₂O (05/95 für 5 min, 05/95 → 60/40 in 10 min, 60/40 für 10 min, v/v, pH 10)

² Hydroxymethyl-Resin, D-1160, Bachem Feinchemikalien AG

### Belspiel 163/Schritte 8 - 13

### SPH-1528

### N-P-METHOXYBENZOYL-PHENYLALANYL-PHENYLALANIN-((4AS,6R,8AS)-6-HYDROXY-3-METHOXY-5,6,9,10-TETRAHYDRO-4AH-[1]BENZOFURO[3A,3,2-EF][2]BENZAZEPIN-11(12H)-YL)AMID(CK-47-1)

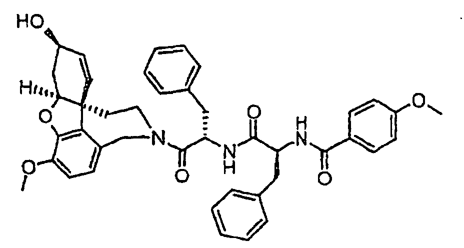

0.245 g (0.076 mmol) N-*tert*.-Butoxycarbonylnorgalanthamin-6-yloxy-1,5-dioxopentyloxymethyl-Merrifieldharz werden in einer beiderseits verschlossenen 5-ml-Polyethylenfritte zweimal 15 Minuten in 3 ml Dimethylformamid gequollen und nach dem Filtrieren in einer Lösung von 0.065 g (0.169 mmol) Fmoc-Phenylalanin und 0.062 g (0.169 mmol) 2-(1H-Benzotriazol-1-yl)-1,1,3,3-tetramethyluroniumtetrafluoroborat in jeweils 1 ml Dimethylformamid suspendiert und fünf Minuten bei Raumtemperatur geschüttelt. Anschließend fügt man 58 µl (0.044 g, 0.338 mmol) Ethyldiisopropylamin in 0.5 ml Dimethylformamid zu. Die Suspension wird bei Raumtemperatur 3 Stunden geschüttelt. Das Harz wird sechsmal mit Dimethylformamid (1 min, 2.5 ml) gewaschen und in einer 20 %gen Piperidin-Lösung in Dimethylformamid für jeweils zwei und zehn Minuten bei Raumtemperatur suspendiert. Nach sechsmaligen Waschen mit Dimethylformamid (1 min, 2.5 ml) wird das Harz analog dem ersten Peptidkupplungsschritt mit 0.065 g (0.169 mmol) Fmoc-Phenylalanin und 0.062 g (0.169 mmol) 2-(1H-Benzotriazol-1-yl)-1,1,3,3-tetramethyluroniumtetrafluoroborat in jeweils 1 ml Dimethylformamid und 58 µl (0.044 g, 0.338 mmol) Ethyldiisopropylamin in 0.5 ml Dimethylformamid umgesetzt, mit Dimethylformamid gewaschen, in 20 %ger Piperidin-Dimethylformamid-Lösung geschüttelt und wiederum sechsmal mit Dimethylformamid (1 min, 2.5 ml) gewaschen. Anschließend gibt man sukzessive Lösungen von 0.065 g (0.169 mmol) Fmoc-Phe-OH in 1 ml Dimethylformamid, 0.062 g (0.169 mmol) 2-(1H-Benzoiriazol-1-yl)-1,1,3,3-tetramethyluroniumtetrafluoroborat in 1 ml Dimethylformamid und 58 µl (0.044 g, 0.338 mmol) Ethyldiisopropylamin in 0.5 ml Dimethylformamid zu, und schüttelt die Suspension 30 Minuten bei Raumtemperatur. Der Kaiser-Test zeigt vollständige Umsetzung an. Anschließend wird analog der oben beschriebenen Prozedur gewaschen, in Piperidin-Dimethylformamid-Lösung suspendiert und wiederum mit Dimethylformamid gewaschen. Nach erneuter sukzessiver Zugabe von 0.026 g (0.169 mmol) *p*-Methoxybenzoesäure in 1 ml Dimethylformamid, 0.062 g (0.169 mmol) 2-(1*H*-Benzotriazol-1-yl)-1,1,3,3-tetramethyluroniumtetrafluoroborat in 1 ml Dimethylformamid und 58 µl (0.044 g, 0.338 mmol) Ethyldiisopropylamin in 0.5 ml Dimethylformamid wird das Harz über Nacht geschüttelt, da der Kaiser-Test nach 30 Minuten aufgrund einer grünbläulichen Färbung nicht eindeutig gewesen ist. Das Harz wird filtriert, dreimal mit Dimethylformamid, fünfmal mit Dichlormethan und mit Methanol gewaschen und anschließend im Vakuum getrocknet. Das Harz (0.251 g) wird für 30 Minuten in THF suspendiert, filtriert und in einer Lösung aus 0.250 g (1.39 mmol) 30 %ger Natriummethanolat-Methanol-Lösung und 2.5 ml Tetrahydrofuran/Methanol (4/1) suspendiert. Nach acht Stunden wird die Lösung abfiltriert, und das Harz sechsmal mit jeweils 3 ml Dichlormethan/Methanol extrahiert. Die vereinigten Filtrate werden mit 106 µl (0.158 g, 1.39 mmol) Trifluoressigsäure neutralisiert, konzentriert und mit präparativer Dünnschichtchromatographie (Kieselgel, Chloroform/Methanol = 100/5) getrennt. Die podukthaltige Kieselgelfraktion wird mit Chloroform/Methanol (9/1) extrahiert, und die vereinigten Extrakte werden nochmals über einen Filter (Porendurchmesser 0.2 µm) gefiltert, eingeengt und im Vakuum getrocknet.
- Ausbeute:: 0.041 g (0.029 g, 0.041 mmol, 53 %), rosafarbenes Wachs (M_{w} = 701.8), Das Produkt enthält ein Nebenprodukt (0.006 g, 0.008 mmol, 10 %), daß laut LC/MS ein Diastereomer des Hauptprodukt ist.
- DC: R_{f} = 0.43 (Chloroform/Methanol = 20/1)
- HPLC:: t_{Ret} = 2.7 min, 70.4 % (Phenomenex Luna-Säule, 3.0 mm x 50 mm, RP-18, 3.0 µm, 0.5 ml/min, 285 nm, Methanol/ 20 mM Cl₃CCO₂H in H₂O (30/70 v/v)
- LC/MS:: t_{Ret} = 30.6 min, 73 %, (Phenomenex Luna-Säule, 3.0 mm x 50 mm, RP-18, 3.0 µm, 0.5 ml/min, Methanol/H₂O (5/95 v/v für 2 min, 5/95 □ 40/60 v/v in 15 min, 40/60 v/v für 5 min)
APCl-PI-MS
702 (56), 421 (100), 274 (6), 256 (13)
APCl-NI-MS
700
t_{Ret} = 35.6 min, 8 %, (diastereomere Nebenprodukt)
APCI-PI-MS
702 (56), 421 (100), 274 (6), 256 (13)
APCI-NI-MS
700

### BEISPIEL 163 - 167: SYNTHESE AM ROBOTER

### N-P-CARBOXYL-DIPEPTOYL-((4AS,6R,8AS)-6-HYDROXY-3-METHOXY-5,6,9,10,-TETRAHYDRO-4AH-[1]BENZOFURO[3A,3,2-EF][2]BENZAZEPIN-11(12H)-YL)AMID (CK-59-1)

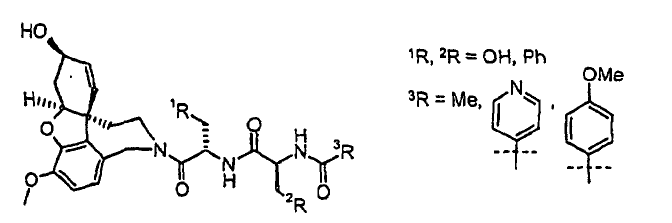

Jeweils 0.300 g (0.102 mmol) Norgalanthamin-6-yloxy-1,5-dioxopentyloxymethyl-Merrlfieldharz werden in Polyethylenreaktoren mit Fritte eines Syntheserobotors (Syro II MultiSynTech) vorgelegt und mit 3 ml absolutem Dimethylformamid versetzt. Nach fünfminutlgen Stehen werden die Suspensionen 25 Minuten bei 23°C in Intervallen gerührt. Alle folgenden Operationen erfolgen bei dieser Temperatur. Danach werden die Harze einmal mit 3 ml Dimethylformamid gewaschen und sukzessive mit einer Lösung bestehend aus 0.119 g (0.306 mmol) Fmoc-Phenylalanin bzw. 0.117 g (0.306 mmol) N-Fmoc-O-*tert*.-Butylserin und 1.5 ml Dimethylformamid und mit einer Lösung aus 0.112 g (0.306 mmol) 2-(1*H*-Benzotriazol-1-yl)-1,1,3,3-tetramethyluroniumtetrafluoroborat und 1 m) Dimethylformamid versetzt. Anschließend werden die Harzsuspensionen fünt Minuten gerührt. Dann fügt man 105 µl (0.079 g, 0.612 mmol) Ethyldiisopropylamin in 0.5 ml Dlmethylformamid zu. Die Suspensionen werden 3 Stunden gerührt, für zwei Minuten abgesaugt und jeweils mit einer Lösung bestehend aus 241 µl (0.260 g, 2.550 mmol) Essigsäureanhydrid, 437 µl (0.330 g, 2.550 mmol) Ethyldiisopropylamin und 3 ml Dimethylformamid versetzt. Nach 15-minütigen Rühren und zweiminütigen Abfiltrieren werden die Harze sechsmal mit Dimethylformamid (3 ml, 2 min) gewaschen, in einer 20 %gen Piperidin-Lösung in Dimethylformamid für jeweils sechs und 15 Minuten suspendiert bzw. gerührt. Noch sechsmaligen Waschen mit Dimethylformamid (2 min, 3 ml) werden die Harze analog dem ersten Peptidkupplungsschritt mit Fmoc-Phenylalanln bzw. N-Fmoc-O-*tert*.-Butylserin umgesetzt, die nicht umgesetzten Aminfunktionen mit Essigsäureanhydrid maskiert und mit 20 %ger Piperidin-Dimethylformamid-Lösung die Fmoc-Schutzgruppe abgespalien. Anschließend wird analog der oben beschriebenen Prozedur gewaschen, mit Lösungen bestehend aus 0.047 g (0.306 mmol) p-Methoxybenzoesäure bzw. 0.038 g (0.306 mmol) Isonicotinsäure in 2.5 ml Dimethylformamid versetzt und fünf Minuten gerührt. Dann werden jeweils Lösungen von 48 µl (0.039 g, 0.306 mmol) Disopropylcarbodilmid 241 µl und 0.5 ml 1,2-Dichloroethan zugefügt. Es wird für drei Stunden gerührt. Die Harze werden filtriert (2 min), dreimal mit Dimethylformamid (2 min, 3 ml), sechsmal mit Dichlormethan (2 min, 3 ml) gewaschen, für zehn Minuten bei 40°C trocken gesaugt und anschließend im Vakuum über Phosphorpentoxid getrocknet. Der Kaiser-Test ist bei allen Harzen (0.250 bis 0.377 g) negativ.
Die Harze, die O-*tert*.-butylgeschützte Serin-Reste enthalten, werden sukzessive mit 1 ml Dichlormethan, 0.125 ml Phenol, 0.125 ml Anisol und 3.75 ml Trifluoressigsäure versetzt und drei Stunden bei Raumtemperatur gerührt. Dann werden die Lösungen abfiltriert, und die Harze werden dreimal mit Dichlormethan (2 min, 3 ml), dreimal mit Dichlormethan/Ethyldiisopropylamin (95/5, 2 min, 3 ml), dreimal mit jeweils Dichlormethan (2 min, 3 ml) und dreimal mit Tetrahydrofuran (2 min, 3 ml) gewaschen und unter verminderten Druck getrocknet.

Die Harze werden in beidseitig verschließbare 5 ml-Polyethylenfritten transferiert, für 30 Minuten in THF suspendiert, filtriert und in einer Lösung aus 0.092 g (0.028 g, 0.510 mmol) 30 %ger Natriummethanolat-Methanol-Lösung und 3 ml Tetrahydrofuran/Methanol (4/1) suspendiert. Nach sechs Stunden wird die Lösung abfiltriert, und das Harz fünfmal mit jeweils 3 ml Tetrahydrofuran/Methanol (4/1 ) extrahiert. Die vereinigten Filtrate werden mit Dowex 50W (eine Spatelspitze) und NaHCO₃ (eine Spatelspitze) neutralisiert. Die Suspensionen werden über etwas Cellite filtriert, die Filtercellite wird zweimal mit 5 ml Dichlormethan nachgespült. Die Filtrate werden konzentriert und mit präparativer Dünnschichtchromatographie (Laufmittel: Chloroform/Methanol = (10/1) für CK-49-1-IPP-3, CK-59-MPP-1, CK-59-IPP-2, CK-59-AcPP-3, Chloroform/Methanol = (4/1) für CK-59-IPP-4 und Chloroform/Methanol = (6/1) für CK-59-MPP-5) gereinigt. Die produkthaltigen Kieselgelfraktionen werden mit Chloroform/Methanol (9/1) extrahiert und filtriert. Die eingeengten Extrakte werden nochmals über einen Filter (Porendurchmesser 0.2 µm) gefiltert, weiter eingeengt und im Vakuum getrocknet.
- HPLC: Phenomenex Synergi Polar-RP-Säule, 4.6 mm × 150 mm, 4.0 µm, 1.0 ml/min, Methanol/ 20 mM Cl₃CCO₂H in H₂O (20/80 für 5 min, 20/80 □ 80/20 in 20 min, 80/20 für 10 min, v/v)
- LC/MS: Phenomenex Luna-Säule, 3.0 mm × 50 mm, RP-18, 3.0 µm, 0.8 ml/min, Methanol/H₂O (10/90 für 2 min, 10/90 □ 100/0 in 15 min, 100/0 für 5 min, v/v)

| Ergebnis | | | | | |
|---|---|---|---|---|---|
| | Beispiel | SPH-Nummer | Aminosäure 1 und 2 | Carboxylrest 3 | R_{f}-Wert |
| CK-59-MPP-1-1 | 163 | SPH-1528 | Ph, Ph | 4-MeOC₆H₄ | 0.60 (CHCl₃/CH₃OH = 10/1) |
| CK-59-MPP-1-2 | | | Ph, Ph | 4-MeOC₆H₄ | 0.45 (CHCl₃/CH₃OH = 10/1) |
| CK-49-1-IPP-3-1 | 166 | SPH-1528 | Ph, Ph | *p*-NC₅H₄ | 0.40 (CHCl₃/CH₃OH = 9/1) |
| CK-59-AcPP-3-1 | 164 | SPH-1528 | Ph, Ph | Me | 0.52 (CHCl₃/CH₃OH = 10/1) |
| CK-59-AcPP-3-2 | | | Ph, Ph | Me | 0.43 (CHCl₃/CH₃OH = 10/1) |
| CK-59-ISS-4-1 | 165 | SPH-1528 | OH, OH | p-NC₅H₄ | 0.22 (CHCl₃/CH₃OH = 6/1) |
| CK-59-ISS-4-2 | | | OH, OH | *p*-NC₅H₄ | 0.17 (CHCl₃/CH₃OH = 6/1) |
| CK-59-MSS-5-1 | 167 | SPH-1528 | OH, OH | 4-MeOC₆H₄ | 0.37 (CHCl₃/CH₃OH = 6/1) |
| CK-59-MSS-5-2 | | | OH, OH | 4-MeOC₆H₄ | 0.27 (CHCl₃/CH₃OH = 6/1) |

| Ausbeuten | | | | |
|---|---|---|---|---|
| Beispiel | Code | HPLC (t_{Ret}, Reinheit) | LC/MS | Ausbeute und Bemerkung |
| 163 | CK-59-MPP-1-1 | 29.90 min (71 %) | t_{Ref} = 10.90 min APCl, Neg 700 (100) APCl, Pos 702 (63), 684 (8), 421 (100), 274 (5), 256 (15) | 0.021 g (0.015 g, 0.021 mmol, 21 %) |
| | CK-59-MPP-1-2 | 29.35 min (24 %) | t_{Ref} = 10.90 min APCI, Neg 700 (100), 682 (7), 606 (13) APCl, Pos 702 (63), 684 (26), 608 (28), 421 (100), 274 (11), 256 (19) | 0.014 g (0.003 g, 0.005 mmol, 5 %); racemisiertes Produkt |
| 166 | CK-49-1-IPP-3-1 | 24.91 min (59 %) | t_{Ref} = 20.22 min APCI, Neg 671 (100) APCl, Pos 673 (100), 655 (18), 421 (7), 274 (6), 256 (53) | 0.067 g (0.040 g, 0.059 mmol, 58 %) |
| 164 | CK-59-AcPP-3-1 | 26.50 min (50 %) | t_{Ref} = 10.08 min APCI, Neg 608 (100) | 0.058 g (0.029 g, 0.048 mmol, 47 %) |
| | | | APCI, Pos 610 (100), 592 (15), 421 (17), 274 (8), 256 (40) | |
| | CK-59-AcPP-3-2 | 26.09 min (54 %) | t_{Ret} = 10.01 min APCl, Neg 608 (100) APCl, Pos 610 (100), 592 (24), 421 (20), 274 (18), 256 (69) | 0.019 g (0.011 mmol, 0.017 mmol, 17 %); racemisiertes Produkt |
| 165 | CK-59-ISS-4-1 | 14.67 min (92 %) | t_{Ref} = 7.49 min APCI, Neg 551 (100), 533 (26), 521 (17), 491 (6) APCl, Pos 553 (100), 535 (36), 403 (6), 385 (9), 256 (100) | 0.009 g (0.008 g, 0.015 mmol, 15 %) |
| | CK-59-ISS-4-2 | 15.90 min (48 %) | t_{Ref} = 7.63 min APCl, Neg 551 (100), 533 (25), 521 (19), 491 (5) APCl, Pos 553 (100), 535 (70), 517 (7), 274 (9), 256 (57) | 0.004 g (0.002 g, 0.003 mmol, 3 %); racemisiertes Produkt |
| 167 | CK-59-MSS-5-1 | 21.12 min (27 %) und 21.57min (26%) | t_{Ref} = 8.35 min APCl, Neg 580 (100), 562 (17), 550 (15), 520 (6) APCl, Pos 582 (49), 564 (43), 546 (67), 361 (100), 256 (55) t_{Ref} = 8.49 min APCI, Neg 580 (100), 562 (21), 550 (16), 520 (9) APCl, Pos 582 (43), 564 (47), 546 (51), 361 (100), 256 (64) | 0.013 g (0.007 g, 0.012 mmol, 12 %); racemisiertes Produkt |
| | CK-59-MSS-5-2 | 21.27 min (10 %) und 21.58 (15 %) | t_{Ref} = 8.39 min APCl, Neg 580 (100) t_{Ref} = 8.64 min APCI, Neg | 0.008 g (0.002 g, 0.003 mmol, 3 %); racemisiertes Produkt |
| | | | 580 (100), 562 (35), 550 (14) APCl, Pos 582 (63), 564 (77), 546 (77), 361 (100), 256 (63) | |

### Beispiel 167b

### SPH-1543

Als Nebenprodukt wurde N-Acetyl-phenylalanin-{(*4aS*,*6R*,*8aS*)-6-hydroxy-3-methoxy-5,6,9,10-fetrahydro-4aH-[1]benzo-furo-[3a,3,2-ef]-[2]benzazepin-11(12H)-yl)amid erhalten:

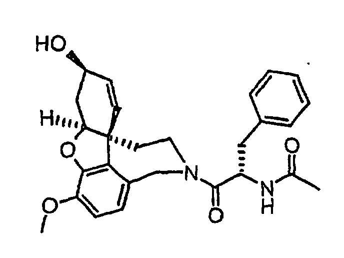

| | Code | HPLC (t_{Ret}, Reinheit) | LC/MS und R_{f}-Wert | Ausbeute und Bemerkung |
|---|---|---|---|---|
| | CK-59-IPP-2-1 | 23.49 min (67 %) | t_{Ret} = 9.37 min APCl, Neg 461 (100) APCl, Pos 463 (100), 445 (8), 274 (17), 256 (31) 0.50 (CHCl₃/CH₃OH = 10/1) | 0.036 g (0.031 g, 0.077 mmol, 76 %) |
| | CK-59-IPP-2-2 | 23.60 min (66 %) | t_{Ret} = 9.40 min APCl, Neg 461 (100) APCl, Pos 463 (100), 445 (17), 274 (32), 256 (60) 0.36 (CHCl₃/CH₃OH = 10/1) | 0.012 g (0.008 g, 0.17 mmol, 17 %); racemisiertes Produkt |

### Beispiel 170

### SPH-1371

### (4aa,6b,8aR*)-4a,5,9,10,11,12-Hexahydro-3-methoxy-11-[3-(1-piperidinyl)propyl]-6Hbenzofuro[3a,3,2-ef][2]benzazepin-6-ol, Dihydrobromid, Dihydrat

Herstellung aus der freien Base durch Behandeln mit HBr/EtOH
[α]_{D}²⁰ = - 92.5° (c 0.24, H₂O)

### Allgemeine Vorschrift - "Thiophen-Derivate" (Beispiel 171-172)

1.1 Äquivalente Norgalanthamin und 1 Äquivalent des entsprechenden Thienyl-halogenides wurden zusammen mit 3 Äquivalenten fein gepulvertem, wasserfreiem K₂CO₃ in trockenem Acetonitril (10 Gew%-ige Lösung) 24 h auf Rückflußtemperatur erhitzt. Der vollständige Umsatz wurde mittels DC geprüft.
Nach Abkühlen der Lösung wurde filtriert und der K₂CO₃-Rückstand mehrmals unter DC Kontrolle mit trockenem Acetonitril gewaschen. Das nach dem Eindampfen erhaltene Rohprodukt wurde mittels Flash-Chromatographie gereinigt.

### Beispiel 171

### SPH-1490

### 6,7-Dihydro-5-(4-((4aS,6R,8aS)-6-hydroxy-3-methoxy-4a,5,9,10-tetrahydro-6H-benzofuro[3a,3,2-ef][2]benzazepin-11(12H)-yl)-butyl)-benzo[b]thiophen-4(5H)-on

### C₂₈H₃₃NO₄S (479.64)

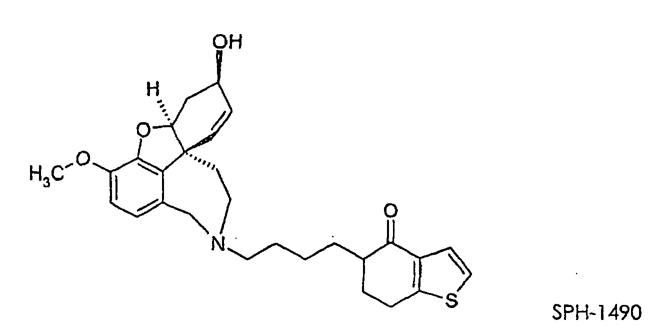

| | |
|---|---|
| Norgalanthamin | 500 mg (1.83 mmol) |
| 5-(4-Brombutyl)-6,7-dihydro-benzo[b]thiophen-4(5H)-on | 479 mg (1.67 mmol) |
| K₂CO₃ | 693 mg (5.01 mmo) |

ca. 4 ml Acetonitril
Rohprodukt: 450 mg
Flash-Chromatographie: Ethylacetate : Triethylamin = 100 : 2
- Ausbeute:: 440 mg (50 %) hellgelbes Pulver
¹H (400 MHz, CDCl₃):
δ 7.34 (d, J = 5.26, 1H), 7.04 (d, J = 5.26, 1H), 6.66 (d, J = 8.18, 1H), 6.62 ( d, J = 8.18, 1H), 6.09 (m, 2H), 4.61 (m, 1H), 4.25-4.07 (m, 2H), 3.92-3.84 (m, 1H), 3.84-3.79 (m, 1H), 3.80 (s, 3H), 3.46-3.56 (m, 1H), 3.30-3.18 (m, 1H), 3.13-3.03 (m, 1H), 3.02-2.91 (m, 1H), 2.73-2.65 (m, 1H), 2.64-2.47 (m, 2H), 2.45-2.36 (m, 1H), 2.32-2.23 (m, 1H), 2.11-1.83 (m, 4H), 1.63-1.09 (m, 6H)
¹³C (100 MHz, CDCl₃):
δ 195.2 (s), 154.8 (2*s), 145.8 (s), 144.4 (s), 136.8 (s), 132.9 (s), 127.9 (d), 126.4 (d), 125.1 (d), 123.2 (d), 122.4 (d), 111.3 (d), 88.6 (d), 61.9 (d), 58.1 (t), 55.9 (q), 51.4 (t), 51.3 (t), 48.2 (s), 462 (d), 29.9 (t), 29.5 (t), 28.9 (t), 28.8 (t), 28.5 (t), 24.8 (t), 24.2 (t)

### Beispiel 172

### SPH-1491

### 6,7-Dihydro-5-(5-((4aS,6R,8aS)-6-hydroxy-3-methoxy-4a,5,9,10-tetrahydro-6H-benzofuro[3a,3,2-ef][2]benzazepin-11(12H)-yl)-pentyl)-benzo[b]thiophen-4(5H)-on

### C₂₉H₃₅NO₄S (493.67)

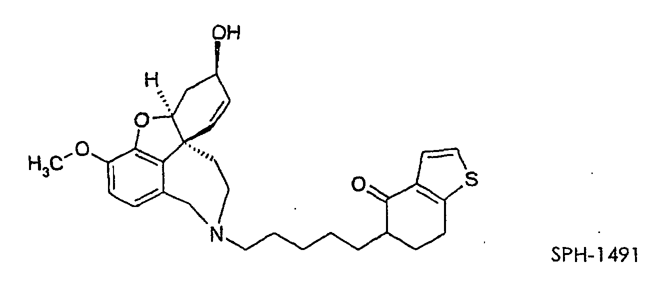

| | |
|---|---|
| Norgalanthamin | 200 mg (0.73 mmol) |
| 5-(5-Brompentyl)-6,7-dihydro-benzo[b]thiophen-4(5H)-on | 201 mg (0.67 mmol) |
| K₂CO₃ | 277 mg (2.00 mmo) |

ca. 4 ml Acetonitril
Rohprodukt: 430 mg
Flash-Chromatographie: Ethylacetate : Triethylamine = 100 : 2
- Ausbeute:: 240 mg (66.3 %) hellgelbes Pulver
¹H NMR (400MHz, CDCl₃):
δ 7.35 (d, *J* = 5.2 Hz, 1H), 7.03 ( d, *J* = 5.2 Hz, 1H), 6.64 ( d, *J* = 8.1 Hz, 1H), 6.61 (d, *J* = 8.1 Hz, 1H), 6.05 (d, *J* = 10.2 Hz, 1H), 5.99 (dd, *J* = 10.2, 4.6 Hz, 1H), 4.60 (m, 1H), 3.83 (d, *J* = 15.2, 1H), 4.14-4.10 (m, 1H), 3.83 (d, *J* = 15.2 Hz, 1H), 3.81 (*s*, 3H), 3.41-3.31 (m, 1H), 3.22-3.14 (m, 1H), 3.07 (dt, *J* = 17.2, 5.2 Hz, 1H), 2.96 (ddd, *J* = 17.2, 9.1, 4.7 Hz, 1H), 2.70-2.62 (m, 1H), 2.56-2.34 (m, 4H), 2.26 (m, 1H), 2.10-1.8 (m, 4H), 1.61-1.21 (m, 8H)
¹³C NMR: (100 MHz, CDCl₃):
δ 195.7 (s), 155.1 (2*s), 146.2 (s), 144.5 (s), 137.3 (s), 133.5 (s), 128.0 (d), 127.3 (d), 125.5 (d), 123.6 (d), 122.5 (d), 111.5 (d), 89.1 (d), 62.5 (d), 58.1 (t), 56.3 (q), 51.3 (t), 48.8 (s), 46.7 (d), 33.2 (t), 30.3 (t), 30.0 (t), 29.9 (t), 29.4 (t), 27.8 (t), 27.6 (t), 27.5 (t), 24.6 (t)

### Allgemeine Vorschrift - "Azacycloalkyl-Derivate" (Beispiel 173-176)

1 Äquivalent Norgalanthamine und 3 Äquivalente des entsprechenden Aminoalkylhalogenides wurden zusammen mit 3 Äquivalenten fein gepulvertem, trockenem K₂CO₃ in trockenem Acetonitril (ca. 10 Gew%-ige Lösung) für 24 Stunden auf Rückflußtemperatur erhitzt. Der vollständige Umsatz wurde mittels DC kontrolliert.

Nach Abdestillieren des Lösungsmittels wurde der verbleibende Rückstand in 2n HCl aufgelöst. Nach 2-matiger Extraktion mit Diethylether wurde mit 10% NaOH-Lösung alkalisch gestellt und erschöpfend mit Chloroform extrahiert. Nach Trocknen über Na₂SO₄ wurde eingedampft und mittels Flash-Chromatographie gereinigt (CHCl₃: MeOH : NH₃ = 10 : 1 : 0.5).
Die angegebenen Ausbeuten der Reaktionen beziehen sich auf 500 mg (1.83 mmol) Norgalanthamin als Ausgangsprodukt und wurden nach Flash-Chromatographie bestimmt

### Beispiel 173

### SPH-1492

### (4aS,6R,8aS)-3-methoxy-11-(6-piperidin-1-yl-hexyl)-5,6,9,10,11,12-hexahydro-4aH-[1]benzofuro[3a,3,2-ef] [2]benzazepin-6-ol

### C₂₇H₄₀N₂O₃ (440.63)

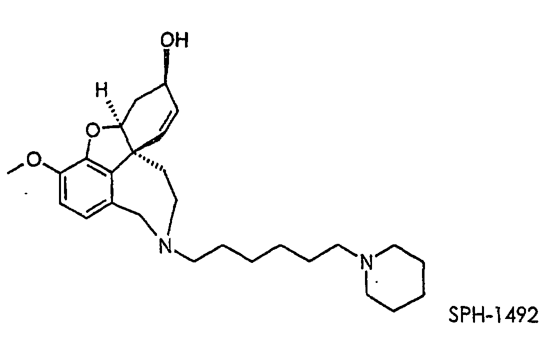

- Ausbeute:: 161 mg (20 %) gelbliches Öl
¹H (400 MHz, CDCl₃):
δ 6.66 (d, *J* = 8.0 Hz, 1H), 6.62 (d, J = 8.0 Hz, 1H), 6.10 (d, J = 10.0 Hz, 1H), 6.01 (dd, *J* = 10.0, 5.4 Hz, 1H), 4.62 (m, 1H), 4.14 (m, 1H), 4.13 (d, *J* = 15.6, 1H), 3.84 (s, 3H), 3.81 (d, *J* = 15.6, 1H), 3.4-3.3 (m, 1H), 3.2-3.15 (m, 1H), 2.69 (ddt, *J* = 15.7, 3.4, 1.6 Hz, 1H) 2.55-2.25 (m, 8H), 2.10-2.0 (m, 1H), 2.01 (ddd, *J* = 12.9, 5.0, 2.6 Hz, 1H),1.65-1.2 (m, 16H)
¹³C (100 MHz, CDCl₃):
δ 146.2 (s), 144.4 (s), 133.6 (s), 129.9 (s), 127.9 (d), 127.4 (d), 122.4 (d), 111.5 (d), 89.1 (d), 62.5 (d), 59.9 (t), 58.1 (t), 56.3 (q), 54.9 (4*t), 51.9 (t), 48.8 (s), 33.3 (t), 30.3 (t), 28.0 (t), 27.9 (t), 27.8 (t), 27.1 (t), 26.2 (t), 24.8 (t)

### Beispiel 174

### SPH-1493

### (4aS,6R,8aS)-3-methoxy-11-(6-(4-methylpiperazin)-1-yl-hexyl)-5,6,9,10,11,12-hexahydro-4aH-[1]benzofuro[3a,3,2-ef][2]benzazepin-6-ol

### C₂₇H₄₁N₃O₃ (455.65)

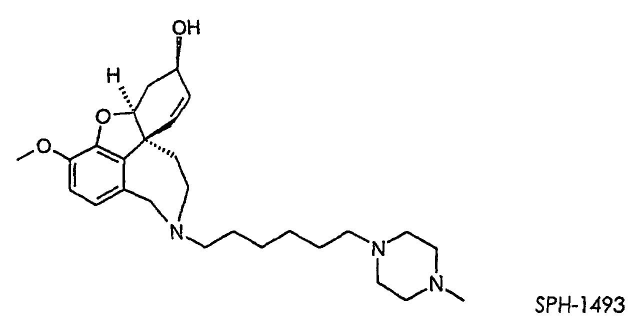

- Ausbeute:: 208 mg (25 %) gelbliches Öl
¹H (400 MHz, CDCl₃):
δ 6.66 (d, J = 8.18, 1H), 6.61 (d, J = 8.18, 1H), 6.09 (d, J = 10.23, 1H), 6.0 (dd, J = 9.94, 4.97, 1H), 4.61 (m, 1H), 4.20-4.08 (m, 2H), 3.84 (s, 3H), 3.80 (d, J = 15.8, 1H), 3.40-3.30 (m 1H), 3.23-3.10 (m, 1H), 2.73-2.63 (m, 1H), 2.63-2.30 (m 12H), 2.29 (s, 3H), 2.10-1.94 (m, 2H), 1.57-1.17 (m, 10H)
¹³C (100 MHz, CDCl₃):
δ 146.2 (s), 144.4 (s), 133.6 (s), 129.9 (s), 127.9 (d), 127.4 (d), 122.4 (d), 111.5 (d), 89.1 (d) 62.5 (d), 59.1 (t), 58.2 (t), 56.3 (q), 55.5 (2*t), 53.6 (3*t), 51.9 (t), 48.8 (s), 46.5 (q), 33.3 (t), 30.4 (t), 27.98 (t), 27.83 (t), 27.74 (t), 27.23 (t)

### Beispiel 175

### SPH-1494

### (4aS,6R,8aS)-3-methoxy-11-(6-(4-hydroxypiperidin)-1-yl-hexyl)-5,6,9,10,11,12-hexahydro-4aH-[1]benzofuro[3a,3,2-ef][2]benzazepin-6-ol

### C₂₇H₄₀N₂O₄ (456.63)

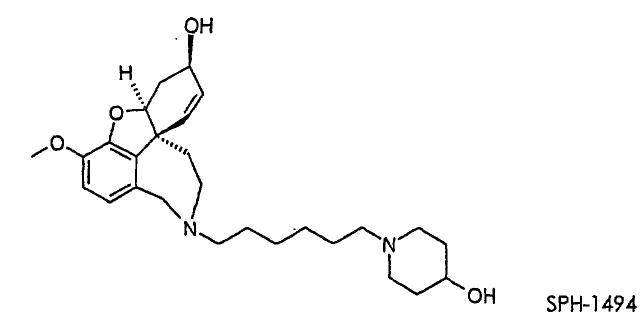

- Ausbeute:: 200 mg (24 %) gelbliches Öl
¹H (400 MHz, CDCl₃):
δ 6.64 (d, *J* = 8.1 Hz, 1H), 6.59 (d, *J* = 8.1 Hz, 1H), 6.07 (d, *J* = 10.2 Hz, 1H), 5.97 (dd, *J* = 10.2, 4.9 Hz, 1H), 4.58 (m, 1H), 4.11 (m, 1H), 4.07 (m, 1H), 3.81 (s+m, 4H), 3.71-3.61 (m, 1H), 3.35 (m, 1H), 3.15 (m, 1H), 2.81-2.71 (m, 2H), 2.67 (d, J = 15.4, 1H), 2.52-2.36 (m, 4H), 2.34-2.26 (m, 2H), 2.20-2.08 (m, 2H), 2.06-1.94 (m, 2H), 1.93-1.84 (m, 2H), 1.63-1.53 (m, 2H), 1.52-1.38 (m, 5H), 1.32-1.20 (m, 4H)
¹³C (100 MHz, CDCl₃):
δ 146.2 (s), 144.5 (s), 133.6 (s), 129.9 (s), 127.9 (d), 127.4 (d), 122.4 (d), 111.5 (d), 89.1 (d), 62,5 (2*d) 58.9 (2*t), 58.2 (t), 56.3 (q), 51.9 (2*t), 51.3 (t), 48.8 (s), 34.6 (t), 33.4 (t), 30.4 (t), 28.0 (t), 27.8 (2*t), 27.7 (t), 27.3 (t)

### Beispiel 176

### SPH-1521

### 1-(6-((4aS,6R,8aS)-6-hydroxy-3-methoxy-4a,5,9,10-tetrahydro-6H-benzofuro[3a,3,2-ef][2]benzazepin-11(12H)-yl)-hexyl)-piperidin-4-on

### C₂₇H₃₈N₂O₄ (454.61)

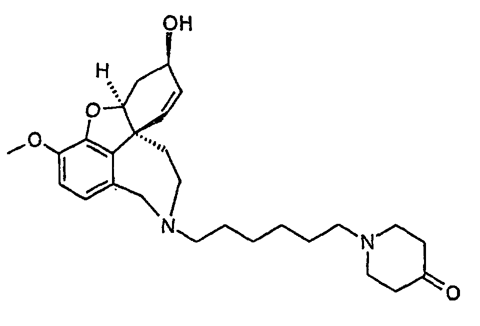

- Ausbeute:: 125 mg (15 %) gelbliches Öl
¹H (400 MHz, CDCl₃):
δ 6.65 ( d, *J* = 8.18 Hz, 1H ), 6.60 ( d, *J* = 8.18 Hz, 1H), 6.07 (d, *J* = 10.8 Hz, 1H), 5.99 (dd, *J* = 10.8, 4.5 Hz, 1H), 4.60 ( m, 1H), 4.18-4.08 (m, 2H), 3.82 (s, 3H), 3.79-3.76 ( m, 1H), 3.40-3.30 (m, 1H), 3.22-3.10 (m, 1H), 2.74-2.68 (m, 4H), 2.67-2.62 (m, 1H), 2.53-2.34 (m, 8H), 2.09-1.93 (m, 2H), 1.57-1.42 (m, 6H), 1.37-1.21 (m, 4H)
¹³C (100 MHz, CDCl₃):
δ 209.7 (s), 146.2 (s), 144.5 (s), 133.5 (s), 129.7 (s), 128.1 (d), 127.4 (d), 122.4 (d), 111.5 (d), 89.0 (d), 62.4 (d), 58.1 (2*t), 57.9 (2*t), 56.4 (q), 56.3 (t), 53.4 (t), 522 (t), 51.9 (t), 48.8 (s), 41.5 (t), 33.4 (t), 30.4 (t), 27.9 (t), 27.8 (t), 27.7 (t)

### Beispiel 180

### SPH-1363

### (4aS,6R,8aS)-6-Hydroxy-3-methoxy-5,6,9,10-tehahydro-4aH-[1]benzofuro[3a,3,2-ef][2]benzazepin-11(12H)-yl)methyl-azodicarbonsäured]-tert.-butylester (CK-24-2)

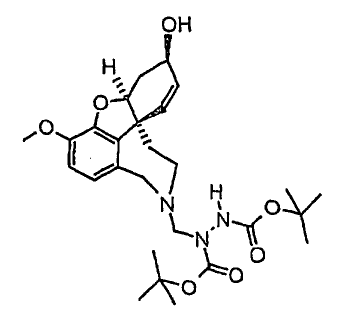

Eine Lösung aus 0.300 g (0.1.04 mmol) Galanthamin in 3 ml Dichlormethan wird bei Raumtemperatur vorgelegt, mit 0.264 g (1.150 mmol) Di-*tert*.-butylazodicarboxylat versetzt und vier Tage bei Raumtemperatur gerührt. Nach dem Einengen am Rotationsverdampfer wird der Rückstand (0.258 g) mittels Flashchromatographie über Kieselgel (25 g, Laufmittel Dichlormethan/Petrolether = 2/3 + 4 % Triethylamin) gereinigt. Nach dem Trocknen im Hochvakuum erhält man 0.292 g eines weißen Schaumes.
- Ausbeute:: 0.292 g (0.56 mmol, 54 %), farbloser Schaum, (Mw = 517.6)
- DC:: R_{f} = 0.71 (Dichlormethan/Methanol = 9/1 + 2 % conc. NH₃-Lösung)
- mp.:: 59 - 62°C (Petrolether/Dichlormethan = 1/1 + 4 % Triethylamin)
- IR:: KBr
v (cm⁻¹) 3557 (v), 3340 (bm), 2932 (s), 2915 (s), 1726 (s), 1711 (s)
- ¹H-NMR:: (200.13 MHz, CDCl₃, TMS)
δ6.49 - 6.66 (m, 2 H), 6.39 (s, 0.6 H), 5.84 - 6.06 (m, 2 H), 4.52 (s, 1H), 4.35 (bs, 1H), 3.99-4.17 (m, 2 H), 3.71 - 3.88 (m, 1H), 3.76 (s, 3 H), 3.07 - 3.40 (m, 2 H), 2.61 (bd, J = 15.7 Hz, 1H), 2.40 (bd, J = 11.8 Hz, 0.3 H), 1.79 - 2.05 (m, 2 H), 1.56 - 1.72 (m, 1H), 1.46 (s, 9 H), 1.45 (s, 9 H)
- ¹³C-NMR:: (50.32 MHz, CDCl₃, TMS)
δ156.7 (s), 155.9 (s), 145.9 (s), 144.0 (s), 132.8 (d), 129.8 (s), 127.6 (d), 126.7 (d), 121.5 (d), 111.1 (d), 88.5 (d), 68.9 (t), 62.4 (t), 61.8 (t), 61.8 (d), 56.6 (t), 55.8 (q), 49.7 (t), 48.1 (s), 35.2 (t), 29.8 (t), 14.32 (q), 14.26 (q)
- LC/MS:: t_{Ret} = 9.56 min, (Zorbax SB-Säule, 2.1 mm x 30 mm, RP-18,3 µm, 0.5 ml/min, Methanol/H₂O (40/60 □ 100/0 (v/v) in 2 min)
APCI-PI-MS
518 (100), 500 (25), 462 (9), 285 (13), 274 (11), 256 (26)
APCI-NI-MS
516

### Beispiel 181

### SPH-1362

### (4aS,6R,8aS)-6-Hydroxy-3-methoxy-5,6,9,10-tetrahydro-4aH-[1]benzofuro[3a,3,2-ef][2]benzazepin-11(12H)-yl)methyl-azodicarbonsäurediethylester (CK-21-3)

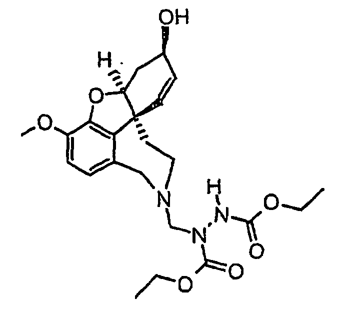

Zu einer Lösung aus 0.150 g (0.52 mmol) Galanthamin in 2 ml Dichlormethan werden 89 µl (0.100 g, 0.57 mmol) Diethylazodicarboxylat zugefügt, und die Lösung wird 72 Stunden bei Raumtemperatur gerührt. Nach dem Einengen am Rotationsverdampfer wird der Rückstand (0.258 g) mittels Flashchromatographie über Kieselgel (25 g, Laufmittel Dichlormethan/Petrolether = 1/1 + 4 % Triethylamin) gereinigt. Nach dem Trocknen im Hochvakuum erhält man 0.168 g eines weißen Schaumes.
- Ausbeute:: 0.168 g (0.36 mmol, 70 %), weißer Schaum, (M_{w} = 461.6)
- DC:: R_{f} = 0.66 (Dichlormethan/Methanol = 9/1 + 2 % conc. NH₃-Lösung)
- mp.:: 40 - 42°C (Petrolether/Dichlormethan = 1/1 + 4 % Triethylamin)
- IR:: KBr
v (cm⁻¹) 3553 (v), 3305 (m), 2981 (s), 2935 (s), 1742 (s), 1722 (s)
- ¹H-NMR:: (200.13 MHz, CDCl₃, TMS)
*δ* 6.77 (bs, 1H), 6.64 (d, *J* = 8.3 Hz, 1H), 6.58 (d, *J* = 8.1 Hz, 1H), 5.84 - 6.06 (m, 2 H), 4.53 (s, 1H), 3.98 - 4.23 (m, 6 H), 3.69 - 3.87 (m, 1H), 3.76 (s, 3 H), 3.01 - 3.42 (m, 2 H), 2.66 (bd, J = 15.7 Hz, 1H), 2.33 (bs, 0.2 H), 1.65 - 1.77 (m, 2 H), 1.35 - 1.47 (m, 1H), 1.09 - 1.35 (m, 6 H)
- ¹³C-NMR:: (50.32 MHz, CDCl₃, TMS)
δ 155.1 (s), 145.9 (s), 143.9 (s), 132.8 (d), 130.2 (s), 127.5 (d), 126.9 (d), 121.4 (d), 111.1 (d), 81.2 (s), 80.9 (s), 69.5 (t), 61.9 (d), 56.8 (t), 55.8 (q), 49.8 (t), 48.1 (s), 35.6 (t), 29.8 (t), 28.1 (q), 28.0 (q)
- LC/MS:: t_{Ret} =8.08 min, (Zorbax SB-Säule, 2.1 mm × 30 mm, RP-18, 3 µm, 0.5 ml/min, Methanol/H₂O (40/60 □ 100/0 (v/v) in 2 min)
APCI-PI-MS
462 (100), 444 (32), 286 (34), 274 (12), 256 (29)
APCI-NI-MS
460

### Verfahren zur Synthese von Norgalanthamin

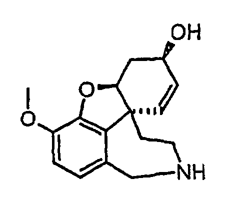

### Methode A

20 g (70 mmol) Galanthamin wird mit 14.206 g (0.07 mol) m-Chlorperbenzoesäure (85 %) in 350 ml Dichlormethan und anschließender Zugabe von 9.730 g (35 mmol) Fe(II)SO₄*7 H₂O in 100 ml Methanol umgesetzt. Die Reaktion wird nach 20 Minuten mit 200 ml 2 N Salzsäure terminiert. Nach dem Abdestillieren der leicht flüchtigen Lösungsmittel, der Säure-Base-Trennung und Abfiltration des Pe(OH)ₓ-Niederschlages mittels einer Hyflo-Nutsche wird Filtrat über Na₂SO₄ getrocknet, filtriert und eingeengt. Man erhält ca. 18 g des Rohprodukts in Form eines gelben Schaumes.

### Aufarbeitung Variante 1 (CK-1-1)

Das Rohprodukt (18.46 g) wird in ca. 200 ml Essigsäureethylester/Methanol/Triethylamin (90/10/2) unter leichten Erwärmen aufgenommen. Beim Abkühlen fällt erneut Fe(OH)× aus, welches abfiltriert wird. Die Reinigung erfolgt mittels MPLC-Chromatographie (Kieselgel, h = 25 cm, d = 3.6 cm, v = 300 nm) mit einem geänderten Laufmittelgemisch (Essigsäureethylester/Methanol/Triethylamin = 95/5/2 → 90/10/2 → 80/20/2). Der Norgalanthamin-Gehalt beträgt laut einer HPLC-Quantifizierung (Coffein als interner Standard) nur 68 %.
Ausbeute: 10.34 g (38 mmol, 54 %), gelblicher, amorpher Feststoff (M_{w} = 273.3)

### Aufarbeitung Variante 2 (CK-1-11)

Das Rohprodukt (16.48 g) wird in 100 ml Methanol gelöst, mit einer Lösung aus 12.86 g (102 mmol) Oxalsäure Dihydrat in 100 ml Methanol versetzt und vorsichtig erwärmt, um die Lösung zu homogenisieren. Danach läßt man die Lösung auf Raumtemperatur und dann auf ca. 5°C abkühlen und filtriert das auskristallisierte Produkt ab und wäscht den Niederschlag mit Methanol. Die vereinigten eingeengten Methanollösungen werden erneut einer Umkristallisation unterworfen.
- Ausbeute:: 16.108 g (43 mmol, 62 %), farbloser kristalliner Feststoff
Ber. für C₁₆H₁₉NO₃. C₂H₂O₄..0.5 H₂O C, 58.06 H,5.95 N, 3.76
Gef. C, 57.91, H, 5.88, N, 3.69

### Methode B (CK-1-10)

2.000 g (6.96 mmol) Galanthamin und 0.981 g (10.44 mmol) Wasserstoffperoxid-Harnstoff-Addukt werden in 25 ml Dichlormethan und 5 ml Methanol bei Raumtemperatur zwei Tage gerührt, anschließend mit 0.030 g Platin/Aktivkohle versetzt und eine Stunde bei Rauumtemperatur gerührt. Bei der Zugabe des Katalysators ist eine starke Gasentwicklung zu beobachten. Dann werden 0.967 g (3.48 mmol) Fe(II)SO₄*7 H₂O in 5 ml MeOH zugesetzt, und die braune Suspension wird stark gerührt. Die Reaktion wird nach 20 Minuten mit 50 ml gesättigter NaHCO₃-Lösung terminiert. Die Reaktionslösung wird mittels einer Hyflo-Filterhilfe filtriert. Die Phasen werden separiert, und die wäßrige Phase wird erschöpfend mit Dichlormethan extrahiert. Die vereinigten organischen Extrakte werden mit gesättigter NaHCO₃-Lösung (50 ml) und mit gesättigter NaCl-Lösung (50 ml) gewaschen, über Na₂SO₄ getrocknet, filtriert und eingeengt. Das Rohprodukt (1.925 g) wird in 10 ml Methanol gelöst, mit einer Lösung aus 1.332 g (10.6 mmol) Oxalsäure Dihydrat in 10 ml Methanol versetzt und vorsichtig erwärmt, um die Lösung zu homogenisieren. Danach läßt man die Lösung auf Raumtemperatur und dann auf ca. 5°C abkühlen und filtriert das auskristallisierte Produkt ab und wäscht den Niederschlag mit Methanol. Die vereinigten eingeengten Methanollösungen werden erneut einer Umkristallisation unterworfen.
- Ausbeute:: 1.010 g (2.7 mmol, 39 %), farbloser kristalliner Feststoff (M_{w} = 371.4), laut HPLC 97 %

### Methode C:

### Demethylierung von Norgalanthamin mittels Diethylazodicarboxylat (CK-1-7)

Zu einer Lösung aus 0.300 g (1.04 mmol) Galanthamin in 3 ml Dichlormethan werden 178 µl (0.199 g, 1.144 mmol) Diethylazodicarboxylat zugefügt, und die Lösung wird drei Tage bei Raumtemperatur gerührt. Nach dem Einengen am Rotationsverdampfer bei 40°C wird der Rückstand in 5 ml Ethanol und 5 ml 4 N Salzsäure gelöst und bei 80°C für eineinhalb Stunden gerührt. Die Reaktion kühlt man auf Raumtemperatur ab und verdünnt die Lösung mit 5 ml Wasser. Der Ethanol-Anteil wird im Rotationsverdampfer abdestilliert, und die wäßrige Phase wird dreimal jeweils 10 ml Diethylether extrahiert. Die wäßrige Phase wird durch Zugabe Natriumcarbonat und Natriumhydroxid auf pH 10 - 11 eingestellt und viermal mit jeweils 20 ml Dichlormethan extrahiert. Die vereinigten Phasen werden mit 40 ml gesättigter Kochsalz-Lösung gewaschen, über Na₂SO₄ getrocknet. Nach dem Filtrieren und dem Einengen wird der Rückstand (0.268 g) mittels MPLC über Kieselgel (60 g, Laufmittel Essigsäureethylester/Ethanol/Triethylamin = 19/1/0.4) gereinigt. Nach dem Trocknen im Hochvakuum erhält man 0.136 g eines gelblichen Schaumes.
- Ausbeute:: 0.136 g (0.495 mmol, 48 %), weißgelblicher Schaum; (M_{w} = 273.3), HPLC identisch mit einer Referenzprobe
- HPLC:: t_{Ret} = 3.79 min, 96.3 % (Merck Purospher-Säule, 4.0 mm × 125 mm, RP-18e, 5 µm, 250 nm, 1 ml/min, Acetonitril/ 20 mM Trichloressigsäure in H₂O (15/80 v/v)

### Methode D:

### Demethylierung von Norgalanthamin mittels Di-tert.-butylazodicarboxylat (CK-1-6)

0.300 g (1.04 mmol) Galanthamin und 0.264 g, (1.144 mmol) Di-*tert*.-butylazodicarboxylat in 3 ml Dichlormethan werden analog der oben beschriebenen Prozedur drei Tage bei Raumtemperatur umgesetzt, eingeengt und in 5 ml Ethanol und 5 ml 4 N Salzsäure für 30 Minuten bei 80°C gerührt. Die Reaktion kühlt man auf Raumtemperatur ab, und verdünnt die Lösung mit 5 ml Wasser. Nach der wäßrigen Aufarbeitung wird der Rückstand (0.259 g) mittels MPLC über Kieselgel (60 g, Laufmittel Essigsäureethylester/Ethanol/Triethylamin = 19/1/0.4) gereinigt. Nach dem Trocknen im Hochvakuum erhält man 0.132 g eines weißgelblichen Schaumes.
- Ausbeute:: 0.132 g (0.48 mmol, 46 %), weißgelblicher Schaum, (M_{w} = 273.3), HPLC identisch mit einer Referenzprobe
- HPLC:: t_{Ret} = 3.74 min, 100 % (Merck Purospher-Säule, 4.0 mm × 125 mm, RP-18e, 5 µm, 250 nm, 1 ml/min, Acetonitril/ 20 mM Trichloressigsäure in H₂O (15/80 v/v)

### Methode E:

### Verseifung von (4aS,6R,8aS)-3-Methoxy-12-trifluoracetyl-5,6,9,10,11,12-hexahydro-4aH-[1]benzofuro[3a,3,2-ef][2]benzazepin-6-ol

### Methode A (CK-40-2)

3.3 ml einer 0.25 M Kaliumhydroxid-Lösung (0.045 g (0.81 mmol), in Dioxan/Methanol/Wasser (10/2/5) werden mit 0.100 g (0.27 mmol) N-Trifluoracetylnorgalanthamin versetzt und eine Stunde bei Raumtemperatur gerührt. Anschließend wird die Lösung mit 0.4 ml 2 N Salzsäure verdünnt und die flüchtigen Bestandteile werden im Rotationsverdampfer abdestilliert. Der Rückstand wird mit gesättigter Natriumcarbonat-Lösung aufgenommen und fünfmal mit Dichlormethan extrahiert. Die vereinigten Extrakte werden mit gesättigter Natriumchlorid-Lösung gewaschen, über Natriumsulfat getrocknet, filtriert und im Rotationsverdampfer unter verminderten Druck eingeengt. Man erhält 0.070 g eines weißgelblichen Schaumes von 95 %iger Reinheit (HPLC, Coffein als interner Standard).
- Ausbeute:: 0.070 g (0.067, 0.25 mmol, 91 %), weißgelblicher Schaum (M_{w} = 273.3)

### Methode B (CK-40-3)

Eine Lösung aus 0.100 (0.27 mmol) N-Trifluoracetylnorgalanthamin und 0.243 g (1.35 mmol) 30 %ger Natriummethanolat-Methanol-Lösung in 3 ml Tetrahydrofuran/Methanol (1/1) wird drei Stunden bei Raumtemperatur gerührt. Die Lösung wird mit 0.7 ml 2 N Salzsäure neutralisiert und die Lösung wird bis zur Trockne eingeengt, der Rückstand wird mit 25 ml Dichlormethan aufgenommen und mit gesättigter Natriumcarbonat-Lösung und mit gesättigter Natriumchlorid-Lösung gewaschen, über Natriumsulfat getrocknet, filtriert und im Rotationsverdampfer unter verminderten Druck eingeengt. Nach dem Trocknen im Vakuum erhält man einen gelblichen Schaum (0.067 g) von 76 %ger Reinheit (HPLC, Coffein als interner Standard).
- Ausbeute:: 0.067 g (0.051 g, 0.19 mmol, 69 %), weißgelblicher Schaum (Mw = 273.3)

### Methode G:

### Trennung der (+) und (-) Isomeren von rac. Norgalanthamin (4)

### Herstellung von (-) Norgalanthamin (8)

Zu einer Lösung von 10.92 g (40.0 mmol) rac. Norgalanthamin (4) in 40 ml Methanol wird eine Lösung von 7.72 g (20.0 mmol) (+)-O,O-Di-p-Toluoylweinsäure in 15 ml Methanol getropft und anschließend mit 1 ml Methanol nachgewaschen. Die Lösung wird mit einem Impfkristall versetzt und zwei Tage bei 4°C stehen gelassen. Dann wird mit einem Glasstab gut durchgerieben und weitere zwei bis fünf Tage bei 4°C stehen gelassen, wobei immer wieder mit einem Glasstab gut durchgerieben wird. Anschließend wird das ausgefallene Salz abgesaugt, dreimal mit eiskaltem Methanol nachgewaschen und in 100 ml Wasser aufgenommen. Die wässrige Phase wird mit konzentriertem wässrigen Ammoniak basisch gemacht und dreimal mit je 60 ml Essigsäureethylester extrahiert. Die vereinigten organischen Phasen werden einmal mit gesättigter wässriger Natriumchloridlösung gewaschen, getrocknet (Na₂SO₄, Aktivkohle), filtriert und eingedampft, wodurch 2.90 g (37.5 % d. Th) farblose Kristalle mit einem Drehwert von α_{D}²²[CHCl₃] = -62,4 an (-) Norgalanthamin (8) erhalten werden. Die methanolische Mutterlauge wird eingedampft, der Rückstand in 100 ml Wasser aufgenommen und wie das reine Salz oben behandelt, wodurch 4.1 g (53.1 % d. Th.) Rohprodukt rückgewonnen werden können, die wie folgt zur Gewinnung von (+) Norgalanthamin verwendet werden.

### Herstellung von (+) Norgalanthamin

Zu einer Lösung von 4.1 g (15.0 mmol) rückgewonnenem Norgalanthamin (dieses ist angereichert an (+) Isomeren) in 21 ml Methanol wird eine Lösung von 2.9 g (7.5 mmol) (-)-O,O-Di-p-Toluoylweinsäure in 5.6 ml Methanol getropft, wobei mit 0.5 ml Methanol nachgewaschen wird. Die Lösung wird mit einem lmpfkristall versetzt und wie bei der Gewinnung von (-) Norgalanthamin behandelt, wodurch 3.0 g (39 % d. Th.) farblose Kristalle mit einem Drehwert von α_{D}²²[CHCl₃] = +57,5° an (+) Norgalanthamin erhalten werden.
Alternativ wird (+) Norgalanthamin auch durch Umsetzung von rac. Norgalanthamin (4) mit (-)-O,O-Di-p-Toluoylweinsäure analog der obigen Vorschrift mit einem Drehwert von α_{D}²²[CHCl₃] = +60,5° erhalten.

### Beispiel 182

### SPH-1534

### (4aS,6R,8aS)-6-Hydroxy-3-methoxy-5,6,9,10-tetrahydro-4aH-[1]benzofuro[3a,3,2-ef][2]benzazepin-11(12H)-yl)carbonsäuretriisopropylsilylester (CK-9-2)

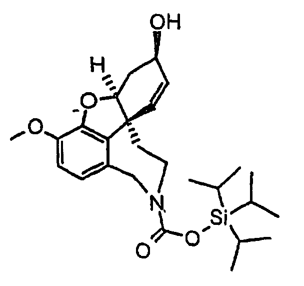

0.200 g (0.732 mmol) Norgalanthamin (68 % (HPLC, CK-1-1)) und 0.47 ml (0.341 g, 3.37 mmol) Triethylamin werden in einem Einhalskolben mit Septum und CO₂-Ballon, der durch Evakuieren und Spülen mit Kohlendioxid befüllt wird, bei -80° C in 6 ml Dichlormethan vorgelegt. Anschließend wird die Lösung so weit abgekühlt, daß festes Kohlendioxid in der Reaktionslösung ausfällt. Nach eineinhalb Stunden bei ca. -80 - -90° C wird mittels einer Spritze Triisopropylsilylchlorid (0.155 ml, 0.141 g, 0.732 mmol) zugegeben. Anschließend erwärmt man die Reaktionslösung langsam über Nacht auf Raumtemperatur. Dabei fällt ein farbloser Niederschlag aus. Die Reaktionslösung wird mit 10 ml 1N Salzsäure aufgenommen, die Phasen werden separiert, und die wäßrige Phase wird zweimal mit 10 ml Dichlormethan extrahiert. Die vereinigten organischen Phasen werden mit 10 ml 1 N Salzsäure und mit 10 ml gesättigter Kochsalz-Lösung gewaschen, über Na₂SO₄ getrocknet, filtriert und eingeengt. Das gelbe viskose Öl (0.315 g) wird mittels Säulenchromatographie über Kieselgel mit dem Laufmittel Petrolether/Essigsäureethylester gereinigt. Nachdem Einengen erhält man 0.208 g des Produktes in Form eines farblosen Schaumes.
- Ausbeute:: 0.208 g (0.44 mmol, 60 %), farbloser Schaum, (M_{w} = 473.7)
- DC:: R_{f} = 0.35 (Petrolether/Essigsäureethylester = 1/1)
- mp.:: 53 - 54°C (Petrolether/Essigsäureethylester = 1/1)
- IR:: KBr
v (cm⁻¹) 3556 (m), 3454 (m), 2946 (s), 1679 (s)
- ¹H-NMR:: (200.13 MHz, CDCl₃, TMS)
δ 6.60 - 6.85 (m, 2 H), 5.93 - 6.09 (m, 2 H), 4.90 (d, *J* = 15.3 Hz, 0.4 H), 4.80 (d, *J* = 15.7 Hz, 0.6 H), 4.57 (s, 1H), 4.06 - 4.40 (m, 3 H), 3.83 (s, 3 H), 3.27 - 3.57 (m, 0.4 H), 2.70 (bd, *J* = 16.3 Hz, 1H), 2.41 (bd, *J* = 11.0 Hz, 0.6 H), 1.60 - 2.11 (m, 3 H), 1.29 (bh, *J* = 5.1 Hz, 3 H), 1.05 (d, *J* = 6.8 Hz, 8 H), 0.98 (d, *J* = 6.9 Hz, 10 H)
- ¹³C-NMR:: (50.32 MHz, CDCl₃, TMS)
δ 154.4 und 153.9 (s), 146.5 und 146.2 (s), 144.3 und 144.1 (s), 132.2 und 131.8 (d), 129.4 und 129.2 (s), 127.9 (d), 126.5 (d), 121.6 und 120.9 (d), 111.1 und 110.9 (d), 88.2 (d), 61.8 (d), 55.8 und 55.7 (q), 52.5 und 51.7 (t), 48.3 (s), 46.4 und 45.8 (t), 37.3 und 36.1
(t), 29.7 (t), 17.77, 17.75, 17.68 und 17.65 (q), 11.9 (d)

### Beispiel 183

### SPH-1535

### (4aS,6R,8aS)-6-Hydroxy-3-methoxy-5,6,9,10-tetrohydro-4aH-[1]benzofuro[3a,3,2-ef][2]benzazepin-11(12H)-yl)carbonsäure-tert.-butyldiphenylsilylester

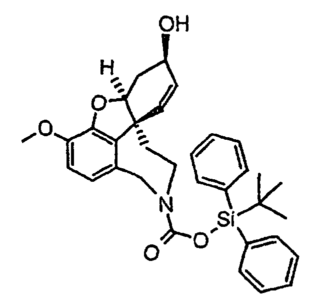

Analog der Vorschrift für das N-Triisopropylsilyloxycarbonylnorgalanthamin [TBDP-CI ) wird Norgalanthamin (68 % (HPLC, CK-1-1)) mit 1 bzw. 1.1 Äquivalenten *tert*.-Butyldiphenylsilylchlorid und 5 Äquivalenten Triethylamin umgesetzt. Anschließend wird die Reaktion mit Wasser bzw. verdünnter Salzsäure terminiert, die Phasen werden separiert, und die wäßrige Phase wird mit Dichlormethan extrahiert. Nach dem Trocknen mit Na₂SO₄, dem Filtrieren und dem Einengen wird der amorphe Schaum mittels Säulenchromatographie (Kieselgel, Essigsäureethylester/Petrolether = 1/1) gereinigt. Man erhält einen farblosen Schaum.

| | Bedingungen: | wäßrige Aufarbeitung: | Ausbeute (M_{w} = 524.7): |
|---|---|---|---|
| CK-10-1 | 0.200 g (0.732 mmol) Norgalanthamin, 0.172 ml (0.181 g, 0.732 mmol) TBDP-Cl | 10 ml 1 N Salzsäure | 0.282 g (0.538 mmol, 73 %) |
| CK-10-2 | 0.200 g (0.732 mmol) Norgalanthamin, 0.172 ml (0.181 g, 0.732 mmol) TBDP-Cl | 10 ml aqua dest | 0.196 g (0.37 mmol, 51 %) |
| CK-10-3 | 0.400 g (1.46 mmol) Norgalanthamin, 0.38 ml (0.398 g, 1.606 mmol) TBDP-Cl | 20 ml 0.3 M Salzsäure | 0.505 g (0.96 mmol, 66 %) |

- DC:: R_{f} = 0.40 (Petrolether/Essigsäureethylester = 1/1)
- mp.:: 71 - 80°C °C (Petrolether/Essigsäureethylester = 1/1)
- IR:: KBr
v (cm⁻¹) 3553 (m), 3454 (bm), 2932 (s), 1686 (s), 1625 (m)
- ¹H-NMR:: (200.13 MHz, CDCl₃, TMS)
δ 7.20 - 7.68 (m, 10 H), 6.56 - 6.77 (m, 2 H), 4.83 - 5.03 (m, 1H), 5.03 (s, 1H), 4.02 - 4.40 (m, 3 H), 3.83 (s, 3 H), 3.63 (bt, *J* = 13.1 Hz, 0.3 H), 3.41 (bt, *J* = 12.9 Hz, 0.7 H), 2.70 (bd, *J* = 15.6 Hz, 1H), 2.41 (bd, *J* = 19.7 Hz, 1H),, 1.78 - 2.10 (m, 2 H), 1.54 - 1.76 (m, 1H), 1.05 (s, 9 H)
- ¹³C-NMR:: (50.32 MHz, CDCl₃, TMS)
δ 153.7 und 153.2 (s), 146.6 und 146.1 (s), 144.4 und 144.2 (s), 135.0 und 134.7 (d), 132.4 und 132.3 (s), 132.4 (s), 132.3 und 131.8 (s), 129,7 und 129.6 (d), 129.2 und 129.0 (s), 126.5 (s), 121.8 und 121.2 (d), 111.1 und 110.9 (d), 88.3 und 88.1 (d), 61.8 (d), 55.9 (q), 52.7 und 51.8 (t), 48.3 (s), 46.5 und 46.1 (t), 37.4 und 35.8 (t), 29.7 (t), 27.0 und 26.9 (q), 19.1 und 18.9 (d)

### Beispiel 184

### SPH-1536

### (4aS,6R,8aS)-3-Methoxy-12-trifluoracetyl-5,6,9,10,11,12-hexahydro-4aH-[1]benzofuro[3a,3,2-ef] [2]benzazepin-6-ol

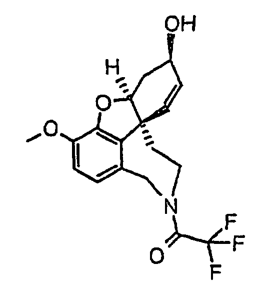

### Methode A (CK-32-1)

Zu einer Lösung von 1.00 g (3.66 mmol) Norgalanthamin (68 % (HPLC, CK-1-1)), 1.5 ml (1.095 g, 10.8 mmol) Triethylamin in 5 ml absolutem Dichlormethan wird bei 0°C eine Lösung bestehend aus 5 ml Dichlormethan und 4.6 ml (3.333 g, 32.9 mmmol) Trifluoressigsäureanhydrid innerhalb von 15 Minuten zugetropft. Anschließend rührt man die Lösung 1.75 Stunden bei 0°C und terminiert dann die Reaktion durch Zugabe von 5.5 ml 2 N Salzsäure. Die Phasen werden separiert, und die wäßrige Phase wird dreimal mit jeweils 20 ml Dichlormethan extrahiert. Die vereinigten organischen Phasen werden mit 30 ml gesättigter Kochsalz-Lösung gewaschen, über Na₂SO₄ getrocknet, filtriert und eingeengt. Das Rohprodukt (0.866 g) wird mittels MPLC (Kieselgel, h = 25 cm, d = 3.6 cm, v = 300 nm, Laufmittel Petrolether/Essigsäureethylester = 2/1) gereinigt. Nach dem Einengen und dem Trocknen im Vakuum wird das Produkt als weißgelblicher Schaum (0.866 g) erhalten.
- Ausbeute:: 0.866 g (1.95 mmol, 53 %), weißgelblicher Schaum, M_{w} = 369.3

### Methode B (CK-32-2)

2.52 g Norgalanthamin (≈ 90 %ig, 2.268 g, 8.30 mmol) und 3.45 ml (2.520 g, 24.9 mmol) Triethylamin werden in 20 ml absoluten Dichlormethan gelöst. Anschließend wird eine Lösung aus 1.211 ml (1.830 g, 8.71 mmol) Trifluoressigsäureanhydrid und 10 ml Dichlormethan innerhalb von 30 Minuten bei 0°C zugetropft. Man erwärmt auf Raumtemperatur, gibt portionsweise weiteres Trifluoressigsäureanhydrid (1.2 ml, 1.830 g, 8.71 mmol) zu und rührt die Reaktionslösung über Nacht. Die Lösung wird mit 120 ml Essigsäureethylester verdünnt und mit jeweils 50 ml 1 N Salzsäure, mit 50 ml gesättigter NaHCO₃-Lösung und zweimal mit jeweils 50 ml gesättigter Kochsalz-Lösung gewaschen, über Na₂SO₄ getrocknet und filtriert. Nach dem Einengen wird der Rückstand in 150 ml Dichlormethan gelöst, und 141.1 g einer 5 %gen NH₃-Lösung werden zugefügt. Die zweiphasige Lösung wird bei Raumtemperatur stark gerührt. Nach 30 Minuten werden die Phasen separiert, und die wäßrige Phase wird mit Dichlormethan (zweimal 50 ml) extrahiert. Die vereinigten organischen Phasen werden mit 1 N Salzsäure (zweimal 50 ml) und mit gesättigter Kochsalz-Lösung (zweimal 50 ml) gewaschen, über Na₂SO₄ getrocknet, filtriert und eingeengt. Der Rückstand (2.77 g) wird mittels MPLC (450 g Kieselgel, v = 300 nm, Laufmittel Petrolether/Essigsäureethylester = 1/1) gereinigt. Nach dem Einengen und dem Trocknen im Vakuum wird das Produkt als weißgelblicher Schaum (2.6171 g) erhalten.
- Ausbeute:: 2.6171 g (7.09 mmol, 85 %), weißgelblicher Schaum (M_{w} = 369.3)
- DC:: R_{f} = 0.23 (Petrolether/Essigsäureethylester = 1/1)
- mp.:: 65 - 68°C (Petrolether/Essigsäureethylester = 1/1)
- IR:: KBr
v (cm⁻¹) 3546.3 (v), 3417 (bv), 2924 (m), 1690 (s)
- ¹H-NMR:: (200.13 MHz, CDCl₃, TMS)
δ 6.62 - 6.92 (m, 2 H), 5.88 - 6.16 (m, 2 H), 5.25 (d, *J* = 15.2 Hz, 0.5 H), 4.85 (d, *J* = 16.6 Hz, 0.5 H), 4.42 - 4.77 (m, 2 H), 4.02 - 4.34 (m, 2 H), 3.84 (s, 3 H), 3.60 - 3.83 (m, 0.5 H), 3.27-3.50 (m, 0.5 H), 2.72 (d, *J* = 16.0 Hz, 1.0 H), 2.29 (bs, 0.7 H), 1.80 - 2.13 (m, 3 H)
- ¹³C-NMR:: (50.32 MHz, CDCl₃, TMS)
δ 156.1 (m), 146.4 und 146.(s), 144.8 und 144.7 (s), 132.0 (s), 128.8 und 128.5 (d), 126.6 und 126.1 (s), 125.7 und 125.3 (d), 120.9 und 119.1 (d), 121.9 (q, *J* = 288 Hz), 111.3 (d), 88.1 und 88.0 (d), 61.6 [d), 55.8 (q), 52.6 und 51.8 (t), 47.9 (s), 46.5 und 46.3 (t), 38.4 und 35.4 (t), 29.64 und 29.58 (t)

### Beispiel 185

### SPH-1537

### (4aS,6R,8aS)-6-Hydroxy-3-methoxy-5,6,9,10-tetrahydro-4aH-[1]benzofuro[3a,3,2-et][2]benzazepin-11(12H)-yl)carbonsäureallylester

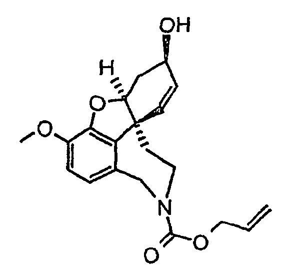

### Variante A (CK-17-1)

3.000 g (11.0 mmol) Norgalanthamin werden bei 0°C in einer Lösung aus 10 ml absolutem Dichlormethan und 4.6 ml (3.333 g, 32.9 mmol) Triethylamin vorgelegt. Bei 0°C wird eine Lösung, bestehend aus 1.454 g (12.1 mmol) Chlorameisensäureallylester und 5 ml absolutem Dichlormethan, innerhalb von 20 Minuten zugetropft. Die Reaktionslösung wird über Nacht gerührt und dabei auf Raumtemperatur erwärmt. Die Reaktionslösung wird mit 50 ml 1 N Salzsäure und 50 ml Dichlormethan aufgenommen. Die Phasen werden separiert, und die wässrige Phase wird dreimal mit jeweils 50 ml Dichlormethan extrahiert. Die vereinigten organischen Phasen werden mit 100 ml gesättigter Kochsalz-Lösung gewaschen, über Na₂SO₄ getrocknet, filtriert und eingeengt. Das Rohprodukt (3.2 g) wird mittels MPLC (Kieselgel, h = 25 cm, d = 3.6 cm, v = 300 nm, Laufmittel Petrolether/Essigsäureethylester = 2/1) getrennt. Nach dem Einengen und dem Trocknen im Vakuum erhält man das Produkt als weißgelblichen Schaum (2.594 g) und 0.232 g eines Nebenproduktes, weiches als N,O-Diallyloxycarbonylnorgalanthamin identifiziert wurde.
- Ausbeute:: 2.594 g (7.26 mmol, 66 %)
- DC:: R_{f} = 0.30 (Petrolether/Essigsäureethylester = 1/1)
- mp.:: 44 - 46°C (Petrolether/Essigsäureethylester = 1/1)
- IR:: KBr
v (cm⁻¹) 3550 (m), 3458 (m), 1700 (s)
- ¹H-NMR:: (200.13 MHz, CDCl₃, TMS)
δ 6.62 - 6.86 (m, 2 H), 5.95 - 6.09 (m, 2 H), 5.76 - 5.94 (m, 1H), 5.10 - 5.33 (m, 2 H), 4.93 und 4.83 (d und d, *J* = 15.1 Hz und J = 15.7 Hz, 1H), 4.45 -4.68 (m, 3 H), 4,05 - 4.44 (m, 3 H), 3.83 (s, 3 H), 3.27 - 3.55 (m, 1H), 2.70 (bdd, *J* = 15.1 Hz und J = 15.7 Hz, 1H), 2.26 (bs, 0.5 H), 1.93 - 2.11 (m, 1H), 1.69 - 1.92 (m, 1H)
- ¹³C-NMR:: (50.32 MHz, CDCl₃, TMS)
δ 155.1 und 155.0 (s), 146.4 (s), 144.3 (s), 132.7 (d), 132.3 und 132.0 (s), 129.1 (s), 128.0 (d), 126.2 (d), 121.4 und 120.8 (d), 117.3 und 116.7 (t), 111.1 und 110.9 (d), 88.1 und 88.0 (d), 65.9 und 65.8 (t), 61.7 (d), 55.7 (q), 51.8 und 51.3 (t), 48.2 (s), 45.8 und 45.3 (t), 37.2 und 36.3 (t), 29.7 (t)

### Beispiel 186

### SPM-1538

### (4aS,6R,8aS)-6-(2-Allyloxycarbonyloxy)-3-methoxy-5,6,9,10-tetrahydro-4aH-[1]benzofuro[3a,3,2-ef][2]benzazepin-11(12H)-yl)carbonsäureallylester

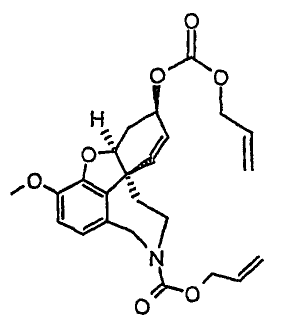

Farbloses Wachs, (M_{w} = 441.5)
- DC:: R_{f} = 0.51 (Petrolether/Essigsäureethylester= 1/1)
- IR:: KBr
v (cm⁻¹) 2947 (m), 1739 (s), 1700 (s)
- ¹H-NMR:: (200.13 MHz, CDCl₃, TMS)
δ 6.56 - 6.81 (m, 2 H), 6.22 (d, *J* = 10.3 Hz, 1H), 5.73 - 6.04 (m, 3 H), 5.08 - 5.40 (m, 5 H), 4.90 (d, *J* = 15.5 Hz, 0.5 H), 4.80 (d, *J* = 15.9 Hz, 0.5 H), 4.44 - 4.64 (m, 5 H), 4.04 - 4.43 (m, 2 H), 3.82 (s, 3 H), 3.24 - 3.54 (m, 1H), 2.78 (bd, J = 16.4 Hz, 1H), 1.65 - 2.21 (m, 3 H)
- ¹³C-NMR:: (50.32 MHz, CDCl₃, TMS)
δ 155.0 und 154.8 (s), 154.4 (s), 147.2 (s), 144.1 (s), 132.7 (d), 131.5 (d), 130.9 (s), 130.6 (d), 128.9 und 128.8 (s), 122.3 (d), 120.6 und 120.1 (d), 118.3 (t), 117.2 und 116.6 (t), 111.5 und 111.3 (d), 85.3 (d), 68.1 (t), 66.5 (d), 65.8 und 65.7 (t), 55.8 (q), 51.6 und 51.2 (t), 47.8 (s), 45.6 und 45.2 (t), 37.6 und 36.7 (t), 27.5 (t)

### Variante B (CK-17-2)

1.000 g (3.66 mmol) Norgalanthamin (68 % (HPLC, CK-1-1)), gelöst in 3 ml absolutem Dichlormethan, und 0.441 g (3.66 mmol) Chlorameisensäureallylester, gelöst in 2 ml absolutem Dichlormethan, werden analog der Methode A mit 1.48 ml (1.448g, 18.3 mmol) Pyridin umgesetzt. Nach der wäßrigen Aufarbeitung analog der Methode A und einer Säulenchromatographie (50 g Kieselgel, Laufmittel Petrolether/Essigsäureethylester = 2/1 □ 1 /1) erhält man 0.784 g eines farblosen Schaumes und 0.214 g des selben Nebenproduktes.
- Ausbeute:: 0.784 g (2.19 mmol, 60 %)

### Beispiel 187

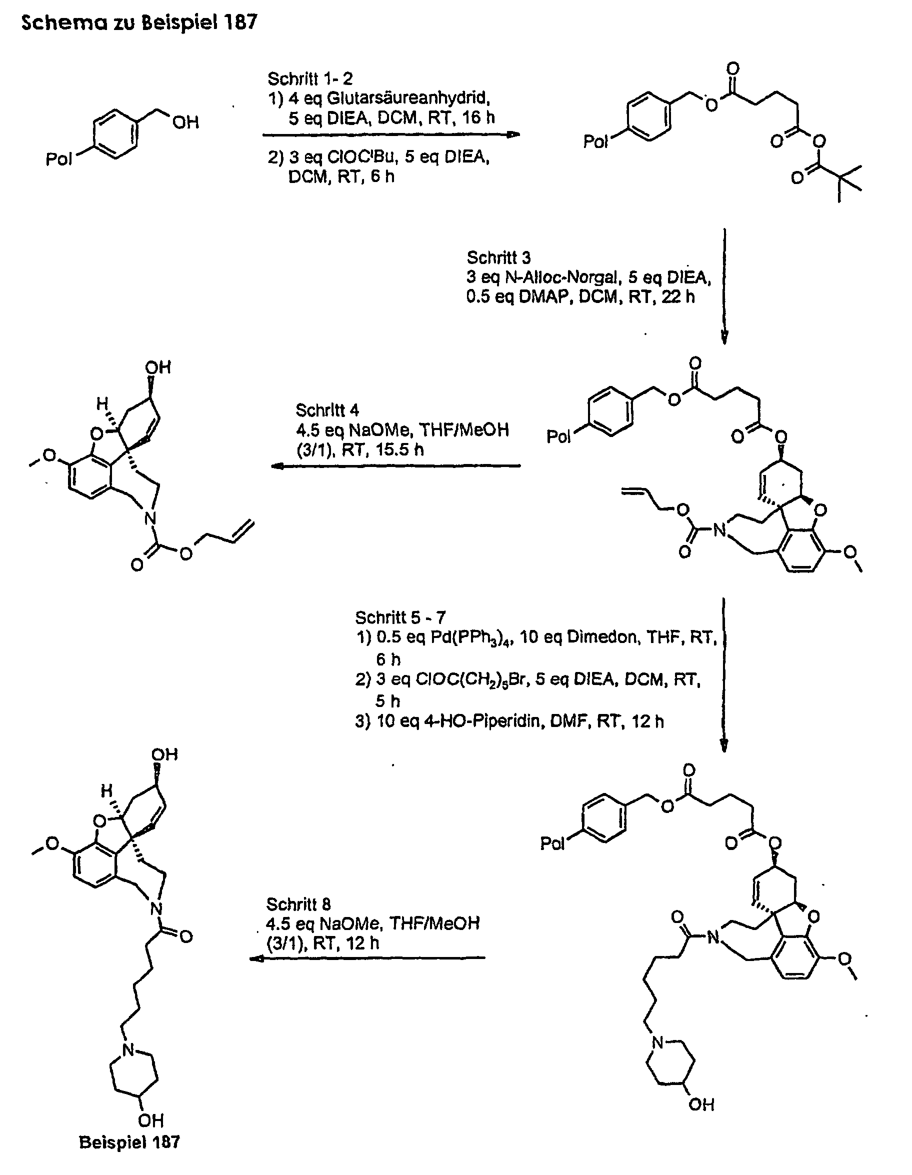

### Schritt 1 - 4

### Immobilisierung von (4aS,6R,8aS)-6-Hydroxy-3-methoxy-5,6,9,10-tetrahydro-4aH-[1]benzofuro-[3a,3,2-ef][2]benzoazepin-11(12H)-yl)carbonsäureallylester an einem Hydroxymethyl-Polystyrolharz (Merrifield-Harz)

In einer beiderseits verschlossenen 5-ml-Polyethylenfritte werden 0.200 g (0.208 mmol) Hydroxymethylpolystyrolharz (1.04 mmol/g, Merrifield-Harz³) in 3 ml Dichlormethan suspendiert und 30 Minuten ca. 40-mal pro Minute geschüttelt. Nach dem Filtrieren werden 0.095 g (0.832 mmol) Glutarsäureanhydrid, 178 µl (0.134 g, 1.04 mmol) Ethyldiisopropylamin in 2 ml Dichlormethan zugefügt, und die Suspension wird 16 Stunden bei Raumtemperatur ca. 40-mal pro Minute geschüttelt. Die Reaktionslösung wird abfiltriert, und das Harz wird einmal mit Dichlormethan, einmal mit Methanol und fünfmal mit Dichlormethan mit jeweils 2.5 ml gewaschen. Nachfolgend wird das Harz in 77 µl (0.075 g, 0.624 mmol) Pivaloylchlorid, 178 µl (0.134 g, 1.04 mmol) Ethyldiisopropylamin in 1.75 ml Dichlormethan suspendiert und sechs Stunden bei Raumtemperatur geschüttelt. Nach dem Filtrieren und dem Waschen mit Dichlormethan (1 × 2.5 ml), Tetrahydrofuran (1 × 2.5 ml) und Dichlormethan (5 × 2.5 ml) wird das Harz in einer Lösung aus 0.230 g (0.624 mmol) N-Allyloxycarbonylnorgalanthamin, 0.013 g (0.104 mmol) 4-Dimethylaminopyridin und 178 µl (0.134 g, 1.04 mmol) Ethyldiisopropylamin in 2 ml Dichlormethan bei Raumtemperatur geschüttelt. Nach 22 Stunden wird die Reaktion durch Abfiltrieren der Reaktionslösung terminiert, das Harz mit Dichlormethan (1 × 2.5 ml), mit Dimethylformamid (2 × 2.5 ml) und mit Dichlormethan (5 × 2.5 ml) gewaschen, trocken gesaugt und im Vakuum bei 30 bis 50 mbar über Nacht getrocknet.

Zur Feststellung der Beladung wird ein Aliquot des Harzes (0.262 g) in 2.5 ml Methanol/Tetrahydrofuran (1/3) 30 Minuten gequollen, filtriert und in einer Lösung von 0.168 g (0.933 mmol) 30 %ger Natriummethanolat-Methanol-Lösung in 0.5 ml Methanol und 1.5 ml Tetrahydrofuran suspendiert. Die Mischung wird 15.5 Stunden bei Raumtemperatur geschüttelt, abfiltriert, und das Harz dreimal mit Methanol/Dichlormethan (1/1, 2.5 ml) und dreimal mit Dichlormethan (2.5 ml) extrahiert. Die vereinigten Filtrate werden mit 95 µl (0.067 g, 1.248 mmol) Trifluoressigsäure neutralisiert und am Rotationsverdampfer eingeengt. Der Rückstand wird mittels Säulenchromatographie (5 g Kieselgel, Laufmittel Petrolether/Essigsäureethylester = 1/1) gereinigt. Nach dem Einengen und dem Trocknen im Hochvakuum erhält man 0.048 g eines farblosen, glasigen Feststoffes.
- Ausbeute:: 0.048 g (0.13 mmol, 65 % bezogen auf den Substitutionsgrad des Hydroxymethylharzes), ¹H-NMR-Spektrum identisch mit dem Ausgangsmaterial

### Beispiel 187/Schritt 5 - 8

### SPH-1539

### 1-(4aS,6R,8aS)-6-Hydroxy-3-methoxy-5,6,9,10-tetrahydro-4aH-[1]benzofuro[3a,3,2-ef]-[2]benzazepin-11(12H)-yl)-6-(4-hydroxy-1-piperidyl)hexan-1-on (CK-36-1) HO

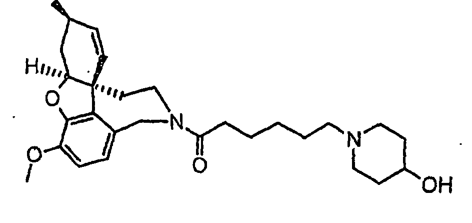

0.273 g Harz, dargestellt aus 0.200 g (0.208 mmol) Hydroxymethylpolystyrolharz¹ (1.04 mmol/g) nach der oben beschriebenen Methode A, 0.120 g (0.104 mmol) Pd(Ph₃P)₄, 0.292 g (2.08 mmol) Dimedon werden in 2.5 ml Tetrahydrofuran in einer beiderseits verschlossenen 5-ml-Polyethylenfritte sechs Stunden bei Raumtemperatur ca. 40-mal pro Minute geschüttelt. Das Harz wird filtriert und mit Dichlormethan (1 × 2.5 ml), mit Dichlormethan/Methanol/Ethyldiisopropylamin (5/4/1) (3 × 2.5 ml) und abschließend mit Dichlormethan (5 × 2.5 ml) gewaschen. Nachfolgend versetzt man das Harz mit einer Lösung aus 96 µl (0.133 g, 0.624 mmol) 6-Bromocaproylchlorid und 178 µl (0.134 g, 1.04 mmol) Ethyldiisopropylamin in 2 ml Dichlormethan und schüttelt fünf Stunden bei Raumtemperatur. Nach dem Waschen mit Dimethylformamid (6 x 2.5 ml) wird das Harz in einer Lösung aus 0.210 g (2.08 mmol) 4-Hydroxypiperidin und 2 ml Dimethylformamid zwölf Stunden bei Raumtemperatur geschüttelt. Das Harz wird dreimal mit jeweils 2.5 ml Dichlormethan und dreimal mit jeweils 2.5 ml Tetrahydrofuran gewaschen und nachfolgend in einer Lösung von 0.168 g (0.933 mmol) 30 %ger Natriummethanolat-Methanol-Lösung in 0.5 ml Methanol und 1.5 ml Tetrahydrofuran suspendiert. Nach zwölf Stunden bei Raumtemperatur wird das Harz filtriert und mit Methanol/Dichlormethan (1/1, 3×2.5 ml) und mit Dichlormethan (3 x 2.5 ml) extrahiert. Die vereinigten Filtrate werden mit 95 µl (0.067 g, 1.248 mmol) Trifluoressigsäure neutralisiert und am Rotationsverdampfer auf ca. 2 ml Volumen eingeengt. Das Rohprodukt wird mittels präparativer Dünnschichtchromatographie (PSC-Fertigplatte², Laufmittel Dichlormethan/Methanol = 9/1 + 3 % Triethylamin) gereinigt. Von der eingeengten Produktfraktion wird anschließend mittels einer Säulenfiltration über Alu miniumoxid (pH 9 - 10, Laufmittel Dichlormethan/Methanol = 20/1) Triethylammoniumtrifluoracetat abgetrennt. Nach dem Einengen und Trocknen im Hochvakuum erhält man 0.012 g des Produktes in Form eines gelblichen Schaumes.
- Ausbeute:: 0.012 g (0.025 mmol, 12 % bezogen auf den Substitutionsgrad des Hydroxymethylharzes), weißbräunliches Wachs (Mw = 470.6)

- DC:: R_{f} = 0.32 (Dichlormethan/Methanol = 8/2 + 2 % Triethylamin)
- HPLC:: t_{Ret} = 5.38 min, 98.6 % (Waters Xterra-Säule, 3.9 mm x 100 mm, RP-18, 3.5 µm, 250 nm, 1 ml/min, Acetonitril/ 20 mM Na₂B₄O₇ in H₂O (20/80 v/v, pH 10)
- ¹H-NMR:: (200.13 MHz, CDCl₃, TMS)
δ 6.81 - 6.88 und 6.61 - 6.71 (m, 2 H), 5.90 - 6.10 (m, 2 H), 4.52 - 4.75 (m, 2 H), 4.51 (d, J = 16.5 Hz, 1H), 4.15 (bs, 1H), 3.84 (s, 3 H), 3.82 (s, 1H), 3.40 - 3.60 und 3.10 - 3.30 (m, 1 H), 2.81 - 3.03 (m, 2 H), 2.70 (bd, J = 16.3 Hz, 1H), 2.33 - 2.62 (m, 4 H), 1.15 - 2.30 (m, 21H)
- LC/MS:: t_{Ref} = 8.7 min, 98 %, (Zorbax SB C 13-Säule, 2.1 mm × 30 mm, RP-18, 3 µm, 0.5 ml/min, Methanol/H₂O (40/60 □ 100/0 (v/v) in 2 min)
APCI-NI-MS
470

¹ Hydroxymethyl-Resin, D-1160, Bachem Feinchemikalien AG
² PSC-Fertigplatte von Merck, Art.-nr.: 113 895, 20 × 20 cm, 1 mm, Kieselgel 60 F₂₅₄

### Beispiel 188

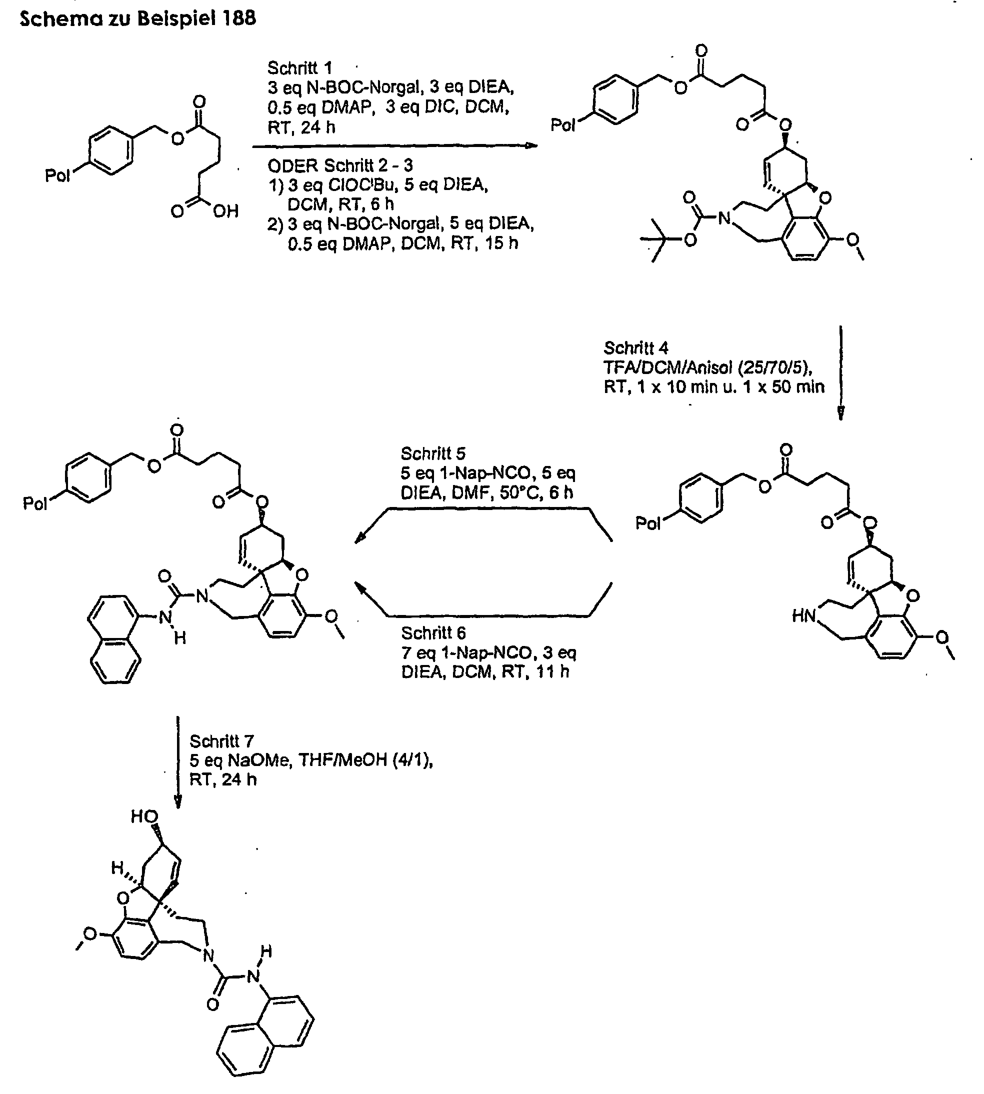

### Beispiel 188/Schritt 1 -7

### SPH-1540

### (4aS,6R,8aS)-6-Hydroxy-3-methoxy-N¹¹-(1-naphthyl)-5,6,9,10-tetrahydro-4aH-[1]benzofuro-[3a,3,2-ef]-[2]benzazepin-11(12H)-carboxamid

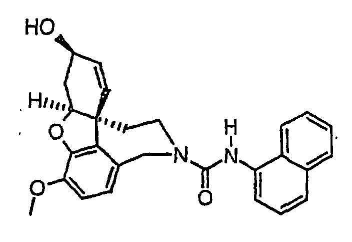

### Variante A (CK-41-3), Schritte 1, 4 und 6 - 7

0.228 g (0.212 mmol) 4-Carboxy-1-oxobut-1-yloxymethyl-Merrifieldharz wird in einer beiderseits verschlossenen 5-ml-Polyethylenfritte 30 Minuten in 3 ml Dichlormethan gequollen und nach dem Filtrieren in einer Lösung von 0.234 g (0.628 mmol) N-tert.-Butoxycarbonylnorgalanthamin, 0.013 g (0.105 mmol) 4-Dimethylaminopyridin und 108 µl (0.082 g, 0.628 mmol) Ethyldiisopropylamin in 1 ml Dichlormethan versetzt. Anschließend fügt man 97 µl (0.079 g, 0.628 mmol) Diisopropylcarbodiimid, gelöst in 1 ml Dichlormethan, zu und schüttelt die Suspension 24 Stunden ca. 40-mal pro Minute bei Raumtemperatur. Nach dem Filtrieren wird das Harz zehn Minuten in 2.5 ml Dichlormethan/Methanol (1/1) unter Schütteln suspendiert, filtriert und mit Dichlormethan (5 × 2.5 ml) gewaschen. Nachfolgend suspendiert man das Harz einmal für zehn Minuten und einmal für 50 Minuten jeweils in 2.5 ml einer Lösung aus Trifluoressigsäure, Dichlormethan und Anisol (25/70/5). Nach dem Filtrieren wird mit Dichlormethan (2 × 2.5 ml), mit Dichlormethan/Methanol/Triethylamin (5/4/1, 3 × 2.5 ml) und abschließend mit Dichlormethan (5 × 2.5 ml) gewaschen. Das Harz wird für elf Stunden in einer Lösung aus 0.208 µl (0.245 g, 1.45 mmol) 1-Naphthylisocyanat, 113 µl (0.085 g, 0.657 mmol) Ethyldiisopropylamin und 2 ml Dichlormethan bei Raumtemperatur geschüttelt. Nach jeweils dreimaligen Waschen mit Dichlormethan (2.5 ml) und Tetrahydrofuran (2.5 ml) suspendiert man das Polymer in einer Lösung von 0.188 g (1.045 mmol) 30 %ger Natriummethanolat-Methanol-Lösung in 0.4 ml Methanol und 1.6 ml Tetrahydrofuran. Nachdem das Harz 24 Stunden bei Raumtemperatur geschüttelt wurde, wird das Harz filtriert und mit Methanol/Dichlormethan (1/1, 3 x 2.5 ml) und mit Dichlormethan (3 × 2.5 ml) extrahiert. Die vereinigten Filtrate werden mit konzentrierter Salzsäure neutralisiert. Die Suspension wird über eine Kieselgelsäule (10 g, Dichlormethan/Methanol = 9/1) filtriert, und das Filtrat wird am Rotationsverdampfer eingeengt. Das Rohprodukt wird mittels präparativer Dünnschichtchromatographie (Kieselgel, Laufmittel Dichlormethan/Methanol = 47/3) gereinigt. Nach dem Einengen und Trocknen im Hochvakuum erhält man 0.091 g eines gelblichen Schaumes. Um Triethylammoniumsalze zu entfernen, wird der Rückstand in Dichlormethan aufgenommen und zweimal mit 1 N Salzsäure und einmal mit gesättigter Kochsalzlösung extrahiert, über MgSO₄ getrocknet, filtriert und eingeengt.
- Ausbeute:: 0.042 g (0.095 mmol, 45 % bezogen auf den Substitutionsgrad des 4-Carboxy-1-oxobut-1-yloxymethyl-Merrifieldharzes), braungelbliches Wachs (M_{w} = 442.5)
- DC:: R_{f} = 0.21 (Dichlormethan/Methanol = 48/2)
- HPLC:: t_{Ret} = 5.15 min, 100 % (Merck Purospher-Säule 4.0 mm x 125 mm, RP-18e, 5 µm, 250 nm, 1 ml/min, Acetonitril/ 20 mM Cl₃CCO₂H in H₂O (40/60 v/v)
- 1H-NMR:: (200.13 MHz, CDCl₃, TMS)
δ 7.71 (d, *J* = 6.9 Hz, 1H), 7.20 - 7.66 (m, 6 H), 6.85 (d, *J* = 8.4 Hz, 1H), 6.63 (d, *J* = 8.2 Hz, 1 H), 5.96 (bs, 2 H), 4.91 (d, *J* = 16.7 Hz, 1H), 4.25 - 4.62 (m, 3 H), 4.09 (bs, 1H), 3.78 (s, 3 H), 3.25 - 3.50 (m, 2 H), 2.55 - 2.67 (m, 1H), 1.87 - 2.04 (m, 2 H), 1.60 - 1.75 (m, 1H)
- ¹³C-NMR:: (50.32 MHz, CDCl₃, TMS)
δ 155.7, 146.9, 144.b, 134.4, 133.8, 132.5, 129.0, 128.7, 128.1, 128.0, 126.3, 125.6, 125.5, 124.8, 122.1, 120.7, 111.0, 88.2, 62.9, 55.9, 51.8, 48.3, 46.1, 36.4, 29.7

### Variante B, , Schritte 2 - 5 und 7

0.250 g (0.233 mmol) 4-Carboxy-1-oxobut-1-yloxymethyl-Merrifieldharz werden in einem Frittenreaktor eines Syntheserobotors (Syro II MultiSynTech) vorgelegt. Anschließend wird das Harz im Reaktor 30 Minuten in Dichlormethan aufgequollen, abgesaugt, dreimal mit Dichlormethan gewaschen und sukzessive mit 0.150 g (1.163 mmol) Ethyldiisopropylamin in 1 ml Dichlormethan und 0.084 g (0.698 mmol) Pivaloylchlorid in 1.5 ml Dichlormethan versetzt. Nach sechs stündigen Rühren bei 23°C wird die Lösung abgesaugt, und das Polymer wird mit jeweils 3 ml Dichlormethan (6 x 2 Minuten) gewaschen. Nach der Zugabe von 0.260 g (0.698 mmol) N-*tert*.-Butoxycarbonylnorgalanthamin, 0.014 g (0.116 mmol) 4-Dimethylaminopyridin und 0.150 g (1.163 mmol) Ethyldiisopropylamin in 2.5 ml Dichlormethan wird die Suspension bei 23°C für 15 Stunden gerührt. Nachdem Absaugen wird das Harz in 2.5 ml Dichlormethan/Methanol (1/1) für zehn Minuten gerührt, abgesaugt, dreimal mit Dichlormethan/Methanol (1/1) (3 ml, 2 Minuten) und fünfmal mit Dichlormethan (3 ml, 2 Minuten) gewaschen. Nachfolgend wird das Polymer einmal für zehn Minuten und einmal für 50 Minuten jeweils in 2.5 ml einer Lösung aus Trifluoressigsäure, Dichlormethan und Anisol (25/70/5) suspendiert. Nach dem Absaugen wird mit Dichlormethan (3 × 3 ml), mit Dichlormethan/Methanol/Triethylamin (5/4/1, 3 × 3 ml) und abschließend mit Dichlormethan (5 x) jeweils für zwei Minuten gewaschen. Danach versetzt man das Harz mit 0.197 g (1.163 mmol) 1-Naphthylisocyanat, 0.150 g (1.163 mmol) Ethyldiisopropylamin und 2.5 ml Dimethylformamid und rührt für sechs Stunden bei 50°C. Die Reaktion wird terminiert, indem man die Lösung absaugt, das Harz bei 23°C sechsmal für jeweils zwei Minuten mit 3 ml Dichlormethan wäscht und bei 40°C für zehn Minuten das Harz trocken saugt. Zur Abspaltung wird das Harz in eine beiderseits verschlossene 5-ml-Polyethylenfritte transferiert und in 2.5 ml Tetrahydrofuran 30 Minuten gequollen. Nach dem Filtrieren suspendiert man das Polymer in einer Lösung aus 0.209 g (1.163 mmol) 30 %ger Natriummethanolat-Methanol-Lösung in 0.75 ml Methanol und 1.25 ml Tetrahydrofuran, und schüttelt 15 Stunden ca. 40-mal pro Minute bei Raumtemperatur. Das Harz wird filtriert und dreimal mit Methanol/Dichlormethan (1/1, 2.5 ml) und dreimal mit Dichlormethan (2.5 ml) extrahiert. Die vereinigten Extrakte werden mit konzentrierter Salzsäure neutralisiert, filtriert und eingeengt. Anschließend wird das Rohprodukt mittels Säulenchromatographie über 10 g Kieselgel (Laufmittel Dichlormethan/Methanol = 99/1) gereinigt. Nach dem Einengen und Trocknen im Vakuum erhält man 0.029 g eines rosafarbenen Wachses von 75 %iger Reinheit (HPLC), identisch mit dem nach Methode A hergestellten Produkt.
- Ausbeute:: 0.029 g (0.021 g, 0.047 mmol, 20 % bezogen auf den Substitutionsgrad des 4-Carboxy-1-oxobut-1-yloxymethyl-Merrifieldharzes)
- HPLC:: t_{Ret} = 14.32 min, 75 % (Merck Purospher-Säule 4.0 mm x 125 mm, RP-18e, 5 µm, 250 nm, 1 ml/min, Acetonitril/ 20 mM Cl₃CCO₂H in H₂O (30/70 v/v)

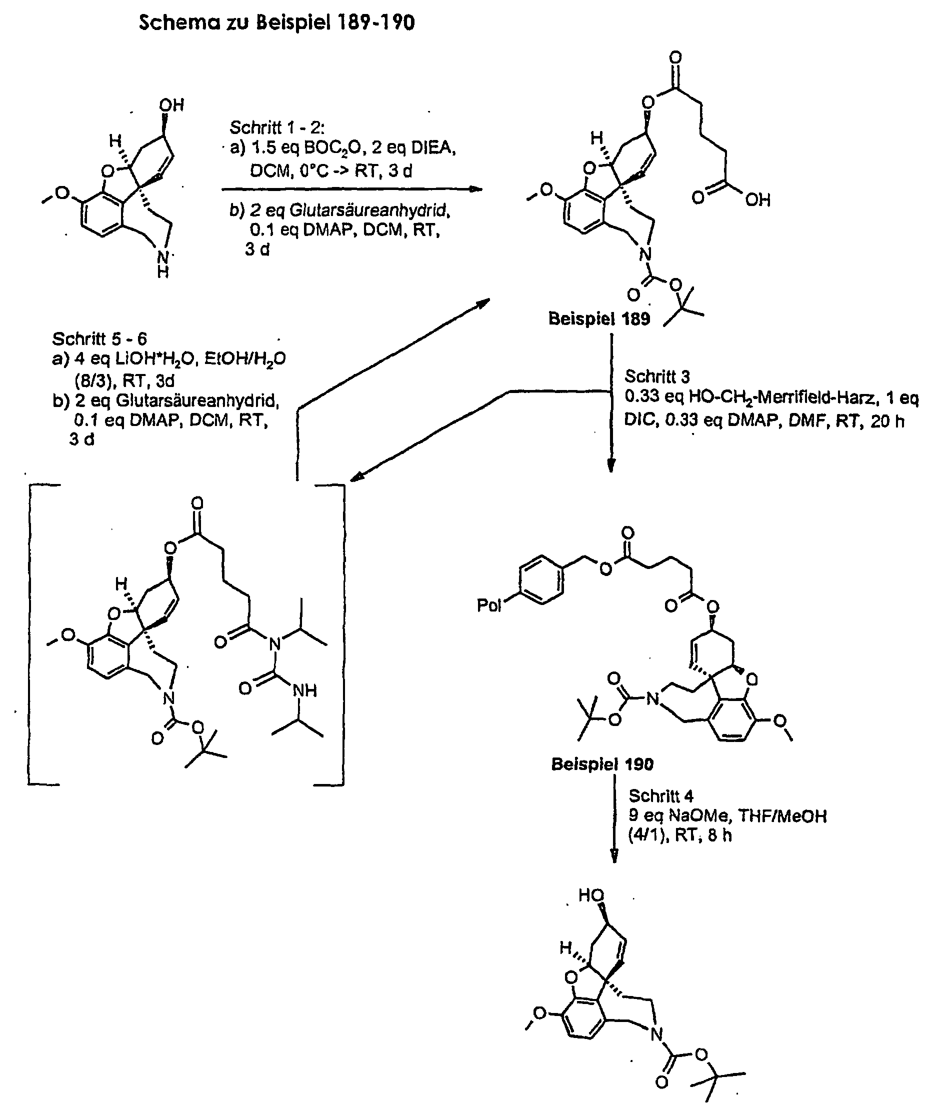

### Beispiel 189

### SPH-1541

### Schritt 1 - 2

### (4aS,6R,8aS)-3-Methoxy-11-tert.-butoxycarbonyl-5,6,9,10-tetrahydro-4aH-[1]benzoturo[3a,3,2-ef][2]benzazepin-6(12H)-yloxy)-5-oxopentansäure (CK-48-1)

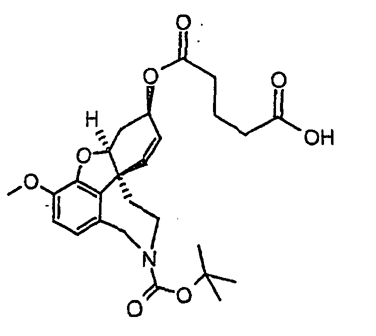

Es werden 5.000 g (18.295 mmol) Norgalanthamin (98 % (HPLC)) und 3.804 ml (2.777 g, 27.442 mmol) Triethylamin in 75 ml absolutem Dichlormethan bei 0° C vorgelegt. Unter Rühren werden innerhalb von 15 Minuten bei 0° C eine Lösung aus 4.393 g (20.124 mmol) Di-*tert*.butyldicarbonat zugetropft. Nach 40 Minuten bei 0° C wird die Reaktionslösung drei Tage lang bei Raumtemperatur gerührt. Nach zwei Tagen werden nochmals 1.598 g (7.318 mmol) Di-*tert*.butyldicarbonat und 1.27 ml (0.926 g, 9.147 mmol) Triethylamin zugefügt. Die Reaktionslösung wird mit 150 ml Dichlormethan aufgenommen, und die organische Phase wird dreimal mit jeweils 100 ml 1 N Salzsäure, dreimal mit jeweils 100 ml gesättigter NaHCO₃-Lösung und zweimal mit 100 ml gesättigter Kochsalz-Lösung gewaschen, über Na₂SO₄ getrocknet, filtriert und unter verminderten Druck eingeengt. Der Rückstand (7.065 g, 18.9 mmol, 103 % Rohausbeute) wird in 75 ml absolutem Dichlormethan aufgelöst, und zu dieser Lösung werden 4.175 g (36.590 mmol) Glutarsäureanhydrid, 0.224 g (1.829 mmol) 4-Dimethylaminopyridin und 3.804 ml (2.777 g, 27.442 mmol) Triethylamin zugefügt. Die Lösung wird bei Raumtemperatur drei Tage gerührt. Die Reaktion wird durch die Zugabe von 200 ml Diethylether und 500 ml wäßriger Ammoniaklösung (ph 10 - 11) terminiert. Die trübe wäßrige Phase wird abgetrennt (schlechte Phasentrennung, Zugabe von etwas Methanol, gegebenfalls vorher die Dichlormethan-Lösung abdestillieren) und dreimal mit 200 ml Diethylether extrahiert und anschließend mit konzentrierter Salzsäure auf pH 2 eingestellt. Dabei wird die trübe Lösung klar. Die wäßrige Phase wird viermal mit jeweils 400 ml Dichlormethan extrahiert. Die vereinigten organischen Extrakte werden dreimal mit jeweils 300 ml aqua dest und zweimal mit 300 ml gesättigter Kochsalz-Lösung gewaschen, über Na₂SO₄ getrocknet, filtriert, mit 50 ml Diisopropylether versetzt und unter verminderten Druck konzentriert, bis das Produkt auskristallisiert. Man läßt die Lösung etwas stehen und filtriert dann den auskristallisierten farblosen Feststoff ab, welcher im Vakuum getrocknet wird.
- Ausbeute:: 7.546 g (15.48 mmol, 84.6 % über beide Stufen), farbloser kristalliner Feststoff, (M_{w} = 487.6)
- DC:: R_{f} = 0.45 (Petrolether/Essigsäureethylester = 1/2)
R_{f} = 0.28 (Aluminiumoxid, Petrolether/Essigsäureethylester = 1/2)
- mp.:: 159 - 163°C (Dichlormethan/Diisopropylether = 1/1)
- IR:: KBr
ν (cm⁻¹) 3245 (bs), 2978 (s), 1715 (s), 1683 (s)
- ¹H-NMR:: (200.13 MHz, CDCl₃, TMS)
δ6.53 - 6.79 (m, 2 H), 6.13 - 6.29 (m, 1H), 5.82 - 5.97 (m, 1H), 5.33 (t, *J* = 4.9 Hz, 1.0 H), 4.87 (d, *J* = 15.6 Hz, 0.3 H), 4.67 (d, *J* = 15.7 Hz, 0.7 H), 4.53 (s, 1H), 3.99 - 4.38 (m, 2 H), 3.83 (s, 3 H), 3.19 - 3.50 (m, 1H), 2.68 (d, *J* = 16.0 Hz , 1.0 H), 2.40 (t, *J* = 7.3 Hz, 2.0 H), 2.39 (t, *J* = 7.0 Hz, 2.0 H), 2.01 - 2.17 (m, 1H), 1.93 (qui, *J* = 7.1 Hz, 2.0 H), 1.66 - 1.84 (m, 1H), 1.41 (s, 3 H), 1.37 (s, 6 H)
- ¹³C-NMR:: (50.32 MHz, CDCl₃, TMS)
δ178.2 (s), 172.6 (s), 154.9 (s), 147.1 (s), 144.0 (s), 131.3 (s), 130.5 (d), 129.6 (s), 123.0 (d), 120.2 (d), 111.0 (d), 85.8 (d), 79.9 (s), 63.2 (d), 55.8 (q), 51.8 und 51.2 (t), 48.0 (s), 45.2 (t), 37.9 und 37.0 (t), 33.4 (t) , 32.9 (t), 28.2 (q), 27.5 (t), 19.7 (t)

### Beispiel 190

### SPH-1542

### (siehe Schema Schritt 3)

### Immobilisierung von (4aS,6R,8aS)-6-Hydroxy-3-methoxy-5,6,9,10-tetrahydro-4aH-[1]benzofuro-[3a,3,2-ef][2]benzoazepin-11(12H)-yl)carbonsäure-tert.-butylester an einem Hydroxymethyl-Polystyrolharz (Merrifield-Harz) (CK-43-5)

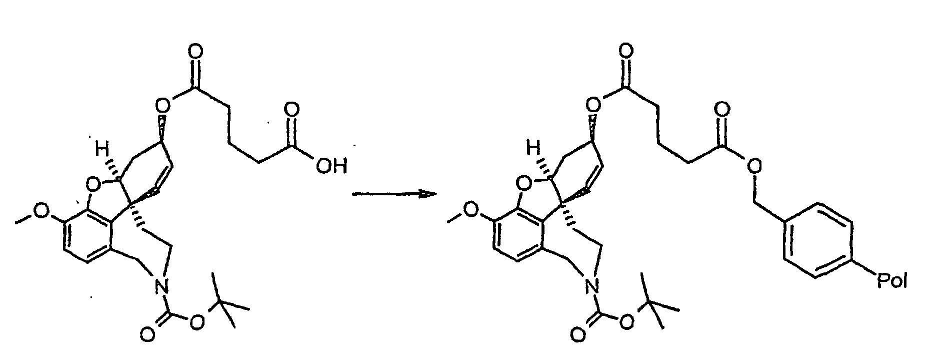

5.000 g (5.2 mmol) Hydroxymethylpolystyrolharz (1.04 mmol/g, Merrifield-Harz¹⁾ werden in einem Dreihalsglasreaktor mit einer am Boden eingelassenen Fritte und KPG-Rührer in 50 ml Dimethylformamid gerührt (300 s⁻¹). Nach dem Filtrieren wird zu dem Harz eine Lösung aus N-*tert*.-Butoxycarbonylnorgalanthamin-6-yloxy-5-oxopentansäure (7.607 g, 15.6 mmol), 4-Dimethylaminopyridin (0.635 g,5.2 mmol) in 30 ml absolutem Dimethylformamid zugegeben. Bei Raumtemperatur wird anschließend eine Lösung aus Diisopropylcarbodiimid (2.42 ml, 1.969 g, 15.6 mmol) und 10 ml Dimethylformamid portionsweise zugefügt. Nach 20 stündigem Rühren bei Raumtemperatur wird filtriert, das Harz wird sechsmal mit Dichlormethan (40 ml, 5 min) und einmal mit Diethylether (40 ml, 5 min) gewaschen und im Vakuum getrocknet.
Zur Feststellung der Beladung wird ein Aliquot des Harzes (0.2465 g) in einer beidseitig verschließbaren Polyethylenfritte in 2.5 ml Methanol/Tetrahydrofuran (1/4) 30 Minuten gequollen, filtriert und in einer Lösung von 0.280 g (1.56 mmol) 30 %ger Natriummethanolat-Methanol-Lösung in 0.5 ml Methanol und 2 ml Tetrahydrofuran suspendiert. Die Mischung wird neun Stunden bei Raumtemperatur geschüttelt, abfiltriert, und das Harz dreimal mit Methanol/Dichlormethan (1/1, 2.5 ml) und dreimal mit Dichlormethan (2.5 ml) extrahiert. Die vereinigten Filtrate werden mit 119 µl (0.178 g, 1.56 mmol) Trifluoressigsäure neutralisiert und am Rotationsverdampfer eingeengt. Der Rückstand wird in 30 ml Essigsäureethylester aufgenommen, zweimal mit gesättigter NaHCO₃-Lösung (10 ml), mit aqua dest (10 ml) und mit gesättigter Kochsalz-Lösung (10 ml) gewaschen, über Na₂SO₄ getrocknet, filtriert und erneut eingeengt. Der Rückstand (0.080 g) wird mittels Säulenchromatographie (10 g Kieselgel, Laufmittel Petrolether/Essigsäureethylester = 1/1 □ 1/2) gereinigt. Nach dem Einengen und dem Trocknen im Hochvakuum erhält man 0.0661 g eines farblosen, glasigen Feststoffes.
- Ausbeute:: 0.0661 g (0.177 mmol, Somit errechnet sich eine Beladung von 0.718 mmol/g, 103 % der theoretischen Maximalbeladung¹⁾, ¹H-NMR-Spektrum identisch mit dem Ausgangsmaterial
- HPLC:: t_{Ret} = 9.18 min, 93.8 % (270 nm) , 97.7 % [285 nm), [Phenomenex Luna-Säule, 3.0 mm × 50 mm, RP-18, 3.0 µm, 0.5 ml/min, Methanol/ 20 mM Trichloressigsäure in H₂O (50/50 v/v)

¹ Hydroxymethyl-Resin, D-1160, Bachem Feinchemikalien AG
¹ = 1.04 mmol/g / (1 g + 1 g * 1.04 mol/g * (487.6 mol/g - 18 mol/g) / 1000).

### Schritt 4

### Recyling des Überschusses an (4aS,6R,8aS)-3-Methoxy-11-tert.-butoxycarbonyl-5,6,9,10-tetrahydro-4aH-[1]benzofuro[3a,3,2-ef][2]benzazepin-6(12H)-yloxy)-5-oxopentansäure aus der Harzimmobilisierung (CK-51-1)

Das Filtrat der Reaktionslösung und die ersten fünf Dichlormethanfiltrate werden vereinigt und mit dreimal 100 ml 1 N Salzsäure, dreimal mit 100 ml aqua dest und zweimal mit gesättigter Natriumchlorid-Lösung gewaschen, mit Na₂SO₄ getrocknet, filtriert und eingeengt. Der amorphe Rückstand (6.806 g) wird in 50 ml Ethanol und 30 ml aqua dest suspendiert, anschließend fügt man 1.97 g (46.9 mmol) Lithiumhydroxid Monohydrat zu. Die Suspension wird drei Tage bei Raumtemperatur gerührt. Die Reaktionslösung wird dreimal mit jeweils 100 ml Dichlormethan extrahiert, und die vereinigten Extrakte werden jeweils mit dreimal 100 ml 1 N Salzsäure und zweimal mit 100 ml gesättigter Kochsalz-Lösung extrahiert. Die organische Phase ergibt nach dem Trocknen über Na₂SO₄, Filtrieren und Einengen unter verminderten Druck 5.06 g eines farblosen Schaumes, der laut HPLC ca. 60% N-*tert*.-Butoxycarbonylnorgalanthamin enthält. Der Rückstand wird analog der oben beschriebenen Prozedur mit 0.164 g (1.339 mmol) Dimethylaminopyridin, 3.056 g (26.78 mmol) Glutarsäureanhydrid und 2.8 ml (2.033 g, 20.09 mmol) Triethylamin in 50 ml Dichlormethan umgesetzt. Die Reaktion wird durch die Zugabe von 200 ml Diethylether und 250 ml wäßriger Ammoniaklösung (ph 10 - 11) terminiert. Die trübe wäßrige Phase wird abgetrennt und zweimal mit 200 ml Diethylether extrahiert und anschließend mit konzentrierter Salzsäure auf pH 2 eingestellt. Die wäßrige Phase wird dreimal mit jeweils 200 ml Dichlormethan extrahiert. Die vereinigten organischen Extrakte werden dreimal mit jeweils 200 ml aqua dest und zweimal mit 200 ml gesättigter Kochsalz-Lösung gewaschen, über Na₂SO₄ getrocknet, filtriert, auf ca. 50 ml eingeengt, mit 50 ml Diisopropylether versetzt und weiter unter verminderten Druck konzentriert, bis das Produkt auskristallisiert. Man läßt die Lösung etwas stehen und filtriert dann den auskristallisierten farblosen Feststoff ab, welcher im Vakuum getrocknet wird.
- Ausbeute:: 4.909 g (10.07 mmol, 96.6 % bezogen auf den eingesetzten Überschuß in der Immobilisierung)
- HPLC:: t_{Ref} = 13.9 min, 99.8 % (Merck Purospher-Säule, 4.0 mm x 125 mm, RP-18e, 5 µm, 285 nm, 1 ml/min, Acetonitril/ 20 mM Cl₃CCO₂H in H₂O (40/60 v/v, pH 10)

### Beispiel 192

### Schritt 1

### N-(Adamantan-1-yl)-6-bromhexansäureamid

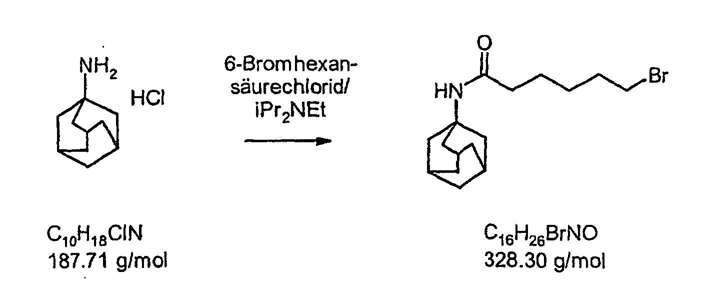

Adamantan-1-amin, Hydrochlorid (2.50 g, 13.3 mmol) und N-Ethyldiisopropylamin (3.79 g, 29.3 mmol) werden in absolutem CH₂Cl₂ (50 mL) 15 Minuten bei Raumtemperatur gerührt. Danach tropft man bei 0 °C 6-Bromhexansäurechlorid (3.13 g, 14.7 mmol) in CH₂Cl₂ (10 mL) zu und rührt eine Stunde bei Raumtemperatur.

Man extrahiert mit 2 N HCl (2 × 50 mL), Wasser (1 × 50 mL), gesättigter Natriumhydrogencarbonatlösung (2 × 50 mL) und gesättigter Kochsalzlösung (1 × 100 mL), trocknet (Natriumsulfat/Aktivkohle), filtriert und kristallisiert den nach Entfernen des Lösungsmittels am Rotationsverdampfer erhaltenen Rückstand aus Petrolether (25 mL)/Diisopropylether (25 mL) um, wodurch das Produkt in Form farbloser Kristalle vom Schmelzpunkt 73 - 75 °C erhalten wird (3.51 g, 80 %).
DC: CHCl₃ : MeOH = 9 : 1, R_{f} = 0.9
¹H NMR (CDCl₃): δ 5.43 (b, 1H), 3.33 (t, J = 6.0 Hz, 2H), 2.21 -1.15 (m, 23 H);
¹³C NMR (CDCl₃): δ 171.6 (s), 51.4 (s), 41.3 (t), 37.0 (t), 36.1 (t), 33.5 (t), 32.2 (t), 29.1 (d), 27.4 (t),
24.6 (t)

### Schritt 2

### SPH-1517

### N-(Adamantan-1-yl)-6-[(4aS,6R,8aS)-4a,5,9,10,11,12-hexahydro-6-hydroxy-3-methoxy-6Hbenzofuro[3a,3,2-ef][2]benzazepin-11-yl]-bromhexansäureamid, Fumarat

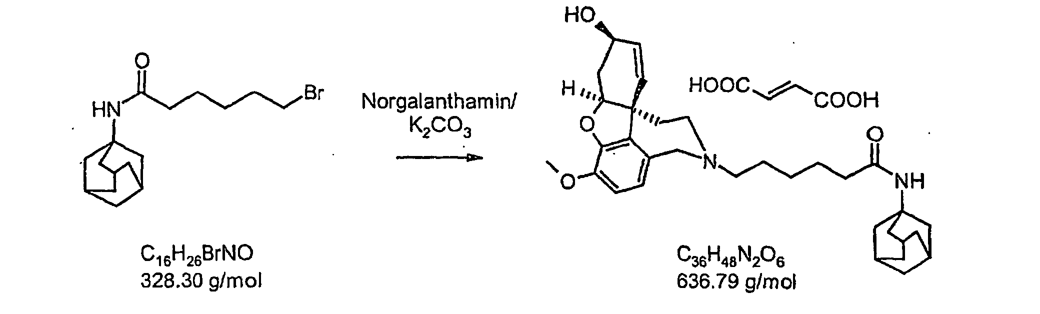

Norgalanthamin (1.00 g, 3.66 mmol), N-(Adamantan-1-yl)-6-bromhexansäureamid (1.20 g, 3.66 mmol) und Kaliumcarbonat (wasserfrei, frisch vermahlen, 1.52 g, 11.3 mmol) werden in absolutem Acetonitril (10 mL) 8 Stunden bei Siedetemperatur gerührt.

Der nach Entfernen des Lösungsmittels am Rotationsverdampfer erhaltene Rückstand wird durch Säulenchromatographie (200 g Kieselgel, Chloroform : Methanol : Ammoniak = 96 : 3 : 1) gereinigt, wodurch das Produkt als hellgelber Schaum (1.73 g, 91 %) erhalten wird.
Die Umwandlung ins Fumarat erfolgte analog zur Herstellung von MT-311 und MT-407 und lieferte das Produkt in Form hellgelber Kristalle vom Schmelzpunkt 109 - 114 °C
DC: CHCl₃ : MeOH : NH₃ = 89 : 10 : 1, R_{f} = 0.6
Mikroelementaranalyse **(JOS 1763):**
C₃₇H₄₂N₂O₉*H₂O

| | | | |
|---|---|---|---|
| Berechnet | C, 66.03; | H, 7.70; | N, 4.28 |
| Gefunden | C, 66.27; | H, 7.61; | N, 4.22 |

¹H NMR (DMSO-d₆): δ 7.20 (b, 1H), 6.90- 6.63 (m, 2H), 6.51 (s, 2H), 6.11 (d, J = 102 Hz, 1H), 5.82 (dd, J = 11.4 Hz, J = 4.7 Hz, 1H), 4.56 (s, 1H), 4.41 (d, J = 14.8 Hz, 1H), 4.22 - 3.86 (m, 2H), 3.76 (s, 3H), 3.62-3.12 (m, 3H), 2.81 -2.47 (m, 3H), 2.44 - 1.04 (m, 26 H);
¹³C NMR (DMSO-d₆): δ 171.6 (s), 167.4 (s), 146.3 (s), 144.1 (s), 134.7 (d), 132.9 (s), 129.0 (d), 126.3 (d), 124.6 (s), 122.0 (d), 111.7 (d), 86.7 (d), 59.8 (d), 55.5 (q), 50.7 (t), 50.5 (t), 47.3 (s), 41.1 (t), 36.1
(t), 36.0 (s), 32.0 (t), 31.0 (t), 28.9 (d), 26.0 (t), 25.2 (t), 24.9 (t)

### Beispiel 193

### Schritt 1

### 2-(5-Brompentyl)-1H-benz[de]isochinolin-1,3(2H)-dion

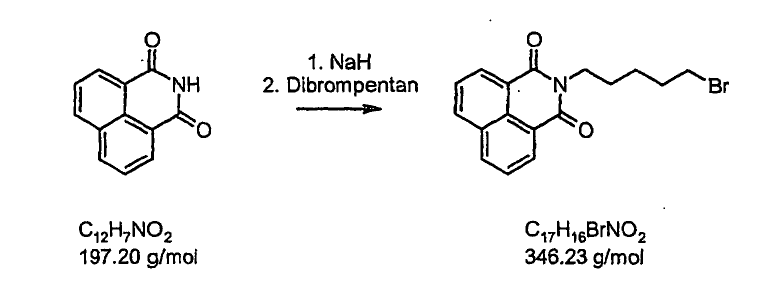

Zu einer Suspension von Natriumhydrid (2.33 g, 55.8 mmol einer 55 %igen Disperion, durch Waschen mit absolutem Petrolether von Weißöl befreit) in absolutem DMF (50 mL) wird 1 H-Benz[de]isochinolin-1,3(2*H*)-dion (10.0 g, 50.7 mmol) in DMF (50 mL) bei Raumtemperatur langsam zugetropft. Man rührt 30 Minuten, erhitzt auf 60 °C, setzt 1,5-Dibrompentan (46.64 g, 202.8 mmol) auf einmal zu und rührt 12 Stunden bei dieser Temperatur.

Man filtriert, und der nach Entfernen des Lösungsmittels am Rotationsverdampfer erhaltene Rückstand wird zwischen Wasser (200 mL) und Ether (200 mL) verteilt. Die wäßrige Phase wird mit Ether extrahiert (3 × 50 mL), die vereinigten organischen Phasen werden mit Wasser (3 × 200 mL), 2 N NaOH (2 × 100 mL) und gesättigter Kochsalzlösung (1 × 200 mL) gewaschen, getrocknet (Natriumsulfat) und am Rotationsverdampfer vom Lösungsmittel befreit. Überschüssiges Dibrompentan wird durch Destillation (100 °C/20 mbar)
abgetrennt, der Rückstand wird aus Methanol (200 mL) umkristallisiert, wodurch man das Produkt in Form farbloser Kristalle (15.45 g, 88 %) vom Schmelzpunkt 114 - 116 °C erhält.
DC: Petrolether : Essigsäureethylester = 4 : 1, R_{f} = 0.35
¹H NMR (CDCl₃): δ 8.48 (dd, J = 7.0 Hz, J = 1.3 Hz, 2H), 8.13 (dd, J = 7.0 Hz, J = 1.3 Hz, 2H), 8.48 (t, J = 7.0 Hz, 2H), 4.21 (t, J = 7.6 Hz, 2H), 3.89 (t, J = 6.6 Hz, 2H), 1.89 (Quintett, J = 6.6 Hz, 2H), 1.79 -1.43 (m,4H);
¹³C NMR (CDCl₃): δ 163.8 (s), 133.7 (d), 131.3 (s), 130.9 (d), 127.8 (s), 126.7 (d), 122.4 (d), 39.8 (t), 33.5 (t), 32.2 (t), 27.0 (t), 25.5 (t)

### Schritt 2

### SPH-1496

### 2-[5-[(4aS,6R,8aS)-4a,5,9,10,11,12-Hexahydro-6-hydroxy-3-methoxy-6H-benzofuro[3a,3,2-ef][2]benzazepin-11-yl]pentyl]-1H-benz[de]isochinolin-1,3(2H)-dion, Fumarat

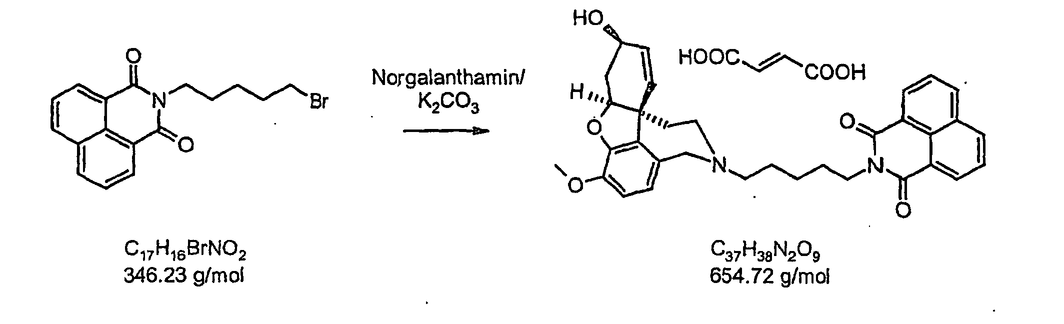

Norgalanthamin (1.00 g, 3.66 mmol), 2-(5-Brompentyl)-1*H*-benz[de]isochinolin-1,3(2*H*)-dion (1.15 g, 3.33 mmol) und Kaliumcarbonat (wasserfrei, frisch vermahlen, 1.15 g, 10.0 mmol) werden in absolutem Acetonitril (10 mL) 12 Stunden bei Siedetemperatur gerührt.
Der nach Entfernen des Lösungsmittels am Rotationsverdampfer erhaltene Rückstand wird durch Säulenchromatographie (100 g Kieselgel, Chloroform : Methanol : Ammoniak = 96 : 3 : 1) gereinigt, wodurch das Produkt als hellgelber Schaum (1.58 g, 88 %) erhalten wird.

Die Umwandlung ins Fumarat erfolgte analog zur Herstellung von MT-311 und MT-407 und lieferte das Produkt in Form hellgelber Kristalle vom Schmelzpunkt 129 - 134 °C
DC: CHCl₃ : MeOH : NH₃ = 89 : 10 : 1, R_{f} = 0.5
Mikroelementaranalyse **(JOS 1790):**
C₃₇H₃₈N₂O₉*1.5H₂O

| | | | |
|---|---|---|---|
| Berechnet | C, 65.19; | H, 6.06; | N, 4.11 |
| Gefunden | C, 65.02; | H, 5.82; | N, 3.98 |

¹H NMR (DMSO-d₆): δ 8.34 (d, *J* = 7.0 Hz, 4H), 7.76 (d, *J* = 7.0 Hz, 2H), 6.81 - 6.49 (m, 4H), 6.07 (d, J = 11.4 Hz, 1H), 5.81 (dd, J = 11.4 Hz, J = 4.7 Hz, 1H), 4.49 (s, 1H), 4.29 (d, J = 14.0 Hz, 1H), 4.16-3.74 (m, 4H), 3.70 (s, 3H), 3.43-3.01 (m, 2H), 2.50 (b, 2H), 2.27 (d, J = 14.8 Hz, 1H), 2.12-1.88 (m, 2H), 1.78 - 1.12 (m, 8H);
¹³C NMR (DMSO-d₆):δ 167.3 (s), 163.3 (s), 146.2 (s), 143.8 (s), 134.7 (d), 134.2 (d), 132.8 (s), 131.2 (s), 130.6 (d), 128.7 (d), 127.2 (d), 127.1 (s), 126.5 (d), 126.1 (s), 121.9 (s), 121.6 (d), 111.5 (d), 86.7 (d), 59.8 (d), 56.0 (t), 55.5 (q), 50.7 (t), 50.2 (t), 47.4 (s), 39.5 (t), 32.2 (t), 30.9 (t), 27.3 (t), 25.3 (t), 24.1 (t)

### Beispiel 194

### Schritt 1

### 6-Brom-1-(3,4-dimethoxyphenyl)-1-hexanon

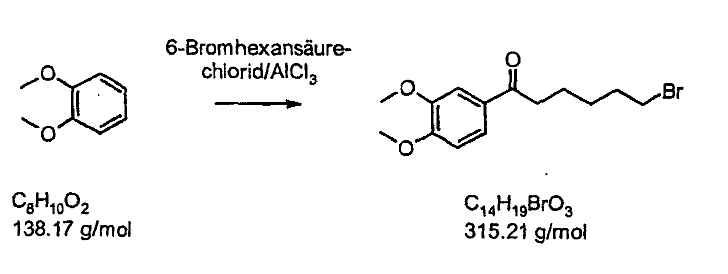

Zu einem Gemisch aus 1,2-Dimethoxybenzol (3.10 g, 22.7 mmol) und Aluminiumchlorid (3.0 g, 22.7 mmol) in absolutem Schwefelkohlenstoff (50 mL) wird bei einer Temperatur von 0 bis 5 °C 6-Bromhexansäurechlorid (4.9 g, 22.7 mmol) innerhalb von 10 Minuten zugetropft.
Man erhitzt innerhalb von 30 Minuten auf 40 °C und rührt eine Stunde bei dieser Temperatur. Man hydrolysiert mit 2 N Salzsäure (20 mL), verteilt zwischen Benzol (30 mL) und 2 N Salzsäure (30 mL) und extrahiert die wäßrige Phase mit Benzol (2 × 15 mL), wäscht die vereinigten organischen Phasen mit 2 N Salzsäure (3 × 50 mL), Wasser (1 × 50 mL), gesättigter Natriumhydrogencarbonatlösung (3 × 50 mL), gesättigter Kochsalzlösung (1 × 50 mL), trocknet (Natriumsulfat/Aktivkohle), filtriert und kristallisiert den nach Eindampfen am Rotationsverdampfer erhaltenen Rückstand aus Pentan (35 mL) um, wodurch das Produkt in Form farbloser Kristalle vom Schmelzpunkt 44 -45 °C erhalten wird (3.2 g, 44.7 %).
DC: Petrolether : Essigsäureethylester = 4 : 1; Rf = 0.85
¹H NMR (CDCl₃): δ 7.54 (dd, J = 1.9 Hz, J = 8.9 Hz, 1H), 7.51 (d, J = 1.9 Hz, 1H), 6.89 (d, J = 8.9 Hz, 1H), 3.97 (s, 6H), 3.40 (t, J = 6.4 Hz, 2H), 2.92 (t, J = 7.0 Hz, 2H), 1.90 (Quintett, J = 6.4 Hz, 2H), 1.73 (Quintett, J = 7.0 Hz, 2H), 1.63 - 1.48 (m, 2H);
¹³C NMR (CDCl₃): δ 198.6 (s), 153.2 (s), 149.0 (s), 130.2 (s), 122.6 (d), 110.1 (d), 110.0 (d), 56.0 (q), 55.9 (q), 37.7 (t), 33.6 (t), 32.6 (t), 27.9 (t), 23.6 (t)

### Schritt 2

### SPH-1497

### 1-(3,4-Dimethoxyphenyl)-6-[(4aS,6R,8aS)-4a,5,9,10,11,12-hexahydro-6-hydroxy-3-methoxy-6Hbenzofuro[3a,3,2-ef][2]benzazepin-11-yl]hexan-1-on, Fumarat

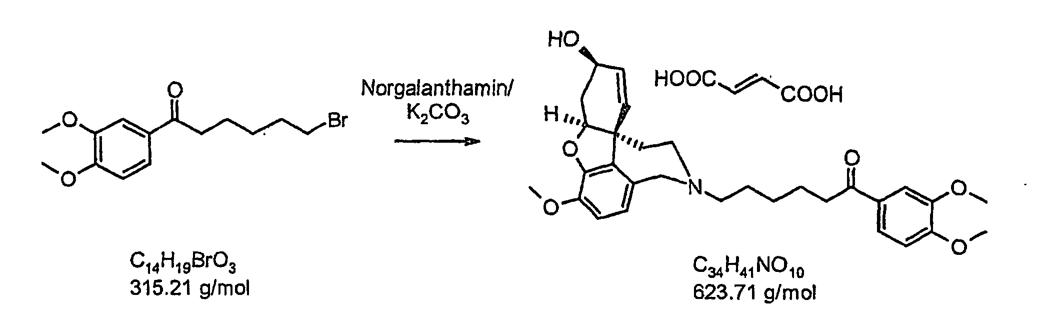

Norgalanthamin (1.00 g, 3.66 mmol), 6-Brom-1-(3,4-dimethoxyphenyl)-1-hexanon (1.15 g, 3.66 mmol) und Kaliumcarbonat (wasserfrei, frisch vermahlen, 1.15 g, 10.0 mmol) werden in absolutem Acetonitril (15 mL) 12 Stunden bei Siedetemperatur gerührt.
Der nach Entfernen des Lösungsmittels am Rotationsverdampfer erhaltene Rückstand wird durch Säulenchromatographie (100 g Kieselgel, Chloroform : Methanol : Ammoniak = 96 : 3 : 1) gereinigt, wodurch das Produkt als hellgelber Schaum (1.70 g, 91 %) erhalten wird.
Die Umwandlung zum Fumarat erfolgte nach der Standardvorschrift.

Die Umwandlung ins Fumarat erfolgte analog zur Herstellung von MT-311 und MT-407 und lieferte das Produkt in Form hellgelber Kristalle vom Schmelzpunkt 88 - 94 °C
DC: CHCl₃ : MeOH : NH₃ = 89 : 10 : 1, R_{f} = 0.5
Mikroelementaranalyse (JOS **1782**):
C₃₅H₄₃NO₁₀*0.5 H₂O

| | | | |
|---|---|---|---|
| Berechnet | C, 65.00; | H, 6.86; | N,2.17 |
| Gefunden | C, 64.81; | H, 6.64; | N,2.09 |

¹H NMR (DMSO-d₆): δ δ 7.61 (d, J = 8.9 Hz, 1H), 7.43 (s, 1H), 7.01 (d, J = 8.9 Hz, 1H), 6.81 - 6.66 (m, 2H), 6.58 [s, 2H), 6.11 (d, J = 11 Hz, 1H), 5.82 (dd, J = 11 Hz, J = 5 Hz, 1H), 4.61 - 4.33 (m 2H), 4.20 - 3.92 (m 2H), 3.84 (s, 3H), 3.80 (s, 3H), 3.72 (s, 3H), 3.62 - 3.12 (m, 2H), 3.10-2.81 (m, 2H), 2.78-2.43 (m, 3H), 2.39 - 1.86 (m, 5H), 1.78-1.40 (m, 5H), 1.38-1.14 (m, 2H);
¹³C NMR (DMSO-d₆): δ 198.5 (s), 167.2 (s), 153.0 (s), 148.6 (s), 146.3 (s), 144.2 (s), 134.6 (d), 132.9 (s), 129.7 (s), 129.0 (s), 126.2 (d), 124.0 (d), 122.7 (d), 122.1 (d), 111.7 (d), 110.9 (d), 110.2 (d), 86.6 (d), 65.0 (d), 59.8 (q), 55.8 (q), 55.5 (q), 50,8 (t), 50.4 (t), 47.3 (s), 37.2 (t), 31.9 (t), 31.0 (t), 26.1 (t), 24.7 (t), 23.8 (t), 15.2 (t)

## Patentansprüche

1. Verbindungen der allgemeinen Formel I 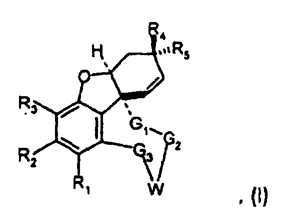 worin die Substituenten die nachstehend erläuterten Bedeutungen haben:
A) R₁ und R₂ sind gleich oder verschieden und bedeuten:
Aa) Wasserstoff, F, Cl, Br, J, CN, NC, OH, SH, NO₂, SO₃H, PO₃H, NH₂, CF₃, OSO₂(CH₂)ₙCF₃, worin n gleich 0, 1 oder 2 ist, -OSO₂-Aryl, -Vinyl- oder -Ethinyl;
Ab) eine niedrige (C₁-C₆), gegebenenfalls verzweigte Alkyl-, Aralkyl-, Alkoxyl-, Aralkoxy-, Cycloalkyl- oder Cycloalkoxygruppe,
Ac) eine Aminogruppe, die gegebenenfalls durch eine oder zwei gleiche oder verschiedene niedrige (C₁-C₆), gegebenenfalls verzweigte Alkyl-, Aralkyl-, oder Alkylcarbonyl-, Aralkylcarbonyl-, oder Alkoxycarbonyl-, Aralkoxycarbonylgruppen oder durch eine Gruppe ausgewählt aus einem Pyrrolidin-, Piperidin-, Morpholin-, Thiomorpholin-, Piperazin- oder Homopiperazinrest substituiert ist;
Ad) eine -COOH, -COOAlkyl-, -COOAralkyl-, CO-Amino-Gruppe, die gegebenenfalls wie oben unter Ac) angegeben, substituiert ist, oder eine COH-Alkylgruppe oder COH-Aralkylgruppe;
Ae) eine -(CH₂)ₙX (worin X = Br, Cl, F oder J ist), -(CH₂)ₙOH-, -(CH₂)ₙCHO- , - (CH₂)ₙCOOH-, -(CH₂)ₙCN-, -(CH₂)ₙNC-, -(CH₂)ₙCOAlkyl- oder -(CH₂)ₙCOAryl-Gruppe, worin n 1-4 ist;
Af) eine -(CH₂)ₙVinyl-, - (CH₂)ₙEthinyl-, -(CH₂)ₙ Cycloalkyl-Gruppe, worin n 0, 1 oder 2 ist, wobei Cycloolkyl ein aliphatischer Ring mit 3 bis 7 C-Atomen ist;
Ag) eine C₃-C₆ substituierte Alkenylgruppe (gegebenenfalls substituiert mit H, F, Br, Cl, CN, CO₂Alkyl, COAlkyl, COAryl);
Ah) eine C₃-C₆ substituierte Alkinylgruppe (gegebenenfalls substituiert mit H, F, Br, Cl, CN, CO₂Alkyl, COAlkyl, COAryl); oder
Ai) R₁ und R₂ bedeuten gemeinsam -CH=CH-CH=CH-, -O(CH₂)ₙO- (n =1 bis 3), -CH=CHA₁- (A₁ ist NH, O oder S), oder -CH₂CH₂A₁- (A₁ ist NH, O oder S);
B) R₃ dieselbe Bedeutung hat wie R₁, insbesondere OH und OCH₃ ist, oder
C) R₂ und R₃ gemeinsam -A₂(CH₂)ₙA₂- bedeuten, worin n 1 bis 3 ist und die Substituenten A₂ gleich oder verschieden sind und NH, O oder S bedeuten;
D) R₄ und R₅ sind entweder
Da) beide Wasserstoff,
oder
Db) einer von R₄ und R₅ ist Wasserstoff, eine Alkyl-, Aralkyl-, Alkenyl-, Aralkenyl-, Alkinyl-, oder Arolkinylgruppe und der andere von R₄ und R₅ ist
Dbi) OR₆, worin R₆ Wasserstoff, eine niedrige (C₁-C₁₀), gegebenenfalls verzweigte Alkylgruppe, oder Cycloalkylgruppe, eine C₃-C₁₀ substituierte Silylgruppe (beispielsweise Triethylsilyl, Trimethylsilyl, t-Butyldimethylsilyl oder Dimethylphenylsilyl), eine C₂-C₁₀-alpha-Alkoxyalkyl-Gruppe, beispielsweise Tetrahydropyranyl, Tetrahydrofuranyl, Methoxymethyl, Ethoxymethyl, 2-Methoxypropyl, Ethoxyethyl, Phenoxymethyl oder 1-Phenoxyethy ist;
Dbii) O-CS-NHR₆ (Thiourethane), worin R₆ die oben unter Dbi) angegebene Bedeutungen hat;
Dbiii) O-CO-NHR'₇ mit der nachstehenden Bedeutung: 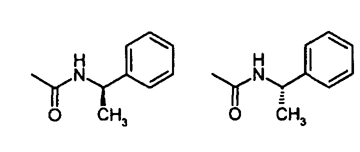
Dbiv) O-CO-HR₆, worin R₆ die oben unter Dbi) genannte Bedeutungen hat, insbesondere Ester mit den Substitutionsmuster von Aminosäuren (beide Enantiomeren), wie 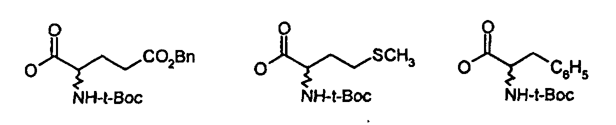
Dbv) NR₇R₇, worin die beiden Substituenten R₇ gleich oder verschieden sind und Wasserstoff, eine niedrige (C₁-C₄), gegebenenfalls verzweigte, Alkylgruppe oder Cycloalkylgruppe bedeuten, oder die Substituenten R₇ sind gemeinsam -(CH₂)ₙ-, worin n 3 bis 5 ist;
Dbvi) NH-COR₆ (Amid), worin R₆ die oben unter Dbi) genannte Bedeutungen hat;
Dbvii) S-R₆, worin R₆ die oben unter Dbi) angegebene Bedeutung hat;
Dbviii) SOₙR₈, worin n 0, 1 oder 2 ist und worin R₈ eine (C₁-C₁₀), gegebenenfalls verzweigte oder cyclische, gegebenenfalls substituierte Alkyl- oder Aralkylgruppe ist;
Dbix) R₄ ist Wasserstoff und R₅ OH, CN, CO₂-Alkyl, CONRₐR_{b}, worin Rₐ Wasserstoff, eine niedrige (C₁ - C₆), gegebenenfalls verzweigte oder cyclische substituierte Alkylgruppe und R_{b} Wasserstoff, eine niedrige (C₁ - C₆), gegebenenfalls verzweigte, oder substituierte Alkylgruppe ist, oder Ra+Rb sind gemeinsam -(CH₂)ₙ-, worin n 2 bis 6 bedeutet, oder -(CH₂)ₙE(CH₂)ₙ-, worin E gleich NH, N-Alkyl, O, oder S und n 0 bis 5 ist, Aryl (Phenyl oder Naphthyl), oder ein 6-π Heterozyklus ist;
Dbx) R₄ und R₅ sind, gemeinsam Carbonyl (=O), Hydrazon (=N-NH-R₉, =N-NR₉R₁₀) oder Oxim (=N-OR₁₀) worin R₉ Wasserstoff, eine niedrige (C₁-C₆), gegebenenfalls verzweigte oder cyclische, gegebenenfalls substituierte Alkyl-, Aralkyl-, Alkylcarbonyl-, Aralkylcarbonyl-, Alkylcarbonyloxy- oder Aralkylcarbonyloxygruppe oder eine Sulfonsäuregruppe, wie Tosyl oder Mesyl ist, und R₁₀ Wasserstoff, eine niedrige (C₁-C₆), gegebenenfalls verzweigte oder cyclische, gegebenenfalls substituierte Alkyl-, Aralkyl-, Alkylcarbonyl-, oder Arolkylcarbonylgruppe, eine Sulfonsäuregruppe, wie eine Tosyl- oder Mesylgruppe ist;
Dbxi) R₄ und R₅ sind, gemeinsam Substituenten der Art 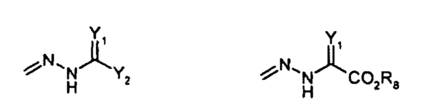
worin Y₁, Y₂ gleich oder verschieden sind und O, S, NH oder N-R₉ (freie Valenzen sind in jedem Fall Wasserstoff), worin R₉ die oben in Dbx) genannten Bedeutungen hat;
E) G₁: -(CH₂)ₓ-, worin x 1 oder 2 ist;
F) G₂: -(CH₂)_{y}-, worin y 0 bis 2 ist;
E/Fa: G₁ und G₂ bedeuten gemeinsam oder getrennt: -C(R₁₁ R₁₂)-, worin R₁₁ und R₁₂ Wasserstoff, OH, eine niedrige, gegebenenfalls verzweigte oder cyclische, gegebenenfalls substituierte Alkyl-, Aralkyl-, Aryl-, Alkyloxy-, Arakyloxy-, oder Aryloxygruppe oder gemeinsam eine Alkylspirogruppe (C₃-C₇-Spiroring) sind;
E/Fb: G₁ und G₂ bedeuten gemeinsam 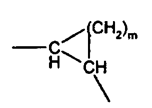 worin m 1 bis 7 ist;
E/Fc: G₁ und G₂ bedeuten gemeinsam oder getrennt -CR₁₁R₁₂-, worin R₁₁ und R₁₂ Wasserstoff, Hydroxy, eine niedrige, gegebenenfalls verzweigte oder cyclische, gegebenenfalls substituierte Alkyl-, Aralkyl-, Aryl-, Alkoxy-, Aralkoxy-, oder Aryloxygruppe bedeuten;
E/Fd: G₁ und G₂ sind gemeinsam Alkylspirogruppe (C₃-C₇-Spiroring) ;
G) G₃: -(CH₂)_{z}-, worin z 0 bis 3 ist, mit der Maßgabe, daß die Summe aus x+y+z wenigstens 2 und höchstens 4 ist, oder worin G₃ die Bedeutung Carbonyl oder Thiocarbonyl, -CH(OH)- oder -C(OH)= ist;
H) W ist:
Ha) CR₁₃R₁₄, worin R₁₃ Wasserstoff und R₁₄ -(CH₂)ₙNR₇R₇, -CO-NR₇R₇ oder -COOR₇ bedeuten, worin n 0 bis 2 ist und R₇ die oben genannten Bedeutungen hat, oder R₇ und R₇ bilden über -(CH₂)ₙ-, worin n 3 bis 5 ist, einen Ring, wobei die Substituenten R₁₃ und R₁₄ vertauscht sein können.
Hb) N-Phenyl (gegebenenfalls substituiert mit Fluor, Brom, Chlor, (C₁ -C₄)Alkyl, CO₂Alkyl, CN, CONH₂, oder Alkoxy), N-Thien- 2- oder 3-yl, oder N-Fur- 2- oder 3-yl, oder N-1,3,5-Triazinyl bedeutet, wobei der Triazinrest weiter mit Cl, OR₆ oder NR₇R₇ substituiert sein kann, und R₆ und R₇ die oben angeführte Bedeutung haben;
Hc) einer der nachstehend wiedergegebenen Substituenten 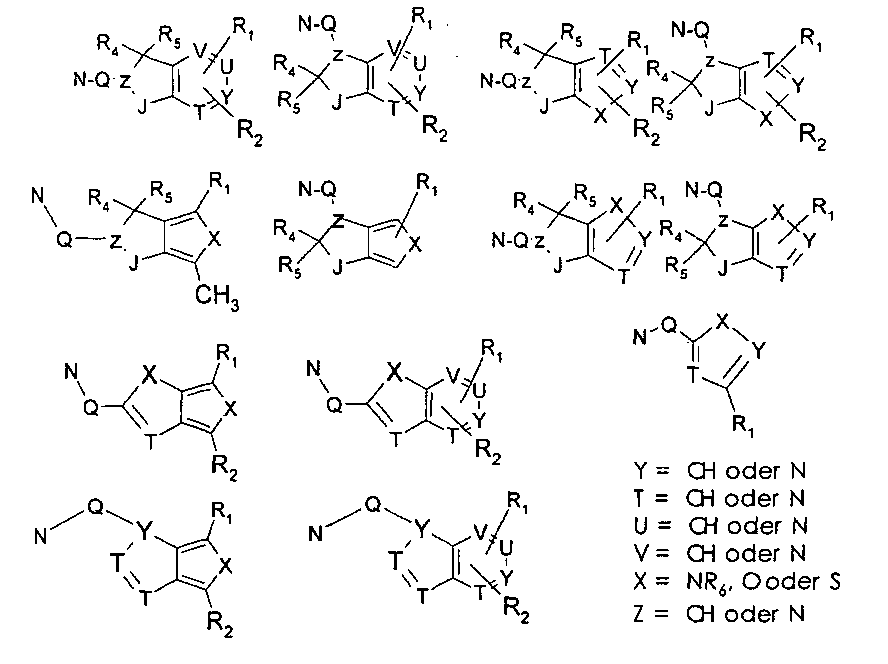 worin J keine Bindung oder -(CH₂)ₙ-, wobei n = 0 bis 3 ist, Carbonyl, Thiocarbonyl, O, S -SO oder SO₂ bedeutet, R₆ die oben angegebenen Bedeutungen hat, und worin
Q -(CH₂)ₙ- M*-(CH₂)ₘ- ist, wobei n = 0 bis 4 und m = 0 bis 4 und M* Alkinyl, Alkenyl, disubstituiertes Phenyl, disubstituiertes Thiophen, disubstituiertes Furan, disubstituiertes Pyrazin, disubstituiertes Pyridazin, einen Spacer einer der nachstehend wiedergegebenen Formeln, einen Peptidspacer L oder einen heterocyclischen Spacer HS der nachstehenden Formeln bedeutet, 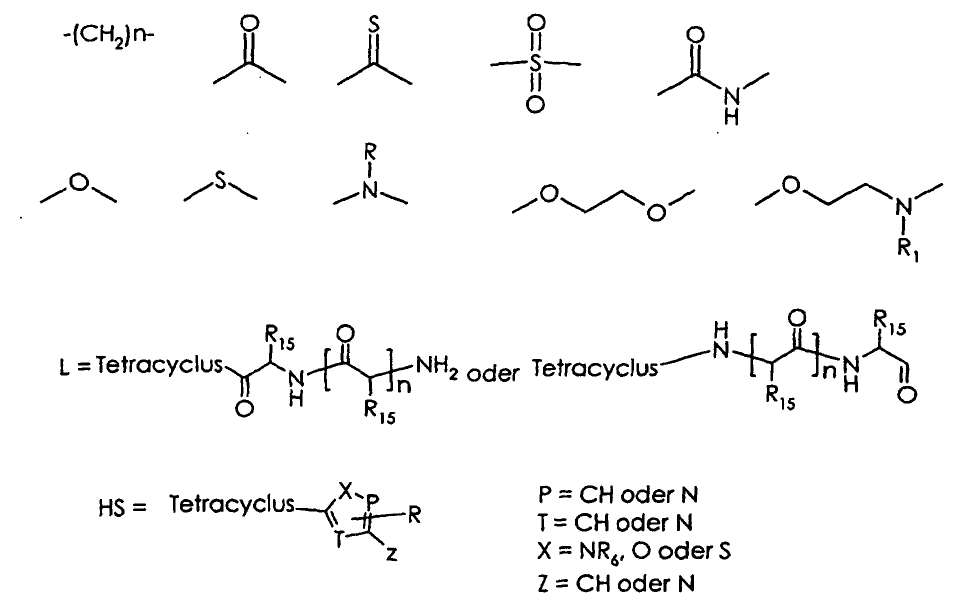 worin R₁₅ die Seitenkette von D-, L-, D,L-Aminosäuren oder unnatürlichen Aminosäuren bedeutet, und für den Fall von n>1 R₁₅ in den einzelnen Resten jeweils eine gleiche oder verschiedene Seitenkette von D-, L-, D,L-Aminosäuren oder unnatürlichen Aminosäuren bedeutet, mit der Maßgabe, daß das Atom N neben Q jeweils mit der Gruppe G2 und G3 der Formel I verbunden ist;
Hdi) ein, gegebenenfalls wenigstens einfach substituierter, tricyclischer Substituent (Tr) mit wenigstens einem heterocyclischen Ring als Ringbestandteil und einer Bindungsstelle an einem Kohlenstoffatom eines anellierten Benzolringes desselben, der über einen Spacer Q und das Q benachbarte Stickstoffatom jeweils mit G₂ und G₃ der Verbindung der Formel I verbunden ist, wobei Q die oben unter Hc) angegebene Bedeutung hat;
Hdii) ein cyclischer Kohlenwasserstoff der nachstehenden Formeln ist: 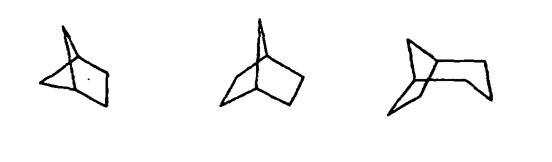 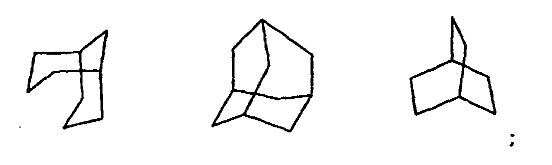
oder
He) -NH-, -O-, -S-, -SO- oder -SO₂-;
Hf) wobei, falls W -NH- ist, z in der Gruppe G₃ 0, 2 oder 3 ist, mit der Maßgabe, dass z in der Gruppe G₃ 1 ist, wenn der Substituent R₁ eine niedrige Alkylgruppe, wie Methyl oder Ethyl, bedeutet.

2. Neue Verbindungen der allgemeinen Formel II 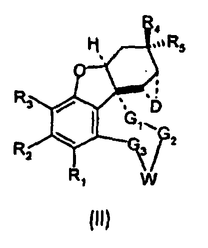 worin D N-H, N-Alkyl, N-Acyl, Sauerstoff oder Schwefel bedeutet und worin die Substituenten R₁ bis R₅, G₁ bis G₃ sowie W die in Patentanspruch 1 bei der allgemeinen Formel I angegebenen Bedeutungen haben.

3. Neue Verbindung der allgemeinen Formel III 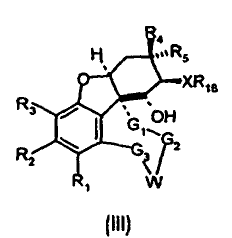 worin X-R₁₆ ein Substituent ist, in dem X Sauerstoff oder Schwefel und R₁₆ Wasserstoff oder eine niedrige (C₁-C₁₀), gegebenenfalls verzweigte, gegebenenfalls substituierte Alkyl- oder Aralkylgruppe ist, und worin die Substituenten R₁ bis R₅, G₁ bis G₃ sowie W die in Patentanspruch 1 bei der allgemeinen Formel I angegebenen Bedeutungen haben.

4. Neue Verbindung der allgemeinen Formel IV 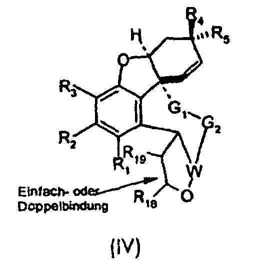 worin R₁₈ und R₁₉ Wasserstoff, Alkyl, Aryl oder Aralkyl bedeuten und in der die die Substituenten R₁₈ und R₁₉ tragenden C-Atome miteinander über eine Einfach- oder eine Doppelbindung verknüpft sind, und worin die Substituenten R₁ bis R₅ und G₁ bis G₃ die in Patentanspruch 1 bei der allgemeinen Formel I angegebenen Bedeutungen haben, wobei W -CH- oder -NHbedeutet.

5. Verbindung nach Anspruch 1, worin der 6-π-Heterozyklus Imidazolyl, Oxazolyl, Isoxazolyl, Triazolyl, Tetrazolyl, Oxadiazolyl, Thiodiazolyl, Pyridazinyl, Pyrimidinyl, Pyrazinyl und substituierte Varianten derselben, Imidazolinyl, Thiazolinyl oder Oxazolinyl ist.

6. Verbindung nach einem der Ansprüche 1 bis 5, worin R₅ eine andere Bedeutung als Wasserstoff hat und R₄ OH ist.

7. Verbindung nach Anspruch 1, worin Y₁ NH und Y₂ N-R₉ ist und worin R₄ und R₅ durch -(CH₂)ₙ-(n = 2, 3 oder 4) verbunden sind.

8. Verbindung nach einem der Ansprüche 1 bis 7, worin der tricyclische Substituent Tr ein kondensierter Benzolring der allgemeinen Formel 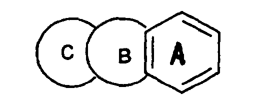 oder 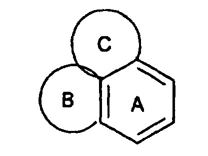 oder 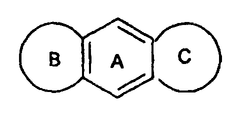 oder 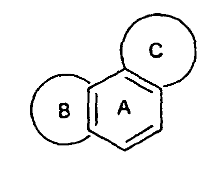 ist.

9. Verbindung nach Anspruch 8, worin der Ring A ein substituierter Benzolring ist.

10. Verbindung nach Anspruch 8 oder 9, worin einer der Ringe B und C ein gegebenenfalls substituierter heterocyclischer Ring ist und der andere ein substituierter Ring ist, der ein oder mehrere Heteroatome im Ring enthalten kann.

11. Verbindung nach einem der Ansprüche 8 bis 10, worin der Benzolring wenigstens einfach substituiert ist, wobei diese Substituenten Halogene, wie Fluor und Chlor, Halogeno-C₁-C₃-Alkylgruppen, wie Trifluormethyl, C₁-C₃-Alkylgruppen, wie Methyl, C₁-C₃-Alkoxygruppen, wie Methoxy, und die Hydroxygruppe, insbesondere ein Halogen, wie Fluor, sind.

12. Verbindung nach einem der Ansprüche 8 bis 11, worin der gegebenenfalls substituierte heterocyclische Ring B oder C ein 4 bis 14-gliedriger Ring, vorzugsweise ein 5 bis 7-gliedriger Ring, insbesondere ein 5- bis 7-gliedriger, nichtaromatischer Ring, der ein oder zwei gleiche oder verschiedene Heteroatome enthält, ist.

13. Verbindung nach Anspruch 12, worin wenigstens ein Heteroatom des heterocyclischen Ringes (1 bis 3 Heteroatome sind möglich) Stickstoff, Sauerstoff, oder Schwefel sind.

14. Verbindung nach Anspruch 13, worin der heterocyclische Ring B oder C Pyridin, Pyrazin, Pyrimidin, Imidazol, Furan, Thiophen, Pyrrolidin, Piperidin, Hexamethylenimin, Tetrahydrofuran, Piperazin, Morpholin oder Thiomorpholin ist.

15. Verbindung nach einem der Ansprüche 8 bis 14, worin der 5- bis 8-gliedrige Ring B oder C ein 5 bis 8-gliedriger heterocyclischer oder alicyclischer Ring, oder ein Kohlenstoffring, der wenigstens einfach substituiert ist, ist.

16. Verbindung nach Anspruch 15, worin der 5 bis 8-gliedrige Kohlenstoff-Ring ein Benzolring oder ein gesättigter oder ungesättigter Ring, beispielsweise Benzol, Cyclopentan, Cyclopenten, Cyclohexan, Cyclohexen, Cyclohexadien, Cycloheptan, Cyclohepten und Cycloheptadien ist.

17. Verbindung nach einem der Ansprüche 1 bis 16, worin der trizyklische Substituent Tr eine Gruppe einer der nachstehend wiedergegebenen Formeln ist

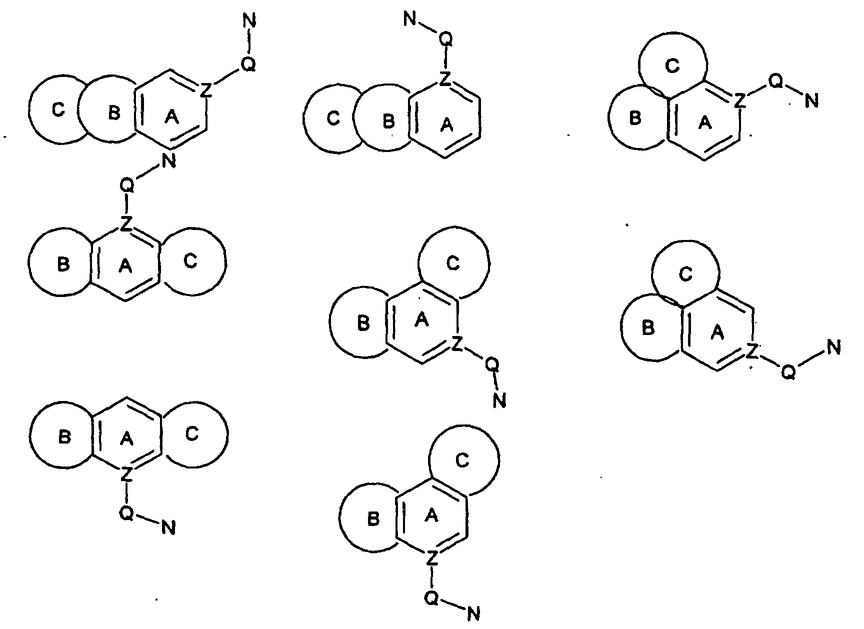

18. Verbindung noch einem der Ansprüche 1 bis 17, worin der trizyklische Substituent Tr eine Gruppe einer der nachstehend wiedergegebenen Formeln ist

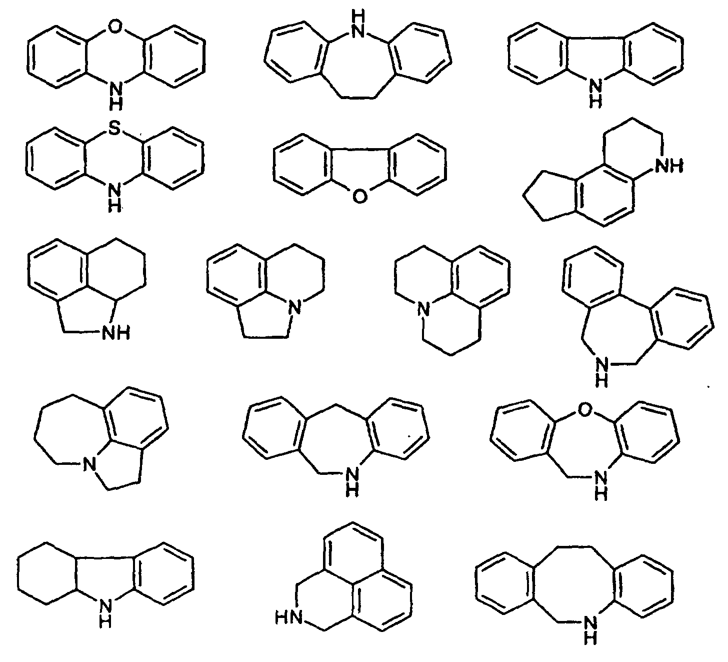

19. Verbindung nach einem der Ansprüche 1 bis 18, worin der Substituent Tr wenigstens einfach mit R₁ substituiert ist und R₁ die in Anspruch 1 unter A) angegebenen Bedeutungen hat.

20. Verbindung nach einem der Ansprüche 1 bis 19, worin der Substituent W -NH- ist und/oder der Substituent G₁ -(CH₂)ₓ-, worin x gleich 1 oder 2 ist und G₂ -(CH₂)_{y}-, worin y gleich 0 bis 2 ist, mit der Maßgabe, daß x + y gemeinsam wenigstens 2 und höchstens 4 bedeuten.

21. Verfahren zum Herstellen von Verbindungen der Ansprüche 1 bis 20, **dadurch gekennzeichnet, daß** die kombinatorische oder Parallel-Synthesetechnologie angewendet wird, wobei das Grundmolekül durch eine funktionelle Gruppe (Linker) auf einer festen Phase immobilisiert, die Synthese der Zielverbindung ausgeführt und dann diese Zielverbindung von der festen Phase abgetrennt wird.

22. Verfahren nach Anspruch 21, **dadurch gekennzeichnet, daß** das Grundmolekül an der festen Phase über ein Kohlenstoffzentrum, ein Stickstoffzentrum oder ein Sauerstoffzentrum immobilisiert wird.

23. Verfahren nach Anspruch 21 oder 22, **dadurch gekennzeichnet, daß** als funktionelle Gruppe (Linker) = -X(CH₂)ₙCO(X = CH₂, CO, O, S, NH), -X(CH₂)ₙOCO (X = CH₂, CO, O, S, NH), -XC₆H₄CH₂-(X = CH₂, CO, O, S, NH), THP, -X(CH₂)ₙSi(alkyl)₂- verwendet wird.

24. Verfahren nach Anspruch 21 oder 22, **dadurch gekennzeichnet, daß** als funktionelle Gruppe (Linker) = -X(CH₂)ₙCO(X = CH₂, O, NH, SOₒ₋₂), -X(CH₂)ₙCS (X = CH₂, O, NH SOₒ₋₂), X( CH₂)ₙJCO (X = CH₂, O, NH, Soₒ₋₂; J = NH, O, S), XC₆H₄CH₂(X = CH₂, O, S) verwendet wird.

25. Verfahren nach Anspruch 21 oder 22, **dadurch gekennzeichnet, daß** als funktionelle Gruppe (Linker) = -(CH₂)ₙSi(alkyl)₂-, -C₆H₄Si(alkyl)₂-, -(CH₂)ₙSn(alkyl)₂-, -C₆H₄Sn(alkyl)₂, -(CH₂)ₙS, -C₆H₄S verwendet wird.

26. Arzneimittel enthaltend wenigstens eine der Verbindungen der allgemeinen Formeln I, II, III oder IV, oder ein pharmazeutisch annehmbares Salz derselben als Wirkstoff.

27. Arzneimittel nach Anspruch 26 zur Behandlung
a) Behandlung der Alzheimer'schen Krankheit,
b) Behandlung der Parkinson'schen Krankheit,
c) Behandlung der Huntington'schen Krankheit (Chorea),
d) Behandlung der Multiplen Sklerose,
e) Behandlung der Amyotrophen Lateralsklerose,
f) Behandlung der Epilepsie,
g) Behandlung der Folgen des Schlaganfalles,
h) Behandlung der Folgen des Schädel-Hirn-Traumas,
i) Behandlung und Prophylaxe der Folgen diffusen Sauerstoff- und Nährstoffmangels im Gehirn, wie sie nach Hypoxie, Anoxie, Asphyxie, Herzstillstand, Vergiftungen, sowie bei Komplikationen bei schweren Geburten am Säugling oder bei Narkosen beobachtet werden,
j) insbesondere auch prophylaktischen Behandlung apoptotischer Degeneration in Neuronen, die durch lokale Radio- oder Chemotherapie von Gehimtumoren geschädigt wurden bzw. werden, und
k) Behandlung der bakteriellen Meningitis und
l) Behandlung von Erkrankungen mit apoptotischer Komponente, besonders im Gefolge von amyloid-assoziierter Zelldegeneration,
m) Behandlung des Diabetes mellitus, insbesondere, wenn die Krankheit mit Amyloiddegeneration der Inselzellen einhergeht,
n) zum Erhöhen der Muskelkraft und
o) zum Erhöhen der Ausdauer von Alzheimer-Patienten.

28. Verwendung wenigstens einer der Verbindungen der allgemeinen Formeln I, II, III oder IV, oder ein pharmazeutisch annehmbares Salz derselben zum Herstellen von Arzneimitteln.

29. Verwendung nach Anspruch 28 zum Herstellen von Arzneimitteln zur Behandlung
a) Behandlung der Alzheimer'schen Krankheit,
b) Behandlung der Parkinson'schen Krankheit,
c) Behandlung der Huntington'schen Krankheit (Chorea),
d) Behandlung der Multiplen Sklerose,
e) Behandlung der Amyotrophen Lateralsklerose,
f) Behandlung der Epilepsie,
g) Behandlung der Folgen des Schlaganfalles,
h) Behandlung der Folgen des Schädel-Him-Traumas,
i) Behandlung und Prophylaxe der Folgen diffusen Sauerstoff- und Nährstoffmangels im Gehirn, wie sie nach Hypoxie, Anoxie, Asphyxie, Herzstillstand, Vergiftungen, sowie bei Komplikationen bei schweren Geburten am Säugling oder bei Narkosen beobachtet werden,
j) insbesondere auch prophylaktischen Behandlung apoptotischer Degeneration in Neuronen, die durch lokale Radio- oder Chemotherapie von Gehimtumoren geschädigt wurden bzw. werden, und
k) Behandlung der bakteriellen Meningitis und
l) Behandlung von Erkrankungen mit apoptotischer Komponente, besonders im Gefolge von amyloid-ossoziierter Zelldegeneration,
m) Behandlung des Diabetes mellitus, insbesondere, wenn die Krankheit mit Amyloiddegeneration der Inselzellen einhergeht, sowie
n) zum Erhöhen der Muskelkraft und
o) zum Erhöhen der Ausdauer von Alzheimer-Patienten.

30. Verfahren zum Herstellen von Arzneimitteln, bei dem wenigstens eine der Verbindungen der allgemeinen Formeln I, II, III oder IV mit einem pharmazeutisch annehmbaren Träger und/oder Formulierungshilfsmittel gemischt wird.

31. Verfahren zum Trennen der (+) und (-)-Isomeren von racemischen Verbindungen der allgemeinen Formeln I, II, III oder IV, insbesondere von racemischen (6R)-3-Methoxy-5,6,9,10,11,12-hexohydro-4aH[1]benzofuro[3a,3,2-ef][2-benzyzepin-6-ol (Norgalanthamin) aus einem Isomerengemisch, **dadurch gekennzeichnet, dass** das Verfahren der fraktionierten Kristallisation mit einer chiralen Säure angewendet wird.

32. Verfahren nach Anspruch 31, **dadurch gekennzeichnet, dass** das Isomerengemisch in einem Lösungsmittel gelöst oder suspendiert wird, die so erhaltene Lösung oder Suspension mit einer Lösung der chiralen Säure versetzt und dann mit Impfkristallen eines Salzes aus einem (-)-Isomeren bzw. (+)-Isomeren der gewünschten Verbindung und einer (+) bzw. (-) chiralen Säure geimpft wird, kristallisieren gelassen wird, die gebildeten Kristalle abgetrennt und getrocknet werden und schließlich das (+)-Isomere bzw. das (-)-Isomere der gewünschten Verbindung der allgemeinen Formeln I, II, III oder IV durch Extrohieren der Kristalle mit einem organischen Lösungsmittel, nachdem diese mit einer Base, wie NH₄ OH, versetzt worden sind, isoliert.

33. Verfahren nach Anspruch 31 oder 32, **dadurch gekennzeichnet, dass** als chirale Säure eine Säure ausgewählt aus der Gruppe bestehend aus (+) oder (-)-Weinsäure, substituierter Weinsäure oder (+) oder (-)-O,O-Di-p-Toluylweinsäure verwendet wird.

34. Verfahren nach einem der Ansprüche 31 bis 33, **dadurch gekennzeichnet, dass** das Isomerengemisch in der 3- bis 50-fachen Menge eines Lösungsmittels ausgewählt aus der Gruppe bestehend aus Wasser, Methanol, Ethanol, Propanol, Isopropanol und Aceton oder einem Gemisch aus wenigstens zwei der genannten Lösungsmittel gelöst oder suspendiert wird.

35. Verfahren nach einem der Ansprüche 32 bis 34, **dadurch gekennzeichnet, dass** zum Extrahieren ein Lösungsmittel ausgewählt aus der Gruppe bestehend aus Chloroform, Methylenchlorid, Ethylacetat, Butylacetat, Diethylether, t-Butylmethylether, Dibutylether, Petrolether, Xylol, Benzol und Toluol verwendet wird.

36. Verfahren nach einem der Ansprüche 32 bis 35, **dadurch gekennzeichnet, dass** das gewünschte Isomere durch Abdestillieren des Lösungsmittels isoliert wird.

## Claims

1. Compounds of the general formula I 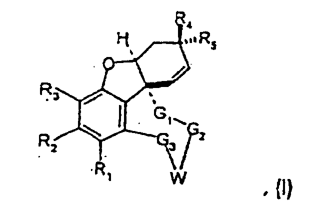 wherein the substituents have the subsequently explained meanings:
A) R₁ and R₂ are the same or different and mean:
Aa) hydrogen, F, Cl, Br, I, CN, NC, OH, SH, NO₂, SO₃H, PO₃H, NH₂, CF₃, OSO₂(CH₂)ₙCF₃, wherein n is equal to 0, 1 or 2, -OSO₂-aryl, -vinyl- or -ethinyl;
Ab) a low (C₁-C₆), if necessary branched alkyl, aralkyl, alkoxyl, aralkoxy, cycloalkyl or cycloalkoxy group,
Ac) an amino group, which if necessary is substituted by one or two identical or different low (C₁-C₆), if necessary branched alkyl, aralkyl, or alkylcarbonyl, aralkylcarbonyl or alkoxycarbonyl, aralkoxycarbonyl groups or by a group selected from a pyrrolidine, piperidine, morpholine, thiomorpholine, piperazine or homopiperazine radical;
Ad) a -COOH, -COOalkyl, -COOaralkyl, CO-amino group, which if necessary as indicated above under Ac), is substituted, or a COH-alkyl group or COH-aralkyl group;
Ae) a -(CH₂)ₙX (wherein X = Br, Cl, F or I), -(CH₂)ₙOH, -(CH₂)ₙCHO, -(CH₂)ₙCOOH, -(CH₂)ₙCN, -(CH₂)ₙNC, -(CH₂)ₙCOalkyl, or -(CH₂)ₙCOaryl group, wherein n is 1 - 4;
Af) a -(CH₂)ₙvinyl, -(CH₂)ₙethinyl, -(CH₂)ₙcycloalkyl group, wherein n is 0, 1 or 2, cycloalkyl being an aliphatic ring with 3 to 7 C-atoms;
Ag) a C₃-C₆ substituted alkenyl group (if necessary substituted by H, F, Br, Cl, CN, CO₂alkyl, COalkyl, COaryl);
Ah) a C₃-C₆ substituted alkinyl group (if necessary substituted by H, F, Br, Cl, CN, CO₂alkyl, COalkyl, COaryl); or
Ai) R₁ and R₂ mean together -CH=CH-CH=CH-, -O(CH₂)ₙO- (n = 1 to 3), -CH=CHA₁- (A₁ is NH, O or S), or -CH₂CH₂A₁- (A₁ is NH, O or S);
B) R₃ has the same meaning as R₁, in particular is OH and OCH₃, or
C) R₂ and R₃ together mean -A₂(CH₂)ₙA₂-, wherein n is 1 to 3, and the substituents A₂ are the same or different and mean NH, O or S;
D) R₄ and R₅ are either
Da) both hydrogen,
or
Db) one of R₄ and Rs is hydrogen, an alkyl, aralkyl, alkenyl, aralkenyl, alkinyl or aralkyinyl group and the other of R₄ and R₅ is
Dbi) OR₆, wherein R₆ is hydrogen, a low (C₁-C₁₀), if necessary branched alkyl group, or cycloalkyl group, a C₃-C₁₀ substituted silyl group (for example triethylsilyl, trimethylsilyl, t-butyldimethylsilyl or dimethylphenylsilyl), a C₂-C₁₀-alphaalkoxyalkyl group, for example tetrahydropyranyl, tetrahydrofuranyl, methoxymethyl, ethoxymethyl, 2-methoxypropyl, ethoxyethyl, phenoxymethyl or 1-phenoxyethyl;
Dbii) O-CS-NHR₆ (thiourethane), wherein R₆ has the meanings indicated above under Dbi);
Dbiii) O-CO-NHR'₇ with the subsequent meaning: 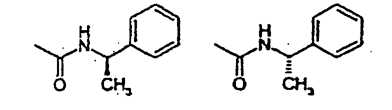
Dbiv) O-CO-HR₆, wherein R₆ has the meanings indicated above under Dbi), in particular esters with the substitution pattern of amino acids (both enantiomers), such as 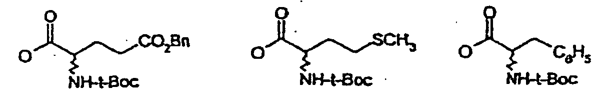
Dbv) NR₇R₇, wherein both substituents R₇ are the same or different and mean hydrogen, a low (C₁-C₄), if necessary branched, alkyl group or cycloalkylgroup, or the substituents R₇ are together -(CH₂)ₙ-, wherein n is 3 to 5;
Dbvi) NH-COR₆ (amide), wherein R₆ has the meanings mentioned above under Dbi);
Dbvii) S-R₆, wherein R₆ has the meaning indicated above under Dbi);
Dbviii) SOₙR₈, wherein n is 0, 1 or 2 and wherein R₈ is a (C₁-C₁₀), if necessary branched or cyclic, if necessary substituted alkyl or aralkyl group;
Dbix) R₄ is hydrogen and Rs OH, CN, CO₂-alkyl, CONRₐR_{b}, wherein Rₐ is hydrogen, a low (C₁-C₆), if necessary branched or cyclic substituted alkyl group and R_{b} is hydrogen, a low (C₁-C₆), if necessary branched or substituted alkyl group, or Rₐ+R_{b} are together -(CH₂)ₙ-, wherein n means 2 to 6, or -(CH₂)ₙE(CH₂)ₙ-, wherein E is equal to NH, N-alkyl, O, or S and n is 0 to 5, aryl (phenyl or naphthyl), or is a 6-π heterocycle;
Dbx) R₄ and R₅ are together carbonyl (=O), hydrazone (=N-NH-R₉, =N-NR₉R₁₀) or oxime (=N-OR₁₀), wherein R₉ is hydrogen, a low (C₁-C₆), if necessary branched or cyclic, if necessary substituted alkyl, aralkyl, alkycarbonyl, aralkylcarbonyl, alkylcarbonyloxy or aralkylcarbonyloxy group or a sulphonic acid group, such as tosyl or mesyl, and R₁₀ is hydrogen, a low (C₁-C₆), if necessary branched or cyclic, if necessary substituted alkyl, aralkyl, alkylcarbonyl or aralkylcarbonyl group, a sulphonic acid group, such as a tosyl or mesyl group,
Dbxi) R₄ and R₅ are together substituents of the type 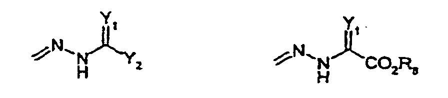
wherein Y₁, Y₂ are the same or different and O, S, NH or N-R₉ (free valencies are in each case hydrogen), wherein R₉ has the meanings mentioned above in Dbx);
E) G₁: -(CH₂)ₓ-, wherein x is 1 or 2;
F) G₂: -(CH₂)_{y}-, wherein y is 0 to 2;
E/Fa: G₁ and G₂ mean together or separately: -C(R₁₁ R₁₂)-, wherein R₁₁ and R₁₂ are hydrogen, OH, a low, if necessary branched or cyclic, if necessary substituted alkyl, aralkyl, aryl, alkyloxy, aralkyloxy, or aryloxy group or together an alkylspiro group (C₃-C₇ spiroring);
E/Fb: G₁ and G₂ mean together 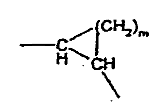 wherein m is 1 to 7;
E/Fc: G₁ and G₂ mean together or separately -CR₁₁R₁₂-, wherein R₁₁ and R₁₂ mean hydrogen, hydroxy, a low, if necessary branched or cyclic, if necessary substituted alkyl, aralkyl, aryl, alkoxy, aralkoxy, or aryloxy group;
E/Fd): G₁ and G₂ are together an alkylspiro group (C₃-C₇ spiroring);
G) G₃: -(CH₂)_{z}-, wherein z is 0 to 3, with the proviso that the sum of x+y+z is at least 2 and at most 4 or, wherein G₃ has the meaning carbonyl or thiocarbonyl, is - CH(OH)- or -C(OH);
H) W: is
Ha) CR₁₃R₁₄, wherein R₁₃ means hydrogen and R₁₄ -(CH₂)ₙNR₇R₇, -CO-NR₇R₇ or -COOR₇, wherein n is 0 to 2 and R₇ has the above-mentioned meanings, or R₇ and R₇ form a ring via -(CH₂)ₙ-, wherein n is 3 to 5, the substituents R₁₃ and R₁₄. being able to be interchanged.
Hb) means N-phenyl (if necessary substituted by fluorine, bromine, chlorine, (C₁-C₄)alkyl, CO₂alkyl, CN, CONH₂ or alkoxy), N-thien-2- or -3-yl, or N-fur-2- or -3-yl, or N-1,3,5,-triazinyl, the triazine radical being able to be further substituted by Cl, OR₆ or NR₇R₇, and R₆ and R₇ have the above-cited meaning;
Hc) one of the subsequently reproduced substituents 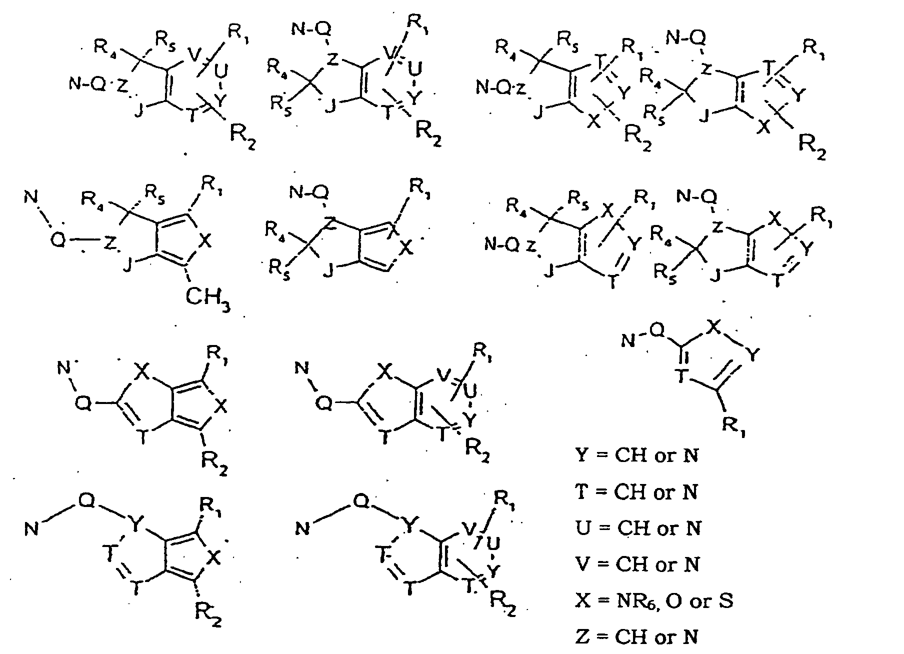 wherein J means no bond or -(CH₂)ₙ-, n being 0 to 3, carbonyl, thiocarbonyl, O, S, - SO or SO₂, R₆- has the above-indicated meanings, and wherein
Q is -(CH₂)ₙ- M*-(CH₂)ₘ-, n = 0 to 4 and m = 0 to 4 and M* meaning alkinyl, alkenyl, disubstituted phenyl, disubstituted thiophene, disubstituted furan, disubstituted pyrazine, disubstituted pyridazine, a spacer of the subsequently reproduced formulae, a peptide spacer L or a heterocyclic spacer HS of the subsequent formulae, 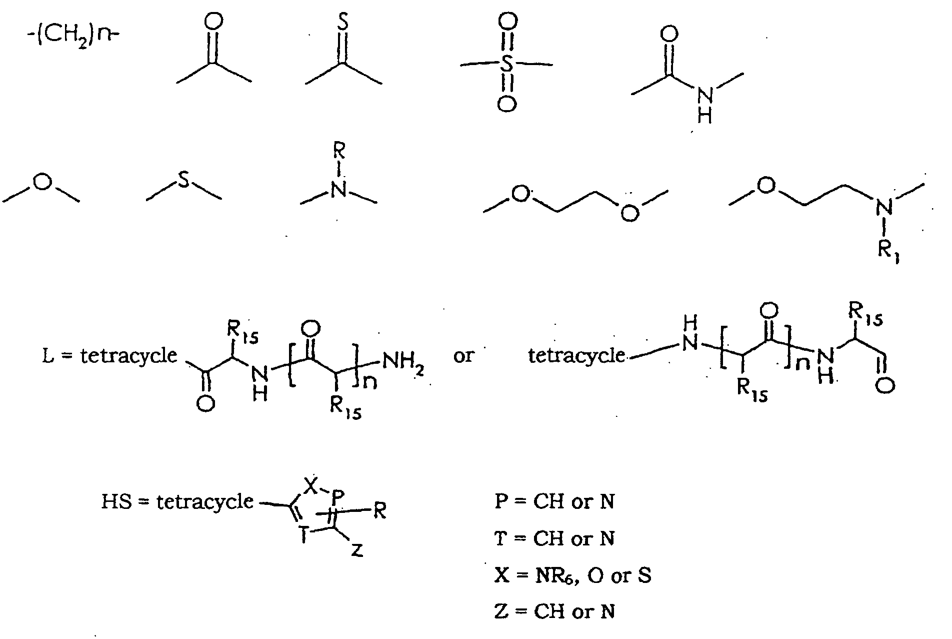 wherein R₁₅ means the side chain of D-, L-, D,L-amino acids or unnatural amino acids, and in the event of n > 1, R₁₅ in the individual radicals means respectively an identical or different side chain of D-, L-, D,L-amino acids or unnatural amino acids, with the proviso that the atom N next to Q is connected respectively to the group G2 and G3 of formula I;
Hdi) an, if necessary at least once substituted, tricyclic substituent (Tr) with at least one heterocyclic ring as ring component and a bonding position on one carbon atom of an anellated benzene ring of the same, which is connected via a spacer Q and the nitrogen atom adjacent to Q to G₂ and G₃ respectively of the compound of formula I, Q having the meaning indicated above under Hc);
Hdii) is a cyclic hydrocarbon of the subsequent formulae: 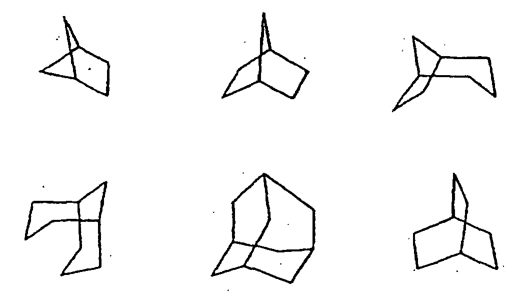 or
He) -NH-, -O-, -S-, -SO- or -SO₂-;
Hf) in the event of W being -NH-, z in the group G₃ being 0, 2 or 3, with the proviso that z in the group G₃ is 1, if the substituent R₁ means a low alkyl group, such as methyl or ethyl.

2. New compounds of the general formula II 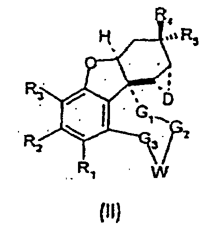 wherein D means N-H, N-alkyl, N-acyl, oxygen or sulphur and wherein the substituents R₁ to R₅, G₁ to G₃ and also W have the meanings indicated in patent claim 1 in the case of the general formula I.

3. New compound of the general formula III 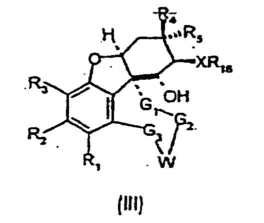 wherein X-R₁₆ is a substituent, in which X is oxygen or sulphur and R₁₆ is hydrogen or a low (C₁-C₁₀), if necessary branched, if necessary substituted alkyl or aralkyl group, and wherein the substituents R₁ to R₅, G₁ to G₃ and also W have the meanings indicated in patent claim 1 in the case of the general formula I.

4. New compound of the general formula IV 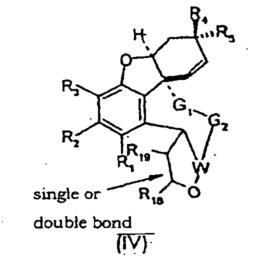 wherein R₁₈ and R₁₉ mean hydrogen, alkyl, aryl or aralkyl and in which the C atoms bearing the substituents R₁₈ and R₁₉ are cross-linked to each other via a single or a double bond, and wherein the substituents R₁ to R₅ and G₁ to G₃ have the meanings indicated in patent claim 1 in the case of the general formula 1, W meaning -CH- or -NH-.

5. Compound according to claim 1, wherein the 6-π heterocycle is imidazolyl, oxazolyl, isoxazolyl, triazolyl, tetrazolyl, oxadiazolyl, thiadiazolyl, pyridazinyl, pyrimidinyl, pyrazinyl and substituted variants of the same, imidazolinyl, thiazolinyl or oxazolinyl.

6. Compound according to one of the claims 1 to 5, wherein R₅ has a meaning other than hydrogen and R₄ is OH.

7. Compound according to claim 1, wherein Y₁ is NH and Y₂ is N-R₉ and wherein R₄ and R₅ are connected by means of -(CH₂)ₙ- (n = 2, 3 or 4).

8. Compound according to one of the claims 1 to 7, wherein the tricylic substituent Tr is a condensed benzene ring of the general formula 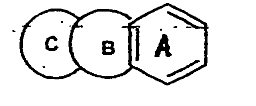 or 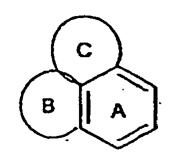 or 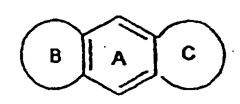 or

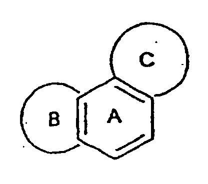

9. Compound according to claim 8, wherein the ring A is a substituted benzene ring.

10. Compound according to claim 8 or 9, wherein one of the rings B and C is an if necessary substituted heterocyclic ring and the other is a substituted ring, which can contain one or more heteratoms in the ring.

11. Compound according to one of the claims 8 to 10, wherein the benzene ring is substituted at least once, these substituents being halogens, such as fluorine and chlorine, halogeno-C₁-C₃-alkyl groups, such as trifluoromethyl, C₁-C₃-alkyl groups, such as methyl, C₁-C₃-alkoxy groups, such as methoxy, and the hydroxy group, in particular a halogen, such as fluorine.

12. Compound according to one of the claims 8 to 11, wherein the if necessary substituted heterocylic ring B or C is a 4 to 14 membered ring, preferably a 5 to 7 membered ring, in particular a 5 to 7 membered non-aromatic ring, which contains one or two identical or different heteroatoms.

13. Compound according to claim 12, wherein at least one heteroatom of the heterocyclic ring (1 to 3 heteroatoms are possible) is nitrogen, oxygen or sulphur.

14. Compound according to claim 13, wherein the heterocyclic ring B or C is pyridine, pyrazine, pyrimidine, imidazole, furan, thiophene, pyrrolidine, piperidine, hexamethyleneimine, tetrahydrofuran, piperazine, morpholine or thiomorpholine.

15. Compound according to one of the claims 8 to 14, wherein the 5 to 8 membered ring B or C is a 5 to 8 membered heterocyclic or alicyclic ring, or a carbon ring, which is substituted at least once.

16. Compound according to claim 15, wherein the 5 to 8 membered carbon ring is a benzene ring or a saturated or unsaturated ring, for example benzene, cyclopentane, cyclopentene, cyclohexane, cyclohexene, cyclohexadiene, cycloheptane, cycloheptene and cycloheptadiene.

17. Compound according to one of the claims 1 to 16, wherein the tricyclic substituent Tr is a group of one of the subsequently reproduced formulae

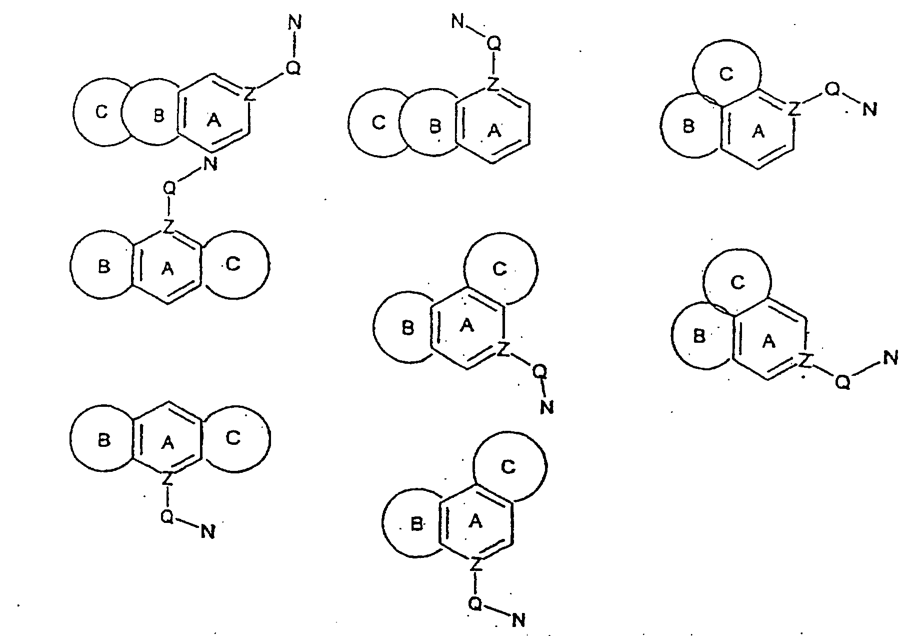

18. Compound according to one of the claims 1 to 17, wherein the tricylic substituent Tr is a group of one of the subsequently reproduced formulae

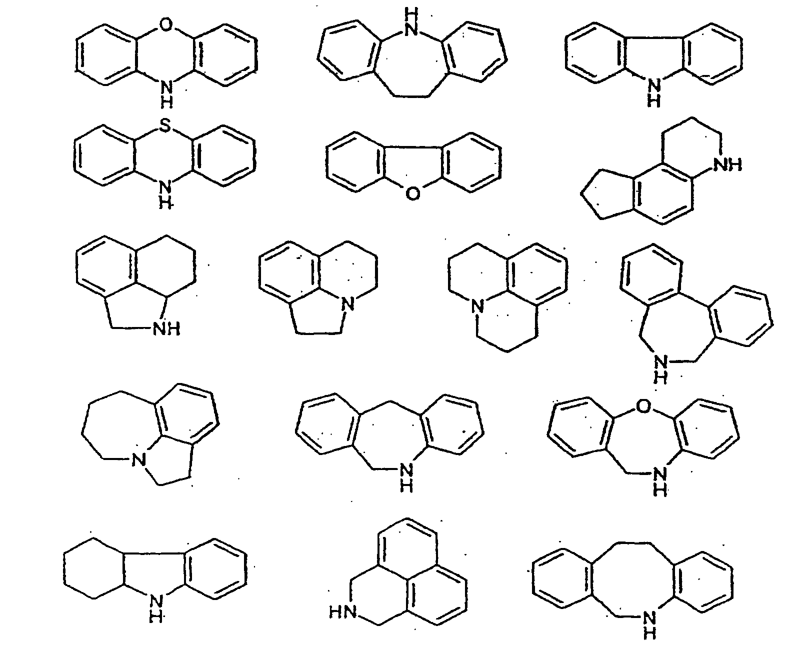

19. Compound according to one of the claims 1 to 18, wherein the substituent Tr is substituted at least once by R₁ and R₁ has the meanings indicated in claim 1 under A).

20. -(CH₂)_{y}-, wherein y is equal to 0 to 2, with the proviso that x + y together mean at least 2 and at most 4.

21. Method for producing compounds of the claims 1 to 20, **characterised in that** combinatory or parallel synthesis technology is used, the basic molecule being immobilised by means of a functional group (linker) on a solid phase, the synthesis of the target compound being implemented and then this target compound being separated from the solid phase.

22. Method according to claim 21, **characterised in that** the basic molecule is immobilised on the solid phase via a carbon centre, a nitrogen centre or an oxygen centre.

23. Method according to claim 21 or 22, **characterised in that**, as functional group, (linker) = -X(CH₂)ₙCO (X = CH₂, CO, O, S, NH), -X(CH₂)ₙOCO (X = CH₂, CO, O, S, NH), -XC₆H₄CH₂- (X = CH₂, CO, O, S, NH), THP, -X(CH₂)ₙSi(alkyl)₂- is used.

24. Method according to claim 21 or 22, **characterised in that**, as functional group, (linker) = -X(CH₂)ₙCO (X = CH₂, O, NH, SOₒ₋₂), -X(CH₂)ₙCS (X = CH₂, O, NH SOₒ₋₂), X(CH₂)ₙJCO (X = CH₂, O, NH, SOₒ₋₂; J = NH, O, S), XC₆H₄CH₂ (X = CH₂, O, S,) is used.

25. Method according to claim 21 or 22, **characterised in that**, as functional group, (linker) = -(CH₂)ₙSi(alkyl)₂-, -C₆H₄Si(alkyl)₂-, -(CH₂)ₙSn(alkyl)₂-, -C₆H₄Sn(alkyl)₂, -(CH₂)ₙS, -C₆H₄S is used.

26. Medicament containing at least one of the compounds of the general formulae I, II, III or N, or a pharmaceutically acceptable salt of the same as active substance.

27. Medicament according to claim 26 for treatment
a) treatment of Alzheimer's disease,
b) treatment of Parkinson's disease,
c) treatment of Huntington's disease (chorea),
d) treatment of multiple sclerosis,
e) treatment of amyotrophic lateral sclerosis,
f) treatment of epilepsy,
g) treatment of the consequences of stroke,
h) treatment of the consequences of head and brain trauma,
i) treatment and prophylaxis of the consequences of diffuse lack of oxygen and nutrients in the brain, such as after hypoxia, anoxia, asphyxia, heart stoppage, poisonings, and when complications are observed in babies during difficult births or with anaesthetics,
j) in particular also prophylactic treatment of apoptotic degeneration in neurons, which have been or are damaged by local radio- or chemotherapy of brain tumours, and
k) treatment of bacterial meningitis and
l) treatment of diseases with an apoptotic component, in particular following amyloid-associated cell degeneration,
m) treatment of diabetes mellitus, in particular when the disease is accompanied by amyloid degeneration of the nesidioblasts,
n) for increasing muscle strength and
o) for increasing the stamina of Alzheimer's patients.

28. Use of at least one of the compounds of the general formulae I, II, III, IV, or a pharmaceutically acceptable salt of the same for producing medicaments.

29. Use according to claim 28 for producing medicaments for treatment
a) treatment of Alzheimer's disease,
b) treatment of Parkinson's disease,
c) treatment of Huntington's disease (chorea),
d) treatment of multiple sclerosis,
e) treatment of amyotrophic lateral sclerosis,
f) treatment of epilepsy,
g) treatment of the consequences of stroke,
h) treatment of the consequences of head and brain trauma,
i) treatment and prophylaxis of the consequences of diffuse lack of oxygen and nutrients in the brain, such as after hypoxia, anoxia, asphyxia, heart stoppage, poisonings, and also when complications are observed in babies during difficult births or with anaesthetics,
j) in particular also prophylactic treatment of apoptotic degeneration in neurons, which have been or are damaged by local radio- or chemotherapy of brain tumours, and
k) treatment of bacterial meningitis and
l) treatment of diseases with an apoptotic component, in particular following amyloid-associated cell degeneration,
m) treatment of diabetes mellitus, in particular when the disease is accompanied by amyloid degeneration of the nesidioblasts,
n) for increasing muscle strength and
o) for increasing the stamina of Alzheimer's patients.

30. Method for producing medicaments, in which at least one of the compounds of the general formulae I, II, III or IV is mixed with a pharmaceutically acceptable carrier and/or formulation adjuvant.

31. Method for separating the (+) and (-) isomers of racemic compounds of the general formulae I, II, III or IV, in particular of racemic (6R)-3-methoxy-5,6,9,10,11,12-hexahydro-4aH[1]benzofuro[3a,3,2-ef][2-benzazepin-6-ol (norgalanthamine) from an isomer mixture, **characterised in that** the method of fractionated crystallisation with a chiral acid is used.

32. Method according to claim 31, **characterised in that** the isomer mixture is dissolved or suspended in a solvent, the thus obtained solution or suspension is mixed with a solution of the chiral acid and then is injected with seed crystals of a salt made of a (-) isomer or (+) isomer of the desired compound and of a (+) or (-) chiral acid, is left to crystallise, the formed crystals are separated and dried and finally the (+) isomer or the (-) isomer of the desired compound of the general formulae I, II, III or IV is isolated by extraction of the crystals with an organic solvent, after said crystals have been mixed with a base, such as NH₄OH.

33. Method according to claim 31 or 32, **characterised in that**, as chiral acid, an acid selected from the group comprising (+) or (-) tartaric acid, substituted tartaric acid or (+) or (-) 0,0-di-p-toluictartaric acid is used.

34. Method according to one of the claims 31 to 33, **characterised in that** the isomer mixture is dissolved or suspended in the 3 to 50 times quantity of a solvent selected from the group comprising water, methanol, ethanol, propanol, isopropanol and acetone or a mixture of at least two of the mentioned solvents.

35. Method according to one of the claims 32 to 34, **characterised in that**, for extraction, a solvent selected from the group comprising chloroform, methylene chloride, ethyl acetate, butyl acetate, diethyl ether, t-butyl methyl ether, dibutyl ether, petroleum ether, xylene, benzene and toluene is used.

36. Method according to one of the claims 32 to 35, **characterised in that** the desired isomer is isolated by distilling off the solvent.

## Revendications

1. Composés de formule générale I : 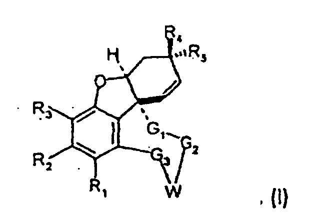 dans laquelle les substituants ont les significations indiquées ci-après :
A) R₁ et R₂ sont identiques ou différents et représentent :
Aa) un atome d'hydrogène, F, CI, Br, I, CN, NC, OH, SH, NO₂, SO₃H, PO₃H, NH₂, CF₃, OSO₂(CH₂)ₙCF₃ où n est égal à 0, 1 ou 2, -OSO₂-aryl, -vinyl ou -éthynyl,
Ab) un groupe alkyle bas (C₁-C₆), éventuellement ramifié, aralkyle, alcoxy, aralcoxy, cycloalkyle ou cycloalcoxy,
Ac) un groupe amino qui est éventuellement substitué par un ou deux groupes identiques ou différents, pris parmi les groupes alkyle bas (C₁-C₆), éventuellement ramifiés, aralkyle, alkylcarbonyle, aralkylcarbonyle, alcoxycarbonyle et aralcoxycarbonyle, ou par un groupe choisi parmi les restes pyrrolidine, pipéridine, morpholine, thiomorpholine, pipérazine et homopipérazine,
Ad) un groupe -COOH, -COO alkyle, -COO aralkyle ou -CO-amino, éventuellement substitué comme cela est indiqué précédemment à Ac),
ou un groupe COH-alkyle ou COH-aralkyle,
Ae) un groupe -(CH₂)ₙX (où X représente Br, Cl, F ou I), -(CH₂)ₙOH, -(CH₂)ₙCHO, -(CH₂)ₙCOOH, -(CH₂)ₙCN, -(CH₂)ₙNC, -(CH₂)ₙCO alkyle ou -(CH₂)ₙCO aryle, où n est égal à 1 à 4,
Af) un groupe -(CH₂)ₙ vinyle, -(CH₂)ₙ éthynyle ou -(CH₂)ₙ cycloalkyle, où n est égal à 0, 1 ou 2 et cycloalkyle représente un noyau aliphatique ayant 3 à 7 atomes de carbone,
Ag) un groupe alcényle en C₃ à C₆ substitué (éventuellement substitué par H, F, Br, CI, CN, CO₂ alkyle, CO alkyle, CO aryle),
Ah) un groupe alcynyle en C₃ à C₆ substitué (éventuellement substitué par H, F, Br, Cl, CN, CO₂ alkyle, CO alkyle, CO aryle), ou bien
Ai) R₁ et R₂ représentent ensemble -CH=CH-CH=CH-, -O(CH₂)ₙO- (n est égal à 1 à 3), -CH=CHA₁- (A₁ représente NH, O ou S), ou -CH₂CH₂A₁-(A₁ représente NH, O ou S),
B) R₃ a la même signification que R₁ et représente en particulier OH ou OCH₃, ou bien
C) R₂ et R₃ représentent ensemble -A₂(CH₂)ₙA₂- où n est égal à 1 à 3 et les A₂ sont identiques ou différents et représentent NH, O ou S,
D) R₄ et R₅
Da) sont tous les deux des atomes d'hydrogène, ou bien
Db) l'un de R₄ et R₅ est un atome d'hydrogène ou un groupe alkyle, aralkyle, alcényle, aralcényle, alcynyle ou aralcynyle, et l'autre de R₄ et R₅ est
Dbi) OR₆ où R₆ représente un atome d'hydrogène, un groupe alkyle bas (C₁-C₁₀), éventuellement ramifié, un groupe cycloalkyle, un groupe silyle substitué en C₃ à C₁₀ (par exemple un groupe triéthylsilyle, triméthylsilyle, t-butyldiméthylsilyle ou diméthylphénylsilyle), ou un groupe α-alcoxyalkyle en C₂ à C₁₀, par exemple un groupe tétrahydropyranyle, tétrahydrofuranyle, méthoxyméthyle, éthoxyméthyle, 2-méthoxypropyle, éthoxyéthyle, phénoxyméthyle ou 1-phénoxyéthyle,
Dbii) O-CS-NHR₆(thiouréthane), où R₆ a les significations indiquées précédemment à Dbi),
Dbiii) O-CO-NHR'₇ avec la signification suivante : 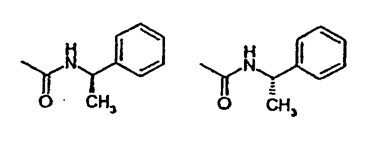
Dbiv) O-CO-HR₆, où R₆ a les significations indiquées précédemment à Dbi), en particulier des esters avec les modèles de substitution d'acides aminés (les deux énantiomères), comme 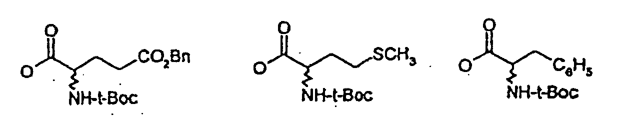
Dbv) NR₇R₇ où les deux substituants R₇ sont identiques ou différents et représentent un atome d'hydrogène, un groupe alkyle bas (C₁-C₄), éventuellement ramifié, ou un groupe cycloalkyle, ou bien les substituants R₇ représentent ensemble -(CH₂)ₙ- où n est égal à 3 à 5,
Dbvi) NH-COR₆(amido), où R₆ a les significations indiquées précédemment à Dbi),
Dbvii) S-R₆ où R₆ a les significations indiquées précédemment à Dbi),
Dbviii) SOₙR₈, où n est égal à 0, 1 ou 2, et R₈ représente un groupe alkyle ou aralkyle, en C₁ à C₁₀, éventuellement ramifié ou cyclique, éventuellement substitué,
Dbix) R₄ représente un atome d'hydrogène et R₅ représente OH, CN, CO₂-alkyle, CONRₐR_{b} où Rₐ représente un atome d'hydrogène ou un groupe alkyle bas (C₁-C₆), éventuellement ramifié ou cyclique, éventuellement substitué, et R_{b} représente un atome d'hydrogène ou un groupe alkyle bas (C₁-C₆), éventuellement ramifié ou substitué, ou bien Rₐ et R_{b} représentent ensemble -(CH₂)ₙ- où n représente 2 à 6, ou -(CH₂)ₙE(CH₂)ₙ- où E représente NH, N-alkyle, O ou S et n est égal à 0 à 5, un groupe aryle (phényle ou naphtyle) ou un hétérocycle 6-π,
Dbx)R₄ et R₅ forment ensemble un groupe carbonyle (=O), hydrazone (=N-NH-R₉, =N-NR₉R₁₀) ou oxime (=N-OR₁₀), où R₉ représente un atome d'hydrogène, un groupe alkyle bas (C₁-C₆), éventuellement ramifié ou cyclique, éventuellement substitué, aralkyle, alkylcarbonyle, aralkylcarbonyle, alkylcarbonyloxy ou aralkylcarbonyloxy, ou un groupe acide sulfonique comme un groupe tosyle ou mésyle, et R₁₀ représente un atome d'hydrogène, un groupe alkyle bas (C₁-C₆), éventuellement ramifié ou cyclique, éventuellement substitué, aralkyle, alkylcarbonyle ou aralkylcarbonyle, ou un groupe acide sulfonique comme un groupe tosyle ou mésyle,
Dbxi) R₄ et R₅ forment ensemble des substituants du type 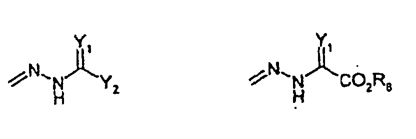
où Y₁ et Y₂ sont identiques ou différents et représentent O, S, NH ou N-R₉ (les valences libres sont dans chaque cas de l'hydrogène), où R₉ a les significations indiquées précédemment à Dbx),
E) G₁ représente -(CH₂)x- où x est égal à 1 ou 2,
F) G₂ représente -(CH₂)y- où y est égal à 0 à 2,
E/Fa : G₁ et G₂ représentent ensemble ou séparément -C(R₁₁R₁₂)- où R₁₁ et R₁₂ représentent un atome d'hydrogène, OH, un groupe alkyle bas, éventuellement ramifié ou cyclique, éventuellement substitué, aralkyle, aryle, alcoxy, aralkyloxy ou aryloxy, ou forment ensemble un groupe spiro-alkyle (cycle spiro en C₃ à C₇),
E/Fb : G₁ et G₂ représentent ensemble 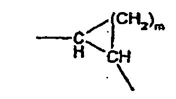 où m est égal à 1 à 7,
E/Fc : G₁ et G₂ représentent ensemble ou séparément -CR₁₁R₁₂- où R₁₁ et R₁₂ représentent un atome d'hydrogène, un groupe hydroxyle, ou un groupe alkyle bas, éventuellement ramifié ou cyclique, éventuellement substitué, aralkyle, aryle, alcoxy, aralcoxy ou aryloxy,
E/Fd : G₁ et G₂ forment ensemble un groupe spiro-alkyle (cycle spiro en C₃ à C₇),
G) G₃ représente -(CH₂)z- où z est égal à 0 à 3, étant entendu que la somme x+y+z est égale au moins à 2 et au plus à 4, ou bien G₃ représente un groupe carbonyle ou thiocarbonyle, -CH(OH)- ou -C(OH)=,
H) W représente :
Ha) CR₁₃R₁₄ où R₁₃ représente un atome d'hydrogène et R₁₄ représente -(CH₂)ₙNR₇R₇, -CO-NR₇R₇ ou -COOR₇, où n est égal à 0 à 2 et R₇ a les significations indiquées précédemment, ou bien R₇ et R₇ forment un cycle par l'intermédiaire d'un groupe -(CH₂)ₙ- où n et égal à 3 à 5, les substituants R₁₃ et R₁₄ pouvant être échangés,
Hb) un groupe N-phényl (éventuellement substitué par F, Br, CI, alkyle en C₁ à C₄, CO₂ alkyle, CN, CONH₂ ou alcoxy), N-thiényl-2 ou -3, N-furyl-2 ou -3, ou N-1,3,5-triazinyl, où le reste triazinyle peut être substitué en outre par CI, OR₆ ou NR₇R₇, R₆ et R₇ ayant les significations indiquées précédemment,
Hc) un des substituants représentés ci-après 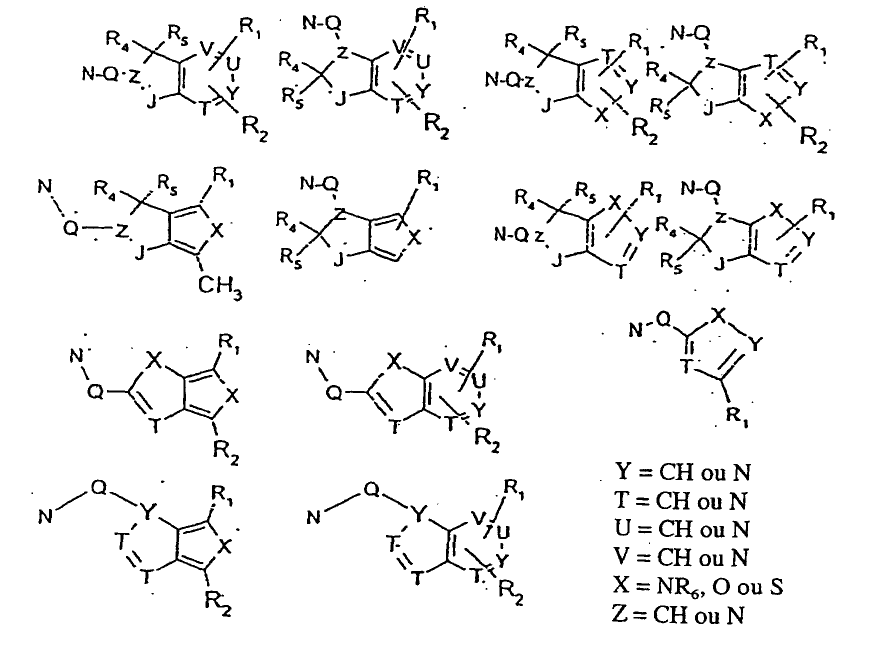 où J représente aucune liaison ou un groupe -(CH₂)ₙ-, où n est égal à 0 à 3, carbonyle, thiocarbonyle, O, S, SO ou SO₂, R₆ a les significations indiquées précédemment, et Q représente -(CH₂)ₙ-M*-(CH₂)ₘ- où n est égal à 0 à 4, m et égal à 0 à 4, et M* représente un groupe alcényle, alcynyle, phényle disubstitué, thiophène disubstitué, furane disubstitué, pyrazine disubstituée, pyridazine disubstituée, un chaînon espaceur ayant l'un des formules indiquées ci-après, un espaceur peptidique L ou un espaceur hétérocyclique HS ayant l'une des formules indiquées ci-après 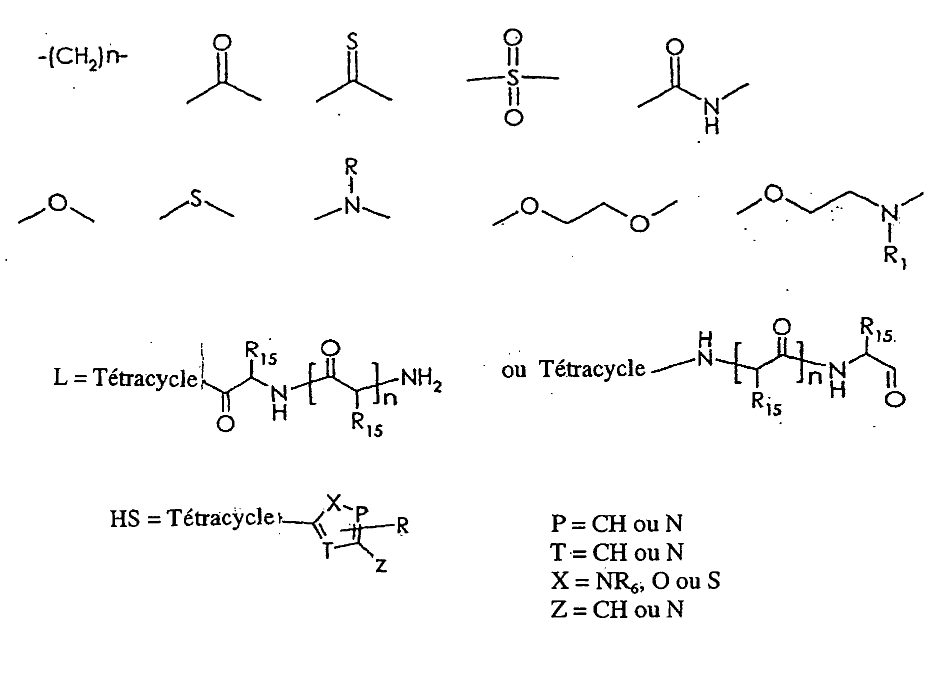 où R₁₅ représente la chaîne latérale d'un acide aminé D, L ou D,L ou d'un acide aminé non-naturel, et, dans le cas où n est supérieur à 1, les divers R₁₅ des restes individuels représentent des chaînes latérales identiques ou différentes d'acides aminés D, L ou D,L ou d'acides aminés non-naturels, étant entendu que l'atome d'azote proche de Q est à chaque fois lié aux groupes G₂ et G₃ de la formule I,
Hdi) un substituant tricyclique (Tr), éventuellement substitué au moins une fois, ayant au moins un noyau hétérocyclique en tant que constituant du cycle et présentant une position de liaison sur un atome de carbone d'un cycle benzénique condensé avec ledit noyau, qui est lié par l'intermédiaire d'un espaceur Q et de l'atome d'azote proche de Q, aux groupes G₂ et G₃ du composé de formule I, Q ayant les significations indiquées précédemment à Hc),
Hdii) un hydrocarbure cyclique ayant l'une des formules suivantes : 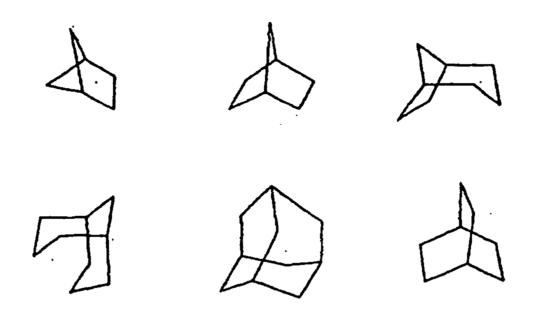
ou bien
He) -NH-, -O-, -S-, -SO- ou -SO₂-,
Hf) z représentant 0, 2 ou 3 dans le groupe G₃ dans le cas où W est - NH-, étant entendu que z est égal à 1 dans le groupe G₃ quand le substituant R₁ représente un groupe alkyle bas tel qu'un groupe méthyle ou éthyle.

2. Composés de formule générale II : 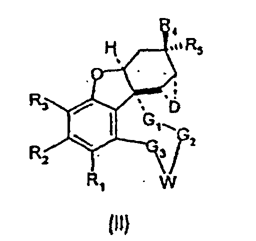 dans laquelle D représente N-H, N-alkyl, N-acyl, un atome d'oxygène ou un atome de soufre, et les substituants R₁ à R₅, G₁ à G₃ et W ont les significations indiquées dans la revendication 1 pour la formule générale I.

3. Composés de formule générale III : 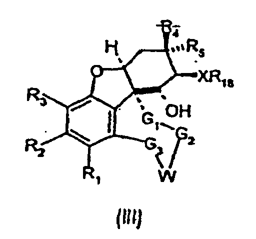 dans laquelle X-R₁₆ représente un substituant où X désigne un atome d'oxygène ou de soufre et R₁₆ désigne un atome d'hydrogène ou un groupe alkyle inférieur ou aralkyle en C₁ à C₁₀, éventuellement ramifié, éventuellement substitué, et les substituants R₁ à R₅, G₁ à G₃ et W ont les significations indiquées dans la revendication 1 pour la formule générale I.

4. Composés de formule générale IV 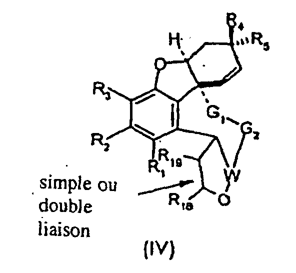 dans laquelle R₁₈ et R₁₉ représentent un atome d'hydrogène, ou un groupe alkyle, aryle ou aralkyle, et dans laquelle les atomes de carbone portant les substituants R₁₈ et R₁₉ sont reliés par une liaison simple ou une double liaison, et dans laquelle les substituants R₁ à R₅ et G₁ à G₃ ont les significations indiquées dans la revendication 1, pour la formule générale I, et W représente -CH- ou -NH-.

5. Composé selon la revendication 1, pour lequel l'hétérocycle 6-π est un groupe imidazolyle, oxazolyle, isoxazolyle, triazolyle, tétrazolyle, oxadiazolyle, thiadiazolyle, pyridazinyle, pyrimidinyle, pyrazinyle et ses variantes substituées, imidazolinyle, thiazolinyle ou oxazolinyle.

6. Composé selon l'une quelconque des revendications 1 à 5, pour lequel R₅ a une signification autre que l'atome d'hydrogène et R₄ représente OH.

7. Composé selon la revendication 1, pour lequel Y₁ représente NH, Y₂ représente N-R₉, et R₄ et R₅ sont reliés par -(CH₂)ₙ- où n est égal à 2, 3 ou 4.

8. Composé selon l'une quelconque des revendications 1 à 7, pour lequel le substituant tricyclique Tr est un noyau benzénique condensé de formule générale 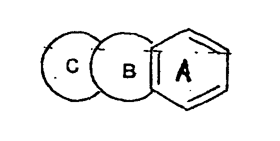 ou 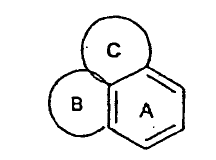 ou 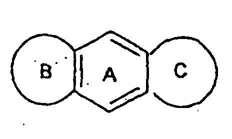 ou

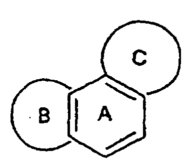

9. Composé selon la revendication 8, pour lequel le noyau A est un noyau benzénique substitué.

10. Composé selon la revendication 8 ou 9, pour lequel l'un des noyaux B et C est un noyau hétérocyclique éventuellement substitué et l'autre est un noyau substitué qui peut contenir dans le cycle un ou plusieurs hétéroatomes.

11. Composé selon l'une quelconque des revendications 8 à 10, pour lequel le noyau benzénique est substitué au moins une fois, le ou les substituants étant pris parmi les atomes d'halogène, comme les atomes de fluor et de chlore, les groupes halogénoalkyle en C₁ à C₃ comme le groupe trifluorométhyle, les groupes alkyle en C₁ à C₃ comme le groupe méthyle, les groupes alcoxy en C₁ à C₃ comme le groupe méthoxy, et le groupe hydroxyle, et étant en particulier un atome d'halogène comme l'atome de fluor.

12. Composé selon l'une quelconque des revendications 8 à 11, pour lequel le noyau hétérocyclique B ou C éventuellement substitué est un noyau à 4 à 14 chaînons, de préférence un noyau à 5 à 7 chaînons, en particulier un noyau non-aromatique à 5 à 7 chaînons, qui contient un ou deux hétéroatomes identiques ou différents.

13. Composé selon la revendication 12, pour lequel au moins un hétéroatome du noyau hétérocyclique (1 à 3 hétéroatomes sont possibles) est un atome d'azote, d'oxygène ou de soufre.

14. Composé selon la revendication 13, pour lequel le noyau hétérocyclique B ou C est la pyridine, la pyrazine, la pyrimidine, l'imidazole, le furane, le thiophène, la pyrrolidine, la pipéridine, l'hexaméthylène-imine, le tétrahydrofurane, la pipérazine, la morpholine ou la thiomorpholine.

15. Composé selon l'une quelconque de revendications 8 à 14, pour lequel le noyau B ou C à 5 à 8 chaînons est un noyau hétérocyclique ou alicyclique à 5 à 8 chaînons, ou un noyau carboné, qui est substitué au moins une fois.

16. Composé selon la revendication 15, pour lequel le noyau carboné à 5 à 8 chaînons est un noyau benzénique ou un noyau saturé ou insaturé, par exemple le benzène, le cyclopentane, le cyclopentène, le cyclohexane, le cyclohexène, le cyclohexadiène, le cycloheptane, le cycloheptène et le cycloheptadiène.

17. Composé selon l'une quelconque des revendications 1 à 16, pour lequel le substituant tricyclique Tr est un groupe ayant l'une des formules indiquées ci-après :

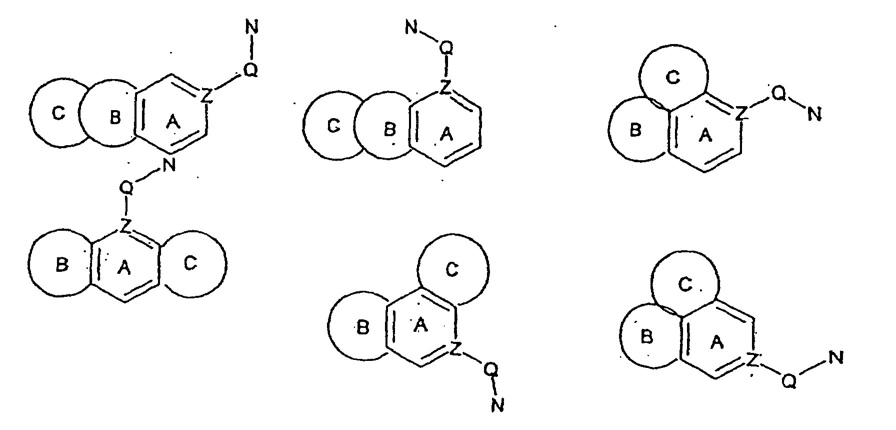

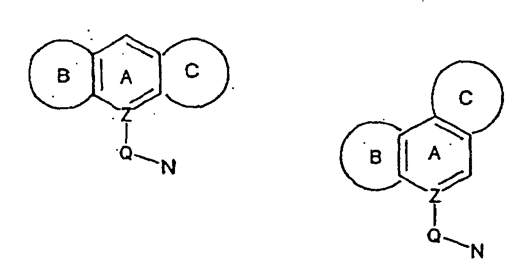

18. Composé selon l'une quelconque des revendications 1 à 17, pour lequel le substituant tricyclique Tr est un groupe ayant l'une des formules indiquées ci-après :

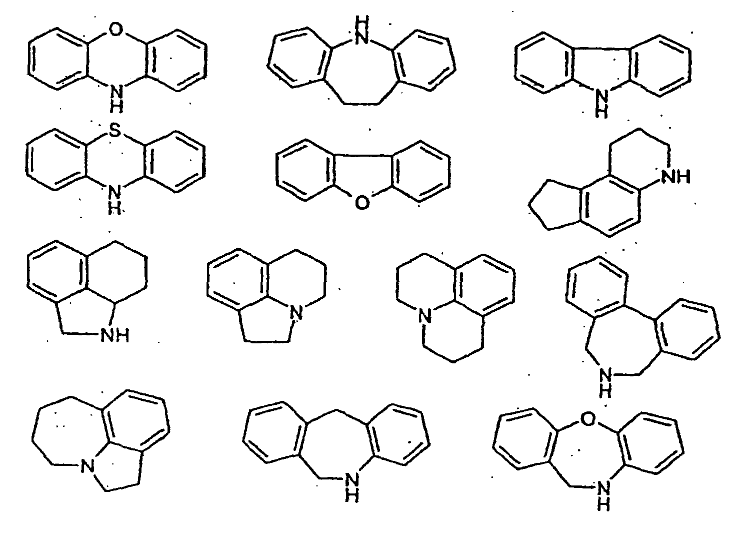

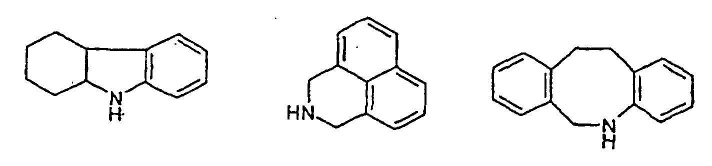

19. Composé selon l'une quelconque des revendications 1 à 18, pour lequel le substituant Tr est substitué au moins une fois par R₁ et R₁ a les significations indiquées dans la revendication 1, dans la partie A).

20. Composé selon l'une quelconque des revendications 1 à 19, pour lequel le substituant W est -NH- et/ou le substituant G₁ est - (CH₂)ₓ- où x est égal à 1 ou 2, et G₂ est -(CH₂)_{y}- où y est égal à 0 à 2, étant entendu que la somme x + y est égale au moins à 2 et au plus à 4.

21. Procédé de préparation des composés des revendications 1 à 20, **caractérisé en ce que** l'on utilise la technologie combinatoire ou de synthèse en parallèle, en immobilisant la molécule de base sur une phase solide au moyen d'un groupe fonctionnel (chaînon de liaison), en réalisant la synthèse du composé visé et en séparant ensuite ce composé visé de la phase solide.

22. Procédé selon la revendication 21, **caractérisé en ce que** la molécule de base est immobilisée sur la phase solide par l'intermédiaire d'un centre carboné, d'un centre azoté ou d'un centre oxygéné.

23. Procédé selon la revendication 21 ou 22, **caractérisé en ce qu'**on utilise comme groupe fonctionnel (chaînon de liaison), - X(CH₂)ₙCO(X=CH₂, CO, O, S, NH), -X(CH₂)ₙOCO(X=CH₂, CO, O, S, NH), -XC₆H₄CH₂-(X=CH₂, CO, O, S, NH), THP, - X(CH₂)ₙSi(alkyl)₂-.

24. Procédé selon la revendication 21 ou 22, **caractérisé en ce qu'**on utilise comme groupe fonctionnel (chaînon de liaison), - X(CH₂)ₙCO(X=CH₂, O, NH, SO_{O-2}), -X(CH₂)ₙCS(X=CH₂, O, NH, SO_{O-2}), X(CH₂)ₙJCO(X=CH₂, O, NH, SO_{O-2} ; J= NH, O, S), XC₆H₄CH₂(X=CH₂, O, S).

25. Procédé selon la revendication 21 ou 22, **caractérisé en ce qu'**on utilise comme groupé fonctionnel (chaînon de liaison), - (CH₂)ₙSi(alkyl)₂-, -C₆H₄Si(alkyl)₂-, -(CH₂)ₙSn(alkyl)₂-, -C₆H₄Sn(alkyl)₂, -(CH₂)ₙS, -C₆H₄S.

26. Médicament contenant au moins l'un des composés de formule générale I, II, III ou IV, ou un sel pharmaceutiquement acceptable d'un tel composé, en tant qu'agent actif.

27. Médicament selon la revendication 26, pour :
a)le traitement de la maladie d'Alzheimer,
b)le traitement de la maladie de Parkinson,
c)le traitement de la maladie de Huntington (chorée),
d)le traitement de la sclérose multiple,
e)le traitement de la sclérose latérale amyotrophique,
f)le traitement de l'épilepsie,
g)le traitement des suites d'une attaque d'apoplexie,
h)le traitement des suites d'un traumatisme du crâne et du cerveau,
i)le traitement et la prophylaxie des suites d'un manque d'oxygène diffus et d'éléments nutritifs dans le cerveau, comme on l'observe après une hypoxie, une anoxie, une asphyxie, un arrêt du coeur, une intoxication, ainsi que lors de complications dans le cas des naissances difficiles des nourrissons, ou lors de narcoses,
j)en particulier également le traitement prophylactique de la dégénérescence apoptotique des neurones qui ont été ou sont lésés par une radiothérapie ou une chimiothérapie locale de tumeurs du cerveau,
k)le traitement de la méningite bactérienne,
l)le traitement de maladies à composante apoptotique, en particulier à la suite d'une dégénérescence cellulaire associée amyloïde,
m)le traitement du diabète mellitus, en particulier lorsque la maladie s'accompagne d'une dégénérescence amyloïde des cellules insulaires,
n)l'augmentation de la force musculaire, et
o)l'augmentation de l'endurance des patients atteints de la maladie d'Alzheimer.

28. Utilisation d'au moins l'un des composés de formule générale I, II, III ou IV, ou d'un sel pharmaceutiquement acceptable d'un tel composé, pour la préparation de médicaments.

29. Utilisation selon la revendication 28, pour la préparation de médicaments pour :
a) le traitement de la maladie d'Alzheimer,
b) le traitement de la maladie de Parkinson,
c) le traitement de la maladie de Huntington (chorée),
d) le traitement de la sclérose multiple,
e)le traitement de la sclérose latérale amyotrophique,
f)le traitement de l'épilepsie,
g)le traitement des suites d'une attaque d'apoplexie,
h)le traitement des suites d'un traumatisme du crâne et du cerveau,
i)le traitement et la prophylaxie des suites d'un manque d'oxygène diffus et d'éléments nutritifs dans le cerveau, comme on l'observe après une hypoxie, une anoxie, une asphyxie, un arrêt du coeur, une intoxication, ainsi que lors de complications dans le cas des naissances difficiles des nourrissons, ou lors de narcoses,
j)en particulier également le traitement prophylactique de la dégénérescence apoptotique des neurones qui ont été ou sont lésés par une radiothérapie ou une chimiothérapie locale de tumeurs du cerveau, k)le traitement de la méningite bactérienne,
l)le traitement de maladies à composante apoptotique, en particulier à la suite d'une dégénérescence cellulaire associée amyloïde,
m)le traitement du diabète mellitus, en particulier lorsque la maladie s'accompagne d'une dégénérescence amyloïde des cellules insulaires,
n)l'augmentation de la force musculaire, et
o)l'augmentation de l'endurance des patients atteints de la maladie d'Alzheimer.

30. Procédé de préparation de médicaments, dans lequel on mélange au moins l'un des composés de formule générale I, II, III ou IV avec un support et/ou adjuvant de formulation pharmaceutiquement acceptable.

31. Procédé pour séparer les isomères (+) et (-) de composés racémiques de formule générale I, II, III ou IV, en particulier du (6R)-3-méthoxy-5,6,9,10,11,12-hexahydro-4aH[1]benzofuro[3a,3,2-ef] [2-benzyzépin-6-ol]racémique(norgalanthamine), à partir d'un mélange d'isomères, **caractérisé en ce qu'**on utilise le procédé de cristallisation fractionnée à l'aide d'un acide chiral.

32. Procédé selon la revendication 31, **caractérisé en ce que** l'on dissous ou met en suspension le mélange d'isomères dans un solvant, on ajoute à la solution ou suspension ainsi obtenue une solution de l'acide chiral et on l'ensemence ensuite avec des germes cristallins d'un sel de l'isomère (-) ou de l'isomère (+) du composé désiré et d'un acide chiral (+) ou (-), on laisse cristalliser, on sépare les cristaux formés et on les sèche, et on isole finalement l'isomère (+) ou l'isomère (-) du composé désiré de formule générale I, II, III ou IV par extraction des cristaux avec un solvant organique, après avoir ajouté à ceux-ci une base telle que NH₄OH.

33. Procédé selon la revendication 31 ou 32, **caractérisé en ce que** l'on emploie comme acide chiral un acide choisi parmi l'acide tartrique (+) ou (-), l'acide tartrique substitué et l'acide O, O-di-p-toluyltartrique (+) ou (-).

34. Procédé selon l'une quelconque des revendications 31 à 33, **caractérisé en ce que** l'on dissout ou met en suspension le mélange d'isomères dans une quantité égale à 3 à 50 fois sa quantité, d'un solvant choisi parmi l'eau, le méthanol, l'éthanol, le propanol, l'isopropanol, l'acétone et les mélanges d'au moins deux des solvants précédents.

35. Procédé selon l'une quelconque des revendications 32 à 34, **caractérisé en ce que** l'on emploie pour l'extraction un solvant choisi parmi le chloroforme, le chlorure de méthylène, l'acétate d'éthyle, l'acétate de butyle, l'oxyde diéthylique, l'oxyde de méthyle et de t-butyle, l'oxyde dibutylique, l'éther de pétrole, le xylène, le benzène et le toluène.

36. Procédé selon l'une quelconque des revendications 32 à 35, **caractérisé en ce que** l'on isole l'isomère désiré en éliminant le solvant par distillation.
